(19) Europäisches Patentamt
European Patent Office
Office européen des brevets

(11) EP 2 329 037 B1

(12) EUROPEAN PATENT SPECIFICATION

(45) Date of publication and mention of the grant of the patent:
**28.01.2015 Bulletin 2015/05**

(21) Application number: **09787623.9**

(22) Date of filing: **17.08.2009**

(51) Int Cl.:
***C12Q 1/68*** *(2006.01)*

(86) International application number:
**PCT/IS2009/000011**

(87) International publication number:
**WO 2010/018601 (18.02.2010 Gazette 2010/07)**

# (54) GENETIC VARIANTS PREDICTIVE OF CANCER RISK

GENETISCHE VARIANTEN ZUR VORHERSAGE DES KREBSRISIKOS

VARIANTS GENETIQUES PREDISANT LES RISQUES DE CANCER.

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **15.08.2008 IS 8756**
**16.01.2009 IS 8783**

(43) Date of publication of application:
**08.06.2011 Bulletin 2011/23**

(73) Proprietor: **Decode Genetics EHF**
**101 Reykjavik (IS)**

(72) Inventors:
- **RAFNAR, Thorunn**
  **IS-108 Reykjavik (IS)**
- **SULEM , Patrick**
  **IS-107 Reykjavik (IS)**
- **STACEY, Simon**
  **IS-201 Kopavogur (IS)**

(74) Representative: **Arnason Faktor**
**Intellectual Property Consulting**
**Gudridarstig 2-4**
**113 Reykjavik (IS)**

(56) References cited:
**EP-A1- 1 947 194**

- **SA SAVAGE ET AL: "Genetic variation in five genes important in telomere biology and risk for breast cancer" BRITISH JOURNAL OF CANCER, NATURE PUBLISHING GROUP, LONDON, GB, vol. 97, 14 August 2007 (2007-08-14), pages 832-836, XP007911714 ISSN: 0007-0920**
- **THORUNN RAFNAR ET AL: "Sequence variants at the TERT-CLPTM1L locus associate with many cancer types" NATURE GENETICS, NATURE PUBLISHING GROUP, NEW YORK, US, vol. 41, no. 2, 1 February 2009 (2009-02-01), pages 221-227, XP007911716 ISSN: 1061-4036 [retrieved on 2009-01-18]**
- **JAMES D MCKAY ET AL: "Lung cancer susceptibility locus at 5p15.33" NATURE GENETICS, NATURE PUBLISHING GROUP, NEW YORK, US, vol. 40, no. 12, 1 December 2008 (2008-12-01), pages 1404-1406, XP007911717 ISSN: 1061-4036 [retrieved on 2008-11-02]**
- **SHANBEH ZIENOLDDINY ET AL: "The TERT-CLPTM1L lung cancer susceptibility variant associates with higher DNA adduct formation in the lung" CARCINOGENESIS, OXFORD UNIVERSITY PRESS, GB, vol. 30, no. 8, 1 January 2009 (2009-01-01), pages 1368-1371, XP007911719 ISSN: 0143-3334 [retrieved on 2009-05-22]**
- **SIMON N STACEY ET AL: "New common variants affecting susceptibility to basal cell carcinoma" NATURE GENETICS, NATURE PUBLISHING GROUP, NEW YORK, US, vol. 41, no. 8, 1 August 2009 (2009-08-01), pages 909-914, XP007911720 ISSN: 1061-4036 [retrieved on 2009-07-05]**
- **K. A. POOLEY ET AL: "No Association between TERT-CLPTM1L Single Nucleotide Polymorphism rs401681 and Mean Telomere Length or Cancer Risk", CANCER EPIDEMIOLOGY, BIOMARKERS & PREVENTION, vol. 19, no. 7, 22 June 2010 (2010-06-22), pages 1862-1865, XP055045700, ISSN: 1055-9965, DOI: 10.1158/1055-9965.EPI-10-0281**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **L. A. HINDORFF ET AL: "Genetic architecture of cancer and other complex diseases: lessons learned and future directions", CARCINOGENESIS, vol. 32, no. 7, 31 March 2011 (2011-03-31) , pages 945-954, XP055045492, ISSN: 0143-3334, DOI: 10.1093/carcin/bgr056**

SEQUENCE LISTING

<110> deCODE genetics ehf

<120> Genetic variants predictive of cancer risk

<130> 2561-PC00

<160> 4

<210> 1
<211> 267235
<212> DNA
<213> Homo sapiens

<400> 1

ggggtagcca ggcagggccg tccacaggag caccagggcc gcactggctg tgtcccactg 60
ccaaaggctg caaacaattc ccacagaccc agggccaggg cctgtgaacc aagaacccca 120
gtctatcttt gtctcaggtt gccaggcctc ctggaaagcc cgaagtcctg gggcagctg 180
tgtccagcag acgctgcacc cagcagtctt gggggttggg ctgacccgag agagtgggca 240
ttgctggtgc ccagacccca ggagaggggg tggccagcat gagggtcaca gtgccagggc 300
atggctgggg tcaagccaca ctcagggcca gtgtatgcct ggacctaggg gcaccaggca 360
ggggggcaca gccaggaggc aggtgtggga tgcatctcag ggtgcctgac tctaggcata 420
aaaggcgctg gtttctaggc ccagctgcct gctgtgggtt attaaagggg aaggaccctc 480
ccctgcagag ttgcgcttgc agcctcgtct cccagaaggc atggccagcc ctgagccacc 540
tcagcccgac accaggaggg gtgatgtgca ctcgtgtcct cggcctggga gagtgtgtgt 600
cctgcaggca ggcgtgtgtg tgtggtggag tgtgtgtgtg cgtggcctga agcccggggc 660
tccgtgtatt gcagagtgcc tcgggcccgg gcctggcctt cgtcgtcttc acggagaccg 720
acctccacat gccgggggct cctgtgtggg ccatgctctt cttcgggatg ctgttcacct 780
tggggctatc gaccatgttc gggaccgtgg aggcggtcat cacacccctg ctggacgtgg 840
gggtcctgcc tagatgggtc cccaaggagg ccctgactgg tgagcgcaca gctccgccgc 900
cctggaggac ccgtccccag catctgactg tccactcccg cccgctgtcc agacgcccct 960
cctggatgga gagcgcaagg ggccaagcct gagttcaggg aaaggctgag ccaggctact 1020
ccttgctgac agccatcaac ggagagccga gggtcgaggg agggtcaggg ctgcccctcc 1080
cccacggccc cggaggccac atcccccatc tggagcagga aagcaggtcc ttcctgctcc 1140
ttgagcacaa tacaggaaag agactgggga gggcagggat ggagggtggg aagccgagag 1200
aagtctccag cccaggtgcc ctggccctcc ccgctgtccg agggcaggtc tgcctggctg 1260
gcttcctcct gaccctcccc acacctccac tccccatccc cttacccccc acaccccttt 1320
cccactgccc cagggctggt ctgcctggtc tgcttcctct ccgccacctg cttcacgctg 1380
cagtctggga actactggct ggagattttc gacaattttg ccgcttcccc gaacctgctc 1440
atgttggcct ttctcgaggt tgtgggtgtc gtttatgttt atggaatgaa acggtgagct 1500
gccgccccgc cgagtgctcc tctgggaccc accagggtgg gacagggaat ggctgccggg 1560
cacaggttca acccgcagct gcccagaccg ccgagaatgt tctgccctgg ggagctgccg 1620
aggcaccaga gggtgcaggt tggaccccag ttagggtccg atcctcggct tggagtgggt 1680
ggaccttcca cagaccatgt gaagcctgag cccagcatct ggtgcaggtt ctgcgatgac 1740
attgcgtgga tgaccgggag gcggcccagc ccctactggc ggctgacctg gagggtggtc 1800
agtcccctgc tgctgaccat ctttgtggct tacatcatcc tcctgttctg gaagccactg 1860
agatacaagg cctggaaccc caaatacgta ggtccttccg gtgggaacct gggaagtcct 1920
gggacccctc gggcttggtc tggcccctac ggtgccatcc ggtgcccgac gacccatgcg 1980
gtgcacattc acgtgctagt gacaaggtgg cgtggtcact tctgcctggc aaactgggaa 2040
gccagggatg tgaacaggat gggctttgtg cagcgcccga gtgcccgctg ggatcagaaa 2100
tggcatgggg gtgaccatgg gcaagcgcct ggtcagtgga gtgaggacct gagcagtgcc 2160
cagagcccag ggaccccaca gggccctgga gctcctcctt ggggatgtgg cttcatcctc 2220
cctgcctgtg tcctgcccat gtggcatggg gacgggcagc cggacaacgg cccattcctg 2280
gtctgtgaca agagaggttg tgcacagacc cctactgcca cccagtgggt cagtttggct 2340
ggaggccgtt tccacacaaa agcctcccga ggagagatca gagtgccagc cacactccga 2400
aaacgcctca ggggctccaa gcagcgccag cccctgacat accatctgag tccccactgg 2460
tggccgctca aaacctcacc atgccacccc ttctcgggaa tgtggacgcc actcctctcc 2520
aaatgtgtct tggaaagtcc ctccgtagca gaacaccaag ggcaggtgaa ggaggagtga 2580
ccatgtattt ttgaagaatt tccacggtac ctgcagccgg gtgggtggtg ccacccagtg 2640
tataggggag aaagctgggg acacggcctc caacgacgcc caatgggttg aggccatgta 2700
gcccctctgg ttccagaaca caacacctag gcctacctcg ggaggcccc agccccagga 2760

gaagctcagc ccaggtcccc aaactcctgg cccggagggg cggtggtgag cggccacacg 2820
gcctggccac tgctctggcc gtgccttttc cattcccatc caagtccggg tgggcaggtg 2880
gctcttgccc cttggattga ggagcacggg ggtcagcctc acggcccagg gtggccggcg 2940
ccagctaatg aggctgtggt cccgcaggag ctgttcccct cgcgtcagga gaagctctac 3000
ccgggctggg cgcgcgccgc ctgtgtgctg ctgtccttgc tgcccgtgct gtgggtcccg 3060
gtggccgcgc ttgctcagct gctcacccgg cggaggcgga cgtggaggga cagggacgcg 3120
cgcccagaca cggacatgcg cccggacacg gacacgcgcc cagacacgga catgcgcccg 3180
gacacggaca tgcgctgaag ccggccggag cggggcctgc atgggcgggt ctgtgggggg 3240
gcttggcctg atggtgggcg gggccccgcc cacagggccg accccaatac accagcgact 3300
caaccttgat gccgctgtgt acgtgtggtt actgctttcc tgacgcccgg gcagcgctgc 3360
tgcttggggc ccgcccctag ggaggtctcg cagggccccg gcatccctgc tgagcccgcg 3420
gcctgggcgt cctgcagcgg ctgaggtccc tgcgcttggc cgggctggct cagggatctt 3480
cgcaggtgcg gggctcccgg tctcacagac tctgaaccta gcctggctgg tctctgggga 3540
agagcggccg cccgggtggc tcaggggggcc gcctgcaccc cggctccagg tcccaggcgg 3600
gctgtccccc tacagctcgc aggcggcagg ggtacccagg acagatgctt cctcccgcct 3660
gggagcgggg gcctggggtc tggagtctgc ggtctggctg acccttcctg aaatttctga 3720
gaaacactgt aggttgccac ttttattttc ccaaactgtc aaactgtatt aaggaggtac 3780
caaaaggctc ctgctgtccc ggggcagagg ggcgacaggc aggagctcgc tcacgtgggg 3840
cagtcacccg cgccccgggc gccaagcgca ctgtgcagcc cccgtccagc ccgccctgct 3900
ccagagtacc ccggctccag gcgccacgcg cgtgcttccc tcctccctct ccctctgttc 3960
cctccgccct cctcctccct ctttcctccc catccttttc cctctcctcc ttcccctgca 4020
acagcaccac ggagccaggg atcaggtctc tgactcgcca cacggaggga ggccccgcag 4080
gtcctggctt cctgccgccg ggagggggccc tgtgaagacc caaggcacct gctcccgctt 4140
tggaaaaggg cgcctggcca ggcagcactg tccgtggtgc tgaaaaatcc cagcatcaag 4200
gcgcggccgc ggtgccaagc gtccattaca cgcattcttg ttgggaaccc gtccttgacc 4260
aaggccagcc cagagtaaac aaaagcacgt gcaccgctac ccacctgcag ataagctgag 4320
tgctaaacat gcagttaacg ccagtgcacc ccacccaagc gcatcctgag cccacagtgg 4380
gggaagctgc ccacctaaac gtgtcctgag cccacggcag gggaagctgc ccacctaagc 4440
gtgtcccgag cccacggcag gggggagct gcccacctaa gcgtgtcctg agcccacggc 4500
aggggaagct gcccacctaa gagtgtcccg agcccacggc aggggaagct gcccacctaa 4560
gcgtgtccca agcccacagc agaggggtgc tgcacttcgt ccctccgtcc tgcagttgtg 4620
atgcccccca caccggtggt aaggcagggg gacttgagga tgaacatgtc acaaagcgag 4680
tcagtccact gtgagataag gctgctgcag aggacagtct ggcatccccc agttcagcct 4740
cacaatcttg ccacaactcc atgccttggg cctaatagcc agcctcttcc attgtaactg 4800
acacaaacaa actcaaacca aatggccttc accagctctc agaactggcc aagttcaggg 4860
tgggcttcag gccctactgg acccaggagt tcacaggatc catgggactc tctccccaca 4920
ctgttcagct tttttaagta gggcgtctct gtgaccattc caaaatcctt ttggttcatg 4980
atctgggtga gatgctcccc cttcagagtc tagacacgaa attgcaggga aaagtgattg 5040
cagagcctgg agggtggggt ggagggaggg gtgcttcccc aaaggaaagc ctccagcagc 5100
caaagcaaca gctgcgttcc atggtgtcac ccactcacag agcctggagg gtggagtgga 5160
gggaggggtg gttccccaaa ggaaagcctc cagaagccaa agcaacagct gcgttccatg 5220
gtgtcaccaa ctcacagacg caaaaggtag agtaacctgc tcagaagcaa acactgccaa 5280
gggcgtgtgt ccccaaaggc agctcttggg acaccttccc caactcacca tagggcctgc 5340
aagtcctgtc gttgacccgg acgagaaaaa caggcagctg ttggcagcag gggtgctgac 5400
aagccactca gacttatccg aggttacatt ttgcgtcaat accatactta gaaaataact 5460
aggatgtgag caggtgcccc ctgctcccac cctggatcag acttttgggt gtagcacatc 5520
ctaacgaggg ctggggctct gcccaacact ctgtatgggg ggagctagaa gcagcatcta 5580
tagccccacc ctgaagcttc agagtccttg tccagctgac gaaaagggaa ggggtggggg 5640
gctgggacag aggacacagt ggacgctcag ccctccagaa aatgccacac cacccatgtg 5700
cctgatgcag ccatggataa aagtgggtag aaaggaggag ggacagaggt ggcccaatag 5760
gtgatgacag gtggacagac ggacgccaca aagggaggca gaggtggtct gtcccatcac 5820
attgccaagg ttgttcaggg acaggggctg aggcctcatg ggctgtggag gtacccgtgc 5880
accatcgagg gaggggaga caagggccag gcggcgagag cagaactgga gggactgacc 5940
ctgcgtggcc acgccccatg ctcaacacct tcaacttgtc cctcaccctt gggaaaaaca 6000
ttccaggggc gggtgtctca gactggccag tgtcgggaca ggaacaaaat gctcctatca 6060
cctgaccaga agtggctgga atagaggatc tgtagctcca caggggctct ccccttccag 6120
gggccaccgt gttgaactgg aaataaagcc ggctgcaaat gaggccagcc ggccccagag 6180
atggacacag ctcccatgac catggtggtc acatgaggac aggctgtccc gtgagcgggt 6240
tcctggcaga gggtgacccc aaggctgctt tgccaggcac agaggagtca gggagcactt 6300
gatgggaaaa cgaggttcag ggcccagttt tcaaaagcct gatggggctc atcctcaggg 6360
gagagtctga ggtcaccaag aaccagggga gaatgcgtga aggacataag tgcagaagga 6420
gaagtgaggc gggaggtcat ggagggcgca gtcagcactg gagctgcccc aggactcacc 6480
cacccagctc caagggaagc agccccttcc agcaagggtg ggcctggacc tgacagagag 6540

gaccccgctg ccacggccag ggagcatgag tctcccgtcc acactggggg ctggggactt 6600
accactccag gtggcgagcc cggggcttcg tgctgtgtca gaggagaaag ccagccaggc 6660
cccctccggc aagactcggt tctttcatgc ctgcatgagg ctgaggtgag gcccaggcct 6720
gtcgtgatgc tcgctctctg ggccgcagaa aatgggacca tggaacctgt gcatccagat 6780
ggcaccttgg agacctgtcc gaccctcctg caggccacag cggagtgccc aacaccaggc 6840
tccacaaacc tgggtgacct gcactcgccg gggggccctgg tcaaagctgg atccagggtg 6900
aagaggctcc atcccttgcc cgtcagtgtg aggactccgc cagcggcctc ggccattgga 6960
gctggatggg cacaggagcc ctgctgcccc agcacacaca tttcatcatg accccaggtg 7020
accagcacgc agggaggctg gcagccccac tgtctctgtt cctttctccc tctcggcagc 7080
aactccacct gagctgtccc acaggatccc caggggtggg ctgagagtca ctctgagaac 7140
tgtccctgta ttcatttccc tgtactgcta ttccatttgc tgctgtgacg aatcactgca 7200
gcttagtggc tgaacacaaa cgcatcatct tacagctctg gaggccggaa agctgaaaca 7260
ggtcccactg gcctgaaatc gaggcgtggg cagggccgtg tgccctcagg accggggagg 7320
attgcttgct tttccagcat ggagcggctg cccccattcc ttggctgggg acccccctcct 7380
ccatctcaaa gccagcagtg gccagtggtc cttctcacgt cccctctctg actctcccct 7440
gcctccctct ctcacttcta gggacccttg tggccatatc aggcccacca gataatccag 7500
gatgacctta agatcggctg actggcagcc gtgattccac ctgcagcctc caccagcctc 7560
tgccttgcgg gtgacacatt cacaggttcc aggagaagga cgtgggcatc tttggggagg 7620
ggctgtaatt gtgcctgcca caagtgcctg gggcttctga aacccaccaa agtttggcaa 7680
gccccctgca cagcatcctt cccaggtggg cacctggcac caacatcgac ggttacagca 7740
ggtgcaggac cggcaggagc gtggggctga ggcaggaaaa caaccactcc ctttcagggg 7800
tcctggctgg tgtcacccac agcctccacc cttgcctgct tctcctccct ttctgctttg 7860
aactcactcg ctccatacac gcttgtctgt ggaaggaagc tgcttgagat gaagttcagg 7920
cctaaggaag tccaaagagc tggggttttt ctttcccttt aaaactttgt tattttcatt 7980
ttgctctgag atttgacagt ctacctagca cctagcaggc ctccttctcc ttctttcttc 8040
cttcttcctt cttcttctct ttccttcttc cttcttcttc tcttctcctt ctccttcttt 8100
tttttttttt ttttttgaga cagggtctca ctctgttgcc caggctggag tgcgatggta 8160
caatcacagc tcactgcagc catgacctcc cgggctcaag cgatcctccc actcagcctc 8220
ccgagcagct gggactacag atgctggacc ccacgcccgg ataacttttt gcattttttg 8280
tagagatggg gtctccctat gttacccatg ctggtctcaa actcctggcc tcagacaatc 8340
cttctgcctc ggcttcccaa actactggga tgacaggtgt ggaagccact ggacctgacc 8400
ttacatagtg gtttttaaaa gtgccaggca agggcagtgg ctcacacctg taatcccagc 8460
actctgggaa gctgaggccg gaggatcact tgagcctggg ggtttgcggc tacagcgagc 8520
catgactgtg ccactgcact aacaacctgg gtgacagagt agacaccatc tctaataata 8580
ataataataa taaataaaaa cagacaaata aagtaggtgc ttagtgagtt aagccatatc 8640
aaagcaaata cctttttttaa gggtctaata ctttagcaaa aaatataagt aatcctgagt 8700
aaaatttcaa caaaaaacac ccggagaaac aggaatctgg agctttgtag aagatggata 8760
gctgaacaat ggcctgacct gttggtctgt gattcaaacc caggtgtgcc tgagcagcgg 8820
cctctgctcc tgggagtccc cgccctgttc tgtccaacct cccactgagg tcgtccgccc 8880
acggcgcccg gggccgctcc cttccagttt gcagggcaaa ggcgcccccca ggccggctgc 8940
caagagcccc tccttcctca cagctcctat acgggggctc caggcaccag agtgcattta 9000
cgacacagtc tgtggacttg ggcccaccat aaacacatta ggctcggtca ctcagacatt 9060
ctctgaagca gcagcaataa atgacgccat atccagtggc cataagcccc caccccgaca 9120
ccaggcacgt ccgggctgca ctcaaaccag aagccctgtc caaggtgcac tcagtaggac 9180
ccggccatac cataaggccc caccagggac ctgctggaag gctctggagc cactcacgca 9240
gggccctcgg tttcgtgtgg cccaagccaa cttctgagga ggggctgttt cttcacccca 9300
agactgcgtc cctctgagcc gcccctcctg ttctctgcaa tccttccagt tccttctgcc 9360
tgaggaagga cgtatgtcca gctccactgc gagcctggca cccgccctgg gaggtaagtc 9420
tggatgagac taggagataa gaggataaga gatgagactg ggagatggta agaggatgac 9480
agatgagact gggagataag aggaagacag atgagactgg gagatgataa gaggatgaga 9540
gatgagactg ggagatgata agaggatgag agagcatcac cagccccacc ctgaagcctc 9600
ctgtgtccac accctccact gccccaccct acccaccctt cagagatcag aatcctggtc 9660
tactcttaag agaatggcca gaggcggaat gtgactttca acctgagttg gggcgttcag 9720
gaggaggaat cctggatttt atacaggatg gtgtcatgcc cctgcccccc acctccatga 9780
aagctgatgg cccagaggat gtgcaccagg gaagcacatc cggatccacg gctgcaggtg 9840
ggaggcaggg gaggaggggg cccagccagg cttccccatc ttccccgcca cccagaccag 9900
cccccccat caccccctgcc accccagctg gggcccccat catccctgcc accctggcca 9960
ggccctccat catccccgcc gcccaagctg ggcccccagc atccctgctg cccttcgggc 10020
ctggacttac tgttatgtct tccagggtgg gggctcccac tgtctatccc ctaccctcct 10080
tcccctcctg cctcacagca tcagaaacct cccaggccca gccaggccat gggcccgcac 10140
tgaccacaac cccattcact catagacttc cgtaaatgca cagccaggcc atgggcccgc 10200
actgaccacc accccgttca ctcacaggct tctgtaattg catacagtgg tgtctatgca 10260
gtgcacatta ggattcaaac atgagatttt tttcaaaact gaaaaactca tatattcagt 10320

```
attttactcc cacagcacct ccccccaatt tgacccacag ggacccccat ccaggtgcag 10380
ggtcctcgcc tgtgtacagg gcacaccttt ggtcactcca aattcccaga gctcccaggg 10440
tccttctcag ggtctccacc tggatggtgg gggtggaagg caaaggaggg cagggcgagg 10500
ggtgaacaat ggcgaatctg ggatggact attcctatgt ggggagtgga agccgggctc 10560
ctggtgagga aaagctggcc ctggggtgga gccgagcgcc agcctgtggg gaagtgaaga 10620
cggcaggtgt gctggacact cagcccttgg ctggacactc gctcaggcct cagccggaca 10680
ctcagccttc agccggacat gcaggcctcg gccaaacact cactcaggcc tcagactccc 10740
agcggtgcgg gcctgggtgt gggccgcccc tccctccctg ggacgtagag cccggcgtga 10800
cagggctgct ggtgtctgct ctcggcctgg ctgtgggcgg gtggccatca gtccaggatg 10860
gtcttgaagt ctgagggcag tgccgggttg gctgcggcct ccagggcagt cagcgtcgtc 10920
cccgggagct tccgactcag ctgcgtctgg gctgcggggc caaaatcaga ctccgttcca 10980
gaagaggcca gaggtggcat cctccaaccc tcctaggacg tgtgggtggc cgggcaggca 11040
gggcctgcac ctcgtggccc tggctggtgg gaacctcagt gaagggacag acacccctcc 11100
accggggcga gcagacaggc agcccagtga cgctggagcg gcggggcggg gagagaactt 11160
gctcaggacc ccaggagctc agggcagcag ccagggaaga ggctgtgaga ggacacaggt 11220
caggggtcag aaggctccca agcgtgggga gccatcgccc ctgagatggg aagcaggggt 11280
tcaagaagtt gtggaccctg ccaggctccg tggcctgggg acaccagcgt ttaatcacat 11340
agggctgcca gcgtggctcc aggcccccag ggtcaggccc gggggcgtct gcacttcagc 11400
agcatctgag gctgctcgcc ccacacaggg cgttcaagga tgacccctgg gcaggtgggg 11460
cccgcactgg cctccaccca cacttgcctg tcctgagtga ccccaggagt ggcacgtagg 11520
tgacacggtg tcgagtcagc ttgagcagga atgcttggtg gcacagccac tgcacggcct 11580
cggagggcag agggccggcg gcgcccttgg cccccagcga catccctggg ggaaaacaga 11640
ggctgaggag tcacaggccc agcccagctc ccctcccagg agacaccgga aagctgccca 11700
cacccgacgg tctgcggggt ggctccatct ctggctgtcc cgacccaagc accgcaggag 11760
tcacgacaga aatgttttct tcggcttctc caggtgaaat ttccacaaca cagaaagaac 11820
agaaagtaga agctgttaag ctcaggaccg caggttcgac atggcttgaa tttcagacgg 11880
atcagaaacc tccctgatgc tggacaaagt gtccaggggc agaggacggt gactgggaag 11940
cagaggacgg cgcggggctc tgtcgtggtg atacgcggcc tctgctcctg agaggggtgg 12000
ggtagcctgg gcctggcctc ctcctaagcc cttggcctgg gtttgcacaa gggccctaag 12060
tgccatggac gggagacagc ccaatcccac cacagggcaa acaggagagg ccaggccccc 12120
caaatcccac catggggcaa acaggagaga ccaggccccc cagcgcttcc ccaggcagag 12180
caaggtggga aggatggcag ctcccagaca gctcctgtcc cttccatcag gtgctctgct 12240
caccccacga gagggcgggc agacgagcct gacagtggtt gggttaaacc acttcctgat 12300
gcgaaaaggg gtaagaactt cctaagccca gattcactca gtctcctgac acactaacac 12360
cagcaggcag gcactgctgc cactgaggcc aggcacctgc acatacctgc gttcttggct 12420
ttcaggatgg agtagcagag ggaggccgtg tcagagatga cgcgcaggaa aaatgtgggg 12480
ttcttccaaa cttgctgatg aaatgggagc tgcagcacac atgcgtgaaa cctgagagga 12540
tggcggacag cgtcagagga aaggcctcct aatcagacgg tgctcgtggg tgtgggcatg 12600
ggcccaccgg tgcctgtgtg cgtgcatgaa tgcacatgca tgggtttcct catgggcaca 12660
ggtgcacaca cacggatgca tgcatgcatg tctgtgtgtg tgcttgtgtg tgcgagtagc 12720
tgcgtgtctg tgtgtgcaca ggtacactgc gtttctgtgc accatctgta tgaacacgca 12780
tgtggaggct gaagcagctc catcctggat gccagccccc catgctggct tctgattagc 12840
ccctgttccg ggaaggcctc taaggtttcc agtttatcca ttgttccttg tgtaccagca 12900
ggtacttatc atgaatcctg cccttcgtca gacaaccttg atgtcatcgt atatcaacgt 12960
cctgcgcatc ccttctgagc cacccgcccc tgtggtgcat aaaccctggg tctggggtga 13020
tggtgtgagg acccatcatc tcatctcact gacaccgaga caccgacttg gcttctgtgt 13080
gtaggttaag ttcctactaa atgtttcttt ccaagaaact gcatttgtca gtcttctttg 13140
ttgttgttga gatgggctct cactctgtca cccaggctgc agtacagtgg tgcaatctca 13200
gctcactgca gcttccacct cctgggctca agtgatcctc ccacttcagc ctcccaagta 13260
ttggaactac aggcgcacac caccatgctc agctaatttt tgtttttatt tttcatagag 13320
atggggttct actatgttgc tcaggctggt cttaaactcc tgggctcaag ggatcctctc 13380
gcctcggcct cccaaagtgc tgggactaca ggtgtgagcc accgcgccag ccctgtcagc 13440
ctccatcttt ggcctctcag cttcctggga ctttggggca ggtgtgcaca gacctggcta 13500
ccatgaaaca gcacatgtgc acatgtgctc atatgtgcgt gtgatcagag cccccgtgta 13560
cctggtctga ccctctgcct gagccccccg tgtacctcat ctcacccagt gcctgagccc 13620
cccatgtacc tggcctcacc cactgcctga gccccccgtg tacctcatct cacccactgc 13680
ctgagccccc catgtacctg gtctcactga cttcctgagc cacttctgga atgacctgag 13740
atcacaccag tcaagccagc accccggttt cagcctctca gtaactctgc tgaggtggga 13800
tgtcttttcc ccatttagca acaacggaag ccaggcccag aatgttttta aacaaataaa 13860
taagcctaag gtcccggggt tgccacacgg ccacgctcct gacccaggag ggaaacacct 13920
agcatgggtg aggggctctc ttccaaaggg agaaatagcc acctgctaga ggtcggggcg 13980
tccaccccaa gcccgtgtgg aggcgcggcc agcacgggcc aggaaggccg agccccagcg 14040
gatttgaatc agaccccgcc cccagcgcca cgtggaccac cacagcctcg cccactgcag 14100
```

cctggctgcc gtgaaatcgg ggcccagctc tccgttctcc cactcctgcc tcgagggaag 14160
gggattcctg ggccttccac gtgcctgact ctgtgtttgg aaacggcccg tggcccatcg 14220
catctcggcc tcctcagggc tacccaatca agcgactacc caagggtccc cacatgggtg 14280
gggaaactca gccgccctgc cctgagccca ggccctctac gggagctcct cggagctaga 14340
gaccctggtg gggaaatgag gacaagactg agctgaatat gggaggagca ggtggcaggt 14400
cacatggccc tgggagtttc ccaggagttt tcacctggga aaactcctct gacctcatca 14460
tggcccaagg aggccccaag cacgccagct ggaccctggg gtcaaccaca gtgtcagaca 14520
cctccagtcc tcagtggcac ctgtatccag cacaccccaa gggtttccgg aaaggagtca 14580
agcagcttgc acgagggccc aggcacgcgt cagggagatg caaacgaggg aagatttgag 14640
gcagagagag aaggcagaga ggagggcggt ctgagccgga cgcaccctgg aactgagaca 14700
gcctggtccc aagcctctgg agctggtgca aaggcggtaa catgcagctg tggctctgcc 14760
ctccctgcca gagcatgaag gcgcaagcac tcgggttaaa aaccattttc atggtacgac 14820
cttgtaatgg aaaactctcc cgtgagccct tgctgggttt ccacgataga cgacgacctc 14880
atttcacatc aagtgtgcac ggtaattcgc gcagcctcgg ccctgggtaa cacaaatgct 14940
cacactcctt ctcagccctc atggggcgag ggcttcggcc tcctggcatt cagctcccag 15000
tggctcggga ggcctgttga ggtggaggcc cgggcctgtg gtgcccgact gccctttggt 15060
acagccccga ggcttgtgcc accacctgcc ctgactccag gcagaagctg gccctgtggc 15120
ctccacagat tggcccttcc ctctgtgata aagttgtcga ttcttggcag gggcaccccc 15180
tgaccagcca ggacagggaa cgttccagga gcacagccat gcccagagca caggtgctgg 15240
ccgggctgtg tggggagcca cagctctgct gcaccttccc gggggtgggg ccagcgaccc 15300
tcacctgcac ccacctcggc cctctttgct gagaccccaa atcctccggg catctgatct 15360
cccatctact gttcagacac cttcccagcc tcctctgcta agactcccca taaacatctc 15420
tttaccccat gcccctgctg ccctgggatg gcctccatga cgtcccacgg ccagtgcaca 15480
ggcacagtgg ggacacctgg ggccacaccg gctcctacca ctacccagag atggagaaca 15540
ggtgctttgc acagagccac gcgaacagaa ctgtgcacag gcagcagcac cactgaaaac 15600
gtaagacatt ccttgcccct aaaacccagg agttccaagg tgaagccccg ggtcagaggt 15660
gagcagagcg cggagggtcc ctggaggctg ggcctgcacc ccttggtggc ggctcacctg 15720
tacgcctgca gcaggaggat cttgtagatg ttggtgcaca ccgtctggag gctgttcacc 15780
tagagtcgcc aagaaagagt gagaaacggt agaaacctct ctgggatttt aagtttttac 15840
tttttgcttt atcatccatt cagatggaac aagaaagagg aacattttga caagaaacta 15900
tccctcttcc cagtgaaatc cggcctggcc ctcacccggc agctgcgaac caccctgggc 15960
gagtcaagac tctgtgtcat ctgcctgccc ccgaggctcg gccaagacag gaaggaacca 16020
ggagagggag tggacgcaaa tgcccacaga gaggggaggt ggacgcagat gcccacagag 16080
agggggagtg gacgcggatg cccacaggag agagggagtg gagtggatgt aaatgcccac 16140
aggagagggg gagtggacac agacgcccac aggagagagg gaatggagtg gatgtaaatg 16200
cccacaggag agggggagtg gacacggacg cccacaggag agagggagtg gatgcagatg 16260
cccacaggag agggagtgga cgcggatgcc cacaggagag gggagtgga gtggatgcag 16320
atgcacacag gagagggagt ggacgtggat gcccacagga gatggagtgg atgcagatgc 16380
ccacaggaga gggagtggac gcggatgccc acaggagagg gagtggacgc agatgcccac 16440
aggagaggga gtggacatgg acgcccacag gagagaggga gcagacgcag atgcccacag 16500
gagagggagt ggatgcggac gcccacagga gggggagtg gacacggacg cccacaggag 16560
agagggagcg gacgcagatg cccacaggag agggagtgga cgcggacgcc cacaggaggg 16620
gggagtggac acggacgccc acaggagaga gggagcagac gcagatgccc acaggagagg 16680
gagtggacgc agatgcccac aggagaggga gtggacatgg atgcccacag gagaggggga 16740
gtggacacgg atgcccacag gagagaggga gtggacgcag atgcccacag gagaggggga 16800
gtggacacgg acgcccacag gagagacgag tggatgcaga tgcccacaga agagggagtg 16860
gacgcggatg cccacaggag agggggagtg gacacagacg cccacaggag agagggagtg 16920
gatgcagatg cccacaggag agggagtgga cgcggacgcc cacaggaggg gggagtggac 16980
acggacaacc acaggagaga gggagtggac acagatgccc acaggagagg gagtggatgc 17040
ggatgcccac aggagagggg gtgtggacgc ccacaggaga gggggagtgg acacagaagc 17100
ccacaggaga gggagtggac gcggatgccc agatgggaca atgccctcag ggctgcagct 17160
tcagagcctc agcccaccaa ggcctgggac cccgtctcat cctgtaagga gtggcactgg 17220
tgctgaggac ctagcggggt gaacacgagg accccctcaat acatgttatg tgcacacatg 17280
ctactcacac tgcacaccca cacgcatgca cctcacgtga atgcccacac gactgtgcac 17340
gaacacacat gcatgtcagg tgtgcattcg catgtgcaca cacttgtgcc cttgcacacc 17400
tgtgcacaca ctgccatgcc ttcatgtaca cacatgcaca catgtatgtg ctcacgtgta 17460
tatgcgtaca tgtgcactct tacgtgcagc cagtcaccat cagccttgca ggcacgcgcg 17520
cacacacaca cacacatact tgcgcacaca cctcctgaac tctgaactct gtgctgacca 17580
tcagcctgct cacctgcaaa tccagaaaca ggctgtgaca cttcagccgc aagaccccaa 17640
agagtttgcg acgcatgttc ctcccagcct tgaagccgcg gttgaaggtg agactggctc 17700
tgatggaggt ccgggcatag ctgagacaca gggggaatg tcagacacag gtgcctgccc 17760
cacacccagc cccctcctgc aaagcttgct ccaaagagcc tcctgccttc ctcccgcttc 17820
cttgtcctgc ctggggcatg cgctgcagcc cgaggggct gggtgctcac tccatggact 17880

ccaaatgcta catgccatcc agactctgca tgaccaaact ccaccaggac ctggctagtg 17940
atgttgagtg tatccaaacc tcgcacggcc gtgtttgtgc actcatcatc tgccctgatg 18000
agatctgcac ataactcagc caaaaacaca taataaactg tggagccctg gctcccgggc 18060
gtccctcgag tgaacacagg tgtgactcag gttttttgtg ggagaacact gagttgggcg 18120
tggggaatct cactgtgttt gaatctcagg gatggaagag ccaggtggca ccagctaaac 18180
agggctccag tgagtgcacg tggcacacat ggtgtctcca gaggggcagg atggaggcat 18240
cccaccgatg tcctccaggg cctcaccacc actcactgcc catgtatgtc catgtccaaa 18300
gatgaacaca catgacatcc acggcaggct cctggggtgt gttcttttac atccagcttt 18360
gaacactgct acattgaaat aaacgttttg tgccattaaa cattcttcaa aaattctttg 18420
aaaaatgttt aatggcagag caagttttgt gatgcataat atctccttag ccctgtattt 18480
gggcaggttc gtgacttgct gtggcaagtc cccctgcctg gaacactgga aatggtaaat 18540
aagacccgtg ataatgtctg gtcacttcag aagtggaatt actgggaaga cacaggacat 18600
tgtgaggact tccatgcaaa caagctgctt tccagaaaag tctttccagc ttgcctcaac 18660
ctgatgctgg ctcttggcaa gcccagaaaa ggagccagca tgagggatgg ggacaggtct 18720
cgccatcagt ttcacgtgcg tttctttgag ggatgggtct cgctgtcagt ttcacatgca 18780
tttctttgac gctgctggta ggaccttccc aagtgtgcat cagacgtcca tttcttcttt 18840
cacagccatc cccaacacga ccttgggctc cggctcaccc tgagcctatg ggtctgactt 18900
gtgtggggtc ctcgggcaga gcaagggccc cggagcagga ggccaggctg caatcatgca 18960
tgccctttgc cagctgggtg accaagggcg gctgctcccc tggagccttt cctcctctac 19020
ctctcaggtg gttaggagaa caaagtgaga ggatgaatgt gcaggacctc acagggcgcc 19080
ttaaataaat cacgtgcaca aacgctccag ggaatggcca ttttcactgt ggctcaggca 19140
agctgagttt ctctgcagtg aaagcaatgc ccgttgcctc actggtttca tccgcctccc 19200
tgtgtgtcct tccccaacac caaaccaggg aacgttcacc tcccttccct ttgagtcttt 19260
tgttttattt ctgttgctgg tgttgtttgc atgtgtctcc tcttctacct agaatctgct 19320
ctggtatatg ctgtaaagac acaacttatt tgcttccagt cagttttctc agaaaaaaaa 19380
attacacaca cactctcttt tccatcagct gtgccacata ccacattaac acacgtttct 19440
gagagatagg gcttccgttt tgttctattg ttctgtccat cagttctgac cacattctac 19500
catctgagct gtggtttggg agactaaaat ctggacaaga aaattcccat cttaccactt 19560
ttcctttttc acttttctta atcattttgc ctgttttctt ctcccagatg cccttcaagc 19620
ctctgctgta tacttcagac acagaggatg tgagttcacc ctgagtatgg cgaaccactg 19680
cctccactgc tgagctttta gagccacctc tgcaagcaca cactctgacc aagtggcctt 19740
tgggagcaca gaatattctg gcattggacc cacatctgat gacctgatgc ccacccatcc 19800
tgcactagcc tctgtggctt tgcaatgtgt ttagagagaa cccattttcc tgatttgttt 19860
gtctgtcctt tgtccttcaa taacattcac tgatgctgtg ccagaggctg gaaccagggg 19920
tgcctgtcct aaaaaataca tttcatgggg agcaaaataa gcagtggaca gttcacagct 19980
gactggtatg gccagcactg cagaggtgcc tgggagaggc gaagaggtgc tgggagccca 20040
ggaaatgtgg aaatcatgca gacaaagcca gtggaggcca ggaccaggta aggctgtgag 20100
agggaagcag ggactgtggg gagcacagga gaccggcatc cttgtgggga gggagctgca 20160
acagggacat gctgggcacc atgcacaagg gtgtctgcac ctgccactcc cccatcatga 20220
gggcgtctgc acctgctgct cccccatcac gagggtgtcc acacctgctg ctcccccatg 20280
aagcaaaccc cagggagcat tctggaaaga ggcaaagtcc agatctggac tgttaactca 20340
gaacgacagg accccagttt aaacctctgg tttaacaacc atccaccact tatttctctt 20400
cccatgaagg tgtgcctttc agaggaaggc acccccagtt ccggcctcta gcacagcacc 20460
agtaaatatt atcgaaggac agaataaaaa aacacctgtc cactatggtt ttggaatgct 20520
gatacttttt tttttttttt taagatgaag tctcactctg ttgcccaggc tggagtgcag 20580
tggtgtgatc tcggctcact gcaacctctg cctcctgggt tcaaatgatt ctcctgcctc 20640
agcctcccgc atagcgggga ctacaggcgt gtgccaccag gccaggctaa tttttgtatt 20700
ttttaagtag aaatggaatt tcactatgtt ggttaggttg gcctcaaact cctggcctca 20760
agtgatccac ccacctcggc ctcccaaagt gctgggatta taggtgtgag ccaccttgcc 20820
ctgcctggaa tgttgatact ttttattcat aaaagaaaca gcacagattt ctgcatctgt 20880
gtccatcttc aattcatttg tgtctgagcc tgagacgggg ccttgagctc tcgggctctg 20940
gggaaacaag accccagaat tttgtagaga cccccccccac accatggccc tgtcccctga 21000
tgggtcacac gtgatcatga actcatggca tcctgtagac tgtggatgag ccttggagtg 21060
tggggctcac agcggagaga ctcacgccca aaagggccct gaagtctctg ggttcggaga 21120
gactcacgcc cagcagggcc ccagggtctc gggttcagag ggactcatgc ccagcagggc 21180
ccctgtgttt cgggtttgga gagactcacg cccagcaggg cccgggcgtc ttgggttcgg 21240
atccagactg ctttctgaag acacctcagt gcacccaggt ctggtgcacc atttcctttt 21300
accaaaatag cacagaaaac tgctcccgtt gtgagcaccc tcactcccac agaaagatgc 21360
atttctgctc agccccagat gggacgaggg gcaccctgtg gcctctgacc ctttgggatt 21420
ggcagtcgcc tgccccacac ggaagcagag gtggacgcaa cggccctgca gcagcacctg 21480
ccccagccgg gcacaggctc cacttccggc caggtgcgct cacctggagt agtcgctctg 21540
cacctccagg gtccgggtat ccagcagcag gccgcaccag gggaataggc cgtgggccgg 21600
catctgaaca aaagccgtgc cacccagggc ctcgtcttct acagggaagt tcaccactgt 21660

```
cttccgcaag ttcaccacgc agccatactc agggacacct cggaccaggg tcctaaggca 21720
gaggggcaat gtcagcccca ggatgcgggg ccgtcaccca ggaggtaacc tgacaccctt 21780
gttaaatgct ttggaaaacc ccagagaagt ggtgatttgg agcagggtgc tgggcctggc 21840
aggagctctg aggagcctgg acccagccct gctccagact tcggggtgct ttccctgtct 21900
cccgggcagg acaggtaggt gagcatgcaa gaacctggcc tggacccggg acagccagga 21960
ctcagatggg aggtgcagcc ccagtggcct ctgtgatggt ccatctcatg tgtccccatg 22020
gcaacaccac aatcccgtta tagactcatc tgtggtccgg ggcagactaa aatctggaca 22080
agaaattgcc accatcccac cttcttttaa gacgtttctc aacgactttg cctgttttct 22140
cctagatgcc ctgaaggcat ctgctgtgct ttagacaaag gggagggttc tgagttcctg 22200
ctcaggcgcg ggacctggct gggctgtgag ctgggcaaca ccagtcgtca gcttcacgtc 22260
aaggaggttc cctcgccgag tcatgagcct cgctcaccca gctcagggag ccccccctgtg 22320
cccggtagcc tctgctctgg cctgggcatc cccttccctt cctgcggcct ctgctgtggc 22380
cccgggcgtc cccctgccct tcctgtggcc tggccctggg acctcggcca gctgcgcagc 22440
tcccctggtg cattcccagc tccctgtggc cacccccttcc tgttacatgg ctgccgactc 22500
atcagagaca gagttctgcc cccggtggga ccccaacata cactctgagc cacctccctg 22560
gccagcaccc tcaggaactt gccacacaga agcagcgttc cccgtctgct ctgtcccctc 22620
agcagcctca ggactcctga gccctcagtt gcttgggacc tgggctcccc ccgagtgaat 22680
cttagtggat ttaaatgtgc acagcctgct gtgctccggg ctgcggcaca aggaacgcca 22740
ggcatacccc cagcccaggg cggccctgga gggtggacct ggagggtgga cttggagggc 22800
gggtccaaat gggttttcac aaacacagcc cagcaaagca cctgaaacca cccctggcgg 22860
ccacattttc tgagggttcc ctttacagct ttcaaggttt ctcgtccctc gtcaaatcgc 22920
actttaaaac aagcccaccg gctctgtggg gcgcccttga gggggaccac attggacaat 22980
cccctgtgct gaggccaggt cccctgtgcc tgattttagt ttgattcaca aaaatcaact 23040
ctgaaaccca ctcctagcca tctccgtggt tcaagacagc agcactcagc aaatccaaat 23100
tctcccaccg atcccaaaca accccacgca ggaacgtgac cgcagcgaac gctgtgggcc 23160
agctcactca gcagctcctg ctctccgcag gctcaggtgc accctgggga ggaggctgcc 23220
gcctcggccc tgcaggccct gtgcacagct cctgggggtc tcactggggc cccaggagcc 23280
gccactcttg actttccaaa gagcagcagg agccaggtca ccgcctgcac tcaccacgtg 23340
tgtaacctgg gtgcgtctct gcaggaccag ggcctgcacg gtacccactc cctcccgaag 23400
gtgcccctgc gcccccaggc ctgggtgcac ggccaagcgc ctctgctctt gcggatccag 23460
caccggcaga gagagaggac ttggcagaga caacgctgcg gtgccagggg caggacacgg 23520
ggggctcaac tgcaaagccc acaggctgtg gaggtcccca cagacacacg gcacgggcct 23580
cacctgagga aggttttcgc gtgggtgagg tgaggtgtca ccaacaagaa atcatccacc 23640
aaacgcagga gcagcctaaa ataagggaaa atacacagca aggttaactt tacacttttt 23700
acgtagtatc tgtctacgag ggactgaatg tcaaacaatt tacacagcca taaataaagt 23760
aacattctcc aaagcggtta aatgaaaaac ttaaatttct ttccaacaga cgagctctct 23820
acagttaact gctacagcaa ttcctcattc attataagta ctcagtgttt accatcagct 23880
tgtgcaattc tgtgccagct gaagacgaac gaaggccatc aggcgcggcc ccacttctca 23940
gactcttagg gaaaaataac tgaagacggt aagaaagctg gggctgggaa cctcgccccc 24000
tctgagccac gtggtgccct ttaccagcat ggcagctgtg gtcctcagca tgatccgaga 24060
cccagatcct atccacagga agctgggcat ggtgcactgt gtgaccctca ggcccaagcc 24120
cagcgcccca tgaccatggg ccagaggtat cctgtcccag agggctgacc agtgtgccgc 24180
cagctcgagg acactttcct ccaaatccca gagcaagttg tgtgtacaag aaatttttgt 24240
ttctgttaca taaacttatt tcaatctaga acttgaacct taatgaagaa gaaccttgat 24300
ccactgtaca tgctataatt ggagctagag tttcaaaaga tgcacaggaa aaacccggag 24360
tcttcgagag aaatggctgt gtggcctgca ggcttcctga cgcatccttc actaactgtc 24420
tgcttgttca gtgatactat tttcataaaa aaataactgc tgtccacatc ctctccagca 24480
ccagaacact tttacactgt tggtgggagt gtaaactagt tcaaccattg tggaagacag 24540
tgtggtgatt cctcaaggat ctagaactag aaataccatt tgacccagcc atcccattac 24600
tgggtatata cccaaaggat tataaatcat gctgctataa agacacatgc acacgtatgt 24660
ttattgcggc actattcaca gtagcaaaga cttgcaacca acccaaatgt ccatcgatga 24720
tagactggat taagaaaatg tggcacatat acaccacgga atactatgca gtcataaaaa 24780
aggatgagtt catgtccttt gtagggacat ggacgaagct ggaaaccatc actctgagca 24840
aactattgca aggaaagaaa accaaacacc acatgttctc actcacaggt gggaactgaa 24900
caatgagaac acttggacac agggtgggga acatcacaca ccagggcctg tcatgggatg 24960
gggggagtgg ggagggatag cattaggaga tatacctaat gtaaatgatg agttaatggg 25020
tgcagcacac caacatggca catgtataca tatgtaacta acctgcacgt cgtgcacatg 25080
taccctagaa cttaaagtat taaaaaaaaa aaagtaactg ctgtcaataa aagccagctg 25140
agttgaacca cattaggttg gcacgatatt taggtgtgtg cgtccttggg tgtagacccc 25200
atgcaagtgg gatgggcaat gggctgaaga ggcgcccagt ccaggccacc tgtcgagggc 25260
ctgctgggag atgtggggcc tcaggctgca ccagccccat ctccccaagg accctaacgg 25320
aacctgggtg acgtgacaga agctggtgtg cttcctcggt gttgaattca catgctcatc 25380
atgcagggca gcggccctcc cgcctgctga gccctgaggc ctctggacat ggccgctgga 25440
```

```
cagagcctgc gtggagcccg cagtcctcag gctgtgcaac ccctcccgtg cggcttcata    25500
ccaagaaggg gctgcaacct tgtctggttc ctcaagacag agcagtcatg gtctccagag    25560
caccaggaat attaacactg aatgcatcaa aagcaaatca acccccaccc aagcccccct    25620
ggggaagagg aggcctcacc cgtcccgccg aatccccgca aacagcttgt tctccatgtc    25680
gccgtagcac aggctgcaga gcagcgtgga gaggatggag ccctgcggga tcccctggca    25740
ctggacgtag gacctggggc gggaagacac aggtgagaga cgggcagggc atgtgctgga    25800
catgcgtaca ctcaaaccga gccacacaca gacacagaac ctcatacaca caaacgacac    25860
gtctaccatt cagccggcaa acacctcaga aacataccag atgagacctc tgaacaaaca    25920
atccctgctc cccactctca ccatgcactg gggcggctga aacagatcca gccacaggtg    25980
tgaccacatc cttgctctga atcatcaagg ttttctttca ttaaggtttc ttaaatctta    26040
gagtttattc acagaaccag acacttgacc cggaatgaat gcttaaccgg actccttttt    26100
tttttttcaa ggaatttgtc cacggtgaaa gactcatgac cctacatgta gccgctgcgc    26160
gccaacggat acaaccttgc ccctagtttc agcatgacca acgagaagca gaaactctcc    26220
aaacaaggat gccaagggtg cgttttcaga caaacagtga gagcagaata gccccgtgga    26280
acctaaaggt gcacacagaa ggcatggctg ccacgcgacc ctcagccatc cccagagccc    26340
acaggacgcc actcctgttg ctaagagacg cttgcagcct actgcaaaaa caagacctgt    26400
tattttcggg aagcgctata ggtggtcacc ttaaaaagag gctttccggc tgggcgtgat    26460
ggctcacacc tgtaatccca gcactttggg aggccgaggt gggtggatca cctgaggtca    26520
ggagttcgag accagcctgg ccaacatggc aaaaacccct ctctactaaa aatacaaaaa    26580
ttagccaggc gcaggggtg ggtgcctgta atctcagctg ctcgggaggc tgaggcagga    26640
gaaatgctgg aacccgggag gcggaggttg cagtgagttg agatcgcgcc actgcactca    26700
agcccaggca gacaacagtg acgttccgtc tcaaaaaaaa aaaaagaaag aaaagaaaag    26760
aggctttcct tgctggtgca gatatcacag tgatgggcaa gaggactgca ctttttgcac    26820
agaagcacac gcaccaactc taggagtccg gccagcccag cgagtcaacg caagcagata    26880
cgcccctgtg agcacatggg atgggaaact ctctctgttc ttagtttttg ggcaacagg    26940
ttttgaagac gtttttattc agaggccaaa ctggaccacg atggggacta gagcttcggg    27000
cctgtccgtg tcctagggac aggacagaca cagtcacctc ctgtctgtca gggcatgagg    27060
acgcagtcat cacctcacat tccccaaagc cctgccacac gtggacggta cgtgacttga    27120
tccatcttaa taaaattcac gaccacgaag gaggcccgtg gtggcttctc tggggaggtg    27180
actttgtcag cgaattctgt gggcctgggg gccgcttaca cacatgtgca cacgtgtccc    27240
tcggccaaaa ttcactctgc tgccatgtgc aatttgtgtt tttccagaac gtgtgtacta    27300
cttaatattt ctaattatac tttaataaat attataattg ctgataccat tagtgattac    27360
ttataatgca ataattaata gttatatttt taaataaaag cttccgaagc tgtgcacagt    27420
cctttatccg ctggagacga tcccagagag aggcctccac gttccacctg actgcggatg    27480
accttgctga tgaccttgag tgtgcacgaa tttcctccgt gctaccactc tccccaggca    27540
cacacagggt cgcaggcaga tgcctgccgg gaggtgtgtg gttttactta aaatccagag    27600
gacgtgatgt ggcaccaggc actgtggctc taccaccgtg agtgtggctc acccagtggc    27660
tgtgtgacgg cagctctccc aggccgcctg ccccatggcc accacaggcc ccccgagagg    27720
agcaaactac acggtcaacc ccgcactttc cagatgggaa atctagtcac agagagaaga    27780
gcccgcgccc tcgcgggggt cacacgacag ggacaggaat gagaatcgga taaaatcctt    27840
ttgtatcggg agagagagat acaagcgtgt gagagccggg tgtccagcgt cagtagtaac    27900
ttggagctga caagctgcag gctcaaacgc tcggcgcaca cctgacccag acacacccc    27960
cgcccctcgt cctccacgca ggagcaactc cacaccccgc ttgccatttc caggcctcgt    28020
gtggcacctg ctccgctccg gctgccgcaa gccgagccat gtttccgggg cctcgggagc    28080
ctgcagccca ggagccggag ggggcggggg ccagaaaagg agactctggt ggccccgggc    28140
agaagggcag tggggaaggg gcaggagaga ggtgagcaga agccctgcca gcccgcccag    28200
ccacccgcag ggcaatggca cctggccacc tgactcactt gcccctgatg cgcacggcgt    28260
ggtggcacat gaagcgtagg aagacgtcga agaggccact gctggcctca ttcagggagg    28320
agctctgcga aagcagacgg gagacacatg ggagtgagcc ggtgggtgct gagacaggca    28380
ggaccacgtg gccaagaccc tccgtccctt ttggggtcag tgtttgtgat gaagtgcggg    28440
gctgggctgg aatgcagggc catcgtgggc tggccgggcc gagtctctgc agacacacat    28500
gggcttaggc agagtgtgtg acacagaatg tctcctctgg gccacctgtg ggtccagctg    28560
ggctcacacc cacacagccc accgtggccc ggatggcata tgtgatccag gagttgctgc    28620
ccagctgccg ggcacagggt ctgggtccca gcagcccagg ccacctccac ccccaccctg    28680
ggagccaagg aaattcgccc agaggtctct gagcctcatg cggtggacac gactgtcccc    28740
tagagccgag caagtgctaa caggcatctg caaagagcca cagggccacg tggacagacc    28800
acatctgcaa agggccacag ggccacgtgg acagacccag cactgagagg cccgctcagc    28860
ggggcgtggc tcaaacacgg cccgtcctcc taagaagggg gacacgtttc ctttggcctc    28920
agacccagca ctgaggggcc agctgggcag gacacggctc aaacatggcc tgccctccta    28980
aaggaggggg acacctttcc tttggcctca agatcaaaac tggcagagaa gacatttagc    29040
aaatcatgaa caaaggcact gcggaactgg agagaggccg cgcagtcaaa cgtgagcagg    29100
tgcctgaatg cagcagctgt cgcagcctgg catggcccag ctcagatttc gccaaggcac    29160
acagctcatc atgcccccat cacagctcat tccccccact gccccccagg gccaacagtc    29220
```

```
tgtccggtca tgagcccagt gattgcccag gggaggaccc actgctggga gtccgtgccc     29280
aaccctgcag ggcagtgccc agacctgctc gatgacgacg gcatccctca gcgggctggt     29340
ctcctgcagg tgagccacga actgtcgcat gtacggctgg aggtctgtca aggtagagac     29400
ctgccggcag aggagagggc atgagccaca aatgtggcct gccccggcca gagctgggca     29460
cttgtttctt ccgatcagga cgtgtggacc tgcggccaag cccgatggcg ggatgaagcc     29520
cagagagcgc ctgggaagct tctgtttggg aaacgggggc cggggggccg aggacgcaaa     29580
gtagctacag acacacctgg gactccacct gcagctacca cacatcagac ccctgtgacc     29640
gatcaccatc cacagtcacc acatcagacc ccacgaccgc catccacagt caccacatca     29700
gaccctacga ccgccatcca cagtcaccac atcagacccc acgaccgcca tccacagtca     29760
ccacacatca gaccccacga ccgccatcca cagtcaccac acatcagacc ccacgaccgc     29820
catccacagt caccacacat cagaccccac gaccgccatc cacagtcacc acacatcaga     29880
ccccacgacc gccatccaca gtcaccacat cagaccccac gaccgccatc cacagtcacc     29940
acacatcaga ccccacgacc gccatccaca gtcaccacat cagaccccac gaccgccatc     30000
cacagtcacc acacatcaga ccccacgacc gccatccaca gtcaccacac atcagacccc     30060
tgtgaccgat cgccatccag tcaccacaca tcagaccccc gggaccaacc gccatccaca     30120
gtcaccacac atcagacccc acgaccgcca tccacagtca ccacacatca gacccctgtg     30180
accgatcacc atccacagtc accacacatc agaccccaca accgccatcc acagtcacca     30240
cacatcagac cccacgaccg ccatccacag tcaccacatc agaccccacg accgccatcc     30300
acagtcacca cacatcagac cccacgaccg ccatccacag tcaccacaca tcagacccca     30360
cgaccgccat ccacagtcac cacacatcag accccacgac cgccatccac agtcaccaca     30420
tcagacccca cgaccgccat ccacagtcac cacacatcag accccacgac cgccatccac     30480
agtcaccaca tcagacccca cgaccgccat ccacagtcac cacacatcag accccacgac     30540
cgccatccac agtcaccaca catcagaccc ctgtgaccga tcgccatcca cagtcaccac     30600
acatcagacc cccgggacca accgccatcc acagtcacca cacatcagac cccacgaccg     30660
ccatccacag tcaccacatc agaccccacg accgccatcc acagtcacca catcagaccc     30720
cacgaccgcc atccacagtc accacacatc agacccccgg gaccaaccgc catccacagt     30780
caccacacat cagaccccac gaccgccatc cacagtcacc acatcagacc ccacgaccgc     30840
catccacagt caccacatca gaccccacga ccgccatcca cagtcaccac acatcagacc     30900
cctgtgacca accgccatcc acagtcacca cacatcagac cccgtgacc gactgccatc     30960
cacagtcacg tggcacaagt ggcactcgag ctcccgggcc tctccagccc agtgctggag     31020
ggattgcagg gatgcaggtg cagccacagc acaggcagga gaagagtctg aagacgcggg     31080
agagagaccg gcaccccac ctcctattct gcagactccc ccaggacgct tgtcatttac     31140
tacgggacct gctaagcccc tgcgtcccca agacaggatg tgagcaggca cgtgacacac     31200
actaacacgg acacaggagg cctcgagctt gtgaggcatc aaaagacgcg cacggtgact     31260
ctgtgcttca gcatgttccc cagcccccag gagctggcgg cagggccgtg ggctaaaacc     31320
acatcgagta cgattcaacc ctgagaacca acctggaagg cagtaacagg aaggtacatg     31380
gggcctgcac ccttcccaac ccaggagcag gcaacacgcc cacaaatagc ccatgggtct     31440
gaggtcggcg gaaagcatca cagaaatccg taattattct ggactcaaca cagaaaagcc     31500
aatatatcaa cactgacgag aggctgctga agcaggtctt tagcacaggg tccccaacca     31560
ttgtggtacc agggactggt ttcgtggaag acgatttttc cacaggacag cagtggggaa     31620
ggggatggtt ttgggagcaa acggttccac ctcagatcat caggcattag attcccataa     31680
ggagcacaga atctagaccc ctctcacgca cagttctcag tagggtgtgt gcgcctctga     31740
gcaccgcatg ccactgctga tctgagaggg gcggggctca ggcgggaacg ctggctcccc     31800
acaccccacc tagcctcccg ctgtgcgccg ggttcctaac aggccccaga ccggtgccac     31860
tccacagcca ggggcctcgg gatccctgct ttagagggaa aacggcaact tcagatgctt     31920
gtttagaaaa gaaagttcaa acatccataa tcgaaacttt tccttcaagg agccggaaaa     31980
acaacaataa acaaagccta aaggaaatgg aaggaggcca tcgataaaca gcagacacca     32040
attacgcagg aaacagccgg gaaatcagca cacgtgaaac tcttctgtga agagagtcat     32100
gaaatggaaa gacccctagc gagacctgga gaaggaaaac ggaggttgtc agcaccaagg     32160
ctgcaggcag ctatcgctgc agatctcacg gaagctgcag gaagcgagtc tcggccgggc     32220
gcggcggttc acacctgtca tcccagcact ttgggaggcc gaggtgggag gatcacctga     32280
ggtcaggagt tcgagaccag cctggccaac atggagaaac ctcgtctcta ctaaaattac     32340
aaaaattagt cagcatggtg gtgggcgcct ataattccag ctacttggga ggctgaggca     32400
agagaattgc ttgaactcag gaagcacagg ttgcagtgag ccaagatcac gccaaaccac     32460
tccagcctgg gcaaaaaaga gcgagactct gttatcaaaa aaaaaaaaaa agaaagaaaa     32520
aaagaaagaa agcaagcctc caactcgcag gggaattcca aggacggagc gtagccaggg     32580
ccacatccag gccaatgaac acaaggaggc ctggaagccc tgcccaccgg ccacacacca     32640
cagcaggcac cacacgaccc cagagtggaa gaaacagaac agggagccca ggggaggcct     32700
ccacacccct gacttactat aaacccgacc atagttcaaa ggaagatatg gtctcctcaa     32760
taaagctgtt ggagcacctg cagacccact acaggaagaa agctcgcctt gaactctgtg     32820
ttaccccact cataaaaacc aatcccacag atccccacct atcccacaca tgaaaaccaa     32880
tcccacagat ctccacctac cccacacatg aaaaccaact ccacagatcc ccacctaccc     32940
cacacatgaa aaccaatccc acagatcccc acctacccca cacatgaaaa ccaatcccac     33000
```

agatccccac ctaccccaca catgaaaacc aatcccacag atctccacct accctacaca 33060
tgaaaaccaa ctccacagat ccccacctac ccccacacat aaaaaccaac tccacagatc 33120
cccacctacc ccacacatga aaaccaatcc cacagatccc cacctacccc acacatgaaa 33180
accaatccca cagatcccca cctaccccac acatgaaaac caatcccaca gatccccacc 33240
taccccacgg atgaaaacca attcacagat ccccacctac cccacacata aaaaccaatc 33300
ccacaggtcc ccacctaccc cacacataga aaccaatccc acagatcccc acctacccca 33360
cggatgaaaa ccaattcaca gatctccacc tacaccacac atgaaaacca actccacaga 33420
tccccaccta tcccacacat gaaaaccaat cccacagatc cccacctacc ccacggatga 33480
aaaccaattc acagatcccc acctacccca cgcataaaaa ccaatcccac agatccccac 33540
ctaccccacg gatgaaaacc aattcacata tccccaccta ccccacacat aaaaaccaat 33600
cccacagatc cccacctacc ccacacaaga aaaccaatcc cacagatccc cacctacccc 33660
gcacatgaaa accaatccca cctaccccac acataaaaac caatcccaca gatccccacc 33720
taccccacgg atgaaaacca attcacagat ctccacctac accacacatg aaaaccaatc 33780
ccacagatcc ccacctaccc cacacatgaa aaccaattcc accaatcccc acccaggttt 33840
gcaagctgac acagtaaaga ctgttctaaa aggaggcgca gaagcaacgt caccacaacc 33900
tcaatacctt gagttcctgc agagaacaca aacagcttta gccacaaaca gctgataaag 33960
tggactgcgt tactggactg tgttagtgga ctgcgataga agtacaatct tccagtcctc 34020
aaaaggcttg acttgagagc acaaaagcaa gacacacacg gcacagagat tcacagtgca 34080
cgcatgtgat gccacacttg cagcagaaca cacaaagagc tcctgcaagc cgatgatggc 34140
aggcaggcgc cccatacaga gcggggcagg acggacagcc acctcccgca agagccccgg 34200
ggcggccagc acgggaacac tgtgggcacg tgggcatcaa gagagccccc tgcacaatcc 34260
cacgaggcgg cttcgacacc ttcaggccag cggccaccgg aggcatcgcc ctcacaagca 34320
gccagccggg gtgtcactgt cacccccctg gaaactccca caacatccct aaagcacgtc 34380
tgtgacccag caaccccacc cctggaattc acccagaggc gagggcctct gacccctggg 34440
acactcccag aacccagcag gctcacagca ggcgcagccg tgcagtccgc acccaggagc 34500
ctccacagca gcgggaagga tacatccacg tggcgagttc cacaacggat gccattcatg 34560
gacagaacgc acagctgagg cgttcagcat agctgaggct cgaaccccaa aacccacctc 34620
ctgcctgatc tcactcacat ggagaccggg agagggagac catggaggtc gaggtcggaa 34680
cgagggtcac ggtgggggcg gtagcttccg ctgcagcggg gatgtggggg ggggtctcct 34740
gggctctggt gactttctac acccgggcac aggtgggcgg tggggacggg ggggtctcct 34800
gggctctagt gactttctac acctgggcac aggtggtggc catcagcttt ccatgagggt 34860
aaaattctgc tgagatttgc aagtagctgc gatagctcaa gaagtcagag aaagaggtcc 34920
ttgtgggttt ccacatgtgc ctctccccag accgaagctc cagggatact tcttcacctc 34980
tgcaccccgg cctggcacga ctttgagggc ttacccctcc agcgtgctca tgaccactgg 35040
gtgggaaccc aagcccaggg cccctttcac ccctgccgag ccccaaggcc cccggccgct 35100
ctgggaggag tgtgtcccgg ggagcgaggc ctgaatcagc cttcctatat ctcatggagc 35160
cggcataggc cagactgggg tcctggggggt gccaggggtc caggaaggta ccaccagcat 35220
ccctactctc ctctcaaacc tggcttacag tctccaaggc ccagccctgt gacaggacag 35280
gggcacccat gctagaagtg aggaccctga atgtggagct ccccaaacca gcacccgaga 35340
gcgtgaggac aatggtgcgg accatgcctg gaggaggaag gagtaaggcc aaggggccac 35400
acacacccat gccatagaca cgcatatgtc acggacacat gcacacacac acaccacaaa 35460
taggcacatc acagacatgc agcacacagc atgaacatgc acgtgtcaca gacacacaca 35520
catgcacacc acaggcacac acaccatgga cacacacgtg ccacacacac acacaccaca 35580
aatatgcacg ccacagatac atgcaccacg acacacacgt gccacacacg catatcacag 35640
atgtgtaaat catggaagta cctccaccac agaaacgcat cacagacacg actgcattct 35700
agacacattc atatcccaga gacacacatc ctggacacga ctatcacacg tgaaccttac 35760
gtggctcttg aaggccttgc ggacgtgccc atgggcggcc ttctggacca cggcataccg 35820
acgcacgcag tacgtgttct ggggtttgat gatgctggcg atgacctccg tgagcctgtc 35880
ctgggggatg gtgtcgtacg cgcccgtcac atccacctgt gtgagtggag gcgaggagac 35940
tgacagtggc cacgcagaaa ctcagacatc acctctgccc tcagggcctg gcctggcggt 36000
gtcccccact ctctctctga cccccaccac tccagacccc aagggcaggg cgggctgtgt 36060
cccctctctg agcctcagga caggagaccc ctggctcagg actggggtgc aaggcaccgg 36120
ggcctggtgg ctgagccgtt gcggttcctt ctctgacgga aactggaatc cagtgggacc 36180
ctccttggga ataggagccc gggcagtcag ggtgcacagt cggccccatg tgctgcagga 36240
aaggctgaag gcctccaccc taggtgccag gtgtgtcctc aacagtgaca gggtcacctg 36300
cactccctgc ggcccaccca ggagtacctc ctccacccaa catgaggtgc caatcaggca 36360
gccactccca aggtccagca gggctgctca cggggtcccc cggcacccac cttgacaaag 36420
tacagctcag gcggcgggtc ctgggcccgc acacgcagca cgaaggtgcg ccaggccctg 36480
tggatatcgt ccaggcccag cacagaggcg cccaggaggc cggggcgccg cgcccgctcg 36540
tagttgagca cgctgaacag tgccttcacc ctcgaggtga gacgctcggc ctggcgggga 36600
cagcatggga gacagtcagg aaagtggatc cggccaagtg cccaccccac catagggacc 36660
caggggagaa gcagcccctc cccagtgtcc ccagccaccc agacccggga cctagaaccc 36720
ctcccagctt cctcagaccc tgtttgaaac gggttcctgg ccgcatgtgt gttgcacacg 36780

ggatcctcat gccacacctc tgtccacctc accccacact ctcctcagat gacggggtca 36840
ccgcagccac cgcagccaca ggggtggggt gcaggagccg tggggcaagg tccaggatcc 36900
tcagagcccg gtgggctcct ggcagtcgtg gcctggtccc cgggccccgt aagcggaagc 36960
gcacggaggc tgcgcaccac agcaggacac ggatccagga cctcgggccg tgctgcatcc 37020
aggccctggc ccggctgctt cttgtggtcc tcagagcctg tccctgcccc aggagctccc 37080
cctacacccc cacgctgctt ttctggaggc accggcacct cggccacctc actgtgcgca 37140
caaatgggtt ggcgccgtgc catggccctg gctgtgtccc gtggcccctc ctccgggccc 37200
ttcatctaag ctgataccaa atgtggggct caaacgcact tctgtttaaa aaggaagtta 37260
aaccaaagca cagccaccct cttttctctg cggaacgttc tggctcccac gacgtagtcc 37320
atgttcacaa tcggccgcag cccgtcaggc ttggggatga agcggagtct ggacgtcagc 37380
agggcgggcc tggcttcccg atgctgcctg acctctgctt ccgacagctc ccgcagctgc 37440
accctcttca agtgctgtct gcaatagaga gcccctcagg aggcttgctc agccagacaa 37500
cagactaggg ggaagctcac gggaagccac aagcccccac cgactcagtg agggctcagg 37560
gcacccacgg cagcacacgc tgaaggccat gcccggggcc acgtccaccc atgccagcca 37620
gacgcctctg agagcccctc tacttgcagg gcacctggac acctggtcct atagtcaacc 37680
ctggggacgc ccagtccggc ggtcaacccc cagggtgact gtcccaccat cagcaccagg 37740
gagcctggtc ccaggctgca tctggggcca cgtgaaccca tgcccctcca cacaggatgc 37800
ctgtccctct ggtacagcca ctggggtagc ccaaggctgg ccccaggcag gacccccagg 37860
tagaaagagc cacatccctt cagatggaga gggcgggaga ggctgctcag ggcagctcag 37920
ccaaggagca gaatcagctc tcatgagccc accagtctcc tgacacctct gccctttggc 37980
cttttccccc aaaaccaccc atgggggcac gggggctgga gcggccacac ctggacctgg 38040
gcatctgtgg ctgacagcct ggcactccgt ggacactggg gccgcgggtg ctccggagct 38100
ggtgccctga ccgcaggccg caggcccagc agagtagaaa aggcacggta ccctgtccag 38160
ggaggagggg cctccaaggc cccccaggca gaacaacagt cccagtagtg acaaggagca 38220
tcttggggac caccacgtct gtctgctcct tccggtcacc ccaattaaat tctttccaaa 38280
atactggtca aatgaccatg ttgggtcgca caccattaaa tacgtgtaaa gtctaagtaa 38340
ggtgcattgt gtattaactt gttcccacag ggaaagccct gaccctcccc cagatgcaag 38400
gaaatggccc cagcaccacg tcggggtccc cagcccagag gctgtgttac ctctgacgag 38460
gtctgctgcc agctccctct gcagacatcc tgatttgggg catggggtgt cacaaggccc 38520
cccagggact cgagtgtgca ggggagctgg ggaagccctg atcttctgac taaagccctc 38580
cacagtcagc ctgggcggga cacggagaag ctaataccat ggcctgggct cagccaggct 38640
ggcatctccc agaccagcgc tcccgtccct cactgatgca aatccagctt gaatctgtca 38700
caatctcaca ctctcatcag ccgaacattg cagacaaaat ctcacagcca ttcaaatgca 38760
gccacctaga agttagctca tggtggaact ctcctatccc caggtgctcc ctgcacaggt 38820
gctccctgca gtgagtcgcc aagtgggtgg gattttatcc tctcatgacc taaatccaat 38880
agattgaaga ttcatacaga acagcatgaa ttaaaagaga atcgggggct gggcacggtg 38940
gcttagcctg taaacccagc actttgggag gccaaggtgg gtggatcacc tgaggtcatg 39000
agttcgagac cagcctggcc aacatggtga aaccccgtct ctcctaaaag tacaaaaatt 39060
agccaggcgt ggtggcaggt gcctgtaatc ccagctactt aggaggctga ggctggtgaa 39120
tcgcttaaac ccaggaggtg gaggttgcag tgagccaaga ttatgccact gcactccagc 39180
ctgggcaaca gagtgagact ccatctcaaa aaagaaaaaa aaaagagaga gagaatcaga 39240
cccacatccc agggaaaggc aaggaggcta gtggtagtgt ttccatctaa tttaaatccc 39300
ttctgcttta caaaccctag agaccacttg gcacaaactc ctgattttac aagtaagaga 39360
acgtgagctc caggcagtca gagccttgca cagaatccac ttggaccagg cctgtgacgg 39420
ggcccctggc tcccagccca gagctgcaca gccagggaag cacaggcaag tggagacagg 39480
cgcatgctga ggccgggctg gtgttccagg acttcgagaa gcagaggcct ggcgtgggga 39540
tacagtacct gattccaatg ctttgcaact tgctccagac actcttccgg tagaaaaaga 39600
gcctgttctt ttgaaacgtg gtctccgtga cataaaagaa agacctgagc agctcgacga 39660
cgtacacact catcagccag tgcaggaact tggccaggat ctcctcacgc agacggtgct 39720
ctgcggccgg aacacagcca accccttaaa cgagaaggac atgccacatc cagatcaccg 39780
agggcctggt gacctcaccc cggacctgca ccatccggac accgcacatc cagctcacca 39840
agggcctggc gacctcaccc cagacctgca ccatccggac acggcacatc cagcccacca 39900
agggcctggc gacctcaccc tggacctgca ccattcggac acggggacac cgcatatcca 39960
gctcaccgag ggcctggcga actcaccccg gacctgcagc atccagacac cacacatcca 40020
gctcaccgca gggcctggcg aactcacccc ggacctgcac catccagaca acacacatac 40080
agcccaccgc agggcctggc gacctcactc cggacctgca ccatccgtac actgcacatc 40140
cagctcaccg cagggcctgg cgacctcacg ccagacctgc accatccgga tacagcacat 40200
ccagctcacc gcagggcctg gcgacctcac cccggacctg caccatccgg atacagcaca 40260
tccagctcac agcagggcct ggcgacctca ccccagacct gcaccatccg gacaccacac 40320
atccagctaa ccgcagggcc tggtgacctc accccggacc tgcaccatcc gacaccgcat 40380
atccagctaa ccgcagggcc tggcgacctc acgccagacc tgcaccatcc ggatacagca 40440
catccagctc actgcagggc ctggcgacct caccccggac ctgcaccatc tggatacagc 40500
acatccagct cacagcaggg cctggcgacc tcaccccgga cctgcaccat ccagacaccg 40560

cacatccagc taaccgcagg gcctggtgac ctcaccccgg acctgcacca tccggacacc 40620
gcatatccag ctaaccgcag ggcctggcga cctcaccccg gacctgcacc atccggacac 40680
cccacatcca gctcaccgaa ggccttggct acctcacccc ggacctgcac catccagaca 40740
ccgcacatcc agctcacagc agggcctggc gacctcacgc cggacctgca ccatccggac 40800
accacacatc cagcccacca agggcctggc gacctcaccc cagacatgca ccatgcagac 40860
accgcacatc cagctcacca aaggcctggc gacctcaccc cagacctgca ccatccggac 40920
acggcacatc cagcccacca agggcctagc gacctcaccc tggacctgca ccatccggac 40980
acggggacac cgcacatcca gctcaccgag ggcctggcga actcaccccg gacctgcagc 41040
aaccggacac tgcacaacca gctcaccgca gggcctggtg acctcacccc agacctgcac 41100
catccggata cagcacatcc agctcacagc agggcctggc gacctcaccc cggacctgca 41160
ccatccggat acagcacatc cagctcacag cagggcctgg caacctcacc ccggacctgc 41220
atcatccgga ctccatacct ccagctcacc gagggcctgg cgaccacacc ccagacctgc 41280
accatccaga cgccgcacat ccagctcaca gcagggcctg gcgacctcac cccggacctg 41340
catcatccgg actccatacc tccagctcac cgagggcctg gcgaccacac cccagacctg 41400
caccatccag acgccgcaca tccagctcac agcagggcct ggcgacctca ccccggacct 41460
gcatcatccg gactccatac ctccagctca ccgagggcct ggagacctca cccctgacct 41520
gcaccatccg gacaccgcat atccagctca cagcagggtc tggcgacctc accccggacc 41580
ggcaccatcc ggactccata catccagctc accgagggcc tggcgacctc actccagacc 41640
tgcaccatcc agacaccgca catccagctc accgcagggt ctggcgacct caccccggac 41700
ctgcaccatc tggacactgc acatccagct caccgagggc ctggcgacct cactccagac 41760
ctgcaccatc cagacaccac acatccagct cacagcaggg cctggcgacc tcaccccaga 41820
cctgcaccat ccagacacca cacatccagc tcacagcagg gcctggtgac ctcacaccgg 41880
acctgcagca tccggacacc ccacatccaa ctcacagcag ggcctggcaa cctcacccca 41940
gacctgcacc atccggacac cgcatatcca gctcaccgca cggtctggca ccgtctggcg 42000
acctcactcc agacctgcac catccagaca ccgcacaccc agctcaccgc agggcctggt 42060
gacctcaccc cagacctgca ccatccagac accacacatc cagctcacag cagggcctgg 42120
cgacctcacc ccggacctgc atcatccgga ctccatacct ccagctcacc gagggcctgg 42180
agacctcacc cctgacctgc accatccaga caccacacat ccagctcaca gcagggcctg 42240
gcgacctcac cccggacctg catcatccgg actccatacc tccagctcac cgagggcctg 42300
gagacctcac ccctgacctg caccatccag acaccacaca tccagctcac agcagggcct 42360
ggcgacctca tcccagacgt gcaccatccg gacactgcac atccagctca ctgagggcct 42420
ggcgacctca tcccggacct gcaccatctg gtacaacagc catggccatg ggaagcccag 42480
caggtccagg ccgagatggc cacgcacggg acctacacac gggatcccgc agacaaagct 42540
caaaaaatgt caaatgtcct gtgagtgatg ctcccaccaa taacacccta aaatgatgag 42600
gtgtggacat gctgggcttc agtaaaatct attattacat taatttcacc ttttcttttt 42660
tacttttttta atgtggctac tagaattttt ttttttttt tttttttga gacagagtct 42720
cgctgtgttg ccaggctgga gtgcagtggt gcaatgtcgg ctcactgcaa cttccaactc 42780
ccaggttcaa gcaattctcc tgcctcagcc tcccgagtag ctgggattac aggcacccgc 42840
caccacgccc agctaatttt tgtgttttta gtacagatgg ggtttcatca tgttggccag 42900
gatggtctcg acctcctgac ctcgtgatcc acccgccttg gcctcccaaa gtgctgggat 42960
tacaggcgtg agccaccgcg cccagcctag aaattttaaa attatccaca tggctcacgt 43020
catgctcctg ttgggcgaca acggctcgga gcctcatcct ttgtcctggt tcccaagcag 43080
aagggaggaa gcagacactc ccccgccagg cagcaccgtc agagctggcg ccacaccgca 43140
ggtttgcgcg atttcaaact cgacaccgcg gagccaccag accccgacat gcctggggtt 43200
ttccaggctg aacccaggcc gagcggacgt tgctgtcact cactggctaa ggaacgctgg 43260
ccacgtggtg ctccagacac tcacgggcca agatgaccgc cctcctcgtg agtctccaca 43320
tcttcatctg tgcatcataa gcagaggtcc ccggacgtcg ctagatgcca tggaggccag 43380
cacaggtaaa gctggggttt accagcaata acaagacaga agaaccacgc aaaggacaag 43440
gaaggggacc ccagacggcg ggcctgagac agaagacaga cggggaacaa aggaggaaaa 43500
gcagggcggg ggcaaagcta cagaaacact caacacggaa aacaatatta ataatgctta 43560
gcttgtgggg gtgtcaagat gcctgagata gaactaagtc agcagggaaa acagcacaca 43620
ggctgggggg tggccagagc taaaatacac taagggtctt attgctcagg acaagggtag 43680
caatgtttca gacttcattt taaaagatca aggtatgccg ggcacggtgg cttgcacctg 43740
taatcccagc actgtgggag gccaaggcgg gcagatcact tgaggtcagg agtttgagac 43800
cagcctggcc aacatggtga aagcctatct ctactaaaaa tacaaaaatt agccaggcgt 43860
ggtggtgggc gcctgtaatc ccagctactc atgaggttga ggaatgagaa ctgcttgaac 43920
cgaggaggca gaggttgcag tgagccgaga ttgtgccatt acactccagc ctgggtgaca 43980
gagtgagact gtctcaataa acaaaagcaa acaacgacaa taaaaagatc aggggtaac 44040
actaagcaaa gagaaataaa agcaaatgcc cagtgaccag gaggcaacga gaggatcaac 44100
acacactcgg caggaaacgc acatggcaac ccaagaggtg gtgagaaaca agggaacgag 44160
gacatgcaac aggcaagtgg agaatcagag tgcaccaggc gggtctctag tgacaaaaaa 44220
atgaaaataa atcaggttat ccagttaaac aacgactgtc agactggatt ggaaattcac 44280
ctacatgctg ttaacaggac atacccacac tataagaata taaaagtgtt gaaaattagg 44340

```
ccagacatgg tggctcatgc ctgtaattca gcactttggg aagccgaggt agatggatcc 44400
cttgagtcca agagtttgag accagcctgg gcaacacagc aagaccctgt ctccagaaaa 44460
aatttaaaaa ttagccaggt gtagcggtgc gtgtctgcag ttccagctat tcaggaggct 44520
gaggtgagag gacggctcag gccagaaggt aaaagttaca ctgacctgtg atcacaccac 44580
tgcactcacc ctgggcgaca gaccaagact ctgtctcaaa aaaaaaaaat atatatatat 44640
atatataaat taaaggcaga aaacagacac agcaggaaaa tactaatcaa aagaagccca 44700
gtgtggcagt actaagagca atttaaaaaa agaattacta gattactaga gacatagaag 44760
tctccaaaag attcactcat caggaagata aaaattctac cacgtgcatg cacctaacaa 44820
tgcctcacat aaatgctacc aaacgagaag aaatgaacag acccatcccc caggtgaggg 44880
actatggcct ccttcttgga atttcatgca tctagaagga gacagaagct ttgcactgga 44940
ccttaataag ctggatatag tgaacatgca tgcccactat tgacaggatg tcaaatttac 45000
aatggacagt tctggaaacc atgcacacac tagatcacaa agcaaggctc cgcaaacttc 45060
aaataattgg tattatacag accactacac aactgagtta gaaatcaata ataaaatgat 45120
aacttaaaat actctacgta tcttgatact ctacatatct tgaacataaa cagtgttcaa 45180
ataactcact gctcaaagaa atcacagaat aagctaaaga atacttaaaa gtcactgata 45240
tcaaaatgat ttcacgtcaa aacacagcgt gcacaaagaa aaaaatcaca gaataagaat 45300
acttaaaaga agtattaaca ctttgaagta ttttaagaac acttgaaagt ggctgatgtt 45360
gagattactt cacatcaaaa cacagggtgc aggtaaggca gcacttagag ggaaaggcat 45420
gactaaacta agaagctaga aaaggaatga aagaataaac caagaaaaag catacaatgg 45480
agagatagaa acgtcagagg taaatcagtg aaattcaaaa ctaagaccca agagggaagt 45540
ctgacgaagg ctggcggaga caccggccct ccacgtgggt ctcagagcag gagacagaca 45600
gagacactcc aacctgtggc tggacgtcaa tccatgtgag gggcgactg gaggcagagc 45660
ccagcctaag caatgagcgg caggtgccca gaataaggtg acaagcgttg ccacccaccc 45720
aaggggggcca agcagagggg cagcttggga gaaaacagga caagtgaggg ggctggggtg 45780
acttgctttc cttcagggca cagagaaccc tttctgggat gtctccagca acacgtggca 45840
gcagacacag gcagcccaga gtccagcctc ggcatgagac aagctgggag aggagtattg 45900
gcagcatgca tgtggcgggc acgtggtgag caagccaggc ccaacagagg caagcggacc 45960
tgaagctgtg gctgcagtgc ctggcacgcg ggaggcgaga gcctgcagtc ccgtgtcccc 46020
gtagcaaaat ggaacggaga ggtgaccaag aagagccgag catcagaaaa gataggcttg 46080
gggaccagca ctgcgcctgc accatctacc caggcaatgg gcaaccggcg cagctgtggc 46140
tatagaaaga gcaaacattc aggagcaagc tcaagtgagc aaacgccaag gacacctggg 46200
gacagagcct cactcaccct acacgagaca gggacaccca gggacagcgc ctcactcacc 46260
ctgcacctga gagggacacc cgggggccgc aactcactca ccctacacgt gacagggaca 46320
cccggggacg gcgcctcact caccctacac gtgacaggga cacccgggga tggcgcctca 46380
ctcaccctac acctgagagg gacacccagg gacggcgcct cactcaccct acacgtgaca 46440
gagacacccg gggacagtgc ctcactcacc ctacacgtga cagggacacc cggggacggg 46500
gcctcactca ccctgcacgt gacagggaca cccggggacg gcgcctcact caccctacac 46560
gtgacaggga cacccgggga cggcgcctca ctcaccctac acgtgacagg gacacccggg 46620
gacggcgcct cactcaccct acacgtgaca gggacacccg gggacggcgc ctcactcacc 46680
ctacacgtga cagggacacc cggggacggc gcctcactca ccctgcacgt gacagggaca 46740
cgcggggacg gcgcctcact caccctgcac gtgacaggga cacccggggg cctcgcgtca 46800
atcaccctgc acgtgacagg gacacccggg gacggcgcct cactcaccct gcacgtgaca 46860
gggacacccg gggacagtgc ctcattcacc ctacacgtga cagggacacc cggggaccgc 46920
gcctcactca ccctgaacgt gacagggaca cccggggaca gtgcctcact caccctgcaa 46980
gtgacaggga cacccggggg ccgcgcctca ctcaccctgc acgtgacagg gacacccggg 47040
ggccgtgcct cactcaccct gcacgtgaca gggacacccg gggccgcgc ctcactcacc 47100
ctgcacgtga cagggacacc cgggggccgc gcctcactca ccctgcacgt gacagggaca 47160
cccggggggcc gcgcctcact caccctacac gtgacaggga cacccggggg ccgcgcctca 47220
ctcaccctgc acgtgacagg gacacccggg ggccgcgcct cactcaccct acacgtgaca 47280
gggacacccg gggacagtgc ctcactcacc ctacacgtga cagtgacacc cggggaccgc 47340
gcctcactca ccctacacgt gacagggaca cccgggggcc gtgcctcact caccctgaac 47400
gtgacaggga cacccgggga cggcgcctca ctcaccctac acgtgacagg gacacccggg 47460
gacggcacct cactcaccct acacgtgaca gggacacccg gggacggcgc ctcactcacc 47520
ctgcacgtga cagggacacg cggggacggc gcctcactca ccctgcacgt gacagggaca 47580
cccgggggcc tcgcgtcaat caccctgcac gtgacaggga cacccgggga cagcgcctca 47640
ctcaccctgc acgtgacagg gacacccggg gacggtgcct cactcaccct acacgtgaca 47700
gggacacccg gggaccgcgc ctcactcacc ctgaacgtga cagggacacc cggggacagt 47760
gcctcactca ccctacacgg gacagggaca cccggggacc gtgcctcact caccctgcac 47820
gtgacaggga cacccgggga ccgcgcctca ctcaccctgc acgtgacagg gacacccggg 47880
gaccgcgcct cactcaccct gcacgtgaca gggacacccg gggaccgcgc ctcactcacc 47940
ctgcacgtga cagggacacc cgggggccgc gcctcactca ccctgcacgt gacagggaca 48000
cccgggggcc gcgcctcact caccctgcac gtgacaggga cacccggggg ccgtgcctca 48060
ctcaccctgc acgtgacagg gacacccggg gacggcgcct cactcaccct gcacgggaca 48120
```

gggacacctg gggaccgcgc ctcactcacc ctgcacggga cagggacacc cggggacagt 48180
gcctcactca ccctacacgt gacagggaca cctggggacc gcgcctcact caccctgcac 48240
gtgacaggga cacccgggga cagtgcctca ctcaccctat acctgggagg gacacccagg 48300
gacggtgcct cactcaccct acacgtgaca gggacacctg gggccgcgcc tcactcaccc 48360
tacacctgag agggacaccc ggggacagcg cctcactcac cctacacctg ggagggacac 48420
ccagggacgg tgcctcactc accctacacg tgacagggac acccggggac cgcgcctcac 48480
tcaccctgca cgtgacaggg acacccgggg acagcgcctc actcaccctg cacgggacag 48540
ggacacccgg ggaccacgcc tcactcaccc tacacgtgac agggacaccc ggggacggcg 48600
cctcactcac cctacacctg agagggacac ccggggacag tgcctcactc accctacacg 48660
tgacagggac acccggggac cgcgcctcac tcaccctgca cgtgacaggg acacccgggg 48720
acagtgcctc actcaccctg cacgggacag ggacacccgg gggccacacc tcaatcacgc 48780
tgcacgggac agggacaccc gggggccgcg cctcactcac cctacacgtg acagggacac 48840
ctgggggcaa cgcctcactc accctgcacg tgacagggac acccggggac cacgcctcac 48900
tccctgcata agccagggc agattgtgac ctcatctgaa gtcagagaac agcaatgaca 48960
ggcagagtcc tgatcagaga actcaaactc tcctcaacga aggaagctgg agcacaaaaa 49020
gcaaaactgg gttgcatgac gcttatctga ctcggcgtgg tccacctgag ccgcagcagg 49080
tgtgaggcag ctgccgttcg atgggtaggg acttccagtc acgcaagacg cagcatttca 49140
agcaacctgc tgtaaacacc gccgagttag caattctgca ctgtacacag aaaacggtgt 49200
taggagtgcc aatctcatgt tatatgactt ttgccaccat aaaaagaaaa aaagaaaaaa 49260
aagagcccca agaaggtcac cctccttgtc tgcatggccg gaagtcttac atgtcttggg 49320
agtttgtggg gaggggtga aatcgggact tcttctagct gccacggtag ggcctgggag 49380
cactgggagc caaaagggg ctggagcgga ggttcctcaa catcaaatcc agaaaaacag 49440
ggtggggaca cggcagggcc cagcagcacc atcccctgaa cacccacaaa cactgtccct 49500
tcctcagcag gtggagccat ctgctgtcct ctgctcccat gtggccctct tcatacctaa 49560
agatgggacc aggatctgtg ctggagaaca gtcttatctc cctccctcta ccctgtcctg 49620
gcacaatcaa cgaacacttt tttttttaa agacagagtt tcactcttgt cgcccaggct 49680
ggagtacaat ggcacaatct cagctcactg cagcctccgc ctcctgggtt caagtgattc 49740
tcctgcctca gcctcccaag tagctgggat tacaggcaca caccaccatg cccagctaat 49800
ttttgtattt ttagtacaga tagggtttca ccatgttggt caggctggtc tcaaactcct 49860
gacctcaggt gatccacctg cctcagcttc ccaaagtgct gggattaccg gcgtgagcca 49920
ccgcacctgg ccgtcaacac acaattaaat cttaaacaca aacctgcata ttggctgacc 49980
acgtgcacct gcaaaaccct tacctcccac ccccaggaag aggggggttct cgtccccacc 50040
tctcattccc acccttgaaa ttgcgaagag gattataggt aacctgcagg caccctcgcc 50100
agagcgtctg tgcttccaga cacttctccc cattgccggc aacccggctc cactgccgcg 50160
cccagcctcc tctgttcact gctctggcct cggcgcctgg aaaccgcgtg tccatcaaaa 50220
cgtgaaggtg aacctcgtaa gtttatgcaa actggacagg agggagagca gaggcagaga 50280
tcaccgtgtc cactcgacgt cctgagcgaa aagccacgtg tgcccacgtg acgatggaga 50340
caggaggacc agggctctgc ctgcccccctt ttctgagccc ctactgcatt cagctctggg 50400
gcctgggccc tcgacggcca ccacctcctc acctgggctc ctgcgcagcc aagcgcagtc 50460
ccgcacgctc atcttccacg tcagctcctg cagcgagagc ttggcatgct tccccaggga 50520
gatgaacttc ttggtgttcc tgaggaagcg gcgttcgttg tgcctggagc cccagaggcc 50580
tggggcacc agccggcgca ggcaggcccg cacgaagccg tacacctgcc aggggctgct 50640
gtgctggcgg agcagctgca ccaggcgacg gggtctgtg tcctcctcct cgggggccgc 50700
cacagagccc tggggcttct cccgggcaca gacaccggct gctggggtga ccgcagctcg 50760
cagcgggcag tgcgtcttga ggagcacccc gtagggcac tgcgcgtggt tcccaagcag 50820
ctccagaaac aggggccgca tttgccagta gcgctggggc aggcggggca acctgcgggg 50880
agtccctggc atccagggcc tggaacccag aaagatggtc tccacgagcc tccgagcgcc 50940
agtcaggctg ggcctcagag agctgagtag gaaggagggc cgcagctgct ccttgtcgcc 51000
tgaggagtag aggaagtgct tggtctcggc gtacaccggg ggacaaggcg tgtcccaggg 51060
acgtggtggc cgcgatgtgg atgggggcc cgcgtggtgc tggcggccca cggatgggtg 51120
ggagtggcgc gtgccagaga gcgcaccctc caaagaggtg gcttcttcgg cgggtctggc 51180
aggtgacacc acacagaaac cacggtcact cggtccacgc gtcctgcccg ggtgggccca 51240
ggacccctgc ccaacgggcg tccgctccgg ctcaggggca gcgccacgcc tgggcctctt 51300
gggcaacggc agacttcggc tggcactgcc cccgcgcctc ctcgcacccg gggctggcag 51360
gcccagggg accccggcct ccctgacgct atggttccag gcccgttcgc atcccagacg 51420
ccttcggggt ccactagcgt gtggcggggg ccgggcctga gtggcagcgc cgagctggta 51480
cagcggcggc ccgcacacct ggtaggcgca gctgggagcc accagcacaa agagcgcgca 51540
gcgtgccagc aggtgaacca gcacgtcgtc gcccacgcgg cgcagcagca gcccccacgc 51600
cccgctcccc cgcagtgcgt cggtcaccgt gttgggcagg tagctgcgca cgctggtggt 51660
gaaggcctcg gggggcccc cgcgggcccc gtccagcagc gcgaagccga aggccagcac 51720
gttcttcgcg ccgcgctcgc acagcctctg cagcactcgg gccaccagct ccttcaggca 51780
ggacacctgc gggggaagcg ccctgagtcg cctgcgctgc tctccgcatg tcgctggttc 51840
cccccggccg ccctcaaccc cagccggacg ccgaccccgg ggaggcccac ctggcggaag 51900

gagggggcgg cgggggggcgg ccgtgcgtcc cagggcacgc acaccaggca ctgggccacc 51960
agcgcgcgga aagccgccgg gtccccgcgc tgcaccagcc gccagccctg gggccccagg 52020
cgccgcacga acgtggccag cggcagcacc tcgcggtagt ggctgcgcag cagggagcgc 52080
acggctcggc agcggggagc gcgcggcatc gcgggggtgg ccggggccag ggcttcccac 52140
gtgcgcagca ggacgcagcg ctgcctgaaa ctcgcgccgc gaggagaggg cggggccgcg 52200
gaaaggaagg ggaggggctg ggagggcccg gagggggctg ggccggggac ccgggagggg 52260
tcgggacggg gcggggtccg cgcggaggag gcggagctgg aaggtgaagg ggcaggacgg 52320
gtgcccgggt ccccagtccc tccgccacgt gggaagcgcg gtcctgggcg tctgtgcccg 52380
cgaatccact gggagcccgg cctggccccg acagcgcagc tgctccgggc ggacccgggg 52440
gtctgggccg cgcttccccg cccgcgcgcc gctcgcgctc ccagggtgca gggacgccag 52500
cgagggcccc agcggagaga ggtcgaatcg gcctaggctg tggggtaacc cgagggaggg 52560
gccatgatgt ggaggccctg ggaacaggtg cgtgcggcga ccctttggcc gctggcctga 52620
tccggagacc cagggctgcc tccaggtccg gacgcggggc gtcgggctcc gggcaccacg 52680
aatgccggac gtgaagggga ggacggaggc gcgtagacgc ggctggggac gaacccgagg 52740
acgcattgct ccctggacgg gcacgcggga cctcccggag tgcctccctg caacacttcc 52800
ccgcgacttg ggctccttga cacaggcccg tcatttctct ttgcaggttc tcaggcggcg 52860
aggggtcccc accatgagca aaccacccca aatctgttaa tcacccaccg gggcggtccc 52920
gtcgagaaag ggtgggaaat ggagccaggc gctcctgctg gccgcgcacc gggcgcctca 52980
caccagccac aacggccttg accctgggcc ccggcactct gtctggcaga tgaggccaac 53040
atctggtcac atcccgcccg cacagggtgg agggcaacct cggggtccag gcacctggct 53100
ccaagcctcg gactgcagag ctaggaggcc cgacttccag cccagcagta gaagccacac 53160
ggccactggt cccctccaga cctgggggccc cggcacaacc gcaggacagc tgaggacttc 53220
ccaggaatcc agactccggg ttgctcaagt ttggatctaa ggggcgagaa acttctgggt 53280
ctcccgaggc cttgcaggga tgctgtagct gaggtcggca aacactgaaa tgctaacaaa 53340
cgcaacctta aatgtaacct ttcctacttt cagaaactgc cggaggaaat tgctttattt 53400
atggagctag catttgaaca ggcctcgcac cctccctggg ctgtcacgct cgctggaggt 53460
tagcctcgtc ttgtaaatac ttaggattac aggtcgctct tctagaaatc cccttagtga 53520
tccctaagcc tttttaaagg gctgtgtttg tgaattgtct ctgccactag ggcaaagggg 53580
cggtttggaa aatttgttcc aacaaaagtt aagttgtagc ttacactggt tctctgcaga 53640
gaagccaaca tagaaaacac aattttaaaa gagggaagag aagaaatgga agcagaagat 53700
tatgctggag taattaacac catgtgcatg gcgaggaaac gcctcccggc attcaatgaa 53760
gatcgctgat acccagaaga caccccagta ttatgggtgc agttagtgtg tctttgaaaa 53820
gctgatgatg tcttagtcat cacagtgtaa aacatcaaga gtgttctaac aacaataaaa 53880
aaattctatc attggcttaa aacaccacaa cacttgagtg gggtgagctt cctacctcag 53940
acccagatgt ttctaaacag agtaaattct gagctgggca tggtggctca cacctgtaat 54000
cccagcactt taggaggcag aggcaggtgg atcatctgag gtcagaagtt cgagaccagc 54060
ctggccaaca tggtgaaccc cacccccacc cccgtctcta ctaaaaatac aaaaaattag 54120
ctgggcatgg tggcgggtgc ctgtaatccc agccaaatgg gaggcggaag caggagaatc 54180
acttgaacct gggaggcaga ggctgcagta agccaagatc gcgccattgc actccctcca 54240
gcctgagcaa caagagtgaa actgcctctc aaaacaaaac aaacaaacca acaaatgaaa 54300
cagtggattc agtaccagca gtgaaaaata acaaagtatc attccttcct ccacaggagt 54360
gagaggaact cctctgcccc tggggggggga tagggaaggg gctcaatccc agtagagtag 54420
gagggatgga ctttaatttc tacatgtttc agcacacact gagacattga gattcaggac 54480
agtgtccaca ctcgcgccct gattctacat caggtttcta ctggcagatg gcaaccccac 54540
tgggacctga agcctgcagc ctcccagctg cccctgcagt gggtcccatg ggataacagg 54600
tggtcacaga aactgtgaat atgtcaagag acggtgtatc cccagtctac gaagaattac 54660
agaaaatatc aaaggagggt aactcctgag taaaggggaa attgaactcc atgaactcct 54720
taccagtggg tgtgacttga gcccagggaa gttttaaaat ggttaaatag gccaggtgca 54780
gtggctcatg cctgtaatcc cagcactttg ggaggctgag gtgggcggat cacctgaggt 54840
caggattttg agatcagcct gaccaacatg gtgaaagccc atctctacta aaaatacaaa 54900
attagccggg tgtggtggtg catgcctgaa atcccagcta cttgggaggc tgaggcacga 54960
gaattgcttg aacccaggag acggaggttg cagtgagcca agatcatgcc gctgcactcc 55020
agcctgggca acaagagcat aactctgtct cagaaagtaa gtaaataaaa taaaatagct 55080
aaataaaatc actcgtgcct gccctgccct tgcccggagg cacttcagcc ctgtccttgg 55140
atgtttctgt aaacctttgt tttgaaataa tcctagattc acagtgcatg ggtttcctgt 55200
tgcttctgca aatccccaca cacttcatgc tagaagggca gcgcgagtgc cctcctatgg 55260
cacggaggcc ggaggtccac gatgggccct ggtgggctaa gggccagccg tgccttcctg 55320
ctgcaggctc cgtggagggt cccttccccg ctttttccag cttctggagg cgcttgcagg 55380
cctgggctcg aggccctccc tccctttca gcctcagact gactgcctcc atcgtcctga 55440
ggctgccccc ctctgaccct cctgcccgcc tcttcccaga acccctggat tacttttcgg 55500
gcctgcaggt agtcccgaag gatcccagac ctcagggtcc tcacggagat cacacctgca 55560
aaggccctgg ggccgggcgg gttctgtcca caggtttggg ggaatcagga cgtgggcatc 55620
tctagagcat tatcctgccc aggacactcg ggcatggcaa agatggcaca gcctccgggt 55680

tcccccatcc ccactcccct atggttagtc tcccataact gagccaatac catcagaacc 55740
aggaaactgg ctttagtaaa aggtgcgtgc agtagccccc agctgccgcc cccaccgtcc 55800
ttaaccccccc taaccactcc cctgccatgg ggtgaaattc tttctttttg agcatgttgc 55860
gtacgtatcc catggaggtg gcagttttga ctcagcgtaa ttcccttgag atgcctgcag 55920
gctttataaa cggcatgttc ctcttcactg tgaggaactg tccaccgtgc ggatggaccc 55980
gtttgtttaa ccagtttgtt aaaggacatt ttagcaggga tttccctgct ttgtagctat 56040
taaaataaaa cagcggggt cggtcgtgga cgggagtgtg tacatgatgt gcgtagtcac 56100
gtgaattccc agggctgtgg ggcaaatgtc caggggtgcc aatgctgggt cttttgggaa 56160
gaggatgctt agtgcttgtg gaaactgcca ctctgtgttc cagagggagg ggggcagaaa 56220
agtgctgggc agagaagggc gggtccctgg cgggggctcc atccccaggc ctgtgcccgt 56280
ggacctaggt gaggacaggc actcctactt tcacacccaa atgttgcatt tcccaagagc 56340
gccctggccc accacgcccc cgtcctatgc ctataaaaac ccccagaccc tagcgggcag 56400
agacacacgc agctggacgt ggaaaggagc acagaaacag aagaacacac cggcaggccc 56460
cggcagatga caggagacgg ggagcgcctg gaaatggagg tcagcgtgca gccggacgcc 56520
aggaacctgc atcgggggcc acgagccagg ttcaggctca gaggagccag tctgggaggc 56580
gcagaatctc ctcccttcaa cgcatgtgag tgttggggaa ggagacagac accccatgtg 56640
aggaggaacc gtgcgagcta cgtcgctgag acaagacaca gtgggacagg tgagacagta 56700
gcgctggaac acggcccacc cagggtttag gaagctgctg gagaagctga ggcctggagg 56760
tggagggtgt ggagacaggg ctggcctcag tgtggcaccc caggctgagt ctccagggca 56820
gatggcgccc tcgggaagca gcaaccaggc cctagaatct gggggagtgc ctgccagggt 56880
ctactagtct agaggaacag actgttcagc cgctgttgtc accccagcct ggtggctgaa 56940
agcagcctca tctctcctgg actcttaata tatcaggggt gtgggccgtg ctctgtgggc 57000
ctagtaaaag agtggtgttt gtactgagtg ttgtcatggc ttgtcacacc cgaagaagtt 57060
gctggaatca ttcaatcctt gggggtgaag atgaataatg atggaaggaa cggtccccat 57120
cagcagagtc gtgtaattta catggccatt tcctgcacac cacttcatta cccttgaccc 57180
acccagggaa gcaggcaggc aagggtcatg ttcgtttcca cgggttcaga gctccgtgac 57240
ttcctgggtt agtgtgggtg ggcccaggaa gccggtatgg ggtcctgagt cacacgcagc 57300
gtggccgcct tcgcgttgcc caatgtggca gctcccaggg aggacggtca gtggtcctgc 57360
tgtcagcctc agcctcccag gccggggact ctctccccgg tggccttgcc gtgctcatag 57420
ccctgccgcc cagtcctctc ctcgtgtact ttcccttgca ccctaagcca ccttatgaaa 57480
accaataaaa agtacttagg gcgaaaaatc cctctgaaca tggttattaa gtccttggac 57540
cttggcaaac atagctcagt tcctgtttta cgcctttgga tttctgcact cctgaatgtt 57600
atttgatgaa atttaagaaa atattaagat aaaatccaaa tccattcgga tgtggaaagg 57660
ttgaatttac tcatggaatt aagtctttga atcccgccgt cgctgtccgc ccccagccac 57720
atcccagttt gttattaagc caggctcaga ctcctctctc cagggcgggg gtctgtggag 57780
ccctttgcaa gagggtcctc agcacgggct cggagaagct ccacgggggt cactctgtcc 57840
cgatcagaaa ccgcgagggc aagagaggga gagaggaaag aggtaaatga ccgcagagcg 57900
ggcgtctggt tttcccttga gaagggtgct tccatgactt tctgttttaa agaaggcacc 57960
taactcaagc ttccctcaga atacaccttg gtcactgctt caggtgaaaa aaagatttaa 58020
gccatacttc gaggtgcctt gactcctctc cctctggcac acgcccgggt gggcgccata 58080
gctgctggcc tgggtttgag caggaggctt ctgactcctt gcattcccgc tggccgcctt 58140
ttccagatgg tatccagagc aatctttgca aagcgtaaat aagggcactt catgcgctgc 58200
tcaccaacga ggcaggcttc tcatcgcccc ttcccatccg agctttcctg tcctcgggtc 58260
atcaggcctc tgccttgcac ctgggtcttg gccagagacc cttgaagatt gaagtggctc 58320
aggtccacag agccacctgc ttcctgccag gtactctaat tcttctggag gaaatcacca 58380
ccatttttcg tttcaggatt catacagatt aagtttaacc aaacacgaac acacaatcga 58440
aaatctccaa gcacacaaga aaaaaagcac cgtgaatgag aggacggtgt gatcagacgt 58500
gccaattctg cagctcgatt ttagagatat ctgtaattat tggtaattaa tagagattac 58560
ctttcaccat tattagcagt tgttttaggg cttatagaat gcctctttaa cttctcccag 58620
cctgtctttg gttaaaatta agtcacttac agagagtatg agcgtctaac aacagtaact 58680
tccactgttc cctcctcaca tttctgccat cgtggccata acttttactt cttcagaagc 58740
tagaaacccc acatacattg ttcttatttt agattaagtg gttaattacc ttttataatt 58800
gttttaattt ttaacttcta atatagtaga gtatatattt atacggtaca taaaaattac 58860
aattttaatc ttcttttttt tgtttttgtt ttgagatgga gtcttgctct gtcacccagg 58920
ctgcagtgca gtggcatgat ctcggctcac tgcaacctcc gcctcccagg ttcaagcaat 58980
tctcctgtct cagcctcctg agtagttggg actacaggta tgtgccacca cgcctggcta 59040
atttttgtat ctttagtaga gacagggttt catcatggtg gccagactgg tttcaaactc 59100
ctgacctcag gtgatccacc gcctcggcct cccaaagtgc tgggattatg gacatgagcc 59160
accgtgcctg gccattaaaa ttttaatttt taatatagta gggtttatat ctatagcata 59220
catgagatat tttgatacag gcatacaatg tgtaataatc acatcagggt aaatggggta 59280
tctgtcacct tcaagcattt atcctttctt tgtgttacaa acaacccaat tatactcttt 59340
tagttatttt aaaacataca ataaattatt gttgactcta gtcaccctgt cccccacccc 59400
tctagaacca ccattctcct ctatttctat gtcaattgtt ttaatttttg ctcccacaag 59460

```
taagggagaa catgaaaagt ttgtctttct gtgcctggct tatttcactt aacatactga 59520
ccagtgcccc ccatgctgtt gcaactgaca ggatctcatt ctttttatg gctgcgtagt 59580
actccattgt gtatgtgcac cacattttct ttttccattc gtctgttgat ggacactgag 59640
gttgcttcca aaccttggct gctatgaaca gtgctgcagt aaatgtggga gtggagatag 59700
cttgttgata cactgattcc cttttttgga gtatatacct agcaatggga ttgctgtatg 59760
gtagctttat ttttagtttt ttgaggaact ttcaaactgt tctccatagt ggtagcaatt 59820
tccattccca ccaacagtgt ccaagggttt ccttttatcc acatcctcac tggcatttgt 59880
tattgcctgt cttttgaata aaagccattt taactggggt gagatgatct ctcatagtag 59940
ttttgatttg catttctctg atgatcagtg gtgttgagca cattttcaga tgcctgtttg 60000
ccatttgtat gacttatttt gagaaatgtc tacccagatc ttttgcttat ttttgaaaat 60060
cagcttatta ggtgtttttc ttatagagta gtttgagctc cttatatata ctggttatta 60120
attccttgtt agatgaatag tttgcaaata tattctccca ttctgtgggt tgtctcttct 60180
ctttgttgat tgtttccttt gctgggcaga agctttttaa cttgatatga tcccatttgt 60240
ctgtttttgc tttggttgcc tgtgcctgca gggaattact caagaagtct ttgcccactc 60300
caatgtcctg gagagtttct ccggtgtttt cttttagtag ttgcaaagtt tgaggtttta 60360
gatttaagcc tttcatccat tttgatttgc ttttgtatat ggtgagagac agagttctag 60420
tattgttatt ttgcatatgg atatccagtt cctccagcac catttattga agagattatc 60480
ttttccccaa agtatgttct tggcaccttt gtcaaaaatg agttcactgt agatgcatgg 60540
atttgtttct ggattctcta ttctgttcct ctggtccatg tctcatctgt ttttatgcca 60600
gtaccatgta gtttgggtta ctatagcttt gtagtataat ttgaagtcag gtaatgtgat 60660
gcttccagtt ttgttctttt tggtcaggat agctttggct attctgggtc ttttgtggtt 60720
ctgtacaaat cttagaattg tttcttctat ttctgtgaag aacgtcatta gtattttaat 60780
agggattgca ttgaatttgt aaattgcttt gggtagtatg gacattttaa cagtatttat 60840
tcttccaatc tatgaacatg gaatatcttt tctttttttg tgccctcttc aatttctttt 60900
atcaatattt tatagttttc attgtagaga tctttcactt ctttggttaa ttcctagctg 60960
tttaatttta tttgtagctg ctttttttggt ttctttttca gattgtttgc tgttggaata 61020
tagaaatact actgattttt gtacattgat tttgtatcct gctgtaccaa atttcagctc 61080
taatagtttt ttagtggtgt cttaagtctt ttccagatat aagatcatat catctgcaaa 61140
caggataatt tgacttcttc ctttccagtt tggatgcctt ttatttcttt ctcttatctg 61200
attgctctag ctaggacttc tagtactatg ttgaatcaca gtggtgaaag tgggcccttg 61260
tcatttccca gatcttagag gaaaggcttt cagtttttcc tcataatact acctcggtct 61320
gtcatatagg gcttttatta tgttgaaata tgtttcttct tcttcctctt tcccttcccc 61380
ttccccttcc acttctcctc cacctcctcc ttctcctcct cctcccttct ccctcccttc 61440
tcccttctcc ttctccttct tcttattcag tggggtcttg ttctgttgtc taggctggag 61500
tacagtgaca tgttcatggc tcactgcagc ctcgacctcc taggcttaag tgattctccc 61560
acctcagcct ccagactagc tgggactaca ggcatatgcc atcatgcctg gctaattttt 61620
taaaaaagtt ttttgtagac caggtctcat tttgttgccc aggctagtct caaactcctg 61680
ggcccaagca atcatcccac cttggcctcc cagagtgctg ggatcgcagg tgtgagccac 61740
tgtgcctggc ctgtatgttt cttgtatacc tggtttttttg agggttttta tcatgaaggg 61800
tgcagaattt tatcaaatgc tttttctgca taaagtgaaa taatcatata gtttttgtac 61860
ttcattttgt tgggatgatg tatcacattg attgatttgc atatgttgaa ccatccttcc 61920
atccctggaa taaatcccac ttgatcacaa tgaatgatct ttcgaaggtg ttgtttaatt 61980
tggtttgcta gtgttttgtt gaggattttt gtatcagtat tcatcaggga tattggcctg 62040
tagttttctt tctttctgtt tttttgtttg tttgtttttg atgtgttttt gtctggtttt 62100
ggaatcaggg taatactggc catgtagaat gagtttggaa gtattctttc ctctattttt 62160
cagaatagtt tgagtaggat tggaattcat tcttctttaa atgattggta aaatccagca 62220
gggaagccat tgggtcctgg gcttctcttt gctaggagac tttttattat ggctttaatc 62280
ccattattgt tattggtctg tttgggcttt ggatttcttc atggttcaat cttggtaggt 62340
tgtatgcatc taggaattta tctatttttt ctaggttttt caatttatta gtgtacagtt 62400
gctcatagta gcctctagtg atcctttgaa tttctgtggt atcagttgta atgtctcttt 62460
tttcatctct gactttattt atttaggtct tctttccttt tttcttagtc tgggcaaagg 62520
tttgtcaatt ttgtttatct tttcaaaaaa ccaactttta attttgttga tcttttgtat 62580
tgttatcttt cagtttcatt tatttctgct ctgatcttta ttatttcttt tcttttacca 62640
attttaggtt tagtttgctc ttgcttttct agttctttaa ggtgcatcat taggttgtat 62700
ttttaagttt ttctactttt ttttttttct tgagacaggg tttcactctt gttacccagg 62760
ctggagtcca atggcgcaat cccggctcac cgcaacctcc acctcctggg ttcaagcggt 62820
tatcctgcct cagcctcccg agtagctggg attacaggca tgtgccacca tgcccagcta 62880
attttgtatt tttagtagcg acaaggtttc tctgtgttgg tcaggctggt ctcgaactcc 62940
tgacctcagg cgatctgccc gccttggcct cccaaagtgc tgggattaca ggcatgagcc 63000
actgcgcctg gcttttctac ttttttgatg tagctgctta tagctataaa cttccttctt 63060
agcactgctt ttgctgtata ccgtgggttt tgagaggtta tgtttccgtt atcatttgct 63120
taaataaatt tttcaatttc ctttttaatt tcttcattga ctcactggtt attcagaagt 63180
atattgttta attttcatgt gtttgtatag ttttcaaaat tcctcttgtt gttgatttct 63240
```

ggtttgttc cactgtggcc agagaagatg ctttatatta tttcaatttt tttttttttt 63300
tttgagacag ggtctcattc tgtcaccaag gctggtgtgt ggtgttgcaa tcatggctca 63360
ccgtagcctt gaactcttgg gctcaagtga tcctcccact tcagcctcct aagtagctgg 63420
gactacaggc acacaccact atgcctggct aattaaaaaa aaaagtttgt agagacaggg 63480
tctcactatt ttgcccaggc tggtctcaaa ctcctgtact caagagatcc tcccacctca 63540
ccctccccaa agtgctgtaa ttacaagcat aagccatcat gcctggctca attttttga 63600
atttttaatg gcttgttctg tggcctaata tatggtctat ccttgagaat gatccatatg 63660
ctgaggaaaa aatgcatatt ctgcaactac tggatgaaat gtcttgtaaa catcaattag 63720
gtacatttgt tctatattat agattaagtc caattttttg gttgattttc tgtctggatg 63780
atctgtccaa tgctaaaagc ggggtgaagt ctccagctat tattgtatgg ggatctctct 63840
ctctccttag ttctaataat atttgctgta tatatctggg tgtcccagtg ttgggcgcat 63900
ataaattcac aattgttata tctttttgct ttattattat ataattactt tgtctctttt 63960
tgtacttttt gtcttgaaat ctattttgtc tgatataagt gtagctagtt ctgttctttt 64020
tttggtttcc atttgcatag aatattttc cttcctttta ttttcagtct atgtgtccct 64080
ttataggtga agtatattct tgtaggcaac agattattga gtcttgtttt tttcgtccat 64140
tcagccactc tatgtctttt gattggagag tttagtccat ttacacccag cattattatt 64200
gataagtcag gaattactcc tgctgttttg ttgtttgttt tctggttgtt tcatggtctt 64260
ctcttccttc tttctttcct tcctgtgttc cttttagtga aggtaatttc ttctggtggc 64320
atgatttaat ttattggttt ttatttttgg tgtatctgtt atatgttttt ttatttgagg 64380
ttaccatgag acttatatgt gccatgagat ttggaaaggt gcccagccaa aagttttttt 64440
atatatttat atatatatgt atatacgtat atacatttat atatatacgt atatacgtat 64500
atacatttat atatatacgt atatacgtat atacatgtat atacgtatat acgtatatac 64560
atgtatatac gtatatacgt atatacatgt atatacgtac gtatatacat gtatatacgt 64620
atatacgtat atacatgtat atacgtatat atgtttatat atgtatatat ttgtatatac 64680
ttgtatattt atatatttat atatgtatat atatttatat atgtatattt atatatgtat 64740
atatttatat atgtatattt atatatgtat atttatatat gtgtatatat atttatatat 64800
gtatatgtat atttatatat gtatatatgt atatttatat atgtatatat ttatatatgt 64860
atatttatat atgtatatat ttatatatgt atatttatat atgtatttat atatgtatat 64920
ttatatatgt atatatttat atatgtatat ttatatatgt atttatatat gtatatttat 64980
atatgtatat atttatatat gtaaatatat tatatatgta tatatatgta tatagattta 65040
tatatgtgta tatatgtata tatgtatata gatttatata tgtgtatata tatttatata 65100
tgtatatata tgtgcatata tatttatgta tatgtatttt gtttgtttgt ttttgagatg 65160
gagtttcact ctgtcaccaa ggctggagtg cactggtgca atcttggctc accacctctg 65220
cctcccagtt caagcaattc tcctgcctta gcctcctgag tacctgggat tacaggcatg 65280
tgtcaccaca cccagctaat ttttgtgttt ttagtacaga tggggtttcg ctatattggc 65340
caggctggtc tcgaactcct gaccttaagc gatccaccca ccttggcctc ccaaagtgct 65400
gggattacag gtgtgagcca ccaagcctgg cctaaaatat gttttgaaat atactttcag 65460
tgggtaaaaa acattttaat gtagcatttt ttttcctttt ggttttaaaa aatgtctttt 65520
gtcttgtgtt tcctacaaga aatctgtatt gttttcgttg tttcttggcc tgtcattaat 65580
ctcttttcct ctgatactct taagatattc tctttgttgc tggttttcag caatttgact 65640
gtgatataac ttttaatttc ttcctgtttc ttgtgctttg ttttgttgag cttcttggat 65700
ctgtggtttt atgtttttcc tcatatttga aaaactttta gacattattt cttaaagtat 65760
tttccccctg cctctgctcc tacagggcct ctgcttacac atatgtaagg ccgatggaac 65820
ttctgctgaa gctcactgat gccctttct tttcgtttct ttatcttttt tttcctgtgt 65880
ttcacttcat gtagtttctg ttgctgtgtg ttcaaatcca ttaatctttt cttcggcaat 65940
gcctcatctt ccattgatct aatccaatgt atttttcttc tcagacatta cactttttgt 66000
ctgtagatgt ttgacaagga tcttttgcta tcttccatgt ctctgcctta acatcttaat 66060
ctttcctcta gcgacttgaa cacgcggaat aaaggtgatc aacgggttta atgtcctttt 66120
ctactaattc tgtcatgtct gtcgctcccc ggtaagctct gggtggtttt cttcccattg 66180
caggaattgt ttccttgctt cctcgcatgc ctggtaatat ggatgcacgc agtgtgcgtt 66240
tcatcttgtt tggtgatgta tatttttgtg ttcctaaaaa tattgtttag ttttttcctg 66300
ggaaacagtt acattccttg gaaaaactcg atccttctga atcctgtgct tgggctccag 66360
taagtgggac cagggcaggg gttgatccac gaggaaggtt ttttctccgc tactgaggcc 66420
agactcttct gaatacgcca gtcaatacct tgtgaattga gcttttctaa ctcacacggt 66480
attgactgga gtactccaaa gagtcatcat gccaagataa ttgacgtttg ggctgggcgt 66540
ggtggctcac gcctgtagtc ccatcacttt gggaggctga ggtaggtgga tcaggatcac 66600
ttgagcccag gagtttgaga ccagcctggg caacatggtg caactctgtc tctacaaaaa 66660
atataaaaat tagccacatg gggtggcatg tgcctgtggt cccagctact tgggaggctg 66720
aggtgagagg atcacctgtg cctgggaagg tcatagctgc ggtaagccaa ggtcgcaccc 66780
ctgtactcca gccttggtga cagtgagacc ctgtctctga ggccaggctc tactgagtac 66840
tccagtcaat acctggtgaa ttagggctgg gggcggtggc tcatgcctgt aatcccagca 66900
ctttgggagg ctgagatggg tggatcactt gaggccaaga gtttgagacc agcctggtca 66960
acatggtgaa accctgtctc tactaaaaat acaaaaatta gccaggcatg gtagcatgca 67020

cctgtactcc cagctactca ggaggttgag gcaggagaat tgcttgaacc cgggaggaag 67080
acattgcagt gagtcaagat cgcgccactg cactccagcc ttagcaatag agtaagacct 67140
tgtctcaaaa acaaacgaac aaaaacaaaa acaaaacaaa acaaataaca acaaaaaaac 67200
ctggtgaatt agaagctttt ctgctctggc taattagaac cggcactatt gctgtgggaa 67260
caccattcct tccaatccta ttgggttgtt cttatttgga ccttttcctg taatccagtc 67320
ctttttctga cacacatgcc gaccagccat cagccacata tcctgaagcc cagtttccac 67380
acagttcact cctccagtgc tttctcagac ctcagccacc ttggcctccc tggtctccag 67440
ctccatctcc tcgccccagg gagacgacca ggtccagcct caggtgggca ccattgcccg 67500
gacagtcttc ccaggtaagc gattgtaggc ttgccgcatt tggctcctgc cttttgaggt 67560
tcaccttctt tccttggctg agcttcagga tctttagggc tgtgatttca tacattttgt 67620
ctagtctatt gttggttgtg ttgtcatgtc aaatgggagg acgaatcccg ttcctgttgc 67680
ttcattttgt ccagaagtga aagttcctag aacaaggcta aagattaatg agctcattaa 67740
ccagctctaa aggtatgaaa tgaaatatga aaatagacca atgaatgtac aaagaaacaa 67800
gtaatggatc tgagcagaaa ttagtgaagg agaaaacaaa atatcaacaa tcagcaaagc 67860
taaacattta acatttactc ttctaagaaa aactaataat aatgaaaatg acaaaaacag 67920
gtcattccag aaaaataaag atacaagtaa ctatgtaagt gatgcaggac acgcaagccc 67980
caaagtgggg cttagcaggt gagggttctt ggcttcactc aaggatgagc cagtggtagt 68040
gttaccagtg gaggttgtcc aggttcttgg tgttttgaac taagaattgg acaaaatcca 68100
caaagcaatg aaaaaaaaaa gcacagattt attaaaacaa aagtacactc cacagagtgg 68160
gagtgggctc aagcaagtgg cccaagagcc caggtacaga atcttggggg tttaaatacc 68220
ctctagaggt ttcccattgg ttacttggtg tatgccctat gtaaatgaag tagtgaccca 68280
cagttggttg gcttggttgt gggaagcgaa caatcagagg ctgaattgaa gttacagcat 68340
tatattccca tacaaatgaa gacttggccc acgaccagtc tgattggttg caggagggga 68400
ctcatcagag gtactttcaa tttttcatct gccactcaga aaaaaggggg aggtattgca 68460
aagggaatag cttctggttc tttggtaact tgggcatgga aagttggggt tttccttttg 68520
atttcgttct aggaagtcag cgtgaatcag ccttaggttc cctgcctcca gaccctattc 68580
tcctgcctca gtagggtgga aggaaacagc tgtatggaag cagcagggtt acaggtcagg 68640
cagtgtgaca gccccaggac tgctcccgtg gggcagggct ccccacaggc ggcgcgctga 68700
gggcagggct cccgaagtca atgcgtccag ggcagggctc cccacaggtg ttgtgacagc 68760
cccggggctg ctcctgccag gcagggctcc tcataggtgg tgtgctgaga gcagcagctc 68820
agggcaggtc tgcagtcagg tttgtaccca cttttaatta catgtagact aaggggcggt 68880
ttatgcagaa atttctaggg aaggggtagt aaccgttgtg tagtggggtc attgccatgg 68940
aagtgggtgg taacgcctgg gggttgccac ggcaatggta aacactggtg ggtgtgtctc 69000
atggaaagcc gcttccgccc tggctgtgtt tcagctagtc ctcaattggg tctggggtcc 69060
aagtcccacc tctgatctca taagaagttg ttacggattg tactgtgtcc tccccaaaat 69120
ctacgtgtta aagtcctaac ccccagaact tcagaatgtg cccttatttg gaaatagaat 69180
atttgcacgt gtgattagtt aagatggggt cattagggtg gccctaagcc agtgactggt 69240
gtccttataa gaaggagaaa tctggacaca gagacagacg ggcatcaagg gaagatgatg 69300
tgaggacaca gggagaagac ggccatgtac aagccaagga gaggctgaca cagagcatcc 69360
ctcgcagcct cagagggagc cagtcctgcc cacacctgat ctgggaccgt ggcctccaga 69420
actgagttgg ccaatgtctg ctattgaagc tgccagccac agtgctttgt tgtggccgcc 69480
cccacaagct cacacagggg tggagaagca gacgttgcca caggtgtcac gtaaatcaag 69540
acagtgctat gccaatgtca tgccaaggca gttgatattt gggccaggcg cagtggtgca 69600
cacctgtagt cctagcactt tgggaggccg aggtgggtgg tggattgctt gagcccagga 69660
gtttgagatt agcctaagca acatggcaag accccattgc tacaaaaaaa tacaaaaatt 69720
ggctgggcat ggtggctcag gcctgtaatc ccagcaattt ggggagctga ggtggacaga 69780
tcaattgagg tcaggagttc aagaccaccc tggccaatat ggtgaaaccg cctctactaa 69840
aaatacaaaa attagccagg tgtgcctgta gtcccagcta ctcaggaggc tgaggtggga 69900
ggatcgcctg tgcccaggga ggttgagact gcagtgagct gagattgcac cactgcactc 69960
cagcctggac aacagagtta gaccttgtct tgaaaaaaaa aaaaaagaca gttgtcattt 70020
taggtcaaac ggacaaattc tttaaacaaa atataataaa acatcaaggg gaaatagaaa 70080
atctgcatgg tattattatc acagaagtta caaaaattaa aacagtagtg aaaaaaatat 70140
cccacaaagg aaaccgcaga cttaggcggt ttcatcagtt gcatccacaa atgccttagg 70200
ggatgatttt agacaacaga caggaggaaa tggtctcagc ctcaccgtcc gtggccacgg 70260
cagcttcagt gagccagccc tgcacaatca gctctgcaag gtgtgattgg agggaacagg 70320
aaacagaaaa cacaaagaga agccacccag agggttggtg aggatacaaa gggttttcaa 70380
cgccgctagt cagcagggaa atgcacattt aaaccgcgaa aaggctccac tacacaccca 70440
ccagagtggc taaaattaga cagcagcatt gtgtgctgga gaaggggtga tgccatgggg 70500
ctcacatgcc acagtgcaca gccagagtgg cacagacccc ttagaacacg ggtgacatgt 70560
actggagctc agcacacatg caccacaaca gccatgtccc ccctgggtct gtgcccaaag 70620
gcaggcacag ccacgtgcat gatttgatcc gtaacggccc cggttcgtgc catggaatgc 70680
cacatggaag aaagtcacaa tgagcaccgc ttgcgacaac acagatggac gccagacaga 70740
gcccagcccg agaacctggg taccagagct cccagcatga ctgttcatgt gtgggacttg 70800

gacccggtga cccatgggct gggttcaccc gcctggtttc cactgagagg gccccgtggg 70860
gccggggtct gtctgtagcc tgactcgggt gcttggtgct ggagtctgga agcatgcagg 70920
gcttgttgag ctgcacgggg tgcatctgcc atgacactgt gtggaaattg tatagcaaca 70980
agtttttttaa aagtgcctgc cggcctggtg cagcaactca tgcctgtaat cccagcacgt 71040
tgggaggcca aggcgggcag atcacgtgag gtcaggagtt caataccagc ctggcaacat 71100
agtgaaaccc catctctact aaaaatacaa aaatgagccg ggcgtggtgg cagatgtctg 71160
taaccccagc tactcgagag gctgaggcag gagaatcgct tgaacccggg tggcagaggt 71220
tgcagtgcca gcctgggtga cagaacgaga ctccgtctca aaaaaaaaaa aaaaaaaaaa 71280
gtgtctgcct ctcagtgcac cagctgagtc cagccagcag gacagaagcc actgggacat 71340
cacactgaag gttcaacaag ggaaatggca ggatgagggg gaggtgggca tgtcccccca 71400
gacatcccgg ttcagcccca gaaccgcgga tgtgatccca cgtgccagag tggactcggt 71460
ggccaccatc acacttggat cttggatggg agatgatgct gggtaatcgt ctgggactga 71520
tgtaaccaca gagggtcggg gtgagaatca gggatatgac cacggaggca aaggttaggg 71580
tgaggcgaag aaggccacaa gcccgggaat gcaaggagca cagctctcga ggtggagagc 71640
acgaggaacg gagcacctgg agcccctgga gggcagggcc tgcccacacc ccagtggccg 71700
cgcagggagg tctggtctgg actcctggcc tccagaacca tcagagaaca aatatgctgc 71760
ttgaagcatg aaagctgtgg ggatggtggc cgtggctggg ggacactcag gcggtggctt 71820
ttctgggacc agtgtctctg cagaggctgc tgctctgaaa gcatggtggg ccacagggcg 71880
ccacacccccc ttgggtcctg tgcctgcttc aggaaagggg taagcagtgt ggtatacgcc 71940
aagacctcct cctggtttcc ctcctccgca tggatcactc ctcgaacaca gcccctgctt 72000
cgtcagggtt ccgtagcctc acctatcgca ggctgcaccc catccttcct ccagacccct 72060
ggcctgtggg gcttaccggg gccgaggcca acagactgat ctcgttgcca aagacccaac 72120
agccaccaaa ctggcttcaa cagggctatg tgtggcaggt acttcacagg gggacgtgtt 72180
ctcattttcc ttggctttct aataaactct ggtctaaaga aaatctcttc tataaaatga 72240
gtggccagtg ccaaggggta gagacagaag ctccttctgg acactggctg ctccagtaga 72300
gcccatcctg cccaggtacg gagcccacca gctgcgatca ggacactgca gttccaccgt 72360
gggggacccc gggccctcac cttgtcagtc ctctgtttca gcttcgaggg ctttgacaca 72420
gtctaaaatc cacaggtact tagcgcattt ctaagtccca cctcatgatc aatcataaga 72480
cagggctttg atggtatgca gaaatatcaa tgaaatccaa gaggaaaaca gaactaaact 72540
catcgggatg aatgacagat aaacccttta agcatgtttc aagtcgccca ggtggagcct 72600
ctgttggtca gtgaagcttc agggttccca tgagatggat ctgcatgagg ctcagtcctc 72660
tcttgaaaca ctgcccattt gcaggagcgc cggcccacag ggcagaagtc agactgtcag 72720
agcaagtgcc ggtcctgaag ctggaaggac tttcaaggct cattccccaa actcattttt 72780
ttttaaacct ctgctcatct taaagaaagg agaggcaggc gccccagaaa cctctcagga 72840
gtaactgaca cacgtgactt gcggtgtctt caaaggaaca ctctgcaact tgctactgtt 72900
taaacagagc aagactaaca gtcaccagca gctggttcac gcattaaggc cctttgcagg 72960
tgaaagaaaa cggcactgga gggccgactg cagaagcaga cgcagtgcac ggtggacatc 73020
tgcccctttt gggggcctgc cacggtgctt cctgaaaact caggttaagc tgactctgct 73080
gtcaggctga tgaggcgcct gcacccacct ttgctcagca cgccagccca aatggaagag 73140
caggtgctat gacaaaaatc agttcttgta gcaactattt ttgatactga aactgaaaag 73200
cgagagcagc aaagctatcc acggagctcc tgacagttag gcccggcttt ggctgctgat 73260
gcaggggccg aatgacgcta tttctgcagc agccggccgc aggcgctgac ccaccgcggg 73320
atgtgcccca ggaggcagaa gaaacgctgg gaaccgcacc taaccttgcc agcatccccc 73380
aggcaaagcc aggcactttg agttcatcgg tggagttggg cgatactcct ttcccgtttg 73440
tgtgcttagc tgctgccaac aggatgtgct gggtgaagcc ttacagaaca cagccagttg 73500
tgttttataa agcaaggctc ccctccccca aagacaaata aaaccacagg tgtggaaggg 73560
acaccctcca ccgcaccccg ccctagtaat ctgacaccgc aggcaaaggc acggcctgtg 73620
tgggtaagat ccattgtaaa cgtttaaaac ttcctgttcc ctgatgcctc aacttccccg 73680
cagaccgacg cacctgtgat gggcagggcc gtgtgacctc gtgaccttca catacaccaa 73740
tgaagaccct cacgccttct gctgtctttc ccccactgct gacttgcctg cgggccccca 73800
ctatctgccc tgcctctgga gccgcctccc gtgtgccccc ccaggcccct taaggtgctg 73860
tgcgtcccat ctggaaccgg cagttcaccc gctctcccag ccacccttcc agaccccact 73920
tacaacacac gactaagtaa cacagtacag gccgtgtatt cgtatttgtt ttcctgtgcc 73980
aactcatctg gtatcttcgt attttaatga ataaaactga aagatgacaa cttgaagccc 74040
cagtgcattc ctctcaaatc gagtgaagtt tacaacacct taattataaa agatttatct 74100
gatccagaag aaagcagagt gtacagaaac ttcactgtca tctgcgaatg gactgagagc 74160
atctggattt ggttctcagc ctgaatgagc atcctggaat ggtgtgtctc gtccatttac 74220
gtggagacac atggaacagg cctgcaggct tggggctctg agcagggctc ccaaagcccc 74280
tcagctgcaa ggaagccagg cccatgagag gggagcctgt tccagaaaca caagaggatg 74340
aaagctgtta ttggcaaagg agacttagag caaggaaatg accaggaata aaaaaagatt 74400
atatatttag aggccaggtg tggtggctca tgcctgtact cccagcactt caaaaggcca 74460
aggcgggtga tcacttgaga tcaggagttc gagaccagcc tggacaacat ggcaaaacca 74520
catctctact aaaaatatta atacaaaaaa gctggatgtg gtggtgcaca cttgtagtcg 74580

cagctactca ggaggctgag gcaagaagat tgtgtgaaca tgggaggtgg aggctgctgt 74640
gagctgagaa caacaaaccc cacaaaaact aacttcaaca aacattaaac ttcactacac 74700
accgagtcca gcagttctcc acccatcttt ctattccatt tctctctctc tgcctcaacc 74760
tagggcaaga taagcttgac ctgatgggtc atggctctgt acaatagttt agtcttaaac 74820
aactaagatg ctacgtatct aagaacactc atgactcaaa ggcttgcaaa gggctcttta 74880
aatttgctct tttaatgcat aaataaatcc atatcatgta ttactttctg gaaaagggtt 74940
aaactcaaat atccgaaata ctttcattat accaggtcaa gaaaaatgcc acagccagaa 75000
aaatttattt taaaatagaa acatacatta agctttaaaa caaccaactc tcaaacaaaa 75060
gaggaaagag cctttgatcc cagagtccat gcggaatgaa ttccatacgt gtttgaaatt 75120
cacataagga gcacttagaa aaccacctga aatggaaatc caacagcccc cttgcctgtg 75180
agggctccca cccctgcccg cgtgaggaca tggccgaacc ccggacactc gtgtgccggg 75240
agccaccaca gctcaaggtg accggcagca cccagctctg tgaccaagac agatgttcac 75300
acgtgggggc atcgtaagcg ctaccagctc caaatctgac gtgatgggaa cttgggagat 75360
gtctgagaaa tgtccgaagg gatttggca acacagaaaa cgcaatgtct aggaattcct 75420
ccaaatgctt ccaaaaatac tcattgacaa ttcaagttgc acttggctgg cggcagcccg 75480
ggcggccttc agtccgtgtg gggcgcccgc gtggccttct cctcgtagga ctccccaaac 75540
tcgttcactc tgcgtttatc cacaggataa agcctgcaat gacacaaatg agcacatcag 75600
caaaccccac tgcaggggct atttttctaa gaggaggact tggcatcctc gatttatttt 75660
ccagtgagct gccacagtga gcaaattaac taaaaagtcg aatcctcaga agttttactg 75720
ccgagggctg aggagggatt tctgagttgt gttttgttct gcatggccag gccagcgtgc 75780
tgctctcaag gaaagggaag cttggccgaa gggacgaccc ggaggctgca cgggctgcca 75840
tggctgaagg ggggacccgg aggctgcacg ggctgccttc tgggaacggt ggcatcacgg 75900
agactcgagt gatcagaaca ggaaccgctg tgttcaggga ccacgtctca tcagctgtga 75960
acacttggca ggacactgcc gccgcctttg atcctgagcg ccccggaggc cttgccaaca 76020
tcacccctac gtatggatga ggaagctgtg gcataagggg cagctctaca cggccgcgaa 76080
cagaagtaca gggtttcagc ttaaaaacag acctgctgcc caccaagggc cctgtgatcc 76140
tggccaaaca ccgtttgcag gtctggagcc attaacacag agcctgagta cagcacagtt 76200
tcaggcacgc cccgggccac ctgcgaggac ttgggagcca cttgaagggc tcacagttga 76260
gacaaccaca ccaggcgcag cagcgtccgg ggctccgtgg cttggacaac ctcaggctct 76320
gcagggggca ggcggccagc tgggggatct gagaatggtg aacgggggac aggaaccaag 76380
ccccgtgtga gtgtgagtgt tgcttatggt gaaatagccc ttcccgctga cacaagctgg 76440
agctcccgtg caggggctgc acgagacagc aacagggtgg gggggggcag ccggcggcca 76500
ggcctggaga gctgggcaag ggcggccatg agccactgtg tggatgtgcc aactgcaccc 76560
gaacaaagag ttaaaccagg cagaaccatg ccagcctcat gctggctggg acagaggctc 76620
tgaacacctg gcgaggttga gtgtggagac agggcgggga cgggagcact gcgctcccca 76680
agggccggca ccccggacct aggcaggacc cgccaggacg gcctggggga agaggatccc 76740
tctcctgcac tagtaggatg gccccaggcc ctgagggaag ggcacaggga agtgtcacag 76800
ccgtggtgtg tgaggtggcc tcctggtcgg gggagggaga tgggaaaagg gaccttgagg 76860
gccagagaaa ggacacagcc aggaggctct gagacacttg gcagcaggtc ttgggggtga 76920
cacacactcc ttcgacccct ggacaaggcc ctggggccgg cactggaggt gagggcagtg 76980
cttgcagtgg tccagatgca accctcccga ggggagcccc agatccctgg ctcctaccct 77040
ggcacagcag agctggaggg aggttttgct gactgagagg gcagatgtca gccctggagg 77100
gaagcgggag gcccagggtg gggcctgagc ctctgcattt ggaaaattcc gaggcccggg 77160
gtggggcctg aacctctgcc tttagaaaat tccacaaagc cttttcctca gccgtcagcc 77220
ttcaacctga aggccaagct gagaggtcca cgcaatgcag tcttcagagg ccctggtggc 77280
tggtttaagg ggtgcggcca atgctgaggt gagtgagctc cccagtgaca ttgtgccctg 77340
caggtccaca ctgctggctg gacacttcga ggctagcgcc aagtatggat tagccatgct 77400
acggtctctg ttctgctcaa tagcaatgac tgtgaatagg taaataaatc gcttaaggag 77460
agacatttgc tttcagtggc tcatcaataa ttcacataga aacgagcatt tctaaagcac 77520
agtgaggaga cagagctgga acagtttctt gccaggacat atcatgggca actggaaccc 77580
aagtttaggc aagacaggaa aaaccaccac ctgcaaatta tcttttccct caaatggata 77640
aacaggcgca gggtgcggtg aaagccgtca ttccgttcag cagcagccac gccgctgaga 77700
cggagcaacg gccgagcata cgcagccgca ctcaccaccg ctggtacagg tagaccagaa 77760
acaccacgtc gtcccggaag caggccagcc ggtgagacgt gggcatggtg atgatgaagg 77820
caaagacgtc atcaatgaag gtgttgaaag cctgcagggc cagacgggag gagggtgaac 77880
cccagttgct ggggctggaa tcctactgtt tttggtaacc taaccaagcc aacggctttt 77940
ggcagatgct tggaactaac tggaactcct cacagcaaca acaaaaagag cagaaagccg 78000
gcaagtggag atacggagct ctgttccctc atgggctccc cacagctgct ggcacccgac 78060
acactgcggg tcttgcccag gctcccacat cggggaaccc aaagagaatg gcctgaaacc 78120
agagccaagg ctggagccgc ccgagacaga gccaagggca gagccacctg agaccagagc 78180
caaggttaga gccgcccgag accacagcca aggggagagc caagggcaga gccacctgag 78240
cattgccctt tcctctggga gcttgtaatt gacttcagaa aagtgcaaat catgtcccat 78300
taactcagct ccgcctgcaa tgccgaggca cttctaatcc cacacggagc caagggggcc 78360

tccacagctc tgccagcggg gaatggccac ggcccatggg ggcttccatt cctgcctggg 78420
aggcctgaat ccgttgtccc caggcaccgg ttactgagcc acagggggtc tgtgtcttga 78480
cacatgtgct aagaagaaac ataaacacta aaaaagaaaa agaaaaccaa atctgtgaca 78540
agtgagagaa ccaccccagt ctttcagggt ctgaagttac tctatagctc ttcgcttttt 78600
tttttaaagg agcctcctca atattcttca atgaaaggga caggtgcaga tgcactctgg 78660
gcagagatgg ccccagtaat gctgttggct ggaagacgcc cttgctcacg ctctgctcag 78720
tgagaaggga ttcctcaccg gctgtcacac tcaccttgta ggtgaaggcc ttccagggca 78780
gatgtgccac tgacttcaac tgaaacagga gagacaggcc caggtcagct gtgggcagca 78840
caggagacgg gggccgggcc gcgggcagca cacaccgcct taccttgtag ttcacaaaga 78900
gctggggcag catgaagagg aaaccaaagg catagacccc tgcagaaaga cagacagcac 78960
tcacgaggtg cggagggccg ggctgccaca tctacgcaca gacggcactc acgaggtgtg 79020
gagggccgag ctgccacgtc tatgcatagt gggcagaaaa caaggtctgc tatccagaca 79080
acttcagagt ccatcatggt gtgaagcagc tttctggctg gtaagtttat caagagtcta 79140
cgacaacttt ggagagcaaa gctcttctat ttattaatca gtgtaactgc tgcccacggg 79200
gtcaagtcag tgagagcact ggagcaccct ggggctatga ggacacggcc tcctgaccca 79260
caaggtcctg ccccaggggt caggtgtggg actcctacca gggaggacgg cagtgcggga 79320
cccacctgct ctgcaccaga caggccctcg cccttaatgc tcacgggacc acttcccaaa 79380
gagaccacaa agctgcgatc agggctggtc tggtggggct gggggatgga cacagcagcc 79440
catgcagctg aaggtgccac ctggggcagg cgggcctccc aggacatgtg gccccgagca 79500
cagggccctg cagggtcccc acactgcatc tgcaggaggg ggcgcgccgt gcaccagagc 79560
ctggtgcttc catttgctct aaggaaccag acgtggatgg tgttgtttta atgcttaggt 79620
ttccttgttt ccatgaagaa aacagacaac agtcatgcac cacacaatgc tgttttcatc 79680
agcgatggac acatatacca ggcggctgca ccacacagcc tggcgtggag gggctgccac 79740
atctggtgtg agcactccct gtcgcttgta caaggacctg tttctcagaa ccagtcactg 79800
ttgtgaagcc acgcagggct ttatctgctc acagcctggg gggagccctg ggcaccgcac 79860
atgtgccaga acagggtggg gagggattag cctcaaatca caggggggct tgccacactg 79920
ggtttcaatg caagaacaat taaatcgctg cctgctggaa acactagtac tgaagcaggg 79980
aagcacatgg actcaccgtt gacgaagctg ttgattaacc aggagtacca gctgaaacgg 80040
aaaaaaaagg agaagtccta tttctcgcat agcttaatat ctgtattaaa ttgatgaaaa 80100
ataattcttt cattaaaaat cctctgaaaa tacccagatg ctctcacggc agctcggcag 80160
ccaagagcag cagtgctgag cctggttctc aagctggaga tagcgctgag cacccccggc 80220
cgggcagcag aggccgggca ctccaggaac ccctcagctc agggccagca ccccacctgg 80280
gcagcagagg ccaggggctc caggacccct ctcagctcag ggccagcacc ctgcccgggc 80340
agcagaggcc gggcactcca ggaacccctc agctcagggc cagcacccca cctgggcagc 80400
agaggctgga ggctccggga tccctcttgg ctcagggcca gcagcccacc tgggcaacag 80460
aggctggggg ctccgggatc cctcttggct cagggccagc accccacccg ggcagcagag 80520
gctggaggct ccaggacccc tctcggctca gggccaacac ccccacccagg cagcagaggc 80580
cgggggctcc gggaaccctc tcagctcagg gccagcaccc cacacgggca gcagaggcca 80640
ggggctccag gacccctctc agctcaggcc caagcctccc atgaagcctg gtggctggag 80700
tcagtcctct cagctacacc ttaccagggg gacactgagg cccagagccc accgtgtgca 80760
gctgcagggg caggacccct cccaggcgct ggcccaagtg ccccggcccc gagtttgccc 80820
tccagtcgca ccccgctctg gctcagtcat ccaaatggct ttctcagggg aaagacgcag 80880
caggggaagc gtgtgcggcc tcctacctct tatatttgat attcaggagt gaatagacag 80940
cacccccgac acagagaggg tacagcaggt atgacaagta cttcatggcc tgcagggaac 81000
aaagatggct tccagttaga ggcccaaacg ccaagcctct gtaaacacac tgcatggagc 81060
tcaccccgac agggacagac agaacgagct acagcgctca gggtggcagg gctgctctgc 81120
agggggcggc gtgagggggca caggcagggc atcgcacact cgccacagcc ccaggaggca 81180
ccgcacacgc cagagcccgg gtgtaactac aggcgctagt taacaataat agatcaattc 81240
tggttggtca aaaccaacca acccacaaca gatcaataaa cataatggaa agaaaccgga 81300
aaaaactgcc tactaagtaa tgtagttaca tcgccagctg tatccattct gatttcagct 81360
ttacaacgcc atgcaagttg gatataagct ggtgaagatt aaaccaaact aaaatcatct 81420
gtgcagaaat gttctattaa ctggaactga gaattccagc tcccccaaca gcaatgggga 81480
aaggtgacgc cctgtgctgc tggggccatt cctccaggcc actgtgcggc cgcggccatt 81540
atggccttgg tgacggcaag tgcgttccca gcaaccacag cagacaccat ggcctgcgtc 81600
agctgcctgg cgaatgtgac ttacaaacga tgaaaatatc cctactatac ggtgacacag 81660
gcaggctgtg cctgtaagga ctgggaaatg agtctctgcg agccccccgcc cttcccagta 81720
agaaatttgg gttttatgtg ttcactcttg ggatccctgc tcaaggcggg ctgacccgta 81780
ctccaaggag acttccgcac tgagcaatga cagtaaaaga cccgtctttg agggtgttgg 81840
atttgcctgg catgcacgtg ccctgaatca caagtgactt acctgagtat cgtactcctc 81900
ggttttcctc tcagattcgc tgtaagtgcc aaactgttgt gaaattcaac acagtgatat 81960
taatagactc agggaggatc tgagcacagc tgagctgtga ctaaggggcg tcacatatgg 82020
ctgcagagct gacccaggcc tcactacaga gggcgctcag gtccctacca ggcgaggaga 82080
ggacccagag gacggggatc cgcatggatc cttcccgcct caccctggcc ctgggtccca 82140

```
caagcctgat gggggcaacg cggcctcact gctgttcctt gctcaggtgg ctcagttttc 82200
agtgccaccg cttcagtgag aacactgcag agcacacagg cacagccaac actgctccga 82260
accagccggc cgggagccat ggggcaaggt ggaacacagt ggctgcgctg cgtgtgctgg 82320
gcttcctgac ccccctgccc acagcatgga aaatctcagc ctgtgactgc tgagggcccc 82380
gcctcaatca aggtccatcc gtgggcactg agattcaact ccagctctgc cccagccctc 82440
atctgcctag gggccttatg ggcgcccagg cactggggca gccaccagct cagggcaggg 82500
gctcactcac ttcaggactg ccaggactgt acggacccca gtcagcacca gcctcgggag 82560
cgcctctgga caaggtgcca aaacgaagca cacgtgctgg ccagccagca tccttcctcc 82620
cagaggccgg ctcaggccca gaagtgctgc cgtctgcact gaagacggtc agaactaggt 82680
atatgaccgg ggaatcctca ggcgaagaga gtggctgcga tcagaaacca gtgctgcctc 82740
caccacggag gctcccctga cagaggcccg cagtgactca tctgcagatg gccatcttac 82800
tctttgcaca gggggcaaaa tcacactctt cagagaggct tggggttcag ccattacaac 82860
taaatcctac ctgaaattcg ggcatcaggc ctctccaaaa aatagtcatc ttcaatgcct 82920
tcttcacttt ccacagctga ggggagaaat caggaaatag ttcctttaat attcgaaggc 82980
caggagccac gaatgaacga cccagacagt aacgggtagg acctgctgcc catggccccg 83040
cgcagcccac tgtcctcttc ctgtccctcc cacagctcag gccagagcgc tggaggctgg 83100
acctgggcct ctaacccaca cacggcaggc gtacctggtc aggtgggctg cacagccacc 83160
gcccagcgga caacaaaaac agcatgtgag ggcagttctt tacgccactc tgcctgcacc 83220
caaataagcc cccacctact tctgcctgcc actcgggcag ccctggagcc agttttggga 83280
ccgctctgag agccgccacc tcatggctaa atgcttggaa ggcatcaccc acggagctca 83340
ggacgggtgc cagagccggg cacggcactg tgactcgtac ctatggaggg accctgcctg 83400
tggagctcca gcgtcatttc atccagctcc tcctctacag ggacattcca tccagctcct 83460
cctctacaga cacattccat gcagctcctc ctctacagac acatttcatc cagctcctcc 83520
tctacagaca catttcatcc agctcctcct cgacagacac atttcatttc atccagctcc 83580
tcctccacag acacatttca tccagctcct cctctacagg gacattccat ccagctcctc 83640
ctctaccgac acatttcatc cagctcctcc tctagacaca ttccatccag ctcctcctct 83700
acagacacat ttcatccagc tcctcctcta cagacacatt tcatccagct cctcctctac 83760
agacacattt catccagctc ctcctctacg ggacattcc atccagctcc tcctctacgg 83820
ggacattcca tccagctcct cctctacggg gacatttcat ccagctcctc ctctacaggg 83880
acaatccatc cagctcctcc tctacaggga catttcatcc agctcctccc ctacagggac 83940
atttcatcca gctcctcctc gacagacaca tttcatccag ctcctcctcg acagacacat 84000
ttcatccagc tcctcctcta cggacacatt tcatccagct cctcctctac ggacacattt 84060
catccagctc ctcctctacg gacacattcc atccagctcc tcctccacag ggacatttca 84120
tccagctcct cctctacagg gacattccac ccagctcctc ctctacaggg acattccacc 84180
cagctcctcc tcgacaggga cattccaccc agctcctcct cgacagacac attccatcca 84240
gctcctcctc tacagacaca tttcatccag ctcctcctct acaggcacat tccatccagc 84300
tcctcctcta cagacacatt ccatccagct cctcctctac agacacattt catccagctc 84360
ctcctcgaca gacacattcc atccagctcc tcctctacag ggacatttca tccagctcct 84420
cctctacaga cacattccat ccagctcctc ctctacagac acattccatc cagctcctcc 84480
tctacaggca cattccatcc agctcctcct ctacaggcac attccatcca gctcctcctc 84540
tacagacata tttcatccag ctcctcctct acagggacat tccatccagc tcctcctcta 84600
cagggacatt tcatccagct cctcctctac agggacattc catccagctc ctcctctaca 84660
gggacattcc atccagctcc tgctctacag ggacattcca tccagctcct cctctacaga 84720
cacatttcat ccagctcctc ctctacagac acattccacc cagctcctcc tctacagaca 84780
catttcatcc agctcctcct ctacagacac atttcatcct gctcctcctc tacagggaca 84840
tttcatccag ctcctcctct acagggacat tccacccagc tcctcctcta cagggacatt 84900
ccacccagct cctcctctac agacacattc catccagctc ctcctctaca gacacattcc 84960
atccagctcc tcctctacag acacatttca tccagctcct cctctacagg gacattccat 85020
ccagctcctc ctctacagac acattccatc cagctcctcc tcgacagaca cattccatcc 85080
agctcctcct ctacagacac attccatcca gctcctcctc tacggacaca ttccatccag 85140
ctcctcctct acagacacat tccatccagc tcctcctcta cagacacatt ccatccagct 85200
cctcctctac agacacattc catccagctc ctcctctaca gacacattcc atccagctcc 85260
tcctctacag acacattcca tccagctcct cctctacaga cacattccat ccagctcctc 85320
ctctacagac acatttcatc cagctcctcc tctgcagaca cattccatcc agctcctcct 85380
ctacaggata cattccatcc agctcctcct ctacaggata catttcatcc agctactcct 85440
ctacggacac atttcatcca gctcctcctc tacggacaca tttcatccag ctcctcctct 85500
acagggacat ttcttccagc tcctcctcta cagacacatt ccatccagct cctcctctac 85560
agacacattt catccagctc ctcctctgca gacacattcc atccagctcc tcctctacag 85620
acacatttca tccagctcct cctctgcaga cacatttcat ccagctcctc ctctacggac 85680
acatttcatc cagctcctcc tctacagaca gatttcatcc agctcctcct ctacagacac 85740
acttcatcca gctcctcctc tacagacagt tttcttccag ctccttgtct acagacacat 85800
ttcatccagc tcctcctcta tagacacatt ccatccagct cctcctctac agacacattt 85860
catccagctc ctcctctaca gggacactcc atccagatcc tcctctacag acacatttca 85920
```

tccagctcct gctctacaga cacattgcat ccagctcctc ctctacagac acatttcatc 85980
cagctcctcc tctacagaca cattccatcc agctcctcct ctacagacac atttcatcca 86040
gctcctcctc tacagggaca ttccatccag ctcctcctct acagacacat ttcatacagc 86100
tcctcctcta cagagacatt gcatccagct cctcctctac aggcatgacg gccgcagagg 86160
cccccaccca caggtgggcc accaactgca gagacctcag actccggctc ctgccggtgt 86220
ggcacagagc tcagtgagga gctaggctta agcctgggcc ctctcaccct gaaggccatc 86280
gcccccctca agggtttgct cctgagctgg tcttgcccca cgaggctccc ttttggccgg 86340
cttttctgag ctccggaaca accaaggctg gaccacaggg caccgagtga tttccgtcag 86400
tgcctacctg gccgctgtgc tcagatctcc agccacagca cgagcctctc aaggccccac 86460
ggtttacacc tctgctttgc acatggtggg atttctcaag cacatgaaaa cactctgttt 86520
ggtggacagg gcctcaggcc tctgcttggt ggacagggcc tcaggactca ccactgcttg 86580
gtggacaggg cctcaggact ctctgcttgg tggacagagc ctcaggactc tctgcttggt 86640
ggacagagcc tcaggactct ctgcttggtg gacagggcct caggactctc tgcttggtgg 86700
acagggcctc aggactctct gcctggtgga cagggcctca ggactctctg cctggtggac 86760
agggcctcag gactctctgc ctggtggaca gggcctcagg actctctgcc tggtggacag 86820
ggcctcagga ctctctgctt ggtggacaga gcctcaggac tctctgcctg gtggacaggg 86880
cctcaggact ctctgcctgg tggacagagc ctcaggactc tctgcttggt ggacagggcc 86940
tcaggactct ctgcttggtg gacagggcct caggactctc tgcttggtgg acagagcctc 87000
aggactctct gcttggtgga cagagcctca ggactctctg cttggtggac agggcctcag 87060
gactctctgc ttggtggaca gggcctcagg actcaccact gcttggtgga cagagcctca 87120
ggactatctg cttggtggac agggcctcag gactcaccac tgcttggtgg acagagcctc 87180
atgagctgat accacccgaa cacgcatttc cttgctctcc acttgttcaa agcctttaca 87240
cccactgcct gccatcctac agcttccaga acactgaggc catcgggaac aagcacacag 87300
gcttccacag aaggtctcag gtcccggggc tgaaagcctt tcctgagtgc gtggaggcac 87360
atggacctca gacagttcag gtcactgccc ggaactcacc tcaatggcgg ctccaacacc 87420
cgccgggacc agcaccagca ggctcgtctg ctcgtccagc aggaacagaa agatgaccac 87480
ggtgctgaag cagcgccaga gcactgggga caggatggtc gggctgggag ggggtgcagg 87540
gcagacccca cctgtgttcc ccaagtcaca cacataccc cagtccccca tcccaaccca 87600
ccacgcccaa gaagcttcag gtactcccca gtccacgtca ccccgtttaa aaagaacaat 87660
gaagcattca gcagctagga aagtgtttgg aaggctgctg aactgaacag ctggccccac 87720
accagctcca caaacacgtg tctgagccac acttgcaaat gagcatggaa cgtgggcaga 87780
ggtcggatga cggggccagc acagctttgc ttccgggcgt gtcttatgcc agttctgtga 87840
taagtgcgtg gcttttcaag tctggatttg tttcctcccc agctgtacct taagaaccac 87900
tccagcttat tcaacacgtc ttcctcctcc tgcaagccct gagggcaagc aggggaagca 87960
gatccatgga gccacaggac aaacagtggt caaggccaca gccactgtct gctggccact 88020
ccaacccagg acgccaggaa aggcattagg gttaggagac aacaacgcag aacacggcac 88080
ggcccccagg cagccagggc cacctgcttg ggcgtctttt ccatgcgaga aaaacaactt 88140
gttcgggtct gatactggag ccaaatgctc ctcactccta accaacagga cacacccact 88200
cctcgtcctc acacccatca accactcgcc cctgtccaga ccgccgctcc ccaccccact 88260
gtgacaacct ccacccctgc aaggtcagga ggacccctca ctcacttggg ccccttaaac 88320
cacggcaggc acccttggcc aggaacactg gtcccagatc ttggcaggcc tcctgcctgg 88380
gctgcctggc ctcaggggtc cctgactgcc ctacctaaca gctgcacact gcctgtcact 88440
gctgggcaca gcaccacggg cctattaact gccatcacct ggcaggcccc acacgcccag 88500
gcctgcgctg gtcggggaag gaacaccggc gggaggacgg ggcgagggaa ccaagacagg 88560
ggatgtgagg ccccagccct gctcagcgcc catgagcagc cgggaaggct cgagaggtcc 88620
atgcatctca ctctccacag cccagaccca gaccccatgc acgccagagt ccgcagccag 88680
gccctgagct gtgcagccgc tggaggccct gagccgtgca gcctctgatg gtcccaccac 88740
aattgccgca acggctccct gggctttacg gtgcctggtc tcagaggctt ctcaagagcc 88800
aagcacacac ccggggctgt gtctgcagcc agcagtgagg caggcagccc tctaccgcag 88860
gctccagtga gctccctggg gcagagtatc tgagcagggc cacgctcggg gggcctacct 88920
gccttggtgg acatgccgat catgctcttc ttcttcttcc agaaactgat gtcattttta 88980
aaggccagga aatcaaagag aagctagaga gaacacacac gacagcaact cacatctcct 89040
gctgtttcca tctcctgtga gacagagata gaggcttcgt gtgtgaccgt gagactgcag 89100
gtgtactcag cctcaggatg catcaggata agtgtctaca ctcgggaccc atgcctctac 89160
gcgcctggcc tgccatgtca gaacctagag tgcccaggcg ggctttgcag ggcacttctc 89220
gaaataatac gtacctttga cttgaactgc tggaaaggcc cccaggcaat tggaggctct 89280
ggattcccct ggatcatggg cctggctggg cacccctgct ctcgggttgt gcccactcag 89340
ggcagcctca tctctcaact gtgctaaaaa cgcaagggct gggcatggtg gcccatgcct 89400
gtgatcccag ctactgcaag gccaaggcca aaggaccacc taagcccagg ggtttgagac 89460
cagcctgggc aacgtactga gacctgtctc caaaaaaaac ttaaaaaaac ctaaccatgc 89520
atgatctcat ccttagagac actgaccttt taataaaaat cgtttaattc tctagtacgg 89580
ctttggaaag ttttagcttg actggcagcc aaactcccag gcactcagaa ccagtgccgg 89640
ccgcactcct gggtgccgcc taaccacagg ccagcctaac agccacaagg atggctctcc 89700

ctagcagctc agctccacga acaacagtaa gagcacagca gccttgcttt acccatggtt 89760
tactgtccat ggtttcggtt agcacggagc agtgcgatga gacagatatt ctgagagtga 89820
gaccacaccc acgtaacttt atcacactgt cctgtcctgt ttctgttacc agttattgtg 89880
ggtaatctct tactgtgctt aacttataaa ttgaacttta tcaaaaccat tacataatac 89940
atatattcat tcattacgtt atttgataca catattcaga catccaatga gggtctttga 90000
acacaccccc tgaggatgag ggactactgt ataacaccag atgaggataa ggggcggact 90060
actgtatata cactggatga gaacaagggg ggactactgt atacacacgg gatgaggata 90120
aggggggaat actgtagaca caccggataa ggggggacta ctgtatacac accggatgag 90180
gataaggggg gactactgta tacacaccgg atgaggataa ggggggacta ctgtagacac 90240
accggataag gggggactac tgtatacaca ccggatgagg ataagggggg actactgtat 90300
acacaccgga tgaggataag gggggactac tgtagacaca ccggataagg ggggactact 90360
gtatacacac cagataaggg gggactactg tatacacacc agatgaggat aaggggagac 90420
tactgtatac acacgggatg aggatgaggg actattgtat acacaccaca tgaggataag 90480
gggggactac tgtatacaca ccagatgagg ataagggggg actactgtat acacacacgg 90540
gatgaggata aggggggact actgtataca cattggatga ggataagggg ggactattgt 90600
atacacacca gatgaggata aggggggact actgtataca caccagatga ggataagggg 90660
ggactactgt atacacaccg gatgaggata aggggagact actgtataca cacgggatga 90720
ggatgaggga ctattgtata cacaccagat gaggataagg gggactactg tatacacacc 90780
ggctgaggat aaggggggac tactgtagac acaccgcaag aggataaggg gggactactg 90840
tatacacacc ggatgaggat aaggggggac tactgtatac acaccggatg aggataaggg 90900
gggactactg tatacacatc gggtaagggg gaactactgt atacacacag gactgacttt 90960
ttacatttaa aaatcagtgt gttagaattt atacaagaga actactttct gctagaggct 91020
gcccagctcc gcccctggct ctgcatgggg tcggggcctg ggcaggggag agtgagatgc 91080
ctgcacctat tcatctcatt ggtgtggaca ctgctcagag taccctggat ccttttcaaa 91140
accgccccca aagccagcac agcccaagtc gccaaggctg tggacggagc tttatggtcc 91200
ctcccttttc agaactggga gaaaccgccc actgggacaa tggtcgcctg gtgtcaggcg 91260
tgctggctgc tgagtgatgg gagtggacac ttgagggcca cagggctgga cggcgcccac 91320
aaaccccagg tccaggaccc ctgcaggagg cccggggccc agggagcccc ccaagtgcct 91380
gcggcaagcc ccccggtgga tgactcacat ggaacgctgc gacaaagaag gtcagcgcca 91440
ggaagtataa gttggtatct acaaaaattc ctttcacctc atcagcatct ttctctgaaa 91500
accctgtcaa ggaaaaaaaa acatacaata taaaacaggc aatgtcaatc ttacctaaca 91560
ttacaaatac agttttcaat ataaaaattt aggccgggcg cagtggctca tgcctgtaaa 91620
cccagcactt tgggaatctc aggcaggcag atcacttgaa gtcaggagtt caagaccagc 91680
ctggtaaaca tggtgaaact ccatctctac taaaaataca aaaattagct gggcgtgggg 91740
gcacgcgcct gtaatcctag ctactggggg gactgaggaa cgagaatcgc ttgaatccgg 91800
ggggcggagg ctgcagtaac cagagattgt gccactgcac tccagcctgg gcaacagagc 91860
gagactctgt ctcaaaacaa acaaacaaac aaacaaacaa acaataaaaa taaatctcat 91920
caacagttaa ggttcatttg accaaactca tggcatttca catattatgt tcttaataaa 91980
gccaatttcc agacaatcaa ccaggtcatc tccaaggaga gaagtgaggc aggcgctgct 92040
agcctctgat tcaacgtctg tgcaaggatc tgcagcagga ggcgaggagg ccccggcctg 92100
tgtcaggatt cgcacttgga tgcccgaggg gctccagggc ggcctcaccc aggcgccggc 92160
cgtgtgcggc acataccgaa ctgctgcagg gagtacacgg cgtcctgcat gtggatccag 92220
aagcgcagcc gccccagtga gaccttgtcg taggacacgg tgaggggcag ctcggtggtg 92280
gagcggttta tgacctgatg aagaaagcca cactgagggc cctgccctca tacccttgca 92340
cccagctgcc tggcagccct cgccaaccct gccatccccc ttcccatccc tgtgtggccc 92400
caggtcagcc atgactgggc ccagcaagca tcccccggtc cctccttctg tcaccacagg 92460
cctcaggccc cagcgtgctg cgttccagca cagggaggtt cccaccccca ccaggacccc 92520
gtgagctcag cagcgctagc agtgcctggt ctgttttgct gcacgtgccc cttcaatttt 92580
aagccgcaca agtcctggtg ccccggaggt ggtgcttgcc acccagctgc taggactcca 92640
tggcgcagca tacggcgctg agcttggcct gcagagccct ggcctgtcct aagagttgga 92700
gaccacggga caagcacagc cctgcagcac atgggcaaag tgcctcaggg cctaaagcac 92760
agagcagggg gtggccgtgg ggacaacaga aagcttctgg aacgcaaagc tagcaggggc 92820
agtagggtgg atgtgtaggt ctcagaacca cgagcttagt cctgcagcct gccggggcct 92880
cacgcacaca ggctgtgggc caggtcccag ggtgccgagg taggagggtg aggaacgggt 92940
tcacaagcgc gagcccacac cctacagcac gtgggcagtg accacacccg gtgagcacca 93000
ccctccacgt tctggaacat ccagcgaggg ctgtgctcta atccacctgc cctgcctcaa 93060
gccctgaccc gccaccctcc acgtgctgga acatccagcg agggctgtgc tctaatccac 93120
ctgctctgct atggcgggca ctgctggtgt cagggtgtgg tggaatcaga gccacaacca 93180
gcaggtgcca ggttgccctg agagcggctg tcagggcgcc ccacgtagcc aagtggcatc 93240
agtgcacatg gatggtggcc tccaggcacc cctcgagctg ccaagcgtgt ccgaagggtg 93300
tcactgcctc ctccatctgg cagtgctggg cccagccaca gaaggtgccg acttcctgca 93360
cctgctgcat cccagctgcc tgcttcctct ctcaagacag cacctctcga atctggcccc 93420
aagtgagaca cagcaacagc gacacatgag agagactgtg atttggggga aaagctgctg 93480

tcggcacacg tgtctccata accactggaa cgcaggccac cactggcaca gctgcgccgc 93540
aaagcctgcc ccgggcctct aacaagacag atctgcagac agacacacag ggcagccttc 93600
tgcagctgcc tgcccctgtc caccatctcc tgaatgcctg caaggagtca gcggcatgag 93660
gcttcacaag aggtgaccac gagctggtgc cacagctcac acagctctgt atggggcatt 93720
ttagcagaac ttgctgtcct gaggtttgtc agcagcacac cagcaaactc cagcaaacag 93780
agaaagaggt tggaattgca ggggccgaca gagaaactac tcagggatag gctgcagcgc 93840
cagacctgct cgccagccac tgcctgtgca gcccccagcc tgcaggttgt ataggagcaa 93900
tcagtgaccc cagaagtgaa ggaggcagta tgttagggac tgcaaggggt ctgagggaac 93960
agcagtacag gggactgtt ctgagagccg cagtcaggag gaggcagcag ggcctgggca 94020
gagatcaaag cagccaggtg ctcgcttctc cgtttcccag ctgcaccccc ccgctagcaa 94080
gccctctgct ctccccagga ctgtttcctc ttcaacaaaa aaggggaaag ggcccgcctc 94140
agaggtcaga ggtgccgaga gccttaagtg gaagtggccg gctcctagtg ggtgatccgg 94200
agtgacagtg atggcttgca gggaggcagg gcagggcagc gatcctgcag ggccccagct 94260
gagcgttcac ctctgaaccc caaataacct catccgtgaa acaacgccac agctcccagc 94320
accacaatta cggaaagatt cacaaagtgc ttagcacagt gcctcatgtg cagtaagaga 94380
cattaaatat taactgcttc tgtgagtctc aatactggcc aaaggttggc cacatttcat 94440
ctcctctcaa agcaactatg ggagagcagg cgctgtcact gcccgtaatc acagggcgcc 94500
agagcccaga gagcagccac ttctattcgc aaaggccttc cgccttgcca ggggccaccc 94560
cgggggctcc agctccgagg aagggctgtc acccgctcct ctggccgcct gtgcaggacc 94620
agccctggtg gggaaagcag gagtttgctc aggatactgt gaattaatct acatggcaca 94680
ccaggagctc acacgcagac gctgtttcac acggtgctca tgtgcctgct ctctaagcgc 94740
tgccctctat gcgctgcata aacaatcttc caggggcctt tcaccagtga cagtccagag 94800
gctcagcagg accccgcagc cagccaacgg caaacccagc acaccaccag ggccccctg 94860
tcacaaccct cgcacactcg aaggcagtgc ttcccagcac gggtaaaagc accgtgtcaa 94920
atgtcagcaa tggcccggtc cattagggtg caggggctgc ggggccagtc ttggggtcag 94980
tgtctcgcca gctgcacaac cagcgggcag aggtgtcact caccatcagg tccttcacgc 95040
ggttgctgag ctggtcgatg aacaggatgg gcaggtaatg cacggttttc cccagctgga 95100
tcctgggatt aaagcacaac tttccaaagt cagcaccaag gcttttacct ttcaatgaat 95160
gaacacatcc agacactggg tttaccccag agaagtgccc ccagcaccac cctcaactgc 95220
ttctcagtca caatgacccc agggcacagc ctccccaaac ggaagcagag agcctgacaa 95280
catatgcacg cttgtgagac ccgctcccca ggaagtgatg gccacaggtg ggaaaatgcc 95340
tccacggcca ctaggagcgg gggaattaca gggacacggc cgtacagcag aacccagggc 95400
cctctgcgca ctgacacgga gcaatcccag gatgcactga cacggagcaa tcccaggatg 95460
tggccactac acttggaagg gcgagtcacc tgcagccacc tgggaggcgg agctgtggcg 95520
caggagttgg ggggggatc cccaacacat gggagagacg aggtgagctc aggtggccaa 95580
acacgtgaat tccaataatt gtatttaact gatggcaagt gatactaaat aatctaaaga 95640
ttaactttta agtaaatcta aataagccaa gttaaaaaat cacaagggga gcaaagcaac 95700
ccgttcccat cagcgccttc cgttttctct ccatggaagc cttcgccagc caggagcaaa 95760
gggcaggatg ctccagcttg aattcctttc tcccccttc cagccctgtg gtagcagcac 95820
aacctcaatg aggaagtggg aaagagctgg gaggcccggg cagcaagtgc ctccccacag 95880
aggggactcc acggtgcagg agtgacctgc tggcgagttc tggccagcca gggtcccagc 95940
accctccccc cggcgctcca gctctggggc cccacttaca tcttcatgta ccgatgcaca 96000
tcggcaggca gggaggaccc gtcaaagaca aagttgtccg ccatcacgtt cagcgccagc 96060
cgcggtcgcc agtgggacac tggctcatcc agggcactcg tcggcttctt ctccgcctcg 96120
atctgctgtt aagtgaggaa aacagaggcc aatgctggga cagaaaacca tgcccaggac 96180
agcagggtca gccccctaac ctgcgaacag aacggccaca cagacaggca aactgtgccc 96240
gggacagcag ggtcagcccc ctaacctgtg aagagatggc cacacagacg ggcaaactgt 96300
gcccgggaca gcagggtcag caccctaacc tgtgaagaga acggccacac agacgggcaa 96360
actgtgcccg ggacagcagg gtcagtgccc taacctgtga acagatggcc acacagactg 96420
gcaaactgtg cccgggacag cagggtcagc gccctaacct gtgaacagat ggccacgcac 96480
atggaaaaac cgcacccgga cagcagggtc agcaccctaa cctgtgaaga gaacggccac 96540
acagacgggc aaactgtgcc cgggacagca gggtcagcgc cctaacctgt gaacagatgg 96600
ccacacagac gggcaaactg tgcccgggac agcagggtca gcgccctaac ctgtgaacag 96660
atggccacgc acatggaaaa accgcgcccg gacagcaggg tcggcaccct aacctgtgaa 96720
gagaacggcc acacagacgg gcaaactgtg ctcgggacag cagggtcagc gccctaacct 96780
gtgaacagat ggccacacag atgggcaaac tgtgcccggg acagcagagt cagcgcccta 96840
acctgtgaac agatggccac gcacatggaa aaaccgcgcc cggacagcag ggtcagcacc 96900
ctaacctgtg aagagaacgg ccacacagac gggcaaactg tgctcgggac agcagggtca 96960
gcgccctaac ctgtgaacag atggccacac agacgggcaa actgtgcccg ggacagcagg 97020
gtcagcgccc taacctgtga acagatggcc acgcacatgg aaaaaccgcg cccggacagc 97080
agggtcggca ccctaacctg tgaacagatg gccacgcaca ggcacaaccg gggagagcc 97140
cagcagagag ggcacagcgg ccccacagct gccaattccg ccagactctc gccaaacagc 97200
cccccatcct tatctccacg gaagccaggg ggtgctgctt tgcaacatgt ggtcacggac 97260

ttcaccaatt ccttttttga ggtcctcaaa ctaagtgggc ccacttctca gaagttgaga 97320
gggtgagagc cccctgacct tggcagaaga ctgtgcaagt ccagggctca cacagccctg 97380
tggctgtaag gagactgaga gggagtcagc ttctaaaacc aagcatgtgg ggctgcctaa 97440
aggagacata ctgaggatta cacacggctc ttacatgaag ttaaacagga catggactga 97500
cagggaaatc ttagctcggc tcagatctga tacatcctag gaagcactat cgcctcaccc 97560
atttcctacc taaagatgca gtttagcgat aatttacatt acaaacggtt ttccacacct 97620
cctcacattt gctacctgca agggaggctt cctgcgctac atgctacatg cccatcaccc 97680
cacccacttt tcaggacact ggagaggagg gatgggggcc ccagatgagg ggacacggag 97740
aggggcaggt gagggatgtt cctaaggaga cagatgcaaa ctggggagag gcaggagcag 97800
gtgttggtag ggctttcaaa accccatcaa ctccgagaga cctctctttc tctctgtctg 97860
gaacaatttc acagcctctt cctggaaagc tgccatcttt cttgttttgt aaatttttct 97920
gaggcagagt ctccctctgt cgcccaggct ggaatgcagt ggcatcatct cacctcacgg 97980
gttcaaacga ttctcctccc tcagcctcct gagtagctgg gattacaggc gcgtgccacc 98040
acatccagct aatttttgta tttttagtag agacggggtt tcaccatgtt ggccaggcta 98100
gtctcgaact cctgactgca ggtgatccac ctgcctcagc ctcccaaagt gctgggatta 98160
caggcatgag ccactgcgcc cggccttgtt ttgtaaatta tctcaccttc cactcaacac 98220
aaaggtgggt gtcagaattc ccttgcttta tccttggcct gtattcatga tcacttaagt 98280
gacttccagt ttttatttgc gcatacacct ttctacagat gtacaaacta caacttgtac 98340
ctctaccttt tccatgatct ccaattacat tcttagaata aacatctagg aatagcccag 98400
tcctacacag aatgcagaat gagcccagct ctcatcccca gagctcctgg tgagaagctg 98460
caggccagct ctgcaggacc agcgctccat cccaccaggt gcagtaggtg cccccagaga 98520
ctgagagggc ccattccccg agggactctg catcgagaat gccagcgatc acttcaactg 98580
tccctaattt tagataaaac ttgtctgcaa actcatggat atgattcagc tgaaagtcag 98640
aaatgcacgc cagtaacatt ccaatggctt ttggctttac tcccaacaaa gtcactggag 98700
ccctagacat cgcccacctg tgtggagaaa gacatgtccg ttctgacgga gaggcacagc 98760
ctgttatcag taacccatga agaccctcac ctgtgtatca gactccccgg tgagcaggtt 98820
gatttcttct ggcttgggga ccatgtaggt ggtcagagga ctgaccaggt gcacctgctt 98880
cccgtcgtgc cacggcagga ccccagcgtg atggaggaag atgtaggcat acagcgtccc 98940
attgtttctc gtttctttg gtacagaaac attaactgtc ctgaaacaga acaatcattt 99000
ccactacata catattatat tctgggtctc tagaacctat tcaaatacct ttataaagct 99060
gcttcaataa actaatttta gctcagaagt actaaaaatg aaacccacct gcccagggct 99120
ttacgagtcg tgtgtgtgtg tgtgtgtgtg tgtgtgtgtg cacgcgcacg cgtgcgcgtc 99180
ctgagaactc ggcacaggtg tgggcgccta cagccgaaag caaaacggcc cgcacttcct 99240
gcctccatct caggaggctg agaggtcaat aacccacctg aggccacaag gctgaagcag 99300
ccagatggaa agcagcttct gggcctggcc agggtaccag ctgaccacac acactgaatc 99360
acccaactcc gaagcgacag aagggaaggg cagcagccac gagggctcca ggtgcctcgg 99420
cccacactac tggaacctca gaaatttaat gtgggtagtt tttttgggtg ttgtagtttt 99480
aaggataaaa agatgatcag ccacaaagga ctgactttca aggctgtcag tcctgactgt 99540
gtggcatcca gcacgagctg aggaaaggcc cggcgagctg ctacaactgt aggcctcgga 99600
ccaggtgcct ggctcttcac ccgcacacca gggcccgacg tccatactac agccccatgg 99660
aaaaacctcg cattccacct gtttacggtt acatgagttc ttcttcctct ttaaaagtct 99720
ctttttgag acaaggtctc gctgtcacca ggctgaaagt gtaggggtgc aatcacagct 99780
cactgcatcc tcaacctcct gagctcaagt gacctcccgc ctcagtctcc cgagtagctg 99840
ggactacagg tgggcgtgcc accatgcctg gctaactttg aaaactgttg tagagatggg 99900
gttttgtcac attgcctagg ctggtcttga actgctgggc tcaagtgatc ctcccgcctt 99960
ggcctcccaa actgctggga ttataggcat gagtcactgt gcccggcctc tccttaaaaa 100020
tcttacggtt ggccgggtgc ggcggctcac gcctgtaatc ccagcacctt gggaggccga 100080
gccgggcaga tcacgaggtc aggagtttca gaccagccca gccaacatgg tgaaaccctg 100140
tctctactaa aaataaaaaa attagccagg cgtggtggcg ggtgcctgta gtctcagcta 100200
ctttggaggc taaggcagga gaatcccttg aacccgggaa ccggaggttg cagtgagcag 100260
agatcacgcc actgcactcc agcctggcaa cagagcaaga ctctatctca aaaaaaaaa 100320
aaaatcttac ggccaaagtt gtaatgaaaa acacaaaggc atctatggct cctcactacc 100380
caggaaagag tatgtctcca ccaggacatc ttaagtgtct ctcttcaggc agagggtgca 100440
gggctgtgta tttctggtgg aatgtgcact tggagacact tgaggcggag ctgtgttaag 100500
agtgctcatt ctccccactt cctccaggct gacactgtgc caggtcaaat ggaaaacctc 100560
actagctgtc cttggtaact ggtacacggc ccggcacgga agaggcagta ttcatgtctg 100620
ggaaattaaa cccagtgcag atgaatcctg agcatttttg aaaggtgtgg gcagctacaa 100680
ccgagcgggc agcttggtca ccagcacttc tggcctcccc ttcatggagg cagagagagg 100740
gcaggaactg gcttgaggtc acaggcctaa agaggccagg cagaaaccgg cttgccagcg 100800
cctccagcta cccccaggga ctggctgcct agagggcgtg gagtccagca tgagtctcaa 100860
actttcatta ttctctcctc agatacctcc caggcaatga agggaaatgg ttgacttgtg 100920
agaagcaaat catatttaaa agtatactta ttcaattaaa aaaaattagg ccatgtcaga 100980
tgtaattttt aagatactct acatatttgg acattgatct attctaacgt cacatgtcac 101040

```
ttatttatga ttttctacaa aattgcttcc tctatcctca tctcttttcc ctcacaagta 101100
acttaagcga actacttcca ccaggaaaag acaagttggt ccccggtagt gaaactgcag 101160
agttgtctgc tctccactgg ttcaccaagc gtggcccctg ctccttcgga gcctccccgt 101220
ccaggacatc ccactgccat cacactctgc tgagaactac ttcttaaacg acattcatca 101280
cctgtcacct tccaggctga atgtttacag gccacgcata ggcctccagt taggatattc 101340
taccacttca agacattcgg taagactagt tctctaaaat aaacatgtta tgtacagaaa 101400
gctaactttt aaacaatctg aacatgagta atgttttccc tttcactggc attttgtcct 101460
acgcccatac ctttcaaatt tggactccac atcaaagtct tccacattca agaccaggtc 101520
gatgttgttc tcagcaccca ggtgggacct cgtcgtggtg tacacgctca gctggaaagg 101580
agggggcgtc gagagtcagc tcggccaggc cctggcagga cgcactcaaa tcccacggcc 101640
agctggaggg cggaagacct ggccgctggg gaaccaggaa aggttccatc tcgactcgcg 101700
cggccacagc tccgaactgg gacgggaagg cgacgtctgg ggcctggagg agaggccccc 101760
accccaaccc gcccggcccc cggccccgcg gacgctcacc tgcagcttgg gccgccgcgc 101820
caggtagggc tggatgcagt tggcgtcgcc ggagcacggg cgggtgtaga cgatgccgta 101880
catgacccag caggtgtgca ccacgtagac cacgaacacg cccaccacca agctggtgaa 101940
ggagctgcgg ccgctccaca tggcggcccc gcgcccggcg cccccggccc gcccgcctct 102000
cagccgcgag ccccgcccgc ccggcgccca gcccgccgct ccgggctccg ccgctcactg 102060
gagagccgcc gcgcgccacc gccaccgccg cgggggaacg aatgcgccgc gcgccgcaga 102120
ccgccggccg ccccgcatgc tccggccccg ctcccacctg gcgccgcggg attcgccggc 102180
cccgcgcgcc gcttccgggc cccgccgcct gccggaagtg gacgcgccgc gcggctccct 102240
gggaaacgga gtctcggccg ctgccgcgca cgcgcatcca ggatgcggcg cgtgtgtgac 102300
gttacccgcc cgcgccgccc tcctggggtg cctctcaggg acccaggcgc tcagctgctg 102360
gggccgcgga gggcgttcgc ggggcgccgg ggaaggctag ggccgcgggg tccccggggc 102420
gccgtcccca accgccgacg cctgccagac caccaagtgg gggccatgtc gacgtgtgag 102480
gagcctggtg gaaggtgata ctggaattcc ggcactgggc ctgcaaccct tttagtccac 102540
agttgtcaga atctaaaggg aagaaaaacg aagtgctcag aaccctcaca gaagccgggc 102600
acctcatccc gtgtgtgggt tttttcccca gtacaaaccc attgtgggga caggcgcttt 102660
tgaagaaaaa attcagattc ccgggctgaa gtggctcctc acagccttga acctgacccc 102720
cagcttcccg ctaaagccga gcgtgcccag cacggcgagg tcagcagagg cccctcggag 102780
ggcggccggg ggaggtcagt gggaggccct cgggcagccg gtggatgggc cggcgacctc 102840
cccgactcca tcagaagcgc tcagtctgtg cccttcagtt actggagcag ctcccgtcct 102900
ttggccagtt ctctggaagg tcgtttccgt ttctgtgtct gccacacgtg cagcacaatc 102960
gaaaaacctt gttcgacatt tctagaaaga gtagatgcta ctgaagaccc aatagattgc 103020
tgagggtttt cagagcaggg acaattgcat cagggtgctg ggctccggga agaactcaga 103080
ggacttggct gacgggcgga tgggacagtg attggtgccg agaatgggcc agagaagcag 103140
gtctgcctgg agcaggcacc cgaggccgcg tgcaggggcg gtcctgggcc accaacccct 103200
ggaagctggg ccaggagctt cggagccatc cgaggctttt ttcgctcctc tcactctcat 103260
gcacctagtt accctcccca cagagtcttc tcacggcgcg gttttcggct accattgcag 103320
tggtgtctga ctacacccag tgcctctatc gttcccccct cagtctgttc tcgcttcttg 103380
gcaaacctgt ccttggaaag ccctctgctg actcctgtta ccctgatcaa atgcatcctc 103440
ctcaggaggc tcgtgagccc tttgcagggt ggcccaagag gcatcacgcc ctgctctcca 103500
gccactcttg gggtccccta cagggccagg cgtcccacac cagcagcggg tgctggtcct 103560
gccacagtct gtgcgacacg tgaatgaagg ctgtagacct gtggtctgcg ggaggcaaat 103620
tccaactcca gctgtcaaac ataaggggac tggtaggctc tccagggtgg attgtctcac 103680
ggcatccaga ggcagggacc gaggcaggga cctagccagg cctcgggaag gagctgcaaa 103740
cccaggagct ctggggtgtg gcttcatctc cctccctccc taccacccta cccccatctc 103800
tccgtgttct tcagatgcct cattttcctg tcaggcacct ggccagctct aggagctgga 103860
atcaggttgg tgcctccttg gtcagtgtcc acccctagtg tcatccacag tgaccaacgg 103920
aaggggtggc cggtggtaca ggacattggc catcttggga aagatcacag agacaggaag 103980
ggcctctcca gctgagcaga tgagaggttc ttcagtggtc tggacacatt tgcccactca 104040
tggccgtccc cctgccctgt tgagagaacc gatgaggaaa gggagccctg catctgtcgc 104100
cttccgactc cgaccgcctg aggctgggct gccggcaagc cgcggcagca tatgacagca 104160
cttgaaagaa agtccctgga aagaatgtgg atcattgcag aactccctca agaaagcgcc 104220
agaggaggac cccattcagc accccgcaga actcctcaaa ctcctgcgtc cacactcccc 104280
cagcgtggtg gctgcttcta tctccccagg cttagctggc ctgggtcttg aaggcctctc 104340
ctcctgctgt ccccacgcag catcctctct gccactgcac agctggatgc agtgaaggcc 104400
aggcggctgt ggctgcttgg gcaacaggag ctgcttgacc ctgttctgct ggccttgttg 104460
gcctcactcc cgtagagagc caaggttacc cacgtctctg aagctaaggc tttctttcta 104520
ggcctggggg agtcacatct tgcattcgta ctcactgagc tccacgtcta tggttagaga 104580
atttatcaac attttataat gcttttatca attccatcat ttgagggccc tctggcaagg 104640
aacctgttag tacatggagt gcctggaatg ttccagaaag ctaacatgaa cagctggccc 104700
ttgctgtcct tatacattga ggactccaaa ggccactcag gatgacagga aatgcaggct 104760
ccaaaagatt cggttccttt gtgaggccct cgggcagtgg tcagacacga aggaaatagg 104820
```

aaatcacccc caaaccacac ctctccctat gaggccacag ctgccgatgc agccttggga 104880
gggggatgga gggagagagg agggcatctg aatgtggaca caccctgctg tgagggctgt 104940
gggcggtcct gagactggag ccagccccca ggctcagctc agctcgggga cctcgtgccc 105000
gggctttcct ggggaggaag gtgttggatc tcctgcctct ctgtattcat tcacactttg 105060
ggtttctctc tcttccgcgc attctaacat ctgcaggatt tttttttttt tttttttttt 105120
tttgagacag agtctcgctc tgttgcccag gctggagtgg agtggtgcga tctcagctcg 105180
ctgcaagctc cgcctcctgg gttcacgcca ttctcgtgcc tcagcctcca gagtaactgg 105240
gaccacaggc gcccgccacg acgcccagct aattttttgt atttttagta gagacggggt 105300
ttcaccgtgt tagccaggat ggtctcgatc tcctgacctt gtgatccgcc cgcctcggcc 105360
tcccaaagtg ctgggattac aggcgtgagc caccgggcct ggcacatctg caggatttat 105420
ggatgaaggg ccacaaaccc acatttggtc tcattcaaca gatgttgtgt cggcagggcg 105480
aggtgggact ctgtctctgc ccacgctacc tgcccctggg gttgtagttc taggaaagca 105540
agtctcagac tgagttttga tctggattaa attctgtccc aggtgcagat atttatagga 105600
aagcagggac aagagccctc aggcaaagct gggtatcctg cgatgggtgc ctctgggagt 105660
tcgcagataa caaggccttt tcctggaggc aaagcagaag cgttctgtgg cagaccagtc 105720
agctttctag caggtcaggg gcagggacat ctaggcccaa ggtgtcacac cgtgcctggc 105780
tgctggctct cctcttgcaa ggacacagct cttacaggac ttcctagccc acccaagaac 105840
atctttccat catcgaaaag ttattgataa gatttttcca tcactgaaaa gttattgata 105900
agtgccacca tgtgcatgag cagccctcca ggtgctggct tgtgggccgg tgtttgagaa 105960
tcttgccagt tgaatgtggg catctgttga gggtttccca aaggctaacc gttcgtgcca 106020
gagggaggcc gtggctcatt cctagggccc tgggtgagac tggggctcat gcacacacag 106080
gtatcgatgg catgggctgt aacgagctga gtgcctgctg ccctttccta gtgggggggg 106140
tgggctcctg gagaaagagg ggaggctgct actctccagg gccacaggga gcccagaacg 106200
ccacctcctg gtgtcagcag cagcaaatcc atataagtct gcagcaactt agcttttgcc 106260
tcctcagagg aaataattca tccaggggc acaaggcagg gtcagagact gaggtaagtt 106320
tcagagcaag agtgaaagtt tattaaaaaa gtgttagagc aggaacgaaa ggaaataaag 106380
tacacttgga ggagggccag gtgggcatct tgagagagca agtacacggt tttgaccttt 106440
gacttggggt ttatatcttg gcatgcttct gggggctgcg tcccttctcc cctgattctt 106500
cccttggggt gggctgtccg catgcgcagt ggcctgccga cacttgggag ggccgcgtgc 106560
acagtgtgct tactggagtt gtgcggtgcc ctcttgaggc agtcttccct taccagttcc 106620
taggggaagg tcacacgctg gttaaacttt gccactttgc ctcgtagtgt gcatgcttga 106680
cctcactcac caactcctga gatttttttt aaatttttta atttaagttc tagggtacat 106740
gtacacaacg tgcaggtttg ttacataggt atgcatgtgc catgttggtt tgctgcatcc 106800
attaactcgt catttacatt aggtatttct cctaatgcta tccttcccac cccacgacag 106860
gccctggtgt gtgatgttgc ccgcactgtg tccaagtgtt ctcattgttc aattcccacc 106920
tgtgccaact cctgggatct tatcgggaag tggctcatca tcagctttag gtgttttcta 106980
tctattggga gcctgccttt ctctggcacc agctgcaacc aataattatt ttagacagtt 107040
taacaacagc ttgaccatca cctgatgatc acctgacatt tctgttgggg cgcgggccca 107100
tctcctgccc cgctcatgtg gggctcagtc tgcccaacct ttccccaagg gcctgtcttt 107160
acttcatttg tggtaagccc caagcagaat ggactcttgt tatgatccca ggaactgctc 107220
acggccacct ctgtggatga acacctagcg gctgctctaa atacatgctg tgaaagttgt 107280
cagaatcaaa atggagtcac taatgttaag aaagccctga caaagagttg gtggggaagg 107340
ccacgaagag aggacactta tgcttgcatg cctgatacca gaaaggacta caaaaaccac 107400
agcccggcac agaggccatc acacccttac acaaaaaata tttctgcaag gacaactgcc 107460
cagcaattgc ctgtccaacc tcagactgga atgacctttg ttattgatgt ttgtagccaa 107520
ggagaattat ctcaaaacca ctgtgatcct gctcgctttt cctttaaaga cctttgtctt 107580
ccttgacctc cctgaatagg catatggttt actatggcgt gtgtactctc cttgtaatgt 107640
tctgttctca agctaacatc tattcttcta gagaacctct ctcttaggct gacaacgctg 107700
tggatctggg ctcccaggaa ctggggctga catcagaggg gtggaaagag aacagcagct 107760
ctccctgagg ggatgcttgc caaggataag gagacgatgg ttctctaagg attattttat 107820
attcatctaa aagggtattt ttgttgttac ccctagcctt ttttcccttt taattttctg 107880
atgaataatt tatataaaga aaacccattc taagtgtgca attctatgag ttttgacaaa 107940
tgtcttcacc catgcaaatc ctgttacaac ggagatatag aacatttcca ttacttaaaa 108000
acagccaggt gctgtagctc atgcctgtaa tcccagcact ttgggaggtc aaggcgagag 108060
gattacttga gcccaggagt tcaagaccag cctgggcaac atagtgatgc cccatctcta 108120
caaaaataga agaaattagc tgggcatggt ggtacatgcc tgtgatccca gctacttggg 108180
aggctgaggt aagaggattg cttgagcctg ggaggtggag gctgcagtaa gccacaatct 108240
caccactgca ctccagcctg ggtgacagtc agaccctgac tccaaacaaa aaccaaaact 108300
tccttcttgc ccctttgcag ccagactgtt ttctgtcatt gtggatgcat ctgttctaga 108360
aatttacatg gatggaatca ctcagtcttt tgtatctggc ttcttttact tggcacagtg 108420
tttgtgaaac tcatcattgt tccacataac agtagttcct tttaattgcc aagtggcatt 108480
ccttggtgtg aatgcaccac agctgggcta gccacccaga tgctgatggg cagtcgatag 108540
gcggtaggat gtccccattt tcagtcacta tgaattggtc tgctgtaagc actagagtag 108600

aaattttttt ttagagacag ggtctcgcac cgtcatccag gctggagggc agtggctcat 108660
agctcactgc agcctcaaac tcctgagctc gggcaatcct cccacctcag cctcccaagt 108720
aactgaaact acaggcacac accactacac ttggctaatt tttaaacttt ttgtagagac 108780
ggggtcttgc tgtgttgccc aggctggtct tgaactcctg ggcacaagca atcctcccac 108840
ctctcaagta gctgtaatag gtgtaagcca ctgcccagca ccatttattg aagaatctgt 108900
cctttccaca gtgagtgtgc ttagcacctt tgttggaaat cagtcggctg tagaaagcgg 108960
attaatttct gggccctctg ttctgttcca ttggtctgtg tgtctgtttt tatgccagtt 109020
ccatgctgtt ttggttactg tagctttgta gtgtattttg agtctgggag tgtgatgcct 109080
ccagctttgt tctttttgtt caggatggct ttggctgttt ggggtctttt gtggttccat 109140
acaaatttta atattatttc tttttctaaa aagaatgcca ttggtatttt gataggcatt 109200
gccttaaatg tgtagattac cttgggtagc acagtcgttt taacaatatt agttcttcca 109260
atccttgagc atgagatgtc ttctcatttg ttcatatcct cttcagtttc ttgcgtcagt 109320
tttttgtagt tttccttgta gaggtctttc acctcttttg ttaaatttat ccctaggtat 109380
tttatattat ttgttgctat tggaaatagg attgccttct tgatctcttt ttcagctagg 109440
tttttgtttg tgtatagaaa tgctactgat ttttgtatat tgccaggggc agcctgtggg 109500
actttttctt aggcccttat tggaggcaca gagccattgg gcaggccagg ggcgtacctg 109560
caggtggggg tgccgtgggg ctgattttca ggccctgagc tcctgctgtc agttgcttgg 109620
aggctggtgg ggcctatgtg gggagacctg cagctgtttg gctcaagggt gggtttgctg 109680
caggtgggag gagcagacag ctggaagagc tgcggtgggg atgggtttcc ctgctgtgca 109740
ggaccagagt cacagctaag cctgggccca agctctatgc agctggggtt gtggtgttca 109800
gctgcccatg tgggcttggt ggcatgaaag tggagcccca gtgctggaga ggtgcaggtg 109860
ctactggccc cagggcagag cccctccaga tgcaatagca gcttggctca tggcatgggt 109920
gtggggtggg aggtgcacac cttttgctcc taatcggggg aacactgctg tgagaattcc 109980
ctgcagtgct cccaactggg ctcagggctt gcaaggactg tgggttctcc tgcagcaagg 110040
actgccagca tttgctgtag cagtggcagc tggtggagat ctgcttacct tttccttgcc 110100
actggaagtc cctctttgct tctaggtcaa actggtgggt gaggaagact gggcagcaga 110160
cgccacctgg gcttccgagc accacagggg tgtctccggc ctctgctgca gtccagcact 110220
ctgccttcga cgctccagtc aaattgtcac tgtcaacttg ttgccttgat ccttctttgt 110280
ggggaggagg ggatgagcgc cagacagttc tagtcagccc tgttgccaac accactctcc 110340
ttctggtgac tttcaaagag aaaagttgaa catttttact acacccaacg taatcatgtt 110400
tcagtgataa gagtttttga attttgctgt atgtgttttg aatttctgtt agtaggagca 110460
tactcatttg gggtcattgt gtcctcttga tgtcttgacc ctgttgtctt tgtttatccc 110520
tggtgatgtt ctttgttctg acatctgctt tgtgtcgtgt caacataact acttcagctt 110580
tcttttgaca gcgcttccct ggcatgccct tttctctcct ttaactttta acctacctgt 110640
gttttaaata tacatatgta tctatctgtc tatctacaca tttttttttg agatggagtt 110700
ttgctcttgt cacccaggct ggagtgcaat ggcactatct aactctcact gcaacctgtg 110760
cctcttaggt tcaagtgatt ctcctgcctc agcctcccga gtagctggga ttataggcac 110820
ctgccaccat gcccagctaa ttttgtagtt ttagtaaagt caggttttca ccatgttggc 110880
cagggtggtc tcgaactcct gacctcaggt gatccacctg ccttggcctc ccaaagtgct 110940
gggattatag gtgtgagcca ccacgcccag cctattttct tttttttaag taaaattttg 111000
agacagggtt ttgctctatc acccaggctg gactggagtg cagtggtgcc atcttggctc 111060
actgcagcgt caccctccca ggctcaagca atcctcccac ctcagcctcc caagtagctg 111120
ggaccacagg ggttcaccac cacacccagg caatatttgt atttcttata gagatggagt 111180
tccaccctgt ttcccaggct ggtcttgaac tcctggactc aagcaatcct cccacctcag 111240
cctcccaaag tgctgagatt acaggcatga gccaccatgc ttggccttaa cctatctgtg 111300
tatttatatt taaaacgggt ttcttgtagg cggcttagag ttagttctgt atttcaatca 111360
acaatctctg ccatttaatt agagtgttac atcatttaaa tttaattatc aatgtttctt 111420
tatttgtcct attcattctt tgttcttttt cctctttttt cttcctgctt ttagaattat 111480
aaatttatct tttttggatt caaatatgtc ttcttattgg cttttcagct agatcttttt 111540
gctttacttg ttcagtgttt ttttctacaa ttgtcaattt gcagcttaaa cttaagctag 111600
tctacttcca gatattataa cccttcatgt ggattttaat gactttaaac agtaacgttc 111660
catttctgtt ctattccttt ctttattgtc atacatttca ctgtcacgtg aaatacagct 111720
gatatatacc ctttagcctc aataacttcc tttagcattt tgtgttatgc agatctactg 111780
aaaatggatt ctaccaattg tcatttgtat ggaaatatcc ttaatttgcc ctcgtttttt 111840
gaaagatatt ttcactaatt ctctaggttg atgggcactt tttcttctgt catgttaaat 111900
cacatcgttc agtccactgt cttctgactt gcattgcttc tgatgacaga ttggcagctg 111960
ttcttctgtg tttctttgtg ttttgtgtgt atttgtctgg cgcttttgcc ttgtctgtcc 112020
tgcttggggc ttactgagct tatcaatctg tgagattcaa gtctgaaaat gttgcagtga 112080
aggtttcttc aaatatttct ctgcctgcct cttccttttg ggactccagt tatatgtata 112140
tgagctgctt gatgctgttt caatgtcagt gaggttctgt ttatgttgaa acttttctcc 112200
cgtctccgtg cttaggtttg aatactttct aatgcttgta ttcaagttca ctggtatttt 112260
tctgcagttt tcaaactgct gctaagctca tattatgaat ttttcatttc aaaggtattt 112320
ttcatctcag aggttccatt tagttatata tatacataat atattttgcc aggatatatc 112380

ctttggttaa aaaaaaagtc tgtaggtatt aaaagcaggg ttacgttgaa tgaccttgaa 112440
catccatttt ctgatatttt gtgactttaa ggtagctgct tagaagtgga attgaaggat 112500
gcagttgtga atgcacacag gtctttccgg gcatctctga gcttcttccc gcaggggctg 112560
tgctgtttgc atcccagcag caaagcatga ggttgtgtgt ttccctgcag cctccccatc 112620
aaggtgtgct ggtgaccttg tggagttttg ccaacctgtt aggagagatg ttgtaactca 112680
gtgttttcat ttctaattct cggatcatga aaactaacag gcttggcttg gtgtggtggc 112740
tcatacctgt aatcccagca ctttaggagg ccaaggcggg tgtaacacct gaggtcagga 112800
gttcaagacc agcctggcca gcatggtgaa accccgtctc tactaaaaat acaaaaaatt 112860
agccgggcgt gatggtaggc gcctgtaatc ccagctactc aggagactga ggcaggagaa 112920
ttgcctgaac ctggaatgtg gaagttgcag tgagccgaga tcgtgccact gcactccagc 112980
ctgggcaaga gagagagaga ctccatctca aaaaaaaaaa gaaaaaagaa aacaacgggc 113040
ttatttacag caggagctgt ggtgcccttg ggggatgtgt gagctccagg gatgacagca 113100
accaccatct gttcatctca gcacttggga gacaagccct gtaggattca ttcagtaaaa 113160
ataaaatcct acgaagtgaa tttgtaggct tggggaagtg tggacaatgc agtttgggca 113220
tcacctacgt acaagcagaa ttcgcaggaa cagctcctgg gcattgggat gaaacatggc 113280
ccctgccatc tgcttcttgg tgtcaggccg gcctcctgct tcccgccttt gttgccagcg 113340
tggaggaagc agaggtcagg gaggcagtca ctgcttgtcc ctgacacggg actcagtctc 113400
cagacaaggc ggctgctctc agtgtcctgt ctcatgactc caggaaggcc cctccctgta 113460
gagtgtcagc aagtgcccac gacccaccca cagagcagct gagcagtgcc tagcgcgcag 113520
ggcaggccac acggcgccac catcagagca cgagaacaga agggctgggg gccgtgggag 113580
cctgggcggg ccagggtaac gaagagcccg ccgtcggtct ctgcatccgt cggctcacgt 113640
ggccacggag cagcaccgtg ttacagcaaa gcggtcctga tccagaaccc aagagagggt 113700
tcttggatct cgcacaagat agaattcagg gggagtccgc agtcccatgt ggaagtgagt 113760
ttattaagag agtaaagtgg cgaaaggacg gctgctccac agacagagca gggcattccc 113820
gaaactaaga ggagaaaggc gcccacctgg ggtacaatcc tggtgtatac ggggagatgt 113880
gctctgctac gagcgttgat gataaagaat taattttctt aattagtata ttttgcaaga 113940
atcaatatca tttatcttta aggcaaaatt aggaatgcct ttgttctctg gatatcgggg 114000
tatctggact cccaagtctg ggtctgttta gtaaacatta tttatttgtt cctttatctc 114060
taaacgtcta gaggctggga atgcccgact ctctgggagt ggagcctagc aagtcctagc 114120
ctcgttttcc agccctcact cgaggtggag tcgctctggt tccagcgcct ctgacgaccg 114180
caggctgggg cttagacagg acatttattg ctcaaagtac agtccatgag gctggcggtc 114240
ccagatccag gtgtggcagg gctacttcct cctgaggcct ctccttggct tccagacacc 114300
gtcttctccc tgagtcctca ccgggtcgcc cctctctgcg tgtccgcctc ctgatctcct 114360
gttcttacaa ggacaccagt cacaacgggc ccaccctctt gatctcattt aactgaaatc 114420
ctttctgtaa agaccccatc tccaaatacc gtcacattct gaggtcctgg gggtcaggac 114480
ttgaacattt aaacttggag gcaggcacag tcccacccat cacagcccat tgtcctccac 114540
agccctccaa tctcacttcg agtcttcccc aagggtcagc acaggctctg cccttgtgag 114600
cttcatggga atgagggtca agggggtaat gagggccgga agtgatcgga gggaggcggg 114660
aagagggagg acttggactc cgcccacggg gtccccacct gggcatggct gtagggaggg 114720
gcatcgtgct gctcttgtcc ttactggggg ccagtgtctg aaggaggaag ggatgggtgg 114780
caggcactgc ggggaggaga gggctctcca gcactcatat tttcatcctt tgaccgtgct 114840
gcctgggcac tgagcatagg aggatgtcgc tggctggcag ggctgtcacc ctcatcgatg 114900
gcccagtttc ccaggaggaa atggatggga tggcaggttt cagcagagag cacacaagcc 114960
ctgctattat cttacaaaaa gccaggccag cctcccacag ctctccatgt gacttagcat 115020
caatattggg ggttttgtag ttacctcagg aaacctactt ttgaacaaat aatagattat 115080
gttctctgga acacaggggg agctaaatgc cttttgacag ccagcagctt atggaaatag 115140
cccttttcct ggcagaagaa gtcggttccc cacggccgcg cccgctgctt catccagctt 115200
cccctcgctc tgtgccgacg ggtgcccgga gttcagcccc tggcctgact gttgctgtca 115260
ctgccccctt gaccactgca atggtgacag ctgtgatgta cgattacaaa gcgggacaca 115320
gagccctccc agagcaaggc agctcccctg tgggccaagc ggaccaggct actgggggac 115380
cctgggctcc tgggacatgg ggtgcgggtg cagacagggc ctgaagtgct cctaggtgca 115440
tgccagtggc aggaagcccc atgtcacaga gatgtggccc aagcgcttga caagagacgg 115500
cgtcagatga tgtaaaagaa accaatggac ctaagtgggt gccatcctga gccccgctgg 115560
tggattaact cagttccatg cccacgactg cccgaggtgg agcgatggtc atgcccactg 115620
cacagatgag gagccgaggc caggaagggt tttgccagat gcctgggctg gggccagggc 115680
tcaggaccac ccactgaact gcctgctcgg cccaccctgg caagtgtgtg caagggcccg 115740
gtggtgccga cgaggagggc catggggagg agatgttgtt gtcctgagac tcccagcccc 115800
acctgagggg gaagagggtg ggagagcaag gctgggagcc acccttgggg gctgtgcatg 115860
tgccccctga cattggagga cacaggccac gccacacctg tgccacccag ggagtgggaa 115920
ggaagcacgt ggccgtggag aggccagcag gtggcaggaa gggctgcaag cccccaacca 115980
cggggtcaca cgtaggggac ccagcacccc atgcaggagc tgggctgtgc cctgcatctg 116040
cacaggccgg ggcatgaact gggcatcagc accgccctcc catggggacg aggacccatg 116100
cccgcgtggt gcaggagctg tgcactgagc agccccatgg gcaactccag cccgcagtgc 116160

caggagcagg gccaggcctt gtggcagggg tgcaggctgg ctcctggcgg tcgctcagct 116220
ctcggggata ggtgaggacg cgtggagagg ggatagcatg ggcatcaagg ccgaggcact 116280
ggcccctccc agaatggcct ccaggaccac cctgcctgcc cccgggtgcc ccgctgtgcc 116340
tggggagggt aagggtcact tcgggttcac ggctccaggg tcgccgtctt gaaattctta 116400
ccaattttat cactaagctt atgtttttca agcaagatag gatgggcatg gagcccctgc 116460
aagagcaagc cacgggcgcc ctccgagccc atcgccgctc agaacctgaa cagagcacat 116520
aagcaaggct cacggcgacc acagggctgg tcgcagccag gacggggtga ggagctgcag 116580
ccccagcctc gccttccctt caagccgagc agcttccagt gggaggaaac gccagcgacc 116640
gggcccccgt ccttcctcct caggcggtcc tgtggaaggt gcggtgctga tggaacgtgg 116700
tgactgggag gtggcataaa agcagccact gtgctggttt tgtgggacct ctcctccttt 116760
ctggggtcc ctgaggacag gtgcatgtgc gaactgtgaa acagaaactg agatttcacc 116820
ttccggatga gttccatgct ttcttttttg catttaaaac cggacaatgt cacattggca 116880
aaaatctaga gttttctgcc gtcttagctg gaaatgagct gcaagttttt ctcaagatgt 116940
agtgtgtaat ccgtcagagc aaaacacgga gagcccttag cagaagccca cttcaatgta 117000
ttttcttcat atccctgaag ttccttaaaa ataggtgacg atgtattggg aagaggagaa 117060
ctgagaagtt cccttgcagg ttttgtatca gtgacatgta aatgagcaat tcacagatga 117120
gcgcgggcac agctctgtgt gctgcgtaca tacgggccgg gctatgatgt ctcacactgg 117180
atgatattcc accttcggaa ttttagtgtt tgaatacaga aaatgggttt aataactcac 117240
tgtggttttg atttatctta tattcatcat ttcttaaaac tcatttctta taaaatttaa 117300
agtaaaaaaa ataaaactag acaatatatg aatggcagat ccatgctaat gattctaatt 117360
ttcagaattt ctttacttag aatgacataa aatagcaact aaaaaataag ttgagaaact 117420
gtggaaaaag aaaaagctag gccgggcacg gtggctcatg cctgtaatcc cagcactttg 117480
agaggccaag gcgggcagat cacctgaggt caggagttca agaccagcct ggccaacatg 117540
gcgaaacccc gtttctacta aaaatactaa aattagctgg gcttggtggc tcatgcctgt 117600
agtcccagct acctgggagg ctgaggcagg agaatcgctt gaacccagga gggagaagtt 117660
gcggtgagct gagatggtga cattgcagtc cagcctgggc gacagagcaa gactctgata 117720
aaaaagaaga agttgtatat tttactacct tgcacagcac tttcccctgc tttttaatga 117780
ggcactccac attttcactt tgcacagtgc cctgagtggg ccagatggag ggacccttct 117840
tctggggtct caccctcatg tggctgctgg tccacacggg ccctgacagt ggctctcggg 117900
gtgatcacag cctgaggctc ctcctttctc aggccctggt cactcaaagg attcccggcg 117960
gggtccctcc agtgaacacc cagctcctgc ttcagagcat gaggggtgtg agttttccaa 118020
aggtaccagc tttggacaga tggggtgaga tggtggccaa ggggtccctg caggggcccc 118080
tcatggtcct ggggacaccc tgactcttct ggccgggtgg agcacagccc cagctacctt 118140
tgcactctcc aaggtcccag gggcacccag gctactgagt gtcccagaca tccacacgca 118200
gggattgggg gaccagaagc tggggagccc taggggtttt gagggcagag ggcaggagga 118260
gggtgtggtt aatggtgtcc aatgcaggtg ctaagccaca ggaggagtgg gctgtggcgt 118320
ggacgatgca ggtagctgtc agcatcaatg agatgtgcag tgggctcatt gggggggtggg 118380
gttttttaag aaaaaagcct cggggtgtgg ctgctggtgg gtcgccctgt cagagaagag 118440
aaagctggag aggacacagg gctggggtgg agggcaggtc tgcgggttcc agggggcaca 118500
ctggagggga cactcagggg ctggaggctg tcttgggttg ggactgaggc tggtgtgggg 118560
tcttctgcag gctgggactc atggtggggc tgggttcagg ggtgggaagt tggaggggga 118620
tgctgggaat atctgctgtg acagtagaaa atgtacattc aggattagcc tagtggctca 118680
cgcctgtaat cccagcactt tgggaggccg agatgggtgg atcacttgag gccaggagtt 118740
tgagaccagc ctggccaaca tggtgatacc ccgtctctac taaaaaataa taaaatcagc 118800
tgagcatggt ggcacgcgcc tgtaatccca actactccga aagctgaagc aggagaattg 118860
cttgaacctg ggcggcagaa gttgtagtga gctgagatcg caccattgca ctccagcctg 118920
tgcaacagag caacaccccg tctcaaaaaa tatatatata tttattctat tttgtcccca 118980
gttcctgaca cagtgcttct tggaatgtcc tgcatggtag gaggtgagaa ttcataacaa 119040
gcccctttca acctctcctg agcttatgct atgaggggac tcttggtagg cccctagata 119100
gcttgagggt ggtgctggtt gctaggggaa ccagccgtgt gattgaaggc tgaaactttc 119160
agccccattt ctcaatctct ggggccaggg gttggagatg gaggccaacg aaaaatcacc 119220
agtgatgtca ccaattgtgt ccacatgaca cctccatcag aaaccctcgc ccatgggatt 119280
tggagagctt cagagttggt gaacacgtcc gcgtgtggga gggggcacac cccaagtcca 119340
tggggacaat ggaatgtcct gtgcccggga ccccaccagc cctcgcccca ggcacctcca 119400
catctggatg ttcctgcgta ttcttcacaa tatcttctcc aagaaagcag taacggtaag 119460
caaagtgttt ccctgacttc cacgagccat cccagcaaat tactgaattt aaggagggga 119520
tggtgggccc atttgcagcc acgttggaca gacatgtggg tggcctgggg acacgatgct 119580
tgtgactggc tgagtggggg cagcctcacg ggcaaagctt tcaaccttcc ggatctgcgc 119640
tagcgccggg cagtgttgga gctgaattaa attgcaggac actcgggtgg tgtctgcaga 119700
gacccggaga attggctggt gtgcaaaacc cacacgcttg ttgtcagccg tgtcgtgaat 119760
cgaggaacag ctttccttta tccacaaaaa aagcgagttt tcattcccta ttcttaagtg 119820
cgtggctcca gcttttgcaa taattctagt ccttatggtg aggagggtaa tgtgagcgag 119880
gctgagaaag ctccggagga gtccacacgg ccatcctctt tctagttcag caacaagagc 119940

caaaatcagg tttccacgga aagtgactgg cgttggctct ccatagggaa atgaaagtaa 120000
aagaagcagt ttggccaggc gcagtggctc acacctgtaa tcccagcact tcgggaggct 120060
gaggagggcg aatcacctga agtcaggagt ttgagaccag cctggtcaac acggggggaaa 120120
ccctgtctct actaaaaata caaaaattag ccaggcatgg tggcgggtgc ctgtagtccc 120180
agctactcgg aaggctgagg caggagagtc actggaactg tggaggtgga ggttgcaagt 120240
gagccgagat cacgccactg cactccagcc tgggcgacag agcaagactc tgtctcaaaa 120300
gaaaaaaaaa gcagcagttt gggccatgca gtgagcgctc tgtggaccag tggactcatc 120360
tgctggggtc caggcagcct gggctgctcc tggttcttcc ctcctccctg catgcacggc 120420
cctgcactgc cctccctggg gctgctgtgg gagttctcgg ggctctcggt ggttgtcccg 120480
ctggctggga agggctgtgg tgtgttgggt gctgcctacc ctgaagtaca cgaattaggg 120540
ttgtgctgtg ttgggtgctg ccctccccag gggctgctgt ggtgtggtgc tgggtgctgg 120600
ctgccctcaa gtacatgaat taaaagttgc ttctcctctt gccagatgca gggaggaggc 120660
tgagcaaagc cctagctcat ttacgtagag acagacacac agaatttttg caggaattta 120720
atgcactctg atttttccgg agatctaaca atagcgcaca tcaaagtggg tggcactggg 120780
ctctgttcag aacattccag caactggtca ctgcagcagc gcactcacat ggcggtgggc 120840
gctcgggctg acctcacctt ctggaaggtg cacctgtgtg cgcaggcatc cgagtccatg 120900
tcacgtgctg gcacggcaat cgccatcctt tcacaacagg tcacgaagcc gactgatttt 120960
gaatggctgt gtgggtaata ctgcccatga ccttcattcc agagagcaac aggcatcagg 121020
aaacgtctgt cacaacaggc agggctggag cttgacacgg ttgagaatcc ctgatgctca 121080
acccgtattt tctggctacc agttgaaaga aagggtgaga ggatgcttgg ctccgagcaa 121140
gtggctgaga aacgcagcca taacattcag ctccttctca ccaaattctt taccccaaag 121200
aaccctggga ggaagacaca tttgctagga gctgggtacc tgaaatcaag gtaaggaaag 121260
gatgccaggt tctctcctcc aaatggcgct ctgggcgtct cccctacaat cagagaggaa 121320
cgaaacttac acgaacctta aacttctgtt agaggtaaaa tagaataata ataataaatc 121380
ccagttcagt gaattcaggt taaccgcaaa gctcatcggt ttggagagac gagctctgac 121440
ctttgtgaaa ttttgtgcac aaacaacctg gaacactgct gtgagagaga gggctgtgtc 121500
ctggccagtg tcggccccac ctgcaggaag tgcttatctg tttcatcggt gaggtttgct 121560
ggctgggctt ggggaataat gggttcccac tttctcaatt gtctccaggg tttggaacac 121620
agggctctgc gcctctttct tcacttgctg ttccacagag aaggtgaccc gagctgcaca 121680
gtccaaaccg aaggacactt agtccccaac tggatgaaac agggaagaag cgaaagcatc 121740
ttcatctatc ggtctcagta tttgaaattt tgctttctag ataggcagac caacggaata 121800
gaagataaag tctacaaata gaaccaaata tatgtagaga ttaggtatac aatgaggtgg 121860
aaaaagacgg acttttgaca ataagttttt gggacagttg gatagccaca tagaaaatga 121920
tacaactggc caggcacagt ggctcacacc tgtaatccca gcactttggg aggccgagga 121980
gggcagatca cgaggtcaag aattcgagac cagcctggtc aatgtggcga aacccataca 122040
tctactaaaa atacaaaagt ggccaggcgt ggtggctcat gcctgtaatc ccagcacttt 122100
gggagctgag gtgggcagat cacgaggcca ggagtttgag accatcctgg ccaacacagt 122160
gaaaccccat ttctactaaa aatacaaaaa ttagccgggt gtggtggcgg gtgcctgcaa 122220
tcctagctac ttgggaggct gaggcaggag aattgcttga acctgggaag cggaggttgc 122280
agtgagctga gatcgtgcca ctgcactcca gcctgagtga cagagcaaga ctatctcgaa 122340
aaaaaataaa agaaaactaa aagatacaac ttaaccttta gcttattttc cacaccagaa 122400
taatccccaa agaccagaga tcttcatgcg agaaggaagt aaggtctggc tgaaaacaca 122460
gctgcaggat gtcacaggag aggaaaaggc tttctaacca tatcttataa tttagaagaa 122520
caaatacaag attgccaaaa tttatcactt aaaataatgt atactcaggt ctggcacggt 122580
ggctcacacc tataatccca gcattttggg aggccggggc aggaggatca cctgaggtca 122640
gcagattgag accagcctgg ccaacattgt gaaaccgtat ctctactaaa aatacaaaaa 122700
ttagccaggc ctggtggcac acgcctgtaa ccccagctac tagcgaggct gaggcaggag 122760
aattgcatga atccgggagg tggaggttgc agtaagccga gatcgcatca ctgcactcca 122820
ggagcatctc aaatatgtgt gtgtgtgtgt gcgcgcgtgt gtgtgtgtgt gtgtgtgtat 122880
actcaatcaa aatcctagca agttattttg tggctattga gaaatgattc tgaagtttac 122940
atagagagag gaaagaccca gcacaaccaa cacaaaattg aagaacaaag tcacgggact 123000
catgctgccc ggctcctagg ctcctgatga agctgcacta atcaaagcag ggtggtgaat 123060
gaaagatgga ggaggggca ggtagggggt ggataagggg gcgggtaggg ggcggataag 123120
ggggcaggtg gggcggataa gcggggggtg ggtagggggc gggtaagggg gcagagtgca 123180
gaacccagaa acagacccat gtggatgcag tcagaggagc aaagccaatt ccatgggaca 123240
aagagtcttt tcaacaaatg gtgctgggac aacaggacac ccatgtgcag gaaagcaaat 123300
ccagacacag acttcccacc cttcacaaaa acgaactcaa aatggatcag acctcaatgt 123360
aaaacacaaa actataacgc tcctagaaga taacatagaa taaaacccag atggccttgg 123420
gcatgagatg acgttttaga ttcaacattc aacatgcctg ttggatctaa aaggcatgat 123480
ccatgagaga aataatggat aagttggact tcattaatgt gagaaacttc tgctctgcaa 123540
gagactctgt caagagaaga caagccacag actgggagaa aatacttgca aaagacttat 123600
ctgatagagg acagtactga aaatatgcaa aaaactctta aaactcaaca ataacaaatg 123660
gacaatagat tttaaaagtg agcaaaatac tggaacgtaa atctcaccga agaagttctg 123720

cacatggcaa tacgtgtgtg agaatgttca gcgtcacgca tcatcggaga aatgcaaatt 123780
acaccaacac tgagacacca cacacaccta ctagcatggc caaaacccag ggactggcaa 123840
catcaaatgc tgacaaggat gcggaacagc aggaactctt gttcatcgct ggtgggaatg 123900
caaaatgggg ccgccgctgt ggaagacagt ttggcagttt ctcacaaaac tagacatgat 123960
ctcaccgtac ggtccagtaa tcatattcct cggtatacac ccaaagggac tgaaaacttc 124020
tgtccgcaca aaacctgtac atgggtgttt atagcagttt tattcataag tttcaaaacc 124080
tggaagcaac cgagatgccc ttcagtaggt gaatgggtaa gcaagctgtg gcacatccag 124140
acagtggact cagcactaaa aggaaatgag ctcccaaact atgaaaaggc agggaaagaa 124200
cttaaatact tatcactgag tgaaagtggc caatgtgaaa aggctacacc ctgtggggtt 124260
ccaactctag gacatttggg aaacggcaaa accatggaga tgttaaatag atcagtggtt 124320
gtcagaggct gggggggagg aaggttgaac aagaggagca tagaggattt ttagggcagt 124380
gaaactcctc tatgtgatgc tataattgta gacacgtgcc gttagatatt tgtccaaacc 124440
cttagagagt acagcaccga gactgagcct gaatgtaggc ttcaggcgct gggtgatgac 124500
gacaggggtc agtgcaggag aatcagctgt aacacatgca cctctggtgg ggaagttcat 124560
cttggggagg ctgtagttgg ggaggggggc tataggaaat ctctgtacct tcgtcttaat 124620
tttactgtga acctaaaact gctctaaaaa ataaaacatt aaaaatgaaa tatacaggct 124680
gggcacagtg gctcacgcct gttaatacca gcactttggg aggccaagga gggtggatca 124740
cctgaggtgg gagttcaaga ccagcctggc caacatggtg aaaccccgtc tctactaaaa 124800
atataaaaat tagctgggcg tggtggcgca tgcctgtaat cccagctact cgggaggctg 124860
aggcaggaga attgtttgaa cccaagaggc agaagttgtg gtgagccaag atggtgccat 124920
tgcactccag cctgggcaac aagagcgaaa ctccgtctca aaaaaaaaaa ccaaaaaaca 124980
aaaaaaacaa aacaaaacaa aaaaccaaaa aatacacatt tttgcatgaa caaaagccca 125040
tgaagaaaat caaaggtgta gcacatgggg aaatgaaagc catagcacat gagaaaatgc 125100
ttccaactta tcgcacaaag gatcaagctc tgcaaactat aacaagctcc taaaaatcaa 125160
ggcaaaaacc caattatctg ttggaaaaat gggcagaaaa agaaaggcaa tgggcagaaa 125220
aagaaaggca atgggctctc ccctgtgaag acacactcag cctcacttac agtaaaagtg 125280
caccacaccc caggtcctct gcacgccatt tcccacccat cagatagtcc gagaggcaga 125340
cttccaacag cgcctctgca tccccaagga gatgggcggc tcatggggca ctgctgcctg 125400
ggaggggagg tggcttttgt tggagggcct cccacttacc cagctacagc aacaaatgtg 125460
acatctgtcc ctgagatggc cagacacgca gcaagtgctc catgctgttc actgtggcat 125520
cacatgtagt ggcaaaggtt tggaaacacc caaatatcaa agagcagaag gtttaataaa 125580
taactgagac acacccacaa aatgcatctg taaagaagaa taaaaaaggt atctctgtgc 125640
tgataggcag ttatcttcca gaggtggcgt tctaagtgca agggaggcgg gagagcggtg 125700
gggcttattt cttggtcaca gccttgctga ccaaagccgg acccggtcca gatgaggtga 125760
agtggagaag ctggcaaaga ccagcagatg gtaagagggc aaaacctggc tgcccttgtt 125820
ggtcactagc ctaagacatg cctaccagtg ccatgacagt ttacaaatgc catgacagtt 125880
tacggaaatt accactgctt tttatggcaa tgacctggaa gttatcaccc ctttcctaga 125940
aagttctata taacctgccc cttaatttgc attgacctgt cccttaagtt gcacgtaatt 126000
gaaagcgggt agaagtgggt ataaatacag ttgccaacaa cccatatgct gtggactctg 126060
ggcacactgc ctatgagtca gccccactct gcaaggaaca gtgctggtca ataaaagttt 126120
gctgcctaac actgccggat tgcccttgaa ttctttcctg ggcaaagcca agaaatctcc 126180
tgggctaagc cccagttttg gcctgcatca cctggcagcc atgaagatga aacagtgggg 126240
atggcagtgg tgggtggagg ggtggtgaga cagcagagag gtgatggtcc acagtgggta 126300
ggacaactgc catctctcaa aagaagtggt gaggtggcaa ttgacaagaa ggctggacgg 126360
tggagaggta gcaagacggc aaccagccgt ctggtgactg gctacacagt gactggtgag 126420
atgggaagaa gcagcaatca gagatggcga ttggctctgc agcgatcaaa gaaaatcaaa 126480
gtcatagagc ggctagtgca gcggagctgt aacctcagcc aaaggctctc ttcagagccg 126540
tcatctttcc tggtaggcag cagagccaag tggttggggg agtggccaca gtgccaccac 126600
cacgcatggg acccctgccc tggctggcag gtcaccggtc catgtgccag tctcctcaca 126660
gcagctgagc ctgcccaagc tggggaaacc ctgggaggag acctttacct agggattaag 126720
gtggagaatg atcagcacca ttttggcccc tgtggttggg tgagtgtcct cccctctgcc 126780
cacccctgcc aatatgagcc aggaaattag gcctctggct agatggtcag tttgaagtcc 126840
ccgtagcaca cctgaccagc cacatccttg tcattccttc tctcgatcct ttctcctcta 126900
atgccatttc attgcctgtt ggccatttta atttctgctt tgaaatgtac gtttcatctg 126960
caatttttgc tttggctccc tgctaactct acttgggcag ttacttaagg aaggacactt 127020
ggttgtggga ggtcccctgt tgtgctgacc ctaggacagc agggtcatgt tgttctgtgg 127080
ccccagcttg gcctttgggg ttcactgtgg gccaccagct agatgctctg gggttttcga 127140
cattggtgtg gggacccttg ttggctgata ctcgactgct ctgggttttt ggcatttggg 127200
attgtaggcc acccccagac gctccagagt ttttggcact ggtattccct ctaggattgt 127260
ggcgttagag gccaccctag gggaatcttg gccttgcctt ttctggtttc gtaccgaaag 127320
ttattttctg aaacagcatt ttcttattgt cactttatct acacttttcc ttctacgctt 127380
tgcttagtaa aaatagttgt tttgtcatat tttgtttgcc agcacttttt taatgacttg 127440
ctaccttgac ttattccttc tctgcaggac atgggaatct gaaaggggat gacagcaaag 127500

```
gtccagctgc ttctgctctt gctagactta gaaaaatgcc tgtgcccagt agaaatcctt    127560
gctagacgta aggatgatga tgagcatccc agaacacttg cagctggagt gtcttttatg    127620
ctggccagtg tttgatgttc ttcagggtac tagttctagc ccaagaacaa ttggcataaa    127680
gctgtggtta gacctgcaga tttctgagca ctggtcgtgg taagagcctg gtggtgtaga    127740
cattaaggtt gcttggtttc gatgttctgg aattgcatct gttttcaggc ccggtgacgg    127800
tacggctcat gaacgtaaaa cgtcttctga cgaaatcagt atatgaattg atatttctat    127860
agggaagact actcaattgt caacttcatc atagcttata gtaatcaata gctttcccgg    127920
ctttagatct gttgagtatg gaagagtcaa aatttaagtc ttcataatct gcatattcat    127980
agcatcagta tcatcagtgt cctgtagttt tgatggtaag cgaagggttg ttaatttcat    128040
ctttgatgta tagaatatgt aatagatgaa attcggctta aagaaattca ttttctattg    128100
caagaccatt tgggtccaac acaaaaatta gaaaatcaag agaactggtc taaacatggt    128160
tctacacatt ataatgctat ttcacaattg gacttattcc ataaaaaaga agaaaagtgg    128220
gaggaggtcc cgtatgtaca ggcttttatg gccctttact ggctcatgtt acttccaggc    128280
accaggaagg cacatctaag ggaacccctc atagctgctc cccctagaag gcctgtgccc    128340
tccttggacc ctcctcagtt ccccaattct gaggggggtgt tcagccagcg caggattcca    128400
cccagggcca tcaggccccc acccccctta tccaactagc cccagcctat acccccaact    128460
gccaagaaag caggtccaac gggcaccccc agaagcaggg ccccatatca gcccttcaag    128520
tcaaacttgt gtctgtggca agaggcagct gacggaaatg gggcacttg gagtgcaggt    128580
gccatttcct atgtctgact tggcttcatg caaggaggca cgtggccggt tttcagataa    128640
tccggggttt tatggaagaa tcaagtctac catgctcttt cacttgtcat gacttacaag    128700
cattgttgct cacttgctgt gacatgaggg agaggcagag ggagacgtgt gagattaagc    128760
cagtcagtta tgacaaggtt agagaaataa ctcatggaag gatgaaaatc acactgtttc    128820
aaggtagttt ggttgaggta ctcgggaaac gtattgacgc aggccccaac tccccagaag    128880
ggcaaggtct cctgggtata cattttatcc ctgaacctgc ctccgacatg aagaggaagc    128940
tacagaaagc agaaacggga cctgaaaccc ctatgagcca actcctaaac atgcccttta    129000
aagtttgcaa caatacgaac agggcaagag gtagaattaa aagcaagata aatagccaaa    129060
atgtacaatt gctaacagtt gctgtcagtg ccttgcccct cagccttacc catcctgaga    129120
gtgtgtttta agattggcat ctgacatgcc cagacaagag ctcttgactt gctggcccct    129180
gggtcagaat cagtgtgcct accataagca aaagggccat tggcaatgag aatgtcctaa    129240
ccctccctgg tgagaaagat aaaagctgcc tgtcaatact agagttaacc ttctgctagt    129300
cccaatgagg tgtttttttgt tttttgagat tgtctcgctc catcacccag gctggagtgc    129360
agtggtgcaa tcttggctcg ctgcaacctc catctcctgg gttcaagcga ttctcctgcc    129420
tcagccttcc aagtagctgg gactacaggt gtgcgccacc acgcctggct aatattttgt    129480
atttttagta gagacagggt ttcaccatat tagcaaggat ggtctctatc tcctgacccc    129540
aatgaggtgt tctgctcaag taagtgtact gggggcctca gacccttggc ctggtggatg    129600
gcttcctgca ggctgacaag tggccacttc aacatttttc tttggtgtct ccgctgctgg    129660
gtgagctctc tggtggctca gggactgcaa ggtttcacac tgagccatat aattcaccac    129720
caccccccctt tcctacagaa tctttctcca cttccccttg tctttcatac ttatcagggc    129780
aaataaaatt tggtcaggta aacaggtccc aattttataa ataatttggg tccagctgtc    129840
ttgtacaggt cacttcattt gcatgatatg tgttgtgact agcatgctat cacattggct    129900
tataaataag agtgctcata aattaaacaa ataagtctaa acatgttagt ttgaagggaa    129960
tgttgtgtct tctaaaattt aatttttacc taggtaaacc agatgttcat aggttttgga    130020
atggttaaaa tggctttagg tagtgagttt tgtatggctt aaaaatcttg aaactgtaga    130080
atgcttctca tctacaggat gctaatgtct gttgggcagt tgaagatttc ttgcttccta    130140
cctgtatata aaatgtgcta gggaagatac attattggga aaagaataac ttttgtccag    130200
aaagtattaa atgaggggct caaaatatga gggaaccagt acgagtagaa aagagagaag    130260
tggggagtta tacatacata catttttttt ttcaggaggg gtatgaagac tgacttacag    130320
gtgcccgcca ccatgcctgg ccaattttg tatttttagt ggagacgggg tttcaccatg    130380
ttggccaggc tggtctcaaa ctcctgacct caggtgatcc acccaccccg gcctcccaaa    130440
gtgttgggat tacaggcgtg agccactgcg cccggccaag aaagactgac tttgtatgag    130500
aaaggatctc tggtcccaga ataaagagac tggttgtgag ggaggtgtag gacaggtcag    130560
agagtccagg catgtcatgg gtggtctgtg tgggttgtaa tggggtttgt gaaggggaac    130620
ttctgagagt agttttgtgt gcaattaagc ctgctgtgat taaagaaatt ttttttttt    130680
tttttggaga cagagtctca ctctgtcacc caggctgcag tgcagtggca tgatctaggc    130740
tcactgcaac ctctgcctcc cgggttcaag tgattctcct gcctcagcct cctgagtagc    130800
tgggactaca ggtgcccacc accacgcctg gctaattttt gtatttttag tggagacagg    130860
gttgcaccat attggccagg ctggtctcga actcctggcc ttgtgatctg cccgccttgg    130920
cctcctaaaa gtgctaggat tacaggcatg agccactgca ccctgctgat taaagaaaaa    130980
tggtttgtgg tagactttct agggaatgat ctatgtattg gaactgggtt ttcttaaggt    131040
attgatttat taagttatga gaatttttgc ttttaatgct ataaccagct tcttctaaaa    131100
cttctttggg ctgagtgcgg tggctcacgc ctgtaatccc agcactttgg gaggccaagg    131160
caggtggatc acttgagcct aggagttcac aaccagcctg ggcaacatgg caaaaaccca    131220
tctctacaaa aaaaaaacaa caaaaaaaaa caccacacac cactttttaa aaaaaatgta    131280
```

gtgtgggtgt ggcgcacatc tgcagtccca gccacttggg aggctgaggt gagaagatca 131340
cttgagacca ggaagtcaag gctgcagtga gctgtgatca caccactgca ctccagcctg 131400
ggcgagagtg cgaaaccctg tcccacaaaa caaacaaaaa ctgcttgggt tggtgtccca 131460
gaggttcagc tgttgtgtcc cactgctgtt aaactgcagg aagtcactcg ctgggtacac 131520
ctgtccaggg ttaaacctgc gtcttctgcg tccctgcagg catgagggga ggacagcatg 131580
cccttcacct gtgagcccct ggaaggtgta aagctgttgt tttgcaaata cacaaatcac 131640
atggcgagga gcacgtgcag tcatcagcca taccgattct tcttgcctgg ttttgacctt 131700
tctcagggcc tttaatgaca tctcatcatt actaactttt ggctccctta ttggattttg 131760
ggtttcttcc cacccaacct tcatttcccc catgatcaga ccaacggtaa agcagcctca 131820
tcttcctttg atgcaggaaa gatgagccct aaaattgggg tttagccagg agggttcttg 131880
gcttcaccca ggaacgagtt acagggcaag ctggcggtgt tagttgctcc ctgtggagcc 131940
gggctgactc acaggcagtg tgcccagagt ccacggcata tgggctgttg gcaaccatgt 132000
gggctgtagg aaaggcaaag gacctttccc aggaccttta ataactatct tatcattact 132060
aacttttggt tcctagcaaa gctcgagtcc tgtagattgc aacaattcca cacaaagaca 132120
acactggtac aggcttcaac ccatcctatc ctctgacctg gagaatgaaa gtgtctggcc 132180
tctggggccc ttagatcaga gattttccct cctctaacct tacacaggac ctatgcccat 132240
gaacacagca ggaagcaatc ccagaagaca gaccctgccc ttctgcaccc ccttaagatt 132300
aaggggaagg atctcatctc tgaagagagg ggatgaggta ggaaggtggc aggacttgtt 132360
ttctggtcac tgacccactg accaaaacag gatctgggct ggatgggatg aagtggagac 132420
aggaatcagg ggacggcgag gagggtgatc cctggctgcc atcattgctc attggcataa 132480
gaccctccca ccagggccat gatggtttgc aaatgccatg gcaatgacct ggaagttacc 132540
accttttttc attgcaacga cccagaagtt gtcacttctt ttctagacaa ttctaaataa 132600
cccacccctc aatttgcatt gatctctact taatctgcat gtaaaagaag tgggtataaa 132660
tacagttgcc aacagcccat acactgtgga ctctgggcac actgcctgtg agtcaacccc 132720
gctccacaag gagcagctaa cacctccagc ttgccctcaa actctttcct gggtgaagtc 132780
aagaaccctc ccggcaaaac cccaatttta gggttcatct ttcctgcatt aaaggacaaa 132840
ggggcagaaa gggtgaggct gccttaccgt tggtctgatc atgggggaaa tgaaggttgg 132900
gtgggaagga acgtaaaatc taataaagca gttatctgta cagcagctag acctctccga 132960
aggaatcctg ctttgtggtt ttggcttccg agggtgtaag tcttccacgt ctgcagaatg 133020
ccgtcctcgc catcgaaagg aagaaggtga actctaaagt ggagacgtga gctggatggt 133080
gatgttggtc tcctgtggct gccataacaa ggtgctgcac accacatggc tcaggacaac 133140
aggcacctgc cgtctcagag gccacagtgt gacagtgacg tgcaggcaga gtgggctcct 133200
tccacccccct tctcccacac agtggctgct ggtggccctg ggtgtgccct ggctgttggc 133260
tgcatccctc cagtctgctc cctctcagcg tggggctctt cctctgtgct ggtctgtctg 133320
atgtcttctc ataagacatc agctacagga tttagggccc gccctaatcc agaatgactt 133380
cactttaact cattgcatct gcaaagagcc tgtttccaaa caagttcaca ttctgaagtt 133440
gtgggcggac atgtgtttgg agggcaccgt ttgccacagg acaactacgt atcacagtgc 133500
tggtgatgca accacacaag tgaaattatt tcaagtaact gtctcaaaac aaaacaaaaa 133560
aagtgtctcg ctctgttgcc caggctggag tgcagtgacg tggtctcggc tcactgcaac 133620
ctccacctcc cgggttccag caattctgtt tcagcctcct gagtagctgg gataccatgc 133680
tggaataatt tctgcatttt tagtagagat ggggtttcac cacgttggtc aggctggtct 133740
tcaactcctg acctcaggtg acccgcccgc cttggcctcc cgaagtgctg ggattacaag 133800
cgtgagtcac cgcgcccagc cttcaggtga cttgtgagca cagcactcac actgtttata 133860
cttagaagga tattctctga ggataaaaga tcatcttgtt ttcattttta atttctaaat 133920
agtcactttg aaatgatttc agtcatgaaa aaagttacca aaaaacaaaa acagtgcaaa 133980
gaattccagc atatcctgta agtagtttcc ctagaagtta tctcacatag aaccatggtg 134040
caatgttcaa aattaggaaa ttaacaccaa cgcaatactg tttttaaaaa attatttgaa 134100
atgcaatact attaactatc tacaggcccc atcccagtgt ctttctggcc cacagcccag 134160
tccaggacca cacattgcat ttggttgtca ttctccctgg ctgcctccca gtggggacag 134220
cttggagtgt ggggccattg cccagcacac agctgcttct catgactgca tttaggacat 134280
atcctctggg caggacccac agaagctatg ccaggctgac ttcagggcat ctcatcagga 134340
gacctatgat ctgggcctgt tactggcgtg ttaagctcgg ttgctgggag caggggtatc 134400
tgccagggct ctctgccaca aagttacatt ttccctttaa ttatcttgtc gggagatagt 134460
ttgaggccaa gaaatatccc atttctcatc ctaattttgc ctactaatcg caacatccct 134520
cgatgattcc tgtgttgttt gccaaatggc cattctctgt taccatcgtt ccttctacat 134580
taactgcaat tcggaggaga cgagcatttc accctcttcc cccttattta tctatgcatt 134640
tatgtatgga cttgtgggca tttagcctta tgagttatga actattacta taattttctt 134700
ttttgagaca gggtctcgct ctgtcaccca gactggagtg cagcagcatg atctcagctc 134760
actgcaacct ctgcctcctg ggttcagatg atcctcccac ctcagcctgc tgagtagctg 134820
ggaccacagg catgcaccac catgcctagc tgattttttta tctcctgtag aggcaaggtt 134880
tcacaccatg tgtccaggct ggtctagaac tccgggactc aagccatcta cctgcctcag 134940
cctcccaaag tgctgggatc acaggcacgt gccactgcac ctggcccatt ggtatactta 135000
tgtattgttt tgctccgatt atcccaggtt tggctctgga gccgtgtaga ttggtcctgc 135060

```
atcctttgg cccccccgc tgccctctga gcgctctgcc ttactgacca cccccccccc 135120
gctgcccttt gggcgctcta ccttactttg tgggaccact ccatgttcac ccgggaaccc 135180
tccctgcctc agtgctatct gggggcccca gtgcctgacc tggagaaaga tgttagtgcc 135240
cgagatccgg ctctgggggt gcccatggct gtagggtgtc cctgctccga ggtcccccag 135300
tggacacagc taaagcctcg gtgagaacac acgggcccat gccaggctca cacccgtcta 135360
cccagccgtc taccgacact tccgtgctga aactacaagc tcctactgcc gcctcccctc 135420
ccagcctccc atgctcctta ctggtagctc ccttctccag cagcgaggct cgtcacccac 135480
agcgcggccg ctggaaggtg acctgagcgg ccagacaggg cctcctgctc agagtttaaa 135540
gctctgctgt cgccatcctt aagttcttaa tgagttttga gcaagggccc tgcaatctca 135600
tttcgcacca ggtcccgcaa attatgtatc cggctctaat tacccacaat atgcttgctt 135660
gctcaagtct ggagcacaca caaagtaatc tcataattgc taacccaggt tgctgtgcaa 135720
aacagcttca caaactaggg cacagtgtct gcacgccatt ctttttgtct tacagcccat 135780
gatactgtct tccaaagcat gcggtaggtt ttcttttccc cactcccttc gatgaggttc 135840
acctgttact gcttatattc catttcggct tcaagccccc atcccggttg attttaatca 135900
cttatttact ttgggggggtg tgtggtatat ttctgtggtc ctggaaggaa cacaaaagct 135960
gcagcctcca taataaggta agcccaccag cgcctgtacc tttgaggacc atgaagggaa 136020
gggtctgtct ccctgttccc tccactaact ccccccatct ctctggcccc cacttctccg 136080
caaagtggaa actccctggg gaggcagaag accagggtgg caggactgca ggcagagagg 136140
gattcctgcc gccccatggc tgggtaccgc tggtaggcca cgcacacact gggtaagtgt 136200
gcactaggca ctgacgttaa gggccaggcg ttggtcctc cgtctcacct gcgccaactc 136260
agcacaggga gagagctgag gctctgacaa ggatacaggg cagagagcag gaaacagcag 136320
ggctcaggct aggggtggga gcccagctgc tcaggagttg aagtgcaggc cccctgtggg 136380
cacctggcgg acaggaaagg aaggagacta aggagtgtgt tgtactttat ctgtacctgg 136440
ctacgtgaga tcagactgtc acccagcatt aactaaacct tacagtggag acagtgggtt 136500
gtatcactcc cgagaagaaa ccacagaacg ctgtaggagt aatgcctgca ccggcttccc 136560
ccgcaccaca cggagcggct gcctacagct ttgttagggg ctgaatgcaa ccccgatgca 136620
ggcaccagcc ctccagagca gcctccatgg aggaatgcgg ttccccactc tgcccacata 136680
cctctcctgc agcagcgctg aacaccgtct ccggacgtct ggagctatca ccatgggcaa 136740
cactttcgtg gtctttattt ttagcttctc ctttagaaac tcactttttg gggtggtttt 136800
gtgtccagaa gttggtgggt tcttagtctc gtcttcaaga atgaagccgc agaccctcgc 136860
ggtacatgtt acggttacct aaaaacggtg tgtctggagt ttgttccttc tggtgcgttc 136920
gtggtctaca ctgatttcag gagtgaaact gcaaacctcc gcggtgagtg tcacagctcc 136980
taaaaatgca gcacgtccag agttctttgc cctcccagt ggatttacag tctcgctggc 137040
ttcgggagtg aagctgtaga cctttaccgt gagcgttgca gctcacaaac gcagcacaga 137100
cacaaacact gagcagcagt aagatttact acgaaaacca aacaaggaaa gctcccagag 137160
gacaaaagca gaccctaaca ggttgccacc gctggctccc gcagcctgct tttattccct 137220
tatctgaccc cacccacatc ctgctgactg gtccatttta cggagaactg attggtccat 137280
tttacagaga gctgattggt ccattttaca gagagctgat tggtccattt ggacagggtg 137340
ctcattggtg catttacaaa cctttagctg gctacagagt gctgattggt gtgtttacaa 137400
tcctttaggt agacagaaaa gttctccaag tcaccaccgg agccagaagc ccagccggcc 137460
tcacctctca gtagcactcc ctttagaact ttgcagcatc taccctgggc actccggcag 137520
cccagaggga gctcatcccc agatcaagcc cagcaggcgt cggcgggcca gccaagcctg 137580
cacccacccg gaacccgcgc cgcccgcgag cgctgcgctc agcccccgct cccgcccgcg 137640
cctctccgcc agcagaggga gctggctcca gcctcggcca gccccagaga ggggccccac 137700
agcgcagcgg caggccgaag ggctcctcaa gcacggccag agccgacacg gaggcccagg 137760
aagcgccgag agccagcgag ggctgctggc acgttgtcac ctttcagttt cacaatatcc 137820
caagatgcat tagtagtttc tgtagcctga gtgttagtgg cccccaaaat tgataggttg 137880
gaacctaata cccaatgtga tggtgttaac aggtgggacc tttgggaggt gagtagatca 137940
cgagggctgt gctctcacga atggcatgag tggcccgggt agaagaggct ccagggagct 138000
ccctcgctct gtccaccctg agccttgtca agaggcgccg tcttggaagc agagggctgc 138060
ctcatgggac gccacatccg ccagcaccct ggtcttggac ttgcagcctc caggactgtg 138120
agcaataaat gtctgttgtt gacaagtggc tcaggcttag gcgtgttgtc acagcagccc 138180
gaatggactc cagcagcaga gcagccaggg ctgcacgtgg tgcgtgaaca cacccgccgc 138240
cctcctcctc ctggggcata tgacggctgc gcagctctgt gtatccagtt tctgggctgt 138300
tccatgcccc tattcccctg attgctatgt tctccagctg aaagagcaca ctgattttgc 138360
cttcagccca ctctcatttt cccccagatt tcactaagtg attttgctaa gtgatggctg 138420
tgcttcccct gtgcgcttct ctctggctgg tttctactcc ggatccacta gtggggccag 138480
aaagagcagt aggaaggaac ctgaggttcc tgtccaccat caggcatgtg cacacctaca 138540
cccacacacc cctgcactgt gcacacccac acacacttgc acacccacct gtgcacacgt 138600
acatccatac acacatgctc acccctacat ggacacccac acacgtgcac gtgcccatac 138660
acatgcacac ccatgcacac acctgtctgt gcacacccca cacccatgca tgccggcaca 138720
cacccatgca catccacaca caggcacata cccacacaca cgtgcacacc tcacacccat 138780
gcatacctgc acacgcttgc acactcacct gtgcacacac atcatacctg catgcacacc 138840
```

cccacacgca cacccacaca cctgtgcaca cacccataca catgcatgcc cacacccaca 138900
tgtgcacaca ccgtacatac ctgcacacac ccacgtatgc acatggtcaa cactcaccca 138960
ctcaggcact cttacatgca cacacgcaca cagacatggc cactttcatg gacacacacc 139020
cacacacgcc cacatataca cgcatgccca cctaaaccac tcacttgacc aaatagcttt 139080
tcaggagagc gcctggtcct gaagctttct aggcatttcg gtagccaaac cctcttgatc 139140
gtgtggttcc agaaaaagcc agtgcggcac ccacttaccc gtactgcagc caccgtggtc 139200
ccgggaagag ggcttgcttc cgcagagcta atgagggaag cggtctctga gagtagaagg 139260
gaggcctcgg agggaggtgg tctccaagag tagccgggag gcctgggagg atggtggtct 139320
ccgagagtag cagggaggcc tgggagggtg gtggtctccg acagtagcag ggaggcctgg 139380
gagggtggtg gtctccgaca gtagcaggga ggcctgggag ggtggtggtc tccgacagta 139440
gcagggaggc ctcagaggga ggtggcagag caggctctgc tctcaggaag tgccccgtgg 139500
gtggcgacgg gccgagaggt gctctggggg acgagttatg gctctgagct gcccgtgcaa 139560
ggcagggagg ctgcgttttc atgcgttcac acctcagtcc ctgggtaagg acctcccagg 139620
atgtacctcc agcaccccgg tcatgcagag gcctgggaaa gcctgttgag gctggagggg 139680
tgggggcaca ttggtgtgtt gggggacctg tgtcgtgggc acggctcccc gtttagggaa 139740
ggcactgcgg tgatcacgca ctaccagtaa gtatttcaga gcccaaaacg cgaaaacaaa 139800
tggaagaagt tccatgtcat agtgagtctt ggctgggcgc agtggctcac ctgtaatccc 139860
agcactttgg gaggctgagg cgggcagatc acttgaggtc aggagtttga gaccagccgg 139920
gccaacatgg tgaaaccctc tctctattaa aaatacaaag ctactcggga ggctgaggca 139980
gaagaattgc ttgaacctgg gaggtggagg ttgcggtgag ccaagatggc gccactgcac 140040
tccagcttgg gcaacagagt gagactccat ctcaaaaaca aaaaagaaaa acaaataaaa 140100
atacaaaaat tagctgggtg tggtggcggg cgcctgtaat cccagctact cgggaggctg 140160
aggcaggaga atcgcttgat cccaggaggt tgtagtgagc caagatggcg acattgcact 140220
gtagcctggg cgatggggtg agactctgtc tcaaaacaaa caaataaaca cacacagaaa 140280
acagtgaatg tttttattac aaataataaa tgtttgcttt tacataccac acctcatttc 140340
cccaggggtt cctgagcacc cccccccaac cccctgctca ccctccttca ttctctccac 140400
agtgtttgag gcatattttg tgccaggccc ctcccagggc tgctaacaag aaaatacaaa 140460
ataaaaatgg cagagaagcg agtgcctccc gcgtatggca atgccagcca tgagccaaac 140520
ggctgaacag agtccacatc tccctccggg tttccggggt cccctgctca ggcctcacca 140580
gtgcagcgcg gacgtcagac cgctaccgtc tcactcaagg ctcccgtggg ccagggtcca 140640
cctgggttct gctccttggg atattggcag gatcccgggc cctgctgctg tggactcggg 140700
acttcacttc cttgctggtc agaggctgcg tgagcttcca gaggccacct gctgttcgtg 140760
tcacctgggg ctccccggcc cagccatgtg cttcctcaaa gccacagggg agggccaggt 140820
tccaaagaaa tgggcattgc aatcctgtgt aacacaatca cattcctcca ctccactttg 140880
ctgtgttctg tgggttagaa tatggttatc gccgggacac caaacaaggg tgtcaccaac 140940
aggaggtggg gggccttggg agtatggcta ccacacagga ggaaacgaca gctccactcc 141000
atcactgaac ctgagctcag cagtggcttt gagtgtcctg gcagccacgc aggaaaggga 141060
ctctgctcag aacacacatg cagaagcagg acacactcct gctctcaagc ccagcgctga 141120
gcccatcagc gctgagccca tcagcgctga gcccatcagg ggcaggtgtc ctgggcctcc 141180
ggtctcaagg gcatcatttc tgtgcaggca acagtcactg ggctgtcctt tcacgccctt 141240
tggacacaca gacaacacaa gtggttccca gaagccctct gtgtcctgtc aggtggtgac 141300
aggtgggggt ggctgtcctg tgtcctctca agtggtgaca ggtgggggtg gctgtcccct 141360
ctgtcctccg tggtggtgac aggtgggggt gactgtcctg tatcctgtca ggtggtggca 141420
gctgcaggtg gctgtcctct gcatcatgac cccacctgtg tcatggcagg gccaggctca 141480
tctccagaaa cctcaggtca gtgatcccta cggatgcctt tttacatcat gtcagaatct 141540
ggaaagtctg ttttatttca ttatctgaaa tacttttaag agtggtaacg gtagcattag 141600
actgatagtt acatggatct gggacatctg tggcactggt gcataaacat ctgagccatt 141660
tccatcattt aacttgtcca atcctttcaa cagccttctg tgctgggcgc gatgactgct 141720
ctctgcacag atgagtaaac tgaggcacag gtgcctggac ccttgccaaa aagtgaggga 141780
gccagagatt ctaacccagg gcccagccct gagtctcacc cctgccctct cccatctgcc 141840
tgtccctcca caaccccgat gcccgaccct gggtggagaa agtacaggga gggtctgcag 141900
ctgcccagct gcagtgtcca gtgtcctggc gacaggaccc ggagcaaacc aggcctcggc 141960
atgggcacgg gatgaactca gggcaccggt ggggtgttgg ccgtttctgg gtaacagacc 142020
tacaggtgat gtcttttcct cttctttcta agctttaaaa attaccattg tgattaatag 142080
aactttttag aattttagat acagtttctc tgtgtgaaca aggcttaaag gatatgagaa 142140
tgtttctgta gcttacaggt cataatacta tctttgaata cacaaattga aaagccactt 142200
aaatattaac ctttgattct gaaccaggat gagcttaacg atggggacct gcagggcgtg 142260
gccggctgga gcccatggcc agcagccatg gctgagggca tacttcaaaa cggtcatttc 142320
tgtcccacta atactgtgta aatatgtatg ccatccaatt tttaaccaaa taaaatgttc 142380
tgtctgacgg gtcccttcat ctatatgaca aacagtttta agtaaagagg atctcagctt 142440
gcagaataag taaaattgtt taaattctag ataaatgtgt tcagtgcttg atgggctaat 142500
gctggctgga tttgtgaagg tcagtcatat acatgaaaat tcttctgcat tcaccttaga 142560
agagcgatat ttttaggcga acagagcttg cgatattttc tttccgtgcc acctgcccgc 142620

ttggtgtctg gccctgcgtc ctcagaggac catggtgggc tggtgttctt ggagaggagg 142680
cctctccagg gccaccaaga gggggctgtg gcccaggctg agctcttcta gtggcatgag 142740
ggacacgctt tattcatgtg agcgttgtga ggtgggaagg gaggccagcc aggggaaggg 142800
tagaagaatg cctgttgctt gggtcggagg ctcgatggac tttcactgct ggacatagtg 142860
tgaacatggg agacgcaggt tcagcgatca cgtttcactt tcccattccc cggacctcaa 142920
actcaggcct gtgtaactaa aggttctgtg ctgccaggct gggcgtttcc cccagggaag 142980
gcacgagaga gcgaatgcag ggccggccgc ggggcctggg gactgtgccg ctcagcacca 143040
cccacagggc gttccttggg gcgtggcctg ggccggggcg gtaggatccc ttggacaggg 143100
ccgtgtctgg ctcacaggaa gctgccctca tggaacgggg tgcagtgggg tgttcgtggt 143160
cctgctgccg ttggctgggc gcagccatcc ctgccggctc cccgcgcct gtgccccggg 143220
ccctactgca cctgcacggg cggggctctg gggccggcgc ggggagtgag gccgccggtc 143280
tcctctgctg tggtcggtgt gggggccggc ggggcgcaga gctgctcacc cggctgactc 143340
ccccttctgc cctgctggcc tctgagccgg ttctctgggg cctctgtgag cgccttgtgc 143400
cccccacacc cgccgtgagt accgtgtctc tctgctcagc caggtgggcg cctctgctgc 143460
tgctgttgcc accgagtgac tgactgacgc agcgacgcag ccaccgacac ccaaacactg 143520
gagcgtttta cccaaatgta aaagtctaaa cttccggctt ctcgaacaaa agaaaacaaa 143580
ggaaaacgtc agacgccccg ttgacgccgg gcgcgcgtgg ccgcggccga gccgctcttc 143640
aaagccggca gcgtctggtc tggcctggct ccgccagctg cccgcaggtc ctgtcctcgt 143700
ggctttctgc cctgtcctcc ctggccggct gcactggctt ctgtgacgct cgctccttgc 143760
tgcgcaagtg caggtgcagc accttaaagg ctgagtgcgt cccacacagc ggggctggag 143820
ctcggcacct tcagtccacc ccaaacaccg ccctcccgct gtgcacggcc actgctgccc 143880
ctcctgcacc ccgacacatg gggagctggt ctgagcctgc cccacctgct ctcctgtgat 143940
ctcccctctg ccaccccat ctcgcgctcc ataccccttc ctcagacctc cttgcctggg 144000
gctgctctcc ccaactcagc agagcagggc tggccagtac taaggacagg ggcgaccact 144060
ggtccccaga aatcctgaat ccaggaaacc cctcagtccg gggcagaccg ggatggccga 144120
ccaccctgac taggggaggg ccgtcagaag tctttgaccc tgatctaagt tctctctgct 144180
ttgctgagtc tctttctgtc tctctcctcc tctccctgtc tgaatgtctg cctgcctctc 144240
tcccacacac atctgagaca caggtctcac agaaccgccc ttgcccttct gtctgtctgt 144300
ctgcctgcct ctctctcaca cacacacctg agacacaggt ctcacagaac cgccccttggc 144360
cctctctgcc aggagggtgg ttctcggagg ttcccatcct ctgtggggat gctgacagct 144420
gtgggtcatg gctggtgccg cggttctcac tcttggctgc agagagcagc tgggggctgc 144480
aggtcaggcc acacctagct ccccatggcc ctggttcact taagtgacag gctggggtga 144540
ggggaggagg aggccagggt gtgaccgtcg gttccatttc tgctttcccc cagctcccca 144600
agtcaggcac cggcacagtg gcagttggtt tgtgggacac ccatctgtca gtagggcatg 144660
agtatccttg gctctgtcct gctgaatcat gggtcctggc tgtggcgcca gggccagctg 144720
tgtttgtgat ctggtgcatc ctcaggagaa ggagagcagg cgaggcgcca gggaatgtag 144780
gatttgctga tgaattgtgt ttcaaaaaag gtggcttttt ttctgttgtc agacttcagg 144840
aaacaggtag aggctgtgac ctttgggcca gtcaaatgaa tctacagaga ttaaaggagg 144900
attgtggagt gaaaaccaaa aaaagccaaa catagaggaa tccgtttacc ctttcagcca 144960
cgtatttcgc tgttgccgaa cctttattaa aatgcttctc tcaaaggaaa aattgtatag 145020
ttttttcagg cttcaaaaac ccaggccatg tgtctaaagg tcacttgacg aacagcacaa 145080
atcccatggt aaagggcagg gtgcggaggg gtgagcctcc catccagcac caaagcgggc 145140
tctgagggct tatccctccc agggagtcca gcaccaaagc gggctctgcg ggctcatccc 145200
tcccagggag cttgggttct gcacaaacag tgtcagaaga aaagacttct tcagacattt 145260
tgaaattaac atttcatttg tattaaacag cccaaccctc cttggagagc aagtactaaa 145320
tacagcatcc acacagtcag ccctgtaggg gaatttgaga ttggaccaga atggcagact 145380
tgtacatgtc atccaggacc ttccacccat ccatctgcac atctatctat ccctccacct 145440
acccatccac tcatccattc ctccatccat ctatccatcc acccatgctt ccatgcatcc 145500
atccatccac acatccatct gtccatccac ccaagcatcc atccatccat gcatccatct 145560
atccatccat tcatccgttc atccatctat ctatccatcc ctccaaccgt ccataaccta 145620
cccacacatc cattcaccca tctacccatc catccatcca tccattcatc catccacaaa 145680
tccacttgtc catccatcca tccatccata acctactcac tccccacatc aacccatcca 145740
cccacacacc acctattcat tcatccatcc atcacatacc cacccacccg tctatccatc 145800
catccatcat caacccatcc actcacctac ccattcatct atccacccat ccatgcatct 145860
attcatccag ccatcatctg cctgcccatc catctaacta tccatatatc catccaccca 145920
accgtaatca accatccatc cacccagcca tctacacacc ccctttccct ctcaccctcc 145980
ctccctatct ccctttcttc tttcttttgt ccatcaatac atccattcat ttactcatcc 146040
atctatccaa ctatccactc atccatttat tcatccaacc atccaccact catccattaa 146100
cccacctgtg cattcatcaa tctatttatc cctttctctc tctctcaccc ttttctctct 146160
ctcctttcat ccatccctcc cttcttctgt ccatccatcc agccttccat ctatccattc 146220
atcccttatc tatccaccca tttatccatc cacccgcccc ttcacccacc cattcattca 146280
tccatctgta aacatattaa atgccactgc cttccatgta ccaggcctgt tcaaaacact 146340
ggcagaagcg agtgagacta actcagttcc tgctgggaca agagacaatg ggccatctgc 146400

cttcaacaga acacctgcta gggaagacct ggggagaatg ggggtttcaa ggaacactgg 146460
ccaagggcca gaacccagaa gctggagtgg gaacagactt tcagtggtga tgtctatggg 146520
aagctgacat ggcataggag ctagtgggtt aaggttggaa agttcatggt gagaacagaa 146580
agaaggtagt tccagatgag ggaacaagag gaagtacagg atgggaagga gagtgaagcc 146640
tgggaaggca tcccaagggc cgggaatgtc tcaggtaccc aaaggcccag gcaggatgga 146700
cctgctttct ctaatgccct ggggacgccc accacaaacg gggcacttga aacaacagaa 146760
gtttatggtc tcaccgctct ggaggccaga agtttgaagt cacagtgttg ccagggttgg 146820
ttcctgctgg aggctctggg gcagcatctg ttccatgtgt gtgtctccag cccctggtga 146880
tggctggcag ccctctgtga gtcttggttt gcaggtgcat ctcttgatgt ctgcctccgt 146940
agtcacacag aggtcctgtc ctggtctctg tttcactgtg tctcttttcc ccttcccagg 147000
acaccagtgg tgttggattc agggtctcag tgtctctcct ctcctcttat agagtccctg 147060
tcatattgga tttagggccc accctactca ggtatgacct cgtcttatct aatcgcatct 147120
gcaacaacct tattttcaca taaggagtca ggacttcagc cttctcaggg gatacagttc 147180
gacctttccc accgccccac cagccagtgc cgcagctcac gggcccttcc tgccgtgagc 147240
gtccactcag tgctggcctt tctgctgctc ccagtctcgg cttttatggc cactgcacac 147300
gcctctccat gtgtctctgt caggcacaca gagagaactg tggggcttgt gattgtctcc 147360
cttaggctgg aatcctgcag actcttgagt ggagattgtt gtgctcctga atcactggga 147420
ctcacaggag aaaaccagga gagagcggga aacaggccaa gggaagggag aggccggagc 147480
agacatggtt tcaggtgggg tccagtggtc ctaacgggga ctctggacat atatcagact 147540
gcaggtcagg atgggggctt tgacctcaag gtggtcaggg tctggccctg ggccaccctg 147600
gcacaggctt caatcccagg ggcccctgga cgagggccca gctgcccaag gcaggccctg 147660
ggggctccag gtgtgagctg ttagggcggc aactgcagcg ggggtggtgg ccaccccca 147720
gggtaagggg catcctatgt gacaatgggg agggtaggag cagcagtaag gctgcaggca 147780
ggttcagccc ttgtcgtttt cctgccatgg agttcactca cccacctgaa agctgcgagt 147840
gtccttcctg tccacacgca gccacacagg ggctcagact ttccaaagcc cagctgctgt 147900
gatgggtgtg ctgccacatt acgtggtggc tgactgcatt tccatgacaa ctggtgaggt 147960
tgggcctctt ttcataggtt tgttggccac ttgggcactt tcccttgctg gcagtcattc 148020
aaggtctctg tccacttttc tgatggggtg tctgcctctt ccctatggac ttgtggagtt 148080
ccttctacat tcagggtgta agtgcccctc cctcacctgc tccctcaggc actccctgag 148140
taaatcactc ggcacgagga ccccgtgtgc acctgcagta tgctgttccg gttgcctgca 148200
gagcctgcaa gctcgccagc ggccagcggg catggggctg gtaccagatg ccatccacag 148260
ctcacacgcc agagaggcgg gatcccacat gtggctcctc actcctccct gctcgaggac 148320
tgccgtgcct ttgttttggg gctggggaat gtcctcagct ggttcttccc ccaatgccct 148380
gatcctgggc tcacatgtga ccggtccctg cagaccccac acctctgaga aaggcttcag 148440
tggcatgggc ctaggctgga ccacgacgca ctcagcaaag cccagctgtc tctgtgacca 148500
acggccatct gggccggccc ctgctgccga gctgctggga ccacaggcag aggagaccca 148560
cctggcagag gagacccacc cagccccgga gcccacctct ccgattgctg gggcttaata 148620
cagtcgccca tgaggctgtc ggagtccttc aagtctcagc ccacatagtg acttatggcc 148680
acccacacag tatctgcctg gaccccagac ctgagtggtc agcagggatc acacctgggg 148740
gctccaggtg tgagctgtta gggcggcacc tgtagcaggc ggggtgtagc cacccctgag 148800
ggtaagggac atcctatgtg acgatgggga cggtgggagg cactagttag gctgcaggca 148860
ggttcagccc ttgtaggcag cccagcactt ttcctgccac agagttcact gacccagctg 148920
aaaggtgcga gcgtccttcc tgtccacacg cagccacgtg ctacgatgca gcagtagcag 148980
tgtccccatg tgctgccagg cacaaagccc tcggtgtggg gtgtgccctc aggcagttcg 149040
gggcagggct gcaggtgggg ctggcgggag tgacgcttgt cttatcacag gtcactgcct 149100
ggaaggggtg ctgccatcct atcacaggtc atgctgaggg cattggcatt tactccaagc 149160
accaggataa ccctggggtt tccttgggct gtggcctgat ctgttggtat ctagaagaat 149220
cctttaggcc acagagcgac gaaagatgct tggactgagg accaggaccc accaagatct 149280
gggatggggc ttggaagagg ctcttgggtt gggcagtggc agtggtcagg cagtgtggtt 149340
ggagtctgga catgtgggga gcccacaaga atgctgaaag gcaggtgagg ggctggggga 149400
gagcggtgca ggatggcacg gaggtccagc ctgagcaagt gggcacgaca taaggtgcca 149460
caacctgcca caggggaccc tgcaggagtg cagacgtggg tgagggtcac cactgcaccc 149520
aacactgtgt gggagcccag ggaaggggaa acagatcaca cccagcacac ctgcgtggga 149580
gcctggggag ggggacaggt cacacccagc acacctgcgt gggagcctgg ggagggggga 149640
caggtcacac ccagcacacc tgcgtgggag cctggggagg gcgacaggtc acacccagca 149700
cacctgcatg ggagcgtagt ggatgtgtta tctctaacgt ctaggcttta ggggaggccg 149760
gaaggagaga tgggccaaga ggcatcaggt gaggaagctt tgctgtgggt gggctggggt 149820
ggggcagaag ctgacctcgg ggtgtgagca ggtgagctgg gcctgtgtaa atggatgggg 149880
cccagcggaa acgagacaag gaggctgagg cagtttttcc attgtggatg tcttttattg 149940
tgcttaggga cccacacgat gctgaataga gattggtcaa cagacacgga caacacctgg 150000
gttgctacac ggagcccctc cccagaaggc gatggggaag ccgctctctg tgctgactgc 150060
agcagccaga aggcgatggg gaagctgtcc tctgtgtccc tccccagaag gcaatgggga 150120
agccgctctc tgtgctgact gcagcggcca gaaggcgatg gggaagccgt cctctgtgtc 150180

cctccccaga aggcaatggg gaagccatcc tctgtgctaa ctgcagctgc ctggcagttc 150240
acaggctgac ggctcccacc gagcattaca ctccagtccc tggagcccac gtccacttgc 150300
aggggacagc catgactggc cctgcacggc ccctgccctc gggcagcacg taggcaggcc 150360
caccgacttg aggcatgaat tcaaactatg gcatccactt tcctgtcagt ggcagagggc 150420
agtgtgccat ggaaagcagc ttttggccaa catccttcac tcagtattgc taattattct 150480
tgtaaacaaa aactgcaata tcagcaagca ggctcgcgga tactgcattc ttgaatttgt 150540
tttgattcac aggtaaatat gattttaaaa agccattcgc aaacataaaa actgttgtta 150600
ttgatgtggc acgcacctga gagaaataaa attccagtgg ggtcccttcc tggaggtcac 150660
ggctcaaggc caggcagagt gtggtctgca ggctgcctgc atgggggccc tgcatgcgtc 150720
ctggggtagt acacgctcct gtgggggccc tgcatgcatc ctggggtagt acacgctcct 150780
gtgggggccc tgcatgcgtc ctggggtagt acacgctcct gtgggggccc tgcatgcgtc 150840
cggtccgggg ataggacacg ctcctgtggg ggccctgcat gcgtcctggg gtagtacacg 150900
ctccagtggg ggccctgcat gcgtcctggg gtagtacacg ctcctgtggg ggccctgcat 150960
gcgttctggg gtagtacacg ctcctgtggg ggccctgcat gcgtccaggg ataggacatg 151020
ctcctgtggg ggccctgcat gcgtccgggg ataggacacg ctcctgtggg ggccctgcat 151080
gcgtccgggg ataggacacg ctcctctcag gccgttccct acaccgcaag gacccacagg 151140
ctgcctgagt ggcagtagcc tgagctggtt tcaaggagtt ttatttctct ctgctgcaag 151200
aacataaaca gaatttcctg gtgagacata ccaggacccc catcctccaa tgcctctgaa 151260
cagactgtgt gtgcaatgtg tcaactgaac gctcaattta cggcctctgc tgggagccac 151320
gcatcgggaa aggactttgc atgaatttgt ggtttcctcc atttcacctt ctgcacagct 151380
aaaccaagca ggacacttgg ctttttaata tgggcaaagt aaatggtcta ggaagctact 151440
gtgagcacgg ggattctcag caggtgcgtc tacaaggatc gtgatccccg cctgagaaca 151500
cagtgcccct ggggcagcct cagagccggg agcagggagc agggagggag ggagcctcac 151560
acagacagca tgaagttaga cgtttttctg ccctgcaggg acaacaacgg ggtggacctc 151620
gctgcacaga tctacgtcgt tattacagca acacaagaca cggcgaggtg cgctcccggc 151680
acggaaaggt gtaaacagtc agaagagagg agtcttctgc tttgttgttt gtgttttcag 151740
tagaggttga agagtagaag ttgccctcct ttctctcgaa acttagattt ccttggtttg 151800
ttcgtgtctc tcccattgca ggatgacttc ctggggtctt cgtctctgct ccctctacac 151860
cttgagccag tggcggagct ggaaagaaaa caggtttagt cagaaaccct ggggcgatgc 151920
cccatttaag agcagctgaa ggtggctaag agcagctgaa ggcagtgagc agacagtttg 151980
cagcctcctg gccatgtgcc ctgggagaag gggaggctca ctgggggcct ggaccaacac 152040
acccttgaca ggtgccgggg atccgatgcc tcactcaaac tcctacaaat caatcattac 152100
ttgatttttt aaaaagccgc gagactgtgg ttggcgtggt ggccatggtg gcctctgggg 152160
cgtattgatc agatgctgag tctgagagat gccgggcacc ctcagtccgg ggctggttcc 152220
agattgtacc ccagcgactt gggtgtgacg tggtgatgtc acctctcccc cacgtccctc 152280
caccttcctg gggcctcag atgctccatc tgagtgacac gctacctctg cagctccatc 152340
ttgctctctg cgacccgcgc gggcgcaagt gcagcgtgta ctgactgctc agggactgat 152400
gagatagaca agagcagcta tttacaccac cagcaaatgt catcgcctgc tattatgcag 152460
ctgttagttc agcctgtttg tgagcgtcaa agcccctcga tagcactaaa tggcaagaag 152520
tgtgcttcat aaacatttcc aaacaggggc atgcggggct tgcttctgac aaaaccacgc 152580
cggccatgga aagccactga agctaccact ggccacacca caggctcctc tggccactcc 152640
accaagagcc tgcagggacc tcctcagcca gggtgggatc acactgcagg ccaggggtct 152700
accgggggcc tggagcagcc acagctgatg ccggcacagg cagcatccag gtgcagctcg 152760
gggctcagtg aaggaagctg gggtcaggga gcccggccag gacccacagc cacgggtggc 152820
ccagcggcca gagccctaga gcagaggctc tgggtggcag ccccacttca ggccagcgat 152880
gaagaagcca tgcttgggac ttcagctgtg agtcctgttc cccccagggt gaagcagttc 152940
tctgtcctgc ccgctgacag aagcagaagg tcccctctgg agggaggtgt gcttggggc 153000
tcccagattc ccacagatta agatcaagcc ataaatgtaa aataaagact agaaacagaa 153060
gacagcagcc cactctaagt gagtccccag aaaccagacc tcaatttgac cccaaaggac 153120
cagagctgtt gcaacagatt acaggatggc cacataggggg actctcagaa aagtgacaca 153180
cgcacatcac agtgatgaga atttcacaag aaacagcaga gcacacgcag cttccagaga 153240
tgaagtagaa aagagaatgg ctgggctgca gaccaggggc agggcagtgg aatgcggagc 153300
gtccatagct gagctgtggt ttgttcacgt gacggacggt cagggaccag tcacggtagg 153360
tgaaacgtag ctatggatac aaacatttct gacctagatg gaataatagg gatcgaattt 153420
accctactgt ccgaaacaag ccccaaataa acaaaacaca cgtgccgatg accttccaga 153480
ccatggacac cagaccgtaa gggtcagtgg tccctgaggg aagccaacaa ggaggccccc 153540
ctgccaccct gctcgctgcc tcgagggagt ttctagattg cagcacaggg agggtgcagc 153600
ctggtggact ccctgagctg agacgctgga aaggagagcg agggaaacgg aggtggctgc 153660
agtttgcagg ccagagcgcc agagaggagg tggccacaca gagagaagac ccgggacaat 153720
ggcccaggac ccctgctgag ggttcagacg agcacatccc gctgtgaatg aggaggctgg 153780
ggagagatcc gccgacagga ccagagggaa ccacctctga tgcccacaca gggctgggca 153840
cggtgcctgt tcccccagcc aggagaatgt catgattctc ggggatctgc atggggtgtg 153900
cataaggtct tgcttcagta ggggttggtt agggctacac caaatgctga agccaaataa 153960

```
tacactttttt aggaagctag aaatatctag agatgcaaca aatgaaattg taagaagggc 154020
aagggaaaga tcaagctaga cgcagataca gaatgggtga ggggcaggca gtttccacag 154080
tgcctagttt tcctgctgtg ggctcacaag ggtctgtttg ttacggtgac aaattaataa 154140
tttaaaaatg gccacatgtg gattaatgat aagatgcgtt actcaactgt gcacctaaga 154200
ttcaaatgaa aacaaaatct aagtgaggta agagacctgg aggacctggg gcaaagtgga 154260
cacaccagag cctcataatg gaaaacacag aaaggcagaa ggcaatcttt cagtgtgtgg 154320
ctgcaaaatt cacagaatga caagatttgt gaatccacag atctggccac aaaacatatc 154380
cagagaggaa aaataaaaag aaaccaaatt aacggtggtg ggtgcctgca gtcccagcta 154440
ctcgggaggc tgaggcagga gaatggcgcg aacccgggag gcggagcttg cagtgagccg 154500
agattgcgcc actgcactcc agcctgggct acagagcgag actccgtctc aaaacaaaca 154560
aacaaacaaa caaacaaaca aaaaagaaac ccaggtcccg acactccgca gtgaacccga 154620
gacaccgaaa gcagaagaaa ctcggccgag tgcaggggtg aggacagagc ccaccacaga 154680
gcaggctcac aggccacaca tctcagcaca gccacggaac actgctgcct ccaaaacgca 154740
gagatagtcc ctgtcagcct agagccctga gcccacaaca gtgctttcgt gaatgagagc 154800
agcataaaga tgttcccaca caccaatagt gagggaggtt agcaagagac ctcctcaaag 154860
gaatgtctga aggatgcttt ttgaaaagca agaaaatggt cctagaagaa aagaagtcta 154920
taacaaaagt atgggaacca acaaacacca agcatgcaca ttagtccaag gaacatgatg 154980
agtccgtaca aaataataac aggaacaaga acaatgacag tgtctaattt gtgggttaaa 155040
aagagagaaa tagatttgaa caatcatagc atgttcatca gtaggaggaa gatttaagtc 155100
cttataatgg gatggggtta aaatatggtt tctctgtaga gtttaagtga aatatgtatg 155160
gtacaacctc aagagtagct attaaaggaa tagaataagt ccgggcacag tggcttacgg 155220
ctgtaatccc agcactctgg gaggcagagg tgggcagatc acttgagatc aggagttcga 155280
gaccagcctg gccaacatgg tcaaaccctg tctctactaa aaacacaaaa attatccagg 155340
tgtggtgctg ggcacctgta atcccagcca cttgagaggc tgaggcagga caattgcttg 155400
aacctgggag gtggaggttg cagtgagcag agatcatgcc actgcactcc agcctgggtg 155460
acagagcaag actccacctc aaaaaaaaaa aaaaaaagaa taaagtatgt aagttctaag 155520
caagtagaga gaaataaacg gaataataaa aatgagtgaa ccaagaaaaa tcaatctata 155580
aaaaggcaat aaaggagaaa aacactaaaa atgggacaaa tggaatagaa agatagtaga 155640
atcaagttca aatatatcaa taatcacaac aaatgtaaat ggattaacct gtccaactgt 155700
cagcgtaaaa aattctaggt gtatgctggg tttgcaaaag acacttaaat cttaaggtta 155760
ctgcaaagtt gaaagttaac agattgcaaa agatatttca ggcaaactca aaagacacct 155820
tggtgcaact atatttgtcc cagacaagtc agactttaag accaaaagca tcattaggga 155880
gtgaagatgg gttcatgtca ccaggaagag atgatcattc tggacatgga tcaacctcag 155940
aaaacagcct cagactacag agagcaaaac ctgatagaac tgaggggaac agcaaacaaa 156000
tatactatcg aggaagaata tgccacaggc atctctcaat tactcatggt caagcacccc 156060
taaagttgac aaagtgtaga caatgtgggt aacataatat ataaccctac tcaaatggac 156120
agaagcagaa ctgtgtattg acaattagag aatagcacat tcatctaaaa gatgtgcaac 156180
gtttatcaaa gttgatcagg tataaggccc taaagaagtc tcaatacatt ttaaataata 156240
ggtatcatat gtgtttgaga acaatgaaat taatttggaa attaaaaaca aaaagatgag 156300
taaaacatcc ccatatattt ggcaatttaa aagcagactt ttaagcaagt tatgagttga 156360
caaagtcaca atggaaatta aaatatactt agaactaaaa gataaagaaa aatactatga 156420
aaaaaatacg gaatgtagca catggagtat tttgagggaa attcatagcc ttgagtgctt 156480
acattagaaa agaacgaagg atgcaaatta ataacctaaa gtccaaatta aaaagttagc 156540
aaaactacaa cagagtagat ccaagaaaaa gagaggaaga aatgataaat acaggagctg 156600
agattaatgt gacaggaagc acagcaagaa tctcacatga agtcaaagtt ggatatttga 156660
gaagactagc aagatcctgt acaaagtgta agaccccccag gtataggact ctcagaggag 156720
agaggctctg gtggagcagg accggaggcc cagtggggag gggctggcat cctctggatg 156780
gatgggatgg gatgcctgga cacgcctcct gtgtttggag caggcaagtc actgggagga 156840
cagaggaaag ggtgccaacg gagggaggca gagcccacac cagaacctga cttgcaggta 156900
ggaggaacaa gctgtgcaga tgtgtgctca gaggcggctt ctccccactg cctcccggcc 156960
tgttccctgc tcagcgtctt gttctgcctg acgtcaccac ccacctccca ggcacgcaga 157020
gccccctccc tctgcctgta tccttgctcc tgccccatgt gcaggtccat gctgggctcc 157080
tcaggcatga gctcatgaga cccctgctgg gaggactgtt tacgagaacc agccacagag 157140
aggtggccag agggaagagg caggtccgag gtggggccgc ggtgccctcc acactatggc 157200
cttctctccc ttttgctgtg cattggactc tgctgtcggc attaggctga cattagtgta 157260
tggaatcagg tcggggctga ggccactctg gggtggagga ggacagtgcc acaggcccag 157320
ggcacagccc aggctcctct cctgctggcc agctctgtga tcttgggcac attccggggc 157380
ctctctgggt ctcagggacc ctggtgtaga gagagtgctg ggctgcctct tagggttgtg 157440
ggagtaaaaa cacgtcaagt gtttactgtg tgcccccggg aatgcacctg ctccacggca 157500
tgtcatgaac atgttcgcac acagctggat ggctcccagg gtgcaggaaa cacctggcgt 157560
gaccatcctc tcactcgaag ccagccccag gctcccaggc ctctcccagc ccctccaggg 157620
ctccctgtgg cttcccatgg ccaggcccag gcctcccctc ctggcactgt cccttgtgcc 157680
cccgtgaggc ggggggtggt ggtcccttgc tggtccttaa cctggctcag ctggccccac 157740
```

agggcactgc tcccgtggtc tgggacatgc ccaggtcttt gcagggacca tgctttcctc 157800
aggcgtctgt gcagccgcac ctcccagggc ccctgcgtca tgtgcccccc gtcccgggca 157860
cacgtctcca ctgtcacctg ccaccactgg ctgcctgtcc tcatagagca catgctggct 157920
gagtaaatga gcaccatcta caccagcctc ggagcccccct gggggctaag aacactgagc 157980
ttgggatcat tctgaattcc cccgagagag gcccagcagg gacctcgacc tcaccgtgaa 158040
ctggcgcacc tcccctctgt ccaccagctc acggtccttc tcgggtgcaa tggcgtaggc 158100
cagtttctga aagagaaaga gagtgcaggg gtcagtgcag accagtaccc actgccagca 158160
cccaccactg actcacactg ccaggaccct cacctaccag caccctcacc accagccctc 158220
ctcacctgcc aatgcccacc cgctggcatc catatcacca gcgcccacca ctgacttaca 158280
ctgccagtgc ccatgccgac cagacagcca gtcaggcccg aagccaacat cctgacggtc 158340
cccttaaagt ctagcgcaga gcagaacatc agcatttgag cactcgcttg aaaataaaac 158400
gtggtcctgg agatggctct tgagtctaag ctaccacgtg tgctgggctc tgagtaagaa 158460
agtgcccaca cccaggtggg ctgcctcggg gccacctgca gctgacatat tccgggagca 158520
cttgcttttt gtcacctgca gctcttggga gagtgaacgc tgaacgtgcc ttccttccac 158580
tgcccgctgc ggcagctcct ggggcctgga cagcaccaag tcctcccaga gcaggcagca 158640
tcttcaggag ctcaccctct cgggcctgga tgcctgtatc tggatctctg aggccaggtg 158700
acctctcaca gggaccccctg tgcgtggccc caccccacgt catcaccaca attccacttg 158760
tacctggctc agctgctgag gttttacttg gcttacgttc agagtttggc catgcgaggg 158820
gtaagggcgc tggctgttca ggtccgccgg gctgtaggca tcctccagct ctgcgctgac 158880
ctgggccccg cctcccacgt ccatccagcc tcccagagga gctctctccc tgcagagact 158940
gcccggctgc ctgctcttcc cctgtgctgg gtcctggcag gtcagcccgt gggggtctga 159000
gagggactgt ccacagcagg gtgccatcag ctttcctggg gccatggcag ccccagagct 159060
cctcccactc cacagaagcc cccagccaca cactgtgctt tgcctgcact gggccttggc 159120
ctggccaggc tgctggagag actggctcag tgccagcctg agagcatctg ccacatgtct 159180
gagcccaggg gacaccgtgt ggccctaagg tggaatcctt gaacacagct ggtgctgctc 159240
tggggttttg atgcccaggg ccaagcgact tctttctgag aaaggctggg atttgaggaa 159300
gcagcttccg ggagttccca gtggtgggat ctcaggtgag ggcgaccctc ttccaaggag 159360
ggagtgtcct tgtctcttcc tggaaccaca gagccagggt gttaaacagg gccctgtgac 159420
tggtccacct gcctttcttc tacacaaagg cggcaaaacc aagcagaaga aaggagatgc 159480
atatcagtga ctccgcaaag cttcctccac tgcctggggc tccagctgcc cctgttcagc 159540
caaagttggg ctcggccctg gggggaccta gatcacccct gattctaccg ccctcagatg 159600
gcttctgtgt ccaccacacc acataccata gtgtgtaggc atgcatgcac tcagtacaca 159660
caaacacttg gacacgaatg tgagcacaga cccaggccca cacacacgtg cacacggaga 159720
cagcacatac agacacagag atacagtgga tgtacacaca ggcacaagca cacacagaca 159780
catgcacaca cagacacaca cagacatgtg gacatactta aagacacaca catgaataca 159840
catatggacg cacacccatg ggcccgggcg tgcaggtgga cacacacacg cacacacaca 159900
cacagtgttg tggtggccac ctccagtctc ctcctcttgg tcacagatga cccaggcagg 159960
tgaggactgg ggccatggac acccacggag cctttctggt ggcctcacac tcgggtgaag 160020
gcgccccgtc caaataccca cctctcgaaa ggacccaggc aggctgcaga acttgtaggc 160080
cgcatagatg ggcaccatgg ccatggagga tgtggcgatg acccagccca gcgcgttggc 160140
ccagtcgggg aagatgtagg ctccgtagtg ggggggtctg aaggtcacaa tgctgaccac 160200
gaccacgaac tgcaaccagc agatacggag gtcaggcagc tctcagccgg cggtgccagg 160260
acaaagcagg gagcgggcag gcacttcatg gcagagcgtc tctcagcccc cacagctgtg 160320
caggtgcaga ccccggccag gcctgcagac atggcagctg ggcatcccgg gcaagccatg 160380
tcctcctgaa gcatgatcta gcctgtcctg tcctatcctg ttctctcccc tcccctcccc 160440
tcccatccca tcctgttcca tcacctctcc tcccctcccc tcccatcctg ttctgtcccc 160500
tcccctcccc tcccatcctg ttccatcccc tctcctccct tcccctccca tcccatcctg 160560
ttctatcccc tctccacccc tcccctccca tcccatccca tcatgttcta ttccatccca 160620
tcctgttcta ttacatcaca tccggctcta tcccatccca tctcatccca tcccatccct 160680
tcctgctgta tcccaccttg ttctattcct tgcttgaaat cacaacaaaa acaaaactga 160740
ccaccaccca gtacactgaa tctatgatca tccatggcct gaagacacaa ctgtgaccca 160800
tgcgggacca ccctagggcc atctgagctc cgtatctcta ctcattctag aagcaaatct 160860
gataaagtca cagacctgtc tcagaaccac caccacaaag gtaacagagg gtggggtgga 160920
tgccacttgg aggggaagct tcccactctc cagaggcttc tcctgaggca gctcatgcat 160980
ttgttcatcc actccacaaa catgcaccac catctaaaaa gatcctccat gtgcctacat 161040
atgcacctca cacctgaatg tgttctgtgc acccacatgt gtactgcacg cctacatatg 161100
tgttctgtgt acctgggtcc ttcccaagga cacaagtgaa gctctgatag atatggttta 161160
tgatgtagct taaaatgcaa ttttttccca attgtccact tttcctcaat ttcagaaaag 161220
ttaaaacatc caagccttcc acctgaaaag catgttggac gcgtgtctca tgccagtctc 161280
agcatcttct tcatgcgcta cttcgggcca cttgtaggtt tggagccggc tagcggagga 161340
ctgggatggg ggctggggtt gtgtctgtca cgtgaccaga gccagggtga gcagcacttt 161400
ggcgagcaaa gccagtggcc tgcagcatct gggatcaggg gcggctgcct tggctgcaca 161460
gagagccctg agcatgcttt aaagagccac gcgtgcatcc ctggacatgt cgggcttgcc 161520

```
tatctgacat ggaggtattg ctgtaatgtg ctctctgtta ttgttcaact tcttgctaga 161580
taccaccttt cttagaaaga actatgcctc cccaggccac agtctgccca tgtaagttgc 161640
ctttgctccg gcataatttc cctttagcag tgctgtggca tcatctgcca cagagtcatc 161700
tgaacgagac tggggcgtgt acacccctta cgaccagagg ttagcacacg ccagagggca 161760
ggggactatg ccgcagcctg aagaaacaga tgataacaca gacgttatgt ttctgctgaa 161820
atcagaactt cataggaatt gtgctgtcat attaaataca aaatgacata gagcaatgaa 161880
attgtcatgt ttctctcaac ttggcaaaat gaagtcctgt ttcgtatagt ttgtagtttt 161940
atctaaaagt ctacgaagtg tgtttctgta tgaagttcga agatttagtc taagatcagt 162000
agaacatttt atctcctaag atcgaggtca gccctctggg tgttaagagg agacaccccc 162060
tcaaggggag ccaacgtcct catgcaaaac agctcagttt caactaatgg cgggggagag 162120
cgggaggtgg gcaggaggct gactttctgg gagggcttcc tcactttccc ctctgaacag 162180
ccatgtagtc tgtgtgctat taaaagcccg ccccatgaat ccaggaacct tcaacgggag 162240
ccttcacaag cgcagttaac tggacttgga acacttacgt gcagccacac cacgcggcct 162300
ggagctcaga cacggtggaa tttctacaag atgccgggca ggttctctca cactatgttg 162360
agctcctccg ggatagggcc tgtctctcat ctctttcctg taccccggaa cccctctgct 162420
ggcaactacg ggctgtcccc acctgctgtg tgcaggtgac gaggggtctc cacgcacgcg 162480
ggccctgctg actccgggac cagcccttgg tccatgagag ggtgcggcct gagtcccctg 162540
ccctgttcca gtccccacct cgtgcctgct ggactcggga ggtgcttctc tacgtgagca 162600
gctcacggga cactctgctt agacttggac agctcttagg ttgcctgccc agatcaggca 162660
gcacagcttt gctttgtgag cctccaggcc tctgtggttc agtgggggaa gcactgcagg 162720
caatccctaa tgaaagtgtg cggccacctt ccaacaaaac tctatttaca aacaccagcg 162780
tccgacggcc ttgcccggtg ggccctgact cggtggcgga tgacagaagc aagtgcctac 162840
tggacacagt gatgctgctg agccagaggg aggcttgcgt gagcaacggg acagggccga 162900
ggcccctgct gctctcatcc agcactcggc atcctttgtc cccactgccc cggaagctct 162960
aacttcgacc ttgatcctca caggcagagg tgagtggaca gcccgactca cctccagctt 163020
cccctcccaa cacagaggcg cggcccaagt gcaggactca caacggactg tgacaggggt 163080
ccaagaccat gtgaggtttt ttcggtgggt ctagagggga gggtggaagc caccttaagg 163140
agactataga tgagttcagg gatcagcctg tccttctggg ccgagtcttg aggcccctga 163200
ctccagccac agtgacaacc cacatgcggg cgctggacct cggggcaggt gccagagtgg 163260
gggcagtggg cagacggggg aagggcaggt ggcccgaggt cccttaccag gagaaagcag 163320
gggctgacca gcttccagca cagccgccag tacaggctgg gccgctgccc ggtcatctgc 163380
tggatgtcgt cgctgaactg cccaacacct gagggagaag aggtggcatc agtgtccatc 163440
agggcagcgc attcccccga tgctggacac gtgtgggggt cctcgctgac tcccaagggc 163500
cccacctacc ggccccaggc ttcggctgca cccagcctcc tgcagaggag gccaggccac 163560
accccagccc actgggtgcc tcgctgacgg gtgggagccc acgtgcctgc tccaccctgg 163620
agatgcacca ggaagagccg tgccccggtg ggtgctcccc gcgccccggc aacagcccct 163680
cgggtcctcc tccccatccc atggctctcc ctgtctccct ctgctgctgg tacttcttgt 163740
tcacttaaaa agattatggt aaaatacaca taacataaaa cttaccattt tagccatttt 163800
tacgtgtaga gttcataatg tatcaataca ttcacatact gtgtaaccgt caccaccgtc 163860
catcttcaga actccatcct ccaaaatgaa attctgccct catgaaacgc caactcctcc 163920
cgacccccaa cccccgccag gccccagcac catctgcttt ttgtctcaat gaatgtgact 163980
cctgtgggaa cctcccgtca gtggagtcac gtggcacggg gcctctgcaa ctggtttact 164040
ccctgtagag tgtccctgag gccacctaag ctgcggccca catcagcatt cccttttgtt 164100
ttaaggccga atcatattcc gtcatgagta taccgtcact gtgtcttcag aaaagctatt 164160
tctagaaaca taataaacat tttttctccc caaaagcaag taccagcagt ccactttaga 164220
aatgcatatt tttctttcac tggtattatc taacttcttc taggaactga ccctaagacc 164280
ttaagagtgg tttctccccc cacatctggc ctggtctgtg actcgtgtga ccgcggctga 164340
tctgctgaga tttggggcaa gtcttgcggt tgtcccgtgt cccatcagaa agaagcctcc 164400
acacagaatc ccctcttcac acccacccgg catccgagct ctgaacatga gagatggacg 164460
tgggaggcag cggggatgca gagaggtgag gccggaagaa gccaagaggg gcgtccccaa 164520
ggcccctcgg cccccagacc ctgcctccca ccagcaggtc ccagggcccc tgccggagca 164580
ggagtcatca gctccaagac ctacaggacc gcagcgtgct ggaagcccgg cgtattccgg 164640
cacatcttat tagagcacag gttttgtttc atttagatga gaggggcgtg gatttctctt 164700
tggaaaaata acacagatct cggtcttctg aggtgcaggt cgccagggcc ggcttctccc 164760
catctcccgt ggttccggag gccatggcaa ttcaaggcac tttctccctt cctctgtgaa 164820
ggtctatgtt ttgttcaggg aagatgacaa tgaaagtgat agacaaagta ttgattccag 164880
acacccgtga aagtacgagg ttgatatttc cgttttctgt agagcaacac tgaaaacaat 164940
gttctcagaa taggagctat ttgtcctatt tgtctcaaag tcagggcgtt tatctggcta 165000
taaattccat ggctctgagg cagagaaaat gaccccttg tttgcaggtg agctgggcgt 165060
cctccaaaac cggggctcac gcagcaccac agacattcag ctttcagccg gatccacaca 165120
ttttttttt tctttttga gacggagtct cactctgttg cccaggctgg agtgcagtgg 165180
tgcaatctcg ggtgactgca acctccgcct tccgggttca agctgaatca tattccatcg 165240
taagtatacc atcagtgtct tcagaaaatc tacagcctcc tgactagctg ggattatagc 165300
```

cttgtgccac cacacccggc taattttttt tttttttttt tttagtaaag atggggtttt 165360
accatgttgg ccaggatggt cttgatctct tgatctcgtg atctgcccac ctcggcctcc 165420
caaagtgctg ggattacagg cgtgagtcac cgcgcccgga ccacacattc atggcgagat 165480
gccctggctc ggcctcgcca cttccctgga agtgagctgg ccaggtaagc ggagctggca 165540
ctcctgccca ttttacagaa gagagaacta aggggagtcg aggtgacttg gccaggtcag 165600
catgtgggga aagggcagcc ctggctccag gctgggtttt ctgttgctgt ttaaaccact 165660
ggtggaagtc ccaggacaca gctggggcag cacaatgggc caggagctgc accccgttcg 165720
agcgccgccc gctgatcatc aggtcccgac tgctctggag ctctggtcag cagggaaggc 165780
gatgacggca cctttgaaag gcaccatgtc acatccctgc tgaaacccca gcatggatct 165840
gattgtgttc ccctgggtgg gatgggctca ccggtgcctc tagcgtggag gaggcagaag 165900
acgcccagcc gctgtgaaca cctctgacca cagtgtgcct cttctggctg ccatccaagc 165960
taagcacctc actgatccca tcaaagggac cagtgccaag cctctcccct ctgcggagct 166020
gtgatgacca caacccaggc ttcctgcagc tgaaaggtgt ttcctcacgg agcctttttc 166080
agaaggggag tggcacagcc accaaacaag agggtgccgg cttggctgcc ttcccccgga 166140
ctcaccatag aaccaggcca ctccgatggc ttcgatgagc actccaaaga ggatggacgt 166200
gccggctgca aaatggtcca ggagcgtgaa gacgtagatg ccaccctgga agagagggga 166260
gcctgtggac ctacagaagg gctttcccca gaggtaaacc cagcctgggg attcactgtg 166320
cacacaaatt gtttcactca ctgcaaaact ctggtttgag tccctaaaat tcaacccaca 166380
tgcagcttct tagggccacg tgtattacaa aaaataaaaa taaaaatgac ctctcctttc 166440
ccttcctagg gcttcatgtg caattagaaa tggaagcaac cgaatgcgga acttggcagt 166500
gcctcccttg gagagggagt ggcctgggct cccccctccca catagatagg ctctgccacc 166560
acacccccag cgtcccccag tccccaaacc ctagaaggag gcagcttcag cctggggtgc 166620
ttctggggga gctgtccagg tgctgaagtg actctgggac caagctctgg atgtgcagtt 166680
accctgtgca ctgctacgag tcattttctg gggtgggtgg gttcgcagct ccctggaagt 166740
gctgcggttc tgtctggcct gacagtccca gccagggcgc cccgtgccac gtgctaagga 166800
gacatgacca ggagaaggcg aagccggcga tggtacgtac gttggtgacg cagaacaggg 166860
acaggaggaa ggtcgccagg acgatgaaga gcgtgaagag ctcacggtgt ctgtgcagca 166920
gctggaactc atcgatgagc ccggtgatca ctgactccat accacccatc tgcacacaga 166980
gcacagggtc gggctgcagg ctggcggcgc tcctgaccgc agcctgggcc aagacctact 167040
tgtcacccag tggacgcaca cccggggatg gacacggaac aggggccaca tggccgtcca 167100
gggtgcagga tgcctggtag tgaggacagc tgttggcgag ggcttctatg gcggcacgac 167160
cgcaggagag aggacccaga gggaccactg tatccacggt aggggggtttg cagcctcttc 167220
gatgtctctg ggtgggtaag atgcggcatg gctaaaacaa tgccaccctg tccactgctg 167280
ctgcacgggc tcagcttggc cagattttcc tgagcaagat gcgtccccca cacaaagctt 167340
tcagctgcac ctggccttgt cattggctac gccttcccga cggagcctcc tgactcctgg 167400
catcctcctg tgggacaccg tccaggcacc tgactcctgc ttagtgacct tcttcacctt 167460
gctcggtcca gacccctctt catgctctca tgtcaggaac atgaggcctg tggtcaaccc 167520
aggagccgtg gacgaggtgg ataggatatg agtgggggc cgggtgcatg accagcgttt 167580
ataaggcaag ggattctggg ttctttggtc ccaagcagct gacagggaag aaggagcctg 167640
agaaagtcca gcaggcccag ggccgatcaa gggctggacc cgccaccccc agcagctgga 167700
ggtgcagggg ccacacacaa agcattcagg gccaggcctg ggattggtag gtgccaggaa 167760
cttctgttgc cctcctcatt cctcatgggg tagctgaggc ttccagggaa agccacgaga 167820
tatgaaggac ctgctgtgtg ccctccaagg ccccaccact ctggtgtccg gagccaagtc 167880
tttggtggct ggggccctgc tacacgtgca tgcatgtctg tgtgtatgtg tgtgtgcatg 167940
cgtgtgtgca tgtgtgtgtt cgtgtgtgtt catgtatgtg tgcgtgtgtg catgtgtgta 168000
tgtgtgtgca tgcatgtgca tggtggtgtg aatgtgtgtg ttcgtgtatg tgtgtatgtg 168060
tgtgcgtgta tgtgtgtgtg tgcatgtgtg tgcacatttc caggatctgt aggtggctca 168120
gggcagggca cacaggtacc ccagagtagg gggataaagg gaaaggtaaa ctccagtcac 168180
cactcactcc agccccgaga aaggtctccc aaataatcac ggggctcgcc caagtcaagg 168240
acaggaggtc tgggggccgt acgtgagccc agggatcttg cctagccctg ggagggcagg 168300
gccccctcgg gtggaaggaa cccaactgcc gaggacaggg ccgggcggtg cgggttactc 168360
acggcgctgt cgatacccag ggtgagcagc atgatgaaga agaccacggc ccaggctgag 168420
gacagaggga gcgtggcgat ggcttccggg tagatgatga agatcagccc tggccctgaa 168480
acagaacccg ccctgcttgc cacagagccc acgctgtgct ctcccgccca tcctgcccca 168540
ccccgccccg agaagcatgg cctgccacag gcctgtagag actagggctg gtgcggctct 168600
gctgaaaagc ccccttctat atgtccagca gaccaggcct gggactcagg aaagcggaaa 168660
cccagaactg atcagagggc tttggttcat gcccaccacc cagcaatggg gctcctccaa 168720
attacctggg tccttttggg ctatgggggt gcttggggaa ggaaggggca gaaacaagga 168780
ggagcaggag gggcttccag gctggtcctg cccttcatcc cagggacatc tgctaatgtc 168840
cttcgagtta gtttttcctg cacataccat gcaacataca cactcagaca cacataccat 168900
gcaacataca cactcagaca cacataccat gcaacataca cactcagaca cacataccat 168960
gcaacataca cactcagaca cacataccat gcaacataca cactcagaca cacataccat 169020
gcaatataca cacacagaca catgcgcgca catgcacgaa cactcatttg tgcattcaaa 169080

ctcatacatg caagaacaca tccacatgca cgcacacaaa cagaaacatg cacgtgcatg 169140
cacaaacata cacaaacaca cacaaatcac acacgtccac actatttagt agcctaattt 169200
ctaaaattag caaaacattt agggatacat tcctgacctc cgccttccag gcacatgagg 169260
aagctgaggt cctcccaccc tcccgtgagg ttagacgtgg ctgcacttgg ccaatgtgct 169320
gtgaacccac agtgagcggg tccctgcgag tgaagcttgg tagaggagtg tgtggctcct 169380
tcaccacctc ccctgccacg gggtcccgca ggccatattg agacaggctc ctgtcagctg 169440
gggccctgag ggctcagagg agcagcacat cctgatggat acaggggccc agatggtggc 169500
ttcggagata agagacaaac ctgctacctt gctatccctg caagcgaggg gcacgccagg 169560
ctgagggcgg catggcaaag gcggaggagg tgttccccca cagctctctc gggaaacgac 169620
acgtgcttcc tgctaccagc aggcagactc ggatggaggt ggaggggacg agagtgtggc 169680
aggcaggcga ccaaaagtca acgcagtttg gtctctgatg accccaaagt aaaaaccagt 169740
cccataggca attgaccttt gaccagttca gaagccttgc atcctccact ggctggcacc 169800
atctcccaag cccaatctgc ctgcaacaga gtcccctgtc ctcagcaggc acgtgtctgg 169860
ccctagcctg caggaatagg cctgcgcctg gccttttaaa taatcaaagg ccagagccag 169920
gggccgttcc taaaggtctt tcaggggcct cactatcagc caagcagcgg ggtcagacct 169980
gggctctcgc agctcccaga taccctggtg gatggggaca gcctccgcgt ggggtggggg 170040
aggcaggctc gcctgagatg ttccggggct tagatggtac cagccaggag cctgggaaga 170100
tattcatcag ctcagggaat gtaatgtcaa cacccgtgcc ttcgtttctg tacctaagtt 170160
aacatttcac cctattttct tgagaattaa ggctgtaaat gacatttact ttgcttgtca 170220
tgaaatgcac agaagaacat tcaactttat tttcaggggt taatagaggt ttttgcaaac 170280
tatgctcgtt atcataataa agggcaaaaa gaatgaagaa aaggcataaa tgcggcaccg 170340
agtaaatttc actgctctgc atttatttgc agatgttccc tttctcccgc tttgactgaa 170400
tttttttcc tagtggaaag aacatgggct taggaggccc acctctgagc tgtggctcca 170460
gccccgaccc tggctttctg ttgcaagcat ggagtgggcc ctttcaggtc tctgagcgtg 170520
ttcctacctg cggtgcgagg gtcctagtgc cccactctgt gagactcagg tgcggtcaag 170580
gctgcccccg cttggtgctt gtcgctcccc gccttcactg agcctcaaca cccaggcaga 170640
gatcagtcag caacaacacc cacacgggac aggggctttt ctgagcacaa tcattcctca 170700
caaatgtagt gcccccaccc cacccccgga gaagacagtc cagaggaagc aaggatgggg 170760
acgcagcatg ggagcccacc caacccaggc ccgtgtctgg agcgaaaggg cctcctccac 170820
atccatgtgg cagagcaggc cgggaggcag gcgggggctc tgctggctga cagagaccga 170880
gaagccttgg gactccctgg agctctcggt tggcccaagt tagggctgcc aacgccacag 170940
ctttccactg caagcaccgg agccaggcac gtggccccaa gtgcctgggc ccactctcag 171000
cccctgcatc tgtgcccgtc ctgccggctc catcctgcct ggcacacttc ttgctgtggc 171060
caaggcctcc ccccaccact cacctgtgcc tgcccgagac ctggacccct ccccaccctc 171120
tcggagacaa ctccaggcgt gcagggacac tcagcgtcat gcgtcggcct ggggagcggg 171180
cagggacacc ctgtgctgga ggccccaggg atctcccggc tgctctgtgg ccaagctgat 171240
gtttgcggca tcccagcctg cctccaggtg ttgctggaac agctcattcc tccccttccc 171300
tgagctgcat gttcccacac cgcgtggcaa gcaaacatct agagggccct cagtgacagc 171360
atctacgcca gaccctggtg ggcagcctc atgtggcatc tgcagggagc agtcgaggga 171420
ctgaggcacc aactatgctg ctcagctgca tttgggcttg aggttcccca gcccagggtg 171480
gaggccccga gaggcacaag gcaggaaagg cactgggtgg gggggcctgg aggggcaggg 171540
cggagaaggc actgggtggg gggccgggag gggcagggcg gggaaggcgc tgggtggggg 171600
gcctggaggg tcagggcggg gaaggcgctg ggtgggggca ggtctgtact gggatgtgta 171660
ccaccatcct tcaagagtga ggcagcaact ctcactgaag tcgctcaggg cccatgcgtc 171720
tccttcctct ttcacaagga aaaaggcttt gctgagagct cggcgctggt gctacacgga 171780
gcaggcccag gtgcagcagg aggggctcac cgtccttggc cacgtccccg atgggcacac 171840
tgtgcttctg tgccatgtac cccaggaagg agaagacgac gaagccggag gagaagctcg 171900
tcagggagtt gatggaggtg gtgacaatcg cgtccctgta agaacaagac acgccgtctc 171960
aggaaccagc tgagctgcag cagctgcaat tttccagtca ttattcttaa tttactgtgt 172020
ctggggaagg gggcgggagg tcttcggagg ggctactttt cccagtgagc acggccagct 172080
ccttagcagc ctggcagagc tgtccttggg ccaccttggt cccctagctg agctgctcaa 172140
ggctcagcca tcaaggctca gggtgggcag aacagacggc cacccctgtg ccccgcgagt 172200
cttgctgtgg acacctcacc cagggcagat cttccagaag ctctaggagc acacacctcc 172260
cggggacctc gctaagatgc agatcctgac tgggcgggtc tcatggggtc tcgggggctg 172320
tgtttcccat gaactcccaa gggcgctggt gctgcgggcc tgggggcccc actctgaggg 172380
ctgagaaacc agagagctgg aggcaagccg aggacgaaga gggagggtcc tggggtcggt 172440
cagggcacca gcgtcctcct cctgggttcc tgtgtggcct gtcactgggg tggaaggacc 172500
acacacttca tctgcagagc acctggaccc aggtggacgc aggtgggctg cgggcaggcg 172560
ctgcctcagg acctgccttc cagcgcccac tctcagtgaa gcctgcttgt ggtctgcctg 172620
gccattctgc tgcacacact gtccttgtat ttttaccgtg tttctagcct catgatgtga 172680
aagaaagtaa gcacaagtcc ataaaggtca aagcaagcag atcccggata aaattctgtt 172740
tctgttttta gaaggatttg gggtccggca cggggcttca ggtgcctgcc agtaagtaac 172800
acggggtgca tatgggatgg ggggacacac tcaggggtt gtgaagctgg accctgagcg 172860

agggaggaca gagaccccccc ggggctggtg tgagggaaga gggtgtgtcc ttgtggaggg 172920
aggggctggc cttgccactt ggggccctca gtggggaatc tggccgcagg tcaaacctag 172980
gcaggtccct cccaggaggc gacacaccca ctggcttcag tgccttcttt aaagttgaaa 173040
tcaaaatcgt gttgggagct gcctcgctgt cgcttctgtc tgaaaggcca cgcagctcac 173100
aggtttgcgg gttcagtgga tccgccctgt tctcatccag ggacacccta tttctggaag 173160
tcagcgacct ctccctagta ttgatgaggc ccctgcctgg ccctgctagg ggctcacctg 173220
tagcagttgt tggtgaactt gttgtagctg gagaaggcga tcagcacccc gaaccccacg 173280
cccagggaga agcacacctg ggtggccgcg tcaatccaaa cctgcagagc cagagggcgg 173340
tgagaggctg tcccaggaga acgcaggcca caggcaaagg acctgagcac ccttcctgtc 173400
ccagccccac actgaggcct ctaaactcaa caccatcctc aagaagtaaa tggcagcaga 173460
cgactggtgg aagatgcagc ctcaggaagt gaaccctccc gggccagcgg cctggaagag 173520
cctgagaagc agggggctgt ctgtgttcat tgatctggga ttccccatgg agtatcaagg 173580
cagcgattcc acagaaccag aggtgccctg ctccatagcc ctcagaacag catggactca 173640
tccacacacg gcatgaccac agcatcctaa taaccctcgg aacagcacgg cctcatctac 173700
acaaaacatg accacagtgt cctaataacc cttagaacag cacggactca cccacacaca 173760
gcatgacctc agcgtcctaa tagccctcag aacagcacgg actcatccac acacggcatg 173820
accgcagcgt cctaatagcc ctcggaacag cacggactca tccacacaca acatgaccgc 173880
agcgtcctaa tagccctcag aagagcacgg actcatccac acacaacatg accgcagcgt 173940
cctaatagcc ctcggaacag cacggcctca tccacacatg acatgaccgc agcgtcctaa 174000
caaccctcgg aacagcacag attcatccac atgctgcata atggtggcac cctgataacc 174060
ctcagaaagt catgaactca tccacacaca acatgaccgc ggcgccctga taaccctcag 174120
aacagcatgg acttatccac actcacacca gccctagcgg ggtctccatc ctcccttgat 174180
cagggtccag cttcacaacc ccctgagtgt gtccccccat cccatgtgcg ccctgctgca 174240
tgatggcagt gccctgaaac cctcagaaca gcacagactc aaccacaggc tacatgatgg 174300
cggcttcctg ataaccctca gaacatcatg gatcatccac aagctgcatg atggcggcac 174360
cctgataacc ctcagaacaa cataaactca tcctgaggct acatgatggt ggcgccctga 174420
caaccctcag aacagcatgg atcatccaca tgctgcatga tggcagcacc ctgataaccc 174480
tcagaaaggc atggacccat ccacatgcta catgatgatg gtgtcctgat tggacgcaca 174540
ccagcaatgc ctgctttgta tccttcccaa aacatgagca gtacaggtgc acagtggcac 174600
ctgggcatgg ccaggagccc acctgcagct tagacactgg tgctgtgcct gcccaactcg 174660
gtggatggca cccacaactc tggacgtgaa aggaaaggtg ggtgggtcca cgattggacc 174720
attgtggtat tttaaaaagt cagccacaac ctgctaaggt gagggcactg cggagaccat 174780
tcagtgactc agatgcccac cttgcccttc cctgggcctt ggatggggga gaaggagcag 174840
ccaacccccc cttggaggca tttgagggac tggtcagtta gggctgttcc tgcaagtccc 174900
tggagcccat ggtacaggcc tgtgggccag gatgctgagg cagagacacc agatgctcct 174960
gctggggagg ccaggtgggt ctcagggcat cccaggtccc aggtcaagct tgcctgagct 175020
tgactgagca agggaactgg gtgctgcaga gggggcgggg ccctctgtcc tttgggcagc 175080
atgtcagagc ctcaaggcaa ccccagttcc tgggacagtg tgtttggagg cagatggcca 175140
ggccccgcag tcaggactct gcaggtggcc agtgtttcag catgagggga ggagccaagc 175200
cgagagtggc tgggtgggca gtgggtggag aggctcatac cctgatgtgg ctcccatgct 175260
ggtcatcagc cacctgcctg gccaggtagc tgcaggctgg atcaggtgtc acaggggagc 175320
gcctgcctcc tcctcaccct gatctgtgtg tgcagctcag gcctctgccc agtgccacac 175380
cagagccagt gcccatggct gcctccctgg gcctcacctc atgcactttc actccaggac 175440
ctgcaattgt cctttccttc catttctttc ctgatttctc ttcactatac atcaatccat 175500
catctattat ctatcaatca atcatctatc tatctatcat ctatcactgt catctatcaa 175560
tccatcacca atctatcatc tatctgtcat ctctataatc tatcaactgt ctatcatcta 175620
tgaatatatg aagtacatgt gtgtgtatgt atctatctcc atccatccta ttgtatatcc 175680
atcctatcta cctacctatc atccatccat ccacccatcc atcatccatc catccaccca 175740
tccatcatcg atccatccat ccatgctatc cagctaccta tcattcatcc atccacccat 175800
ctgtccatca tccaccaatc cacccatcat ccatccatcc atccatccac ccacccatcc 175860
atcatccatc catccatcca tgctgtccag ctacctacct atcattcatc catccaccca 175920
aacatccatc atccagcaat ccacccatca tccatccacc cacccgtcat ccatccatta 175980
tccatccatc catccatcct atctagctac ctacctatca ttcatccacc cattcaccca 176040
tccatcatcc attcatcatc catccatcca tccatccatg ctatccagct acctacctat 176100
cattcatcca tccacccagc catccatcat ccagcaatcc acccatcatc catccaccca 176160
tccatcccat ccatcatcca tccattatcc atctgtccat cctatctagc tacctatcat 176220
tcatccaccc attcacccat gcatcatcca ttcatccatc catccatcca tccatcatcc 176280
atccatccat actatccagc tacctaccta tcatccattc ctccatccac ccatccatta 176340
tccatacatc cagccagcca gccttccatc cacccatcct atctatccat tctatctacc 176400
cacctatcat ccatccatcc tatccaccta cttacctagc attcatccat ccatccatcc 176460
atccatccat ccatccatcc atccatccaa tctattcact gtctccacct aatttctgac 176520
atctcatctt tgtaatttgt tctccagtga gcatcttttc acatgcactg gtgtttgttt 176580
tgcctcctcc tggggctgcc tattcttcct agctggagca ctaggctcta cgttgtcctt 176640

gtggtatttt tctttgattc tgtaacatgc catggtcctg caaacacacc ccttggcttc 176700
attttggcct ttctggggtc cagacgccct gctgacttca tggtttgtct ggctccagct 176760
ctgcacttgt gggcccccgg ccacagctga ttttgctgcc ctggttgtcc tcctctctct 176820
tccttggctc ccgtgggcca cacgcctgct gcacccatgg ctcctgtctc cacagggctt 176880
ccccttcagt gctgcaggca cacttccacc cccaccccca cccaactgct ttgcagcctc 176940
ataggtgcca tgtccaggtg ccctctctca ctgcacctgt cagctgctgc ctccctgggg 177000
cttcctggga ggcttgcagc catgcccgcc catcccttct ccccttcctt cccacttctg 177060
ccttgggttt acacttgcct catgaccccc agacctggct ccaccggccc acccacacag 177120
ggcagcacct ctcaacggcc tcctgcctca taccccagct gagatggccc ttcccctctt 177180
gctgcattgt cctcccacag caacatggat gcccctgggc ggctttgggg cctgccacct 177240
cccccactta gtccacctga tttccaacga tgccccaagg gagctgccat ttttgtcatg 177300
ccagccttcc gccaacagct gcttctgttt ctgggcctgt ttgccaggaa gggtgcagcc 177360
ctttctcctc cctttctgga caagggtccc atcccccatg gcccagctga gtctcctctc 177420
tccgggaggc tttctccaaa tattttaacc ctgaattatg tcttcttttg ctgaccaaag 177480
ctgcagcctg agtctagatg gagtttctga tgtaatttga tttagatgac tcgtgctgta 177540
tcacaggtac ttgggatttg gcttcccgag gaaagaaccc tggtgtctgc aactctgaca 177600
cctctcagcc ctggcaggca atgcatatgg aaacctggga atgccagagc ccctgtggac 177660
tgtgaagcag tgagcagact gtactcacag acgcctcgca gagccggtag aagtcaacgc 177720
tcaggtatgc tctgatgccg tctatggctc cagggagggt gaccccacgc aggagcaggg 177780
cagtgaggac cacgtatggc atggtggctg tgatccatac cacctgcagg agaggacagt 177840
gtcaccaggc tgcacaggca gggcccttgg tgggagcaga cactggcaca agggcaccgg 177900
gttgccctga cggcttgtcc tctgattggg aggcccaatt cccaaacatt tcctgggacg 177960
ccagctcagc aaatgctcct tggagtgaga aggtcagaca ctttctgtcc tgcacaccct 178020
ccccagggat ggaaagggaa gaacaacttg gcagggaaag cacattcctt ttctgcattc 178080
ggtccgccag agtagctggg agtggctgat gggggttttc tgatgcgtgg gacgtgagtg 178140
gattcacact ggtgaacggc actgtggcac gatgaagggc tgctctgggc tgtgtccctg 178200
cgggaggctc ccgtggctgg tgccatacta cacgcacttt tccaagggag gagtcgtatc 178260
ttggtcaaaa ctggttttgg cccatataac aaccaaagtt tttttttttt ttttgagatg 178320
gagttttgct ctttttgccc aggctggagt gcagtggcat gatcttggct cactgcaacc 178380
tccgcctcct gggttcaagc aattctcctg cctcagcctc ccacgtagct gggattatag 178440
gcacgtgcca ccgtgcctgg ctaatttcgt atttttagta gagatggggt ttcatcatgt 178500
tggccaggct gttcttgaac ccctgacctc aggtgatctg cccgtctcag cctcccaaag 178560
tatttttatc aacaaaaaaa tcatcttgtg tgtgccttcc tgccagaaac actgctgtga 178620
gtgcttccct cctgaggccc ttccccaaac acagccacac gtggacccaa aacccaactg 178680
tgccgtgtgt ccgcccagcc cagccacggc cacgtgtccc cccacccacc catggccgcg 178740
cgtctaccca agccaacccg gcacagccac aggtgtaccc tcaatcatgg ccacgtgtac 178800
actcccaacc tgattatggc catatgagtc tcccaaactc aaccatggcc atgtgtccac 178860
cccaacctgg ccatggccac attggtagca caaaacccaa ctgaggccac acgtgcacct 178920
cctgtccagc cacggccaca tgtccacttg gtggccccat gtctacaggc ccaattggtg 178980
acccccgagc ctcaccttcc ctgaggtctt cacgcccttc cagaggctga agtagagcag 179040
cacgatgacc agcaccaggc aggctgtgag ctgccaccgc ggaggcccca ggtcgtcgat 179100
gccatggctc tggtggaggt gcagcacgcc acgtctgcag aggggagtca gcgggggact 179160
ctgtgggtgg ctgtcaaccc acctggaact ggacggctga gcttgtccgg ggacctgccc 179220
tccccatctc agcagggcag aaagcatcca gtctgatgga caagagatgc cttccaggga 179280
ggtctggccc acaggccctg gcagaggtgt cagggccgcc cttgaaaccc gtggagcacg 179340
tgggaggccc aggaagccct gcctcaccat ctgcccttgg cacccctgct cactggagct 179400
gcggaggcgg agggcacctt gggtctttga agaaaaggag ccacagaaac caaaaggaag 179460
ttgctgagca atgctgggtg cccaccactg cccagtgctg cccaaggtgc tgggtgccca 179520
ccgctgccca cggtgctgcc cacgctgctg ggtacccacc gctgcccaca gtgctgccca 179580
cgctgctggg tgcccaccgc tgcccacagt gctgcccacg ctgctgggtg cccaccgctg 179640
cccacagtgc tgcccacgct gctgggtgcc caccgctgcc cacagtgctg cccacgctgc 179700
tgggtaccca ccgctgccca gtgctgccca tggtgctggg tgcccaccgc tgcccagtgc 179760
tgcccatggt gctgggtacc caccgctgcc cagtgctgcc catggtgctg ggtgcccacc 179820
actgcccaca gtgctgccca cggtgctggg tgcccaccgc tgcccagtgc tgcccaaggt 179880
gctgggtgcc caccgctgcc cacagtgctg cccacggtgc tgggtaccca ccgctgccca 179940
cagtgctgcc cacgctgctg ggtgcccact gctgcccagt gctgcccatg gtgctgggtg 180000
cccaccgctg cccacagtgc tgcccatggt gctgggtacc caccgctgcc cacagtgctg 180060
cccacgctgc tgggtgccca ccgctgccca cagtgctgcc cacgctgctg ggtacccact 180120
gctgcccagt gctgcccaag gtgctgggta cccaccactg cccacagtac tgcccaaggt 180180
gctgggtacc caccactgcc cacagtgctg cccacgctgc tgggtaccca ctgctgccca 180240
gtgctgccca cggtgctagg tgcccaccgc tgcccacagt gctgcccacg ctgctgggta 180300
cccactgctg cccagtgctg cccatggtgc tgggtaccca ccgctgccca cggtgctgcc 180360
catggtgctg ggtacccact gctgcccaca gtgctgccca cggtgctggg tacccaccgc 180420

tgcccagtgc tgcccatggt gctgggtacc caccgctgcc cacagtgctg cccacgctgc 180480
tgggtaccca ctgctgccca caagacagcc ccagggtccc tctccaggga tgaggccgag 180540
caaactctag caataatgcc aggcacacag gcaccagcca gcactgtttt ctgaggggat 180600
ggctcacgac acctgctcac agcccctgct gctatgagga ttccacaagg aacagattca 180660
gtggatgact ctgctccagc aggaatcact cgcagttcca ctgtttttg aaggatccct 180720
cagaaagggc agaacagagg gtgagggatg gagctgacca tcggctgtgc tccttgcccc 180780
aggcacaggt cctgggcccc ctgtctgtgg gaactcattc catcctggtg acactctctg 180840
tgggagaaca tgcaccaggc tacatgcagt cagctgcgtc accactgcag actgtgactt 180900
ggggccaaga caactgtaac agccgtgccg ctgccaccgt agagtcagga gctgtgacag 180960
acagaagccg cgttatgaaa ggagctccca ctgcccaggt ttgcttctgt tgtgggtcca 181020
ctgaggtccc caggagataa aacctgacac ccatggtgga gcccggccga atgcagcctt 181080
ggggagcttt ccaccagctg caaggcattc cagggccatg tcccaggaat tcggctcttc 181140
aaactccaga caccctggct gttgccaaca gggaaggccc cacgagccct gaaagcgctg 181200
gacagtgcct ccgtcctatc ctggagggcg cccaggcaga ggccccaggt tggcacaggc 181260
cccaggttgg ccgggtgggc agatggctgc accactgaaa ccatcgccac cattctctgc 181320
tcttctccaa gggcaagccg ctcagtcaag aggctatccg tcacccgcac tgtgcaggga 181380
aagttgtctg agctggtgga ggtcacgcct gcccttcctg ggcagtgggg gactcccagg 181440
gccttcttct cactcctcct gccacacacc aggctctgcc cctgctggcc agtccccgtc 181500
gttctgctcc cccagcaagg tctcatcact gagagcttct gaggcctccc agcaactttg 181560
tgcagcccgg accactcctc cctcactgcc catggtgtca ctgaccccag catgccaccc 181620
gcccacgcct tggcagcctc agtgcctcac acacacacgt gcatgaatta tacacccaga 181680
ccagacagca caccctctcc ccttgcccag gtgcctggag aaaaggcaaa ctcagtcaat 181740
gcccatacat ggcacaggcc ctcctggacc agagggggag gcccagggaa agtgcgctgc 181800
cccactcagg gccttctcac aaggttgcgt ccacagcaca cacagctggc tcagcattaa 181860
acatgaaagc aacttcagac gcagcacctt gacacactgg gtcgagcccc ttctcgagat 181920
tagaaagtca ctgtgctttg aggttatttg aaaacccaca gacaatttag agaaccggac 181980
actcctgagt gctgtcaacc tgagaagagg ggtccccaga gctgaccaca gcatgggaac 182040
cagccatgcc aggagcaggg gaaagctgtg cgtctcccag catgtccatg cttctgaggg 182100
cagggcaggc gagaccatca aaggacatcc gggaaggggc ataagtgcag gcctacgtgc 182160
aggtgtttca taagccataa tagttgggag gaccagaaaa gtgttggccc aggatgttca 182220
ccaggcgtgg aaaagacaca gcacctccca atttctactg ggctcttttt cctggtgaat 182280
tcctggccat gattcaattg ggtgacagtg gagccactgg tggtgatcca ggcatgctca 182340
ggcctcccat gcatcattgt gatgacctca cagcagccct ctcaccccgt tttaccaaga 182400
gggagccaag gcagtgaggt ggtgaaacaa gcttccaaag gataaaattg tgccaaagca 182460
cgttaacttc ctattagaac aaatctaaca ctctctaaac aaagacgaca agagctaaat 182520
acccattagt ataaaactca ccaagtctag catctaataa aaaattacta gacatgacaa 182580
gaagcaagaa atgtgatcta caaccagagg aaaatcagtg aacagctact tcacatgata 182640
tagaaaaatt aacccaaaat ggataaaaga gccaaatata agggctaaaa ccataaaact 182700
cttggaagag aacatagggt aaattttcac aatcttggac ttggcaatgg tttcttagat 182760
atgacacaaa agatgaatta gacttcatca aaatttaaaa cttttgtgca ctgaaggatg 182820
ctatcaacag agtaaaaagg aaaactacag aatgagagaa aatattcgca aatcacctat 182880
taatctgaaa agagaataat atctaaaata tgtaaagaac tcctaaaact caacaacaaa 182940
aaaactgaca gcccaattca aaaatgggca aaggacttga atagacattg ctccaaagat 183000
ggtatacaaa tggccaataa acacatgaag agatgcccaa cagtcactaa tcattaagga 183060
aatacaaatc aaaaatacaa tgacagacca cttcacaccc attaggatgg ctatgatcaa 183120
aacaaacaag cagaaagtaa gtgttgtcaa ggatgtgggg aaatcagaac ccttgttccg 183180
atttgcatcc actgtgggaa acagtgtggt ggttcctcaa aaaattaaac acagaattat 183240
cacatagctc cagcaattca acttctgggt atctactcaa aagtaggtac tcaatattga 183300
ggttcatagc agcatgtttt tacagtagcc aaaatgtgga agcaacccaa atgtccatca 183360
aaagataaat agatctggcc aggtgtggtg gcttacattt gtaattgcag tgctctggga 183420
agctgaggcg ggaggattac ttgagcccag gagtttaaga caggcctggg caacatggca 183480
agaccccact ttacaaaaaa ttttaaaaaa ttagctggat gtggtgtcac atacctgtag 183540
tcccagctac ttgggagtct gaggcaggag gattgcttga gcccagaagg tcaagcctgc 183600
agtgagccgt gatcatacca ctgcacactc cagcctggac aacagagtga gatcctgtct 183660
ctaaaaagga aaaagataaa tggataaaca aaacatgata tatccataca atggaattga 183720
aatattattt ggccttaaaa aggaatgaaa ttctggttct gaaattcttc aataaacctt 183780
gaagacatat gctaaggaaa ataaggcagt cacaaaaaaa taagtgattc tactcagagg 183840
agggacctag agtagacaaa ttcacagaga cagaaagtag aatggtagct gccaggggct 183900
gcagtgaggg aggatgggga cttagtattt aatggggaca aaatttcagc tttggaggag 183960
gagaaagttc caatggatga tggtgatggt tgcacaacat gtgattgtac ctaatgccac 184020
tgaactgtgc actgaaaaat ggctaaggtg gtaaaagcaa tgctatgtat agcttaccac 184080
aatagccccc caagaaggca aaatactttt gtgtaaagcc caagctgaat cactgccagc 184140
aagtctacgc tatgagaaat gttaaaggaa attctttaga tggaaggaag atggtcttaa 184200

```
atggaaaccc caatctacat acaggaccgg acagcactag agatggtgcc tatgcaggta      184260
aatataaaac atttctttgt tagatttatt taaaatgtaa atgcttaaag cagaaataac      184320
agcaaggtat catggagttg atacatattg tagaaaaaat ctatgacaac aattgcacaa      184380
aggatgtgag gaaaacagaa gtctactgtt gtaggccctt ctgctctgtg aaacggtatc      184440
atgtcacttg aaggtcagtg ttgtaaaccc tataaaatcc acgaagaaat gaaaacagaa      184500
agctatagtt tattagctaa tagtgttgat aaaatgaaag cctaaaaaat taattcaaaa      184560
cagggaataa atagaggaaa caagaaacaa agaagagagg gggcaaaaaa ttagcacaat      184620
gaaagactta aagccagcca tgctgacaat tattgaatgg aaactctaat taaaaaacag      184680
agattgccat attggataaa aagcacaacc aactcgatgg tgtctacaat aaatccattt      184740
tatatataat gacacatata gtttaaaaat aaaaggctgg aaaagataat accacacaaa      184800
cagtaatgat acgaaagttg gggtggccat attactatca gacaaagtag acttcagaat      184860
gagaaatcta acccagaagg atagttcaaa tggtaaagcg gtcaattcaa agactaataa      184920
taataaacat gtatgcactt catagcagag tttcaaaata tgtgaatgaa gcaaatactg      184980
atagaagtga aaagaaatag acaaatctac agttacagtt gagatttcag taccccttc      185040
tcagtaactg atagagcaag tggacagaaa accagtaagg atgtagaaaa cctgaaaaac      185100
acagtgacct aacataacct ctctgacatt tatgcaactt tccaaccaaa gacagcagaa      185160
tacatattct ttctaggtgc atgtggaaca ttcaccagtg taaaccatct agtaggtcat      185220
aaacaagtct caatatattt taaaatatta aagatgtata agcatatcat cttgccaaaa      185280
cagaattaaa ttagaaatca ataacaaaaa tacctagcaa acttccaaat atttcaaatt      185340
aaacaacaaa cttctaaaaa tgccctgtta gtcaaataga aatcaaaaga aagattaggg      185400
aatactttga actgaatgaa atgaaaacac agcatatcaa actttgtggg atgcagctaa      185460
tgcagtccat ttgaggcaaa ttaatagctt taatttataa ctttataatt tatattaggc      185520
agtgtcgata agtatttatg gaagtgggga aactgtaaac tggtcacagt tatgtggtcc      185580
tgtgttgggg ttttgaaatg aaaacacagc atatcaaatg tgtgggatgc agccaatcaa      185640
taatctccag gttcctacaa ggaagggttc cttttatttc tctccaggaa ccatttctgc      185700
cacctccctc tggtgggtgg gctgctgccc aagaaggaag tggcaatgca ggaaggctgg      185760
ctgggacccc tgggggccag gggtgccagc aactcccaca ggatacgttt catttgccaa      185820
ccctgaggaa cactgggtgt aggagccagc actgcacacg gcattcatgg cacatggagg      185880
aagcaccatt agtggggcag ctcagcagtc ttttctgact tagggcccag aagacatggc      185940
cagcgtctca cccaggcctc acggttactt cctgaggacc tgtagtaaaa acgcacgata      186000
aagccctttc agatctactc ttgctaacat cttagttgtc aaatttatga cacgtcatgt      186060
ttcgtacatt gtgtaggaaa ctcaatccct tttgtgttta ctgacaaagt caggcatgcc      186120
cattatctag gacaaagagc tgcactgtca cacatcacag ccactctggc ctttatggcc      186180
tttcacagtg agacatccac tccctgatta catgcgctgg caggaacccg acatcctttg      186240
tccagtgatc tgctttataa agcaggtttg aaatccagaa cacccctggc agacctgagt      186300
acgttatgac tgtcctccct gctccccaag agtcactggg gcctgtttca ggggaacctc      186360
agtcccactg gtgggaaaat caccttcaga aggtgtggga ggagtgccaa gcacctcagt      186420
gccttgggtc attaatcatt tgtgaattga tcttaaacct tctccaactc cccacgtctg      186480
ctcttgagct gccgccagct aaagtgtacc acgtgccagt gcggacaggg cttctgagtg      186540
aagggctggg cacctttctg tttccttcct tccttccaga ctgagaactt ccaaatagac      186600
ttcgctagtc tgatcgttta aaataataac acgtgttctt tccttctagc attcttaaca      186660
acgaatcact ggactaatca ctacactaag ttttcctctc caattctgtt atctgaacat      186720
cacttcaatg agtcaacatt tcccacactg cccaaacaaa aggaaggcag tccccacatg      186780
gcagcacttg gcagctttaa cagggcgttg ggtaccggga actctcaggc tcttgccctg      186840
gtggcacctc tgcggccact gcccctggct ctgtgtgccc cccactttct cctgctgtcc      186900
cctctctttg atcacatgct aaccctgaga gctgggcaca cagcaagagc acatgcagcc      186960
caggcctggg gtgttcatag ggtgtttaat tagggaaatg taggtgtgaa caggccatcc      187020
acacgcaatg ggagttttct ccctcgagaa tgttaaaatc tacttggaca aatggtgtga      187080
ctgtgcatta tttggacaat tttatcattt cccgcttgcc tgtcacccac gagagtcctg      187140
cccagactct cccacgacct tgcctgcagc tgcgcttcct atggcgatgc tcgtccaagt      187200
gtcttcaggc aaacaaccag gactcatccc gggtgcatct tctctcccgc ctgccccacc      187260
ttctgaaacc agccctcctc ccatgagcgt cttcctggcg ccccagcctg gactctctct      187320
cctcctgctg ggtacttggt gggccactct ctcccatcgt ggcaccgtcc tcctcagagt      187380
tcgtcactct tgtccactcc caaaccacaa gaaaggccca cacaacgctc accccacgcg      187440
acccatggtc ttcctcatgc tctaagaaaa acgccgggtg gccccccaaa acctgcctcc      187500
gccccggctc ctcacctcca aggccagtcc cggcaatatg cttccatgcc cgaggaggaa      187560
ttctagaacc aagcacttgg gttctgaata tctgagtgca gagcagtttg agagaccgaa      187620
cgcctgccca ccatgagaac tggtcccttt ccggcagcat cagccctact ttctccaagc      187680
cacaccctcc ggcctgcccg aaggctccat ctcagccatc tctgacaaga cccccaggag      187740
agggcgaagg tcccttcctt gcaggaaaag acctcagtga tgtctgttga tacggttttc      187800
agatatagaa agcatctctc tgggaggctg tgcgccccga aggtgccctt gcagaccaag      187860
accaaagttc tcggggatga tcctccccca gcagaggcga ggtgggggct tctctgctca      187920
ccgtgacttg tacaaactta acatgagatg cttcatttgc tttcaagctc cctgttttct      187980
```

gactcaaaac aaattttgca aaggcaccta ctggctgtta aaactggaat tagtcttact 188040
ggtcaaagtt ccttttccta ccttgaagtg gactcatttt ggcatataaa cgtgaataag 188100
atgcgtctaa ccaggcaagc gagcccccca aacaggagcg ccctggctga cgggaaacac 188160
tggatctggg gagaatcgcg caaacgtcct ctcctcctct ccaaaatgac ggctgagtcg 188220
aagagtccgc ctgtcagaac ggcgacccgg cacgaggccc caggcccggc tggcgagggc 188280
aggaagatgt ggatggcacc cgccagatcc agattccggg aggggtccag gggaaacagg 188340
gaacccctgg gtgtcagtaa cgacccttcc aggctgggcc aggccattgg tgacgaggga 188400
gggccagcag gagctggcac ggggcccaaa aggacttcat cggagacatc ggggtccagg 188460
aaagcaggtg ctgcaggtgg atggtgaggg gcgggcctgg cctgggtgga cagtgaggga 188520
cgggcctgga tggggtggac tgtgcgggtt ggggctggtc ggggtggact gtatgggttg 188580
ggcctggctg ggatggacag tgaggatcag gcctggccgg ggtggatagt gagggatggg 188640
cctgaccagg gtagacagtg tgggttgggt ctggccgggg tggacggtga ggggtgggcc 188700
tggccggggt ggaccatgag gggcgggcct ggccggggtg gacggtgtgg ggtgggcctg 188760
gccgaggtgg acagtgagcg aggggcctgg ctggggtgga ctttgtggtt tgtgcctggc 188820
tggggtggac agtgagggtt gggcctggct ggggtgggca gtgaggaact ggcctggctg 188880
gggaggactg tgcaggtcag gcctggccag ggcaaaggga gaaggagaca gagacccagg 188940
aggctgctgg acatgggata cgctggcatg ggaggtggaa agaggcccag agacagaaat 189000
aaagccatgc aggtaaacaa caaaacaaag tcttgtgaaa agcggacggc tgggtcaccc 189060
tggagacagg ggctgcctac tgaagccaga gacttcttgc gcagtgacac ccagaaagtt 189120
atcttttctg gagagggcaa gggccggagt gaggcagttc ggccccatgc tatgcacaga 189180
ggaagggtag ggaggaaggg agcccagaca tggggctgac tcactccaga ggcgccactg 189240
cagctcccag agactggagc ttgcccacct tctgcacagg gtctgctgtg ttccccatca 189300
gccccctccc accctgagct gccgctggga gcagccaagg gagctgctca gtgccccatc 189360
cttgctgagg gctcgcccta caggagcttt gctgagctcc tggagaccac cacagtgatt 189420
cagaaatgtg cccctccaga gcactaaagg gatggagtgc aggaacagat tccccctccc 189480
cgcaccctgg gagtcagcaa gggctggtgt ccaaccaagg ggctaccatg ggggacctgg 189540
ctgcgccatc tctcccgttc ccgaggaccc gactcccact tactcaaagt actcggcagc 189600
aggtgtggtc ccaaaagtgt cgttgaggcc cgagctgtct ccactggagt caccaggatg 189660
ggcatccgag cagttggggc tgttccagga gttgttgcag tggatccagg ggagctccgt 189720
ggtgaaggag gagaagagat agtgcagcgc ccaggcgatg atgacgttgt agaagaagcc 189780
gacatacagt gagatgagga tgaccgtgaa gcccacacct agcgggaagg gggaggccat 189840
ggagcccacg caggtggagc acagagccac catcagcaac gtgtcccttc cacctcccct 189900
cacgggagac attaccctga gcccacaact cctggacagc cctgatgcca gacagcatct 189960
tcaaacatgc agggaaggcc cgtgaaaggc ctgagaccag atttcacact gtgaccacag 190020
agaagaagct gcccactccc caaaaccatg cactcctaca caaggacttt cagggtgtct 190080
ctgagagaca tccagagcca gcaaggtcac ccagagctat ggcgagtatc cagggccacc 190140
cttatgcacc cagagacccc cagggacacc aaggtcaccc ccagccacat atggtcatcc 190200
atccccacca cagccactca ggatccataa gagtcatcca ggatcattga ggccatctag 190260
ggggtactct gggtgaccca gggcaaccca actcatggtt aaccagggct atcaggccca 190320
tgtgaaacca tcagaatcat ccacggccac ccagaagcac ccagggctac ctagggagcc 190380
catgcaaata gggccatcaa aggtcgtcta gggttaccca ggaacaccca gtgtctccca 190440
gagccatcaa gactgccaat agtcactaag ggacacctgg ggtccttgac agccatccac 190500
agccaccagg ctcattcagg actatggagg gcacccagga ccacatatgg ccacccaggc 190560
taatccaggg ccactttgag tcgcaaaggg ccatccatgg ccatccacag ccatctccat 190620
tcatccacaa ctatccacgg tcagctatag ccatcctcaa ccattcatgg ccatccacgg 190680
ccatccacat ccatccgcag ccatccacaa ccatccatcc atccacagcc atccacagtc 190740
atccatggcc atccacatcc acccctggcc atccacaaca atccactgtc attcacaacc 190800
atccacagcc atctacagac atccacaact atccatggtc atccatagcc atccacaacc 190860
attcatggcc acccacatcc atccacggcc atccacaacc atccatccat ccacaaccat 190920
ccacaaccat ccacacccaa ccacaaccat ccacatccat ccacagccat ccatagtcat 190980
ccatggctgt ccacaacctc ccacatccac ccggccatcc acaacaatcc attgccattc 191040
aaaaccatcc acagccatct acagacatcc acagccatcc acagccatct gcatttgccc 191100
ataactatct acggccaccc atggccatcc acagccaccc atggccatcc agagccatcc 191160
atagccatct atggccatct gtggccatct atagctgctc atgcctctcc tgcaagacta 191220
ctcagaagag tcatctgagc tcatttacag ccatgtggag ccatccaagg tcacactacc 191280
catgcatgac catccagggt ttcctagaat cacccagggc cacgtaagac caccagggtc 191340
cacccagggc aggtaagatc atccagggct tccttgggcc acccagagcc ttgcatgact 191400
atgaaggact actcagagcc atgtttgacc ttccgaggcc ttccagggcc atgcaagacc 191460
atcaaggacc agccagggcc atccatggcc atgcaatgcc atccacggtc ttttagagtg 191520
tcctttccag gatcacagac ctggaatttg ggatccatca tttatggggt ctcctggtaa 191580
actggaacag aactcagtgg tcagcagtct ttggagtggt gtcagcctga ctggtgggca 191640
gatgggcctg gtggtcagat gacttcctcc ttgttcactg ctggatgggc accatcacgt 191700
tcttgctact ggggttcact ttactaagct ccaggaagaa ttttttcat ttccacattt 191760

```
cttctcagtt tactgacatg attagtttgg gtacaacttt gatttacttt attaaaaccc 191820
agtaggcacc atgccgtctt gcctgttcat ggccggggta ggagcatacc ttcaaggtga 191880
cttttgcagg gggtggcctg ggttgctttt tatagtctca atccttagga gaagctgttc 191940
tcatgctcag ggcacctcag taaagttctc ttcctacctt gaaagcaaaa agaagactca 192000
aaactttgtt ttaaacaaat ctaagatcag gagtgcgggt aatccccctt ggaaagcatg 192060
ctcagtagtg gtgacgatga aagccaagct gctgcctttg gaaggtcttc tcctatgtgg 192120
atctggtcca tattaaataa ttctgtattg cagtctgtca tggattgcct ttgaaaagcc 192180
ttctcccata tggatctggt ccatattaaa taattctgta ttgcagtctg tcacggattg 192240
cctttggaag gccttctccc acgtggatct ggtccatatt aaataattct atattaccgt 192300
ctgtccggag tcccttagga gagaggctgg gttgtgatcc acagtgaact cacccaggca 192360
gccaggttgg gagccacact gtcggtgagc cacattgttt ccagagagca ctgtggaggg 192420
ttttccatgg tggtaactga caatggatgc ccaatttgaa aatttttcaa ggttaaaact 192480
ttctgtatca agtccttagc ttgaaaccct gggaagtcct tgatcaaaca acaccagagt 192540
ggatcccaat ccagagtccg aaggaagggc tgagtgtacc ctgggtcctt ccagagccac 192600
tgcttttgtt tcctgccctg caccacagat tggcagggcc accatgggag catgacatca 192660
ccatggccac tgtgcatgac atcaccactg gccacactgg ctgatgcggc tcccagagca 192720
aatgggcctg ctctgatcca tgggacagtg cagcccccac accagagtcc cccttaccaa 192780
gaggacccct cctggttgag gatgcttccc tgggcacctg ggtgaggaaa tggctccctt 192840
gaacagtggt ggccagtccc ctcctggatg cttccctggg cacctgggtg aggaaatggc 192900
tcccttgaac ggtggtggcc agtcccctcc tggatgcttc cctgggcacc tgggtgagga 192960
aatggctccc ttgaacggtg gtggccagtc ccctcctgga tgcttccctg ggcacctggg 193020
tgaggaaaca gccttgaacg gtggtggcca gtcccctcct ggatgcttcc ctgggcacct 193080
cggtgaggaa atggctccct tgaacagtgg tggccagtcc cctcctggat gcttccctgg 193140
gcacctgggt gaggaaatgg ctcccttgaa cggtggtggc cagtcccctc ctggatgctt 193200
ccctgggcac ctgggtgagg aaatggctcc cttgaacggt agtggccact gtcagcactg 193260
gccaggcatt cggttgctcc aagccctgcc cttcaggaag gcagagcccg tgacctgggc 193320
cactgacgtg tagcccctca ccaaccagcc aacttcacca cgagctcctc aagctcaccg 193380
aaggcccaga cgcaggaaac ccactaccct gttggctaca gaaccttcta gtgatctctg 193440
ctcatgacat cactgtgggc tccagatgaa gcttcacctg acacatttct taaacaccat 193500
ttagtgacac gggagactgt ctccccaagg aggtggcagg caaagtccct gctcgcgttt 193560
ccagggaaaa cattcatgag aacatggcgc ttcccttccc tcctgtctga ctcccaggaa 193620
gctcggcgct aagtgtgaat ttcaagcata gcttttaagg cccatttcct taaatttatt 193680
tttgcttagt atcttttttct ttatattaac cttgtgtgta gtttgttttg tgtttgagaa 193740
atcacactaa aatatgactg actataaaat attttatatt caatacaatc gaatggtggg 193800
gtttccaggg cgtctgtaaa atggggttgc ccttggcaat gaaaccgaaa cctgacaaac 193860
agaagctggc tgagggtcgt gcctgcaaat ggtgcttcgc ccaccagagc acggccaccc 193920
tgccccaccc ctgaacacca gtacacccca gggcacccac gagagctggc ttcaggagag 193980
ggtgttgtca acgcatgcac ggatcccgct ggaggaagcc tctggtggag cagcccctcc 194040
atacctcaca ggaggcatca agcaggtctt tggaagaaag acgggagagg gccgggcacg 194100
gtggctcact cctgtaatcc cagcactttg ggaggccaag gcaggcggat catgaggtca 194160
ggcaatcgag accatcctgg ctaacacgat gaaaccccgt ctctactaaa aatacaaaaa 194220
attagcgggg cgtggtggca cgcacctgta gccccagcta ctcgggaggc tggggcagga 194280
gaatcgcttg aacccgggac gcgcaggtga cagtgagcca agatcgcgcc tttgcactcc 194340
agcctggtga cagagcgaga ttccgtctca caaaaaaaaa aaaaaagaaa agaaaagaaa 194400
agaaagaggg gagaggacgc ctctctgtgt cagtctgggt gtctttagta tgtgcttgct 194460
gtgtgtgcat gtacccgaga gacagagagg agaagagaca gaacaggaga gagacagaga 194520
ggagaagaga cagaacagga gagacacaga gaggaaagga gggagagaga cagaaagaga 194580
cacagggaga gggaaagaga gcgaggggaa gggaggagac ggagagtttt aaagacccac 194640
ctctcaggac cctggggtgc actgcggggc tgaaggcccc agaggcccct ctggctgtgg 194700
tgcccatccc aagctgctct ggtgagacta cgggaaatgc acgtgagtca tggctgctga 194760
gcagctgggc ctcaccatga actccgccct ctggctttca agggtggatc ttggctccca 194820
gttaggagca gggagcagct cgcaggtctg caggagggga agggccagga acaaccaggc 194880
ttcctggaga gctggcttca ttcccccatg gaattgccac ctgctcaggg ctggatggcc 194940
ttttagctcc acaatgattg ttgtgggcac tttagctgat gtcttaattt accacgtggc 195000
acttctattc aatgagacat gaaaacgtat gcatttttat taaccagatt tttaaaaagg 195060
acaaaggcac atgtatcagg gtgccggggg tgcatggtgt acatctgatt tcataagcaa 195120
tgtcagtctc ctctaaactg gcatcctgcg cttgacaggt aggcagaaca aacgggacgc 195180
tggcaccgga acctgcagcg ttactgagat tcaacaactc aaggtgtctc gtctctaaaa 195240
agaagtccag ctttcttgca ctgattcatc tatcccttcg tgggtcgaac agttcacttt 195300
ctgtgatttg caattttcct tcctcaccag gactaggtga gtgtgaacaa gctcttccaa 195360
agatccgccc gcagggccac gagccttcct tcctgcggcc acactttgca gccctcctct 195420
gggactagtc ttcagaacga agctggcggc atggaatgcg ggattgtggg cacagggtct 195480
gtgacacaga ggagacgact gcccagctta ccccggctgc gcattgtgga gaacacgatg 195540
```

```
atatgcccac atccgccggc tgcatttctt cagaacgagg tgccactgct cacgctgatg    195600
gaagtcagag gttctctact agcccaagtt gagttggtgc tctgaaattc ttagaaattg    195660
cttcttgaca ttttatggac tacaccatga agacccacaa cggcagcttt gttggtgaac    195720
atcctttctg tgtgacagac ggaaggaaat gagctcttat ctgcggacct acaggtccct    195780
ttttctcctg ccctcctcac ctgaatcctg cacgtgaaga ggtgatttaa cttgctccaa    195840
caccaggctc actggcggga gtggggcaca gggctgtgcc ttgtaagaca cttgtgacag    195900
actccagggt ggctcttgaa aatccagcaa agcacagttg gatgtcgctg cagcctgtgc    195960
cagagcacaa tgtcatcttt cctcagtttc ccctacccca gtgggccggc ttcatctctg    196020
actcagcatc ccaaggctct gagccctcag atactctcca acacggacca cggtgcaagc    196080
tcagcatcgc ttacgactgc aggctcagag gcagcactcc tctctgaaac acgtccgtgc    196140
cgctggccag ggccaggcag gacacaggta acgcgtgtgt cttgaactgt tagcctgggt    196200
tcactgcaac gccggatttg acacgcgccc ctgtctgtct gtaacaacag gcctgggtct    196260
tccgttggct gctgcattct tccattgggg acctgatatc agggacctga cgacggggac    196320
ggaggcatgt tctgagcaat tcttgtacgg tttgacttct acctgcagat cccgccaagg    196380
ggtcgtctgg caccagtgca tgcccaaagc atctgtctct cactctgaag atgtgtgtgg    196440
ggtgggggtg ggggtggggg tggggaggga ggcctctttt tgtagctcca tttcagtgca    196500
gtccagtgaa tgtggagccc aggaagaaat tactgaagtc ataagattca ctaagcagcc    196560
cattaagaac gaatgagtct cgtgcctggg aagaaccatc acacctgtcc agtctcagca    196620
tttccacggc tgcgagactg acttctcctc ctggagcctg acctcagcag ctgtgaaatg    196680
ccatctgttc cctctcctaa gggaggtcag cggctcgcct ctgaaggccc acccctgacc    196740
cattggtaat gcgtcctggg gccgtgctgg gctcggctgt cttctaaaca cacagctttt    196800
ccagaacttc tatgagtatc cagaggctga ccttctagta gtcattttct taatgccaat    196860
tttctgagtg gtgcctctga gtaaatgcct gaatcctccc tgaaccacca gcccctgaga    196920
ggtggctttg gcccactggg caagtgtgac ctgcgtgcag ggattgggga gtcagaaacg    196980
tctgggatca aatcccactc tgctctgatg gggacaccag ggggaacgac cccggccctc    197040
tggtcctcag catctgcatc tccaacacag agacctagtg tgtgcccttc ccagggtctc    197100
atgaagatgg agtcagaaat gctgggagag ggcttggtgc tgggcaggcc acctgcactc    197160
aacacgaggt gtgtccattt catggtgcca gagcaggaca tcagcagccg gaaaggttca    197220
gtgacaacta gatggatcaa tagctagtag ataggtggat aggtcgatag gtggatggat    197280
ggattaatcc atacatagat gaatggatgg atggatggaa caatggataa cagatggatg    197340
aatggataga tgaatagata gaagatggat ggatggatag atccatagat agatgaatgg    197400
atgaatgaat ggatggatgg atagatagat ggatcgatag gtagatggat ggatcaatag    197460
ataatggatg gatggatgga tggatggatg atagatccat agatagatga atggatggat    197520
gactggatgg gtggatgaat ggattgatag gtggatggat caaaagaaaa tagatggatg    197580
aatgggtaga tgacagacgg gtgaatagat gattgacaga taatagatag atagaattgt    197640
gggatgtgtc cctcccccaa gttcatctgt tgaagttcta accccgcaaa cctcagaatg    197700
tgactttatt tagagacaag gtctttacag aggtgatcaa gttaaagtga ggtcattagg    197760
gtgtccctaa tccaacagga cttctggcct cataaaaacg gggaatttgg ttacagagat    197820
atgcatagaa gcaagaagta taaagaggca cagggagaag acaatctctg agacaaggag    197880
agaggctaga acagatgctt cctcacagcc tcagaagaaa tcaaccctac agacaccttg    197940
atcttggact tccagcctcc agagctgtga gacaataaac gtgtgccttt taagcctccc    198000
agtttgtgtc atttgttaca gcagccacag gaaacagata cagatagatg atgatgatag    198060
atggatggat taggtagata ggtagataga tgggtgaatg attgatataa agaccaataa    198120
taaagataga taatggatag atagataaca gatgattgac ggatggaccg atggatgata    198180
ggtagatggc agatccatag atagacatgt gttttatctt aactacattt tagcatcttg    198240
acaaagtaac accacaaatg aattgcagga ccctcaccta gagcaaggga atgaactatc    198300
cagtccttgt cactgcagat ctttaaggtg ggacccaagt tcaagtggcg tgtgccatgc    198360
ctgtgtctta gcaaagcagg gctggatgcc catgatgcgg ctggcttgct ttgaaagctc    198420
cagcgtcacc accatgatcc gcgctgcgcc agggtgaagg gaggcacccg catattacct    198480
ttcagtatgg ggcagatctt ccagacacca gcggcccctt ccctgttgaa ctggccgagg    198540
gccagctcca tgtagaaaag tggcatccca gcaatgacca tgaagagcag gtaggggacc    198600
aggaaggcac ctgtggggca gaaaagcacc tttagtttgg ggcctcggaa gggcccatct    198660
ctcctcgtgg gagcttgggc ggctacccca gtcaaccatc catgtcctgg cccgaccgtt    198720
tcaccccact ctccaacggg aagtcccagg ctagggtaga tgagtcccga agcccgccct    198780
ccactgctgt cagtgcctga cacgtccctc ctggatcccc ttgtcattgc gcacctcgag    198840
ccatgaagtc aaacccaaga gcagcacagg gaggccctgc ccatgctcag cggtgctcta    198900
ggtactatcc ttccccgtgg tggttcgggg tgcccttccc tggcctgcgt ccccccactt    198960
tgccctgatc ctctttgcct cttgagttgt gaatctgtga ccccccaact ctgcccctga    199020
gctgagtggg ccctgccgtc ctgtccccag cttctggggc cctcagaggc tgcccctatt    199080
gactgtggct gagggtcttt gctgccccag gtgctgggag cccctccagg gtaaggccta    199140
gttcctctga aacctgcacc tcccccaccc ccagcacaaa gcccagggaa ccctggtctc    199200
aacctcctaa attccctctg ctcccctggt ctgactggag tgagggagag ctttgccact    199260
ctctctcctg ggaagggga gggggaggca tggggcccct cagctctgtc tttgggcaac    199320
```

ttcctgtacc tgctgaggcc tctgtcttcc cctcttcaag atgggcataa aaatgccagc 199380
ctcctcgcag gcatcctgtg aggcttcccc ccagattctt ccccaggcct cccttcccag 199440
acctagcata gaggccaatg agggaggcca ggcagggagg ctgggaggcc caaagaggct 199500
cctgggaagg gatcaccaat gttcttggac gtccccggtg gccctgcctc gatcttcgct 199560
ttctggctct gtggctgggt tctggagggt gcaggggaca cagtcacttt ccaggggcct 199620
cctctgccac tccaggctcc agggcctcca catgctttgc aaaggctgcc aaagtcagcc 199680
gcaggctgtt ctttggacct ttgagaattt tctttccaaa gcgaagatag cctctggaaa 199740
caggaggcag agccaagctg ccccgtctgc cgccccccac cccccagctt cttcgcgggc 199800
ctccctttcc taaactctga aatgcaggcg tgggacaagg cagctccgag tcctgctcaa 199860
tggttttgtg acatcctctg ggaggatctg caccggccgt gagctctcac agggagctcc 199920
gtcttcacgc atgggaacag cttcatctcg tttccgtacg tgccttggcc ccggctgccc 199980
ctacgacccc cgcccggcca gcatgctcag ggaggctgag atgggactta ccgccaccat 200040
ttttgtagca caggtagggg aaccgccaga cgttggccag gtccacagca aagccaatga 200100
cggacaggag aaagtcgatc ttcttgcccc aggtctcccg atcctgggcc tccacggggc 200160
tctgccgcgg gttggtgagg gtggagctgg tgagctgcac tccgttctgc tccttgacaa 200220
ggatgagctc cacctccttc gggcccacgg cattgggctc cttagccggg gccaccacgg 200280
aagacatgag tcccacggag catttgctct tactcatggg cacactggga gttgaggaat 200340
tctgtgcttc ttccctcttg gtcttcagcc aatatgaaaa ataaacacac aacaggaacg 200400
caacaattca gcagccaggg ctagggacat acctggtgcg gagggcagag ccccgaggca 200460
ttcacgggca ttcctctggg aagggatggg tgcgttctca gcgcctgaga tggtacagct 200520
ggggcacggg agctgggaag tgccagccct caccacaggg ctggggcact ggcccatgga 200580
ggcctcaaga cagacactct ggtttgctcc tccttcccct gcacccctcc ccactcctat 200640
caaagtgcaa ggctgtgtga gctctagcgt ggctttctac taacaatgca tggatatcac 200700
ttccaccttc ccccacactt ccagagagca attttacaat cttctaagag cattcaataa 200760
tgtcttcatg actgttcatc tgtattcttt tttcttttttt ttttcttttt taacatgagg 200820
tctcgctctg tcacccagga tggaatacag ttgtgattac aactcactgc ctcaacctcc 200880
taggttcaag tgatccttgc acctcagcct cctgagcagc tgagactaca ggtgtgcacc 200940
acagcaccgg ctaagttatt tttgtgtgtg tttttttgtt gttgttgttg tttttttgta 201000
gagacagggt ctgactattg ttgccaaggc tggttttgaa ctcctggtct caagcgatcc 201060
tcctgcctca gcctcccaaa gtgctgagat gacaggcatg agccaccaag cctgggcttg 201120
tattgtttat actcagtaat aaagagcaaa atttatataa gcaaaatcag ccactgtaaa 201180
gagagtttag acataaccat cacaactgct tcctcaaaaa gtgtctgtac aagccacatc 201240
cacctagggc aggtggcacc accccgcgtg agagagctgg gcggaggatg gacagggctt 201300
catcgcggga actagctcct gggccagcag ggtaacccgg gaaatcctgt ccaaacggct 201360
acataaaccc ccctgcaacg ctgagcagga gagcaggagc gtctacaccg ccccagccag 201420
ggcgacttct gtcatttgga tccagcagca gcgtgtttca gtcgagcgca cgcacacaca 201480
gacacacaga cacacacaga ctcacaaaca cacatacaca ctcacacaca cacaggcgag 201540
cacatacact tacacacata cacactcaca ctcacacagg tgcgcacaca cacacaacga 201600
ctgaagcagg tcgcaggtgg aggctctaac aggcaacttt ccctgcatcc aagtttgcac 201660
aaaacctcac ccatgagcta tcacgagcta tgccacgcac ccggtgacaa cctcaaccgg 201720
cacgacccct ccggtgggta aaacacccga cgaaggggtt ccgcggcgcg agcaggaggc 201780
cgcgttggga gagggcgtcg ggtgcggagc tcgcgagtct ccggcaagcc gccgccgccc 201840
tggggccacc tgaccccgct cctgaacgcg gggcctcaga ggcgagtttt gggtctacac 201900
agcaaaatgt ggcccgttag aaagcgtcct tcctcaccgc gcccagaaca agaagcggcg 201960
ctgcctggcg agtcctcgcc cggcctcccc gccctgccca gagcaccagc gcgcagcccg 202020
cgcccgccac cacccccaac acagacaaag cccccgcgag atggagcggc ggtgtacaaa 202080
acccactccg atcggatctg ggatgcgccg cacccctagt agggttaagt tccacgagaa 202140
gaaaccagga cccccgagtc acagccatag acacccacgc gtcccctaag ccggtgactg 202200
caccaggtgc ccacccggtt ctgagcctgg agcggggcca ggggcagccc tgcaccctcc 202260
tcgcgcctcg gacggcctca gggtcccatg tggcgctatc gggggcgct cggcacccgc 202320
gactgttggc gactttggag acggcgcggt ctgcgggacg cggggacccc agactgtgcc 202380
cggtccccgg cccccgcccc tgcgccccac ttcggggtct gcggaaggag cggggcgccc 202440
cgatgccgcg agcgacgctg gcctcacctg gccgcccggg cctgggcttt gcacgcgagt 202500
cctggcgccg agagcgttcc gcgaagcctc ccctcccgct ccgcagcgct gggcggtctc 202560
agcctcggcc tcgggctctt atccagtaga cagggtccgc cccgccccgc cctcccgccg 202620
acggggtcgc cctcgcgtcc tggctcctcc tcctcacctc cccgggccgc gcccccgccc 202680
ccgcctcccg gacgcagatg gctgggagca agcggcggcc acggctccac tgtcctgcgc 202740
gtcccggagc ctcggacacg gggtgcgggg gcctgggggt ccgagacgtc gcggatctag 202800
acgggcgcct tccacgcggg aactgtctgg gccgccctgc gccctcgccc gcacccctgg 202860
gggctgcccc gggcacccgc tatcttcgga cgcccagggc tggcggctcc cagctggacg 202920
gggagggagc cgagcggctg cgcgcgggga actgctggga ccccgtcggg ccgacccttc 202980
cctccctccc catcaggggc ccctacggtg gccccgcgct ctggggacca cgtccctccc 203040
accccgccgc tccacgctct gaccagcggg ctcgggaccc cgccgaggac tctgcacgct 203100

tcccggcgtg cgggctgcgg agacgcgggt ccttccgtgg ccttggggtc tccgcgacct 203160
cgaggcgacc gaggtcccct aggcctagtc cccgccttgc cggcatctag tgcgcctgca 203220
tcggaaccga ggggcccgcc tcgcactcgc ctaagaaaac catttcccgg gacgggcctg 203280
gaaagccctg gcggttggta tccggctcca gggccgaatg gactccgagg ctttccttcc 203340
gagatgcccg ggcgggattt cttccattca gcgcgcggag gaatggagcc cccaggccgc 203400
caaggcccag gatgtccagg tccttagaag gcaccgaggt gagtggccgc gcccagctct 203460
gtgcgtcgcc cctagggtcg cccgaacccc ggcgttcctc cccgtgtacc ctcgatgctc 203520
ccgtgagcac cctgccctgg gcgtccaggc gagccctggg gctctccagc cattccagcc 203580
tgcagcccgc gctcagcagt gagggaggca gggacccttg gtgcctgact ccgcagtacc 203640
cgcccgcagc gcggtgcccc accccctgcg cccgccccgc actccccgca caccaccact 203700
cccctccccg ccccccgctc ccccgctccc cggctccggg gctggcaccc aggcagaagg 203760
cactcagggc tgtgacttca cgctgagttg gaatcaagtg ggagcctagt gaccccctgg 203820
ggctgccatg acaaaaaact ccacagcctg gggctcaca cagaagcctt cagttctaac 203880
ggttccggtg tcccgaggcc aaggcctggc cgactctgtg tctggtgagg gccgcttcgg 203940
ggttgcagac agttctctcc ctgtatctcc ccacgctagg ggctggggag agctctccgg 204000
ggtctgtttt atgagggcac tggtcccatt cctcagggtg gagccctcct ggcctgacca 204060
cctgaaggcc tcctgacacc acctcccatc aggggtggga cttcatcgcc agggaggtga 204120
ggggacttca ggagaggggg atgtgccacc tcgaagctgt agtgtgtcag tggctgccac 204180
agaagggatc tctctacccc tattatctcc atggcacagg tttcaataaa aaggagtgag 204240
ccagggcttg gccccagagc agcagggttg aggaaacagt ctcccaagga aaagactttg 204300
caggtgacta agggaggctg ttaaataaaa cacacagaga gaatggatgt tcaggtatat 204360
taaaaataca caggccagga gtgagggctc acgcttgtaa tcccagcact ctgggaggcc 204420
gaggaaggag gatctcttga ggccaggagt ttgagaccag cctgggccac atggcaaaac 204480
cctgactttg aaaaaaaaaa ttagccaggc atggtggccc acacctgtag tcccagctac 204540
tggggaggca gaggtgggag aatagcttga agctgggagg tggaggttgt agtgagctga 204600
gattgcgcca tgggccacag gatgagaccc tgtctcgaaa caaaacaaca cacaggcaaa 204660
cagcgtttgg gaagggattg caagcgctgc aggagcgcag aattgtgacc caaagctaac 204720
agggtacagg caggaaggtg cagggactag aggtcacaca gatccaggca gccacatcct 204780
tactgaccag tagcacacag agaatgcacc tgccacactc aggaggggcc actgagtgat 204840
cacacgcatg taacctgcac tctgccaaga caaagagcat tcctccaccc ctgcctcaac 204900
cccgacttcc aaccacatga attaggataa aggagctttg ggtgctctaa gaacaaacta 204960
actgcgggag ttgggctcat ggatgacaca gagaaagcgc agctgccaga cccatgggtc 205020
tccatcctcc cacaacagga atgctccgtg aactgtgcag gtagaagcag tgtggttatg 205080
agctgatcag acctgatcaa acggccgacc tttgcatgct tggaactttg ccctaaatgc 205140
caagtcggct ttattatcat tgtggttgct gctatggctg ctgtgggctc agggcgttgg 205200
gtgcaggcag gtggagcccc cctgcctctg cacttcctgc ccatgctcgg cacctctccc 205260
cactggctgg gaccgcagct gcagagccca caaggagccc ctggccactg actgctgtct 205320
ccaaagcaac cttgccccac gggtcccccc ctccttgggt gctgcctcct gggaggccct 205380
cagtgctgga tgttgcgccg tgtctcgcct gatttggaag acagcgtgga tgctcccccc 205440
atgagtgctg tatctgagag gggacaagga cagtggcagt gagatgacag tcatttacct 205500
gcctgcgtat ttactatatt gagggtgtcc tgtgtgcttt cagaggaagt cccagggagc 205560
ccagctcaga ctttaaagac cctgacctct cactggtgct gggaaggccc tgggggtagg 205620
gggcttgggg agggcaaggg tgaggggacc agtaggcagg agtggctttt gtccaggggt 205680
gtgggaggtc agatcccaga ccttgtctgt ccaggttctg agaatgatgg aaggcgctgg 205740
ctccaggcag gaggccctgc tcactgctgc acccctgtgc cctccccggg ggccacctca 205800
ggaggaagat agtttcctct cgcatggcag ggacagcagt acacttccaa tgtcttctct 205860
cagagctgta tcattttccg gctcactgct ggttttagtc acagacatct tgatggtctt 205920
gccactgcac agcatgtcac agtggtctgc cacactgatg acatcaggct gaatcaacct 205980
ggggagcaaa aaatgaaaaa tgccaagatg ccttggtaag acacgtgctt gctaaaaatt 206040
gaaagagaaa ataacacatt ttcaaaacct gccaccttgg tgaactttct gtgggtccat 206100
tggtctgatt ggtcaaaata tatatccaaa atgaaagaaa acacgctgtc tctggcaagc 206160
aagactacca ctaagaaaga agtatcatac ctggtgaggc tcttccggtt tgcaaggtga 206220
ggtgcaagac actcggggat gcttctctgg cctgttttgt gttcttcata aagcgtttgg 206280
ttttgagtag ggcccagagt aagaaagatt ctgctgctgg tctggatggg caaactgctg 206340
ccccaggagg ccacacaatc tggtgttgtt ctccgacttt agctggcatc acagtcacct 206400
acataaacct ttacatgcat aggaggcaca gatcacctcg ctccatcctt agagacgcag 206460
gttcagtagg tctgaggtgg gacctgagga cctgcgtttc tagaaagttc tcaggtgaca 206520
ctgacactgc tggcccagga accacacttt gagaacactg atctggcaga accagtggga 206580
attggagggt ctgtggcaga cggagatgtt gtaggcagcg cagaaaataa cagttgaata 206640
agagctgaac gctctcctga aaatacgtcc tctccttttg agtaagagca gccctgagcc 206700
atagcagaaa ctcagggagc tctgttacca tagaagctga gcggcccagc agaagctatt 206760
tgttatcgga cccacagagg ttgtgtagac agcatcctgc cgtcctcagg tggaatgttt 206820
acgacacaag gcttgagcag gtctggaagg cgcggtaagt tccatgaaca agcacccact 206880

cttgctgcat ggagaccgct tccttaaccc agacctctgg ctacatgaga gtttcttatg 206940
acattgaacg gagaaaatgc atgcgttggg gcctggatta catgtgtcta tacacagcac 207000
actgcccccca ccagaaagtg aaaggctgtg tcatcatggc cttctgtgaa agaccagtga 207060
aagaaagctt cccagtgggc acatattcaa accatgcatc aggttcatca tctagccaag 207120
gagggatggc cagaggatgg ccctaaatgt agtcataggc agtggcgaat ggtctggcta 207180
cagagcagga ggcttcaaaa aaattgaaat attgatgaca agatttttct tggtagattt 207240
catgtttgta gaaaatcttg aggaatttac aaaaaaaact actagaattt ataaatgaat 207300
ttagcaaggc catgggatct gatgaaaacc aattgtattt ctatgtactg gcagcaaaca 207360
attctaaaat aaaacctaaa ataatttcac ttacaatagc atccgaaaca aaatgcttgg 207420
ggataaaccg tatccgagac ttgtgcaccg aaaacctaga atccattgct gtgagaaact 207480
tttgaaagtc tagacaagca gaggagtgtg tatgtttatg actgaaaggc tcaatattaa 207540
tgagatgtca gttcttcata aattgacata cagattcaat gcaatctcaa tcaaaattct 207600
aacttgcttt tttgaagaaa ttaaccagct ggttctaaaa tgtgtttgga gatgtgggtg 207660
acctagagta gccaaaagaa tcttgaaaaa gaacaacatt ggaggaccta ggagatgcct 207720
ttcatgattc caccatccta acaaaactaa ttggactttt tttctgtaat tatctcggca 207780
tttgtgtctt tttaaaaaca gccttgctga ggtgtaatgg atatgcaata ttccaagtgt 207840
acgatttggt gagtgactga tggggtcatg gcaacgcgat cacacagaag atgatggcgg 207900
tgctcctccc tcgctgactt ccctctgtct gtgtctcttt acctcttccc atccccaggc 207960
aaccactgat ttgctctttt tttttttctt tttgtgagac ggagtctcac tctgtcacct 208020
aggctggagt gcagtggcac gatctcagct cactgcagcc tttgcctcct cagttcaagc 208080
aattctcctg cctcagcctc ccaagtagct gggattacag atgcccgcca ccacacccag 208140
ctaatttttg tatttttagt agaaacaggg tttcactatg ttggccaggc tggtcttgaa 208200
ctcccaacct caagtgataa gctcgccttg gcctcccaaa atgctggaat gatttgtttt 208260
ctatgattac agaacaatgt gtttttaata gaattttata taaatagaat tatacagtaa 208320
gtactgtttt ctgggtctgt tcttttgcat tcagcatatt tgagaagaat ccacatcatt 208380
gcatcggcag ttaattccct tttagttatt tgtagtatcc attgtgtgga tatgggatat 208440
gatgtacttg gcttttccat ctgcctattg atggacatct ggctgttaca aataaagcta 208500
ctatgaacat ttgtgtgcaa gtctttgaac ataggctctc attactttgg ggtaggtaag 208560
aagtacttag ttgtggaatg gctgggtcat ttggtagata tatgtttaac tattttttta 208620
aaaactgtta aattcttttg caaagtagtt gtatcatttt atatttccat gaacagtgta 208680
tgataatttt agttacttca tattctaatg aatacttagt atggtcagtc ttttaaattt 208740
tagacatcct aatatgttta cagtaatatc tcctatactg ctctaatgag cgtgatcttt 208800
gaacaatttc tcatgtgtta gctgcatcag tgccacttcg tgttaagtgt ctgttcaaat 208860
cttttgcacc tttgggttgt ttaattgagc tttgagagtt ctttactatt ctggatacca 208920
gtcccttatc agatgggtac tttgcaaata tttttcccag tcttggactg tcttttcatt 208980
ctcttaatag attctttcag agaagagtta ttaattttga tgaagtctaa tttatccatt 209040
tttctttcac aaatcctgct tttagtgttg tatttaagaa atctttacca aattcatgat 209100
catcaagatt ttctcctaag ttttcctcta aactttttaa aattatagat tgcatattta 209160
cacatagaat gcatttttaa actagcttgg tgtaagacac caattgaagt tcaccacttt 209220
acctccgaag agccagttgt tcagtgttct gctgagttgc ttttgcacct ttgttgaaaa 209280
gtcagttgta tgtatgtgtg tgggtttatt tctgggctcc ctgttttatg atctgttaat 209340
ccattttgat gctaatacca cacttgctgt ggactgaatt gtttaccccc aaattcatag 209400
gttaaagcca taacccccgc aaggtgactt tatctggaga tagggacttt aggaggtgat 209460
taggttaaat taaattgtaa gggtggggtc ataacccagg aggactgtgg ccttataaga 209520
agaggaagct ctctccattt tactttctgc catgtgaaga tagagtaaga aggcggccat 209580
ctgcaagcca ggaagagagg cctcaccagg aactgaatca gctgccttga tgttggattt 209640
ccagtctcca gaactgtgag aaacaaatgt ctgctgttaa gcccccaaat ctggtgtatt 209700
ttgttgtggc agcctgagga gattaaaatg gcactatctt cattatggtc cttttgtaat 209760
acatttttaa atcaggtaat gtaagtcaga caactttgtt cattttcaaa gttgttttgc 209820
atattctcgg tcctgtgctt tttcatatga attttggaat caacttttca atttcttcca 209880
aagtcccgca tgggatttct tcgggtggag gctgccatca atttttcatc attattcctt 209940
tctgaggcag gcttcatgtc tagattttct tctaagttct gatttagcca aattccaaaa 210000
ggtttgatat gttgttttgt tttcattcag ttcaaaatat tttctgattt ccctttttgat 210060
gtcctctttg acccattgtt atttagatat gtgattagtt tccaaatatt agaagattgt 210120
ctggatatgc ttctttattt tttgccaaat gtatttttca ttttggtatc aaatttattt 210180
gcctctttgt tggagaactt gctttacatg acctgaatcc ctaaagggaa ttttatggtc 210240
tggactgtgg tcgatcttgg taagcttttc acatgcattt gaaaagaatg tgcactctgc 210300
tattattgga tgaattattg tataaatatc aaataggtca tgttagttcg tactgttttt 210360
gaagtcttct gtatccttaa aagtttctgt gtatctgttc tatcaactat tgggaaagag 210420
gtattgaaat ctccaagtat aattgtgtga actgctctat gtctccttgc agttctatca 210480
gtttttgccg aattcattat gaagctatgt tattaggtac ataaacattt gtccttttgg 210540
tgaactgatg ctatcataaa atgacattgt ttagacatga tctgttttga ggattcctat 210600
ggcttgcata ttaggtggct tgaagtccca cagttctctg atgcttggtt catttttttg 210660

```
tctttttct ttatttcatt ttgaacagtt tgcactgcca tctatgtctt caagttcatg 210720
tatccttttc ttctgagatg ttgaatccac ccctcctctc aaccagtgca ttttgcatct 210780
gagacattgt ggttttcatc tctagaattt ggattcgtgt cttttaaaaa atatctgtca 210840
tgtctctatt aaacatattc agtctcgcct ttggctagct cttggaatga ggtaacagtt 210900
atgattgtca atgcctctct aattctatca tttgagtcat ctctgggtct gtttctattg 210960
actgtaggtc acattttccc accttttgc attgtgctaa tttccagtgg atgcccagca 211020
ttgtgaattt aaccctgttg gtttgtggat aacactgtac taatgtgaat attctggaag 211080
tttgtctttg gccactggta aggtccctgt tgtagtttgg tcattctttc aggttttgct 211140
tttaagctct gcccaggagg accagagcag tgctttgctc agggctgatg cttcttccct 211200
gctggggcaa agcccatctt agtttcctgc ctaatgacca tgtttcccaa cggagggtaa 211260
atccagtctc tactcttcca ttttgactgg aagcaaaaat tgtggaccca ttcacttttc 211320
tttttctttt cttttctttg agatggagtc tcactctgtt gcccaggctg gagtgcagtg 211380
gtgcgatctc agctctctgc aacttctgct tcctgggttc aggcaattct cttgcctcag 211440
cctccagagt acctgggatt acaggcgtgc accaccacac ccagctaatt ttcatattct 211500
tagtagagac agggtttcac tgtgatggcc acactggtct ggaactccta accttaggtg 211560
atctacatgc ctcagcctcc caaagtgttg ggattacagg catgagccac tgcgtctggc 211620
ccccattcac ttttcttaca tttaagggag ttaatctgca cctagccagc ccttgacagt 211680
gtcacatgac agacagtggc ctcctcatca gtattgctgt ttctgttgtc cttcctcagc 211740
aaatgttttc cagttactct tgtagcttct cggccaagta gctcagttct gccctgggga 211800
atatgggcag aattcatgac atttagcttc atttagatga gagaacatac tctgtgtgat 211860
ttaaacatct tgaaatttat ctgtggtcaa gcacatggtc tattttgtca actgcttcat 211920
gtgcacttga aatggcacat catttggtga atagagctgt ttttctatgt aactacattt 211980
ttccctgata gttatatcag ttactaagag atctatgtta aaatctccaa ctatgtttgc 212040
agatttattt ctcctttaaa tcctgtcatt gatgcttaat atatttaagc tatgtgatta 212100
gatacatgca cttttaaaca ttgtgtcttc caattgaatt gaacttttat catcatgaaa 212160
tgtctgtctt taactctgct aattctccta gtctctaaag tacacttagt atgacaatct 212220
tgccaaataa actttcttaa gcttctgttt gcatgttata aatatatttc ctattctttt 212280
actttcttac ctttttgttt tgttttttta tgagacagag tcttgctgtg tcacccaggc 212340
tggagtgcag tggcgcaatc tcagctcact gcaacctctg cctccagggt tcacgtgatt 212400
ctcctgcctt agcctcccaa gtagctggga ctacaggcat gtgccaccac gcccagctaa 212460
ttttttgca tttttactag agatggggtt tcaccatgtt ggacaggctg gtcttgacct 212520
cctgacctca aatgatctac ctgcctcggc ctcccaaagt gctgggatta taggcaggag 212580
ccactgtgcc cagtctattc ttctactttc aatctatatt taaacagcat atagttaggt 212640
attttatttt ttatttagtc caacaaatgt catttaggag tttttagtct atgtttaatg 212700
taactactaa tatgattgga tttaaattta ccgttttgct atttgttatc tatctctatt 212760
ctctgtcctt tgttctttcc ctcccacctt ccttcttagt ttgggtaaaa tttacattta 212820
tattagttat acttctttgg ttctgttttc tatgggaggg ggtcttacta tgtgtatttt 212880
tttttagat gaagttttgc tcggtcacct caggctggaa tgcaatggca ccatatcagc 212940
tcactgcaac ctccacctcc agggttcaag agattctccc acgtcagcct ctcaagtagc 213000
tgggattaca ggtacccacc atcatgccca gctaattttc atacttttag tagagatggg 213060
gttttaccat gttggccaca aggtctcctg acctcagatg atccacccac ttcaggctcc 213120
caaagtgctg ggattacagg cgtgagccac catgcctggc cactatgtgt atttttaact 213180
taccacaatc taccttcaaa tgccaaattt ctgcatggta ttctcttatt tctgcctgta 213240
gaactctgaa cattgttggt cattatgaac tactggcagt gaattatctc agcttttgct 213300
gatttcaaaa tgtttctatt atgccttgat ttttaaaaga tgtttatttt agcatagaat 213360
tccagggtgg ccgcattcgt ctttagtctc tttgaagatg tcatcagtca tctggattgc 213420
atagtttctg cagagaagtc ggctgtcgtt cttatctctg ttccttgacg tctgggttgc 213480
atagtttctg cagagaagtc ggctgtcgtt cttatctctg ttccttgacg tctgggttgc 213540
atagtttctg cagagaagtc ggctgtcgtt cttatctctg ttccttgacg tctgggttgc 213600
atagtttctg cagagaagtc ggctgtcgtt cttatctctg ttccttgacg tctgggttgc 213660
atagtttctg cagagaagtc ggctgtcgtt cttatctctg ttccttgacg tctgggttgc 213720
atagtttctg cagagaagtc ggctgtcgtt cttatctctg ttccttgacg tctgggttgc 213780
atagtttctg aagagaagtt ggctttcatt cttatctctg ttccttgata gtatgttgtt 213840
tttctccagc tgcttttaag atttttctcc ttttcagtgg ctttcagcag tttgtgatgt 213900
gtgtcttgct gtgtttttat tgtatttttt ctgctgggtg cttattgagc ttcttgggta 213960
tcttagttta tagatttgga aaaaaattct ggccattatt tcatcaaata atttttctgt 214020
cctgtcctcc tccttttcta gggctccaat cacatgtgat gggccttgtg gcattattcc 214080
atgcccactt gtgcttcatt tctgtctgtt tctccctgta catcactggg atcacacata 214140
gctgtatttt caggttcact aatattttct cttgtcgggt ctaatttact atagtctctg 214200
ccagggtatg acacaggcag tggatgtcag cctgtctctt tcccagccaa cccagtgttt 214260
acctagtaga accattcaca aactggcact ggaaataggg atggagatga gtgtggatca 214320
ataacaaaga gctgacccat caacaatagc ctaggctgat gcagttatca ctggactccc 214380
aacatgtcgg aagcagggtc ccatacttag ccccaggtac cattcctggg aaagggacta 214440
```

```
gccagccccc tggaggcagg ttgatcacac tggactcttc catcattgag gggagtatct      214500
cttggtttca gtttccctta ttccagatat ggatatgtct ttgctgtcag tgtttctgac      214560
agcaccatct gtgggcttga tgaatggctt attcactatt gcccacctcc tatggcattg      214620
ttaaggaatt tactttacag caaaggcagc atgggaacag gtacacactt ggaatcccta      214680
cgtttcccat cacccagagg tggcgagtct gattaagcat gaggatgact tcttgaagct      214740
ttaggtgtgc tgcccatggg gaggcagtgc cctagaacat tggactgcca tcctgatgga      214800
tgacatagtg cctcgaactg tggaccaaga catggcacca tttcatagcc agaataatgt      214860
ctgtaatgtc aatgggtgga attaggactg gccctgctca ccaccacatc caacacttac      214920
tgcagggtgg ctggtgctgc tgttcagaag atccttcacc tcctttgagg ggctcccttg      214980
caggactgta cgtctctggc tgttgaattc agctgtgacc acatggctta ctgtggccaa      215040
tgaaccagga gtgggcctgc tgtattctgc gtctgtgcag cagatatagc atctaggcag      215100
gcttgcttgt cacctcttcc caaagccatc tgactggctg agtcccagga ggggctgctg      215160
tgctgggctg gatgcagatg gtgacatgga gcagagctgc agctgacctg cctgggagag      215220
acgggcgagg accacatctg tttagctgac cctgacattt tattggttta catttgttac      215280
cgcagcatca atgatcccag gtgggctgat aaacccattc acatattttt gcttttctcg      215340
tctgtgacat tgggctcttt tgctttcaag gtcctagttc ccaaggctgg aatgctctta      215400
cctgggacct cagtgatcca tctgctgcac tgaaagttca gacggtcatc agctatttgg      215460
ggctgctcat gccacttagc taatagttca agaaaagcat gctgtcttgg tggggatggc      215520
taaccccaat gaaagggcta tcttggagct cagtggattc tctaggtact tctcagggct      215580
ttcacgacta atattgtgac taataaaggc aagacgacaa ctgcatcaga cacactggaa      215640
tgaaaatttg agtcctctca ctgggtaaag aaccctgacc atccaaagta ctgcttaggg      215700
tcaagaaaac gtggaatggg tagtggaaga aggaaggtgt agctgctgat cacagcctca      215760
cagctagcca cagcatgagg acagtaccgg gcatgcaggc tgtcctcttt gcttgctgtg      215820
tgtgtgtgag catgtgtggg agcatataag tgtgtgtgca ttagtgtgag catgtgcatg      215880
catgtgaatg tgtctatgaa gacgtgcatg tgaatgtgct catgagcatg tgtgtacatg      215940
tgtaagcctc agcatgagtg catgtgagca tgtgtgtaca tgtaagcctc agcatgagtg      216000
catgtgagca tgtgtacatg tgtaagcctc agcatgagtg catgtgagca tgtgtgtaca      216060
tatgtaagcc tcagcatgag tgcatgtgag catgtgtaca tgtgtaagcc tcagcatgag      216120
tgcatgtgag cctcagcgtg agtgcatgtg agcgtgtgta catgtgtaag cctcagcatg      216180
agtgcatgtg agcatgtgtg tacatgtgta agcctcagca tgagtgcatg tgagcatgtg      216240
tgtacatgta agcctcagca tgagtgcatg tgagcatgtg tacatgtgta agcctcagca      216300
tgagtgcatg tgagcatgtg tgtacatatg taagcctcag catgagtgca tgtgagcatg      216360
tgtacatgtg taagcctcag catgagtgca tgtgagcctc agcatgagtg catgtgagcg      216420
tgtgtacatg tgtaagcctc agcatgagtg catgtgagca tgtgtgtaca tgtgtaagcc      216480
tcagcatgag tgcatgtgag cacgtgtgta cgtgtaagcc tcagcatgag tgcatgtgag      216540
cctcagcatg agtgcatgtt agcatgtgtg tacatgcata agcctcagca tgagtgcatg      216600
ttagcatgtg tgtacatgtg taagcctcag catgagtgca tgtgagcatg tgtacatgtg      216660
taagcctcag catgagtgca tgtgagcatg tgtgtacatg tgtaagcctc agcatgagtg      216720
tatgtgagca tgtgtacatg tgtaagcctc agcatgagtg catgtgagca tgtgtgtacg      216780
tgtaagcctc agcatgagtg catgtgagca tgtgtacatg tgtaagcctc agcatgagtg      216840
catgtgagca tgtgtgtaca tgtgtaagcc tcagcatgag tgcatgtgag catgtgtgta      216900
tcattgtgtg agcatgtgtg gatgtgaacg tgttgtgcat atgtgtgtgt gcacgtgagc      216960
ctgggagcat gcaagtcatt tgttaattga ttactccttt tcccacttct cagcatcccc      217020
actattttac acagggaata ttagtggtgg tagatggtga actttgaaac ttaagttttt      217080
catgtcagag aatgtccaga tgggtttgtg gctgtaccag agaaaccatt agtggcgagg      217140
aagactcatg ggtctctact gtttctgggg aaggtaaaag cacctgtgtt gggtggtgtg      217200
agtacagcac tgtgttggaa gatgtctgag tgtgtgactt acttgtggac acagagcagc      217260
tatgggtggg cagtgctggt ccttcactac cccgtttgcc ctcagcccca cttctcacat      217320
tcccgagctc tgcttcatgg cccagggggc ctctcctccc agccagcttc tggttaggat      217380
cagcatacgg cagcagcagg tgcagctgtg tcaactcttg cgtgaacacc gggggtgggt      217440
ttgcttttc ctgagggtcc caccttcatt tctcctggct ccaatgctga gtggagaact      217500
gaacacacag aagcagaggc atgttcgatg acaaacacgt gtgctgcaaa ccacacgagt      217560
taccctgctg tgcacctccc tgggtgcacc cgccgggcac ggccaccctg cccacagccg      217620
gcacctggag ccagacgctc ctccttctcg gtctcacacc agccgcatct tagcagcagc      217680
tgccccatcc agtgctgatg tgagatgtgc agatctagtt tttgttttaa cccttgcttt      217740
acttgaaaac ttggacatat tcaatcaatt tcaacggcag tattcatgta ttaaaaatgg      217800
cagatgcata caagcatgaa gaagtaaaaa ttgctgttgc ttgaaataat gtcattttgg      217860
tgcacattct ctctcactga gtgaggtcag actgcacagc tccctggtag cccatgtctg      217920
agacttacct tcctgaacaa cgtaacgatt catttttcac cctcccagta tcccccataa      217980
ttgtacacag ggaaatgagt ggtggtggat ggtgaacttt gaaacttcgt ttttcaaagt      218040
attaaaaagg agaaacacct gcagattggc catttgcagg cttttctcct taagtttttc      218100
ttccatcctt gacgctggtg gagacacaat cttgattctt cacgtgcagt tttggagagt      218160
gtaacaggtg agctcggtct tttacctcta gttccaaacc ttttactgac tcagtaggac      218220
```

taaatgccag atccattttt ctctctgctt gacaaaacac gcttaaggtg catgtgctta 218280
aaattgagaa attacgtaag tcaacccacc aggcaggcag ccagccatcc atgtttcaac 218340
tctgtaagat gtctgctact gtccgcaaaa taccagaaca ctccggagcg tccctttgcc 218400
accaagacag ctttttctac agaatttcaa ttcttgtgtt ttccaaacat gctagaacca 218460
caccaagaaa atctcatctg gattggctct cctctcacaa tgcctgttgt tgtctttcta 218520
ataaagaacc acacaaaaca acatcattcc caagtataga ctttatgttt tacttattcc 218580
attgaaaatc ccaagttcct tcatggcaac cctccctctg ccctcacacc ccagcaggtc 218640
cccgcaggcc agggcccatg ccccacctgc ccacgggtct ggcccccagg ccaccagggg 218700
cccgctgcgt cctgtctcac acacaagggg tttaactcaa cgctatgtac attcacagtt 218760
ccgaatatcc gccaactcta agtcgccacg aagagaaaag agaatcagga ggaacaaaca 218820
tccattcaaa gtctgtttat cagagatttt tttttctgaa aatgcacagt ggcatttcat 218880
tcaaaaaacc cttcatctgc agacctgcgg aagggaggtg gcctgggtcc cttccctcgg 218940
aatatcttag tttatttacc tccttcattg gccatttgca ggctcttctc cttgaaaaat 219000
gaatctttac gcattctcca attataaaat cagtgactgt tagctaccaa aggctgcact 219060
aggatttctt ctgtgtccaa cacgccaaga gccctgaaat tgacttcggt ttactccatc 219120
cctgtctgtc ctgctggcag tgtccatgct aaaaaacaag tcgaggtgat tgattgaaac 219180
ggagctgaag gttgttttta aatgtctgtc agtggagaaa cgcgtatcac gaatctctga 219240
ggaagttagt aaacattttt tcccgtactt actggccttg gtggtcactt tctatagaga 219300
tgccccaaat ataaaaatca attcatttca gaaatcaaaa aaattttcca aacaaacccg 219360
gagcctttgc tttaggaagc aaactcaagt ctgtgatgat tattgtgctt gaaggatggg 219420
tttctaattc tgtcataaaa aatgatgaaa caaggtgatg tcagagggac ttcaggtgac 219480
accaaaatac cccatccagg gcctggaagc ccctctgcct ttgactctaa gatggggcag 219540
gacccccgct ctcagtggga tgtgcccgcg accctggcgc tgggaggcag tgctgcagag 219600
aggagtggtg tccgcacggc tgtcaccaga catcctcatc accctccgca ccgcacagcc 219660
cagcgtctcc ttccacaagc aaaagactgc ggctcctctc tctgcaggaa ggtcagccat 219720
actcgggtgg cagcgtgacg ccagtcacca tggaaaccag ggctgacagg aagatgcagc 219780
cgcttccagc agggacctgg cctgagggca agggaggagc ggcacggcgg cggctgcctt 219840
gcgcttccag ctcgggccct gctcatttga acaatatcca acctcggact ggagtttagg 219900
ctacagtgaa atggattacg ttatgccgaa acagaaacat caaactgcca acgtttataa 219960
aaaaagaggg gactggaaaa ggattaagaa aagcagaaaa agtgttttgg ggctaggccc 220020
ttgagttcac acctccccag gggacccagc ggaaggagag ggattacctg ctggagagag 220080
gcctgatgga aatttaagtc aaagcccaaa cagcctcaga ttctatatta aaaaaaaaaa 220140
ggcctataaa taaatatcct ttgactctaa aaggaaaaat gaagtagaaa aacaattttc 220200
atttttttaaa aaatgctgca tgtttcctga agtacctaaa tggagtttat aaaatactca 220260
tgagctccag cacacaggta gaaacactga aaaatcaggt gacggctcgg ctctgccagg 220320
ctgtggatcc acagaggcag acaggtttcg tgccagagtg acacgcaagc gcacgctgtg 220380
tggcaggcgt gtgctgagtg tgcacggagg cccgcccggt gcacgctgcc gccggaagag 220440
gctgtgacag agactcgaaa ccagggcccc gtgggagaac acggggcggc accggtgccc 220500
cccgcccggc agggaacctg accccacggg gtccaggcca ggcgggggat ccgcgtgcgc 220560
gccctccgat tctcgcacag taagcgtcgg ttctgcaaca cacttcacaa aggaacaaga 220620
tacaaacagc ccccgaggcc cctggaactc gggctgaaga cagtgcctgt acagcaggag 220680
tgaggagcgg agcccagagg tcactcgcaa agaggctcat ggcggtgatg tccacgcggg 220740
aggggccgcg tctctccgca accctgggtc ctaatccagc ttcttgcgaa caggcttccg 220800
cccagcgtct gagttttccg ggctgaaatc ggcacagaag ccgtttggga ttggcgcagg 220860
tgaggtctct gcacagcttt cgaaatgtgt ctgatccggg tacaggtatt cctctgcgaa 220920
ggcagggtac atttctgcaa acctgtggaa gtcagctgga aagacaaagg cacctgtggt 220980
tatggaatgg ggacgagcaa atgtctagga tttggaggct cttttcccac ggccctggtg 221040
ggtgtccacc tcacgccctc cagggagggt tagaatcgtc tgtgaaacgg atgctttcag 221100
ggtggagaga agaacttgcg cttactttct ttgcagcgca cgaagtcacc ctgacaaccg 221160
gggcaggctg ggtgtcctcc tagctactcc tgcttttggg aaaccctaca gaaagcttct 221220
ggttttgaat tcgacagata tatcccgggg acttagcact tctgaggcac tgtaaccaga 221280
gaagctggct cagggacaag accaagtgtc tggacagtga tctggcgctc tgaggggtg 221340
ccctcgtcca ccctgggaga cccggtaggc aggggccgt cctcacagag cctcggactg 221400
cacttcctgg ccgctgtgcc tggctctcat gtgagcgccc tcggctccta ctgatggcgc 221460
catggaggct tctggtgggg ctggaaagat ggtgcagggg gctggtcatg ctaggccgct 221520
ccagaaacac tcagaacctg ggccacccgg cgagaaatcc agggatggtt gttactggaa 221580
attgcagctg gtcggtgccc actacaaaca tcaaagagcc cgaacacacc aaggagcatt 221640
gcacactttc aaaatggaaa caagcgcacg catgcacaca tgcaaacaca cacacacggt 221700
aaccaaacac acatgtgcgc acacacacaa aacaagcaca cacacggtaa ccacacatgc 221760
gtgcacacac acacacacaa aagcacgcac acacacggta aacaaacaca tgcgtgcaca 221820
cacacaaaga gcacacacgg taaacacaca tgcacgcaga cacacacaca caaaacaagc 221880
acacacacgg taaccaaaca cacgtgcgcg cacacacaca aaacaagcac acacacggta 221940
atcacacatg cacgtacaca caagtgtgtg cacacacagt aaacacatgc gtgtacacac 222000

```
aaacaagtgc acacacacac taactaaaca cacatgcgtg cacacacaaa acaagcacat 222060
gcacacacat ggtaactaca cacatgcacg cacacataca aaacaagtgc aggcacacaa 222120
taacatgcac acacacaaaa caagcgcagg cacactaaac acacatgcgc acgcacaagt 222180
gcacgcacac acacggtaac acacatgcgc atgcacacat ggtaaccaca cacatgcaca 222240
cacacacaaa acaagcgcag gcacacgcgg taactaaaca tgcatgcaca cacacaaaac 222300
aagcgcacgc gcacacagta actaaacaca catgcgtgca cacacacaaa acaagcacac 222360
gcacacagca actaaacaca catgcccatg cacacacaca cacgcacggt actaaacaca 222420
tgtgcgcgca cacacaaaac aagcgcaggc acacagtaac taaacacaca tgcgcacgca 222480
caagtgcacg aacacacacg gtaacacaca tgcgcatgca cacatggtaa ctacacacat 222540
gcacacacac acaaaacaag cgcaggtaca cacagtaact aaacatgcat gcacacacaa 222600
aacaagcgca tgcgcacaca gtaaacacgt gtgcacacac acaaaacaag cacacgcaca 222660
cagcaactaa acacacatgc ccatgcacac acacaagtgc acgcacacac acgcacggta 222720
ctaaacacgt gcgcacacac acaaaacaag cacaagagca cagacacggt aactaaacac 222780
gtgcgctttc aaaatggaaa caagcatgca catgcacgca cacacacaac acacatgcac 222840
gctttcaata ctgaaacaag catgcacatg tgtacacaaa cgtgtgcaca cacggtaaac 222900
atgcacactt tcaatactaa aagaagcacg cacatgcgtg cacacacgta tgcacacgca 222960
cggtaacaca cgtgcacttt cagtactgaa acaaacgcac acaggcgcac acgcacacac 223020
acgtgtgcac acacacggta actaagcaca catacatgct ttcaatactg aaaccagtgc 223080
gcacatccgc actcccgcct ccggctcccg cacagacgct gtgcacgcag ctctctcgcg 223140
tgtcttctcg tttgctctaa gaccaccctt ccaggtgggg attgcggcat ccactgggat 223200
gagccaggcc agggcctgca gccctgggag cctcgggtgg atgcagcgta gctcattcac 223260
actgcagggg agggaggcgc cccgggctcg ggtttgcgga ttttgcacag caatttaaaa 223320
atatcaaccg ccattaccga cagctcgtgc tctggaatca gccgtgttac cagtcacctc 223380
atcagatgtc cacagacagg tgacttttct cgggaggaag tcgccctggg cggcacgggg 223440
gtctgcagcg cccgcctcct cgtctctcct gacagacccc tcccaggaga gggggggatgt 223500
gagccacacc ccggctggca gtgacaaagc ccacagccag tggcctcgag ggctgggtgc 223560
gggtatcccc cacctccagg cacctgaatc ccagcctgag gccatggaag gctcagagct 223620
tcggaactgg gcttggagtt tggttgtata aatcgccaaa acaaatcaga ttggaaagct 223680
ccctacgaac gctgccaggt ctccagaacc ttccttctca ggggtttctt tgttacacag 223740
ggcagcctgg tgaatggagg catggcgggg actccactcc tgtcctgggc tcccacggag 223800
actcgcccca cactctgtcc agcccccgcc caagggtcgc gaggacgacc ccactcctgc 223860
gggctcaccg aatgtgatct tccccttctc ctcttggtca atggctcgga ataggtcggt 223920
cacggtgagc tctgccaccc ccagggccgt cttgaggatg caggacaggt caccttcgcc 223980
gacgctgccg tcctcttgcg ctccgtacat ctgcaaggca aactgggtgt caccttgcag 224040
cctcctcccc tgcccaccag gccccgctgt ccagggacac cccagcggcc ctgccccgct 224100
ttgtcagagc tgctgggcac ctgcagggcc ggcggctgga cacgggggac tcgaacttcc 224160
atgtggagcc atgcagcagg gccctgtgcc ttggatctgg ccccctccag gtgcctcgtg 224220
gacacacagc agatgcttct cgggcaaggg ctcagacgct gcccacccca cccgtgatag 224280
ctcagcaaac caaccagcac ctgtggctgc cactgggagg ccgcggaagg gtcctgcctg 224340
ggccacgggc gagcgcggcg caagcttggg ctgcagagtt cccccacggc cctctgcctc 224400
cgtgagtgca ggctacagca gacgtgttca caagcatcag taataacatg aaaaagccct 224460
gatggttaga aatcgacagg gcagatcagg agcagaaatg agaaggacct gggagggccg 224520
ggagggctcc agcggggagc gggaaccagg acccgggctc tctcctgagc ctcacaggtg 224580
acgcctcctc tcttcctcct gctccaggtg tggaagcggc ggctccggcc ctctctgtgc 224640
agggcccagt gccgaggcgc ctgccgcccg ctccccacaa ctccacggcg ctcccctccc 224700
tggctccaca gactctgtgc cctggagatc ccccagaccc cttgatcagg gcccgcccat 224760
ccctttgtcc ctctacctcc ttggaaacac ttcggctccc tgtggggtgc agggtcctgc 224820
cccagccctt taggctcgag cctggcccac gggctctgtg gcagcgcttt cctctgaggt 224880
cccgacgccg cccgtgtcct tccgctccaa cccctgggct ccctcagatg ggccagctct 224940
tctacctggt tcttccccga gtcctctgcc tggggctgca ccatcccccg ccgccctctc 225000
tctgcctggg gtccacggtt cctggggctc cgacggggcc gtcgccctga ctccgagtcc 225060
ccttctcctg ctgcagcgcg ctgtgatctt gtgagcgttt gctccaccct ccgagaagtg 225120
ggctgcctgg gaggggcgtc gcctctcttc agggaggggc tgcgccttca cctccaccgg 225180
ctcctccttg cggtgctcag ggaagaaacg ctgacgcctc cagccttccg gcctggcaag 225240
ctgtccccgc gttcacaccc tcttcccggg gctctgcctc taccccatgg accgggtctg 225300
ctccaggctc tggagacccc atcctcaccc tccactctcc ctcccctggg cctcatccca 225360
cagcgctgcc gcacgcggcc accctgtctt ctgtggtcta cgtttctctc ctgcttttag 225420
atttgctgaa tcaaacatct gcagacgaaa tccacgttgg tgctgctgga gcctcagaac 225480
gggccgggtc aaagcttcag ggccacggcc tccagctcct cggccgacct ggatcccact 225540
cagcccccag agagcagtgc ctcctgcccc ctagtggttg caaggctgct tcagagggga 225600
caacctgaca tctgtgtcca tttggacatg agaactgcag atgcagactt tcatcctcgg 225660
agcctcgtgt agatgctgca cgggaaacgc agaggtagga ctgaggctac cacaggcaac 225720
agacttcagg ttctcactgt ccactctctg agccgctgga agttttacca gggccacatc 225780
```

ttaaggtttt acacagtaat gataacgaaa aagaacaaaa acaaggggcc ccaaggcgta 225840
tgaagcacat cggtgcgctg cgcatccgga gtcctgagca ccggcgtgtg cctctggggt 225900
cctaggtgct aggagttctg tgtccacagt cagcctgggg ggcagcttcc tggatctctg 225960
tgtcccaaga gcagcacgct ggtgcacttg ccacgctctc tcacacgaga ctgctccgtg 226020
gaagcccttc ctgctacatg cagtcccccc aaaatgtgcc aggctcccag agcagcagca 226080
aagcacaatg gccatgactt caggaaatga tgacaccatg aaaggtatag gctgcagaaa 226140
gggacatggg gaagcagcct gcaagaaggt gggaggaagg cagggcatat gccgtcaggg 226200
cggagctgct gagggtggag gtgaggcggg accctggtgt cgccgtgggg acgcagccat 226260
atcagcggcc tggaagcctg tgagaagtga tcacccatcc tgcccccagg tggtcactgt 226320
agggcagcct aggcctcacc catcgcctgg ttcacctgag aaagacctcc tagtggcaca 226380
taccccaccc caagcaggga cccggtgcac actgtcctcc ccagtgggcg tcctcaaccc 226440
taccacagcc agttaccctg cccacctgca ggtggggcct gtgcaggagg aaggtcgggc 226500
tccacagccc attgttcatg ttgagaaaca aggcaactgc cacccccacc gctgctgtca 226560
ttcgttccag gttatacttg gtggctctac agcaggggct ggtggggcca gcagaacggg 226620
gggcggtggc tcatgcctgt aatcccagca ctttgggatg ctgaggtggg cggatcactt 226680
gaggccagga gtttgagacc agcctggcca acatggtgaa accctgtttc ttctaaaatt 226740
acaaaaatta gctgggtttc atggcacgag cctgtaatcc cagctacttg ggaggctgag 226800
gcacgagaat cacttgaacc tgggaggcag aggttggttg cagtgagcca agattgcacc 226860
actgcactcc agcctggaca acagagcgag actccatctc acaaaaatta aaaaaaaaaa 226920
aacaacaggg cacagtgaaa gcagggccag caggggccgc agggccacag ggcctggctc 226980
agatcagggg ccctaggagg caggctgccc aggcctggag gaggcaggga ggtcaggctt 227040
tcttacacag agctgccaaa tgttgaggtc atcgagttca tcctgcctgg ggagaccccc 227100
acaagccacc ttttcatgtg gggacattcc ccagtgttgc tatggagagg gtgtgctggc 227160
atcagaagca agagaaggga gttcttggag cggtgagaga catgcagctt cttgactcac 227220
acatcaggcc cttgagagtc tggctaatgt gagtcacacc ctgtgagaag ccttggtaaa 227280
gttgtgcctc tgtacaggca aagccggccc agctctatcc cagatgaaat ccacattggt 227340
gctgctggag cctcagaata ggccaggcca aaccctcagg gttacctggc ctcccgccct 227400
ccaggcctga cccagacccc actcagcccc cagggagcag cgactccctg tggtgaggca 227460
agtcctgagg gcttcccagg agtctgcagc tgggctggga ccccagactc ccccagtggt 227520
gatgactggg ctctgacatg ttgtagcccc tggggttagc tgaggtgggc agctcctaag 227580
ccgtgctttc ttcaccagtc cagtctgagg gggagagagg acaaggtgtg tggcccctgt 227640
gttaggtgcc ctgtagtgtg tgatggtgtg tgacaccacc agccccaccc aggtgcccca 227700
ggacccagga agaacctcag ccatgctttc tgatgagtgt ggattgctaa tccaggaaga 227760
aataaatctg ggagttttct gggcatttta caaacaacag ctagcataaa aagcttacgt 227820
aaaaatagca gttgggcaaa tgatcaatta actgatatta taaggaacta gagaaatgaa 227880
caatggtgct gacgtgactg caccgccctg tcccccagtc aaacccatgt gaactctgca 227940
ctcaccttga aagccagctg gatggtgtcc agggtccggg ccggccggca gacgacagac 228000
agggcaacca cacactctcg caggtccacc tcgccgctgc cgctctgtgg ggagagacgc 228060
tctcagccac agctcggccg ccttcggcac tggagaaacg ccagggtttc ccatgggtgc 228120
tggtgttaat taaaggccag tcccactctc actcgggaac atctgcccat gtgaaaaggg 228180
aatcttggaa ggacattcca ccaagtgggt tctccaaggt cactgtgctg tgtacacttc 228240
ttggggccat tacagtgact tcccaaagtt cacaaggctg ccacaggaag cacctcagca 228300
aagcacacgc gatgcaatgc ctgccacctg ccaagacctg catttgagaa tacccyctaa 228360
gcaccaaggc tgattctggg ctctaggaat ttggtctgtt tatggcaacc cattcatcct 228420
gcagaaagaa ggtgaacccg gagctgtgac gtggagctaa acagcagccc gggccagctg 228480
tggaagccag tcagccccgc gctgaccatt ctcagtaact cctcacaccc agagtctcca 228540
gggacatgct gatgtgagtg agcagggtgg tgagaggcac aggtgggggc tgacagggtc 228600
tgcacttctg atgacgatgg ggacagtctg cacgtggctg cagggtgtgc ccaggccaca 228660
ggaaagaacc tcccaggtca gggcgggagg aggtgaggag caccctggac agggggaaga 228720
ctctctggtc agacagcagg agatgaaggg caaccaggag tggagggagg actccctggt 228780
cagagagcag gaggaggttc caggaagaga gcagcaggag catccagggg tagagggagg 228840
gctcccaggt tggagagaac aagagaaggt ggggagcaca caggataggg gggaggactc 228900
tggccagaca gcaagtgaga agcactcagg acagggggag gactctggcc agagagcagg 228960
aggtgaggag cacccagagg caaaggaagg actccctggc tggagagagc aagagaagat 229020
gaggagcacc caggagaggg gaagaatttt ggccagagag caggaggtga ggagcaccca 229080
ggacaggggg aggactctgg ccaggcagca ggaggtgagg agcactcagg agcagaggga 229140
ggacgctctg gtcagagagc aggaggaagt gaggtgagga gcacccgggg cagaaggact 229200
ccctgatcag agagcaggag ataatgagca cccaggatgg gggaggactc tggccagaga 229260
gcaggtgagg agcacccaag ggcagaggga gaactctggt cagagcagga ggaggcgagg 229320
tgaggagcac ccagggggcgg agggaggact ccctgttcag agagctgggg gtgaggagca 229380
cccaggggca gagggaggac tttgaccagt agttgcccct ctacgcttgt gtcaaccaca 229440
ggctgtaggt gctaggggtg agacgcacac aggtccctga aggtcttctg agggaactga 229500
tagctttctt tgtgctacgc cacacccctt gcaaggcatg gggtactggg actggggctt 229560

gggtgtctgg caggtggaaa tgactacaag tccctggctc tgggaaggtc tcgggctggg 229620
ggtctgaggt cctgggtgta aatggagaat actcctatct cataagatca tgtgcatctg 229680
aaattgcaga aaataaaaaa ttaactgcac agtgtcataa gttacctcgg aacgctgcct 229740
gtgaaaccag gaatgctgcc tgtgaaacca gtattcacca tttcacgttt caattccaac 229800
actgtggtaa agcgcctgag tgcaaacgca gctgcagatg tggcacggac accaggaggg 229860
actcgcatcc gagacgctgt ttgcagctgc ctcgtagtag agggcccgcg gctgggggcc 229920
ccttacggac actaggcagg actcgcatcc gagacactgt ttccagctgc cctgtagcag 229980
agggcccgtg gctgggggcc cctctggtgc gtgtgggaga atgccctgag ccacgcactg 230040
tgatccttgg ttcacactct gcatacttgc ggggcagcct ccttcccctg tcctgggtgc 230100
tcctcatctt ctcttgctct ctccaaccag ggagtccttc ctttgcctct gggtgctcct 230160
ctctcctctc tcctgctcct cctgctctct ggccagagtc ctccccctgt cctgagtgct 230220
cctcacctgc tctctggcca gagaggcctc ctgccagccc gggttccacc caggactgca 230280
ctgcagctgc tccgtgttgg tgcaaaacat atatggtgct gtcacacggg acatctcatg 230340
ctgtcacggg acagtcatgg ctgaaggagc cttctacaca caccctggcg tatcaccagc 230400
tcctccagga ccacagtgta agtgtccatg ctgtgacact aacacccaga aaagaccagg 230460
attggctgac ctgtggctcg ccgtggcagc ctgtcccgcc cctgcgtgcc acctcgcacc 230520
ttcctaaaac cccgaggctg cccgccatgt tcagggccac ctggtgtgac cgtggatgcc 230580
tcatcaatta cgacgaggac ctggggatga cgtgcggagg gcagagggga gtgggcggct 230640
gcaccaggag gaagccctcc cacacccaca ggctggcggg gagcctctaa gccgatcata 230700
gaggacgggc gtgttattag aaactctgac gctgcaaaga gcagctctgg tgagcagggg 230760
accctgcagg gggtcccttg gagaccccag gctgggtgtc acagtttgat attgttagac 230820
tccgtaatca cactgaaaaa ccccaaacca caggcacatt catcagtgaa gggtaattta 230880
aacgtcaaat gtgtacgcgt atttaaaagc atggaaggaa atacctcaaa atgatagggt 230940
ggggtggtga tgggtggttt tccttctctg ctttcaaatt catgtgaaaa tatatttata 231000
ttagaatgag aacaatttat gcttcaaaaa gcaggaggtt ttgccgacac cccgctccgc 231060
aggcgacagg ccctacctcg tcgaacagtg aaaacatgtc ttccagcaag tcagaaacgg 231120
ggacttccag ggaggcggca aactccgcaa tacctatctt ctctcctccc ttcatcctgg 231180
ctctttctga gtatctgtcc agatcttttt caagcttttc tggttttagc ctagacaaaa 231240
aaagagggaa agctttcttt ttcaataaaa tggtggcatg ctaacaccag atttacttct 231300
aagactcatc aaaatattaa aattgacaga agtagcagca agctagtttt caagaaaagg 231360
cattctccta attggaaggt gcgagatgtg ttctgtccaa gtgtcaagcc aggtgcctgt 231420
gcccctgaga cccgttccct tcccagggca gatgtggcag gacacagggt ggccctcctg 231480
gggcgccgtc cacagacagc tgatgccagc attaagggat tgaaagacac tatgtgggct 231540
taagttgata tttgaaaaaa gccagaataa ttaataatta tctcctcagt tcccctccct 231600
gcaaccccag gcagcccccg ccagacctcg gcatcacggg ttctagctaa tgctcaagga 231660
agaagaacca ggtactcacc cgaggccccg cacgagcctg gcaaattcta aaaggcaagt 231720
gtcagcgggg agacggagct gtccttccgc cagggccagc tggcagtcct cgaacgtgta 231780
gtcagtcacg gagacaccca aggccctaca aggagggcag cacccccgtc agcccagcct 231840
cgtggcagcc agttcccacc gccccaccag aataaccgcc gatggctcag gggagaggag 231900
ggctgaggag aggcagtgag acaggggaag ggccagttgg tggggcagtc acacattcat 231960
cgatcagctt cacctctcgt gggtgctctg aaacgatgac ggcagccaca ccaaaggtca 232020
ctgacacaga ctgccatcac gtgagaatcg tgaaaaagtt tgaaacagcg tgagaattac 232080
caacatgtga cgtggagaca cgaagtgagc tcatggtgcg agaattacca acatgtgacc 232140
cagagacgcc aaagtgagct cacagtgctg gaaaacggtg ctaataggct tgctcaacac 232200
agggttgcca caaacgtgaa gcaactgtct gtgaagcaca ggatgaagca gagtgaggcg 232260
ggctgtgccc tcactcccag agctgctctg accgtgccgt gtgtgctcag gtgatgagaa 232320
atcggtgcct ctctagtcct agttgagagc cgctgaagtt ccccttggca aacttaggca 232380
tcatcatttg gaacaggaga gtctcaggct atccacagga aaggacaagg cccaactgag 232440
cggctgcctg tgaagctcac agcagtcctg gccccgccac gtgtgactcc aggtgagcca 232500
gggaccagga gccctggctg ggagaaccac ccacctccgc cagccacacc tggggccatc 232560
cccggcctct cctgatggcc cgtcctgggt ggcacctctc tccaggccag cactgcacac 232620
gtgccattca actcagaacc ccaaatgact cagaggcgag ccccatccca gatgaaaacc 232680
aaggtcgagg aaggatgtgt gcgctctgcc accctggggt gccaccatcc ttgcagcggg 232740
caggggagga gaacgtgctt ccaacactgc ctcctaggac ccactacggg agtgggaaac 232800
cagccgggag gaggcgctgc cagggtccct ctgctcatga tcaactccac tccccgagtg 232860
gggctgcacg gccccgccca ggcccaggag tccgaggctg cttctccctc ccatctccac 232920
tccgagtggg gctgcacggc cccgcccagg cccaggagtc cgaggctgct tctccctccc 232980
atctccactc cgagtggggc tgcacaggcc ccgcccaggc ccaggagtcc gaggctgctt 233040
ctccctccca tgttccttgg tgggggtttt tgctgacggt ggcagaccct ccaacggccc 233100
tgatgtgatg aaacatggtt cccggtaatt tggagtgggg ttttaagaga attctgaatt 233160
actacaatgg aaatggcagc actctcgtac cctaaaaaaa gtacaagttc ctcggagtct 233220
gtttgaaatg gagggttcta aaataatcag tgaggcccac aggcgatctc tccttgagcg 233280
ctgtaaaatc tgttctcttc ctcctaagtc cccaaaagta gccagtattg gcagcagtcc 233340

```
ggagcacagg tgctgggctt ggaggtggga atgcattccc ctggctcggc gcctactcag 233400
ctttttgtat tgttcagagc tgcctgtggt gggggttgag tggagctttg cgggacagag 233460
actgccgggc ccatttctac cctcggacac atgctttggg agggagagga tgggaacgtg 233520
agggaacggg ccgcccagct gaatcttcag ggtgtcagcg gagtgtgaac aaagtggctc 233580
ggaggtcaga gccaggcaca ctctgatggg cccggccgtg gctgtgttcc cctctgggct 233640
ctctggagag gcgagttccc agccatgccc cgacctgctg cagcaggagc ctgggggtgg 233700
ggccggtgga ccgaggctga tgtggcttcc aagtggctcc tgcagctcag gtctggcagc 233760
cgcccggtgg aggggaggct ctagctccac tggacagagc tttgcaggag gaagcactag 233820
ggaggcattc acgtgggggt aaatccaggt gggaggacct ggttgcaagg acaagtgtgg 233880
ggggaatggg acctgaggga tggaaggtgc tgagggaggg agatggggag ggcgtgtgag 233940
ctgacgacac cgagggaggg agaggggggag ggcgtgtgag ccgacagcac tgccggccat 234000
gcaagcaggg gacctgggac catgggaagc gaggacattc cctcttccta actgcatgca 234060
cgacaactgt gtaagcagca caagtcacct tacaagatta aatgtgcaac caccactcca 234120
ctctgccaga tagagtaagg gcaccggtaa gtatcacaca ggcagatgag caaaggtagg 234180
cgtggaggcc gccgcacaga tgtgaagttg gcccaggcca gcgccccgct ggctcattta 234240
gggcacagga aacataggac ctggggtgac caacggctgc aggcaggtct ggaagaacca 234300
gtcatgcatc ttcccaacgt caaagagggt gaaatgccat cagagggaaa caaggggcgc 234360
acagcacaag gccaagcacg cttcaccaac atgcatgaag ctggttcccg cacttctgca 234420
agaatgcctt ttcctaacgc tgttgcctat tttaaatata cagagagcag cactctggga 234480
gacaccccctg actcggcgat gggggaagga gctgctcact tactcggcca tgactcgccg 234540
cacgttgctg gcatacagcg cggggttcct cttctcctcc tcagaagggc tgtacacagg 234600
aaggaactga gcacacagag aagctgcgtt agtatcacaa gacgctgacc tcagcaacac 234660
cactgtaacg acaactggga ggctgacaaa attaagatct gtcaaagtaa cgcccattag 234720
aaccgtcttc aaagttgtca tgtgcacgtg tgtgtacgca cacacacacc cccatgatgc 234780
atgaactgca cacgtgtgca cctgaacact caccctcatt ggtgctccct gggagcacct 234840
gctgcctaca tcagaacatc tggctctctg atctacactc acccccgtca gtgctcctgg 234900
gagcgcctgc tgcctacatc agaacatctg gctctctgat ctacactcac ccccgtcagt 234960
gctcctggga gcgcctgctg cctacatcag aacatctggc tctctgatct acactcaccc 235020
ccgtcagtgc tcctgggagc gcctgctgcc tacatcagaa catctggctc tctgatctac 235080
actcaccccc gtcagtgctc ctaggagctc ctgctgccta catcagaaca tccagctccc 235140
tgatctcaca gaggggctta cgcccatttc tgacagcatc agttttgtaa tggggataat 235200
gcagcacgtt attcctacat ttggaacaga ttattggcag cgttttgcca cctcttgcat 235260
tttgaaacta aaagaaattg tccagtgaat ttgtccttgc ctgagtgaca cttacacagc 235320
attttaaatg aatatgaccc acattgcaca ccgtgcagga aagggcacaa gactaccctg 235380
gatgtccttt tctctaatac ggaatatttc agaatacagg aacatcctga aaccccaatt 235440
ctcgctgtta tttctaaaac ttccgtaaac tgccatggtt ccttgcatca acatttgcat 235500
cttgtatgaa caacgtcagt aaggacagag cagttttgca cagccattca ctttcttttc 235560
aactcttaaa attagaaacc aggcctgtaa cacagccatt caacaccagg gagccctcag 235620
gagctcaagc tcagtccctg tgttctgtgc tcgaggcctg gcctgcaccc tcccggccac 235680
tgcccatcca gccacataat cctgctgggt gggggaactg ccgccctcag gcccgggcca 235740
aggcccttca gcccagtcct ctgtaatgag agcagcttat cctctatgga agtgaacagc 235800
cggctgaaa gccgggctta tcagtatatt tcgtagtgct ctccaatatt tactggaagg 235860
cagcaaattg ttttattaaa tatttaagtt gcatgtgact catcttttat ttccagatgc 235920
aagaagtgca tattaattcc ccttttaaac aaaaaaagaa ctactcctca tcgacttaga 235980
aaaaattgtg caggctacac agaggtgact atttagccca aggtcttgac tctggagacc 236040
tgggtcaggc tgagaagggg gatctttgac tatgtattaa acagagcatg gtgctatgac 236100
aaactcagcg gtattaaata tgtaaagtaa aactaagagc caaacaaata attatttgca 236160
tgtgggccag cttcatatgt ccttaagcta caatacccat aataatgaat ggtaggttta 236220
aaaaatcctt tccagctggg tgtggtagtt cacgcctgtg atccccgcac tttaggaggc 236280
caaggcggga ggactgcttg agcccaggag tttgagacca gcctaggcaa caaagtgaga 236340
cccatctcta caaaaattag ctaggcatgg cgtgtgcctg tggtccaatt acgtgggagg 236400
ctgaggcagg gagatcactt gggcccagga ggtcgaggct gcagtgagcc catcgctaca 236460
ctccagcctg ggcaacagag caagacaaaa tgctgtctca gaaaaacaaa aaacaaaaac 236520
ccaaatctta tccaagtggt tcagactcat aagagagaca taaagtcatt gaggtaaagc 236580
aaccagaggc tcctcgaagg agccctgaga aacggaaaga tgggaaagca agacaggtga 236640
tatgccacat tgtacaaggc ttatctgggc atcctggtgt aagctcgtgt ctgcatcaac 236700
cactgtgaag agcgctgtgt aactctgtat ccatacgaac ctcgatttcc acttggttgt 236760
gaaactgaca cagcgtgagc cacaggattt ccagccttaa aaacagattg cacaatgttg 236820
agcagattta caactcgggt taacaagtaa attactccca attctggggt gaaacctcca 236880
gacgcgccct ccagagggcg ctactgggca gtcaacgctc ccacggaggg ggtgccgtgc 236940
ccacccagcc cgagccccag tgtgacgtac agccgtgggt ggaaatgctg cttttccgat 237000
tcaacacctg cttatgtcct tggttcgtct agcaagacac catctcctgc ttgttctcca 237060
gctcaggaga cctcgtacag cccacacctg tccctccaca cctgtccctc ctcgctgtcc 237120
```

ccaggcagca caggccctga gtcatgccac ccctccagcc tggatccaga gttcccaacc 237180
tcagaaccag gatcaccatg cctttgccgc aggagttgac atgggagaca tttggagctg 237240
ctcccagggc cacactcacg cctccgacag cccagtgccc caaccctctt tctgccctgt 237300
catgccagcc ccagccctag gcccccggga ccccagaacc cctatacccc ccagagcccc 237360
ctcccagctc tgctcccagc tgggagcctc cacacgccag cctgggaagc gctgacctca 237420
acaccttcac ctgaaagcac cagcggaccc acatcctccc aaacgcctgg aatggagctg 237480
ctctctcttg gactttcccg ctccctctgc cctcccgacg tcagctcaga cgtcagcacc 237540
caggaggccc tgaccagcct gtggccccgg gcttctcctc tggggctcgt tcccgtttcc 237600
gtggggttgt tcattgttgc ataactgacc ttcggtgtct ctgctggggg gacagggaca 237660
ctgactcaca gcaggggctg gcctcccacc cagcactact cagtctctgt gcctgtgcag 237720
acggggtccc cccagacacc cctaaatctc cactttcctt accaggggcc accaggtgga 237780
cgatcgtaat tactgagctg gcaaactcga gagccgaatg caagactcac agttcccttc 237840
ctgctggtac tgaagacgtg caacttaaga ccccagaatc caggagtgtc aggcctgggc 237900
cacatctgta cgtttagttt tcacaatggg ctgaacctaa cggctgtccc acacctgctt 237960
tcacaactgc aaaagtaact agcgtgcaca gcagacccca agcagcccct acgtgttcat 238020
ggaacaacag gacaaagagg acgacacggc gttcaactta cgctccaggt ccttgccacg 238080
tccatgtgat ggtgtcctgg ggaggaagag gcagggtcag tcagcatggg gcctgcaccc 238140
agggcctgca ccaagcacct gcgtgtgccg gcctctccct gcacctccca ccccacagag 238200
gcgctgcagc caggggggaag ggaggagggt gctagagctc cagcttccca gagtccctga 238260
ggatccagga ctcggaccag ccccccagcc tgaggtcccc agagcctcta aggacccaga 238320
gaaccaccac cttacaggtc cccagagttc ccccaagatc cccagagtcc ctgaggactc 238380
agagccccct cttataggtt cccagagccc ctagaacctg ggaaaagggt cctccctccc 238440
tccatgctct ccgctttcct cacagacctg ggacaccaa ttccagtgtg cacccgggac 238500
cccggccctc tcctgcccac cgctctctcc aactgctctg agtgttcccc caaccacggt 238560
gcatgtgcag ggccccccca ccccgggtgc tctgaagtcc catcttcagc ctcagtgccg 238620
ccgggcaggg accacacagc aggggccgtg acggcagagg cccagacacc gtcaccctgg 238680
ggtggacctt ctgctcccct gacggctaag ctagtgcttc ttggtgcatc cagtaatagt 238740
gtgttgcttt aaacacactg atgaaacaat cttaaaagtg gaaaatctag atgactgcac 238800
agatcggagt ctgttgtcct cgggttgact tttttgccca caggagttgc actggggcat 238860
ggtttccgca gcggaggcag gaaggggccc ttggccagga gcctggctga caaacgcacg 238920
cctcagtaag aaggaagagg tctttcagtc gtgtgactac cggccctgac tccattttat 238980
tacctgaccg tcagctctcc gggcaaagtg aagcccggca ggcatcggac gggacaggcc 239040
ctgaggcgga tggcccagaa ccctggcacc cagaggtcac ctgtatttct gccccactat 239100
gtcctgaccc acgggggttt tctttcagtg ctttcctttg ccatcacaaa aaggaactgc 239160
tatagacggg aagctcgctg tgatgggcag gccctcctcg gtgctctccc aggtgggtgc 239220
tccaccagga acctgcagtg agcccagcac cagtgggctg tggggacctt gagtccacct 239280
gggccgtctt tatgctccag gagcccatga aggggcaagg acgtgcggga gtcattgctg 239340
tggaaggggg aacacagctg gcaccaaatg aatgccctga gctcggcggc aggcctgtga 239400
ggcaaggtcg cctaagtggg gagggccgat ggaaccggaa gtattctgag catccaagag 239460
cacctggagg aaggtgcagc agccacctta caggtgtgga aactgaggca ggacatgggc 239520
aggggcgggg ccagggggtg gggctgcggc caggggcagg gatggagcag ggcctgggtc 239580
agggtggggt ggggcggggc agagccaggg caggctgggg tggggtgggg tgggctgggg 239640
tggggtgggg tggggcagag ccagggcagg gtggggtggg gtagggtggg gcagagccag 239700
ggcagggtgg ggtggggtag ggtggggcag agccagggca ggctggggtg ggctggggtg 239760
gggcagagcc ggggtgggct gggatggggg gagtagggtg gggcaggggg gtggggtgtg 239820
atgggccagg gcaggctggg gtggggtggg gtggggtgtg gtgtggtggg ctggggcaga 239880
gccagggcag gctaagcctc ctccagaggt ggcaaggccc catggggaac tgctctgagg 239940
cctcgtccag actttctccc aaaggtgggg tctaccacct tcgccccagg agctctggac 240000
cccaaacacc ttcttaattc tcttaaacca aacaaaacct gggcactaaa tgataaaacc 240060
acagaaatga caaagtcacc tcctttctct ccagctgcct ccgccttcca gaacttaaat 240120
gttcttaatt tttaaacttc ctggagcatg aactgggtac tgaaagctgc tggggggccct 240180
ggccggtgat gtggggtgga ggggttccct ctccaaaatg tcaggtttgg cacacagagg 240240
aaagggcagc tttgtcagga gctatggaag cagggctctg gaacactggg gaccactgtg 240300
gaatcatggg gttgatcagg gacacgtgca tagaacaggc cgcactcagg aacgtgccga 240360
gcccagggcc cggcctgtc ctgccctctc cagcgctgag gccggatgga ctcagcatgg 240420
ccaagtgtgg gctgtggcgc agataaaggg tgtggagaga cagagacaca ggtgcacaga 240480
ggcagctcgc agttcccgtt tcccggagaa ttcccctgga agctgacccc aaaaaaacac 240540
aactcaccag tttatttgga tatcgtaaaa ccacaggctg gacgggcgct ccagggatga 240600
atgcacctgc cgagaaagga acagcggtgt tgcccatggc agcccacgca ggacagcgtc 240660
tgctgcgttt ctcatgctgc ccttgggata aaagtgagct ttctgtcta ggccttcctg 240720
gtcagcaagg gcactccatg cctggactat ctcaacatct gtcctgaccg taaacaccca 240780
gcgccacagc acggatgggc aggaacatcg gccagggggcg ctccccggcc aaggaacact 240840
ttctgtttta acatcgcgca cgagctggaa ggcgtctgtg gagggacact tgctattata 240900

```
acattgcgca cgagctggaa gacatccgtg gagggacact ttctgctgta acatcgcgca     240960
ccagctggaa ggcgtctgtg gagggacact tgctattata acattgcgca cgagctggaa     241020
gacatccgtg gagggacact ttctgctgta acatcgcgca ccagctggaa ggcgttggtg     241080
ggaagtgggg gtcctcatca ccggccaatg ctcacccacc tgacggggtt agggtctgcc     241140
ctcaccaagc acatacacct gcctcggggc cccacccgcc gccctcacct gcccctgggg     241200
ctcagctcca aggacgggcg gatttggtca cagctgcagc aggacttgcc cgggatctgc     241260
ctctctctct gagtggctgc cacctgatgg gctcccaagg aatcaggagg agccccagcg     241320
aggcccagct agagccacag gccgcagagg ctcaggaact tccccgttcc cactggaatt     241380
gtggctgtac ccagcagccg ctccgccttc tcactggccg gagttgtgcc cttgcgggct     241440
ggagcaaggc accatggact ctgtggatga ttgtttctat tttaaattct atttaaatct     241500
ctattgaaag agttttttt taactggaaa actcaggcat cagaagtact caagttttag      241560
aacaggacag gcggtctgag aagggctccc ctgcctggcg acctgaggcc aggcgagtga     241620
atccccaggc agcgcgttcc tcccaggctg ccagcgagtg ccactatgtg gtcaggaatt     241680
tgcggttggt cctggctgcg ggattctgca caattctggg agaatggagc ccatgcttgc     241740
ccctagggct gccaggagca tctgtcccta gggccaattc tcccttcacg gtggcctgag     241800
gccagaggcc cgtgtcaggg ctgaaacctc gagctgaccg cccaacccac tcagctttcg     241860
tctcatgcag gacatccacc tgcagccttg gtgtccagct ggcaccccaa gtccctgggc     241920
ccgtgtgccg cagcttcccc tgtgtccagg gctgggttgc cccccttcctg ctgtgggctc    241980
agcagcacct gggagcaagc tcatgcagtt cacccagcac ttctgtgtgc ccaggtggac     242040
gggtggctgt gtcaggggag gcggaggacg gcagggctgg cacagaccca ggctcaagcc     242100
ccagctccaa cgcccggcag ccgagtccca gcccctgaga cacgcagtgt ggaggaaata     242160
gtcctcattg cctgggaccg agcccaggag ctcgcgtgga gtgccgagac ctgctgtggg     242220
tgataatggc tctgctgcta cagctcagcc gaccatcccg accatgtgtc aaggggacat     242280
cactgaaact cagtaagaat caaaggaaac ctcaacaccc tggggacctc tcaggaaagt     242340
ccccaagagc acagaaaccc cccatgagcc tgaaaggtca gtgacttcat ggaaaccccg     242400
gtgggaaagt ctgcaggggt ctctggcctc cctagtctcc caggggcccc tgagggtcag     242460
cagcacccag accccgccag cactgtgcac ccaaggcagg ctgcacaagg gccaggctgg     242520
gagaaaccaa gatgaggcag tcagagccca gggcttctga atgcaggaac aaggcagagg     242580
ggaggaggta cggcttcta aactgcacag aaacctccat attcacacat agcttgtgct      242640
gggcatgggt ctaacgtcac tagttgcaac aatgaggctg gaagatggct caacagggac     242700
ccctgaggcc cccagaaggc catcacccct tattcaccct gaccatcccc cacagcccac     242760
cccacaggaa gaaacagtcc accatggccc agggagagcc gtgtccacat ctgctcacag     242820
cctgtgcagg gaggccgctg cggcccaggg acagccacat ccatgtttgc tcacagcccg     242880
tgcagggagg ccgggggagag ccacatccat gtctgctcac agcccgtgca gggaggccag    242940
ggacagccac atccatgtct gttcacagcc cgtgcaggga ggccactgtg gcccagtgag     243000
agctgcgtcc acttccaaca gccacatttg gtaggcccgc ctctgcatcc tcgggctgtc     243060
agtgccatcg ctgggcccac gcagcatcct agggccttct cactgtcttc gttctgctct     243120
gagagcttgg agctggcagg caggtggggt gtgggcatgt ggagggcgtg ggcccaggac     243180
cttccaggag gggtctccag gtacccagtc agccgatcct gctctagtgc cctctgacaa     243240
ggagggtgag tgtgggcact ggagaggaag gccccacatg ggcctgtttc aggagcaact     243300
caatccctgc agagccctga gggtcccagc cctgctggaa cccaccacat ccagcagacc     243360
ccacagggct ctgctgagct ctatcacccc cacctcagag gcggcacccc tggcctgggt     243420
cagaacaggg ggactcaggt ggacagtgca gcacagacaa gagacactca ggagaaagaa     243480
ggcacctggg cccaggagga ccctgctgga ggctccagga gcctgaggct ggaggctggg     243540
cccaatgcgg gaaggtctcc ggggatgaag gcggggagtc agcagtggtg cccaaacggg     243600
tgaccccagc aacgtgaccc caacatcagg cgatgtgatg aaaggtggag ggagggtctg     243660
agtgggtggg ccgccatgga gctggggcag ctggagccca aggcagcagg caggttgtgg     243720
gtgcctctgt tgtcaggctc aaggcgggag acgctcacgg ctctggaggg ctccaccacg     243780
aggcgtcagg atcacgcacc acacaccaat ttcacctcca aacaggaggc tccaaacctc     243840
actaagaagt acccagctaa gacgcatgag ggcctgagag attccgaaca cgtgactcga     243900
ggcagccgga aacccgggcc tgcaggtgac acgcagaact catggctgga cccactggaa     243960
atggagacgg ggaaaggatc aagccaccgt gtcatctccc cagaccccgt ggaacccacc     244020
tggggcacag cgagcgccct tcctggactg cacacagtga gctcctactg ggcaccgcca     244080
tgagtcccct cagtttcgtg atatgcgttg agaggtgctc acacgccctc ctcacgtcta     244140
cacaagggcc gccagctcca aagggcaagg cgatggccct ccccaggggc ctccctgcag     244200
gcacgaggct ccactcccga ggaacacctg cccttgaaga cagggcccag cagtgacccc     244260
agccccacac cttgtcaagg aacaagacct gacgtacttg ccagctgtct tctgcctggg     244320
gccttggaaa cacggcccac ttttggcctt caccaaggtt gcccacaggc cacgcccagg     244380
cacggaccca gtgtggtggg ggcacacgga ttcgtggtca ccgcgtgccg cctccacccct    244440
ctgcatctct ggttctgatc agaatgactc agaaccacgc gtggtgaaga caatggaacg     244500
ggaagaccca gtaccttcca gtgagctgca ccttgaagcc tctggaagca aaacctgcca     244560
tctgcctgct gcaccaagga gggtggaggg tgaaagcacg cgcatctgag ctggaggagg     244620
atggccacag gctcggaaac cccggcggca cacgtaggtg agtgactgca cactttctcg     244680
```

```
acccagctcc cataaatgtc cagacactca ggttgtgcgg ccccagaggc acggcagccg     244740
gtgccagtgt gagcgaggaa ctggcttttc cacgtgactc tgatccaacc acagagaaca     244800
ggtctgcatg ccccagtatg gacacagttg atgaaagcca ctactttcta aagtagccca     244860
agggagacga caggatccag gtggaccctc tgacacgccc aagggtgtgc agaattccaa     244920
acagactcag cgcaaaaaga acagtggact ctgctgcctg ctcactgcca cacttcccac     244980
gtcaggggtg cagacacaat actagaccca ggtaaccgcc gcgggagaga cccaagttca     245040
cacgcatttg agtccccccct ccccagggag aaagaacact gtctaactgg ggacctgggg     245100
acctgggcat ctgaggatgg ctcgcaggga tttgcacctg ggattcccag gcgggtgtgg     245160
ggcgtgagct gcaggccaag aagggtaccc tgagccattc aaaggcagtg agaccccttc     245220
tgcacccagg accctccagc ctggacctca aaacagaggc aaacatctat ctccacctca     245280
ctggaaacgc cctgaaaccg taacttctcc atcttcctgc aaacatgata atccttgact     245340
cacattctca aatttatttt gtgacttttc ttaggaatac ctctaagaac cccagcacac     245400
aggataaaaa cagaaactgt tcttatgtgc acagcacatt attaaaaaac atctctttaa     245460
tattttcctt ttctctagga gaaaaataaa catttaagaa aactacatac caggtttgaa     245520
ggtaattagg caggtcctgt ttgtacaagt tccttctgga aaaatcatta tctggaagtg     245580
ggagaaagaa aaggatcagc attaaactgt acataattat aattcagaac ttacagaagc     245640
caatcttcac aatatatttt cttttctgct gacataattt ggacactggg atcatattca     245700
gtacgtaaat tattaaaatg cattatggaa gcacacactg atttccgctc tgctggtttt     245760
attaaagtgt ggcagcagta tcaacacata aacagcatct gtcttcttca actacagctc     245820
ctggaaagca gggtagtcgc ggtgcccacc tctcagacac tgtgagaacc aggtgcaact     245880
aaatataacc cacggttggc acaacataag cacctcctgg gggcagcact cattctcatc     245940
aggaagaagg tggggaggag agcaaagacc cacaaatggc agtgcctggc tgatggagcc     246000
gaaggagggc tgggacccca gcaacgggct ggatgagcag gagagccgag ccccagtcag     246060
gcagcaccct ccacaccggg gtttcaattc ctctgactca cactggcctg cagtagcaaa     246120
aactgccctc aaactcccag ccccgcaggc tgctttcagt tctgggcccc gtgaggaaga     246180
cagacctcag gggggccggc gtgggctcac cctggcaagc ccagggccag gcaggccaaa     246240
tgggtggggg aggagactgc ctgtgcttgt gggggcagag ctggggcacc ctggaagtca     246300
cagccctcta aggggctgcc cccagccaga ccctgcaaag cttcgtgagc aatcagatcc     246360
cagacagttc tgtaaaattt ccagcttcta atgatggctc agctggaaag aagaaaaaca     246420
gcgtccaagc taatgtcagc caggctgccc ctacagctgg gttcagggct tctgaatgcc     246480
tgggaaccag catacagacc aggaagttta ctccaggact tttagaacca cccaacacac     246540
agcaggctga cacatcaact ccaaaatcac actgcaccag gagggcgggt gagcaatgct     246600
ctatctgagt cagaacttag ttcagaaaag atgattcagg aagctgagat gcatttgaga     246660
attgtttttc tttactttaa ctttaaattt cgggcaccga aaccaaatgc acctgacagc     246720
accctcctga gttcacgcac tcactaggag aagagaatca gaatccagcc ccggaggaag     246780
ggccccagtt tctttctccc cactcgcgaa gttcgcatct ttcggaataa aaccactgaa     246840
acacaatcag ggctacgtgc attacctgtg gccactttcc gttggactgc gcccgtctct     246900
tgatttcttc tactgttttc ctgcgagaat cctggtctga ccgggacacg aacacaggcc     246960
gtatatactg gatcagagct ggaagagagg aggggagacg gatcacgtgg aatgcacggc     247020
tcccgccagg cagggctgag gaacgagggg gctgctgcat ggcgcccagt ggggcgggag     247080
actccagatg ccccaggggc tgtgccatgg gtgggctgtg agtccctgac tgagggaacg     247140
ggaacagcac ccgggcttcc catgtctgtg acagcaggaa gccctgtagc ctcagtgcca     247200
caagtaacaa acagcaagtc catggcaccc actgaggaca cggagcacag aacggaggtc     247260
aatgaaagac aaccgccagg aagcagaaag tctttagacc cagtcttcac agaggacagg     247320
gttgaaggtg ccaggtccct agattggacc ccgccttccc cctgtgggtt gaactgcatc     247380
ccctaaaaga caagctgaag tcctcactcc tggtaccggg aatgggacct catttcgaaa     247440
caggtggtag cagatgctca aggtcccgga tgagggtccc tatgggaaga ggagacaaag     247500
cagatgctca agctaaaatc ccagatgagg gtccctatgg gaagaggaga cgaagagggg     247560
gagagaaggc caatgatgaa ggaggccata ggaggatgtg gccacagccc ccaccacgag     247620
ccgggagggc cgagggagct ttcagaagag cccaatgcgg ccagcacttt gttctggcac     247680
ttctgctttt agacggcggg agaatatgct cctgttgttc taagccacca gctttgtggt     247740
gctttgtagc acagccccag tttaaccatg gcaccccaca gcctcctgga agttgcgggg     247800
ttggggtaca agggacaccc cgccatgaag gctggtgctc agccaaggca acgtggccac     247860
ggcacctgtc tttatgcaag aggattgaac gtggaaacat acttcttaga agagaaaact     247920
ctgaagactt taaaatgaaa ctatccctga gtggagcttc ctttttgagg acggtgagca     247980
ggatccacac tcggcgcccc ccaggccaac tgaaggtgtc tctgacccca ccacgggctc     248040
ctaggctgct gcaggtcagc cccacctccc caccccagtt cagtttccag tttcttttct     248100
tagcgttttt attacttgta aggagtagca tttcatgact tttattcttt cttggaggtt     248160
ctctcctcca agaacgagaa aattgaagcc aaaaaaagaa aattccttct atcctgtggt     248220
tgtctgttgg gtctctgtgg tctttcctat gtcttgaatc attttgcttt ttcacgattc     248280
cagtactatt tcatccttgc tcattggtta ttcccaactc tgctaaaaag gacgatatcg     248340
gaagggaaga atggcggagt cgtctcgcag gatggcgcac gcgtcaaaga aatggccctg     248400
attgcgtggt ctttcggtgc ccactgtttt ggccaaagcg tcccatggtc gtcccggggc     248460
```

acacggggtc tggagatgcc accctgccac cttcgcaatc ctgtcagctg gtttgttttg 248520
tgaacagagt agagatttca gaagttgtct ataacggcaa agaacagaac agtgacgtgg 248580
gggcgtttca cactcagatg ggtccggggg taaatgtggg gggtgcaggg ggaggactct 248640
gagtgaagtc ttgggccgtg cgtcttcatg tgggttgccc ggtgtgcctt tcacgctgag 248700
aactgggcag tgaggacagc aaggagatgc agctcctccc cgggagccct gaggagggaa 248760
gggtgtcacc acgccgtgtg cacagccaca ctgggactgg cgcattttgc cagcaagaag 248820
gtgacagcgt tctgccgtct tttgggcatt tgaggctcaa actttccttg acaaggtttg 248880
taaatggatg gtgcggatat gaaggtcact gaaaggaaac taagaatgtg aaaaggaaaa 248940
aatctacctg gttgatcact ttcattggta ttcatacatc taaaaatgaa aatattttga 249000
acattttcaa cattcatctc ttcaacaaag ttgtgagctg tcaaagggtc cacgaaagga 249060
accagaagtc accacaagct gggaccgggg agatgtctct gagctggaaa cctgggagac 249120
gtctctgagc tgggaccagg ggagatgtct ctgagctgga aaccagggag atgtctctga 249180
gctgggacca tgggagatgt ctctgagctg gaaaccaggg agatgtctct gagctgggac 249240
caggggagat tatctctgag ctgggaccac gggagatgtc tctgagctgg aaacctggga 249300
gatgtctctg agctgggatg ggggaagatt atctctgagc tgggacaagg ggagatgtct 249360
ctgagctgga gcctggggag atgtctctga gctggggcct ggggagatgt ctctgagctg 249420
gggcctgggg agacgtctct gagctggggc ctggggagac gtctctgagc tgggacctgg 249480
ggagatggtt ttgagctgga gcctggggag atggttctga gctcccgaat cagagggtgc 249540
tccaggtgcg cagtagagga gtcatcagcc acgccccagg acaatggccc tttcaggcca 249600
ggacagcagg gagcaaaatg tggttcctgc taagtctgaa ttctaacgaa aatttctgat 249660
aagcttgtca ctatcctctc cctcgcaggt ccgtggatta tctctgaaat gacaagaaac 249720
acgcagggct ccacgagggc cctgcacaca gcagcatggc tgtggccatt cttcctgctg 249780
cgccgagatg aaggtcagtg ctgggtcacc tgggttgatc tggaagaggt ggaatcccgg 249840
gcctctagaa agataggact acaacgtcca gcatcaccgc accaaacact ccttcgtgtt 249900
ggtgatctcc acagggaggc accaagggca cagggagagc ccggccaacg ctcagattca 249960
ctctgggtgg gccagccccc ctgtccaagg acccctgcag ctgcgagggt gcctgggcac 250020
acagctccac aatccccctc cctgagcaga tgacgaacga ccatgggcct ttgccacagt 250080
gggggggcaa atgtggtttc ctgggtatgc cagaaccaca ggatgcaaag ggtgctgagt 250140
ggagctgggg ctgccccatg agcctgtgga gcttgcagag cggggaaacc tgcgtgtgag 250200
agccaaggcc atcactcgga tggtgcctgc agtccagtct cgcctccagt gccggcctgt 250260
ccagcctcct ccaggcttcc ccagccagcc gagtgcagtg agccctccag tcccaccaag 250320
gaccatcccc gatttccaag cctgacccag gcagactcag gccacccaca actgcccagc 250380
tgcaggcaca gtctggacac cttccggctg ctgcaggccc aatcccccat gggccatgcc 250440
agggttcctc accccacttc cagccttcct atcccagctg tggccaccac cctcagtggt 250500
cagcaccaca gaatgtgtcc ccacccagtc catactgaat gtgtcccccc agatattttc 250560
tagaaaactg tcatatgaat gctaacgtta ggtgctgcat ggaaacagag ccggcaggag 250620
tggccaggtg agggccccgg ccataggcga cgcccctcgt cccagcagct ccttccctca 250680
gcaccgccca ctgccaaccc aggacccgct gtgccccaga acgtgctgtg ctgtgctgat 250740
ggcggctgtg aaggaagcac gaggacagaa cgggcccaga tgccctgaag agcaccgagg 250800
acagagctct ggtgggtcag ggctgcgcac aggtgggtga ggggccacgc agggctccct 250860
acagtcctcc cagagcagtg ggtccatggg atgcggcccc caaagatggg tccacatgct 250920
gaccctggaa cctgtgaatg aggccttatt tggaaaaagg tttttacaga ggtaactcgt 250980
taaggatctc aagatgagag catcctggat cacccaatga ctggtcctta taagagactc 251040
atagaggaga cacttggagg gagccaggcc agtcgaccac tgaggcagag tcagagggac 251100
gcagccccca gaggaggaag aggcaggaag gggccgcaca gggccttcgg cccagagacg 251160
ccttgatttt gaacatctgg tccccagatg acgagagggt gaatctctgc tggagtgagc 251220
ccccggcttt ggggaggggt catggcagca ccaggtgctc aggtccctgg ttcacggcac 251280
agccgcggtg catgggcagc cctgccgacc tgctcccaaa gacttccaga actgccatct 251340
gataaaggtg agccacccctt gccaagcaga cctggagcaa gcactgtagc ctgttatgat 251400
agtgagggca ctcaggtagc aataaaaata acaattacaa caacgaaaat tacacaggaa 251460
accccaacac agaaaatggg gcacaaggcc aatgtgcccg gcggtggggg ctcgatcact 251520
cccagcagcg tggtgggggg ggggtccctc actcccggta gggagggtct cactcatttc 251580
cagcaagtgg gggtccctca ttcctagcag ggagggatct ctcattccca gaaggggggg 251640
tccctaaaaa ccagcaggga gtccctcatt gccagcaaca gagggggtct cactcattcc 251700
caacagaggg gggaccctca ctcccagcag gaggggaatc tcttattctc agcagcaggg 251760
ggatctctca ctcccagcag ggcagggtcc ctcactccca gcaggggtgt ctcactcact 251820
tccccagatc gggatgtctc tgctctctgc cttcatcacg atggaggaca tcgtcatggt 251880
cacagggatg gcgtcgaagt aggacgagtg aggcgcgagc gtgaggatgg ccgcctcggt 251940
gggcagcgcc tgccgcccct tcacggccac ccggtggaag ccgccggcga accacatggt 252000
gcgcatgatg gccttcagca ggaagtccac aaccctgcaa aagagggcgc tgcgtcacgc 252060
gggcacacgt ccgcagtctc ggagtctgtg tgaggcacag gggcggtccc acgggagagc 252120
cctccagggc gcagtccagg ccacgggctt cctgtggtcg ccgccgctgg gacatctgct 252180
tgagggaaga aaagacgccg cgccttcctg gcctccgcct gtgtcctcgc tggctgcgct 252240

ctcacctacg aaggaggccg cggggccctg tgtggtggtc acgggccacg cggcaggcag 252300
tgctaagaca acatgagact ccggaacaca gacagcacaa gtcccaggcc agaagacctc 252360
cggaccttca tgttagcaac ttatttatca gcatcctatc attttgaaat gggaaaaaca 252420
ttaaaacacc taaaacgcat atcgcaatat gggggggggg gcgctcagag ctgagggcgg 252480
aagctgagac ctgcgtgcga acttccccat ctcatctcca caggaagccc ggggttccca 252540
caaccaggcg ttgtgttatt acagtgagct gtttctggag gaaattagat ggccaataaa 252600
tttggaaaat aaagtaaaaa tcagcctagg ttaactgaat ttgtgtactc tttatctctt 252660
tttaaaatag gtacaaattt agccttttaa atgtgtgtat tctctttcat atgaggctac 252720
agattaggct gttattccac acctcaggga attgacacgc agcagacagc cacggtgtgt 252780
gagaagccac aggtcaggca cacagagaac atgattcagc ctcatgagaa gccacggctc 252840
actcggccac tgggttaagt gggcgcatca cactgggtaa aactactgca cagagaaaca 252900
agagcctgaa gccttactgg attcacattg ccctgggaca gatgatcctc ccagaagact 252960
tccaggacag gggtccccat ggatctgagc caaggcggtt gctaggaggg cacaaagcct 253020
cctctcgtgg ggccaccttg gaatggcctt tgtgtatgcg atgataaaca atggtgtagt 253080
tacacatgga gatgctgggg accaacccag ggtgctgctt gtctagagga gggggattta 253140
cacaggtgtg cacttcttca ccacctgttc agctgcatat ttaaaaatgt acttttctgg 253200
ccaggcatgg tggctcacgc ctgtaatcac agcactttgg gaggccaagg caggtggatc 253260
acttgaggtc aggagctcaa gaccagcctg gccaacatgg tgaaaccctg tctctactaa 253320
aaatacaaaa attagctggg catggtggca ggcgtctgta atcccagcca ctcaggaggc 253380
tgaggcagga gaactgcttg aacccaaggg gcggaggttg cagtgagccg agattgcgcc 253440
actgcactcc agcctgggca acagagtgag actccatctc aagaaaacta aactaaaaaa 253500
taaagatgta ctcttctgtg cacatgttat tttccacaaa aaaaaaaaaa aagtacaaaa 253560
acaaaagcca aaagacagca attgatgcct aagatggaag aaacaaggca gcggcggagg 253620
gagaagcgag gctgcagagg aggctgcggc gggcacagga gccagtctcg ggcaggttca 253680
ggggcaggag agaggacagc aggaaaaaag ggattaaaac ccgggcggcc cttagaggaa 253740
cacacctgcc agccccgaaa tcgaattgca gggacagacg gtgctcaggg gaactgtact 253800
gggtgtcggg gcagggaaaa agcagcctca gctcttctct aaagatcttg gaggaatttt 253860
atacccaaac tatccacatg tgaaaacccg gactgctggt gtggatgaca gcctcagaga 253920
aaagctccgc ttgctgtggg gttggggacc cagcacccac tcaccccaag gcaaagggag 253980
ccagcacaag cccagggtgc tcaggtgcct gggcagccca ttcccagggg aatgtgaatc 254040
aggagacaga tctatctgca tacacaggag cggggatctc catcagcacc cccaggaaat 254100
ccactccgtc ctaccgccct cacacggacg acacccaaga atcctgagac gtgtgagcag 254160
agcagctgca tggcagagaa gcagaggact gaccccaagg agacagaaag ggtgggcatc 254220
agaagagggg tgcaaaacat ccaccacgct cgccaagaga gtctggaaga acagggcgct 254280
gaggagcatc aaagaacaag aaaacactct cagaaaccaa gactgtcact gtggagacaa 254340
gcaactcaca ggaaagactg ggaaatgagg aggaggaaat ctcccagaac atgcaggggg 254400
aaaaggagga aggccgctga ggcctgcatc tcctcaagcc agctggcatg tggaggtggg 254460
tgggggaagc ccacacgtct aagcgtgagg ggcaggcgca ggccccggct ctgcaggttc 254520
caaatgttct gctgtgagca gaggcaagag gcagagtgga gccggccctg gagaggacct 254580
atacacatgt acacagcctg agtgctggct acggtgccag gccggtgggg tagggcaaag 254640
gaaggggccc tggggccacg tctggcgggg cttcaaggag gcaagtctgg tgatccacgt 254700
ggactggctt agctgggtca gggacggccc ctgggtggct ggtgggaggg ctgtgttggg 254760
ctggcaggtg tggaggggca gccccaggct gagacgcagg tggcaggagc tgctggcttc 254820
tgggagacca gataaagtgg tttgatgctc aagttcaagc tccaacctcc aggccaacat 254880
acggggagta cctagagtca tcatggggat ggcatcccca aacttgggaa caggtgttcc 254940
cacacagagc attcaggaaa gggcaccatc catctgaacc atccggtcag ccaaggggca 255000
gcccctggag gacctggccc agctcaggac ttggcccgtg gtcaaggatt cagcgccaga 255060
tggtcgggtt ttgcaaaacg atgctacggt ttttacaaca ctgtgtgtgt tgaaagtaac 255120
ctaaaatagt gccttttgcg aaactgtgtc attctttcac tgaaagccta agatttttat 255180
tttaggaagg tttgaatgac actaacttac attcagcaaa aatgctttaa ctcaacatca 255240
atctattcaa aactgcagag cagtgcataa cgtgagccaa cttaagtccc aaaccgacta 255300
cgtaattatt attcagcggt acactctgaa tctgctactt caaattcagt ttcctcctga 255360
tcccttacgt ccctaactac ggtgcttttg tgactaggtt tccccttggg gagttcagaa 255420
ttcagggttt gttctctctt ctgtttccct tcaagcacca agccaagccc aggactgtgc 255480
accaagggag ctttcatcag tgctgctgaa ggagacgcca ggggctccag gggtgggatt 255540
caatgccagg cagcacgggc agcttgagtg cccacaagca tccgtgcata ctcccacaca 255600
tgcacacatt cacacacata cacagacatc gctctcatat atactcacat gcacacacac 255660
actcatacat atgtacacac atatactctc atacacatat gtacatacat catacatatg 255720
tggacacctg tacacacgta cacttgcaca tatacacaca tacatgtaca cagagacaca 255780
acactcatac acatgtacat actgtcatac acgtacatgt atgtgcacac gatacagaca 255840
tccatccaca catatataca tgcactcaca tgtacacaca tgcaacacac acaggtacac 255900
agcacacaca tatacacatg tacacactca tatacatact tgcactggtg gctcacatac 255960
acacagtaat gcaaccacat gcctatgcac gtgtacctac tcccgtgcat gcacatacat 256020

atgtatgcac actcacatgc gcatatgtac acacatacac accctctctg cacacataca 256080
cagaatgata cacagtgagt ctccacagac ccagatgccc aaagatctca tgaagcagag 256140
gtgacagaaa ccccaacgtg tcactgtcac gggacacttc acacgtgcat gagatgtaca 256200
tatgaaagcg tctctgaagt acagctgcta gaataagagt ccgttgtggt ccatttatcg 256260
cctgttgaag ccaagtcaat tattcacaga caagaaaagc caccagggtt agcatgggga 256320
aacggaaaat tgccccaagc catctatttt gaaaagtgag agcctgtgct cgcacggggc 256380
tcctgcctcc tgagacagaa cagaggaggc ggggagctcg gcccaacaag gacatgggga 256440
gtaaagagaa agggtgccag gaaagcacca cgaagccatt gctgcccaca gcaccacatt 256500
cagccaggtc acctgcagcc tgcttcacac tacagaaccc ccactttcta gctgtggctt 256560
ctcaactcag caaaactgaa agcctgactt tgatttggcc ttgatttact ttgcttggtt 256620
atatatttga tacccaattc ctattttatg gttaaaacgt ttcaaaaaca aaccaagata 256680
aagcataaga gaattttaaa aatctagaaa ggctaaatga agaactaaag cccgtgatga 256740
tcctgttgcc cctaaagaac ccaggctgcc ccaggtcacc actacccagc aagctgctca 256800
acgtccaccc actcagcagg gcccagagtg agggaccccc aacgcccagg ctgccctctc 256860
ctatgactgc accatgtctg cacgcaacgg tgcggcaaac ccgggaccct aagtggccgt 256920
cttccccctg cacctaactg catgtctgag tgtggccctg ggtatcaagg agaaccgaag 256980
ctcaggcaga tgtcagctcc catcagcctc ttggccgtga aaactttgct gtacatacag 257040
cccacagatc agacaccttc cagagaaagg aggggcctgg gcgtgcccat tacacctttc 257100
ggaggccagg cagtgaatgc tttaggctct acagccacac ggcctctgtc ccaaccactt 257160
ggcccggttc ccccaccaatg cagccctgaa catcacgcag gccagcgggc gtgcccgtgc 257220
tccagtaaaa ctttacttat agtcaggcag cagcctccaa ttaactacta atattcctgt 257280
ttctgaataa aagattcagg gagcccctcc agaatgatga gagatgttaa caacacatgg 257340
aagggttgtc agagctgtgg ttataggaca cgcgtggtaa cttttaagat actgaaaata 257400
tttatcaaag tatttatgat atctcaacat tttcatttgc taatggcaga tactttcagt 257460
gaaagcacag ctttaggaat ttaaatgaaa caagaaaata ttgtgattta gtttaatttt 257520
ttgtggtctg ttttggatct ttagtttaga tgtattcctc atcgggtagg tgtgctttat 257580
ttatattccg tgcttaaaag ccaggaagag tttaggtcca caaagcaagg gaaagacggc 257640
cctggagggg tggtgccagc aggcagcagg ccaggccaac gttggctgac atgcagtctc 257700
ggagggcggg gaaggacccg agcctcctgt ccgaaggcag ggctgggcct ggctccagcc 257760
agttgctggc cctggggagc aggcccactg gccggagctt ctgcttttcc aagactggcc 257820
agaaatgggg attttgatgt caagtcactg ggcctttatg atgcggcaac ctgttcccac 257880
ttagcccaat tttaataaga ccaggttggc aggagatgcc atggtggcca cctgcccccc 257940
gagcagccgt tcccctcatt gctgctgctc tcacacctgg aagatggccc ctcccgggcc 258000
cacggcaccc gcaccaggtc cagctgcctc tgcatggggg ccactgaggc agggacctcc 258060
ctaggcagga ggcctgtgtc actgtggtgc cctgtcactg cagcccaaga ggccgtccac 258120
tcacagcctg agggtctggg ggaggccacc atgggcaggg ccagagctga gggtctgagg 258180
tgggaggggt aagggggctg gcagtcacaa agccaggaga gggtgtccag aagcctgact 258240
ttcgtcagca acagggagcc ccagcaggtt ctggagcaga gaaggctacg gtgtaggggg 258300
gtctctgtgc tcaagggtgt gaaacaatgg ccacacatcc tgcagacccc tggaaaatga 258360
agcctcagag caggactgtg gcggaggcgt ggaagacaga ggcaacgggg aggtggccga 258420
gtccccactg gcagggggca gccctggtgg acgctgggct cagaagggaa ggacagatgg 258480
gctagggtgg agacacacaa ccccactgtg agaatgggtt cccactgttt ggccgcgtga 258540
cccccccca ggaggagagc acggcacacg cgcaacttac ttcctccaca gggccggggg 258600
ctgctcgggt tccttctccg cagagcccag ggatgcgaca agtgcgaggg gccaggccag 258660
cagcatcatg gcagcggcaa ccaggagccg gaccgggaag agcgtcagtg tcatgagggc 258720
cacctgcaga cagaggggggg cattatccag agaatccatg taggacacca ggcagctccc 258780
cacccggcag aggctagccc agggggacag cgcgccccac agagtggaga gctggggagg 258840
ggctgcagct ggcacacagc tgaccaaggc tgaccagcac tgaccgaggc tgaccaaggc 258900
tgaccgaggc tgaccagtgc tgaccggcgc tgaccaaggc taaccaacac tgaccagcac 258960
tgaccaaggc tgaccggcac tgaccaaggc tgaccaacat tgaccggcac tgaccaaggc 259020
tgaccggtgc tgaccaaggc tgaccaacac tgaccgacac tgaccaaggc tgaccggcac 259080
tgaccaaggc tgaccaacac tgactggcac tgaccaaggc tgaccaacat tgaccggcac 259140
tgaccaaggc tgaccggtgc tgatgccgac cagccctcac catggctgtg gacactggcc 259200
agtgctaact gacatgctcc acacccactg cccgcttcct gcctccttac tggagctgcc 259260
atgcgagtgg cttgggaaag acacagtgga gcttcctcct gcaaattcaa gggccactct 259320
gacgcacagc acacaacccc aggcagctcc accccgcagg acgcaccccc aggcagctcc 259380
gccccccagg acacaccccc agccccgcag gcctcactca gcagtttccc ctgaaatcgt 259440
ttatgtggac atgagaaagc atacgccacc ctaggcagtg ttggtgacag ggggaggtag 259500
ctggcctgca gtttgcaagg ttggacctca gaccacagag aagacggcag aatgaaggtg 259560
tgactttcca gtcaatggaa gacccggcgc aggagagtga actggcggga gggcaggggc 259620
gcaggtcaat atgggggctc aggaaggccc ccccaagccag ggcaggcccc cacgccaggg 259680
aaggccgagg ctgcgggatc ccaggcagcc cagggtctga ggatggaggt ccctccggga 259740
ggcgggcagg actccgtgta gaggggcggg aggctttggg tgaggggaaa caccagtcct 259800

gaggtgcagg tttagtgcag agacagaaaa gacccaggag acacagatcg cagttggctc 259860
cccaagcagc agccatccct aatttctgat gcctgtcatg agagcgctcc cactctgttt 259920
ccttggtttg tccctagatt cttttgagtt ctcaacggag accagcatag cccaagagaa 259980
acatgcctgc cacatgtgtc attttaaatt ttcgagtggc cacatttaaa gggaatgaca 260040
atttaatata cagctcattt cgcccggtat atctgaaata ccatttcgac acgtactcaa 260100
cgtagaagtt gctactggct agtttgcatt ttctcccgct gtcttggatt gtgcagtgtg 260160
catttcactc tcacagcttt atgccacctg tgtctccacc ctgtcagtgc ccagtggtca 260220
ggtcagcctg ttgggtacgc agatctagat gatgtttggt ttgcgagaga tgacgacctc 260280
taatttgcct ccccttcccc gcttctgggg gctgctcctc accccagctg ggtccccgct 260340
tcagcatcac actcgctgca ccaagcaacc acaaatctgt tatcccgcca ttggtgacga 260400
tcgtcactgt tccacatcgt gttactggca ttcagcaaac gttctttact gctgagaaac 260460
cttcccaggc ctcatcctcc acgagttttg ttttgcttta aatcatgaac gggtgctgag 260520
ctttatcaga ggtgcttcct gcatctgtgg ggagagccac gtgctgcacc ccacttgaag 260580
ccactaacat gatgccttac ggccacggtc tctctaatac tgaaagctag cttcagcgct 260640
gtgcagctcc ctgttaggat tgttttttaa aaggaattgc taggtctgtt ggccactgtc 260700
accagctgtt aagtaagacg cgcctgtccg ctcctgtcac ttcctcccag cggcttcctc 260760
gtgcagtcgc cctacgaaac cagcgtgcac ccatgcctgc cggcagctcc tgaccgttgc 260820
ttcactgttt ccaaccctac tgtttataga gtcccttttt ttttgggttg acttcactgt 260880
ttttgtcaat ggggaagtct cttcctggaa gcttttggca tatttttccc tcccgctacc 260940
aaggtctcct cccagctgcc tttaagataa cagtgaagtt gcccgccaca ctctccttgg 261000
gttcacacca agcctgctct ctggtttcat ttctaatgtt atctgtgtct gccaaaactt 261060
ctaaacctac gttgccagat atttttctat tttatcagtc ttcttacaga accatctcag 261120
atttgttcat tctctctgtt gtgacctaat catattaatt ttggctctta ttagttcctc 261180
tttcttctgc tttgaattta gtcaaatatt cttttcaact ttttgaggga aaagcccact 261240
ttgttatttt cagaaatacc ttatttcctg atagtcacat ttagaacaca gctcccccta 261300
aggaccacct tgatttaatc ccacaagtct aaggggcagc cttttccttt atttgtaaaa 261360
tttagtttta aatatttcat ccattcccat ctactgttac tgtttcatat tttgttgcag 261420
tggaggagag cgcagcggag gagaccgtag cctcggcctt cagcccacct ggtcccgggg 261480
ctccagggcc actggctggg aagaggccac tctgcagtgc acaagcctgg ctctgctgtt 261540
aaagccgaca gctaggcagg gcgtggtggc tcacatctgt aatcccagca ctttgggagg 261600
ccaaggtggg aggatcgcct gcggtcggga gttcgagacc agcctgacca acatggagaa 261660
cccatctcta ctaaaaatac aaaagtagcc gggcgtggtg gcgcatgcct gtaatcacag 261720
ctactctgga ggctgaggca ggagaatcac ttggacccgg gagtcagagg ttgtggggag 261780
ctgagatcgc gccattgcgc tctagcctgg gcaacaagag cgaatctccg tcccaaaaaa 261840
aaaaaaaaaa aagccgacaa taagttactg ggtaagtttc ttcttcccat aaattgttct 261900
tagaacttgt tgcctccctg ggcagacgcc atgaagtgtg ataaccagca ctaagagaaa 261960
gtctcaggaa gttccccggt ctgaccctgg ggccacggca gcctctgccc acagcctgct 262020
tcagtccacc tctggtgctg cagccaactc tgctacaaca ctgcttccac cagagtgtgg 262080
aataaaatca cagtaaccca cgctgttcct gaaacagcac cgactgcaac actgcttcca 262140
ccacagggtg tggaataaaa tcacagtaac ccacgctgtt cctgaaacag caccgactgc 262200
aacactgctt ccaccacagg gtgtggaata aaatcacagt aacccacgct gttcctgaaa 262260
cagcaccgac tgcaacactg cttccaccag agtgcggaat aaaatcacag taacccacgg 262320
tgttcctgaa acagcaccga ctacaactgc ttccaccaga gtgcggaata aaatcacagt 262380
aacccacgct gttcctgaaa cagcaccgac tgcaacactg cttccaccac agagtgcgga 262440
ataaaatcac agtaacccac ggtgttcctg aaacagcacc gactgcaaca ctgcttccac 262500
cacagagtgc ggaataaaat cacagtaacc catggtgttc ctgaaacagc accgactgcc 262560
aggccattcc atatgggacc acccactggg aacagctccg gggctggggg agcagcgccc 262620
catccccaaa caacacgggg catgaatgag ccctgggtgg ggaggccccg cctgcatgcc 262680
gggtagggta gtcaggctgc tggttcctta gcacatgggg ttcgctgggc cagccagtgg 262740
ggggatgcac tgtgagacca cgtgaccctc ggctcgcgca cccccacgcc tgctcctgcg 262800
cacataggga agactgggcg accacctgag cctcgactta cacattcctg cacctactcc 262860
ttcgtgcatg ggttagactg catgaccacc tgaccctcac ctcacacgtc catgcatcca 262920
tgcacctgct gcttcgtgca cggggaggac tgtgtgaaca agcgaccctc tgcaccagct 262980
ccttcctcta tgggaaagaa tgcctgacca cgtgacgctc cccaccagtc agtgcacctg 263040
ctcctgttgg tgtcccagaa gactgcgtga ccatgtgacc ctcagcccat gcaccccttgt 263100
gcctgctcgt ggtgcatcag ggaatgcggc ccacacttct gagcttagca gcagcaagga 263160
gaggggcctg tgggccacag ttgcaatcgc tgatcctcgg caaacagggc tgatgagggg 263220
agggtggaga gggaacatgg ccagggagga gctccccagg cctccagcag atggggaggg 263280
tgtggctctg ctctgcagtg ctgtgaaaac tgcttggaga gggggcctct gggagcccca 263340
ggcaggagga ggagcagtgc tcctggtgaa gagcccgggc atctccaccg atctgccttc 263400
acctcaccca gctctgggcc tgtgcgctct ttctctgcca ggctcggccc gcccacagga 263460
gccgggagcc ctcaccaagg agaacaggac ttgggtgact gctgtgactc cttccagaag 263520
cttctatgac cccgcacctt ctgcccacct gtctccacag gccccacccc tgcgtccagc 263580

```
ctcaggcaca cccattggga tccctccatt cctcatgccc ccacgccctg gtgcagatgg 263640
gtctggatgg actctagccc cccaggagat gccagtatgg cagagctgca gagagagaag 263700
ctggctgggg cagctgcggc agccaagccc ctcagcactg gcaggggtgg ccaagggagg 263760
tgctgggtag gcaaagaaat tcttagaaaa tcctggctag accagaaact cccagaacaa 263820
tggccttcta aggaaagggg gctggcagtg cggtctccca ggcagcgtcg ctggagactc 263880
aggtcgctaa agtggaggca ccgggcacag ccattcctcc ccacggcagc tttgcagtgt 263940
ccactgagag caagcgtgcg tttacagacc tgcagccaca gggacacctg tcagagaggg 264000
caggtgtctt ttctcctcac ccaagcaagg cccagcaccc tggctcagag gcttccacag 264060
aagcccccctt ggcaccttca ccagaggcag caggggcagc atgcggaacc aagacatcac 264120
tgcaagtcgt ggacagggat aagcgggctg ttgcgtggac aaggtgggca gatgtgggga 264180
gcgggagcag cctccaggca gtgtggccag aggctgaatg cggagagggg gagggactca 264240
gagaggagaa cacggaccac gctcctggtc accataggtc aggcctccac gcggcagcca 264300
actggggagg acactgtgga cgcctgcaga cgctgagacc tgcaacctgg ccctcccaag 264360
acaagctgct gatggctgtg ggccggctgt caccacagga ccctgatcac cctgagaaat 264420
gccagttgga ggaagcaata tggtcaccct gtgtgagcca taagtgaggg tttatagcat 264480
aatcctaatg aggaactttg tctgaagtct gaggctgagt tacttcaact atttgaaagt 264540
tggctacgat aaaccaaggc gtccaggcag tgacctgccc acatgcaggg agagctttgg 264600
tgcggactgc ggccgctaaa taccacttcc cagcagaagg cccagggctc ctcagccggg 264660
cttgctccag gtcacaggtg ggaaatgcag agatgaagat gggacagcac ggcgcagccc 264720
acatcaggga agctggcagg ggccgggcgc gagcgctcac gcctgcagtc ccaggagcac 264780
ggcgcggccc aaatcaagga agctggcaga ggctcatgtc ctgagggctg gctcgaggcc 264840
tacccagctc acactcacgt gacagggatc cagcaacagc caatgccgag cctggtgcag 264900
gagcagcaca gcagaccagt gggaactgac cctctctggg tctagatgca accaccagct 264960
tccagaaagc cccaatgaca acaaggagat gcaatcaatc agaaaatgca gagaactcca 265020
caaaacacaa aactccacaa aacacagccc aaatccattg cagcaaacaa ggaggagctg 265080
gaggaccctc ctgatccaca cgcaccagag gcccgaggga gctcgttcac ccctccacct 265140
tgcgaggaca cagaaggtgc catctattga cccggagggg gtccttggca gacaccaaat 265200
ctgccggcat cttggacttc agcctccaga atggtgagaa gtccgtgtct gctgttcggg 265260
aggcacctcg tctgtggtcc tttgtgacag cagccgcgcc aagcagggcg ggcacagtgg 265320
gagtgcacgt gaccccggcc tggccacaca ccgtcattct ccgggctggg ggacaggtcc 265380
caggcccact gtattatcac tgctttggtg ttctaggttg aattttgcgc cccccttcccc 265440
aattcctgtt gatgtcctga cccccaggac tccagagggt gactgtattt ggagaagggg 265500
ctgttagaga ggtgacgaag gtaaaatgag atcatctggg ggccctgagc caacctgcct 265560
ggtgtcctta taagaagagg agatgaggac acagacaccc agggagggac ggccctgtga 265620
ggacacaggg tgaaggcacc atctgcaagc ccaggagagg cctcaggagg aaccagccct 265680
gctgcgcctc ggtctcggac tccagcctcc aggatggtga gacaacaatg tctgtggttt 265740
aagccccatc tgaggcactt tattatggaa gccggagctg agaaagacac ttgacatatg 265800
tttgaaattt cccatgataa aaagtataaa agtgatgact cttggcttaa atgtaaacaa 265860
ggccatccaa atgttggcct ggaactctct ggaagtctct gagtgaaggc gccggtggtc 265920
acaatgccca ggcccaggct ctctgatttc aggcagctgg caaacccctc cctcgtctct 265980
gccaaggcca ccagggccgt aagcagcccc aggccccagc atcacccggc gtttcacaat 266040
cagtgctgaa ctcaaagaca cgaggttgcc agagaacatg tggtccctct cgtgtgcact 266100
cacgccatcg cggtgcccga caggggtgct gcggtgaggc ccaccaggcg cagggatgct 266160
gcggcccctc cgagcccact tgttggagat gtcacactgc agtgccccag ccagtgaagg 266220
ggctccaatg cacctgcaac gaggcagggg tggaggggag gctgcccaaa ggatggcgcc 266280
cggagactcg aaggccgcga ggccgcgtca tcccgcacag gcggccgggg cagatgctca 266340
gtgccagcca ccccaagctc tacggttagc gcggtggagg cagctcacag gaatgctccc 266400
tcctgaaggt gcatcccaac attgctgctt taattatgtg gaaaaaatta tcccaactcc 266460
acaggatatg cgacagtgcg cttgcaaatc cacgagagaa cgtgcactcc tcccacggtg 266520
agagggcgag cggcagcaca cacaggcgcc tgctgtccaa ggtctaattt tcttttgctt 266580
tttcctttta gagaaagaac aactttgaa agcacagcag ttctctcagg catcccatcg 266640
gacggagccg gcacttccat cagcaaacac caccagcccg ccctcggcat ctgagagctg 266700
gagggagctt cgggaatcac ccactcccgt ccagcaccca cagacacaaa ccgagcaagg 266760
cagcagcagg aaacgccctc cttttctgtc tccccaatca ccaaaacacc tttattcata 266820
taacatgact tctggtccaa accaaacgct gccttcaaat atggaatcag cgatcacgcc 266880
cacaatgaat tctcatagtt aacgatacta aaagcagaca ggaaatgtga aaaagtggca 266940
ccgtgaacca ttgccgtggg ctctctctgc acacacaacc actcttacct ttggagcccg 267000
aggcacggcc cgcaccccag gcgctacaga tcccaggtga ggcctgcagg aggagggatg 267060
ggctgccagg agcgcaggcc acatcacggg gacggtttca ttgggaggaa cattttatgt 267120
ttactgggta ttcccatttc tgtttgtgta attttctcat ttatataaat gtatatacat 267180
cttgtgccaa atttatggtg ggtttctttg tctttttctc acccctgtga acatc 267235
```

<210> 2
<211> 599
<212> DNA
<213> Homo sapiens

<400> 2

```
acttcaactg aaacaggaga gacaggccca ggtcagctgt gggcagcaca ggagacgggg      60
gccgggccgc gggcagcaca caccgcctta ccttgtagtt cacaaagagc tggggcagca     120
tgaagaggaa accaaaggca tagacccctg cagaaagaca gacagcactc acgaggtgcg     180
gagggccggg ctgccacatc tacgcacaga cggcactcac gaggtgtgga gggccgagct     240
gccacgtcta tgcatagtgg gcagaaaaca aggtctgcta tccagacaac ttcagagtcy     300
atcatggtgt gaagcagctt tctggctggt aagtttatca agagtctacg acaactttgg     360
agagcaaagc tcttctattt attaatcagt gtaactgctg cccacggggt caagtcagtg     420
agagcactgg agcaccctgg ggctatgagg acacggcctc ctgacccaca aggtcctgcc     480
ccaggggtca ggtgtgggac tcctaccagg gaggacggca gtgcgggacc cacctgctct     540
gcaccagaca ggccctcgcc cttaatgctc acgggaccac ttcccaaaga gaccacaaa      599
```

<210> 3
<211> 599
<212> DNA
<213> Homo sapiens

<400> 3

```
tcaccatgtt ggccaggctg gtctcaaact cctgacctca agtgatctgc ccgccttggc      60
ctcccacagt gctgggatta caggtgcaag ccaccgtgcc cggcatacct tgatctttta     120
aaatgaagtc tgaaacattg ctacccttgt cctgagcaat aagaccctta gtgtatttta     180
gctctggcca ccccccagcc tgtgtgctgt tttccctgct gacttagttc tatctcaggc     240
atcttgacac ccccacaagc taagcattat taatattgtt ttccgtgttg agtgtttctk     300
tagctttgcc cccgccctgc ttttcctcct ttgttccccg tctgtcttct gtctcaggcc     360
cgccgtctgg ggtccccttc cttgtccttt gcgtggttct tctgtcttgt tattgctggt     420
aaaccccagc tttacctgtg ctggcctcca tggcatctag cgacgtccgg ggacctctgc     480
ttatgatgca cagatgaaga tgtggagact cacgaggagg gcggtcatct tggcccgtga     540
gtgtctggag caccacgtgg ccagcgttcc ttagccagtg agtgacagca acgtccgct      599
```

<210> 4
<211> 599
<212> DNA
<213> Homo sapiens

<400> 4

```
agcgtcaggg aggccggggt ccccctgggc ctgccagccc cgggtgcgag gaggcgcggg      60
ggcagtgcca gccgaagtct gccgttgccc aagaggccca ggcgtggcgc tgcccctgag     120
ccggagcgga cgcccgttgg gcaggggtcc tgggcccacc cgggcaggac gcgtggaccg     180
agtgaccgtg gtttctgtgt ggtgtcacct gccagacccg ccgaagaagc cacctctttg     240
gagggtgcgc tctctggcac gcgccactcc cacccatccg tgggccgcca gcaccacgcr     300
ggcccccccat ccacatcgcg gccaccacgt ccctgggaca cgccttgtcc cccggtgtac     360
gccgagacca agcacttcct ctactcctca ggcgacaagg agcagctgcg gccctccttc     420
ctactcagct ctctgaggcc cagcctgact ggcgctcgga ggctcgtgga gaccatcttt     480
ctgggttcca ggccctggat gccagggact ccccgcaggt tgccccgcct gccccagcgc     540
tactggcaaa tgcggcccct gtttctggag ctgcttggga accacgcgca gtgccccta      599
```

## Description

## INTRODUCTION

Cancer, the uncontrolled growth of malignant cells, is a major health problem of the modern medical era and is one of the leading causes of death in developed countries. In the United States, one in four deaths is caused by cancer (Jemal, A. et al., CA Cancer J. Clin. 52:23-47 (2002)). Cancer initiation results from the complex interplay of genetic and environmental factors. The estimated contribution of genetic factors varies widely between cancer sites, with prostate cancer generally considered to have the largest genetic component {Lichtenstein, 2000 #18}. However, genetic factors also play a role in cancer types with strong environmental factors such as lung cancer (Jonsson, S., et al. JAMA 292:2977-83 (2004); Hemminki, K., et al. Genet Epidemiol 20:107-116 (2001)).

All cancers are subject to the accumulation of genetic changes that lead to aberrant cell growth and survival. Thus, it could be expected that genetic polymorphisms that affect certain basic cellular processes, such as DNA repair, cell cycle regulation and apoptosis could increase an individual's life-long risk of developing cancer - the actual site of cancer could be determined by other factors, environmental or genetic (Hanahan, D. and Weinber, R.A., Cell 100:57-70 (2000). Indeed, studies on cancer risk in relatives of cancer patients lends strong evidence for shared genetic factors that increase the risk of more than one cancer type (Cannon-Albright, L.A., et al. Cancer Res 54:2378-85 1994); Amundadottir, L.T., et al. PLoS Med 1:e65 (2004)). Furthermore, mutations in strongly cancer-predisposing genes are associated with an increased risk of more than one type of cancer, as exemplified by the spectrum of cancer types in Li-Fraumeni syndrome that are caused by mutations in *TP53* (Malkin, D., et al. Science 250:1233-38 (1990).. However, highly penetrant mutations explain only a small fraction of total cancer cases and the majority of genetic cancer risk is thought to be due to the presence of multiple common genetic variants of low penetrance.

**Basal Cell Carcinoma.** Cutaneous basal cell carcinoma (BCC) is the most common cancer amongst whites and incidence rates show an increasing trend. The average lifetime risk for Caucasians to develop BCC is approximately 30% [Roewert-Huber, et al., (2007), Br J Dermatol, 157 Suppl 2, 47-51]. Although it is rarely invasive, BCC can cause considerable morbidity and 40-50% of patients will develop new primary lesions within 5 years[Lear, et al., (2005), Clin Exp Dermatol, 30, 49-55]. Indices of exposure to ultraviolet (UV) light are strongly associated with risk of BCC [Xu and Koo, (2006), Int J Dermatol, 45, 1275-83]. In particular, chronic sun exposure (rather than intense episodic sun exposures as in melanoma) appears to be the major risk factor [Roewert-Huber, et al., (2007), Br J Dermatol, 157 Suppl 2, 47-51]. Squamous cell carcinoma of the skin (SCC) shares these risk factors, as well as several genetic risk factors with BCC [Xu and Koo, (2006), Int J Dermatol, 45, 1275-83; Bastiaens, et al., (2001), Am J Hum Genet, 68, 884-94; Han, et al., (2006), Int J Epidemiol, 35, 1514-21]. Photochemotherapy for skin conditions such as psoriasis with psoralen and UV irradiation (PUVA) have been associated with increased risk of SCC and BCC. Immunosuppressive treatments increase the incidence of both SCC and BCC, with the incidence rate of BCC in transplant recipients being up to 100 times the population risk [Hartevelt, et al., (1990), Transplantation, 49, 506-9; Lindelof, et al., (2000), Br Dermatol, 143, 513-9]. BCC's may be particularly aggressive in immunosuppressed individuals.

There is an unmet clinical need to identify individuals who are at increased risk of BCC and/or SCC. Such individuals might be offered regular skin examinations to identify incipient tumours, and they might be counseled to avoid excessive UV exposure. Chemoprevention either using sunscreens or pharmaceutical agents [Bowden, (2004), Nat Rev Cancer, **4**, 23-35.] might, be employed. For individuals who have been diagnosed with BCC or SCC, knowledge of the underlying genetic predisposition may be useful in determining appropriate treatments and evaluating risks of recurrence and new primary tumours. Screening for susceptibility to BCC or SCC might be important in planning the clinical management of transplant recipients and other immunosuppressed individuals.

**Melanoma.** Cutaneous Melanoma (CM) was once a rare cancer but has over the past 40 years shown rapidly increasing incidence rates. In the U.S.A. and Canada, CM incidence has increased at a faster rate than any other cancer except bronchogenic carcinoma in women. Until recently incidence rates increased at 5-7% a year, doubling the population risk every 10-15 years.

The current worldwide incidence is in excess of 130,000 new cases diagnosed each year [Parkin, et al., (2001), Int J Cancer, 94, 153-6]. The incidence is highest in developed countries, particularly where fair-skinned people live in sunny areas. The highest incidence rates occur in Australia and New Zealand with approximately 36 cases per 100,000 per year. The U.S.A. has the second highest worldwide incidence rates with about 11 cases per 100,000. In Northern Europe rates of approximately 9-12 per 100,000 are typically observed, with the highest rates in the Nordic countries. Currently in the U.S.A., CM is the sixth most commonly diagnosed cancer (excluding non-melanoma skin cancers). In the year 2008 it is estimated that 62,480 new cases of invasive CM will have been diagnosed in the U.S.A. and 8,420 people will have died from metastatic melanoma. A further 54,020 cases of in-situ CM are expected to be diagnosed during the year.

Deaths from CM have also been on the increase although at lower rates than incidence. However, the death rate from CM continues to rise faster than for most cancers, except non-Hodgkin's lymphoma, testicular cancer and lung cancer in women [Lens and Dawes, (2004), Br Dermatol, 150, 179-85.]. When identified early, CM is highly treatable by surgical excision, with 5 year survival rates over 90%. However, malignant melanoma has an exceptional ability to metastasize to almost every organ system in the body. Once it has done so, the prognosis is very poor. Median survival for disseminated (stage IV) disease is 7 ½ months, with no improvements in this figure for the past 22 years. Clearly, early detection is of paramount importance in melanoma control.

CM shows environmental and endogenous host risk factors, the latter including genetic factors. These factors interact with each other in complex ways. The major environmental risk factor is UV irradiation. Intense episodic exposures rather than total dose represent the major risk [Markovic, et al., (2007), Mayo Clin Proc, 82, 364-80].

It has long been recognized that pigmentation characteristics such as light or red hair, blue eyes, fair skin and a tendency to freckle predispose for CM, with relative risks typically 1.5-2.5. Numbers of nevi represent strong risk factors for CM. Relative risks as high as 46-fold have been reported for individuals with >50 nevi. Dysplastic or clinically atypical nevi are also important risk factors with odds ratios that can exceed 30-fold [Xu and Koo, (2006), Int J Dermatol, 45, 1275-83].

**Lung Cancer.** Lung cancer causes more deaths from cancer worldwide than any other form of cancer (Goodman, G.E., Thorax 57:994-999 (2002)). In the United States, lung cancer is the primary cause of cancer death among both men and women. In 2007, the death rate from lung cancer was an estimated 160,390 deaths, exceeding the combined total for breast, prostate and colon cancer (America Cancer Society, www.cancer.org). Lung cancer is also the leading cause of cancer death in all European countries and is rapidly increasing in developing countries. While environmental factors, such as lifestyle factors (*e.g.*, smoking) and dietary factors, play an important role in lung cancer, genetic factors also contribute to the disease. For example, a family of enzymes responsible for carcinogen activation, degradation and subsequent DNA repair has been implicated in susceptibility to lung cancer. In addition, an increased risk to familial members outside of the nuclear family has been shown by deCODE geneticists by analysing all lung cancer cases diagnosed in Iceland over 48 years. This increased risk could not be entirely accounted for by smoking indicating that genetic variants may predispose certain individuals to lung cancer (Jonsson et.al., JAMA 292(24):2977-83 (2004); Amundadottir et.al., PLoS Med. 1(3):e65 (2004)).

The five-year survival rate among all lung cancer patients, regardless of the stage of disease at diagnosis, is only 13%. This contrasts with a five-year survival rate of 46% among cases detected while the disease is still localized. However, only 16% of lung cancers are discovered before the disease has spread. Early detection is difficult as clinical symptoms are often not observed until the disease has reached an advanced stage. Currently, diagnosis is aided by the use of chest x-rays, analysis of the type of cells contained in sputum and fiberoptic examination of the bronchial passages. Treatment regimens are determined by the type and stage of the cancer, and include surgery, radiation therapy and/or chemotherapy. In spite of considerable research into therapies for this and other cancers, lung cancer remains difficult to diagnose and treat effectively. Accordingly, there is a great need in the art for improved methods for detecting and treating such cancers.

Smoking of tobacco products, and in particular cigarettes, is the largest known risk factor lung cancer with a global attributable proportion estimated to be approximately 90% in men and 80% in women. Although the risk of lung cancer associated with tobacco smoking is strongly related to duration of smoking, and declines with increasing time from cessation, the estimated lifetime risk of lung cancer among former smokers remains high, ranging from approximately 6% in smokers who give up at the age of 50, to 10% for smokers who give up at age 60, compared to 15% for lifelong smokers and less than 1% in never-smokers (Peto et al. 2000 BMJ, 321, 323-32, Brennan, et al. 2006 Am J Epidemiol 164, 1233-1241). In populations where the large majority of smokers have quit smoking, such as men in the US and UK, the majority of lung cancer cases now occurs among ex-smokers (Doll et al. 1994 BMJ 309, 901-911, Zhu et al. 2001 Cancer Res, 61, 7825-7829). This emphasizes the importance of developing alternative prevention measures for lung cancer including the identification of high risk subgroups.

Notably, only about 15% of lifelong smokers will develop lung cancer by the age of 75, and approximately 5 to 10% of lifetime smokers will develop another tobacco-related cancer (Kjaerheim et al. 1998 Cancer Causes Control 9, 99-108). A possible explanation for these large differences in risk for individuals with similar level of tobacco exposures could be that genetic factors play a determining role in lung cancer susceptibility (Spitz et al. 2005 J Clin Oncol 23, 267-275). Identifying genes, which influence the risk of lung cancer could be important for several aspects of management of the disease.

Segregation analyses predict that the majority of genetic risk for lung cancer is most likely to be polygenic in nature, with multiple risk alleles that confer low to moderate risk and which may interact with each other and with environmental risk factors. Many studies have investigated lung cancer susceptibility based on the presence of low-penetrance, highfrequency single nucleotide polymorphisms in candidate genes belonging to specific metabolic pathways. Genetic polymorphisms of xenobiotic metabolism, DNA repair, cell-cycle control, immunity, addiction and nutritional status have been described as promising candidates but have in many cases proven difficult to confirm (Hung et al. 2005 J Natl Cancer Inst 97, 567-576, Hung et al. 2006 Cancer Res 66, 8280-8286, Landi et al. 2006 Carcinogenesis, in press, Brennan et al.2005 Lancet 366, 1558-60, Hung et al. 2007 Carcinogenesis 28,1334-40, Campa et al. 2007 Cancer Causes Control 18, 449-455, Gemignani et al. 2007 Carcinogenesis 28(6), 1287-93, Hall et al. 2007 Carcinogenesis 28, 665-671, Campa et al. 2005 Cancer Epidemiol Biomarkers Prev 14, 2457-2458, Campa et al. 2005 Cancer Epidemiol Biomarkers Prev 14, 538-539, Hashibe et al. 2006 Cancer Epidemiol Biomarkers Prev 15, 696-703).

For cancers that show a familial risk of around two-fold such as lung cancer (Jonsson et al. 2004 JAMA 292, 2977-2983, Li and Hemminki 2005 Lung Cancer 47, 301-307, Goldgar et al. 1994 J Natl Cancer Inst 86, 1600-1608), the majority of cases will arise from approximately 10%-15% of the population at greatest risk (Pharoah et al. 2002 NatGenet 31, 33-36). The identification of common genetic variants that affect the risk of lung cancer may enable the identification of individuals who are at a very high risk because of their increased genetic susceptibility or, in the case of genes related to nicotine metabolism, because of their inability to quit smoking. Such findings could potentially lead to chemoprevention programs for high risk individuals, and are especially of importance given the high residual risk that remains among ex-smokers, among whom the majority of lung cancers in the US and Europe now occur.

**Bladder Cancer.** Urinary bladder cancer (UBC) is the 6th most common type of cancer in the United States with approximately 67,000 new cases and 14,000 deaths from the disease in 2007. UBC tends to occur most commonly in individuals over 60 years of age. Exposure to certain industrially used chemicals (derivatives of compounds called arylamines) is strong risk factor for the development of bladder cancers. Tobacco use (specifically cigarette smoking) is thought to cause 50% of bladder cancers discovered in male patients and 30% of those found in female patients. Thirty percent of bladder tumors probably result from occupational exposure in the workplace to carcinogens such as benzidine. Occupations at risk are metal industry workers, rubber industry workers, workers in the textile industry and people who work in printing. Certain drugs such as cyclophosphamide and phenacetin are known to predispose to bladder cancer. Chronic bladder irritation (infection, bladder stones, catheters, and bilharzia) predisposes to squamous cell carcinoma of the bladder.

Familial clustering of UBC cases suggests that there is a genetic component to the risk of the disease (Aben, K.K. et al. Int J Cancer 98, 274-8 (2002); Amundadottir, L.T. et al. PLoS Med 1, e65 Epub 2004 Dec 28 (2004); Murta-Nascimento, C. et al. Cancer Epidemiol Biomarkers Prev 16, 1595-600 (2007)). Genetic segregation analyses have suggested that this component is multifactorial with many genes conferring small risks (Aben, K.K. et al. Eur J Cancer 42, 1428-33 (2006)). Many epidemiological studies have evaluated potential associations between sequence variants in candidate genes and bladder cancer, but the most consistent risk association to the disease is found for variations in the NAT2 gene. (Sanderson, S. et al., Am J Epidemiol 166, 741-51 (2007)).

Majority (>90%) of bladder cancers are transitional cell carcinomas (TCC) and arise from the urothelium. Other bladder cancer types include squamous cell carcinoma, adenocarcinoma, sarcoma, small cell carcinoma and secondary deposits from cancers elsewhere in the body. TCCs are often multifocal, with 30-40% of patients having a more than one tumor at diagnosis. The pattern of growth of TCCs can be papillary, sessile (flat) or carcinoma-in-situ (CIS). Superficial tumors are defined as tumors that either do not invade, or those that invade but stay superficial to the deep muscle wall of the bladder. At initial diagnosis, 70% of patients with bladder cancers have superficial disease. Tumors that are clinically superficial are composed of three distinctive pathologic types. The majority of superficial urothelial carcinomas present as noninvasive, papillary tumors (pathologic stage pTa). About 70% of these superficial papillary tumors will recur over a prolonged clinical course, causing significant morbidity. In addition, 5-10% of these papillary lesions will eventually progress to invasive carcinomas. These tumors are pathologically graded as either low malignant potential, low grade or high grade. High grade tumors have a higher risk of progression. Flat urothelial carcinoma in situ (CIS) are highly aggressive lesions and progress more rapidly than the papillary tumors. A minority of tumors invade only superficially into the lamina propria. These tumors recur 80% of the time, and eventually invade the detrusor muscle in 30% of cases. Approximately 30% of urothelial carcinomas invade the detrusor muscle at presentation. These cancers are highly aggressive. Those invasive tumors may spread by way of the lymph and blood systems to invade bone, liver, and lungs and have high morbidity (Kaufman, D.S. *Ann Oncol* 17, v106-112 (2006)).

The treatment of transitional cell or urothelial carcinoma is different for superficial tumors and muscle invasive tumors. Superficial bladder cancers can be managed without cystectomy (removing the bladder). The standard initial treatment of superficial tumors includes cystoscopy with trans-urethral resection of the tumor (TUR). The cystoscope allows visualization and entire removal of a bladder tumor. Adjuvant intravesical drug therapy after TUR is commonly prescribed for patients with tumors that are large, multiple, high grade or superficially invasive. Intravesical therapy consists of drugs placed directly into the bladder through a urethral catheter, in an attempt to minimize the risk of tumor recurrence and progression. About 50-70% of patients with superficial bladder cancer have a very good response to intravesical therapy. The current standard of care consists of urethro-cystoscopy and urine cytology every 3-4 months for the first two years and at a longer interval in subsequent years.

Cystectomy is indicated when bladder cancer.is invasive into the muscle wall of the bladder or when patients with superficial tumors have frequent recurrences that are not responsive to intravesical therapy. The benefits of surgically removing the bladder are disease control, eradication of symptoms associated with bladder cancer, and long-term survival. For advanced bladder cancer that has extended beyond the bladder wall, radiation and chemotherapy are treatment options. Local lymph nodes are frequently radiated as part of the therapy to treat the microscopic cancer cells which may have spread to the nodes. Current treatment of advanced bladder cancer can involve a combination of radiation and chemotherapy.

Early detection can improve prognosis, treatment options as well as quality of life of the patient. If screening methods could detect bladder cancers destined to become muscle invading while they are still superficial it is likely that a significant reduction in morbidity and mortality would result. Cystoscopic examination is costly and causes substantial discomfort for the patient. Urine cytology has poor sensitivity in detecting low-grade disease and its accuracy can vary between pathology labs. Many urine-based tumor markers have been developed for detection and surveillance of the disease and some of these are used in routine patient care (Lokeshwar, V.B. et al. Urology 66, 35-63 (2005); Friedrich, M.G. et al. BJU Int 92, 389-92 (2003); Ramakumar, S. et al. J Urol 161, 388-94 (1999); Sozen, S. et al. Eur Urol 36, 225-9 (1999); Heicappell, R. et al. Urol Int 65, 181-4 (2000)). However, no biomarker reported to date has shown sufficient sensitivity and specificity for detecting all types of bladder cancers in the clinic. It should be remembered that efficiency of screening increases with the disease's prevalence in the screened population. Therefore, the efficiency of the test could be increased by limiting the screening program to people at high risk. For bladder cancer, this may mean restricting participation to people with occupational exposure to known bladder carcinogens or individuals with known cancer predisposing variants.

The genetic polymorphisms in a number of metabolic enzymes and other genes have been found as the modulators of bladder cancer risk. The most studied polymorphisms in connection with bladder cancer risk are polymorphisms in genes for some important enzymes, especially N-acetyltransferases (NATs), glutathione S-transferases (GSTs), DNA repair enzymes, and many others. An improved understanding of the molecular biology of urothelial malignancies is helping to define more clearly the role of new prognostic indices and multidisciplinary treatment for this disease.

Despite intensive efforts, the genes that account for a substantial fraction of bladder cancer risk have not been identified. Although studies have implied that genetic factors are likely to be prominent in bladder cancer, only few genes have been identified as being associated with an increased risk for the disease. Thus, it is clear that the majority of genetic risk factors for bladder cancer remain to be found. It is likely that these genetic risk factors will include a relatively high number of low-to-medium risk genetic variants. These low-to-medium risk genetic variants may, however, be responsible for a substantial fraction of bladder cancer, and their identification, therefore, a great benefit for public health.

There is clearly a need for improved diagnostic procedures that would facilitate early-stage bladder cancer detection and prognosis, as well as aid in preventive and curative treatments of the disease. In addition, there is a need to develop tools to better identify those patients who are more likely to have aggressive forms of bladder cancer from those patients that are diagnosed with the superficial disease. This would help to avoid invasive and costly procedures for patients not at significant risk.

**Prostate Cancer.** The incidence of prostate cancer has dramatically increased over the last decades and prostate cancer is now a leading cause of death in the United States and Western Europe (Peschel, R.E. and J.W. Colberg, Lancet 4:233-41 (2003); Nelson, W.G. et al., N. Engl. J. Med. 349(4):366-81 (2003)). Prostate cancer is the most frequently diagnosed noncutaneous malignancy among men in industrialized countries, and in the United States, 1 in 8 men will develop prostate cancer during his life (Simard, J. et al., Endocrinology 143(6):2029-40 (2002)). Although environmental factors, such as dietary factors and lifestyle-related factors, contribute to the risk of prostate cancer, genetic factors have also been shown to play an important role. Indeed, a positive family history is among the strongest epidemiological risk factors for prostate cancer, and twin studies comparing the concordant occurrence of prostate cancer in monozygotic twins have consistently revealed a stronger hereditary component in the risk of prostate cancer than in any other type of cancer (Nelson, W.G. et al., N. Engl. J. Med. 349(4):366-81 (2003); Lichtenstein P. et.al., N. Engl. J. Med. 343(2):78-85 (2000)). In addition, an increased risk of prostate cancer is seen in 1$^{st}$ to 5$^{th}$ degree relatives of prostate cancer cases in a nation wide study on the familiality of all cancer cases diagnosed in Iceland from 1955-2003 (Amundadottir et.al., PLoS Medicine 1(3):e65 (2004)). The genetic basis for this disease, emphasized by the increased risk among relatives, is further supported by studies of prostate cancer among particular populations: for example, African Americans have among the highest incidence of prostate cancer and mortality rate attributable to this disease: they are 1.6 times as likely to develop prostate cancer and 2.4 times as likely to die from this disease than European Americans (Ries, L.A.G. et al., NIH Pub. No. 99-4649 (1999)).

An average 40% reduction in life expectancy affects males with prostate cancer. If detected early, prior to metastasis and local spread beyond the capsule, prostate cancer can be cured (*e.g.*, using surgery). However, if diagnosed after spread and metastasis from the prostate, prostate cancer is typically a fatal disease with low cure rates. While prostate-specific antigen (PSA)-based screening has aided early diagnosis of prostate cancer, it is neither highly sensitive nor specific (Punglia et.al, N Engl J Med. 349(4):335-42 (2003)). This means that a high percentage of false negative and false positive diagnoses are associated with the test. The consequences are both many instances of missed cancers and unnecessary follow-up biopsies for those without cancer. As many as 65 to 85% of individuals (depending on age) with prostate cancer have a PSA value less than or equal to 4.0 ng/mL, which has traditionally been used as the upper limit for a normal PSA level (Punglia et.al., N Engl J Med. 349(4):335-42 (2003); Cookston, M.S., Cancer Control 8(2):133-40 (2001); Thompson, I.M. et.al., N Engl J Med. 350:2239-46 (2004)). A significant fraction of those cancers with low PSA levels are scored as Gleason grade 7 or higher, which is a measure of an aggressive prostate cancer.

In addition to the sensitivity problem outlined above, PSA testing also has difficulty with specificity and predicting prognosis. PSA levels can be abnormal in those without prostate cancer. For example, benign prostatic hyperplasia (BPH) is one common cause of a false-positive PSA test. In addition, a variety of non-cancer conditions may elevate serum PSA levels, including urinary retention, prostatitis, vigorous prostate massage and ejaculation. Subsequent confirmation of prostate cancer using needle biopsy in patients with positive PSA levels is difficult if the tumor is too small to see by ultrasound. Multiple random samples are typically taken but diagnosis of prostate cancer may be missed because of the sampling of only small amounts of tissue. Digital rectal examination (DRE) also misses many cancers because only the posterior lobe of the prostate is examined. As early cancers are nonpalpable, cancers detected by DRE may already have spread outside the prostate (Mistry K.J., Am. Board Fam. Pract.16(2):95-101 (2003)).

Thus, there is clearly a great need for improved diagnostic procedures that would facilitate early-stage prostate cancer detection and prognosis, as well as aid in preventive and curative treatments of the disease. In addition, there is a need to develop tools to better identify those patients who are more likely to have aggressive forms of prostate cancer from those patients that are more likely to have more benign forms of prostate cancer that remain localized within the prostate and do not contribute significantly to morbidity or mortality. This would help to avoid invasive and costly procedures for patients not at significant risk.

Although genetic factors are among the strongest epidemiological risk factors for prostate cancer, the search for genetic determinants involved in the disease has been challenging. Studies have revealed that linking candidate genetic markers to prostate cancer has been more difficult than identifying susceptibility genes for other cancers, such as breast, ovary and colon cancer. Several reasons have been proposed for this increased difficulty including: the fact that prostate cancer is often diagnosed at a late age thereby often making it difficult to obtain DNA samples from living affected individuals for more than one generation; the presence within high-risk pedigrees of phenocopies that are associated with a lack of distinguishing features between hereditary and sporadic forms; and the genetic heterogeneity of prostate cancer and the accompanying difficulty of developing appropriate statistical transmission models for this complex disease (Simard, J. et al., Endocrinology 143(6):2029-40 (2002)).

Various genome scans for prostate cancer-susceptibility genes have been conducted and several prostate cancer susceptibility loci have been reported. For example, HPC1 (1q24-q25), PCAP (1q42-q43), HCPX (Xq27-q28), CAPB (1p36), HPC20 (20q13), HPC2/ELAC2 (17p11) and 16q23 have been proposed as prostate cancer susceptibility loci (Simard, J. et al., Endocrinology 143(6):2029-40 (2002); Nwosu, V. et al., Hum. Mol. Genet. 10(20):2313-18 (2001)). In a genome scan conducted by Smith *et al.,* the strongest evidence for linkage was at HPC1, although two-point analysis also revealed a LOD score of ≥ 1.5 at D4S430 and LOD scores ≥ 1.0 at several loci, including markers at Xq27-28 (Ostrander E.A. and J.L. Stanford, Am. J. Hum. Genet. 67:1367-75 (2000)). In other genome scans, two-point LOD scores of ≥ 1.5 for chromosomes 10q, 12q and 14q using an autosomal dominant model of inheritance, and chromosomes 1q, 8q, 10q and 16p using a recessive model of inheritance, have been reported, as well as nominal evidence for linkage to chr 2q, 12p, 15q, 16q and 16p. A genome scan for prostate cancer predisposition loci using a small set of Utah high risk prostate cancer pedigrees and a set of 300 polymorphic markers provided evidence for linkage to a locus on chromosome 17p (Simard, J. et al., Endocrinology 143(6):2029-40 (2002)). Eight new linkage analyses were published in late 2003, which depicted remarkable heterogeneity. Eleven peaks with LOD scores higher than 2.0 were reported, none of which overlapped (*see* Actane consortium, Schleutker *et.al,* Wiklund *et.al.,* Witte *et.al.,* Janer *et.al.,* Xu *et.al.,* Lange *et.al.,* Cunningham et.al.; all of which appear in Prostate, vol. 57 (2003)).

As described above, identification of particular genes involved in prostate cancer has been challenging. One gene that has been implicated is RNASEL, which encodes a widely expressed latent endoribonuclease that participates in an interferon-inducible RNA-decay pathway believed to degrade viral and cellular RNA, and has been linked to the HPC locus (Carpten, J. et al., Nat. Genet. 30:181-84 (2002); Casey, G. et al., Nat. Genet. 32(4):581-83 (2002)). Mutations in RNASEL have been associated with increased susceptibility to prostate cancer. For example, in one family, four brothers with prostate cancer carried a disabling mutation in RNASEL, while in another family, four of six brothers with prostate cancer carried a base substitution affecting the initiator methionine codon of RNASEL. Other studies have revealed mutant RNASEL alleles associated with an increased risk of prostate cancer in Finnish men with familial prostate cancer and an Ashkenazi Jewish population (Rokman, A. et al., Am J. Hum. Genet. 70:1299-1304 (2002); Rennert, H. et al., Am J. Hum. Genet. 71:981-84 (2002)). In addition, the Ser217Leu genotype has been proposed to account for approximately 9% of all sporadic cases in Caucasian Americans younger than 65 years (Stanford, J.L., Cancer Epidemiol. Biomarkers Prev. 12(9):876-81 (2003)). In contrast to these positive reports, however, some studies have failed to detect any association between RNASEL alleles with inactivating mutations and prostate cancer (Wang, L. et al., Am. J. Hum. Genet. 71:116-23 (2002); Wiklund, F. et al., Clin. Cancer Res. 10(21):7150-56 (2004); Maier, C. et.al., Br. J. Cancer 92(6):1159-64(2005)).

The macrophage-scavenger receptor 1 (MSR1) gene, which is located at 8p22, has also been identified as a candidate prostate cancer-susceptibility gene (Xu, J. et al., Nat. Genet. 32:321-25 (2002)). A mutant MSR1 allele was detected in approximately 3% of men with nonhereditary prostate cancer but only 0.4% of unaffected men. However, not all subsequent reports have confirmed these initial findings (*see, e.g.,* Lindmark, F. et al., Prostate 59(2):132-40 (2004); Seppala, E.H. et al., Clin. Cancer Res. 9(14):5252-56 (2003); Wang, L. et al., Nat Genet. 35(2):128-29 (2003); Miller, D.C. et al., Cancer Res. 63(13):3486-89 (2003)). MSR1 encodes subunits of a macrophage-scavenger receptor that is capable of binding a variety of ligands, including bacterial lipopolysaccharide and lipoteicholic acid, and oxidized high-density lipoprotein and low-density lipoprotein in serum (Nelson, W.G. et al., N. Engl. J. Med. 349(4):366-81 (2003)).

The *EL4C2* gene on Chr17p was the first prostate cancer susceptibility gene to be cloned in high risk prostate cancer families from Utah (Tavtigian, S.V., et al., Nat. Genet. 27(2):172-80 (2001)). A frameshift mutation (1641InsG) was found in one pedigree. Three additional missense changes: Ser217Leu; Ala541Thr; and Arg781His, were also found to associate with an increased risk of prostate cancer. The relative risk of prostate cancer in men carrying both Ser217Leu and Ala541Thr was found to be 2.37 in a cohort not selected on the basis of family history of prostate cancer (Rebbeck, T.R., et al., Am. J. Hum. Genet. 67(4):1014-19 (2000)). Another study described a new termination mutation (Glu216X) in one high incidence prostate cancer family (Wang, L., et al., Cancer Res. 61(17):6494-99 (2001)). Other reports have not demonstrated strong association with the three missense mutations, and a recent metaanalysis suggests that the familial risk associated with these mutations is more moderate than was indicated in initial reports (Vesprini, D., et al., Am. J. Hum. Genet. 68(4):912-17 (2001); Shea, P.R., et al., Hum. Genet. 111(4-5):398-400 (2002); Suarez, B.K., et al., Cancer Res. 61(13):4982-84 (2001); Severi, G., et al., J. Natl. Cancer Inst. 95(11):818-24 (2003); Fujiwara, H., et al., J. Hum. Genet. 47(12):641-48 (2002); Camp, N.J., et al., Am. J. Hum. Genet. 71(6):1475-78 (2002)).

Polymorphic variants of genes involved in androgen action (*e.g.*, the androgen receptor (AR) gene, the cytochrome P-450c17 (CYP17) gene, and the steroid-5-$\alpha$-reductase type II (SRD5A2) gene), have also been implicated in increased risk of prostate cancer (Nelson, W.G. et al., N. Engl. J. Med. 349(4):366-81 (2003)). With respect to AR, which encodes the androgen receptor, several genetic epidemiological studies have shown a correlation between an increased risk of prostate cancer and the presence of short androgen-receptor polyglutamine repeats, while other studies have failed to detect such a correlation. Linkage data has also implicated an allelic form of CYP17, an enzyme that catalyzes key reactions in sex-steroid biosynthesis, with prostate cancer (Chang, B. et al., Int. J. Cancer 95:354-59 (2001)). Allelic variants of SRD5A2, which encodes the predominant isozyme of 5-$\alpha$-reductase in the prostate and functions to convert testosterone to the more potent dihydrotestosterone, have been associated with an increased risk of prostate cancer and with a poor prognosis for men with prostate cancer (Makridakis, N.M. et al., Lancet 354:975-78 (1999); Nam, R.K. et al., Urology 57:199-204 (2001)).

It is likely that a relatively high number of low-to-medium risk genetic variants contribute to risk of prostate cancer. These low-to-medium risk genetic variants may, however, be responsible for a substantial fraction of prostate cancer, and their identification, therefore, a great benefit for public health. Several such variants have been identified in the last two years, mainly through large-scale genome-wide association studies (Gudmundsson, J., et al. Nat Genet 40:281-283 (2008); Thomas, G., et al. Nat Genet 40:310-315 (2008); Gudmundsson, J., et al. Nat Genet 39:977-983 (2007); Yeager, M., et al Nat Genet 39:645-649 (2007); Gudmundsson, J., et al. Nat Genet 39:631-637 (2007); Amundadottir, L.T., et al. Nat Genet 38:652-658 (2007); Haiman, C.A., et al. Nat Genet 39:638-644 (2007)). **Colorectal Cancer** (CRC) is one of the most commonly diagnosed cancers and one of the leading causes of cancer mortality (Parkin DM, et.al. CA Cancer J Clin, 55,:74-108 (2005)). Cancers of the colon and rectum accounted for about 1 million new cases in 2002 (9.4% of cancer cases world-wide) and it affects men and women almost equally. The average lifetime risk for an individual in the US to develop CRC is 6% (Jemal A, et.al. CA Cancer J Clin., 56:106-30 (2006)). The prognosis is strongly associated with the stage of the disease at diagnosis; therefore, CRC screening presents an opportunity for early cancer detection and cancer prevention.

Colorectal cancer is a consequence of environmental exposures acting upon a background of genetically determined susceptibility. Studies indicate that 30 - 35% of colorectal cancer risk could be explained by genetic factors (Lichtenstein P, et.al. N Engl J Med, 343:78-85 (2000);) Peto J and Mack TM. Nat Genet, 26:411-4 (2000); Risch N. Cancer Epidemiol Biomarkers Prev, 10:733-41 (2001)). The analysis of cancer occurrence in relatives of cancer patients also lends strong evidence for genetic factors that increase the risk of cancer.

At present only a small percentage of the heritable risk of CRC is identified, usually through the investigation of rare cancer syndromes. High-penetrance mutations in several genes have been identified in rare hereditary colorectal cancer syndromes. The most common of these are the familial adenomatous polyposis (FAP) syndrome and hereditary non-polyposis colorectal cancer (HNPCC) or Lynch syndrome (LS). FAP, caused by mutations in the APC gene, is an autosomal dominant syndrome, characterized by early onset of multiple adenomatous polyps in the colon that eventually progress to cancer. LS is caused by mutations in DNA mismatch repair (MMR) genes and is considered to be the most common hereditary CRC syndrome, comprising approximately 3-5% of all CRCs (de la Chapelle, A. Fam Cancer, 4:233-7 (2005)).

The search for additional highly-penetrant CRC genes has not been fruitful and accumulating evidence supports the notion that no single susceptibility gene is likely to explain a large proportion of highly familial or early onset CRC. This has led to the currently favored hypothesis that most of the inherited CRC risk is due to multiple, low genetic risk variants. Each such variant would be expected to carry a small increase in risk; however, if the variant is common, it may contribute significantly to the population attributable risk (PAR).

**Cervical Cancer.** Cervical cancer (CC) is the second most common cancer and the third most frequent cause of cancer death in women, accounting for over 490,000 cases and nearly 300,000 deaths annually (Parkin, D. M., et al., CA Cancer J Clin, 55: 74-108 (2005)). CC used to be a major cause of death in women in child-bearing age in the US and Europe but with the introduction of the Papanicolaou (PAP) smear in the 1950s, the incidence of invasive cervical cancer declined dramatically. Currently, about 70% of cervical cancer deaths occur in low-to medium income countries where population-based screening has not been implemented and access to healthcare is poor. In 2008, an estimated 11,070 women in the United States will be diagnosed with CC, and an estimated 3,870 will die of the disease (SEER Cancer Statistics Review, 1975-2005. Bethesda: National Cancer Institute, (2007)). In certain populations and geographic areas of the United States, cervical cancer death rates are still high, in large part due to limited access to health care and cervical cancer screening.

CC is almost invariably associated with infection by an oncogenic subtype of Human Papillomavirus (HPV) (Munoz, N. J Clin Virol, 19: 1-5 (2000); Walboomers, J. M., et al., J Pathol, 189: 12-9 (1999)). The majority of cases, or close to 70% of cervical cancer worldwide, is caused by HPV16 and 18, while HPV45, 31, 33 and other less common variants are found in the remaining cases. Infection by HPV causes dysplastic lesions in the cervical epithelium that, in the great majority of cases, are self-limiting, demonstrating effective host immune response to the virally infected cells (zur Hausen, H., J Natl Cancer Inst, 92: 690-8 (2000)). However, in some cases, the immune system fails to clear the infection which may become chronic and eventually lead to growth of malignant cells and the development of invasive cancer. Several cofactors have been identified that slightly increase the risk of cervical cancer in HPV-infected individuals, e.g. previous chlamydia infection (Anttila, T., et al., JAMA, 285: 47-51 (2001)), multiple sexual partners and cigarette smoking (Murthy, N. S. and Mathew, A., Eur J Cancer Prev, 9: 5-14 (2000)).

Genetic factors have been shown to play a role in the development of CC. Studies based on the nationwide Swedish Family-Cancer Database suggested that close to 22% of CC risk could be attributed to genetic factors while shared environmental effects did not contribute to the disease (Czene, K., et al., Int J Cancer, 99: 260-6 (2002)). In a subsequent study, full and half-siblings were identified from the Family-Cancer Database and it was shown that the familial risk for full siblings was 1.84, compared with 1.40 for maternal and 1.27 for paternal half-siblings. These data were used to apportion familial risk for cervical tumors in full siblings into a heritable component, accounting for 64%, and an environmental component, accounting for 36% of the total risk (Hemminki, K., and Chen, B., Cancer Epidemiol Biomarkers Prev, 15: 1413-4 (2006)). Finally, a study of 18,199 women with invasive and/or in situ cervix cancers and 72,796 women free of cervical tumors suggested a heritable component of 71% and an environmental component of 29% in young familial cervical tumors (Couto, E., and Hemminki, K., Int J Cancer, 119: 2699-701 (2006)). Taken together, these studies show that genetic factors play a substantial role in cervical cancer development, possibly by affecting immunological mechanisms that help clear HPV infection.

While cytological examination of PAP smears is highly effective in detecting dysplastic lesions and early stage CC which can be effectively treated by cone operation, a fraction of cases present with a persistent infection or re-infection which may progress to invasive cancer (Schiffman, M., et al., Lancet, 370: 890-907 (2007)). These cases often need to be followed for years and subjected to repeated biopsies. There is an unmet clinical need to identify women with persistent or recurring infection that have the greatest risk of progressing to invasive CC. Such individuals might be subjected to a more rigorous follow-up protocols or advised on how to reduce the risk by lifestyle changes. Knowledge of the underlying genetic predisposition might be useful in evaluating risks of progression. Screening for susceptibility to CC might also be important in planning the clinical management of transplant recipients and other immunosuppressed individuals.

Recently, vaccines against the major oncogenic subtypes of HPV have been developed that hold a great promise in the battle against the disease (Cutts, F. T., et al., Bull World Health Organ, 85: 719-26 (2007)). However, the vaccines are only effective in women with no prior infection with HPV and therefore it will take decades before the majority of women are protected. Furthermore, until all oncogenic subtypes are included in the vaccine, some form of organized screening will be necessary in order to catch those cases.

Clearly, identification of markers and genes that are responsible for susceptibility to cancer is one of the major challenges facing oncology today. Some of the pathways underlying cancer are shared in different forms of cancer. As a consequence, genetic risk factors identified for one particular form of cancer may also represent a risk factor for other cancer types. Diagnostic and therapeutic methods utilizing these risk factors may therefore have a common utility. Accordingly, therapeutic measures developed to target such risk factors may have implications for cancer in general, and not necessarily only the cancer for which the risk factor is originally identified. There is a need to identify means for the early detection of individuals that have a genetic susceptibility to cancer so that more aggressive screening and intervention regimens may be instituted for the early detection and treatment of cancer. Cancer genes may also reveal key molecular pathways that may be manipulated (e.g., using small or large molecule weight drugs) and may lead to more effective treatments regardless of the cancer stage when a particular cancer is first diagnosed.

Recently, genome-wide association studies of several cancers have identified common genetic variants that associate with increased cancer risk (Gudmundsson, J., et al. Nat Genet 39:631-637 (2007); Stacey, S.N., et al., Nat Genet. 39:865-69 (2007); Yeager, M. et al. Nat Genet 39:645-649 (2007); Gudmundsson, J., et al. Nat Genet 39:977-983 (2007); Haiman, C.A., et al. Nat Genet 39:638-644 (2007); Eason, D.F., et al. Nature 447:1087-1093 (2007); Tomlinson, I., et al. Nat Genet 39:984-988 (2007); Gudbjartsson, D.F., et al. Nat Genet 40:886-891 (2008); Stacey, S.N., et al. Nat Genet 40:703-706 (2008); Thorgeirsson, T.E., et al. Nature 452:638-642 (2008); Gudmundsson, J., et al. Nat Genet 40:281-283 (2008); Eeles, R.A., et al. Nat Genet 40:316-321 (2008); Hung, R.J., et al. Nature 452:633-637 (2008); Amos, C.I., et al. Nat Genet 40:616-622 (2008); Thomas, G., et al. Nat Genet 40:310-315 (2008)). Notably, in most cases the reported variants seem to be specific to the particular cancer type under study. This tissue specificity even holds true in the region on chromosome 8q24, which has been found to associate with several different types of cancer. The present inventors have now surprisingly found that variants on chromosome 5p13.3 associate with risk of several cancer types.

## SUMMARY OF THE INVENTION

The present invention relates to methods of risk management of cancer, based on the discovery that certain genetic variants are correlated with risk of cancer. Thus, the invention includes methods of determining an increased susceptibility or increased risk of cancer, as well as methods of determining a decreased susceptibility of cancer, through evaluation of certain markers that have been found to be correlated with susceptibility of cancer in humans. Other aspects of the invention relate to methods of assessing prognosis of individuals diagnosed with cancer, methods of assessing the probability of response to a therapeutic agents or therapy for cancer, as well as methods of monitoring progress of treatment of individuals diagnosed with cancer.

In one aspect, the invention relates to a method of determining a susceptibility to a cancer in a human individual, selected from the group consisting of basal cell carcinoma, lung cancer, bladder cancer, prostate cancer, cervical cancer, endometrial cancer and cutaneous melanoma, comprising determining whether at least one at-risk allele in at least one polymorphic marker is present in a genotype dataset derived from a sample from the individual, wherein the at least one polymorphic marker is selected from markers rs401681, and markers in linkage disequilibrium therewith as defined in the claims, and wherein determination of the presence of the at least one at-risk allele is indicative of increased susceptibility to cancer in the individual.

The genotype dataset comprises in one embodiment information about marker identity and the allelic status of the individual for at least one allele of a marker, i.e. information about the identity of at least one allele of the marker in the individual. The genotype dataset may comprise allelic information (information about allelic status) about one or more marker, including two or more markers, three or more markers, five or more markers, ten or more markers, one hundred or more markers, and so on. In some embodiments, the genotype dataset comprises genotype information from a whole-genome assessment of the individual, that may include hundreds of thousands of markers, or even one million or more markers spanning the entire genome of the individual.

In general, polymorphic genetic markers lead to alternate sequences at the nucleic acid level. If the nucleic acid marker changes the codon of a polypeptide encoded by the nucleic acid, then the marker will also result in alternate sequence at the amino acid level of the encoded polypeptide (polypeptide markers). Determination of the identity of particular alleles at polymorphic markers in a nucleic acid or particular alleles at polypeptide markers comprises whether particular alleles are present at a certain position in the sequence. Sequence data identifying a particular allele at a marker comprises sufficient sequence to detect the particular allele. For single nucleotide polymorphisms (SNPs) or amino acid polymorphisms described herein, sequence data can comprise sequence at a single position, i.e. the identity of a nucleotide or amino acid at a single position within a sequence. The sequence data can optionally include information about sequence flanking the polymorphic site, which in the case of SNPs spans a single nucleotide.

In certain embodiments, it may be useful to determine the nucleic acid sequence for at least two polymorphic markers. In other embodiments, the nucleic acid sequence for at least three, at least four or at least five or more polymorphic markers is determined. Haplotype information can be derived from an analysis of two or more polymorphic markers. Thus, in certain embodiments, a further step is performed, whereby haplotype information is derived based on sequence data for at least two polymorphic markers.

Also disclosed a method of determining a susceptibility to cancer in a human individual, the method comprising obtaining nucleic acid sequence data about a human individual identifying both alleles of at least two polymorphic markers selected from rs401681, and markers in linkage disequilibrium therewith as defined in the claims, determine the identity of at least one haplotype based on the sequence data, and determine a susceptibility to cancer from the haplotype data.

In certain embodiments, determination of a susceptibility comprises comparing the nucleic acid sequence data to a database containing correlation data between the at least one polymorphic marker and susceptibility to cancer. In some embodiments, the database comprises at least one risk measure of susceptibility to cancer for the at least one marker. The sequence database can for example be provided as a look-up table that contains data that indicates the susceptibility of cancer for any one, or a plurality of, particular polymorphisms. The database may also contain data that indicates the susceptibility for a particular haplotype that comprises at least two polymorphic markers.

Obtaining nucleic acid sequence data can in certain embodiments comprise obtaining a biological sample from the human individual and analyzing sequence of the at least one polymorphic marker in nucleic acid in the sample. Analyzing sequence can comprise determining the presence or absence of at least one allele of the at least one polymorphic marker. Determination of the presence of a particular susceptibility allele *(e.g.,* an at-risk allele) is indicative of susceptibility to cancer in the human individual. Determination of the absence of a particular susceptibility allele is indicative that the particular susceptibility due to the at least one polymorphism is not present in the individual.

Susceptibility determined by the diagnostic methods of the invention can be reported to a particular entity. In some embodiments, the at least one entity is selected from the group consisting of the individual, a guardian of the individual, a genetic service provider, a physician, a medical organization, and a medical insurer.

In certain embodiments, determination of a susceptibility comprises comparing the nucleic acid sequence data to a database containing correlation data between the at least one polymorphic marker and susceptibility to cancer. In one such embodiment, the database comprises at least one risk measure of susceptibility to cancer for the at least one polymorphic marker. In another embodiment, the database comprises a look-up table containing at least one risk measure of the at least one condition for the at least one polymorphic marker.

In certain embodiments, obtaining nucleic acid sequence data comprises obtaining a biological sample from the human individual and analyzing sequence of the at least one polymorphic marker in nucleic acid in the sample. Analyzing sequence of the at least one polymorphic marker can comprise determining the presence or absence of at least one allele of the at least one polymorphic marker.

Certain embodiments relate to obtaining nucleic acid sequence data about at least two polymorphic markers selected from rs401681, and markers in linkage disequilibrium therewith as defined in the claims.

In certain embodiments a further step of assessing the frequency of at least one haplotype in the individual is performed. In such embodiments, two or more markers, including three, four, five, six, seven, eight, nine or ten or more markers can be included in the haplotype. In certain embodiments, the at least one haplotype comprises markers selected from the haplotype. In certain embodiments, the at least one haplotype comprises markers selected from rs401681, and markers in linkage disequilibrium therewith as defined in the claims. In certain such embodiments, the at least one haplotype is representative of the genomic structure of a particular genomic region (such as an LD block), to which any one of the above-mentioned markers reside.

The markers conferring risk of cancer, as described herein, can be combined with other genetic markers for cancer. Such markers are typically not in linkage disequilibrium with any one of the markers described herein, in particular marker rs401681. Any of the methods described herein can be practiced by combining the genetic risk factors described herein with additional genetic risk factors for cancer. Such additional risk factors are in certain embodiments risk factors for a particular type of cancer, i.e. cancer at a particular site. In certain other embodiments, such additional risk factors are susceptibility variants for multiple forms of cancer.

Thus, in certain embodiments, a further step is included, comprising determining whether at least one at-risk allele of at least one at-risk variant for cancer not in linkage disequilibrium with any one of the markers rs401681 are present in a sample comprising genomic DNA from a human individual or a genotype dataset derived from a human individual. In other words, genetic markers in other locations in the genome can be useful in combination with the markers of the present invention, so as to determine overall risk of cancer based on multiple genetic variants. In one embodiment, the at least one at-risk variant for cancer is not in linkage disequilibrium with marker rs2736098. Selection of markers that are not in linkage disequilibrium (not in LD) can be based on a suitable measure for linkage disequilibrium, as described further herein. In certain embodiments, markers that are not in linkage disequilibrium have values for the LD measure $r^2$ correlating the markers of less than 0.2. In certain other embodiments, markers that are not in LD have values for $r^2$ correlating the markers of less than 0.15, including less than 0.10, less than 0.05, less than 0.02 and less than 0.01. Other suitable numerical values for establishing that markers are not in LD are contemplated, including values bridging any of the above-mentioned values. In certain embodiments, multiple markers as described herein are determined to determine overall risk of cancer. Thus, in certain embodiments, an additional step is included, the step comprising determining whether at least one allele in each of at least two polymorphic markers is present in a sample comprising genomic DNA from a human individual or a genotype dataset derived from a human individual, wherein the presence of the at least one allele in the at least two polymorphic markers is indicative of an increased susceptibility to cancer. In one embodiment, the markers are selected from rs401681, and markers in linkage disequilibrium therewith as defined in the claims.

The genetic markers can also be combined with non-genetic information to establish overall risk for an individual. Thus, in certain embodiments, a further step is included, comprising analyzing non-genetic information to make risk assessment, diagnosis, or prognosis of the individual. The non-genetic information can be any information pertaining to the disease status of the individual or other information that can influence the estimate of overall risk of cancer for the individual. In one embodiment, the non-genetic information is selected from age, gender, ethnicity, socioeconomic status, previous disease diagnosis, medical history of subject, family history of cancer, biochemical measurements, and clinical measurements.

The invention also provides computer-implemented aspects.

The invention further provides an apparatus for determining a genetic indicator for cancer in a human individual, comprising:

a processor,

a computer readable memory having computer executable instructions adapted to be executed on the processor to analyze marker and/or haplotype information defined in the claims for at least one human individual with respect to cancer, and

generate an output based on the marker or haplotype information, wherein the output comprises a risk measure of the at least one marker or haplotype as a genetic indicator of cancer for the human individual. In one embodiment, the computer readable memory comprises data indicative of the frequency of at least one allele of at least one polymorphic marker as defined in the claims in a plurality of individuals diagnosed with cancer, and data indicative of the frequency of at the least one allele of at least one polymorphic marker or at least one haplotype in a plurality of reference individuals, and wherein a risk measure is based on a comparison of the at least one marker and/or haplotype status for the human individual to the data indicative of the frequency of the at least one marker and/or haplotype information for the plurality of individuals diagnosed with cancer. In one embodiment, the computer readable memory further comprises data indicative of a risk of developing cancer associated with at least one allele of at least one polymorphic marker or at least one haplotype, and wherein a risk measure for the human individual is based on a comparison of the at least one marker and/or haplotype status for the human individual to the risk associated with the at least one allele of the at least one polymorphic marker or the at least one haplotype. In another embodiment, the computer readable memory further comprises data indicative of the frequency of at least one allele of at least one polymorphic marker or at least one haplotype in a plurality of individuals diagnosed with cancer, and data indicative of the frequency of at the least one allele of at least one polymorphic marker or at least one haplotype in a plurality of reference individuals, and wherein risk of developing cancer is based on a comparison of the frequency of the at least one allele or haplotype in individuals diagnosed with cancer, and reference individuals. In a preferred embodiment, the at least one marker is selected from rs401681, and markers in linkage disequilibrium therewith as defined in the claims.

In another aspect, the invention relates to a method of identification of a marker for use in assessing susceptibility to cancer, the method comprising: identifying at least one polymorphic marker in linkage disequilibrium with rs401681; determining the genotype status of a sample of individuals diagnosed with, or having a susceptibility to, cancer; and determining the genotype status of a sample of control individuals; wherein a significant difference in frequency of at least one allele in at least one polymorphism in individuals diagnosed with, or having a susceptibility to, cancer, as compared with the frequency of the at least one allele in the control sample is indicative of the at least one polymorphism being useful for assessing susceptibility to cancer. Significant difference can be estimated on statistical analysis of allelic counts at certain polymorphic markers in cancer patients and controls. In one embodiment, a significant difference is based on a calculated P-value between cancer patients and controls of less than 0.05. In other embodiments, a significant difference is based on a lower value of the calculated P-value, such as less than 0.005, 0.0005, or less than 0.00005. In one embodiment, an increase in frequency of the at least one allele in the at least one polymorphism in individuals diagnosed with, or having a susceptibility to, cancer, as compared with the frequency of the at least one allele in the control sample is indicative of the at least one polymorphism being useful for assessing increased susceptibility to cancer. In another embodiment, a decrease in frequency of the at least one allele in the at least one polymorphism in individuals diagnosed with, or having a susceptibility to, cancer, as compared with the frequency of the at least one allele in the control sample is indicative of the at least one polymorphism being useful for assessing decreased susceptibility to, or protection against, cancer.

The invention also relates to a method of genotyping a nucleic acid sample obtained from a human individual comprising determining whether at least one allele of at least one polymorphic marker is present in a nucleic acid sample from the individual sample, wherein the at least one marker is selected from rs401681, and markers in linkage disequilibrium therewith as defined in the claims, and wherein determination of the presence of the at least one allele in the sample is indicative of a susceptibility to cancer in the individual. In one embodiment, determination of the presence of allele C of rs401681 is indicative of increased susceptibility of cancer in the individual. Alternatively, marker alleles in linkage disequilibrium with allele C of rs401681, are indicative of increased susceptibility of the cancer. In another embodiment, determination of the presence of allele C of rs401681, or marker alleles in linkage disequilibrium therewith as defined in the claims is indicative of a decreased susceptibility of melanoma cancer or colorectal cancer in an individual. In one embodiment, genotyping comprises amplifying a segment of a nucleic acid that comprises the at least one polymorphic marker by Polymerase Chain Reaction (PCR), using a nucleotide primer pair flanking the at least one polymorphic marker. In another embodiment, genotyping is performed using a process selected from allele-specific probe hybridization, allele-specific primer extension, allele-specific amplification, nucleic acid sequencing, 5'-exonuclease digestion, molecular beacon assay, oligonucleotide ligation assay, size analysis, single-stranded conformation analysis and microarray technology. In one embodiment, the microarray technology is Molecular Inversion Probe array technology or BeadArray Technologies. In one embodiment, the process comprises allele-specific probe hybridization. In another embodiment, the process comprises microarray technology. One preferred embodiment comprises the steps of (1) contacting copies of the nucleic acid with a detection oligonucleotide probe and an enhancer oligonucleotide probe under conditions for specific hybridization of the oligonucleotide probe with the nucleic acid; wherein (a) the detection oligonucleotide probe is from 5-100 nucleotides in length and specifically hybridizes to a first segment of a nucleic acid whose nucleotide sequence is set forth in SEQ ID NO:1; (b) the detection oligonucleotide probe comprises a detectable label at its 3' terminus and a quenching moiety at its 5' terminus; (c) the enhancer oligonucleotide is from 5-100 nucleotides in length and is complementary to a second segment of the nucleotide sequence that is 5' relative to the oligonucleotide probe, such that the enhancer oligonucleotide is located 3' relative to the detection oligonucleotide probe when both oligonucleotides are hybridized to the nucleic acid; and (d) a single base gap exists between the first segment and the second segment, such that when the oligonucleotide probe and the enhancer oligonucleotide probe are both hybridized to the nucleic acid, a single base gap exists between the oligonucleotides; (2) treating the nucleic acid with an endonuclease that will cleave the detectable label from the 3' terminus of the detection probe to release free detectable label when the detection probe is hybridized to the nucleic acid; and (3) measuring free detectable label, wherein the presence of the free detectable label indicates that the detection probe specifically hybridizes to the first segment of the nucleic acid, and indicates the sequence of the polymorphic site as the complement of the detection probe.

The present disclosure, in its broadest sense relates to cancer selected from Basal Cell Carcinoma, Lung Cancer, Bladder Cancer, Prostate Cancer, Cervical Cancer, Thyroid Cancer, Cutaneous Melanoma, Colorectal Cancer and Endometrial Cancer. Any combinations of these cancer types are also contemplated. Also, the present disclosure is contemplated to relate to any particular sub-phenotype of these cancer types.

In some embodiments of the methods described therein, the susceptibility determined in the method is increased susceptibility. In one such embodiment, the increased susceptibility is characterized by a relative risk (RR) of at least 1.08. In another embodiment, the increased susceptibility is characterized by a relative risk of at least 1.10. In another embodiment, the increased susceptibility is characterized by a relative risk of at least 1.11. In another embodiment, the increased susceptibility is characterized by a relative risk of at least 1.12. In yet another embodiment, the increased susceptibility is characterized by a relative risk of at least 1.13. In a further embodiment, the increased susceptibility is characterized by a relative risk of at least 1.14. In a further embodiment, the increased susceptibility is characterized by a relative risk of at least 1.15. In yet another embodiment, the increased susceptibility is characterized by a relative risk of at least 1.20. Certain other embodiments are characterized by relative risk of the at-risk variant of at least 1.16, 1.17, 1.18, 1.19, 1.21, 1.22, 1.23, 1.24, 1.25, 1.26, 1.27, 1.28, 1.29, 1.30, and so on. Other numeric values of odds ratios, including those bridging any of these above-mentioned values are also possible, and these are also within scope of the invention.

In some embodiments of the methods described therein, the susceptibility determined in the method is decreased susceptibility. In one such embodiment, the decreased susceptibility is characterized by a relative risk (RR) of less than 0.9. In another embodiment, the decreased susceptibility is characterized by a relative risk (RR) of less than 0.85. In another embodiment, the decreased susceptibility is characterized by a relative risk (RR) of less than 0.8. In yet another embodiment, the decreased susceptibility is characterized by a relative risk (RR) of less than 0.75. Other cutoffs, such as relative risk of less than 0.89, 0.88, 0.87, 0.86, 0.84, 0.83, 0.82, 0.81, 0.79, and so on, are also contemplated and are within scope of the invention.

Also disclosed kit for assessing susceptibility to cancer in a human individual. The kit comprises reagents necessary for selectively detecting at least one allele of at least one polymorphic marker selected from rs401681, and markers in linkage disequilibrium therewith as defined in the claims, in the genome of the individual, wherein the presence of the at least one allele is indicative of increased susceptibility to cancer. Another kit for assessing susceptibility to cancer in a human individual comprises reagents for selectively detecting at least one allele of at least one polymorphic marker in the genome of the individual, wherein the polymorphic marker is selected from rs401681, and wherein the presence of the at least one allele is indicative of a susceptibility to cancer. In certain embodiments, the kit further comprises a collection of data comprising correlation data between the polymorphic markers assessed by the kit and susceptibility to the cancer.

Kit reagents may comprise at least one contiguous oligonucleotide that hybridizes to a fragment of the genome of the individual comprising the at least one polymorphic marker. In another embodiment, the kit comprises at least one pair of oligonucleotides that hybridize to opposite strands of a genomic segment obtained from the subject, wherein each oligonucleotide primer pair is designed to selectively amplify a fragment of the genome of the individual that includes one polymorphism, wherein the polymorphism is selected from the group consisting of the polymorphisms as defined in Table 5, Table 6 and Table 7, and wherein the fragment is at least 20 base pairs in size. In one embodiment, the oligonucleotide is completely complementary to the genome of the individual. In another embodiment, the kit further contains buffer and enzyme for amplifying said segment. In another embodiment, the reagents further comprise a label for detecting said fragment.

Kits may also be used in other methods , including methods of assessing risk of developing at least a second primary tumor in an individual previously diagnosed with cancer, methods of assessing an individual for probability of response to a cancer therapeutic agent, and methods of monitoring progress of a treatment of an individual diagnosed with cancer and given a treatment for the disease.

In certain embodiments of the methods, apparatus of the invention, the at least one polymorphic marker that provides information about susceptibility to cancer is associated with the TERT gene. Being "associated with", in this context, means that the at least one marker is in linkage disequilibrium with the TERT gene or its regulatory regions. Such markers can be located within the TERT gene, or its regulatory regions, or they can be in linkage disequilibrium with at least one marker within the TERT gene or its regulatory region that has a direct impact on the function of the gene. The functional consequence of the susceptibility variants associated with the TERT can be on the expression level of the TERT gene, the stability of its transcript or through amino acid alterations at the protein level, as described in more detail herein. Exemplary markers associated with the TERT gene are indicated in Table 8 herein, and certain embodiments relate to any one or more of those markers.

In certain other embodiments, the at least one polymorphic marker is associated with the CLPTM1L gene.

The skilled person will realize that the markers that are described herein to be associated with cancer can all be used in the various aspects of the invention, including the methods, uses, apparatus, procedures described herein. In certain other embodiments, the invention relates to marker rs401681, , and markers in linkage disequilibrium therewith as defined in the claims.

In certain embodiments, the at least one marker allele conferring increased risk of cancer is rs401681 allele C (SEQ ID NO:2),. In these embodiments, the presence of the allele (the at-risk allele) is indicative of increased risk of cancer.

In certain embodiments, the at least one marker allele conferring a decreased risk of cancer is selected from rs401681 allele C, and marker alleles in linkage disequilibrium therewith, and wherein the cancer is melanoma cancer and/or colorectal cancer.

Linkage disequilibrium can be determined using the linkage disequilibrium measures $r^2$ and |D'|, which give a quantitative measure of the extent of linkage disequilibrium (LD) between two genetic element (*e.g.*, polymorphic markers). Certain numerical values of these measures for particular markers are indicative of the markers being in linkage disequilibrium, as described further herein. In one embodiment of the invention, linkage disequilibrium between marker (*i.e.*, LD values indicative of the markers being in linkage disequilibrium) is defined as $r^2 > 0.1$. In another embodiment, linkage disequilibrium is defined as $r^2 > 0.2$. Other embodiments can include other definitions of linkage disequilibrium, such as $r^2 > 0.25$, $r^2>0.3$, $r^2>0.35$, $r^2>0.4$, $r^2>0.4^5$, $r^2>0.5$, $r^2>0.55$, $r^2>0.6$, $r^2>0.65$, $r^2>0.7$, $r^2> 0.75$, $r^2 > 0.8$, $r^2 > 0.85$, $r^2 > 0.9$, $r^2 > 0.95$, $r^2 > 0.96$, $r^2 > 0.97$, $r^2 > 0.98$, or $r^2 > 0.99$. Linkage disequilibrium can in certain embodiments also be defined as |D'| > 0.2, or as |D'| > 0.3, |D'| > 0.4, | D'| > 0.5, |D'| > 0.6, |D'| > 0.7, |D'| > 0.8, | D'| > 0.9, |D'| > 0.95, |D'| > 0.98 or |D'| > 0.99. In certain embodiments, linkage disequilibrium is defined as fulfilling two criteria of $r^2$ and |D'|, such as $r^2 > 0.2$ and |D'| > 0.8. Other combinations of values for $r^2$ and |D'| are also possible.

It should be understood that all combinations of features described herein are contemplated, even if the combination of feature is not specifically found in the same sentence or paragraph herein. This includes in particular the use of all markers disclosed herein, alone or in combination, for analysis individually or in haplotypes, in all aspects of the invention as described herein, as well as any particular cancer type (cancer at particular site), or combination of cancer types.

## BRIEF DESCRIPTION OF THE DRAWINGS

The foregoing and other objects, features and advantages of the invention will be apparent from the following more particular description of preferred embodiments of the invention.

**FIG 1** provides a diagram illustrating a computer-implemented system utilizing risk variants as described herein.

**FIG 2** A) shows a pair-wise correlation structure in a 200 kb interval (1.225 - 1.425 Mb, NCBI B36) on chromosome 5. The upper plot shows pair-wise D' for 100 common SNPs (with minor allelic frequency > 5%) from the HapMap (v22) CEU dataset. The lower plot shows the corresponding $r^2$ values; B) shows estimated recombination rates (saRR) in cM / Mb from the HapMap Phase II data (Frazer, K.A. et al. Nature 449, 851-61 (2007).); C) shows location of known genes in the region; D) shows chematic view of the association with basal cell carcinoma (BCC) in the Icelandic discovery sample set for directly genotyped SNPs (blue dots) and imputed SNPs (red dots).

**FIG 3** shows observed telomere length, as measured by quantitative PCR, as a function of SNP genotype for a) women born between 1925 and 1935 as a function of rs401681 genotype, b) women born between 1925 and 1935 as a function of rs2736098 genotype, c) women born between 1940 and 1950 as a function of rs401681 genotype, d) women born between 1940 and 1950 as a function of rs2736098 genotype.

## DETAILED DESCRIPTION

*Definitions*

Unless otherwise indicated, nucleic acid sequences are written left to right in a 5' to 3' orientation. Numeric ranges recited within the specification are inclusive of the numbers defining the range and include each integer or any non-integer fraction within the defined range. Unless defined otherwise, all technical and scientific terms used herein have the same meaning as commonly understood by the ordinary person skilled in the art to which the invention pertains.

The following terms shall, in the present context, have the meaning as indicated:

A "polymorphic marker", sometime referred to as a "marker", as described herein, refers to a genomic polymorphic site. Each polymorphic marker has at least two sequence variations characteristic of particular alleles at the polymorphic site. Thus, genetic association to a polymorphic marker implies that there is association to at least one specific allele of that particular polymorphic marker. The marker can comprise any allele of any variant type found in the genome, including SNPs, mini- or microsatellites, translocations and copy number variations (insertions, deletions, duplications). Polymorphic markers can be of any measurable frequency in the population. For mapping of disease genes, polymorphic markers with population frequency higher than 5-10% are in general most useful. However, polymorphic markers may also have lower population frequencies, such as 1-5% frequency, or even lower frequency, in particular copy number variations (CNVs). The term shall, in the present context, be taken to include polymorphic markers with any population frequency.

An "allele" refers to the nucleotide sequence of a given locus (position) on a chromosome. A polymorphic marker allele thus refers to the composition (i.e., sequence) of the marker on a chromosome. Genomic DNA from an individual contains two alleles (e.g., allele-specific sequences) for any given polymorphic marker, representative of each copy of the marker on each chromosome. Sequence codes for nucleotides used herein are: A = 1, C = 2, G = 3, T = 4. For microsatellite alleles, the CEPH sample (Centre d'Etudes du Polymorphisme Humain, genomics repository, CEPH sample 1347-02) is used as a reference, the shorter allele of each microsatellite in this sample is set as 0 and all other alleles in other samples are numbered in relation to this reference. Thus, e.g., allele 1 is 1 bp longer than the shorter allele in the CEPH sample, allele 2 is 2 bp longer than the shorter allele in the CEPH sample, allele 3 is 3 bp longer than the lower allele in the CEPH sample, etc., and allele -1 is 1 bp shorter than the shorter allele in the CEPH sample, allele -2 is 2 bp shorter than the shorter allele in the CEPH sample, etc.

Sequence conucleotide ambiguity as described herein, including sequence listing, is as proposed by IUPAC-IUB. These codes are compatible with the codes used by the EMBL, GenBank, and PIR databases.

| IUB code | Meaning |
|---|---|
| A | Adenosine |
| C | Cytidine |
| G | Guanine |
| T | Thymidine |
| R | G or A |
| Y | T or C |
| K | G or T |
| M | A or C |
| S | G or C |
| W | A or T |
| B | C, G or T |

(continued)

| IUB code | Meaning |
|---|---|
| D | A, G or T |
| H | A, C or T |
| V | A, C or G |
| N | A, C, G or T (Any base) |

A nucleotide position at which more than one sequence is possible in a population (either a natural population or a synthetic population, *e.g.*, a library of synthetic molecules) is referred to herein as a "polymorphic site".

A "Single Nucleotide Polymorphism" or "SNP" is a DNA sequence variation occurring when a single nucleotide at a specific location in the genome differs between members of a species or between paired chromosomes in an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The SNP nomenclature as reported herein refers to the official Reference SNP (rs) ID identification tag as assigned to each unique SNP by the National Center for Biotechnological Information (NCBI).

A "variant", as described herein, refers to a segment of DNA that differs from the reference DNA. A "marker" or a "polymorphic marker", as defined herein, is a variant. Alleles that differ from the reference are referred to as "variant" alleles.

A "microsatellite" is a polymorphic marker that has multiple small repeats of bases that are 2-8 nucleotides in length (such as CA repeats) at a particular site, in which the number of repeat lengths varies in the general population. An "indel" is a common form of polymorphism comprising a small insertion or deletion that is typically only a few nucleotides long.

A "haplotype," as described herein, refers to a segment of genomic DNA that is characterized by a specific combination of alleles arranged along the segment. For diploid organisms such as humans, a haplotype comprises one member of the pair of alleles for each polymorphic marker or locus along the segment. In a certain embodiment, the haplotype can comprise two or more alleles, three or more alleles, four or more alleles, or five or more alleles. Haplotypes are described herein in the context of the marker name and the allele of the marker in that haplotype, e.g., "3 rs401681" refers to the 3 allele of marker rs401681 being in the haplotype, and is equivalent to "rs401681 allele 3". Furthermore, allelic codes in haplotypes are as for individual markers, i.e. 1 = A, 2 = C, 3 = G and 4 = T.

The term "susceptibility", as described herein, refers to the proneness of an individual towards the development of a certain state (*e.g.*, a certain trait, phenotype or disease), or towards being less able to resist a particular state than the average individual. The term encompasses both increased susceptibility and decreased susceptibility. Thus, particular alleles at polymorphic markers and/or haplotypes of the invention as described herein may be characteristic of increased susceptibility (i.e., increased risk) of cancer, as characterized by a relative risk (RR) or odds ratio (OR) of greater than one for the particular allele or haplotype. Alternatively, the markers and/or haplotypes of the invention are characteristic of decreased susceptibility (i.e., decreased risk) of cancer, as characterized by a relative risk of less than one.

The term "and/or" shall in the present context be understood to indicate that either or both of the items connected by it are involved. In other words, the term herein shall be taken to mean "one or the other or both".

The term "look-up table", as described herein, is a table that correlates one form of data to another form, or one or more forms of data to a predicted outcome to which the data is relevant, such as phenotype or trait. For example, a look-up table can comprise a correlation between allelic data for at least one polymorphic marker and a particular trait or phenotype, such as a particular disease diagnosis, that an individual who comprises the particular allelic data is likely to display, or is more likely to display than individuals who do not comprise the particular allelic data. Look-up tables can be multidimensional, *i.e,* they can contain information about multiple alleles for single markers simultaneously, or the can contain information about multiple markers, and they may also comprise other factors, such as particulars about diseases diagnoses, racial information, biomarkers, biochemical measurements, therapeutic methods or drugs, etc.

A "computer-readable medium", is an information storage medium that can be accessed by a computer using a commercially available or custom-made interface. Exemplary computer-readable media include memory (*e.g.*, RAM, ROM, flash memory, etc.), optical storage media (*e.g.*, CD-ROM), magnetic storage media (*e.g.*, computer hard drives, floppy disks, etc.), punch cards, or other commercially available media. Information may be transferred between a system of interest and a medium, between computers, or between computers and the computer-readable medium for storage or access of stored information. Such transmission can be electrical, or by other available methods, such as IR links, wireless connections, etc.

A "nucleic acid sample" as described herein, refers to a sample obtained from an individual that contains nucleic acid (DNA or RNA). In certain embodiments, i.e. the detection of specific polymorphic markers and/or haplotypes, the nucleic acid sample comprises genomic DNA. Such a nucleic acid sample can be obtained from any source that contains genomic DNA, including a blood sample, sample of amniotic fluid, sample of cerebrospinal fluid, or tissue sample from skin, muscle, buccal or conjunctival mucosa, placenta, gastrointestinal tract or other organs.

The term "cancer therapeutic agent" refers to an agent that can be used to ameliorate or prevent symptoms associated with cancer.

The term "cancer-associated nucleic acid", as described herein, refers to a nucleic acid that has been found to be associated to cancer. This includes, but is not limited to, the markers and haplotypes described herein and markers and haplotypes in strong linkage disequilibrium (LD) therewith. In one embodiment, a cancer-associated nucleic acid refers to a genomic region, such as an LD-block, found to be associated with risk of cancer through at least one polymorphic marker located within the region or LD block.

The term "CLPTM1L" or "CLPTM1L gene", as described herein, refers to the Cisplatin Resistance Related Protein on chromosome 5p13.3. The gene is also known as CLPTM1-like and CRR9p.

The term "TERT" or "TERT gene", as described herein, refers to the Telomerase Reverse Transcriptase gene on chromosome 5p13.3.

The present inventors have discovered that variants on chromosome 5p13.3 associate with cancer at multiple sites. As shown in Tables 1-3 and 16 herein, the rs401681, rs2736100 and rs2736098 variants associate with risk of a variety of cancers, including Basal Cell Carcinoma, Lung Cancer, Bladder Cancer, Prostate Cancer, Cervical Cancer, Thyroid Cancer and Endometrial cancer. The most significant association was observed for Basal Cell Carcinoma (OR=1.27, P=$7.96 \times 10^{-11}$ in Iceland for marker rs401681). The most significant site after BCC was lung cancer, reaching genome wide significance (OR=1.15, P=$8.55 \times 10^{-8}$ for rs401681) in the combined analysis of 4 populations from Iceland, the Netherlands and Spain in addition to the dataset from the IARC. Risk for the different cancers is comparable, ranging in most cases from 1.10 - 1.25.

The two known, genes in the region showing association to cancer, *CLPTM1L* and *TERT,* have both previously been studied in the context of cancer. *CLPTM1L* was identified in an ovarian cancer model as a gene that affected cisplatin-induced apoptosis but has not been extensively studied (Yamamoto, K., et al. Biochem Biophys Res Comm 280:1148-54 (2001). The *TERT* gene plays a leading role in maintenance of functional telomeres and has been firmly established as a key gene in cancer development. In particular, the well-documented association between telomere function and environmental insults such as radiation suggests a potential link between *TERT* and predisposition to BCC (reviewed in Ayouaz, A., et al. Biochimie 90:60-72 (2008)).

Based on these results, any one of the markers rs401681, rs2736100 and rs2736098 can be used to assess susceptibility to cancer, as described further herein. Furthermore, markers in linkage disequilibrium (LD) with these markers are equally useful in such applications. For example, the markers set forth in Tables 5, 6 and 7 represent markers in LD with the rs401681, rs2736100 and rs2736098 markers, respectively. Thus, in certain embodiments, markers in linkage disequilibrium with rs401681 are suitably selected from the group consisting of the markers listed in Table 5 herein.

Marker alleles that were found to be indicative of increased risk of several cancer types were found to be indicative of decreased risk of particular cancers, i.e. melanoma cancer and colorectal cancer. Thus, allele C of rs401681, which is indicative of increased risk of several cancers as shown herein was found to be indicative of decreased risk of melanoma cancer and colorectal cancer, i.e. the marker allele is protective for these particular cancers.

Telomeres are specific functional structures at the ends of eukaryotic chromosomes which are indispensable for chromosome protection and integrity (Collins, K. Curr Opin Cell Biol, 12: 378-83 (2000)). In proliferating cells lacking telomerase activity, telomeres progressively shorten with every cell division due to the end-replication problem and replication-associated erosion (Allsopp, R. C., et al. Proc Natl Acad Sci U S A, 89: 10114-8 (1992)). Eventually, when telomeres are shortened and no longer protective, cells exit the cell cycle and enter a non-replicative state termed senescence ( Campisi, J. Eur J Cancer, 33: 703-9 (1997)). Telomerase, a unique ribonucleoprotein with reverse transcriptase activity, catalyzes the *de novo* addition of telomeric repeat sequences onto the eroding chromosome ends and thereby counterbalances telomere-dependent replicative aging ( Greider, C. W., and Blackburn, E. H. Cell, 43: 405-13 (1985)). Telomerase is repressed in most human somatic cells, but reactivated in more than 80% of all human cancers (reviewed in Deng, Y., and Chang, S. Lab Invest, 87: 1071-6 (2007))). Because of its involvement in carcinogenesis, telomerase is a promising candidate as both tumor marker and therapeutic target for telomerase inhibitors or antisense constructs ( Harley, C. B. Nat Rev Cancer, 8: 167-79 (2008)).

The length of telomeric sequences is inversely related to age, reflecting the progressive shortening with each cell division. Thus, the average telomere size in peripheral blood cells and colorectal mucosa epithelia from older individuals was found to be shorter than that from younger individuals, corresponding to a rate of telomere loss of 33 bp/year (Hastie, N. D., et al. Nature, 346: 866-8 (1990)). However, telomere length also varies considerably between individuals in the same age group and it has been shown that this variation is to a large extent genetically determined ( Slagboom, P. E., et al. Am J Hum Genet, 55: 876-82 (1994)). Recently, multiple studies have reported an association between short telomeres and increased risk of cancer at several sites, including lung, head and neck, bladder, kidney and breast (Wu, X., et al. J Natl Cancer Inst, 95: 1211-8 (2003); Shen, J., et al. Cancer Res, 67: 5538-44 (2007); Jang, J. S., et al. Cancer Sci, 99: 1385-9 (2008)) . These findings suggest that the genetic factors that determine telomere length may also affect the risk for multiple types of cancer.

Telomeres are directly affected by several stimuli that are known risk factors for cancer. Telomere shortening is accelerated in response to oxidative stress caused by environmental factors such as radiation and cigarette smoke ( Ayouaz, A., et al. Biochimie, 90: 60-72 (2008); McGrath, M., et al. Cancer Epidemiol Biomarkers Prev, 16: 815-9 (2007)) and chronic psychological stress has been associated with telomere shortening ( Epel, E. S., et al. Proc Natl Acad Sci U S A, 101: 17312-5 (2004)).

In light of this biological context, it is possible that the biological effect of the association to cancer described herein is through an effect on the *TERT* gene. Thus, markers within the gene, or markers in linkage disequilibrium with the gene (such as rs401681, rs2736100 and rs2736098) can be used to assess susceptibility to cancer. Furthermore, other markers within or in near proximity to the *TERT* gene, such as the markers set forth in Tables 8 and 9 herein, may represent variants with comparable or even more significant association to cancer (represented by larger OR values). Such variants are also useful for assessing susceptibility to cancer, and are within scope of the present invention.

*Assessment for markers and haplotypes*

The genomic sequence within populations is not identical when individuals are compared. Rather, the genome exhibits sequence variability between individuals at many locations in the genome. Such variations in sequence are commonly referred to as polymorphisms, and there are many such sites within each genome. For example, the human genome exhibits sequence variations which occur on average every 500 base pairs. The most common sequence variant consists of base variations at a single base position in the genome, and such sequence variants, or polymorphisms, are commonly called Single Nucleotide Polymorphisms ("SNPs"). These SNPs are believed to have occurred in a single mutational event, and therefore there are usually two possible alleles possible at each SNPsite; the original allele and the mutated allele. Due to natural genetic drift and possibly also selective pressure, the original mutation has resulted in a polymorphism characterized by a particular frequency of its alleles in any given population. Many other types of sequence variants are found in the human genome, including mini- and microsatellites, and insertions, deletions and inversions (also called copy number variations (CNVs)). A polymorphic microsatellite has multiple small repeats of bases (such as CA repeats, TG on the complimentary strand) at a particular site in which the number of repeat lengths varies in the general population. In general terms, each version of the sequence with respect to the polymorphic site represents a specific allele of the polymorphic site. These sequence variants can all be referred to as polymorphisms, occurring at specific polymorphic sites characteristic of the sequence variant in question. In general terms, polymorphisms can comprise any number of specific alleles. Thus in one embodiment of the invention, the polymorphism is characterized by the presence of two or more alleles in any given population. In another embodiment, the polymorphism is characterized by the presence of three or more alleles. In other embodiments, the polymorphism is characterized by four or more alleles, five or more alleles, six or more alleles, seven or more alleles, nine or more alleles, or ten or more alleles. All such polymorphisms can be utilized in the methods and kits of the present invention, and are thus within the scope of the invention.

Due to their abundance, SNPs account for a majority of sequence variation in the human genome. Over 6 million SNPs have been validated to date (http://www.ncbi.nlm.nih.gov/projects/SNP/snp summary.cgi). However, CNVs are receiving increased attention. These large-scale polymorphisms (typically 1kb or larger) account for polymorphic variation affecting a substantial proportion of the assembled human genome; known CNVs covery over 15% of the human genome sequence (Estivill, X Armengol; L., PloS Genetics 3:1787-99 (2007). A http://projects.tcag.ca/variation/). Most of these polymorphisms are however very rare, and on average affect only a fraction of the genomic sequence of each individual. CNVs are known to affect gene expression, phenotypic variation and adaptation by disrupting gene dosage, and are also known to cause disease (microdeletion and microduplication disorders) and confer risk of common complex diseases, including HIV-1 infection and glomerulonephritis (Redon, R., et al. Nature 23:444-454 (2006)). It is thus possible that either previously described or unknown CNVs represent causative variants in linkage disequilibrium with the markers described herein to be associated with cancer. Methods for detecting CNVs include comparative genomic hybridization (CGH) and genotyping, including use of genotyping arrays, as described by Carter (Nature Genetics 39:S16-S21 (2007)). The Database of Genomic Variants (http://projects.tcag.ca/variation/) contains updated information about the location, type and size of described CNVs. The database currently contains data for over 15,000 CNVs.

In some instances, reference is made to different alleles at a polymorphic site without choosing a reference allele. Alternatively, a reference sequence can be referred to for a particular polymorphic site. The reference allele is sometimes referred to as the "wild-type" allele and it usually is chosen as either the first sequenced allele or as the allele from a "non-affected" individual (e.g., an individual that does not display a trait or disease phenotype).

Alleles for SNP markers as referred to herein refer to the bases A, C, G or T as they occur at the polymorphic site in the SNP assay employed. The allele codes for SNPs used herein are as follows: 1= A, 2=C, 3=G, 4=T. The person skilled in the art will however realise that by assaying or reading the opposite DNA strand, the complementary allele can in each case be measured. Thus, for a polymorphic site (polymorphic marker) characterized by an A/G polymorphism, the assay employed may be designed to specifically detect the presence of one or both of the two bases possible, i.e. A and G. Alternatively, by designing an assay that is designed to detect the complimentary strand on the DNA template, the presence of the complementary bases T and C can be measured. Quantitatively (for example, in terms of relative risk), identical results would be obtained from measurement of either DNA strand (+ strand or - strand).

Typically, a reference sequence is referred to for a particular sequence. Alleles that differ from the reference are sometimes referred to as "variant" alleles. A variant sequence, as used herein, refers to a sequence that differs from the reference sequence but is otherwise substantially similar. Alleles at the polymorphic genetic markers described herein are variants. Variants can include changes that affect a polypeptide. Sequence differences, when compared to a reference nucleotide sequence, can include the insertion or deletion of a single nucleotide, or of more than one nucleotide, resulting in a frame shift; the change of at least one nucleotide, resulting in a change in the encoded amino acid; the change of at least one nucleotide, resulting in the generation of a premature stop codon; the deletion of several nucleotides, resulting in a deletion of one or more amino acids encoded by the nucleotides; the insertion of one or several nucleotides, such as by unequal recombination or gene conversion, resulting in an interruption of the coding sequence of a reading frame; duplication of all or a part of a sequence; transposition; or a rearrangement of a nucleotide sequence,. Such sequence changes can alter the polypeptide encoded by the nucleic acid. For example, if the change in the nucleic acid sequence causes a frame shift, the frame shift can result in a change in the encoded amino acids, and/or can result in the generation of a premature stop codon, causing generation of a truncated polypeptide. Alternatively, a polymorphism associated with a disease or trait can be a synonymous change in one or more nucleotides (*i.e.*, a change that does not result in a change in the amino acid sequence). Such a polymorphism can, for example, alter splice sites, affect the stability or transport of mRNA, or otherwise affect the transcription or translation of an encoded polypeptide. It can also alter DNA to increase the possibility that structural changes, such as amplifications or deletions, occur at the somatic level. The polypeptide encoded by the reference nucleotide sequence is the "reference" polypeptide with a particular reference amino acid sequence, and polypeptides encoded by variant alleles are referred to as "variant" polypeptides with variant amino acid sequences.

A haplotype refers to a segment of DNA that is characterized by a specific combination of alleles arranged along the segment. For diploid organisms such as humans, a haplotype comprises one member of the pair of alleles for each polymorphic marker or locus. In a certain embodiment, the haplotype can comprise two or more alleles, three or more alleles, four or more alleles, or five or more alleles, each allele corresponding to a specific polymorphic marker along the segment. Haplotypes can comprise a combination of various polymorphic markers, e.g., SNPs and microsatellites, having particular alleles at the polymorphic sites. The haplotypes thus comprise a combination of alleles at various genetic markers.

Detecting specific polymorphic markers and/or haplotypes can be accomplished by methods known in the art for detecting sequences at polymorphic sites. For example, standard techniques for genotyping for the presence of SNPs and/or microsatellite markers can be used, such as fluorescence-based techniques (*e.g.*, Chen, X. et al., Genome Res. 9(5): 492-98 (1999); Kutyavin et al., Nucleic Acid Res. 34:e128 (2006)), utilizing PCR, LCR, Nested PCR and other techniques for nucleic acid amplification. Specific commercial methodologies available for SNP genotyping include, but are not limited to, TaqMan genotyping assays and SNPlex platforms (Applied Biosystems), gel electrophoresis (Applied Biosystems), mass spectrometry (*e.g.*, MassARRAY system from Sequenom), minisequencing methods, real-time PCR, Bio-Plex system (BioRad), CEQ and SNPstream systems (Beckman), array hybridization technology(*e.g.*, Affymetrix GeneChip; Perlegen), BeadArray Technologies (*e.g.*, Illumina GoldenGate and Infinium assays), array tag technology (*e.g.*, Parallele), and endonuclease-based fluorescence hybridization technology (Invader; Third Wave). Some of the available array platforms, including Affymetrix SNP Array 6.0 and Illumina CNV370-Duo and 1M BeadChips, include SNPs that tag certain CNVs. This allows detection of CNVs via surrogate SNPs included in these platforms. Thus, by use of these or other methods available to the person skilled in the art, one or more alleles at polymorphic markers, including microsatellites, SNPs or other types of polymorphic markers, can be identified.

In the present context, and individual who is at an increased susceptibility (i.e., increased risk) for a disease, is an individual in whom at least one specific allele at one or more polymorphic marker or haplotype conferring increased susceptibility (increased risk) for the disease is identified (i.e., at-risk marker alleles or haplotypes). The at-risk marker or haplotype is one that confers an increased risk (increased susceptibility) of the disease. In one embodiment, significance associated with a marker or haplotype is measured by a relative risk (RR). In another embodiment, significance associated with a marker or haplotye is measured by an odds ratio (OR). In a further embodiment, the significance is measured by a percentage. In one embodiment, a significant increased risk is measured as a risk (relative risk and/or odds ratio) of at least 1.1, including but not limited to: at least 1.2, at least 1.3, at least 1.4, at least 1.5, at least 1.6, at least 1.7, 1.8, at least 1.9, at least 2.0, at least 2.5, at least 3.0, at least 4.0, and at least 5.0. In a particular embodiment, a risk (relative risk and/or odds ratio) of at least 1.2 is significant. In another particular embodiment, a risk of at least 1.08 is significant. In yet another embodiment, a risk of at least 1.10 is significant. In a further embodiment, a relative risk of at least 1.15 is significant. In another further embodiment, a significant increase in risk is at least 1.17 is significant. However, other cutoffs are also contemplated, e.g., at least 1.11, 1.12, 1.13, 1.14, 1.15, 1.16, 1.17, 1.18, 1.19, 1.20, 1.21, 1.22, 1.23, 1.24, 1.25, and so on, and such cutoffs are also within scope of the present invention. In other embodiments, a significant increase in risk is at least about 80%, including but not limited to about 10%, 11%, 12%, 13%, 14%, 15%, 16%, 17%, 18%, 19%, 20%, 21%, 22%, 23%, 24%, 25%, 30%, 35%, 40%, 45%, 50%, 55%, 60%, 65%, 70%, 75%, 80%, 85%, 90%, 95%, 100%, 150%, 200%, 300%, and 500%. In one particular embodiment, a significant increase in risk is at least 10%. In other embodiments, a significant increase in risk is at least 15%, at least 17%, at least 18%, at least 19%, at least 20%, at least 25%, at least 30% and at least 40%. Other cutoffs or ranges as deemed suitable by the person skilled in the art to characterize the invention are however also contemplated, and those are also within scope of the present invention. In certain embodiments, a significant increase in risk is characterized by a p-value, such as a p-value of less than 0.05, less than 0.01, less than 0.001, less than 0.0001, less than 0.00001, less than 0.000001, less than 0.0000001, less than 0.00000001, or less than 0.000000001.

An at-risk polymorphic marker or haplotype as described herein is one where at least one allele of at least one marker or haplotype is more frequently present in an individual at risk for the disease (or trait) (affected), or diagnosed with the disease, compared to the frequency of its presence in a comparison group (control), such that the presence of the marker or haplotype is indicative of susceptibility to the disease. The control group may in one embodiment be a population sample, i.e. a random sample from the general population. In another embodiment, the control group is represented by a group of individuals who are disease-free. Such disease-free controls may in one embodiment be characterized by the absence of one or more specific disease-associated symptoms. Alternatively, the disesae-free controls are those that have not been diagnosed with the disease. In another embodiment, the disease-free control group is characterized by the absence of one or more disease-specific risk factors. Such risk factors are in one embodiment at least one environmental risk factor. Representative environmental factors are natural products, minerals or other chemicals which are known to affect, or contemplated to affect, the risk of developing the specific disease or trait. Other environmental risk factors are risk factors related to lifestyle, including but not limited to food and drink habits, geographical location of main habitat, and occupational risk factors. In another embodiment, the risk factors comprise at least one additional genetic risk factor.

As an example of a simple test for correlation would be a Fisher-exact test on a two by two table. Given a cohort of chromosomes, the two by two table is constructed out of the number of chromosomes that include both of the markers or haplotypes, one of the markers or haplotypes but not the other and neither of the markers or haplotypes. Other statistical tests of association known to the skilled person are also contemplated and are also within scope of the invention.

In other embodiments of the invention, an individual who is at a decreased susceptibility (i.e., at a decreased risk) for a disease or trait is an individual in whom at least one specific allele at one or more polymorphic marker or haplotype conferring decreased susceptibility for the disease or trait is identified. The marker alleles and/or haplotypes conferring decreased risk are also said to be protective. In one aspect, the protective marker or haplotype is one that confers a significant decreased risk (or susceptibility) of the disease or trait. In certain embodiments, the marker is rs401681, wherein the presence of allele C is indicative of decreased risk of melanoma cancer and/or colorectal cancer. Alternatively, marker alleles in linkage disequilibrium with rs401681 allele C are indicative of decreased risk of melanoma cancer and/or colorectal cancer. In a preferred embodiment, the presence of allele C in rs401681, or a marker allele in linkage disequilibrium therewith, is indicative of a protection against melanoma cancer in the individual. In one embodiment, significant decreased risk is measured as a relative risk (or odds ratio) of less than 0.9, including but not limited to less than 0.9, less than 0.8, less than 0.7, less than 0.6, less than 0.5, less than 0.4, less than 0.3, less than 0.2 and less than 0.1. In one particular embodiment, significant decreased risk is less than 0.7. In another embodiment, significant decreased risk is less than 0.5. In yet another embodiment, significant decreased risk is less than 0.3. In another embodiment, the decrease in risk (or susceptibility) is at least 20%, including but not limited to at least 25%, at least 30%, at least 35%, at least 40%, at least 45%, at least 50%, at least 55%, at least 60%, at least 65%, at least 70%, at least 75%, at least 80%, at least 85%, at least 90%, at least 95% and at least 98%. In one particular embodiment, a significant decrease in risk is at least about 30%. In another embodiment, a significant decrease in risk is at least about 50%. In another embodiment, the decrease in risk is at least about 70%. Other cutoffs or ranges as deemed suitable by the person skilled in the art to characterize the invention are however also contemplated, and those are also within scope of the present invention.

The person skilled in the art will appreciate that for markers with two alleles present in the population being studied (such as SNPs), and wherein one allele is found in increased frequency in a group of individuals with a trait or disease in the population, compared with controls, the other allele of the marker will be found in decreased frequency in the group of individuals with the trait or disease, compared with controls. In such a case, one allele of the marker (the one found in increased frequency in individuals with the trait or disease) will be the at-risk allele, while the other allele will be a protective allele.

Thus, for rs401681, allele C is found to be indicative of protection against melanoma cancer and colorectal cancer. Therefore, the alternate allele, allele T, is an at-risk allele for melanoma cancer and colorectal cancer. Determination of the presence of this allele in individuals (in genotype datasets, samples containing DNA or in sequence data from individuals) is thus indicative of an increased risk of melanoma cancer and/or colorectal cancer in such individuals.

A genetic variant associated with a disease or a trait can be used alone to predict the risk of the disease for a given genotype. For a biallelic marker, such as a SNP, there are 3 possible genotypes: homozygote for the at risk variant, heterozygote, and non carrier of the at risk variant. Risk associated with variants at multiple loci can be used to estimate overall risk. For multiple SNP variants, there are *k* possible genotypes $k = 3^n \times 2^p$; where *n* is the number autosomal loci and *p* the number of gonosomal (sex chromosomal) loci. Overall risk assessment calculations for a plurality of risk variants usually assume that the relative risks of different genetic variants multiply, *i.e,* the overall risk (*e.g.,* RR or OR) associated with a particular genotype combination is the product of the risk values for the genotype at each locus. If the risk presented is the relative risk for a person, or a specific genotype for a person, compared to a reference population with matched gender and ethnicity, then the combined risk - is the product of the locus specific risk values - and which also corresponds to an overall risk estimate compared with the population. If the risk for a person is based on a comparison to non-carriers of the at risk allele, then the combined risk corresponds to an estimate that compares the person with a given combination of genotypes at all loci to a group of individuals who do not carry risk variants at any of those loci. The group of non-carriers of any at risk variant has the lowest estimated risk and has a combined risk, compared with itself (*i.e.*, non-carriers) of 1.0, but has an overall risk, compare with the population, of less than 1.0. It should be noted that the group of non-carriers can potentially be very small, especially for large number of loci, and in that case, its relevance is correspondingly small.

The multiplicative model is a parsimonious model that usually fits the data of complex traits reasonably well. Deviations from multiplicity have been rarely described in the context of common variants for common diseases, and if reported are usually only suggestive since very large sample sizes are usually required to be able to demonstrate statistical interactions between loci.

By way of an example, let us consider a total of eight variants that have been described to associate with prostate cancer (Gudmundsson, J., et al., Nat Genet 39:631-7 (2007), Gudmundsson, J., et al., Nat Genet 39:977-83 (2007); Yeager, M., et al, Nat Genet 39:645-49 (2007), Amundadottir, L., et al., Nat Genet 38:652-8 (2006); Haiman, C.A., et al., Nat Genet 39:638-44 (2007)). Seven of these loci are on autosomes, and the remaining locus is on chromosome X. The total number of theoretical genotypic combinations is then $3^7 \times 2^1 = 4374$. Some of those genotypic classes are very rare, but are still possible, and should be considered for overall risk assessment. It is likely that the multiplicative model applied in the case of multiple genetic variant will also be valid in conjugation with non-genetic risk variants assuming that the genetic variant does not clearly correlate with the "environmental" factor. In other words, genetic and non-genetic at-risk variants can be assessed under the multiplicative model to estimate combined risk, assuming that the non-genetic and genetic risk factors do not interact.

Using the same quantitative approach, the combined or overall risk associated with particular cancers may be assessed, including combinations of any one of the markers rs401681, rs2736100 and rs2736098, or markers in linkage disequilibrium therewith, with any other markes associated with risk of any one particular cancer. Such combinations may include any particular marker, or combination of markers, known to be associated with risk of the particular cancer.

*Linkage Disequilibrium*

The natural phenomenon of recombination, which occurs on average once for each chromosomal pair during each meiotic event, represents one way in which nature provides variations in sequence (and biological function by consequence). It has been discovered that recombination does not occur randomly in the genome; rather, there are large variations in the frequency of recombination rates, resulting in small regions of high recombination frequency (also called recombination hotspots) and larger regions of low recombination frequency, which are commonly referred to as Linkage Disequilibrium (LD) blocks (Myers, S. et al., Biochem Soc Trans 34:526-530 (2006); Jeffreys, A.J., et a., Nature Genet 29:217-222 (2001); May, C.A., et al., Nature Genet 31:272-275(2002)).

Linkage Disequilibrium (LD) refers to a non-random assortment of two genetic elements. For example, if a particular genetic element (*e.g.*, an allele of a polymorphic marker, or a haplotype) occurs in a population at a frequency of 0.50 (50%) and another element occurs at a frequency of 0.50 (50%), then the predicted occurrance of a person's having both elements is 0.25 (25%), assuming a random distribution of the elements. However, if it is discovered that the two elements occur together at a frequency higher than 0.25, then the elements are said to be in linkage disequilibrium, since they tend to be inherited together at a higher rate than what their independent frequencies of occurrence (*e.g.*, allele or haplotype frequencies) would predict. Roughly speaking, LD is generally correlated with the frequency of recombination events between the two elements. Allele or haplotype frequencies can be determined in a population by genotyping individuals in a population and determining the frequency of the occurence of each allele or haplotype in the population. For populations of diploids, e.g., human populations, individuals will typically have two alleles or allelic combinations for each genetic element (*e.g.*, a marker, haplotype or gene).

Many different measures have been proposed for assessing the strength of linkage disequilibrium (LD; reviewed in Devlin, B. & Risch, N., Genomics 29:311-22 (1995))). Most capture the strength of association between pairs of biallelic sites. Two important pairwise measures of LD are $r^2$ (sometimes denoted $\Delta^2$) and |D'| (Lewontin, R., Genetics 49:49-67 (1964); Hill, W.G. & Robertson, A. Theor. Appl. Genet. 22:226-231 (1968)). Both measures range from 0 (no disequilibrium) to 1 ('complete' disequilibrium), but their interpretation is slightly different. |D'| is defined in such a way that it is equal to 1 if just two or three of the possible haplotypes are present, and it is <1 if all four possible haplotypes are present. Therefore, a value of |D'| that is <1 indicates that historical recombination may have occurred between two sites (recurrent mutation can also cause |D'| to be <1, but for single nucleotide polymorphisms (SNPs) this is usually regarded as being less likely than recombination). The measure $r^2$ represents the statistical correlation between two sites, and takes the value of 1 if only two haplotypes are present.

The $r^2$ measure is arguably the most relevant measure for association mapping, because there is a simple inverse relationship between $r^2$ and the sample size required to detect association between susceptibility loci and SNPs. These measures are defined for pairs of sites, but for some applications a determination of how strong LD is across an entire region that contains many polymorphic sites might be desirable (*e.g.*, testing whether the strength of LD differs significantly among loci or across populations, or whether there is more or less LD in a region than predicted under a particular model). Measuring LD across a region is not straightforward, but one approach is to use the measure r, which was developed in population genetics. Roughly speaking, r measures how much recombination would be required under a particular population model to generate the LD that is seen in the data. This type of method can potentially also provide a statistically rigorous approach to the problem of determining whether LD data provide evidence for the presence of recombination hotspots. For the methods described herein, a significant $r^2$ value can be at least 0.1 such as at least 0.1, 0.15, 0.2, 0.25, 0.3, 0.35, 0.4, 0.45, 0.5, 0.55, 0.6, 0.65, 0.7, 0.75, 0.8, 0.85, 0.9, 0.91, 0.92, 0.93, 0.94, 0.95, 0.96, 0.97, 0.98, or at lesat 0.99. In one preferred embodiment, the significant $r^2$ value can be at least 0.2. Alternatively, linkage disequilibrium as described herein, refers to linkage disequilibrium characterized by values of |D'| of at least 0.2, such as 0.3, 0.4, 0.5, 0.6, 0.7, 0.8, 0.85, 0.9, 0.95, 0.96, 0.97, 0.98, or at least 0.99. Thus, linkage disequilibrium represents a correlation between alleles of distinct markers. It is measured by correlation coefficient or |D'| ($r^2$ up to 1.0 and |D'| up to 1.0). In certain embodiments, linkage disequilibrium is defined in terms of values for both the $r^2$ and |D'| measures. In one such embodiment, a significant linkage disequilibrium is defined as $r^2 > 0.1$ and |D'| >0.8. In another embodiment, a significant linkage disequilibrium is defined as $r^2 > 0.2$ and |D'| >0.9. Other combinations and permutations of values of $r^2$ and |D'| for determining linkage disequilibrium are also contemplated, and are also within the scope of the invention. Linkage disequilibrium can be determined in a single human population, as defined herein, or it can be determined in a collection of samples comprising individuals from more than one human population. In one embodiment of the invention, LD is determined in a sample from one or more of the HapMap populations (caucasian, african, japanese, chinese), as defined (http://www.hapmap.org). In one such embodiment, LD is determined in the CEU population of the HapMap samples. In another embodiment, LD is determined in the YRI population. In yet another embodiment, LD is determined in samples from the Icelandic population.

If all polymorphisms in the genome were independent at the population level (*i.e.*, no LD), then every single one of them would need to be investigated in association studies, to assess all the different polymorphic states. However, due to linkage disequilibrium between polymorphisms, tightly linked polymorphisms are strongly correlated, which reduces the number of polymorphisms that need to be investigated in an association study to observe a significant association. Another consequence of LD is that many polymorphisms may give an association signal due to the fact that these polymorphisms are strongly correlated.

Genomic LD maps have been generated across the genome, and such LD maps have been proposed to serve as framework for mapping disease-genes (Risch, N. & Merkiangas, K, Science 273:1516-1517 (1996); Maniatis, N., et al., Proc Natl Acad Sci USA 99:2228-2233 (2002); Reich, DE et al, Nature 411:199-204 (2001)).

It is now established that many portions of the human genome can be broken into series of discrete haplotype blocks containing a few common haplotypes; for these blocks, linkage disequilibrium data provides little evidence indicating recombination (see, *e.g.*, Wall., J.D. and Pritchard, J.K., Nature Reviews Genetics 4:587-597 (2003); Daly, M. et al., Nature Genet. 29:229-232 (2001); Gabriel, S.B. et al., Science 296:2225-2229 (2002); Patil, N. et al., Science 294:1719-1723 (2001); Dawson, E. et al., Nature 418:544-548 (2002); Phillips, M.S. et al., Nature Genet. 33:382-387 (2003)).

There are two main methods for defining these haplotype blocks: blocks can be defined as regions of DNA that have limited haplotype diversity (see, *e.g.,* Daly, M. et al., Nature Genet. 29:229-232 (2001); Patil, N. et al., Science 294:1719-1723 (2001); Dawson, E. et al., Nature 418:544-548 (2002); Zhang, K. et al., Proc. Natl. Acad. Sci. USA 99:7335-7339 (2002)), or as regions between transition zones having extensive historical recombination, identified using linkage disequilibrium (see, *e.g.,* Gabriel, S.B. et al., Science 296:2225-2229 (2002); Phillips, M.S. et al., Nature Genet. 33:382-387 (2003); Wang, N. et al., Am. J. Hum. Genet. 71:1227-1234 (2002); Stumpf, M.P., and Goldstein, D.B., Curr. Biol. 13:1-8 (2003)). More recently, a fine-scale map of recombination rates and corresponding hotspots across the human genome has been generated (Myers, S., et al., Science 310:321-32324 (2005); Myers, S. et al., Biochem Soc Trans 34:526530 (2006)). The map reveals the enormous variation in recombination across the genome, with recombination rates as high as 10-60 cM/Mb in hotspots, while closer to 0 in intervening regions, which thus represent regions of limited haplotype diversity and high LD. The map can therefore be used to define haplotype blocks/LD blocks as regions flanked by recombination hotspots. As used herein, the terms "haplotype block" or "LD block" includes blocks defined by any of the above described characteristics, or other alternative methods used by the person skilled in the art to define such regions.

Haplotype blocks (LD blocks) can be used to map associations between phenotype and haplotype status, using single markers or haplotypes comprising a plurality of markers. The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks.

It has thus become apparent that for any given observed association to a polymorphic marker in the genome, it is likely that additional markers in the genome also show association. This is a natural consequence of the uneven distribution of LD across the genome, as observed by the large variation in recombination rates. The markers used to detect association thus in a sense represent "tags" for a genomic region (i.e., a haplotype block or LD block) that is associating with a given disease or trait, and as such are useful for use in the methods and kits of the present invention. One or more causative (functional) variants or mutations may reside within the region found to be associating to the disease or trait. The functional variant may be another SNP, a tandem repeat polymorphism (such as a minisatellite or a microsatellite), a transposable element, or a copy number variation, such as an inversion, deletion or insertion. Such variants in LD with the variants described herein may confer a higher relative risk (RR) or odds ratio (OR) than observed for the tagging markers used to detect the association. The present invention thus refers to the markers used for detecting association to the disease, as described herein, as well as markers in linkage disequilibrium with the markers. Thus, in certain embodiments of the invention, markers that are in LD with the markers and/or haplotypes of the invention, as described herein, may be used as surrogate markers. The surrogate markers have in one embodiment relative risk (RR) and/or odds ratio (OR) values smaller than for the markers or haplotypes initially found to be associating with the disease, as described herein. In other embodiments, the surrogate markers have RR or OR values greater than those initially determined for the markers initially found to be associating with the disease, as described herein. An example of such an embodiment would be a rare, or relatively rare (such as < 10% allelic population frequency) variant in LD with a more common variant (> 10% population frequency) initially found to be associating with the disease, such as the variants described herein. Identifying and using such markers for detecting the association discovered by the inventors as described herein can be performed by routine methods well known to the person skilled in the art, and are therefore within the scope of the present invention.

*Determination of haplotype frequency*

The frequencies of haplotypes in patient and control groups can be estimated using an expectation-maximization algorithm (Dempster A. et al., J. R. Stat. Soc. B, 39:1-38 (1977)). An implementation of this algorithm that can handle missing genotypes and uncertainty with the phase can be used. Under the null hypothesis, the patients and the controls are assumed to have identical frequencies. Using a likelihood approach, an alternative hypothesis is tested, where a candidate at-risk-haplotype, which can include the markers described herein, is allowed to have a higher frequency in patients than controls, while the ratios of the frequencies of other haplotypes are assumed to be the same in both groups. Likelihoods are maximized separately under both hypotheses and a corresponding 1-df likelihood ratio statistic is used to evaluate the statistical significance.

To look for at-risk and protective markers and haplotypes within a susceptibility region, for example within an LD block, association of all possible combinations of genotyped markers within the region is studied. The combined patient and control groups can be randomly divided into two sets, equal in size to the original group of patients and controls. The marker and haplotype analysis is then repeated and the most significant p-value registered is determined. This randomization scheme can be repeated, for example, over 100 times to construct an empirical distribution of p-values. In a preferred embodiment, a p-value of <0.05 is indicative of a significant marker and/or haplotype association.

*Haplotype Analysis*

One general approach to haplotype analysis involves using likelihood-based inference applied to NEsted MOdels (Gretarsdottir S., et al., Nat. Genet. 35:131-38 (2003)). The method is implemented in the program NEMO, which allows for many polymorphic markers, SNPs and microsatellites. The method and software are specifically designed for case-control studies where the purpose is to identify haplotype groups that confer different risks. It is also a tool for studying LD structures. In NEMO, maximum likelihood estimates, likelihood ratios and p-values are calculated directly, with the aid of the EM algorithm, for the observed data treating it as a missing-data problem.

Even though likelihood ratio tests based on likelihoods computed directly for the observed data, which have captured the information loss due to uncertainty in phase and missing genotypes, can be relied on to give valid p-values, it would still be of interest to know how much information had been lost due to the information being incomplete. The information measure for haplotype analysis is described in Nicolae and Kong (Technical Report 537, Department of Statistics, University of Statistics, University of Chicago; Biometrics, 60(2):368-75 (2004)) as a natural extension of information measures defined for linkage analysis, and is implemented in NEMO.

For single marker association to a disease, the Fisher exact test can be used to calculate two-sided p-values for each individual allele. Usually, all p-values are presented unadjusted for multiple comparisons unless specifically indicated. The presented frequencies (for microsatellites, SNPs and haplotypes) are allelic frequencies as opposed to carrier frequencies. To minimize any bias due the relatedness of the patients who were recruited as families to the study, first and second-degree relatives can be eliminated from the patient list. Furthermore, the test can be repeated for association correcting for any remaining relatedness among the patients, by extending a variance adjustment procedure previously described (Risch, N. & Teng, J. Genome Res., 8:1273-1288 (1998)) for sibships so that it can be applied to general familial relationships, and present both adjusted and unadjusted p-values for comparison. The method of genomic controls (Devlin, B. & Roeder, K. Biometrics 55:997 (1999)) can also be used to adjust for the relatedness of the individuals and possible stratification. The differences are in general very small as expected. To assess the significance of single-marker association corrected for multiple testing we can carry out a randomization test using the same genotype data. Cohorts of patients and controls can be randomized and the association analysis redone multiple times (*e.g.*, up to 500,000 times) and the p-value is the fraction of replications that produced a p-value for some marker allele that is lower than or equal to the p-value we observed using the original patient and control cohorts.

For both single-marker and haplotype analyses, relative risk (RR) and the population attributable risk (PAR) can be calculated assuming a multiplicative model (haplotype relative risk model) (Terwilliger, J.D. & Ott, J., Hum. Hered. 42:337-46 (1992) and Falk, C.T. & Rubinstein, P, Ann. Hum. Genet. 51 (Pt 3):227-33 (1987)), i.e., that the risks of the two alleles/haplotypes a person carries multiply. For example, if RR is the risk of A relative to a, then the risk of a person homozygote AA will be RR times that of a heterozygote Aa and $RR^2$ times that of a homozygote aa. The multiplicative model has a nice property that simplifies analysis and computations - haplotypes are independent, *i.e.,* in Hardy-Weinberg equilibrium, within the affected population as well as within the control population. As a consequence, haplotype counts of the affecteds and controls each have multinomial distributions, but with different haplotype frequencies under the alternative hypothesis. Specifically, for two haplotypes, $h_i$ and $h_j$, risk($h_i$)/risk($h_j$) = $(f_i/p_i)/(f_j/p_j)$, where $f$ and $p$ denote, respectively, frequencies in the affected population and in the control population. While there is some power loss if the true model is not multiplicative, the loss tends to be mild except for extreme cases. Most importantly, p-values are always valid since they are computed with respect to null hypothesis.

An association signal detected in one association study may be replicated in a second cohort, ideally from a different population (*e.g.*, different region of same country, or a different country) of the same or different ethnicity. The advantage of replication studies is that the number of tests performed in the replication study, and hence the less stringent the statistical measure that is applied. For example, for a genome-wide search for susceptibility variants for a particular disease or trait using 300,000 SNPs, a correction for the 300,000 tests performed (one for each SNP) can be performed. Since many SNPs on the arrays typically used are correlated (*i.e.*, in LD), they are not independent. Thus, the correction is conservative. Nevertheless, applying this correction factor requires an observed P-value of less than 0.05/300,000 = $1.7 \times 10^{-7}$ for the signal to be considered significant applying this conservative test on results from a single study cohort. Obviously, signals found in a genome-wide association study with P-values less than this conservative threshold are a measure of a true genetic effect, and replication in additional cohorts is not necessarily from a statistical point of view. However, since the correction factor depends on the number of statistical tests performed, if one signal (one SNP) from an initial study is replicated in a second case-control cohort, the appropriate statistical test for significance is that for a single statistical test, *i.e.,* P-value less than 0.05. Replication studies in one or even several additional case-control cohorts have the added advantage of providing assessment of the association signal in additional populations, thus simultaneously confirming the initial finding and providing an assessment of the overall significance of the genetic variant(s) being tested in human populations in general.

The results from several case-control cohorts can also be combined to provide an overall assessment of the underlying effect. The methodology commonly used to combine results from multiple genetic association studies is the Mantel-Haenszel model (Mantel and Haenszel, J Natl Cancer Inst 22:719-48 (1959)). The model is designed to deal with the situation where association results from different populations, with each possibly having a different population frequency of the genetic variant, are combined. The model combines the results assuming that the effect of the variant on the risk of the disease, a measured by the OR or RR, is the same in all populations, while the frequency of the variant may differ between the poplations. Combining the results from several populations has the added advantage that the overall power to detect a real underlying association signal is increased, due to the increased statistical power provided by the combined cohorts. Furthermore, any deficiencies in individual studies, for example due to unequal matching of cases and controls or population stratification will tend to balance out when results from multiple cohorts are combined, again providing a better estimate of the true underlying genetic effect.

*Risk assessment and Diagnostics*

Within any given population, there is an absolute risk of developing a disease or trait, defined as the chance of a person developing a specific disease or trait over a specified time-period. For example, a woman's lifetime absolute risk of breast cancer is one in nine. That is to say, one woman in every nine will develop breast cancer at some point in their lives. Risk is typically measured by looking at very large numbers of people, rather than at a particular individual. Risk is often presented in terms of Absolute Risk (AR) and Relative Risk (RR). Relative Risk is used to compare risks associating with two variants or the risks of two different groups of people. For example, it can be used to compare a group of people with a certain genotype with another group having a different genotype. For a disease, a relative risk of 2 means that one group has twice the chance of developing a disease as the other group. The risk presented is usually the relative risk for a person, or a specific genotype of a person, compared to the population with matched gender and ethnicity. Risks of two individuals of the same gender and ethnicity could be compared in a simple manner. For example, if, compared to the population, the first individual has relative risk 1.5 and the second has relative risk 0.5, then the risk of the first individual compared to the second individual is 1.5/0.5 = 3.

As described herein, certain polymorphic markers and haplotypes comprising such markers are found to be useful for risk assessment of cancer. Risk assessment can involve the use of the markers for determining a susceptibility to cancer. Particular alleles of polymorphic markers (*e.g.*, SNPs) are found more frequently in individuals with cancer, than in individuals without diagnosis of cancer. Therefore, these marker alleles have predictive value for detecting cancer, or a susceptibility to cancer, in an individual. Tagging markers in linkage disequilibrium with at-risk variants (or protective variants) described herein can be used as surrogates for these markers (and/or haplotypes). Such surrogate markers can be located within a particular haplotype block or LD block. Such surrogate markers can also sometimes be located outside the physical boundaries of such a haplotype block or LD block, either in close vicinity of the LD block/haplotype block, but possibly also located in a more distant genomic location.

Long-distance LD can for example arise if particular genomic regions (*e.g.*, genes) are in a functional relationship. For example, if two genes encode proteins that play a role in a shared metabolic pathway, then particular variants in one gene may have a direct impact on observed variants for the other gene. Let us consider the case where a variant in one gene leads to increased expression of the gene product. To counteract this effect and preserve overall flux of the particular pathway, this variant may have led to selection of one (or more) variants at a second gene that confers decreased expression levels of that gene. These two genes may be located in different genomic locations, possibly on different chromosomes, but variants within the genes are in apparent LD, not because of their shared physical location within a region of high LD, but rather due to evolutionary forces. Such LD is also contemplated and within scope of the present invention. The skilled person will appreciate that many other scenarios of functional gene-gene interaction are possible, and the particular example discussed here represents only one such possible scenario.

Markers with values of $r^2$ equal to 1 are perfect surrogates for the at-risk variants, i.e. genotypes for one marker perfectly predicts genotypes for the other. Markers with smaller values of $r^2$ than 1 can also be surrogates for the at-risk variant, or alternatively represent variants with relative risk values as high as or possibly even higher than the at-risk variant. The at-risk variant identified may not be the functional variant itself, but is in this instance in linkage disequilibrium with the true functional variant. The functional variant may for example be a tandem repeat, such as a minisatellite or a microsatellite, a transposable element (*e.g.*, an *Alu* element), or a structural alteration, such as a deletion, insertion or inversion (sometimes also called copy number variations, or CNVs). The present invention encompasses the assessment of such surrogate markers for the markers as disclosed herein. Such markers are annotated, mapped and listed in public databases, as well known to the skilled person, or can alternatively be readily identified by sequencing the region or a part of the region identified by the markers of the present invention in a group of individuals, and identify polymorphisms in the resulting group of sequences. As a consequence, the person skilled in the art can readily and without undue experimentation genotype surrogate markers in linkage disequilibrium with the markers and/or haplotypes as described herein. The tagging or surrogate markers in LD with the at-risk variants detected, also have predictive value for detecting association to the disease, or a susceptibility to the disease, in an individual. These tagging or surrogate markers that are in LD with the markers of the present invention can also include other markers that distinguish among haplotypes, as these similarly have predictive value for detecting susceptibility to the particular disease.

The present invention can in certain embodiments be practiced by assessing a sample comprising genomic DNA from an individual for the presence of variants described herein to be associated with cancer. Such assessment typically steps that detect the presence or absence of at least one allele of at least one polymorphic marker, using methods well known to the skilled person and further described herein, and based on the outcome of such assessment, determine whether the individual from whom the sample is derived is at increased or decreased risk (increased or decreased susceptibility) of cancer. Detecting particular alleles of polymorphic markers can in certain embodiments be done by obtaining nucleic acid sequence data about a particular human individual, that identifies at least one allele of at least one polymorphic marker. Different alleles of the at least one marker are associated with different susceptibility to the disease in humans. Obtaining nucleic acid sequence data can comprise nucleic acid sequence at a single nucleotide position, which is sufficient to identify alleles at SNPs. The nucleic acid sequence data can also comprise sequence at any other number of nucleotide positions, in particular for genetic markers that comprise multiple nucleotide positions, and can be anywhere from two to hundreds of thousands, possibly even millions, of nucleotides (in particular, in the case of copy number variations (CNVs)).

In certain embodiments, the invention can be practiced utilizing a dataset comprising information about the genotype status of at least one polymorphic marker associated with a disease (or markers in linkage disequilibrium with at least one marker associated with the disease). In other words, a dataset containing information about such genetic status, for example in the form of genotype counts at a certain polymorphic marker, or a plurality of markers (*e.g.*, an indication of the presence or absence of certain at-risk alleles), or actual genotypes for one or more markers, can be queried for the presence or absence of certain at-risk alleles at certain polymorphic markers shown by the present inventors to be associated with the disease. A positive result for a variant (*e.g.*, marker allele) associated with the disease, is indicative of the individual from which the dataset is derived is at increased susceptibility (increased risk) of the disease.

In certain embodiments of the invention, a polymorphic marker is correlated to a disease by referencing genotype data for the polymorphic marker to a look-up table that comprises correlations between at least one allele of the polymorphism and the disease. In some embodiments, the table comprises a correlation for one polymorphism. In other embodiments, the table comprises a correlation for a plurality of polymorphisms. In both scenarios, by referencing to a look-up table that gives an indication of a correlation between a marker and the disease, a risk for the disease, or a susceptibility to the disease, can be identified in the individual from whom the sample is derived. In some embodiments, the correlation is reported as a statistical measure. The statistical measure may be reported as a risk measure, such as a relative risk (RR), an absolute risk (AR) or an odds ratio (OR).

The markers described herein, *e.g.*, the markers presented in Tables 5, 6, 7, 8, and 9, e.g. rs401681, rs2736100 and rs2736098, may be useful for risk assessment and diagnostic purposes, either alone or in combination. Results of cancer risk based on the markers described herein can also be combined with data for other genetic markers or risk factors for cancer, to establish overall risk. Thus, even in cases where the increase in risk by individual markers is relatively modest, *e.g*. on the order of 10-30%, the association may have significant implications. Thus, relatively common variants may have significant contribution to the overall risk (Population Attributable Risk is high), or combination of markers can be used to define groups of individual who, based on the combined risk of the markers, is at significant combined risk of developing the disease.

Thus, in certain embodiments of the invention, a plurality of variants (genetic markers, biomarkers and/or haplotypes) is used for overall risk assessment. These variants are in one embodiment selected from the variants as disclosed herein. Other embodiments include the use of the variants of the present invention in combination with other variants known to be useful for diagnosing a susceptibility to cancer. In such embodiments, the genotype status of a plurality of markers and/or haplotypes is determined in an individual, and the status of the individual compared with the population frequency of the associated variants, or the frequency of the variants in clinically healthy subjects, such as age-matched and sex-matched subjects. Methods known in the art, such as multivariate analyses or joint risk analyses or other methods known to the skilled person, may subsequently be used to determine the overall risk conferred based on the genotype status at the multiple loci. Assessment of risk based on such analysis may subsequently be used in the methods, uses and kits of the invention, as described herein.

As described in the above, the haplotype block structure of the human genome has the effect that a large number of variants (markers and/or haplotypes) in linkage disequilibrium with the variant originally associated with a disease or trait may be used as surrogate markers for assessing association to the disease or trait. The number of such surrogate markers will depend on factors such as the historical recombination rate in the region, the mutational frequency in the region (i.e., the number of polymorphic sites or markers in the region), and the extent of LD (size of the LD block) in the region. These markers are usually located within the physical boundaries of the LD block or haplotype block in question as defined using the methods described herein, or by other methods known to the person skilled in the art. However, sometimes marker and haplotype association is found to extend beyond the physical boundaries of the haplotype block as defined, as discussed in the above. Such markers and/or haplotypes may in those cases be also used as surrogate markers and/or haplotypes for the markers and/or haplotypes physically residing within the haplotype block as defined. As a consequence, markers and haplotypes in LD (typically characterized by inter-marker $r^2$ values of greater than 0.1, such as $r^2$ greater than 0.2, including $r^2$ greater than 0.3, also including markers correlated by values for $r^2$ greater than 0.4) with the markers and haplotypes of the present invention are also useful, even if they are physically located beyond the boundaries of the haplotype block as defined. This includes markers that are described herein (*e.g.*, rs401681, rs2736100 and rs2736098), but may also include other markers that are in strong LD (*e.g.*, characterized by

$r^2$ greater than 0.1 or 0.2 and/or |D'| > 0.8) with rs401681, rs2736100 and rs2736098 (e.g., the markers set forth in Table 5, 6 and 7).

For the SNP markers described herein, the opposite allele to the allele found to be in excess in patients (at-risk allele) is found in decreased frequency in cancer. These markers and haplotypes in LD and/or comprising such markers, are thus protective for cancer, i.e. they confer a decreased risk or susceptibility of individuals carrying these markers and/or haplotypes developing cancer. It is noteworthy that while allele C of rs401681 is predictive of increased risk of multiple cancers as shown herein, this allele is predictive of decreased risk of cutaneous melanoma cancer and colorectal cancer, i.e. the allele is protective for these cancers.

Certain variants of the present invention, including certain haplotypes comprise, in some cases, a combination of various genetic markers, *e.g.*, SNPs and microsatellites. Detecting haplotypes can be accomplished by methods known in the art and/or described herein for detecting sequences at polymorphic sites. Furthermore, correlation between certain haplotypes or sets of markers and disease phenotype can be verified using standard techniques. A representative example of a simple test for correlation would be a Fisher-exact test on a two by two table.

In specific embodiments, a marker allele or haplotype found to be associated with cancer, (*e.g.*, marker alleles as listed in Tables 1, 2 and 3) is one in which the marker allele or haplotype is more frequently present in an individual at risk for cancer (affected), compared to the frequency of its presence in a healthy individual (control), or in randomly selected individual from the population, wherein the presence of the marker allele or haplotype is indicative of a susceptibility to cancer. In other embodiments, at-risk markers in linkage disequilibrium with one or more markers shown herein to be associated with cancer (*e.g.*, marker alleles as listed in Tables 1, 2 and 3) are tagging markers that are more frequently present in an individual at risk for cancer (affected), compared to the frequency of their presence in a healthy individual (control) or in a randomly selected individual from the population, wherein the presence of the tagging markers is indicative of increased susceptibility to cancer. In a further embodiment, at-risk markers alleles (i.e. conferring increased susceptibility) in linkage disequilibrium with one or more markers found to be associated with cancer, are markers comprising one or more allele that is more frequently present in an individual at risk for cancer, compared to the frequency of their presence in a healthy individual (control), wherein the presence of the markers is indicative of increased susceptibility to cancer.

*Study population*

In a general sense, the methods of the invention can be utilized from samples containing nucleic acid material (DNA or RNA) from any source and from any individual, or from genotype data derived from such samples. In preferred embodiments, the individual is a human individual. The individual can be an adult, child, or fetus. The nucleic acid source may be any sample comprising nucleic acid material, including biological samples, or a sample comprising nucleic acid material derived therefrom. The present invention also provides for assessing markers and/or haplotypes in individuals who are members of a target population. Such a target population is in one embodiment a population or group of individuals at risk of developing cancer, based on other genetic factors, biomarkers, biophysical parameters, history of cancer or related diseases, previous diagnosis of cancer, family history of cancer. A target population is in certain embodiments is a population or group with known radiation exposure, such as radiation exposure due to diagnostic or therapeutic medicine, radioactive fallout from nuclear explosions, radioactive exposure due to nuclear power plants or other sources of radioactivity, etc.

The invention provides for embodiments that include individuals from specific age subgroups, such as those over the age of 40, over age of 45, or over age of 50, 55, 60, 65, 70, 75, 80, or 85. Other embodiments of the invention pertain to other age groups, such as individuals aged less than 85, such as less than age 80, less than age 75, or less than age 70, 65, 60, 55, 50, 45, 40, 35, or age 30. Other embodiments relate to individuals with age at onset of cancer in any of the age ranges described in the above. It is also contemplated that a range of ages may be relevant in certain embodiments, such as age at onset at more than age 45 but less than age 60. Other age ranges are however also contemplated, including all age ranges bracketed by the age values listed in the above. The invention furthermore relates to individuals of either gender, males or females.

The Icelandic population is a Caucasian population of Northern European ancestry. A large number of studies reporting results of genetic linkage and association in the Icelandic population have been published in the last few years. Many of those studies show replication of variants, originally identified in the Icelandic population as being associating with a particular disease, in other populations (Styrkarsdottir, U., et al. N Engl J Med Apr 29 2008 (Epub ahead of print); Thorgeirsson, T., et al. Nature 452:638-42 (2008); Gudmundsson, J., et al. Nat Genet. 40:281-3 (2008); Stacey, S.N., et al., Nat Genet. 39:865-69 (2007); Helgadottir, A., et al., Science 316:1491-93 (2007); Steinthorsdottir, V., et al., Nat Genet. 39:770-75 (2007); Gudmundsson, J., et al., Nat Genet. 39:631-37 (2007); Frayling, TM, Nature Reviews Genet 8:657-662 (2007); Amundadottir, L.T., et al., Nat Genet. 38:652-58 (2006); Grant, S.F., et al., Nat Genet. 38:320-23 (2006)). Thus, genetic findings in the Icelandic population have in general been replicated in other populations, including populations from Africa and Asia.

It is thus believed that the markers of the present invention found to be associated with cancer will show similar association in other human populations. Particular embodiments comprising individual human populations are thus also contemplated. Such embodiments relate to human subjects that are from one or more human population including, but not limited to, Caucasian populations, European populations, American populations, Eurasian populations, Asian populations, Central/South Asian populations, East Asian populations, Middle Eastern populations, African populations, Hispanic populations, and Oceanian populations. European populations include, but are not limited to, Swedish, Norwegian, Finnish, Russian, Danish, Icelandic, Irish, Kelt, English, Scottish, Dutch, Belgian, French, German, Spanish, Portuguese, Italian, Polish, Bulgarian, Slavic, Serbian, Bosnian, Czech, Greek and Turkish populations.

The racial contribution in individual subjects may also be determined by genetic analysis. Genetic analysis of ancestry may be carried out using unlinked microsatellite markers such as those set out in Smith et al. (Am J Hum Genet 74, 1001-13 (2004)).

In certain embodiments, the invention relates to markers and/or haplotypes identified in specific populations, as described in the above. The person skilled in the art will appreciate that measures of linkage disequilibrium (LD) may give different results when applied to different populations. This is due to different population history of different human populations as well as differential selective pressures that may have led to differences in LD in specific genomic regions. It is also well known to the person skilled in the art that certain markers, *e.g.* SNP markers, have different population frequency in different populations, or are polymorphic in one population but not in another. The person skilled in the art will however apply the methods available and as thought herein to practice the present invention in any given human population. This may include assessment of polymorphic markers in the LD region of the present invention, so as to identify those markers that give strongest association within the specific population. Thus, the at-risk variants of the present invention may reside on different haplotype background and in different frequencies in various human populations. However, utilizing methods known in the art and the markers of the present invention, the invention can be practiced in any given human population.

*Utility of Genetic Testing*

The person skilled in the art will appreciate and understand that the variants described herein in general do not, by themselves, provide an absolute identification of individuals who will develop a particular form of cancer. The variants described herein do however indicate increased and/or decreased likelihood that individuals carrying the at-risk or protective variants of the invention will develop cancer, such as cancer of the lung, bladder, prostate, cervix, endometrium, thyroid and/or basal cells of the skin. This information is however extremely valuable in itself, as outlined in more detail in the below, as it can be used to, for example, initiate preventive measures at an early stage, perform regular physical exams to monitor the progress and/or appearance of symptoms, or to schedule exams at a regular interval to identify early symptoms, so as to be able to apply treatment at an early stage.

Analysis of the functional role of the genetic cancer risk variants may provide information on the molecular pathways that lead to cancer development and/or disease progression. Thus, on one hand there will be true "predisposition" variants that affect mostly whether an individual develops a disease or not. On the other hand, other variants may also associate with a particular course of the disease by influencing subsequent genetic changes in the tumor. Characterization of these changes may lead to the development of treatment strategies that would be particularly suitable in individuals carrying the genetic risk variant.

*Genetic testing for predisposition to multiple cancers*

In general, the knowledge of genetic variants that confer a risk of developing cancer offers the opportunity to apply a genetic test to distinguish between individuals with increased risk of cancer (i.e. carriers of the at-risk variants) and those with decreased risk of developing them (i.e. carriers of protective variants, and/or non-carriers of at-risk variants). The core value of genetic testing is the possibility of being able to diagnose disease, or a predisposition to disease, at an early stage and provide information to the clinician about prognosis/aggressiveness of the disease in order to be able to apply the most appropriate treatment.

The variants described herein show association to multiple forms of cancer. Thus it can be envisioned that they could have a utility in genetic testing for cancer predisposition in general. Notably, the variants show preferential association to cancer types that have a strong environmental component as well, such as UV radiation, smoking and exposure to industrial chemicals. Testing for the variants may be useful in a setting where individuals are exposed to these environmental agents. Individuals at high genetic risk could then be targeted for more frequent cancer screening. Also, intervention strategies that reduce or limit exposure to the environmental risk factors could be emphasized particularly in this group of individuals. An indication of possible intervention strategies for each cancer type are described below.

*Genetic Testing for Basal Cell Carcinoma*

The strongest known risk factors for BCC include exposure to UV radiation, fair pigmentation traits and genetic factors. A positive family history is a risk factor for BCC and SCC (Hemminki, K. et al., Arch Dermatol, 139, 885 (2003); Vitasa, B.C. et al., Cancer, 65, 2811 (1990)) suggesting an inherited component to the risk of BCC. Several rare genetic conditions have been associated with increased risks of BCC, including Nevoid Basal Cell Syndrome (Gorlin's Syndrome), Xeroderma Pigmentosum (XP), and Bazex's Syndrome. XP is underpinned by mutations in a variety of XP complementation group genes. Gorlin's Syndrome results from mutations in the PTCH1 gene. In addition, variants in the CYP2D6 and GSTT1 genes have been associated with BCC (Wong, et al., BMJ, 327, 794 (2003)) .

Fair pigmentation traits are known risk factors for BCC and are thought act, at least in part, through a reduced protection from UV irradiation. Thus, genes underlying these fair pigmentation traits have been associated with risk. MC1R, ASIP, and TYR have been shown to confer risk for BCC and/or SCC (Gudbjartsson, et.al., Nat. Gen. 40, 886 (2008); Bastiaens, et al., Am J Hum Genet, 68, 884 (2001); Han, et al., Int J Epidemiol, 35, 1514 (2006)).

Elucidation of genetic variants that affect risk of BCC, either though pigmentation traits or other mechanisms, can help identify individuals who have a high risk of developing these diseases. Thus, individuals who are at increased risk of BCC might be offered regular skin examinations to identify incipient tumours, and they might be counseled to avoid excessive UV exposure. Chemoprevention either using sunscreens or pharmaceutical agents (Bowden, Nat Rev Cancer, 4, 23 (2004)). might be employed. For individuals who have been diagnosed with BCC, knowledge of the underlying genetic predisposition may be useful in determining appropriate treatments and evaluating risks of recurrence and new tumors. Finally, screening for susceptibility to BCC or SCC might be important in planning the clinical management of transplant recipients and other immunosuppressed individuals.

*Genetic Testing for Melanoma.*

Relatives of melanoma patients are themselves at increased risk of melanoma, suggesting an inherited predisposition [Amundadottir, et al., (2004), PLoS Med, 1, e65. Epub 2004 Dec 28.]. A series of linkage based studies implicated CDKN2a on 9p21 as a major CM susceptibility gene [Bataille, (2003), Eur J Cancer, 39, 1341-7.]. CDK4 was identified as a pathway candidate shortly afterwards, however mutations have only been observed in a few families worldwide[Zuo, et al., (1996), Nat Genet, 12, 97-9.]. CDKN2a encodes the cyclin dependent kinase inhibitor p16 which inhibits CDK4 and CDK6, preventing G1-S cell cycle transit. An alternate transcript of CKDN2a produces p14ARF, encoding a cell cycle inhibitor that acts through the MDM2-p53 pathway. It is likely that CDKN2a mutant melanocytes are deficient in cell cycle control or the establishment of senescence, either as a developmental state or in response to DNA damage. Overall penetrance of CDKN2a mutations in familial CM cases is 67% by age 80. However penetrance is increased in areas of high melanoma prevalence [Bishop, et al., (2002), J Natl Cancer Inst, 94, 894-903].

Individual who are at increased risk of melanoma might be offered regular skin examinations to identify incipient tumours, and they might be counselled to avoid excessive UV exposure. Chemoprevention either using sunscreens or pharmaceutical agents [Bowden, (2004), Nat Rev Cancer, 4, 23-35.] might be employed. For individuals who have been diagnosed with melanoma, knowledge of the underlying genetic predisposition may be useful in determining appropriate treatments and evaluating risks of recurrence and new primary tumours.

Endogenous host risk factors for CM are in part under genetic control. It follows that a proportion of the genetic risk for CM resides in the genes that underpin variation in pigmentation and nevi. The Melanocortin 1 Receptor (MC1R) is a G-protein coupled receptor involved in promoting the switch from pheomelanin to eumelanin synthesis. Numerous, well characterized variants of the MC1R gene have been implicated in red haired, pale skinned and freckle prone phenotypes. We and others have demonstrated the MC1R variants confer risk of melanoma (Gudbjartsson et.al., Nature Genetics 40:886-91 (2008)). Other pigmentation trait-associated variants, in the ASIP, TYR and TYRP1 genes have also been implicated in melanoma risk (Gudbjartsson et.al., Nature Genetics 40:886-91 (2008)). ASIP encodes the agouti signalling protein, a negative regulator of the melanocortin 1 receptor. TYR and TYRP1 are enzymes involved in melanin synthesis and are regulated by the MC1R pathway. Individuals at risk for BCC and/or SCC might be offered regular skin examinations to identify incipient tumours, and they might be counselled to avoid excessive UV exposure. Chemoprevention either using sunscreens or pharmaceutical agents [Bowden, (2004), Nat Rev Cancer, 4, 23-35.] might, be employed. For individuals who have been diagnosed with BCC or SCC, knowledge of the underlying genetic predisposition may be useful in determining appropriate treatments and evaluating risks of recurrence and new primary tumours. Screening for susceptibility to BCC or SCC might be important in planning the clinical management of transplant recipients and other immunosuppressed individuals.

*Genetic testing for Prostate cancer*

Epidemiological studies suggest that the genetic component of the risk of prostate cancer is greater than in any other cancer (Lichtenstein et al, N Engl J Med 343, 78 (2000)). Despite strong evidence for genetic factors, highly penetrant susceptibility genes for prostate cancer have proven difficult to find. Analysis of data from large twin studies has suggested that the majority of genetic prostate cancer risk may be attributable to recessive and/or multiple interacting genetic variants (Risch, Cancer Epidemiol Biomarkers Prev 7, 733 (2001)). Recently, several common genetic variants have been identified that affect the risk of prostate cancer Amundadottir, et al, Nat Gen 38, 652 (2006); Gudmundsson, et al, Nat Gen 39, 631 (2007); Gudmundsson, et al., Nat Gen 39, 977 (2007); Gudmundsson, et al., Nat Gen 40, 281 (2008); Eeles, et al., Nat Gen 40, 316 (2008); Yeager, et al., Nature Gen 39, 645 (2007)).

The characterization of genetic risk variants for prostate cancer can be put to use in at least two ways. First, a genetic risk model can be incorporated into a screening protocol to aid in early detection of the disease when chances of cure are the highest. Second, genetic variants may be found that associate with progression of the disease and could be use to direct treatment selection.

1. Early detection

Early diagnosis and treatment are key factors in determining the survival of certain sets of prostate cancer patients. The test most frequently used to screen for prostate cancer, the PSA blood test, is effective at detecting early stage prostate cancer but has limited specificity for the aggressive form of the disease, resulting in an extremely high rate of "over-diagnosis" of up to 50% (Draisma G, et al. J Natl Cancer Inst 95,:868 (2003)). Consequently, prostate cancer incidence has risen rapidly in those European countries where opportunistic PSA screening is commonplace and, due to lack of prognostic tests, has led to excessive treatment of localized lesions that might never progress to symptomatic cancer. This over-treatment carries heavy costs, both financial and personal as side-effects of treatment can be considerable, including impaired urinary continence and sexual dysfunction. A genetic variant that is shown to associate with a clinically relevant for of the disease might be useful in increasing the sensitivity and specificity of the already generally applied Prostate Specific Antigen (PSA) test and Digital Rectal Examination (DRE). This can lead to lower rates of false positives (thus minimize unnecessary procedures such as needle biopsies) and false negatives, thereby increasing detection of occult disease and minimizing morbidity and mortality due to prostate cancer. Also, an individual determined to be a carrier of a risk allele for the development of prostate cancer will likely undergo more frequent PSA testing and have a lower threshold for needle biopsy in the presence of an abnormal PSA value.

2. Predicting progression of early-stage prostate cancer

Today most men with screen-detected prostate cancer have localized disease at diagnosis. Many of these men may harbour clinically insignificant disease that will not impact their quality of life and life expectancy while in other men prostate cancer will progress to an advanced or lethal disease if left alone. Because of these uncertainties, and the lack of reliable prognostic markers, most men with localized disease are subjected to radical prostatectomy or radiotherapy which can adversely impact their urinary and sexual health. The reasons why some cancers are more aggressive than others remain poorly understood and the need for diagnostic resources to help differentiate between the two is immense. Identification of genetic markers that preferentially associate with an aggressive form of the disease could have important utility in guiding treatment selection. For example, if prostate cancer is diagnosed in an individual that is a carrier of an allele that confers increased risk of developing an aggressive form of prostate cancer, then the clinician would likely advise a more aggressive treatment strategy such as a prostatectomy instead of a less aggressive treatment strategy.

*Genetic testing for Lung cancer*

Although the large majority of lung cancer cases can be attributed to smoking, the disease is also influenced by genetic factors (Jonsson et.al., JAMA 292, 2977 (2004); Amundadottir et.al., PLoS Med. 1, e65 (2004)). Recently, a genetic variant on chromosome 15q was identified that affects smoking behaviour and increases risk of lung cancer (Thorgeirsson et al., Nature 452, 638 (2008)).

Individuals with a family history of lung cancer and carriers of at-risk variants may benefit from genetic testing since the knowledge of the presence of genetic risk factors, or evidence for increased risk of being a carrier of one or more genetic risk factors, may provide incentive for implementing a healthier lifestyle, by avoiding or minimizing known environmental risk factors for lung cancer. For example, an individual who is a current smoker and is identified as a carrier of one or more of the variants shown herein to be associated with increased risk of lung cancer, may, due to his/her increased risk of developing the disease, choose to quit smoking.

Integration of Genetic Risk Models into Clinical Management of Lung Cancer:

Management of lung cancer currently relies on a combination of primary prevention (most importantly abstinence from smoking), early diagnosis and appropriate treatments. There are clear clinical imperatives for integrating genetic testing into several aspects of these management areas. Identification of cancer susceptibility genes may also reveal key molecular pathways that may be manipulated (e.g., using small or large molecular weight drugs) and may lead to more effective treatments.

1. Primary prevention

Primary prevention options currently focus on avoiding exposure to tobacco smoke or other environmental toxins that have been associated with the development of lung cancer. Knowledge of the genetic risk for lung cancer may encourage individuals to abstain from smoking.

2. Early Diagnosis

Patients who are identified as being at high risk for lung cancer may be referred to have chest X-rays or sputum cytology examination. In addition, a spiral CT scan is a newly-developed procedure for lung cancer screening. Numerous lung cancer screening trials are currently taking place but presently, the U.S. Preventive Services Task Force (USPSTF) concludes that evidence is insufficient to recommend for or against screening asymptomatic persons for lung cancer with either low dose computerized tomography (LDCT), chest x-ray, sputum cytology, or a combination of these tests.

Many of the screening protocols being evaluated involve some form of radiation or and invasive procedure such as bronchoscopy. These protocols carry certain risks and may prove hard to implement due to the considerable costs involved. In light of the fact that only about 15% of lifetime smokers develop lung cancer, it is clear that the great majority of individuals at risk would be needlessly subjected to repeated screening tests with the associated costs and negative side-effects. The identification of genetic biomarkers that affect the risk of developing lung cancer could be used to help identify individuals should be offered extreme help in risk reduction programs such as smoking termination. In the case of failure to stop smoking, or in the case of ex-smokers, such genetic biomarkers could help in defining the subpopulation of individuals that would benefit the most from screening.

Less than 10% of lung cancer cases arise in individuals that have never smoked. Genetic biomarkers that predict the risk of lung cancer would be particularly useful in this group. The genetic component of this form of the disease is likely to be even stronger than in tobacco-related lung cancer. If genetic variants that affect the risk of non-smoking lung cancer were known, it might be possible to identify individuals at high risk for this disease and subject them to regular screening tests.

*Genetic testing for Urinary Bladder cancer (UBC)*

Cigarette smoking and occupational exposure to specific carcinogens are the strongest known risk factors for UBC. Familial clustering of UBC cases suggests that there is a genetic component to the risk of the disease (Aben, K.K. et al. Int J Cancer 98, 274 (2002); Amundadottir, L.T. et al. PLoS Med 1, e65 (2004); Murta-Nascimento, C. et al. Cancer Epidemiol Biomarkers Prev 16, 1595 (2007)). Segregation analyses have suggested that this component consists of many genes, each conferring a small risk (Aben, K.K. et al., Eur J Cancer 42, 1428 (2006)). Epidemiological studies have evaluated potential associations between sequence variants in candidate genes and UBC but the results have in many cases been difficult to replicate.

Identification of genetic variants that confer a risk of developing UBC offers the opportunity to distinguish between individuals with increased risk of developing UBC (i.e. carriers of the at-risk variant) and those with decreased risk of developing UBC (i.e. carriers of the protective variant). In the case of increased genetic risk, an individual may be offered more frequent screening for the disease or be advised to take extra steps to avoid known environmental risk factors. The polymorphic markers of the present invention can be used alone or in combination, as well as in combination with other factors, including other genetic risk factors or biomarkers, for risk assessment of an individual for UBC. Many factors known to affect the predisposition of an individual towards developing risk of developing UBC are known to the person skilled in the art and can be utilized in such assessment. These include, but are not limited to, age, gender, smoking status and/or smoking history, family history of cancer, and of UBC in particular. Methods known in the art can be used for such assessment, including multivariate analyses or logistic regression.

Current clinical treatment options for UBC include different surgical procedures, depending on the severity of the cases, e.g. whether the cancer is invasive into the muscle wall of the bladder. Treatment options also include radiation therapy, for which a proportion of patients experience adverse symptoms. One application of genetic risk markers for UBC includes the use of such markers to assess response to these therapeutic options, or to predict the efficacy of therapy using any one of these treatment options. Thus, genetic profiling can be used to select the appropriate treatment strategy based on the genetic status of the individual, or it may be used to predict the outcome of the particular treatment option, and thus be useful in the strategic selection of treatment options or a combination of available treatment options.

Again, such profiling and classification of individuals is supported further by first analysing known groups of patients for marker and/or haplotype status, as described further herein.

Genetic profiling based on the markers described herein, and model building based on such markers, can thus be useful in various aspects of bladder cancer risk management, including prediction of lifetime risk, management of disease at its various stages, and selection of appropriate treatment regimens.

*Genetic testing for Cervical cancer*

Cervical cancer is invariably associated with an infection with an oncogenic subtype of human papillomavirus (HPV). Infection with HPV is very common but in the great majority of cases, infection is cleared by the immune system and does not develop into a malignant state. It has been shown that genetic factors play a substantial role in the development of cervical cancer (Czene, K. et al., Int J Cancer 99, 260 (2002); Hemminki, K., and Chen, B. Cancer Epidemiol Biomarkers Prev 15, 1413 (2006); Couto, E., and Hemminki, K Int J Cancer 119, 2699 (2006)). Some of these genetic factors may affect mechanisms that help clear the viral infection and indeed, several polymorphisms in immune response genes have been associated with susceptibility to chronic HPV infection and cancer development (Hildesheim and Wang, Virus Res 89, 229 (2002).

Integration of Genetic Risk Models into Clinical Management of Cervical Cancer:

Management of cervical cancer currently focuses on early diagnosis through PAP test-based screening and appropriate treatments of dysplastic lesions/invasive cancer. There are clear clinical imperatives for integrating genetic testing into several aspects of these management areas. Identification of cancer susceptibility genes may also reveal key molecular pathways that may be manipulated (e.g., using small or large molecular weight drugs) and may lead to more effective treatments.

Primary prevention

Young women who have not been infected with HPV can get vaccinated against the most common subtypes of the virus. However, vaccination has not proven to prevent cancer in women who have already been infected with an oncogenic subtype of the virus and may not provide protection against rare virus types that are not included in the vaccine.

The most important prevention strategy against cervical cancer for the great majority of women is avoiding infection with HPV by limiting the number of sexual partners and using condoms during sexual intercourse. This strategy also limits exposure to other sexual transmitted diseases which may act as co-factors in cervical cancer development. A person who is carries genetic risk factors for cervical cancer might be encouraged to apply all the preventive measures available.

Finally, cervical cancer occurs at a higher rate in immunosuppressed individuals. Screening for susceptibility to CC might be useful in planning the clinical management of transplant recipients and other immunosuppressed individuals.

Early Diagnosis

Excluding the future effect of vaccinations, the most effective strategy in the fight against cervical cancer is regular screening in order to detect the disease before it becomes invasive. Screening for cervical cancer varies widely between countries, ranging from the organized, population-based screening programs (e.g. the Nordic countries), to ad hoc screening (e.g. the United States). The frequency of screening visits also varies between locations but commonly it is recommended that a woman gets a PAP test performed every year or every two years between age 22 and 70. Considerable evidence suggests that this screening regime is unnecessarily intensive and that a woman who has had 2 consecutive negative tests could be told to come every 5 years, greatly reducing the cost of the screening effort. However, considering the severity of the disease if not caught early, there is reluctance in changing these recommendations until further evidence is provided to support the safety of the alternative schedule. Assessment of genetic risk could be a tool to help determine the appropriate intervals between screening.

While cytological examination of PAP smears is highly effective in detecting dysplastic lesions and early stage CC which can be effectively treated by cone operation, a fraction of cases present with a persistent infection or re-infection which may progress to invasive cancer (Schiffman, M., et al., Lancet 370, 890 (2007)). These cases often need to be followed for years and subjected to repeated biopsies. There is an unmet clinical need to identify women with persistent or recurring infections that have the greatest risk of progressing to invasive CC. Such individuals might be subjected to more rigorous follow-up protocols or advised on how to reduce the risk by lifestyle changes. Knowledge of the underlying genetic predisposition might be useful in evaluating risks of progression.

*Genetic testing for Thyroid cancer*

The primary known risk factor for thyroid cancer is radiation exposure. Thyroid cancer incidence within the US has been rising for several decades, which may be attributable to increased detection of sub-clinical cancers, as opposed to an increase in the true occurrence of thyroid cancer (Davies, L. and Welch, H. G., Jama, 295, 2164 (2006)). The introduction of ultrasonography and fine-needle aspiration biopsy in the 1980s improved the detection of small nodules and made cytological assessment of a nodule more routine (Rojeski, M. T. and Gharib, H., N Engl J Med 313, 428 (1985); Ross, D. S., J Clin Endocrinol Metab, 91, 4253 (2006)). This increased diagnostic scrutiny may allow early detection of potentially lethal thyroid cancers. However, several studies report thyroid cancers as a common autopsy finding (up to 35%) in persons without a diagnosis of thyroid cancer (Bondeson, L. and Ljungberg, O., Cancer, 47, 319 (1981); Harach, H. R., et al., Cancer, 56, 531 (1985); Solares, C. A., et al., Am J Otolaryngol, 26, 87 (2005); Sobrinho-Simoes, M. A. et al., Cancer, 43, 1702 (1979)). This suggests that many people live with sub-clinical forms of thyroid cancer which are of little or no threat to their health.

Individuals with a family history of thyroid cancer and carriers of at-risk variants may benefit from genetic testing since the knowledge of the presence of a genetic risk factor, or evidence for increased risk of being a carrier of one or more risk factors, may provide increased incentive for implementing a healthier lifestyle, by avoiding or minimizing known environmental risk factors for the disease. Genetic testing of patients diagnosed with thyroid cancer may furthermore give valuable information about the primary cause of the disease and can aid the clinician in selecting the best treatment options and medication for each individual.

The knowledge of underlying genetic risk factors for thyroid cancer can be utilized in the application of screening programs for thyroid cancer. Thus, carriers of at-risk variants for thyroid cancer may benefit from more frequent screening than do non-carriers. Homozygous carriers of at-risk variants are particularly at risk for developing thyroid cancer. Also, carriers may benefit from more extensive screening, including ultrasonography and /or fine needle biopsy. The goal of screening programs is to detect cancer at an early stage. Knowledge of genetic status of individuals with respect to known risk variants can aid in the selection of applicable screening programs. In certain embodiments, it may be useful to use the at-risk variants for thyroid cancer described herein together with one or more diagnostic tool selected from Radioactive Iodine (RAI) Scan, Ultrasound examination, CT scan (CAT scan), Magnetic Resonance Imaging (MRI), Positron Emission Tomography (PET) scan, Fine needle aspiration biopsy and surgical biopsy.

## METHODS

Methods for cancer risk assessment and risk management are described herein and are encompassed by the invention. Also described are methods of assessing an individual for probability of response to therapeutic agents, methods for predicting the effectiveness of therapeutic agents, nucleic acids, polypeptides and antibodies and computer-implemented aspects of the invention. Kits for use in the various methods presented herein are also useful with the invention.

*Diagnostic and screening methods*

In certain embodiments, the present invention pertains to methods of determining a susceptibility to cancer, by detecting particular alleles at genetic markers that appear more frequently in subjects diagnosed with cancer or subjects who are susceptible to cancer. In particular embodiments, the invention is a method of determining a susceptibility to cancer by detecting at least one allele of at least one polymorphic marker (e.g., the markers described herein). In other embodiments, the invention relates to a method of determining a susceptibility to cancer by detecting at least one allele of at least one polymorphic marker. The present invention describes methods whereby detection of particular alleles of particular markers or haplotypes is indicative of a susceptibility to cancer. Such prognostic or predictive assays can also be used to determine prophylactic treatment of a subject based on determination of the genetic risk of cancer for the subject.

The present invention pertains in some embodiments to methods of clinical applications of diagnosis, *e.g.,* diagnosis performed by a medical professional. In other embodiments, the invention pertains to methods of diagnosis or determination of a susceptibility performed by a layman. The layman can be the customer of a genotyping service. The layman may also be a genotype service provider, who performs genotype analysis on a DNA sample from an individual, in order to provide service related to genetic risk factors for particular traits or diseases, based on the genotype status of the individual (*i.e.,* the customer). Recent technological advances in genotyping technologies, including high-throughput genotyping of SNP markers, such as Molecular Inversion Probe array technology (*e.g.*, Affymetrix GeneChip), and BeadArray Technologies (*e.g.*, Illumina GoldenGate and Infinium assays) have made it possible for individuals to have their own genome assessed for up to one million SNPs simultaneously, at relatively little cost. The resulting genotype information, which can be made available to the individual, can be compared to information about disease or trait risk associated with various SNPs, including information from public litterature and scientific publications. The diagnostic application of disease-associated alleles as described herein, can thus for example be performed by the individual, through analysis of his/her genotype data, by a health professional based on results of a clinical test, or by a third party, including the genotype service provider. The third party may also be service provider who interprets genotype information from the customer to provide service related to specific genetic risk factors, including the genetic markers described herein. In other words, the diagnosis or determination of a susceptibility of genetic risk can be made by health professionals, genetic counselors, third parties providing genotyping service, third parties providing risk assessment service or by the layman (*e.g.,* the individual), based on information about the genotype status of an individual and knowledge about the risk conferred by particular genetic risk factors (*e.g*., particular SNPs). In the present context, the term "diagnosing", "diagnose a susceptibility" and "determine a susceptibility" is meant to refer to any available diagnostic method, including those mentioned above.

In certain embodiments, a sample containing genomic DNA from an individual is collected. Such sample can for example be a buccal swab, a saliva sample, a blood sample, or other suitable samples containing genomic DNA, as described further herein. The genomic DNA is then analyzed using any common technique available to the skilled person, such as high-throughput array technologies. Results from such genotyping are stored in a convenient data storage unit, such as a data carrier, including computer databases, data storage disks, or by other convenient data storage means. In certain embodiments, the computer database is an object database, a relational database or a post-relational database. The genotype data is subsequently analyzed for the presence of certain variants known to be susceptibility variants for a particular human condition, such as the genetic variants described herein. Genotype data can be retrieved from the data storage unit using any convenient data query method. Calculating risk conferred by a particular genotype for the individual can be based on comparing the genotype of the individual to previously determined risk (expressed as a relative risk (RR) or and odds ratio (OR), for example) for the genotype, for example for an heterozygous carrier of an at-risk variant for a particular disease or trait (such as cancer). The calculated risk for the individual can be the relative risk for a person, or for a specific genotype of a person, compared to the average population with matched gender and ethnicity. The average population risk can be expressed as a weighted average of the risks of different genotypes, using results from a reference population, and the appropriate calculations to calculate the risk of a genotype group relative to the population can then be performed. Alternatively, the risk for an individual is based on a comparison of particular genotypes, for example heterozygous carriers of an at-risk allele of a marker compared with non-carriers of the at-risk allele. Using the population average may in certain embodiments be more convenient, since it provides a measure which is easy to interpret for the user, i.e. a measure that gives the risk for the individual, based on his/her genotype, compared with the average in the population. The calculated risk estimated can be made available to the customer via a website, preferably a secure website.

In certain embodiments, a service provider will include in the provided service all of the steps of isolating genomic DNA from a sample provided by the customer, performing genotyping of the isolated DNA, calculating genetic risk based on the genotype data, and report the risk to the customer. In some other embodiments, the service provider will include in the service the interpretation of genotype data for the individual, *i.e.,* risk estimates for particular genetic variants based on the genotype data for the individual. In some other embodiments, the service provider may include service that includes genotyping service and interpretation of the genotype data, starting from a sample of isolated DNA from the individual (the customer).

Overall risk for multiple risk variants can be performed using standard methodology. For example, assuming a multiplicative model, *i.e.* assuming that the risk of individual risk variants multiply to establish the overall effect, allows for a straight-forward calculation of the overall risk for multiple markers.

In addition, in certain other embodiments, the present invention pertains to methods of determining a decreased susceptibility to cancer, by detecting particular genetic marker alleles or haplotypes that appear less frequently in patients with cancer than in individuals not diagnosed with cancer, or in the general population.

As described and exemplified herein, particular marker alleles or haplotypes (*e.g.* markers on chromosome 5p13.3, *e.g*. rs401681, rs2736100 and rs2736098, and markers in linkage disequilibrium therewith) are associated with cancer. In one embodiment, the marker allele or haplotype is one that confers a significant risk or susceptibility to cancer. In another embodiment, the invention relates to a method of determining a susceptibility to cancer in a human individual, the method comprising determining the presence or absence of at least one allele of at least one polymorphic marker in a nucleic acid sample obtained from the individual, wherein the at least one polymorphic marker is defined in the claims. In another embodiment, the marker allele or haplotype is more frequently present in a subject having, or who is susceptible to, cancer (affected), as compared to the frequency of its presence in a healthy subject (control, such as population controls). In certain embodiments, the significance of association of the at least one marker allele or haplotype is characterized by a p value $< 0.05$. In other embodiments, the significance of association is characterized by smaller p-values, such as $< 0.01$, $<0.001$, $<0.0001$, $<0.00001$, $<0.000001$, $<0.0000001$, $<0.00000001$ or $<0.000000001$.

In these embodiments, the presence of the at least one marker allele or haplotype is indicative of a susceptibility to cancer. These diagnostic methods involve determining whether particular alleles or haplotypes that are associated with risk of cancer are present in particular individuals. The haplotypes described herein include combinations of alleles at various genetic markers (*e.g.*, SNPs, microsatellites or other genetic variants). The detection of the particular genetic marker alleles that make up particular haplotypes can be performed by a variety of methods described herein and/or known in the art. For example, genetic markers can be detected at the nucleic acid level (*e.g.*, by direct nucleotide sequencing, or by other genotyping means known to the skilled in the art) or at the amino acid level if the genetic marker affects the coding sequence of a protein (*e.g.*, by protein sequencing or by immunoassays using antibodies that recognize such a protein). The marker alleles or haplotypes of the present invention correspond to fragments of a genomic segments (*e.g.*, genes) associated with cancer. Such fragments encompass the DNA sequence of the polymorphic marker or haplotype in question, but may also include DNA segments in strong LD (linkage disequilibrium) with the marker or haplotype..

In one embodiment, determination of a susceptibility to cancer can be accomplished using hybridization methods. (see Current Protocols in Molecular Biology, Ausubel, F. et al., eds., John Wiley & Sons, including all supplements). The presence of a specific marker allele can be indicated by sequence-specific hybridization of a nucleic acid probe specific for the particular allele. The presence of more than one specific marker allele or a specific haplotype can be indicated by using several sequence-specific nucleic acid probes, each being specific for a particular allele. A sequence-specific probe can be directed to hybridize to genomic DNA, RNA, or cDNA. A "nucleic acid probe", as used herein, can be a DNA probe or an RNA probe that hybridizes to a complementary sequence. One of skill in the art would know how to design such a probe so that sequence specific hybridization will occur only if a particular allele is present in a genomic sequence from a test sample. The invention can also be reduced to practice using any convenient genotyping method, including commercially available technologies and methods for genotyping particular polymorphic markers.

To determine a susceptibility to cancer, a hybridization sample can be formed by contacting the test sample containing an cancer-associated nucleic acid, such as a genomic DNA sample, with at least one nucleic acid probe. A non-limiting example of a probe for detecting mRNA or genomic DNA is a labeled nucleic acid probe that is capable of hybridizing to mRNA or genomic DNA sequences described herein. The nucleic acid probe can be, for example, a full-length nucleic acid molecule, or a portion thereof, such as an oligonucleotide of at least 15, 30, 50, 100, 250 or 500 nucleotides in length that is sufficient to specifically hybridize under stringent conditions to appropriate mRNA or genomic DNA. For example, the nucleic acid probe can comprise all or a portion of the nucleotide sequence of SEQ ID NO:1, as described herein, optionally comprising at least one allele of a marker described herein, or at least one haplotype described herein, or the probe can be the complementary sequence of such a sequence. The nucleic acid probe can also comprise all or a portion of the nucleotide sequence of the *TERT gene.* In a particular embodiment, the nucleic acid probe is a portion of the nucleotide sequence of SEQ ID NO:1, as described herein, optionally comprising at least one allele of at least one of the polymorphic markers set forth in Table 5 herein, or the probe can be the complementary sequence of such a sequence. Other suitable probes for use in the diagnostic assays of the invention are described herein. Hybridization can be performed by methods well known to the person skilled in the art (see, *e.g.*, Current Protocols in Molecular Biology, Ausubel, F. et al., eds., John Wiley & Sons, including all supplements). In one embodiment, hybridization refers to specific hybridization, i.e., hybridization with no mismatches (exact hybridization). In one embodiment, the hybridization conditions for specific hybridization are high stringency.

Specific hybridization, if present, is detected using standard methods. If specific hybridization occurs between the nucleic acid probe and the nucleic acid in the test sample, then the sample contains the allele that is complementary to the nucleotide that is present in the nucleic acid probe. The process can be repeated for any markers of the present invention, or markers that make up a haplotype of the present invention, or multiple probes can be used concurrently to detect more than one marker alleles at a time. It is also possible to design a single probe containing more than one marker alleles of a particular haplotype (*e.g.*, a probe containing alleles complementary to 2, 3, 4, 5 or all of the markers that make up a particular haplotype). Detection of the particular markers of the haplotype in the sample is indicative that the source of the sample has the particular haplotype (*e.g.*, a haplotype) and therefore is susceptible to cancer.

In one preferred embodiment, a method utilizing a detection oligonucleotide probe comprising a fluorescent moiety or group at its 3' terminus and a quencher at its 5' terminus, and an enhancer oligonucleotide, is employed, as described by Kutyavin *et al.* *(Nucleic Acid Res.* **34:**e128 (2006)). The fluorescent moiety can be Gig Harbor Green or Yakima Yellow, or other suitable fluorescent moieties. The detection probe is designed to hybridize to a short nucleotide sequence that includes the SNP polymorphism to be detected. Preferably, the SNP is anywhere from the terminal residue to -6 residues from the 3' end of the detection probe. The enhancer is a short oligonucleotide probe which hybridizes to the DNA template 3' relative to the detection probe. The probes are designed such that a single nucleotide gap exists between the detection probe and the enhancer nucleotide probe when both are bound to the template. The gap creates a synthetic abasic site that is recognized by an endonuclease, such as Endonuclease IV. The enzyme cleaves the dye off the fully complementary detection probe, but cannot cleave a detection probe containing a mismatch. Thus, by measuring the fluorescence of the released fluorescent moiety, assessment of the presence of a particular allele defined by nucleotide sequence of the detection probe can be performed.

The detection probe can be of any suitable size, although preferably the probe is relatively short. In one embodiment, the probe is from 5-100 nucleotides in length. In another embodiment, the probe is from 10-50 nucleotides in length, and in another embodiment, the probe is from 12-30 nucleotides in length. Other lengths of the probe are possible and within scope of the skill of the average person skilled in the art.

In a preferred embodiment, the DNA template containing the SNP polymorphism is amplified by Polymerase Chain Reaction (PCR) prior to detection. In such an embodiment, the amplified DNA serves as the template for the detection probe and the enhancer probe.

Certain embodiments of the detection probe, the enhancer probe, and/or the primers used for amplification of the template by PCR include the use of modified bases, including modified A and modified G. The use of modified bases can be useful for adjusting the melting temperature of the nucleotide molecule (probe and/or primer) to the template DNA, for example for increasing the melting temperature in regions containing a low percentage of G or C bases, in which modified A with the capability of forming three hydrogen bonds to its complementary T can be used, or for decreasing the melting temperature in regions containing a high percentage of G or C bases, for example by using modified G bases that form only two hydrogen bonds to their complementary C base in a double stranded DNA molecule. In a preferred embodiment, modified bases are used in the design of the detection nucleotide probe. Any modified base known to the skilled person can be selected in these methods, and the selection of suitable bases is well within the scope of the skilled person based on the teachings herein and known bases available from commercial sources as known to the skilled person.

Alternatively, a peptide nucleic acid (PNA) probe can be used in addition to, or instead of, a nucleic acid probe in the hybridization methods described herein. A PNA is a DNA mimic having a peptide-like, inorganic backbone, such as N-(2-aminoethyl)glycine units, with an organic base (A, G, C, T or U) attached to the glycine nitrogen via a methylene carbonyl linker (see, for example, Nielsen, P., et al., Bioconjug. Chem. 5:3-7 (1994)). The PNA probe can be designed to specifically hybridize to a molecule in a sample suspected of containing one or more of the marker alleles or haplotypes that are associated with cancer. Hybridization of the PNA probe is thus diagnostic for cancer or a susceptibility to cancer.

In one embodiment of the invention, a test sample containing genomic DNA obtained from the subject is collected and the polymerase chain reaction (PCR) is used to amplify a fragment comprising one or more markers or haplotypes of the present invention. As described herein, identification of a particular marker allele or haplotype can be accomplished using a variety of methods (*e.g.*, sequence analysis, analysis by restriction digestion, specific hybridization, single stranded conformation polymorphism assays (SSCP), electrophoretic analysis, etc.). In another embodiment, diagnosis is accomplished by expression analysis, for example by using quantitative PCR (kinetic thermal cycling). This technique can, for example, utilize commercially available technologies, such as TaqMan® (Applied Biosystems, Foster City, CA). The technique can assess the presence of an alteration in the expression or composition of a polypeptide or splicing variant(s). Further, the expression of the variant(s) can be quantified as physically or functionally different.

In another embodiment of the methods of the invention, analysis by restriction digestion can be used to detect a particular allele if the allele results in the creation or elimination of a restriction site relative to a reference sequence. Restriction fragment length polymorphism (RFLP) analysis can be conducted, *e.g.*, as described in Current Protocols in Molecular Biology, *supra.* The digestion pattern of the relevant DNA fragment indicates the presence or absence of the particular allele in the sample.

Sequence analysis can also be used to detect specific alleles or haplotypes. Therefore, in one embodiment, determination of the presence or absence of a particular marker alleles or haplotypes comprises sequence analysis of a test sample of DNA or RNA obtained from a subject or individual. PCR or other appropriate methods can be used to amplify a portion of a nucleic acid that contains a polymorphic marker or haplotype, and the presence of specific alleles can then be detected directly by sequencing the polymorphic site (or multiple polymorphic sites in a haplotype) of the genomic DNA in the sample.

In another embodiment, arrays of oligonucleotide probes that are complementary to target nucleic acid sequence segments from a subject, can be used to identify particular alleles at polymorphic sites. For example, an oligonucleotide array can be used. Oligonucleotide arrays typically comprise a plurality of different oligonucleotide probes that are coupled to a surface of a substrate in different known locations. These arrays can generally be produced using mechanical synthesis methods or light directed synthesis methods that incorporate a combination of photolithographic methods and solid phase oligonucleotide synthesis methods, or by other methods known to the person skilled in the art (see, *e.g.,* Bier, F.F., et al. Adv Biochem Eng Biotechnol 109:433-53 (2008); Hoheisel, J.D., Nat Rev Genet 7:200-10 (2006); Fan, J.B., et al. Methods Enzymol 410:57-73 (2006); Raqoussis, J. & Elvidge, G., Expert Rev Mol Diagn 6:145-52 (2006); Mockler, T.C., et al Genomics 85:1-15 (2005), and references cited therein). Many additional descriptions of the preparation and use of oligonucleotide arrays for detection of polymorphisms can be found, for example, in US 6,858,394, US 6,429,027, US 5,445,934, US 5,700,637, US 5,744,305, US 5,945,334, US 6,054,270, US 6,300,063, US 6,733,977, US 7,364,858, EP 619 321, and EP 373 203.

Other methods of nucleic acid analysis that are available to those skilled in the art can be used to detect a particular allele at a polymorphic site. Representative methods include, for example, direct manual sequencing (Church and Gilbert, Proc. Natl. Acad. Sci. USA, 81: 1991-1995 (1988); Sanger, F., et al., Proc. Natl. Acad. Sci. USA, 74:5463-5467 (1977); Beavis, et al., U.S. Patent No. 5,288,644); automated fluorescent sequencing; single-stranded conformation polymorphism assays (SSCP); clamped denaturing gel electrophoresis (CDGE); denaturing gradient gel electrophoresis (DGGE) (Sheffield, V., et al., Proc. Natl. Acad. Sci. USA, 86:232-236 (1989)), mobility shift analysis (Orita, M., et al., Proc. Natl. Acad. Sci. USA, 86:2766-2770 (1989)), restriction enzyme analysis (Flavell, R., et al., Cell, 15:25-41 (1978); Geever, R., et al., Proc. Natl. Acad. Sci. USA, 78:5081-5085 (1981)); heteroduplex analysis; chemical mismatch cleavage (CMC) (Cotton, R., et al., Proc. Natl. Acad. Sci. USA, 85:4397-4401 (1985)); RNase protection assays (Myers, R., et al., Science, 230:1242-1246 (1985); use of polypeptides that recognize nucleotide mismatches, such as *E. coli* mutS protein; and allele-specific PCR.

In another embodiment of the invention, diagnosis of cancer or a determination of a susceptibility to cancer can be made by examining expression and/or composition of a polypeptide encoded by a nucleic acid associated with cancer in those instances where the genetic marker(s) or haplotype(s) of the present invention result in a change in the composition or expression of the polypeptide. Thus, determination of a susceptibility to cancer can be made by examining expression and/or composition of one of these polypeptides, or another polypeptide encoded by a nucleic acid associated with cancer, in those instances where the genetic marker or haplotype of the present invention results in a change in the composition or expression of the polypeptide. The markers of the present invention that show association to cancer may play a role through their effect on one or more of these nearby genes. In certain embodiments, the markers show an effect on the FoxE1 gene. Possible mechanisms affecting these genes (e.g., the *TERT or CLPTM1L* genes) include, *e.g.*, effects on transcription, effects on RNA splicing, alterations in relative amounts of alternative splice forms of mRNA, effects on RNA stability, effects on transport from the nucleus to cytoplasm, and effects on the efficiency and accuracy of translation.

Thus, in another embodiment, the variants (markers or haplotypes) presented herein affect the expression of the *TERT gene* and/or the *CLPTM1L* gene. It is well known that regulatory element affecting gene expression may be located far away, even as far as tenths or hundreds of kilobases away, from the promoter region of a gene. Variants within such regions may thus affect the expression of distant genes affected by the regulatory region. Thus, by assaying for the presence or absence of at least one allele of at least one polymorphic marker within such regions, it is thus possible to assess the expression level of affected genes. It is thus contemplated that the detection of the markers as described herein, or haplotypes comprising such markers, can be used for assessing and/or predicting the expression of the *TERT gene* and/or the *CLPTM1L* gene, or another nearby gene associated with any one of the markers shown herein to confer risk of cancer.

A variety of methods can be used for detecting protein expression levels, including enzyme linked immunosorbent assays (ELISA), Western blots, immunoprecipitations and immunofluorescence. A test sample from a subject is assessed for the presence of an alteration in the expression and/or an alteration in composition of the polypeptide encoded by a particular nucleic acid. An alteration in expression of a polypeptide encoded by the nucleic acid can be, for example, an alteration in the quantitative polypeptide expression (i.e., the amount of polypeptide produced). An alteration in the composition of a polypeptide encoded by the nucleic acid is an alteration in the qualitative polypeptide expression (*e.g.*, expression of a mutant polypeptide or of a different splicing variant). In one embodiment, diagnosis of a susceptibility to cancer is made by detecting a particular splicing variant encoded by a nucleic acid associated with cancer, or a particular pattern of splicing variants.

Both such alterations (quantitative and qualitative) can also be present. An "alteration" in the polypeptide expression or composition, as used herein, refers to an alteration in expression or composition in a test sample, as compared to the expression or composition of the polypeptide in a control sample. A control sample is a sample that corresponds to the test sample (*e.g.*, is from the same type of cells), and is from a subject who is not affected by, and/or who does not have a susceptibility to, cancer. In one embodiment, the control sample is from a subject that does not possess a marker allele or haplotype associated with cancer, as described herein. Similarly, the presence of one or more different splicing variants in the test sample, or the presence of significantly different amounts of different splicing variants in the test sample, as compared with the control sample, can be indicative of a susceptibility to cancer. An alteration in the expression or composition of the polypeptide in the test sample, as compared with the control sample, can be indicative of a specific allele in the instance where the allele alters a splice site relative to the reference in the control sample. Various means of examining expression or composition of a polypeptide encoded by a nucleic acid are known to the person skilled in the art and can be used, including spectroscopy, colorimetry, electrophoresis, isoelectric focusing, and immunoassays (*e.g.*, David et al., U.S. Pat. No. 4,376,110) such as immunoblotting (see, *e.g.*, Current Protocols in Molecular Biology, particularly chapter 10, *supra).*

For example, in one embodiment, an antibody (*e.g.*, an antibody with a detectable label) that is capable of binding to a polypeptide encoded by a nucleic acid associated with cancer can be used. Antibodies can be polyclonal or monoclonal. An intact antibody, or a fragment thereof (*e.g.*, Fv, Fab, Fab', $F(ab')_2$) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a labeled secondary antibody (*e.g.*, a fluorescently-labeled secondary antibody) and end-labeling of a DNA probe with biotin such that it can be detected with fluorescently- labeled streptavidin.

In one embodiment of this method, the level or amount of a polypeptide in a test sample is compared with the level or amount of the polypeptide in a control sample. A level or amount of the polypeptide in the test sample that is higher or lower than the level or amount of the polypeptide in the control sample, such that the difference is statistically significant, is indicative of an alteration in the expression of the polypeptide encoded by the nucleic acid, and is diagnostic for a particular allele or haplotype responsible for causing the difference in expression. Alternatively, the composition of the polypeptide in a test sample is compared with the composition of the polypeptide in a control sample. In another embodiment, both the level or amount and the composition of the polypeptide can be assessed in the test sample and in the control sample.

In another embodiment, determination of a susceptibility to cancer is made by detecting at least one marker or haplotype of the present invention, in combination with an additional protein-based, RNA-based or DNA-based assay.

*Kits*

Kits useful in the methods disclosed herein comprise components useful in any of the methods described herein, including for example, primers for nucleic acid amplification, hybridization probes, restriction enzymes (*e.g.*, for RFLP analysis), allele-specific oligonucleotides, antibodies that bind to an altered polypeptide encoded by a nucleic acid of the invention as described herein (*e.g.,* a genomic segment comprising at least one polymorphic marker and/or haplotype of the present invention) or to a non-altered (native) polypeptide encoded by a nucleic acid of the invention as described herein, means for amplification of a nucleic acid associated with cancer, means for analyzing the nucleic acid sequence of a nucleic acid associated with cancer, means for analyzing the amino acid sequence of a polypeptide encoded by a nucleic acid associated with cancer, etc. The kits can for example include necessary buffers, nucleic acid primers for amplifying nucleic acids of the invention (*e.g.*, a nucleic acid segment comprising one or more of the polymorphic markers as described herein), and reagents for allele-specific detection of the fragments amplified using such primers and necessary enzymes (*e.g.*, DNA polymerase). Additionally, kits can provide reagents for assays to be used in combination with the methods of the present invention, *e.g.*, reagents for use with other diagnostic assays for cancer.

A useful kit for assaying a sample from a subject to detect a susceptibility to cancer in a subject comprises reagents necessary for selectively detecting at least one allele of at least one polymorphism of the present invention in the genome of the individual. In a particular embodiment, the reagents comprise at least one contiguous oligonucleotide that hybridizes to a fragment of the genome of the individual comprising at least one polymorphism of the present invention. In another embodiment, the reagents comprise at least one pair of oligonucleotides that hybridize to opposite strands of a genomic segment obtained from a subject, wherein each oligonucleotide primer pair is designed to selectively amplify a fragment of the genome of the individual that includes at least one polymorphism associated with cancer risk. In one such embodiment, the polymorphism is selected from the group consisting of the polymorphisms as set forth in Tables 5, 6, 7 and 8 herein. In another embodiment, the polymorphism is selected from rs401681, rs2736100 and rs2736098, and markers in linkage disequilibrium therewith. In another embodiment, the polymorphism is selected from rs401681, rs2736100 and rs2736098. In yet another embodiment the fragment is at least 20 base pairs in size. Such oligonucleotides or nucleic acids (*e.g.*, oligonucleotide primers) can be designed using portions of the nucleic acid sequence flanking polymorphisms (*e.g.*, SNPs or microsatellites) that are associated with risk of cancer. In another embodiment, the kit comprises one or more labeled nucleic acids capable of allele-specific detection of one or more specific polymorphic markers or haplotypes, and reagents for detection of the label. Suitable labels include, *e.g.,* a radioisotope, a fluorescent label, an enzyme label, an enzyme co-factor label, a magnetic label, a spin label, an epitope label.

In particular embodiments, the polymorphic marker or haplotype to be detected by the reagents of the kit comprises one or more markers, two or more markers, three or more markers, four or more markers or five or more markers selected from the group consisting of the markers set forth in Tables 5, 6, 7 and 8. In another embodiment, the marker or haplotype to be detected comprises one or more markers, two or more markers, three or more markers, four or more markers or five or more markers selected from the group consisting of the markers rs401681, rs2736100 and rs2736098, and markers in linkage disequilibrium therewith. In another embodiment, the marker to be detected is selected from marker rs401681, rs2736100 and rs2736098.

In one preferred embodiment, the kit for detecting the markers of the invention comprises a detection oligonucleotide probe, that hybridizes to a segment of template DNA containing a SNP polymorphisms to be detected, an enhancer oligonucleotide probe and an endonuclease. As explained in the above, the detection oligonucleotide probe comprises a fluorescent moiety or group at its 3' terminus and a quencher at its 5' terminus, and an enhancer oligonucleotide, is employed, as described by Kutyavin et al. (Nucleic Acid Res. 34:e128 (2006)). The fluorescent moiety can be Gig Harbor Green or Yakima Yellow, or other suitable fluorescent moieties. The detection probe is designed to hybridize to a short nucleotide sequence that includes the SNP polymorphism to be detected. Preferably, the SNP is anywhere from the terminal residue to -6 residues from the 3' end of the detection probe. The enhancer is a short oligonucleotide probe which hybridizes to the DNA template 3' relative to the detection probe. The probes are designed such that a single nucleotide gap exists between the detection probe and the enhancer nucleotide probe when both are bound to the template. The gap creates a synthetic abasic site that is recognized by an endonuclease, such as Endonuclease IV. The enzyme cleaves the dye off the fully complementary detection probe, but cannot cleave a detection probe containing a mismatch. Thus, by measuring the fluorescence of the released fluorescent moiety, assessment of the presence of a particular allele defined by nucleotide sequence of the detection probe can be performed.

The detection probe can be of any suitable size, although preferably the probe is relatively short. In one embodiment, the probe is from 5-100 nucleotides in length. In another embodiment, the probe is from 10-50 nucleotides in length, and in another embodiment, the probe is from 12-30 nucleotides in length. Other lengths of the probe are possible and within scope of the skill of the average person skilled in the art.

In a preferred embodiment, the DNA template containing the SNP polymorphism is amplified by Polymerase Chain Reaction (PCR) prior to detection, and primers for such amplification are included in the reagent kit. In such an embodiment, the amplified DNA serves as the template for the detection probe and the enhancer probe.

In one embodiment, the DNA template is amplified by means of Whole Genome Amplification (WGA) methods, prior to assessment for the presence of specific polymorphic markers as described herein. Standard methods well known to the skilled person for performing WGA may be utilized, and are within scope of the invention. In one such embodiment, reagents for performing WGA are included in the reagent kit.

Certain embodiments of the detection probe, the enhancer probe, and/or the primers used for amplification of the template by PCR include the use of modified bases, including modified A and modified G. The use of modified bases can be useful for adjusting the melting temperature of the nucleotide molecule (probe and/or primer) to the template DNA, for example for increasing the melting temperature in regions containing a low percentage of G or C bases, in which modified A with the capability of forming three hydrogen bonds to its complementary T can be used, or for decreasing the melting temperature in regions containing a high percentage of G or C bases, for example by using modified G bases that form only two hydrogen bonds to their complementary C base in a double stranded DNA molecule. In a preferred embodiment, modified bases are used in the design of the detection nucleotide probe. Any modified base known to the skilled person can be selected in these methods, and the selection of suitable bases is well within the scope of the skilled person based on the teachings herein and known bases available from commercial sources as known to the skilled person.

In one such embodiment, determination of the presence of the marker or haplotype is indicative of a susceptibility (increased susceptibility or decreased susceptibility) to cancer. In another embodiment, determination of the presence of the marker or haplotype is indicative of response to a therapeutic agent for cancer. In another embodiment, the presence of the marker or haplotype is indicative of prognosis of cancer. In yet another embodiment, the presence of the marker or haplotype is indicative of progress of cancer treatment. Such treatment may include intervention by surgery, medication or by other means (*e.g.*, lifestyle changes).

A pharmaceutical pack (kit) is also useful, the pack comprising a therapeutic agent and a set of instructions for administration of the therapeutic agent to humans diagnostically tested for one or more variants of the present invention, as disclosed herein. The therapeutic agent can be a small molecule drug, an antibody, a peptide, an antisense or RNAi molecule, or other therapeutic molecules. In one embodiment, an individual identified as a carrier of at least one variant of the present invention is instructed to take a prescribed dose of the therapeutic agent. In one such embodiment, an individual identified as a homozygous carrier of at least one variant of the present invention is instructed to take a prescribed dose of the therapeutic agent. In another embodiment, an individual identified as a non-carrier of at least one variant of the present invention is instructed to take a prescribed dose of the therapeutic agent.

In certain embodiments, the kit further comprises a set of instructions for using the reagents comprising the kit.

*Therapeutic agents and methods*

Treatment options for cancer include current standard treatment methods and those that are in clinical trials. Aspects of the disclosure relating to the use of risk markers for cancer for predicting therapeutic outcome of a particular treatment module, or aspects relating to the application of certain treatment modules for the particular cancer, are contemplated to be useful in the context any therapeutic agents and methods of treating cancer.

Current treatment options for cancer include:

*Treatment by Surgery.* In principle, non-hematological cancers can be cured if entirely removed by surgery, but this is not always possible. When the cancer has metastasized to other sites in the body prior to surgery, complete surgical excision is usually impossible. In the Halstedian model of cancer progression, tumors grow locally, then spread to the lymph nodes, then to the rest of the body. This has given rise to the popularity of local-only treatments such as surgery for small cancers. Even small localized tumors are increasingly recognized as possessing metastatic potential.

Examples of surgical procedures for cancer include mastectomy for breast cancer and prostatectomy for prostate cancer. The goal of the surgery can be either the removal of only the tumor, or the entire organ. A single cancer cell is invisible to the naked eye but can regrow into a new tumor, a process called recurrence. For this reason, the pathologist will examine the surgical specimen to determine if a margin of healthy tissue is present, thus decreasing the chance that microscopic cancer cells are left in the patient.

In addition to removal of the primary tumor, surgery is often necessary for staging, e.g. determining the extent of the disease and whether it has metastasized to regional lymph nodes. Staging is a major determinant of prognosis and of the need for adjuvant therapy.

Occasionally, surgery is necessary to control symptoms, such as spinal cord compression or bowel obstruction. This is referred to as palliative treatment.

*Treatment by Radiation therapy.* Also called radiotherapy, X-ray therapy, or irradiation, radiation therapy is the use of ionizing radiation to kill cancer cells and shrink tumors. Radiation therapy can be administered externally via external beam radiotherapy (EBRT) or internally via brachytherapy. The effects of radiation therapy are localised and confined to the region being treated. Radiation therapy injures or destroys cells in the area being treated (the "target tissue") by damaging their genetic material, making it impossible for these cells to continue to grow and divide. Although radiation damages both cancer cells and normal cells, most normal cells can recover from the effects of radiation and function properly. The goal of radiation therapy is to damage as many cancer cells as possible, while limiting harm to nearby healthy tissue.

Radiation therapy may be used to treat almost every type of solid tumor, including cancers of the brain, breast, cervix, larynx, lung, pancreas, prostate, skin, stomach, uterus, or soft tissue sarcomas. Radiation is also used to treat leukemia and lymphoma. Radiation dose to each site depends on a number of factors, including the radiosensitivity of each cancer type and whether there are tissues and organs nearby that may be damaged by radiation.

*Treatment by Chemotherapy.* Chemotherapy is the treatment of cancer with drugs ("anticancer drugs") that can destroy cancer cells. In current usage, the term usually refers to *cytotoxic* drugs which affect rapidly dividing cells in general, in contrast with *targeted therapy.* Chemotherapy drugs interfere with cell division in various possible ways, e.g. with the duplication of DNA or the separation of newly formed chromosomes. Most forms of chemotherapy target all rapidly dividing cells and are not specific for cancer cells, although some degree of specificity may come from the inability of many cancer cells to repair DNA damage, while normal cells generally can. Hence, chemotherapy has the potential to harm healthy tissue, especially those tissues that have a high replacement rate (e.g. intestinal lining). These cells usually repair themselves after chemotherapy.

Because some drugs work better together than alone, two or more drugs are often given at the same time. This is called "combination chemotherapy"; most chemotherapy regimens are given in a combination.

The treatment of some leukaemia's and lymphomas requires the use of high-dose chemotherapy, and total body irradiation (TBI). This treatment ablates the bone marrow, and hence the body's ability to recover and repopulate the blood. For this reason, bone marrow, or peripheral blood stem cell harvesting is carried out before the ablative part of the therapy, to enable "rescue" after the treatment has been given. This is known as autologous stem cell transplantation. Alternatively, hematopoietic stem cells may be transplanted from a matched unrelated donor (MUD).

*Treatment by Targeted Therapy.* Targeted therapy constitutes the use of agents specific for the deregulated proteins of cancer cells. Small molecule targeted therapy drugs are generally inhibitors of enzymatic domains on mutated, overexpressed, or otherwise critical proteins within the cancer cell. Prominent examples are the tyrosine kinase inhibitors imatinib and gefitinib.

Monoclonal antibody therapy is another strategy in which the therapeutic agent is an antibody which specifically binds to a protein on the surface of the cancer cells. Examples include the anti-HER2/neu antibody trastuzumab (Herceptin) used in breast cancer, and the anti-CD20 antibody rituximab, used in a variety of B-cell malignancies.

Targeted therapy can also involve small peptides as "homing devices" which can bind to cell surface receptors or affected extracellular matrix surrounding the tumor. Radionuclides which are attached to this peptides (e.g. RGDs) eventually kill the cancer cell if the nuclide decays in the vicinity of the cell. Especially oligo- or multimers of these binding motifs are of great interest, since this can lead to enhanced tumor specificity and avidity.

Photodynamic therapy (PDT) is a ternary treatment for cancer involving a photosensitizer, tissue oxygen, and light (often using lasers). PDT can be used as treatment for basal cell carcinoma (BCC) or lung cancer; PDT can also be useful in removing traces of malignant tissue after surgical removal of large tumors.

*Treatment by Immunotherapy.* Cancer immunotherapy refers to a diverse set of therapeutic strategies designed to induce the patient's own immune system to fight the tumor. Contemporary methods for generating an immune response against tumours include intravesical BCG immunotherapy for superficial bladder cancer, and use of interferons and other cytokines to induce an immune response in renal cell carcinoma and melanoma patients. Vaccines to generate specific immune responses are the subject of intensive research for a number of tumours, notably malignant melanoma and renal cell carcinoma. Sipuleucel-T is a vaccine-like strategy in late clinical trials for prostate cancer in which dendritic cells from the patient are loaded with prostatic acid phosphatase peptides to induce a specific immune response against prostate-derived cells.

Allogeneic hematopoietic stem cell transplantation ("bone marrow transplantation" from a genetically non-identical donor) can be considered a form of immunotherapy, since the donor's immune cells will often attack the tumor in a phenomenon known as graft-versus-tumor effect. For this reason, allogeneic HSCT leads to a higher cure rate than autologous transplantation for several cancer types, although the side effects are also more severe.

*Treatment by Hormonal Therapy.* The growth of some cancers can be inhibited by providing or blocking certain hormones. Common examples of hormone-sensitive tumors include certain types of breast and prostate cancers. Removing or blocking estrogen or testosterone is often an important additional treatment. In certain cancers, administration of hormone agonists, such as progestogens may be therapeutically beneficial.

*Treatment by Angiogenesis inhibitors.* Angiogenesis inhibitors prevent the extensive growth of blood vessels (angiogenesis) that tumors require to survive. Some, such as bevacizumab, have been approved and are in clinical use.

*Symptom control.* Although the control of the symptoms of cancer is not typically thought of as a treatment directed at the cancer, it is an important determinant of the quality of life of cancer patients, and plays an important role in the decision whether the patient is able to undergo other treatments. Although doctors generally have the therapeutic skills to reduce pain, nausea, vomiting, diarrhea, hemorrhage and other common problems in cancer patients, the multidisciplinary specialty of palliative care has arisen specifically in response to the symptom control needs of this group of patients.

Pain medication, such as morphine and oxycodone, and antiemetics, drugs to suppress nausea and vomiting, are very commonly used in patients with cancer-related symptoms. Improved antiemetics such as ondansetron and analogues, as well as aprepitant have made aggressive treatments much more feasible in cancer patients.

Chronic pain due to cancer is almost always associated with continuing tissue damage due to the disease process or the treatment (i.e. surgery, radiation, chemotherapy). Although there is always a role for environmental factors and affective disturbances in the genesis of pain behaviours, these are not usually the predominant etiologic factors in patients with cancer pain. Furthermore, many patients with severe pain associated with cancer are nearing the end of their lives and palliative therapies are required. The typical strategy for cancer pain management is to get the patient as comfortable as possible using opioids and other medications, surgery, and physical measures.

The variants disclosed herein to confer increased risk of cancer can also be used to identify novel therapeutic targets for cancer. For example, genes containing, or in linkage disequilibrium with, one or more of these variants, or their products (e.g., the *TERT* gene, the *CLPTM1L* gene and their gene products), as well as genes or their products that are directly or indirectly regulated by or interact with these genes or their products, can be targeted for the development of therapeutic agents to treat cancer, or prevent or delay onset of symptoms associated with cancer. Therapeutic agents may comprise one or more of, for example, small non-protein and non-nucleic acid molecules, proteins, peptides, protein fragments, nucleic acids (DNA, RNA), PNA (peptide nucleic acids), or their derivatives or mimetics which can modulate the function and/or levels of the target genes or their gene products.

The nucleic acids and/or variants or nucleic acids comprising their complementary sequence, may be used as antisense constructs to control gene expression in cells, tissues or organs. The methodology associated with antisense techniques is well known to the skilled artisan, and is described and reviewed in AntisenseDrug Technology: Principles, Strategies, and Applications, Crooke, ed., Marcel Dekker Inc., New York (2001). In general, antisense nucleic acid molecules are designed to be complementary to a region of mRNA expressed by a gene, so that the antisense molecule hybridizes to the mRNA, thus blocking translation of the mRNA into protein. Several classes of antisense oligonucleotide are known to those skilled in the art, including cleavers and blockers. The former bind to target RNA sites, activate intracellular nucleases (*e.g.*, RnaseH or Rnase L), that cleave the target RNA. Blockers bind to target RNA, inhibit protein translation by steric hindrance of the ribosomes. Examples of blockers include nucleic acids, morpholino compounds, locked nucleic acids and methylphosphonates (Thompson, Drug Discovery Today, 7:912-917 (2002)). Antisense oligonucleotides are useful directly as therapeutic agents, and are also useful for determining and validating gene function, for example by gene knock-out or gene knock-down experiments. Antisense technology is further described in Lavery et al., Curr. Opin. Drug Discov. Devel. 6:561-569 (2003), Stephens et al., Curr. Opin. Mol. Ther. 5:118-122 (2003), Kurreck, Eur. J. Biochem. 270:1628-44 (2003), Dias et al., Mol. Cancer Ter. 1:347-55 (2002), Chen, Methods Mol. Med. 75:621-636 (2003), Wang et al., Curr. Cancer Drug Targets 1:177-96 (2001), and Bennett, Antisense Nucleic Acid Drug.Dev. 12:215-24 (2002)

The variants described herein can be used for the selection and design of antisense reagents that are specific for particular variants. Using information about the variants described herein, antisense oligonucleotides or other antisense molecules that specifically target mRNA molecules that contain one or more variants of the invention can be designed. In this manner, expression of mRNA molecules that contain one or more variant of the present invention (markers and/or haplotypes) can be inhibited or blocked. In one embodiment, the antisense molecules are designed to specifically bind a particular allelic form (i.e., one or several variants (alleles and/or haplotypes)) of the target nucleic acid, thereby inhibiting translation of a product originating from this specific allele or haplotype, but which do not bind other or alternate variants at the specific polymorphic sites of the target nucleic acid molecule.

As antisense molecules can be used to inactivate mRNA so as to inhibit gene expression, and thus protein expression, the molecules can be used for disease treatment. The methodology can involve cleavage by means of ribozymes containing nucleotide sequences complementary to one or more regions in the mRNA that attenuate the ability of the mRNA to be translated. Such mRNA regions include, for example, protein-coding regions, in particular protein-coding regions corresponding to catalytic activity, substrate and/or ligand binding sites, or other functional domains of a protein.

The phenomenon of RNA interference (RNAi) has been actively studied for the last decade, since its original discovery in *C. elegans* (Fire et al.,Nature 391:806-11 (1998)), and in recent years its potential use in treatment of human disease has been actively pursued (reviewed in Kim & Rossi, Nature Rev. Genet. 8:173-204 (2007)). RNA interference (RNAi), also called gene silencing, is based on using double-stranded RNA molecules (dsRNA) to turn off specific genes. In the cell, cytoplasmic double-stranded RNA molecules (dsRNA) are processed by cellular complexes into small interfering RNA (siRNA). The siRNA guide the targeting of a protein-RNA complex to specific sites on a target mRNA, leading to cleavage of the mRNA (Thompson, Drug Discovery Today, 7:912-917 (2002)). The siRNA molecules are typically about 20, 21, 22 or 23 nucleotides in length. Thus, one aspect of the invention relates to isolated nucleic acid molecules, and the use of those molecules for RNA interference, i.e. as small interfering RNA molecules (siRNA). In one embodiment, the isolated nucleic acid molecules are 18-26 nucleotides in length, preferably 19-25 nucleotides in length, more preferably 20-24 nucleotides in length, and more preferably 21, 22 or 23 nucleotides in length.

Another pathway for RNAi-mediated gene silencing originates in endogenously encoded primary microRNA (pri-miRNA) transcripts, which are processed in the cell to generate precursor miRNA (pre-miRNA). These miRNA molecules are exported from the nucleus to the cytoplasm, where they undergo processing to generate mature miRNA molecules (miRNA), which direct translational inhibition by recognizing target sites in the 3' untranslated regions of mRNAs, and subsequent mRNA degradation by processing P-bodies (reviewed in Kim & Rossi, Nature Rev. Genet. 8:173-204 (2007)).

Clinical applications of RNAi include the incorporation of synthetic siRNA duplexes, which preferably are approximately 20-23 nucleotides in size, and preferably have 3' overlaps of 2 nucleotides. Knockdown of gene expression is established by sequence-specific design for the target mRNA. Several commercial sites for optimal design and synthesis of such molecules are known to those skilled in the art.

Other applications provide longer siRNA molecules (typically 25-30 nucleotides in length, preferably about 27 nucleotides), as well as small hairpin RNAs (shRNAs; typically about 29 nucleotides in length). The latter are naturally expressed, as described in Amarzguioui *et al.* (FEBS Lett. 579:5974-81 (2005)). Chemically synthetic siRNAs and shRNAs are substrates for *in vivo* processing, and in some cases provide more potent gene-silencing than shorter designs (Kim et al., Nature Biotechnol. 23:222-226 (2005); Siolas et al., Nature Biotechnol. 23:227-231 (2005)). In general siRNAs provide for transient silencing of gene expression, because their intracellular concentration is diluted by subsequent cell divisions. By contrast, expressed shRNAs mediate long-term, stable knockdown of target transcripts, for as long as transcription of the shRNA takes place (Marques et al., Nature Biotechnol. 23:559-565 (2006); Brummelkamp et al., Science 296: 550-553 (2002)).

Since RNAi molecules, including siRNA, miRNA and shRNA, act in a sequence-dependent manner, the variants presented herein (*e.g.*, the markers and haplotypes set forth in Table 2) can be used to design RNAi reagents that recognize specific nucleic acid molecules comprising specific alleles and/or haplotypes (*e.g.*, the alleles and/or haplotypes of the present invention), while not recognizing nucleic acid molecules comprising other alleles or haplotypes. These RNAi reagents can thus recognize and destroy the target nucleic acid molecules. As with antisense reagents, RNAi reagents can be useful as therapeutic agents (i.e., for turning off disease-associated genes or disease-associated gene variants), but may also be useful for characterizing and validating gene function (*e.g.*, by gene knock-out or gene knock-down experiments).

Delivery of RNAi may be performed by a range of methodologies known to those skilled in the art. Methods utilizing non-viral delivery include cholesterol, stable nucleic acid-lipid particle (SNALP), heavy-chain antibody fragment (Fab), aptamers and nanoparticles. Viral delivery methods include use of lentivirus, adenovirus and adeno-associated virus. The siRNA molecules are in some embodiments chemically modified to increase their stability. This can include modifications at the 2' position of the ribose, including 2'-O-methylpurines and 2'-fluoropyrimidines, which provide resistance to Rnase activity. Other chemical modifications are possible and known to those skilled in the art.

The following references provide a further summary of RNAi, and possibilities for targeting specific genes using RNAi: Kim & Rossi, Nat. Rev. Genet. 8:173-184 (2007), Chen & Rajewsky, Nat. Rev. Genet. 8: 93-103 (2007), Reynolds, et al., Nat. Biotechnol. 22:326-330 (2004), Chi et al., Proc. Natl. Acad. Sci. USA 100:6343-6346 (2003), Vickers et al., J. Biol. Chem. 278:7108-7118 (2003), Agami, Curr. Opin. Chem. Biol. 6:829-834 (2002), Lavery, et al., Curr. Opin. Drug Discov. Devel. 6:561-569 (2003), Shi, Trends Genet. 19:9-12 (2003), Shuey et al., Drug Discov. Today 7:1040-46 (2002), McManus et al., Nat. Rev. Genet. 3:737-747 (2002), Xia et al., Nat. Biotechnol. 20:1006-10 (2002), Plasterk et al., curr. Opin. Genet. Dev. 10:562-7 (2000), Bosher et al., Nat. Cell Biol. 2:E31-6 (2000), and Hunter, Curr. Biol. 9:R440-442 (1999).

A genetic defect leading to increased predisposition or risk for development of a disease, such as cancer, or a defect causing the disease, may be corrected permanently by administering to a subject carrying the defect a nucleic acid fragment that incorporates a repair sequence that supplies the normal/wild-type nucleotide(s) at the site of the genetic defect. Such site-specific repair sequence may concompass an RNA/DNA oligonucleotide that operates to promote endogenous repair of a subject's genomic DNA. The administration of the repair sequence may be performed by an appropriate vehicle, such as a complex with polyethelenimine, encapsulated in anionic liposomes, a viral vector such as an adenovirus vector, or other pharmaceutical compositions suitable for promoting intracellular uptake of the adminstered nucleic acid. The genetic defect may then be overcome, since the chimeric oligonucleotides induce the incorporation of the normal sequence into the genome of the subject, leading to expression of the normal/wild-type gene product. The replacement is propagated, thus rendering a permanent repair and alleviation of the symptoms associated with the disease or condition.

The present invention is useful for identifying compounds or agents that can be used to treat cancer. Thus, the variants of the invention are useful as targets for the identification and/or development of therapeutic agents. Such methods include assaying the ability of an agent or compound to modulate the activity and/or expression of a nucleic acid that includes at least one of the variants (markers and/or haplotypes) of the present invention, or the encoded product of the nucleic acid. In certain embodiments, the agent or compound modulates the activity or expression of the *TERT gene* and/or the *CLPTM1L* gene. The agents or compounds may also inhibit or alter the undesired activity or expression of the encoded nucleic acid product, i.e. the TERT and/or CLPTM1L protein product. Assays for performing such experiments can be performed in cell-based systems or in cell-free systems, as known to the skilled person. Cell-based systems include cells naturally expressing the nucleic acid molecules of interest, or recombinant cells that have been genetically modified so as to express a certain desired nucleic acid molecule.

Variant gene expression in a patient can be assessed by expression of a variant-containing nucleic acid sequence (for example, a gene containing at least one variant of the present invention, which can be transcribed into RNA containing the at least one variant, and in turn translated into protein), or by altered expression of a normal/wild-type nucleic acid sequence due to variants affecting the level or pattern of expression of the normal transcripts, for example variants in the regulatory or control region of the gene. Assays for gene expression include direct nucleic acid assays (mRNA), assays for expressed protein levels, or assays of collateral compounds involved in a pathway, for example a signal pathway. Furthermore, the expression of genes that are up- or down-regulated in response to the signal pathway can also be assayed. One embodiment includes operably linking a reporter gene, such as luciferase, to the regulatory region of the gene(s) of interest.

Modulators of gene expression can in one embodiment be identified when a cell is contacted with a candidate compound or agent, and the expression of mRNA is determined. The expression level of mRNA in the presence of the candidate compound or agent is compared to the expression level in the absence of the compound or agent. Based on this comparison, candidate compounds or agents for treating cancer can be identified as those modulating the gene expression of the variant gene. When expression of mRNA or the encoded protein is statistically significantly greater in the presence of the candidate compound or agent than in its absence, then the candidate compound or agent is identified as a stimulator or up-regulator of expression of the nucleic acid. When nucleic acid expression or protein level is statistically significantly less in the presence of the candidate compound or agent than in its absence, then the candidate compound is identified as an inhibitor or down-regulator of the nucleic acid expression.

*Methods of assessing probability of response to therapeutic agents, methods of monitoring progress of treatment and methods of treatment*

As is known in the art, individuals can have differential responses to a particular therapy (*e.g.*, a therapeutic agent or therapeutic method). Pharmacogenomics addresses the issue of how genetic variations (*e.g.*, the variants (markers and/or haplotypes) of the present invention) affect drug response, due to altered drug disposition and/or abnormal or altered action of the drug. Thus, the basis of the differential response may be genetically determined in part. Clinical outcomes due to genetic variations affecting drug response may result in toxicity of the drug in certain individuals (*e.g.*, carriers or non-carriers of the genetic variants of the present invention), or therapeutic failure of the drug. Therefore, the variants of the present invention may determine the manner in which a therapeutic agent and/or method acts on the body, or the way in which the body metabolizes the therapeutic agent.

Accordingly, in one useful application, the presence of a particular allele at a polymorphic site as described herein, *e.g.* rs401681, rs2736100 and/or rs2736098, or markers in linkage disequilibrium therewith, is indicative of a different response, *e.g.* a different response rate, to a particular treatment modality. This means that a patient diagnosed with cancer, and carrying a certain allele at a polymorphic or haplotype of the present invention (*e.g.*, the at-risk and protective alleles and/or haplotypes of the invention) would respond better to, or worse to, a specific therapeutic, drug and/or other therapy used to treat the disease. Therefore, the presence or absence of the marker allele or haplotype could aid in deciding what treatment should be used for the patient. The treatment may include any of the treatment options described in more detail in the above under *Therapeutic Agents and* Methods. For example, for a newly diagnosed patient, the presence of a marker of the present invention may be assessed (*e.g.*, through testing DNA derived from a blood sample, as described herein). If the patient is positive for a marker allele or haplotype (that is, at least one specific allele of the marker, or haplotype, is present), then the physician recommends one particular therapy, while if the patient is negative for the at least one allele of a marker, or a haplotype, then a different course of therapy may be recommended (which may include recommending that no immediate therapy, other than serial monitoring for progression of the disease, be performed). Thus, the patient's carrier status could be used to help determine whether a particular treatment modality should be administered. The value lies within the possibilities of being able to diagnose the disease at an early stage, to select the most appropriate treatment, and provide information to the clinician about prognosis/aggressiveness of the disease in order to be able to apply the most appropriate treatment.

Any of the treatment methods and compounds described in the above under *Therapeutic agents and Methods* can be used in such methods. I.e., a treatment for cancer using any of the compounds or methods described or contemplated in the above may, in certain embodiments, benefit from screening for the presence of particular alleles for at least one of the polymorphic markers described herein, wherein the presence of the particular allele is predictive of the treatment outcome for the particular compound or method.

In certain useful applications, a therapeutic agent (drug) for treating cancer is provided together with a kit for determining the allelic status at a polymorphic marker as described herein (e.g., rs965513, or markers in linkage disequilibrium therewith). If an individual is positive for the particular allele or plurality of alleles being tested, the individual is more likely to benefit from the particular compound than non-carriers of the allele. In certain other embodiments, genotype information about the at least one polymorphic marker predictive of the treatment outcome of the particular compound is predetermined and stored in a database, in a look-up table or by other suitable means, and can for example be accessed from a database or look-up table by conventional data query methods known to the skilled person. If a particular individual is determined to carry certain alleles predictive of positive treatment outcome of a particular compound or drug for treating cancer, then the individual is likely to benefit from administration of the particular compound.

The present invention also can be useful for monitoring progress or effectiveness of a treatment for cancer. This can be done based on the genotype and/or haplotype status of the markers and haplotypes of the present invention, i.e., by assessing the absence or presence of at least one allele of at least one polymorphic marker as disclosed herein, or by monitoring expression of genes that are associated with the variants (markers and haplotypes) of the present invention. The risk gene mRNA or the encoded polypeptide can be measured in a tissue sample (*e.g.*, a peripheral blood sample, or a biopsy sample). Expression levels and/or mRNA levels can thus be determined before and during treatment to monitor its effectiveness. Alternatively, or concomitantly, the genotype and/or haplotype status of at least one risk variant for cancer as presented herein is determined before and during treatment to monitor its effectiveness.

Alternatively, biological networks or metabolic pathways related to the markers and haplotypes of the present invention can be monitored by determining mRNA and/or polypeptide levels. This can be done for example, by monitoring expression levels or polypeptides for several genes belonging to the network and/or pathway, in samples taken before and during treatment. Alternatively, metabolites belonging to the biological network or metabolic pathway can be determined before and during treatment. Effectiveness of the treatment is determined by comparing observed changes in expression levels/metabolite levels during treatment to corresponding data from healthy subjects.

The markers of the present invention can be used to increase power and effectiveness of clinical trials. Thus, individuals who are carriers of at least one at-risk variant of the present invention may be more likely to respond favorably to a particular treatment modality. In one embodiment, individuals who carry at-risk variants for gene(s) in a pathway and/or metabolic network for which a particular treatment (*e.g.*, small molecule drug) is targeting, are more likely to be responders to the treatment. In another embodiment, individuals who carry at-risk variants for a gene, which expression and/or function is altered by the at-risk variant, are more likely to be responders to a treatment modality targeting that gene, its expression or its gene product. This application can improve the safety of clinical trials, but can also enhance the chance that a clinical trial will demonstrate statistically significant efficacy, which may be limited to a certain subgroup of the population. Thus, one possible outcome of such a trial is that carriers of certain genetic variants, *e.g.*, the markers and haplotypes of the present invention, are statistically significantly likely to show positive response to the therapeutic agent, i.e. experience alleviation of symptoms associated with cancer when taking the therapeutic agent or drug as prescribed.

The markers and haplotypes of the present invention can be used for targeting the selection of pharmaceutical agents for specific individuals. Personalized selection of treatment modalities, lifestyle changes or combination of lifestyle changes and administration of particular treatment, can be realized by the utilization of the at-risk variants of the present invention. Thus, the knowledge of an individual's status for particular markers of the present invention, can be useful for selection of treatment options that target genes or gene products affected by the at-risk variants of the invention. Certain combinations of variants may be suitable for one selection of treatment options, while other gene variant combinations may target other treatment options. Such combination of variant may include one variant, two variants, three variants, or four or more variants, as needed to determine with clinically reliable accuracy the selection of treatment module.

*Computer-implemented aspects*

As understood by those of ordinary skill in the art, the methods and information described herein may be implemented, in all or in part, as computer executable instructions on known computer readable media. For example, the methods described herein may be implemented in hardware. Alternatively, the method may be implemented in software stored in, for example, one or more memories or other computer readable medium and implemented on one or more processors. As is known, the processors may be associated with one or more controllers, calculation units and/or other units of a computer system, or implanted in firmware as desired. If implemented in software, the routines may be stored in any computer readable memory such as in RAM, ROM, flash memory, a magnetic disk, a laser disk, or other storage medium, as is also known. Likewise, this software may be delivered to a computing device via any known delivery method including, for example, over a communication channel such as a telephone line, the Internet, a wireless connection, etc., or via a transportable medium, such as a computer readable disk, flash drive, etc.

More generally, and as understood by those of ordinary skill in the art, the various steps described above may be implemented as various blocks, operations, tools, modules and techniques which, in turn, may be implemented in hardware, firmware, software, or any combination of hardware, firmware, and/or software. When implemented in hardware, some or all of the blocks, operations, techniques, etc. may be implemented in, for example, a custom integrated circuit (IC), an application specific integrated circuit (ASIC), a field programmable logic array (FPGA), a programmable logic array (PLA), etc.

When implemented in software, the software may be stored in any known computer readable medium such as on a magnetic disk, an optical disk, or other storage medium, in a RAM or ROM or flash memory of a computer, processor, hard disk drive, optical disk drive, tape drive, etc. Likewise, the software may be delivered to a user or a computing system via any known delivery method including, for example, on a computer readable disk or other transportable computer storage mechanism.

Fig. 1 illustrates an example of a suitable computing system environment 100 on which a system for the steps of the claimed method and apparatus may be implemented. The computing system environment 100 is only one example of a suitable computing environment and is not intended to suggest any limitation as to the scope of use or functionality of the method or apparatus of the claims. Neither should the computing environment 100 be interpreted as having any dependency or requirement relating to any one or combination of components illustrated in the exemplary operating environment 100.

The steps of the claimed method and system are operational with numerous other general purpose or special purpose computing system environments or configurations. Examples of well known computing systems, environments, and/or configurations that may be suitable for use with the methods or system of the claims include, but are not limited to, personal computers, server computers, hand-held or laptop devices, multiprocessor systems, microprocessor-based systems, set top boxes, programmable consumer electronics, network PCs, minicomputers, mainframe computers, distributed computing environments that include any of the above systems or devices, and the like.

The steps of the claimed method and system may be described in the general context of computer-executable instructions, such as program modules, being executed by a computer. Generally, program modules include routines, programs, objects, components, data structures, etc. that perform particular tasks or implement particular abstract data types. The methods and apparatus may also be practiced in distributed computing environments where tasks are performed by remote processing devices that are linked through a communications network. In both integrated and distributed computing environments, program modules may be located in both local and remote computer storage media including memory storage devices.

With reference to Fig. 1, an exemplary system for implementing the steps of the claimed method and system includes a general purpose computing device in the form of a computer 110. Components of computer 110 may include, but are not limited to, a processing unit 120, a system memory 130, and a system bus 121 that couples various system components including the system memory to the processing unit 120. The system bus 121 may be any of several types of bus structures including a memory bus or memory controller, a peripheral bus, and a local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus also known as Mezzanine bus.

Computer 110 typically includes a variety of computer readable media. Computer readable media can be any available media that can be accessed by computer 110 and includes both volatile and nonvolatile media, removable and non-removable media. By way of example, and not limitation, computer readable media may comprise computer storage media and communication media. Computer storage media includes both volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium which can be used to store the desired information and which can accessed by computer 110. Communication media typically embodies computer readable instructions, data structures, program modules or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared and other wireless media. Combinations of the any of the above should also be included within the scope of computer readable media.

The system memory 130 includes computer storage media in the form of volatile and/or nonvolatile memory such as read only memory (ROM) 131 and random access memory (RAM) 132. A basic input/output system 133 (BIOS), containing the basic routines that help to transfer information between elements within computer 110, such as during start-up, is typically stored in ROM 131. RAM 132 typically contains data and/or program modules that are immediately accessible to and/or presently being operated on by processing unit 120. By way of example, and not limitation, Fig. 1 illustrates operating system 134, application programs 135, other program modules 136, and program data 137.

The computer 110 may also include other removable/non-removable, volatile/nonvolatile computer storage media. By way of example only, Fig. 1 illustrates a hard disk drive 140 that reads from or writes to non-removable, nonvolatile magnetic media, a magnetic disk drive 151 that reads from or writes to a removable, nonvolatile magnetic disk 152, and an optical disk drive 155 that reads from or writes to a removable, nonvolatile optical disk 156 such as a CD ROM or other optical media. Other removable/non-removable, volatile/nonvolatile computer storage media that can be used in the exemplary operating environment include, but are not limited to, magnetic tape cassettes, flash memory cards, digital versatile disks, digital video tape, solid state RAM, solid state ROM, and the like. The hard disk drive 141 is typically connected to the system bus 121 through a non-removable memory interface such as interface 140, and magnetic disk drive 151 and optical disk drive 155 are typically connected to the system bus 121 by a removable memory interface, such as interface 150.

The drives and their associated computer storage media discussed above and illustrated in Fig. 1, provide storage of computer readable instructions, data structures, program modules and other data for the computer 110. In Fig. 1, for example, hard disk drive 141 is illustrated as storing operating system 144, application programs 145, other program modules 146, and program data 147. Note that these components can either be the same as or different from operating system 134, application programs 135, other program modules 136, and program data 137. Operating system 144, application programs 145, other program modules 146, and program data 147 are given different numbers here to illustrate that, at a minimum, they are different copies. A user may enter commands and information into the computer 20 through input devices such as a keyboard 162 and pointing device 161, commonly referred to as a mouse, trackball or touch pad. Other input devices (not shown) may include a microphone, joystick, game pad, satellite dish, scanner, or the like. These and other input devices are often connected to the processing unit 120 through a user input interface 160 that is coupled to the system bus, but may be connected by other interface and bus structures, such as a parallel port, game port or a universal serial bus (USB). A monitor 191 or other type of display device is also connected to the system bus 121 via an interface, such as a video interface 190. In addition to the monitor, computers may also include other peripheral output devices such as speakers 197 and printer 196, which may be connected through an output peripheral interface 190.

The computer 110 may operate in a networked environment using logical connections to one or more remote computers, such as a remote computer 180. The remote computer 180 may be a personal computer, a server, a router, a network PC, a peer device or other common network node, and typically includes many or all of the elements described above relative to the computer 110, although only a memory storage device 181 has been illustrated in Fig. 1. The logical connections depicted in Fig. 1 include a local area network (LAN) 171 and a wide area network (WAN) 173, but may also include other networks. Such networking environments are commonplace in offices, enterprise-wide computer networks, intranets and the Internet.

When used in a LAN networking environment, the computer 110 is connected to the LAN 171 through a network interface or adapter 170. When used in a WAN networking environment, the computer 110 typically includes a modem 172 or other means for establishing communications over the WAN 173, such as the Internet. The modem 172, which may be internal or external, may be connected to the system bus 121 via the user input interface 160, or other appropriate mechanism. In a networked environment, program modules depicted relative to the computer 110, or portions thereof, may be stored in the remote memory storage device. By way of example, and not limitation, Fig. 1 illustrates remote application programs 185 as residing on memory device 181. It will be appreciated that the network connections shown are exemplary and other means of establishing a communications link between the computers may be used.

Although the forgoing text sets forth a detailed description of numerous different embodiments of the invention, it should be understood that the scope of the invention is defined by the words of the claims set forth at the end of this patent. The detailed description is to be construed as exemplary only and does not describe every possibly embodiment of the invention because describing every possible embodiment would be impractical, if not impossible. Numerous alternative embodiments could be implemented, using either current technology or technology developed after the filing date of this patent, which would still fall within the scope of the claims defining the invention.

While the risk evaluation system and method, and other elements, have been described as preferably being implemented in software, they may be implemented in hardware, firmware, etc., and may be implemented by any other processor. Thus, the elements described herein may be implemented in a standard multi-purpose CPU or on specifically designed hardware or firmware such as an application-specific integrated circuit (ASIC) or other hard-wired device as desired, including, but not limited to, the computer 110 of Fig. 1. When implemented in software, the software routine may be stored in any computer readable memory such as on a magnetic disk, a laser disk, or other storage medium, in a RAM or ROM of a computer or processor, in any database, etc. Likewise, this software may be delivered to a user or a diagnostic system via any known or desired delivery method including, for example, on a computer readable disk or other transportable computer storage mechanism or over a communication channel such as a telephone line, the internet, wireless communication, etc. (which are viewed as being the same as or interchangeable with providing such software via a transportable storage medium).

Accordingly, the invention relates to computer-implemented applications using the polymorphic markers and haplotypes described herein, and genotype and/or disease-association data derived therefrom. Such applications can be useful for storing, manipulating or otherwise analyzing genotype data that is useful in the methods of the invention. One example pertains to storing genotype information derived from an individual on readable media, so as to be able to provide the genotype information to a third party (*e.g.*, the individual, a guardian of the individual, a health care provider or genetic analysis service provider), or for deriving information from the genotype data, *e.g.*, by comparing the genotype data to information about genetic risk factors contributing to increased susceptibility to the cancer, and reporting results based on such comparison.

In general terms, computer-readable media has capabilities of storing (i) identifier information for at least one polymorphic marker or a haplotype, as described herein; (ii) an indicator of the frequency of at least one allele of said at least one marker, or the frequency of a haplotype, in individuals with cancer; and an indicator of the frequency of at least one allele of said at least one marker, or the frequency of a haplotype, in a reference population. The reference population can be a disease-free population of individuals. Alternatively, the reference population is a random sample from the general population, and is thus representative of the population at large. The frequency indicator may be a calculated frequency, a count of alleles and/or haplotype copies, or normalized or otherwise manipulated values of the actual frequencies that are suitable for the particular medium.

The markers and haplotypes described herein to be associated with increased susceptibility (*e.g.*, increased risk) of cancer, are in certain embodiments useful for interpretation and/or analysis of genotype data. Thus in certain embodiments, an identification of an at-risk allele for cancer, as shown herein, or an allele at a polymorphic marker in LD with any one of the markers shown herein to be associated with cancer, is indicative of the individual from whom the genotype data originates is at increased risk of cancer. In one such embodiment, genotype data is generated for at least one polymorphic marker shown herein to be associated with cancer, or a marker in linkage disequilibrium therewith. The genotype data is subsequently made available to a third party, such as the individual from whom the data originates, his/her guardian or representative, a physician or health care worker, genetic counselor, or insurance agent, for example via a user interface accessible over the internet, together with an interpretation of the genotype data, *e.g.*, in the form of a risk measure (such as an absolute risk (AR), risk ratio (RR) or odds ratio (OR)) for the disease. In another embodiment, at-risk markers identified in a genotype dataset derived from an individual are assessed and results from the assessment of the risk conferred by the presence of such at-risk varians in the dataset are made available to the third party, for example via a secure web interface, or by other communication means. The results of such risk assessment can be reported in numeric form (*e.g.*, by risk values, such as absolute risk, relative risk, and/or an odds ratio, or by a percentage increase in risk compared with a reference), by graphical means, or by other means suitable to illustrate the risk to the individual from whom the genotype data is derived.

*Nucleic acids and polypeptides*

The nucleic acids and polypeptides described herein can be used in methods of the present invention. An "isolated" nucleic acid molecule, as used herein, is one that is separated from nucleic acids that normally flank the gene or nucleotide sequence (as in genomic sequences) and/or has been completely or partially purified from other transcribed sequences (e.g., as in an RNA library). For example, an isolated nucleic acid of the invention can be substantially isolated with respect to the complex cellular milieu in which it naturally occurs, or culture medium when produced by recombinant techniques, or chemical precursors or other chemicals when chemically synthesized. In some instances, the isolated material will form part of a composition (for example, a crude extract containing other substances), buffer system or reagent mix. In other circumstances, the material can be purified to essential homogeneity, for example as determined by polyacrylamide gel electrophoresis (PAGE) or column chromatography (e.g., HPLC). An isolated nucleic acid molecule of the invention can comprise at least about 50%, at least about 80% or at least about 90% (on a molar basis) of all macromolecular species present. With regard to genomic DNA, the term "isolated" also can refer to nucleic acid molecules that are separated from the chromosome with which the genomic DNA is naturally associated. For example, the isolated nucleic acid molecule can contain less than about 250 kb, 200 kb, 150 kb, 100 kb, 75 kb, 50 kb, 25 kb, 10 kb, 5 kb, 4 kb, 3 kb, 2 kb, 1 kb, 0.5 kb or 0.1 kb of the nucleotides that flank the nucleic acid molecule in the genomic DNA of the cell from which the nucleic acid molecule is derived.

The nucleic acid molecule can be fused to other coding or regulatory sequences and still be considered isolated. Thus, recombinant DNA contained in a vector is included in the definition of "isolated" as used herein. Also, isolated nucleic acid molecules include recombinant DNA molecules in heterologous host cells or heterologous organisms, as well as partially or substantially purified DNA molecules in solution. "Isolated" nucleic acid molecules also encompass *in vivo* and *in vitro* RNA transcripts of the DNA molecules of the present invention. An isolated nucleic acid molecule or nucleotide sequence can include a nucleic acid molecule or nucleotide sequence that is synthesized chemically or by recombinant means. Such isolated nucleotide sequences are useful, for example, in the manufacture of the encoded polypeptide, as probes for isolating homologous sequences (*e.g.*, from other mammalian species), for gene mapping (*e.g.*, by *in situ* hybridization with chromosomes), or for detecting expression of the gene in tissue (*e.g.*, human tissue), such as by Northern blot analysis or other hybridization techniques.

The disclosure also pertains to nucleic acid molecules that hybridize under high stringency hybridization conditions, such as for selective hybridization, to a nucleotide sequence described herein (*e.g.*, nucleic acid molecules that specifically hybridize to a nucleotide sequence containing a polymorphic site associated with a marker or haplotype described herein). Such nucleic acid molecules can be detected and/or isolated by allele- or sequence-specific hybridization (*e.g.*, under high stringency conditions). Stringency conditions and methods for nucleic acid hybridizations are well known to the skilled person (see, *e.g.,* Current Protocols in Molecular Biology, Ausubel, F. et al, John Wiley & Sons, (1998), and Kraus, M. and Aaronson, S., Methods Enzymol., 200:546-556 (1991).

The percent identity of two nucleotide or amino acid sequences can be determined by aligning the sequences for optimal comparison purposes (*e.g.*, gaps can be introduced in the sequence of a first sequence). The nucleotides or amino acids at corresponding positions are then compared, and the percent identity between the two sequences is a function of the number of identical positions shared by the sequences (i.e., % identity = # of identical positions/total # of positions x 100). In certain embodiments, the length of a sequence aligned for comparison purposes is at least 30%, at least 40%, at least 50%, at least 60%, at least 70%, at least 80%, at least 90%, or at least 95%, of the length of the reference sequence. The actual comparison of the two sequences can be accomplished by well-known methods, for example, using a mathematical algorithm. A non-limiting example of such a mathematical algorithm is described in Karlin, S. and Altschul, S., Proc. Natl. Acad. Sci. USA, 90:5873-5877 (1993). Such an algorithm is incorporated into the NBLAST and XBLAST programs (version 2.0), as described in Altschul, S. et al., Nucleic Acids Res., 25:3389-3402 (1997). When utilizing BLAST and Gapped BLAST programs, the default parameters of the respective programs (*e.g.*, NBLAST) can be used. See the website on the world wide web at ncbi.nlm.nih.gov. In one embodiment, parameters for sequence comparison can be set at score=100, wordlength=12, or can be varied (*e.g.*, W=5 or W=20). Another example of an algorithm is BLAT (Kent, W.J. Genome Res. 12:656-64 (2002)).

Other examples include the algorithm of Myers and Miller, CABIOS (1989), ADVANCE and ADAM as described in Torellis, A. and Robotti, C., Comput. Appl. Biosci. 10:3-5 (1994); and FASTA described in Pearson, W. and Lipman, D., Proc. Natl. Acad. Sci. USA, 85:2444-48 (1988).

In another embodiment, the percent identity between two amino acid sequences can be accomplished using the GAP program in the GCG software package (Accelrys, Cambridge, UK).

Also disclosed isolated nucleic acid molecules that contain a fragment or portion that hybridizes under highly stringent conditions to a nucleic acid that comprises, or consists of, the nucleotide sequence of SEQ ID NO.1, or a nucleotide sequence comprising, or consisting of, the complement of the nucleotide sequence of SEQ ID NO:1, wherein the nucleotide sequence comprises at least one polymorphic allele contained in the markers and haplotypes described herein. The nucleic acid fragments of the invention are at least about 15, at least about 18, 20, 23 or 25 nucleotides, and can be 30, 40, 50, 100, 200, 500, 1000, 10,000 or more nucleotides in length.

The nucleic acid fragments are used as probes or primers in assays such as those described herein. "Probes" or "primers" are oligonucleotides that hybridize in a base-specific manner to a complementary strand of a nucleic acid molecule. In addition to DNA and RNA, such probes and primers include polypeptide nucleic acids (PNA), as described in Nielsen, P. et al., Science 254:1497-1500 (1991). A probe or primer comprises a region of nucleotide sequence that hybridizes to at least about 15, typically about 20-25, and in certain embodiments about 40, 50 or 75, consecutive nucleotides of a nucleic acid molecule. In one embodiment, the probe or primer comprises at least one allele of at least one polymorphic marker or at least one haplotype described herein, or the complement thereof. In particular embodiments, a probe or primer can comprise 100 or fewer nucleotides; for example, in certain embodiments from 6 to 50 nucleotides, or, for example, from 12 to 30 nucleotides. In other embodiments, the probe or primer is at least 70% identical, at least 80% identical, at least 85% identical, at least 90% identical, or at least 95% identical, to the contiguous nucleotide sequence or to the complement of the contiguous nucleotide sequence. In another embodiment, the probe or primer is capable of selectively hybridizing to the contiguous nucleotide sequence or to the complement of the contiguous nucleotide sequence. Often, the probe or primer further comprises a label, *e.g.,* a radioisotope, a fluorescent label, an enzyme label, an enzyme co-factor label, a magnetic label, a spin label, an epitope label.

The nucleic acid molecules such as those described above, can be identified and isolated using standard molecular biology techniques well known to the skilled person. The amplified DNA can be labeled (*e.g.*, radiolabeled, fluorescently labeled) and used as a probe for screening a cDNA library derived from human cells. The cDNA can be derived from mRNA and contained in a suitable vector. Corresponding clones can be isolated, DNA obtained following *in vivo* excision, and the cloned insert can be sequenced in either or both orientations by art-recognized methods to identify the correct reading frame encoding a polypeptide of the appropriate molecular weight. Using these or similar methods, the polypeptide and the DNA encoding the polypeptide can be isolated, sequenced and further characterized.

*Antibodies*

Polyclonal antibodies and/or monoclonal antibodies that specifically bind one form of the gene product but not to the other form of the gene product are also provided. Antibodies are also provided which bind a portion of either the variant or the reference gene product that contains the polymorphic site or sites. The term "antibody" as used herein refers to immunoglobulin molecules and immunologically active portions of immunoglobulin molecules, *i.e.,* molecules that contain antigen-binding sites that specifically bind an antigen. A molecule that specifically binds to a polypeptide of the invention is a molecule that binds to that polypeptide or a fragment thereof, but does not substantially bind other molecules in a sample, *e.g.*, a biological sample, which naturally contains the polypeptide. Examples of immunologically active portions of immunoglobulin molecules include F(ab) and $F(ab')_2$ fragments which can be generated by treating the antibody with an enzyme such as pepsin. The invention provides polyclonal and monoclonal antibodies that bind to a polypeptide of the invention. The term "monoclonal antibody" or "monoclonal antibody composition", as used herein, refers to a population of antibody molecules that contain only one species of an antigen binding site capable of immunoreacting with a particular epitope of a polypeptide of the invention. A monoclonal antibody composition thus typically displays a single binding affinity for a particular polypeptide of the invention with which it immunoreacts.

Polyclonal antibodies can be prepared as described above by immunizing a suitable subject with a desired immunogen, *e.g.*, polypeptide of the invention or a fragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (*e.g.*, from the blood) and further purified by well-known techniques, such as protein A chromatography to obtain the IgG fraction. At an appropriate time after immunization, *e.g.*, when the antibody titers are highest, antibody-producing cells can be obtained from the subject and used to prepare monoclonal antibodies by standard techniques, such as the hybridoma technique originally described by Kohler and Milstein, Nature 256:495-497 (1975), the human B cell hybridoma technique (Kozbor et al., Immunol. Today 4: 72 (1983)), the EBV-hybridoma technique (Cole et al., Monoclonal Antibodies and Cancer Therapy, Alan R. Liss,1985, Inc., pp. 77-96) or trioma techniques. The technology for producing hybridomas is well known (see generally Current Protocols in Immunology (1994) Coligan et al., (eds.) John Wiley & Sons, Inc., New York, NY). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide of the invention.

Any of the many well known protocols used for fusing lymphocytes and immortalized cell lines can be applied for the purpose of generating a monoclonal antibody to a polypeptide of the invention (see, *e.g., Current Protocols in Immunology, supra;* Galfre et al., Nature 266:55052 (1977); R.H. Kenneth, in Monoclonal Antibodies: A New Dimension In Biological Analyses, Plenum Publishing Corp., New York, New York (1980); and Lerner, Yale J. Biol. Med. 54:387-402 (1981)). Moreover, the ordinarily skilled worker will appreciate that there are many variations of such methods that also would be useful.

Alternative to preparing monoclonal antibody-secreting hybridomas, a monoclonal antibody to a polypeptide of the invention can be identified and isolated by screening a recombinant combinatorial immunoglobulin library (*e.g.*, an antibody phage display library) with the polypeptide to thereby isolate immunoglobulin library members that bind the polypeptide. Kits for generating and screening phage display libraries are commercially available (*e.g.*, the Pharmacia *Recombinant Phage Antibody System,* Catalog No. 27-9400-01; and the Stratagene *Surf*ZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., Bio/Technology 9: 1370-1372 (1991); Hay et al., Hum. Antibod. Hybridomas 3:81-85 (1992); Huse et al., Science 246: 1275-1281 (1989); and Griffiths et al., EMBO J. 12:725-734 (1993).

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art.

In general, antibodies (*e.g.,* a monoclonal antibody) can be used to isolate a polypeptide of the invention (*e.g.*, a TERT or CLPTM1L polypeptide) by standard techniques, such as affinity chromatography or immunoprecipitation. A polypeptide-specific antibody can facilitate the purification of natural polypeptide from cells and of recombinantly produced polypeptide expressed in host cells. Moreover, an antibody specific for a polypeptide of the invention can be used to detect the polypeptide (*e.g.*, in a cellular lysate, cell supernatant, or tissue sample) in order to evaluate the abundance and pattern of expression of the polypeptide. Antibodies can be used diagnostically to monitor protein levels in tissue as part of a clinical testing procedure, *e.g.*, to, for example, determine the efficacy of a given treatment regimen. The antibody can be coupled to a detectable substance to facilitate its detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}I$, $^{131}I$, $^{35}S$ or $^{3}H$.

Antibodies may also be useful in pharmacogenomic analysis. In such embodiments, antibodies against variant proteins encoded by nucleic acids according to the invention, such as variant proteins that are encoded by nucleic acids that contain at least one polymorpic marker of the invention, can be used to identify individuals that require modified treatment modalities.

Antibodies can furthermore be useful for assessing expression of variant proteins (*e.g.*, TERT and/or CLPTM1L) in disease states, such as in active stages of a disease, or in an individual with a predisposition to a disease related to the function of the protein, in particular cancer. Antibodies specific for a variant protein of the present invention that is encoded by a nucleic acid that comprises at least one polymorphic marker or haplotype as described herein can be used to screen for the presence of the variant protein, for example to screen for a predisposition to cancer as indicated by the presence of the variant protein.

Antibodies can be used in other methods. Thus, antibodies are useful as diagnostic tools for evaluating proteins, such as variant proteins of the invention, in conjunction with analysis by electrophoretic mobility, isoelectric point, tryptic or other protease digest, or for use in other physical assays known to those skilled in the art. Antibodies may also be used in tissue typing. In one such embodiment, a specific variant protein has been correlated with expression in a specific tissue type, and antibodies specific for the variant protein can then be used to identify the specific tissue type.

Subcellular localization of proteins, including variant proteins, can also be determined using antibodies, and can be applied to assess aberrant subcellular localization of the protein in cells in various tissues. Such use can be applied in genetic testing, but also in monitoring a particular treatment modality. In the case where treatment is aimed at correcting the expression level or presence of the variant protein or aberrant tissue distribution or developmental expression of the variant protein, antibodies specific for the variant protein or fragments thereof can be used to monitor therapeutic efficacy.

Antibodies are further useful for inhibiting variant protein function, for example by blocking the binding of a variant protein to a binding molecule or partner. Such uses can also be applied in a therapeutic context in which treatment involves inhibiting a variant protein's function. An antibody can be for example be used to block or competitively inhibit binding, thereby modulating (i.e., agonizing or antagonizing) the activity of the protein. Antibodies can be prepared against specific protein fragments containing sites required for specific function or against an intact protein that is associated with a cell or cell membrane. For administration *in vivo,* an antibody may be linked with an additional therapeutic payload, such as radionuclide, an enzyme, an immunogenic epitope, or a cytotoxic agent, including bacterial toxins (diphtheria or plant toxins, such as ricin). The *in vivo* half-life of an antibody or a fragment thereof may be increased by pegylation through conjugation to polyethylene glycol.

The present disclosure further relates to kits for using antibodies in the methods described herein. This includes, but is not limited to, kits for detecting the presence of a variant protein in a test sample. One preferred embodiment comprises antibodies such as a labelled or labelable antibody and a compound or agent for detecting variant proteins in a biological sample, means for determining the amount or the presence and/or absence of variant protein in the sample, and means for comparing the amount of variant protein in the sample with a standard, as well as instructions for use of the kit.

The present invention will now be exemplified by the following non-limiting examples.

## EXAMPLE 1

### SEQUENCE VARIANTS ON CHROMOSOME 5p13.3 THAT ASSOCIATE WITH CANCER AT MULTIPLE SITES

Recently, genome-wide association studies of several cancers have identified common genetic variants that associate with increased cancer risk (Gudmundsson, J., et al. Nat Genet 39:631-637 (2007); Stacey, S.N., et al., Nat Genet. 39:865-69 (2007); Yeager, M. et al. Nat Genet 39:645-649 (2007); Gudmundsson, J., et al. Nat Genet 39:977-983 (2007); Haiman, C.A., et al. Nat Genet 39:638-644 (2007); Eason, D.F., et al. Nature 447:1087-1093 (2007); Tomlinson, I., et al. Nat Genet 39:984-988 (2007); Gudbjartsson, D.F., et al. Nat Genet 40:886-891 (2008); Stacey, S.N., et al. Nat Genet 40:703-706 (2008); Thorgeirsson, T.E., et al. Nature 452:638-642 (2008); Gudmundsson, J., et al. Nat Genet 40:281-283 (2008); Eeles, R.A., et al. Nat Genet 40:316-321 (2008); Hung, R.J., et al. Nature 452:633-637 (2008); Amos, C.I., et al. Nat Genet 40:616-622 (2008); Thomas, G., et al. Nat Genet 40:310-315 (2008)). Notably, in most cases the reported variants seem to be specific to the particular cancer type under study. This tissue specificity even holds true in the region on chromosome 8q24, which has been found to associate with several different types of cancer. Independent variants in the 8q24 region have been found that associate with risk of prostate, breast and bladder cancer. Only one of the prostate cancer variants has been shown also to associate with risk of another cancer, i.e. colorectal cancer (Tomlinson, I., et al. Nat Genet 39:984-988 (2007)). We now show that variants on chromosome 5p13.3 associate with cancer at multiple sites.

### Cohorts

The cohorts are described in the following:

*Prostate cancer:* Described in Gudmundsson J, et al. Nature Genetics 39, 631-637 (2007).

*Lung cancer:* Described in Thorgeirsson TE, et al. Nature 452, 638-642

*Bladder cancer:* Described in Kiemeney LA, et al. Nature Genetics (in press)

*BCC: Described in* Stacey SN, et al. Nature Genetics (in press)

*Cervical cancer and Thyroid cancer:* The cervical cancer program at deCODE genetics is a part of a larger program termed the Icelandic Cancer Project. The major hypothesis of the Icelandic Cancer Project is that cancer is a group of related disorders that have common genetic causes and can be viewed as a single phenotype. The projects have been approved by the Data Protection Authority of Iceland and the National Bioethics Committee. Cancer cases were identified based on records from the nation-wide Icelandic Cancer Registry (ICR; www.krabbameinsskra.is) which include information on the year and age at diagnosis, year of death, SNOMED code and ICD-10 classification. All participants are recruited by trained nurses through special recruitment clinics where they donate a blood sample and answer a lifestyle questionnaire. Clinical information on cancer patients were extracted from medical records at treatment centers. Written informed consent was obtained from all participants. Personal identifiers associated with medical information and blood samples were encrypted with a third-party encryption system in which the Data Protection Authority maintains the code. A total of 803 women were diagnosed with cervical cancer between January 1, 1955 and December 31, 2007. Samples from approximately 300 women were available for genotyping and genotypes of additional 150 women could be imputed.

### Genotyping

All cases and controls were assayed using genotyping systems and specialized software from Illumina (Human Hap300 and HumanCNV370-duo Bead Arrays, Illumina). Furthermore, all Dutch bladder cancer cases and controls have been genotyped with the HumanCNV370-duo Bead Arrays. These chips provide about 75% genomic coverage in the Utah CEPH (CEU) HapMap samples for common SNPs at r2>0.8 (Barrett, J.C. and Cardon, L.R., Nat Genet 38:659-662 (2006)). SNP data were discarded if the minor allele frequency in the combined case and control was <0.001 or had less than 95% yield or showed a very significant distortion from Hardy-Weinberg equilibrium in the controls ($P<1x10^{-10}$). Any chips with a call rate below 98% of the SNPs were excluded from the genome-wide association analysis.

All single SNP genotyping was carried out applying the Centaurus (Nanogen) platform (Kutyavin, I.V., et al. Nucleic Acids Res 34:e128 (2006)). The quality of each Centaurus SNP assay was evaluated by genotyping each assay in the CEU HapMap samples and comparing the results with the HapMap publicly released data. Assays with > 1.5% mismatch rate were not used and a linkage disequilibrium (LD) test was used for markers known to be in LD. Approximately 10% of the Icelandic case samples that were genotyped on the Illumina platform were also genotyped using the Centaurus assays and the observed mismatch rate was lower than 0.5%. All genotyping was carried out at the deCODE Genetics facility.

**Statistical analysis**

*Association analysis.* A likelihood procedure described in a previous publication (Yeager, M., et al. Nat Genet 39:645-649 (2007)) and implemented in the NEMO software was used for the association analyses. An attempt was made to genotype all individuals and all SNPs reported, and for each of the SNPs, the yield was higher than 95% in every study group. The SNPs rs4645960 and rs16901979 are not a part of the Human Hap300/HumanCNV370-duo chips. For these SNPs, a subset of the large Icelandic control set as well as all Icelandic cases and all individuals from the replication study groups were genotyped by single track assays. We tested the association of an allele to UBC using a standard likelihood ratio statistic that, if the subjects were unrelated, would have asymptotically a $\chi 2$ distribution with one degree of freedom under the null hypothesis. Allelic frequencies rather than carrier frequencies are presented for the markers in the main text. Allele-specific ORs and associated P values were calculated assuming a multiplicative model for the two chromosomes of an individual (Gudmundsson, J., et al. Nat Genet 39:977-983 (2007)). Results from multiple case-control groups were combined using a Mantel-Haenszel model (Haiman, C.A., et al. Nat Genet 39:638-644 (2007)) in which the groups were allowed to have different population frequencies for alleles, haplotypes and genotypes but were assumed to have common relative risks.

Correction of the GWA studies by genomic control. To adjust for possible population stratification and the relatedness amongst individuals, we divided the $\chi 2$ test statistics from the individual scans using the method of genomic control (13), i.e. the 302,140 $\chi 2$ test statistics were divided by their means, which were 1.04 and 1.075 for Iceland and the Netherlands, respectively.

**Results**

In a genome-wide association study of Basal Cell Carcinoma, we identified two signals on Chr1p and one on Chrlq that reached genome-wide significance in analysis of Icelandic and European sample sets (Stacey 2008, submitted). Subsequently, we followed up the initial GWA scan, increasing the effective sample size by chip-genotyping additional Icelandic BCC cases (total 1011 cases) and imputing genotypes from un-typed BCC cases, using the genealogy database of all Icelanders as previously described (Gudbjartsson, D.F., et al. Nat Genet 40:609-15 (2008)). The results were adjusted for relatedness between individuals and for potential population stratification using the method of genomic control. The third strongest signal from the GWA was located on chromosome 5p15.3 and represented by three SNPs, rs31489, rs401681 and rs4975616 (minimum P=$1.9\times10^{-7}$). Those three markers are strongly correlated for all pairwise LD tests, D' > 0.9 and r2 > 0.75 (from 0.75 to 0.87), and belong to an area of high linkage disequilibrium (LD) (Figure 2). This area overlaps with two genes, *CLPTM1L* (encoding cisplatin resistance related protein CRR9p) and *TERT* (encoding human telomerase reverse transcriptase).

To further confirm the association with 5p15.3, we genotyped rs401681 by single track assays in additional 731 BCC cases from Iceland and 525 BCC cases and 525 controls from Eastern Europe. The association of allele C of rs401681 to BCC in the combined analysis of the Icelandic and Eastern Europe reached genome-wide significance (P=$3.13\times10^{-11}$, OR=1.25) (Table 1A). We did not observe heterogeneity of the ORs between the Icelandic and East European groups. Results from all groups combined demonstrated that the association of rs401681 allele C to BCC did not deviate from the multiplicative model (P>0.05).

The two genes in the LD region, *CLPTM1L* and *TERT* have both been studied in the context of cancer. *CLPTM1L* was identified in an ovarian cancer model as a gene that affected cisplatin-induced apoptosis but has not been extensively studied (Yamamoto, K. et al. Biochem Biophys Res Comm 280:1148-54 (2001)). The *TERT* gene plays a leading role in maintenance of functional telomeres and has been firmly established as a key gene in cancer development. In particular, the well-documented association between telomere function and environmental insults such as radiation suggests a potential link between *TERT* and predisposition to BCC (reviewed in Ayouaz, A., et al. Biochimie 90:60-72 (2008)). Given the relevance of this genomic region to general cancer biology, we assessed the association of rs401681 allele C to 16 cancer types in individuals of European ancestry, making use of samples and data collected through the Icelandic Cancer Project (Rafnar, T., et al. Nat Rev Cancer 4:488-92 (2004)) and by a number of collaborators in Europe. This Icelandic sample collection includes cases with various types of cancer, ascertained through the nation-wide Icelandic Cancer Registry. We have previously genotyped over 38,000 Icelandic individuals, using the HumanHap300/HumanCNV370-duo BeadChips. In addition to the chip data, we used single track genotyping on available cases that had not been genotyped on the chip and imputations of un-genotyped individuals using a database containing the genealogy of all Icelanders. We also genotyped all non-Icelandic case control sets by single-track genotyping and combined all genotype information with result summaries of public GWA datasets (CGEMS, ICR, IARC). In total, we assessed the association of rs401681allele C to 16 individual cancer sites using approximately 30,000 cases and 40,000 controls.

In total, 6 cancer sites showed nominal positive association ($P < 0.05$) with rs401681 (Table 1A). The most significant site after BCC was lung cancer, reaching genome wide significance (OR=1.15, P=8.55x10$^{-8}$) in the combined analysis of 4 populations from Iceland, the Netherlands and Spain in addition to the dataset from the IARC, made publically available in 2008 (20). The ORs were very similar in the 4 groups, ranging from 1.13 to 1.19. We divided the lung cancer cases in the Icelandic Dutch and Spanish populations into 4 groups based on histology (small cell, squamous cell, adenocarcinoma, others) and assessed the frequency of rs401681allele C in these different types. In all three populations, we observed a higher frequency of the risk variant among cases with squamous cell carcinoma compared to the other histologies (combined OR 1.29, P=0.0019). Comparing squamous cell carcinomas to the control group, the effect was even stronger with an OR of 1.41. We observed an association between rs401681 allele C and bladder cancer in 9 European case control groups (combined OR=1.12, P=4.05x10$^{-5}$). While some groups were small, all groups showed an effect in the same direction, ranging from 1.02 to 1.23. For prostate cancer, we were able to analyse data from 5 groups (over 9,000 cases), including the public CGEMS dataset, and demonstrated a significant effect which was consistent between populations (combined OR=1.07, P=4.45x10$^{-4}$). Finally, we detected a significant association between rs401681allele C and cervical cancer in Iceland (OR=1.31, P=7.48x10$^{-4}$). In this group of cases, a trend towards a stronger association was noted in cases with the squamous cell carcinoma than cases with adenocarcinoma.

Imputation of un-genotyped SNPs in the area was performed using the HapMap CEU database and the genotyped SNPs on the chip. This analysis showed that multiple markers in the area are also associated with BCC, including rs2736100 (OR 1.13, P=1.7x10-6) and rs2736098 (OR 1.27; P=3.9x10$^{-8}$) , the latter being a synonymous coding SNP (A305A) in the second exon of the *TERT gene* (Figure 2). The SNP rs2736098 is correlated with rs31489, rs401681 and rs4975616 (for all pairwise LD tests D' is over 0.9 and the r2 >0.3 (from 0.33 to 0.39)) with rs401681 being the best correlated marker.

Follow-up analysis revealed that markers rs2736100 and rs2736098 are both associated strongly with cancer at multiple sites (Table 3 and Table 4). It is noteworthy that the risk for rs2736098 is even higher than for rs401681 (Table 4). The rs2736098 marker is correlated to rs401681 and rs2736100 by $r^2$-values of 0.39 and 0.12, respectively (HapMap CEU sample, Release 22). Markers rs401681 and rs2736100 are however in poor LD (r2 < 0.05). While the signal for rs2736098 is strongest of these three markers, it appears not to fully explain the association to the other two markers. Thus, there may be another, not yet identified, identified genetic variant in LD with these three markers that has an even stronger association to cancer. Alternatively, the association signal in the region is more complex, with multiple unrelated association signals.

It is of interest that 4 of the 5 cancers associated with the risk variants are cancer types that have strong environmental contribution to risk, i.e. smoking and occupational exposures for lung and bladder cancer, UV irradiation for BCC and infection with human papillomavirus for cervical cancer. The majority of cancers in these organs arise in the epithelial layer that is in closest contact with the environment. Although no strong environmental risk factors are currently known for prostate cancer, several external factors such as diet, physical activity and inflammation may have an effect on disease risk. Although telomere length is partly inherited (Slagboom, PE, et al. Am J Hum Genet 55:876-82 (1994)) various environmental factors such as smoking and radiation also affect telomere length (Valdes, IP, et al. Nat Genet 40:623-30 (2008)).

**Table 1A.** Association of rs401681 allele C on Chr 5p15.3 to basal cell carcinoma and cancers of the lung, bladder, prostate, cervix and endometrium in Iceland.

| Study population | Number | | Frequency | | OR | 95% CI | *P* value |
|---|---|---|---|---|---|---|---|
| | Cases | Controls | Cases | Controls | | | |
| Basal cell carcinoma | 2,032[a] | 28,787 | 0.603 | 0.545 | 1.27 | 1.18-1.36 | 7.96x10-11 |
| Lung cancer | 1,381[a] | 28,787 | 0.576 | 0.545 | 1.13 | 1.04-1.23 | 4.21x10-3 |
| Bladder cancer | 823[a] | 28,787 | 0.585 | 0.545 | 1.17 | 1.06-1.30 | 2.87x10-3 |
| Prostate cancer | 2,294[a] | 28,787 | 0.569 | 0.545 | 1.10 | 1.03-1.18 | 5.69x10-3 |
| Cervical cancer | 369[a] | 28,787 | 0.611 | 0.545 | 1.31 | 1.12-1.53 | 7.48x10-4 |

(continued)

| Study population | Number | | Frequency | | OR | 95% CI | *P* value |
|---|---|---|---|---|---|---|---|
| | Cases | Controls | Cases | Controls | | | |
| Endometrial cancer | 470[a] | 28,787 | 0.592 | 0.545 | 1.21 | 1.06-1.38 | 5.50x10-3 |
| All P values shown are two-sided. Shown are the corresponding numbers of cases and controls (N), allelic frequencies of variants in affected and control individuals, the allelic odds-ratio (OR) with P values based on the multiplicative model. [a] The number reflects the effective sample size obtained by combining results from genotyped individuals and imputation of un-genotyped individuals. See supplementary material. | | | | | | | |

**Table 1B.** Association to Basal Cell Carcinoma for markers in 200Kb interval on chromosome 5p13.3. Genotypes for markers in the HapMap data set were imputed, as described under Methods.

| Marker 1 | Marker 2 | *P* value | Position build 36 |
|---|---|---|---|
| rs401681 | rs4246740 | 0.75 | 1239086 |
| rs401681 | rs6554634 | 0.56 | 1239121 |
| rs401681 | rs4640842 | 0.56 | 1240074 |
| rs401681 | rs4460173 | 0.61 | 1240180 |
| rs401681 | rs4072529 | 0.56 | 1240281 |
| rs401681 | rs4975599 | 0.37 | 1241837 |
| rs401681 | rs7736074 | 0.21 | 1242456 |
| rs401681 | rs7719875 | 0.87 | 1243088 |
| rs401681 | rs6884486 | 0.4 | 1243440 |
| rs401681 | rs10060236 | 0.4 | 1244061 |
| rs401681 | rs10061926 | 0.25 | 1244336 |
| rs401681 | rs6872717 | 0.6 | 1248209 |
| rs401681 | rs4975607 | 0.79 | 1255817 |
| rs401681 | rs12520454 | 0.2 | 1257868 |
| rs401681 | rs10072823 | 0.25 | 1258636 |
| rs401681 | rs11133684 | 0.54 | 1268474 |
| rs401681 | rs4975629 | 0.89 | 1269775 |
| rs401681 | rs6554665 | 0.12 | 1270223 |
| rs401681 | rs6554666 | 0.12 | 1270471 |
| rs401681 | rs4975628 | 0.64 | 1273032 |
| rs401681 | rs9418 | 0.35 | 1278121 |
| rs401681 | rs7704882 | 0.96 | 1278434 |
| rs401681 | rs7728646 | 0.96 | 1278564 |
| rs401681 | rs7728667 | 0.96 | 1278626 |
| rs401681 | rs4975623 | 0.61 | 1285491 |
| rs401681 | rs6554677 | 0.84 | 1289291 |
| rs401681 | rs6554679 | 0.017 | 1289690 |

(continued)

| Marker 1 | Marker 2 | *P* value | Position build 36 |
|---|---|---|---|
| rs401681 | rs7447815 | 0.24 | 1293757 |
| rs401681 | rs6554684 | 0.87 | 1293848 |
| rs401681 | rs12513872 | 0.021 | 1301354 |
| rs401681 | rs6554691 | 0.13 | 1301873 |
| rs401681 | rs10078761 | 0.11 | 1302594 |
| rs401681 | rs2853691 | 0.43 | 1305950 |
| rs401681 | rs2736118 | 0.0068 | 1313195 |
| rs401681 | rs2075786 | 0.0067 | 1319310 |
| rs401681 | rs4246742 | 0.29 | 1320356 |
| rs401681 | rs10069690 | 0.0089 | 1332790 |
| rs401681 | rs2242652 | 0.18 | 1333028 |
| rs401681 | rs2736098 | 3.90E-08 | 1347086 |
| rs401681 | rs2735845 | 0.00029 | 1353584 |
| rs401681 | rs4975615 | 1.00E-05 | 1368343 |
| rs401681 | rs6554759 | 0.28 | 1370102 |
| rs401681 | rs1801075 | 0.28 | 1370949 |
| rs401681 | rs7727912 | 0.09 | 1371960 |
| rs401681 | rs451360 | 0.26 | 1372680 |
| rs401681 | rs421629 | 2.50E-06 | 1373136 |
| rs401681 | rs380286 | 2.50E-06 | 1373247 |
| rs401681 | rs10073340 | 3.00E-05 | 1374873 |
| rs401681 | rs466502 | 3.40E-06 | 1378767 |
| rs401681 | rs465498 | 2.50E-06 | 1378803 |
| rs401681 | rs452932 | 2.50E-06 | 1383253 |
| rs401681 | rs452384 | 2.50E-06 | 1383840 |
| rs401681 | rs467095 | 2.50E-06 | 1389221 |
| rs401681 | rs31484 | 2.50E-06 | 1390906 |
| rs401681 | rs11948616 | 0.46 | 1396682 |
| rs401681 | rs31490 | 7.90E-06 | 1397458 |
| rs401681 | rs27070 | 3.60E-06 | 1399303 |
| rs401681 | rs37008 | 6.50E-06 | 1404538 |
| rs401681 | rs37005 | 6.30E-05 | 1409450 |
| rs401681 | rs27064 | 1.50E-05 | 1412938 |
| rs401681 | rs27063 | 0.63 | 1413125 |
| rs401681 | rs2292024 | 0.72 | 1417242 |
| rs401681 | rs409932 | 0.22 | 1420736 |
| rs401681 | rs506156 | 0.31 | 1421678 |
| rs401681 | rs461193 | 0.45 | 1421997 |

(continued)

| Marker 1 | Marker 2 | *P* value | Position build 36 |
|---|---|---|---|
| rs401681 | rs10075239 | 0.46 | 1423300 |
| rs401681 | rs6873098 | 0.022 | 1424093 |
| rs401681 | rs881618 | 0.022 | 1424719 |

**Table 1C**. Association to Basal Cell Carcinoma for markers in 200Kb interval on chromosome 5p13.3. Results are based on a combination of genotyped cases on the Illumina Hap300 chip and un-genotyped, imputed cases, as described under Methods.

| Marker 1 | Marker 2 | *P* value | Position build 36 |
|---|---|---|---|
| rs401681 | rs4975536 | 0.36 | 1229601 |
| rs401681 | rs4975603 | 0.63 | 1234556 |
| rs401681 | rs4246736 | 0.38 | 1239853 |
| rs401681 | rs4975596 | 0.96 | 1242347 |
| rs401681 | rs11747247 | 0.27 | 1253573 |
| rs401681 | rs13159461 | 0.23 | 1256437 |
| rs401681 | rs6554660 | 0.61 | 1260527 |
| rs401681 | rs7727745 | 0.75 | 1265357 |
| rs401681 | rs6554667 | 0.38 | 1270494 |
| rs401681 | rs4975542 | 0.19 | 1275480 |
| rs401681 | rs10060827 | 0.64 | 1276062 |
| rs401681 | rs4975625 | 0.84 | 1281215 |
| rs401681 | rs7445640 | 0.73 | 1289212 |
| rs401681 | rs4075202 | 0.17 | 1296475 |
| rs401681 | rs4073918 | 0.012 | 1297425 |
| rs401681 | rs2736122 | 0.072 | 1310621 |
| rs401681 | rs4975605 | 0.16 | 1328528 |
| rs401681 | rs2736100 | 0.0041 | 1339516 |
| rs401681 | rs2853676 | 0.93 | 1341547 |
| rs401681 | rs2853668 | 0.003 | 1353025 |
| rs401681 | rs4635969 | 0.055 | 1361552 |
| rs401681 | rs4975616 | 2.20E-06 | 1368660 |
| rs401681 | rs402710 | 0.00018 | 1373722 |
| rs401681 | rs401681 | 2.30E-07 | 1375087 |
| rs401681 | rs31489 | 1.90E-07 | 1395714 |
| rs401681 | rs27061 | 0.93 | 1415793 |
| rs401681 | rs2963265 | 0.11 | 1423832 |

**Table 2.** Association of rs401681 allele C on Chr 5p15.3 to basal cell carcinoma and cancers of the lung, bladder, prostate, cervix, thyroid and endometrium in Iceland and other populations.

| **Study population** | **Number** | | **Frequency** | | **OR** | **95% CI** | ***P* value** |
|---|---|---|---|---|---|---|---|
| | Cases | Controls | Cases | Controls | | | |
| | | | | | | | |
| Basal cell carcinoma | | | | | | | |
| Iceland | 2,032[a] | 28,787 | 0.603 | 0.545 | 1.27 | 1.18-1.36 | 7.96x10-11 |
| Eastern Europe | 525 | 525 | 0.616 | 0.573 | 1.16 | 0.97-1.39 | 0.098 |
| All combined[b] | 2,557 | 29,312 | 0.610 | 0.560 | 1.25 | 1.17-1.34 | 3.13x10-11 |
| | | | | | | | |
| Lung cancer | | | | | | | |
| Iceland | 1,381[a] | 28,787 | 0.576 | 0.545 | 1.13 | 1.04-1.23 | 4.21x10-3 |
| The Netherlands | 529 | 1,832 | 0.610 | 0.570 | 1.18 | 1.02-1.35 | 0.021 |
| Spain | 367 | 1,427 | 0.582 | 0.538 | 1.19 | 1.01-1.41 | 0.034 |
| IARC | 1,920 | 2,517 | 0.617 | 0.586 | 1.16 | 1.06-1.27 | 8x10-4 |
| All combined[b] | 4,197 | 34,563 | 0.596 | 0.560 | 1.15 | 1.10-1.22 | 8.55x10-8 |
| | | | | | | | |
| Bladder cancer | | | | | | | |
| Iceland | 823[a] | 28.787 | 0.585 | 0.545 | 1.17 | 1.06-1.30 | 2.87x10-3 |
| The Netherlands | 1,277 | 1,832 | 0.584 | 0.570 | 1.06 | 0.96-1.17 | 0.27 |
| UK | 707 | 506 | 0.564 | 0.514 | 1.23 | 1.04-1.44 | 0,014 |
| Italy-Torino | 329 | 379 | 0.550 | 0.545 | 1.02 | 0.84-1.24 | 0.84 |
| Italy-Brescia | 122 | 156 | 0.574 | 0.564 | 1.04 | 0.74-1.46 | 0.82 |
| Belgium | 199 | 378 | 0.603 | 0.554 | 1.22 | 0.95-1.56 | 0.11 |
| Eastern Europe | 214 | 515 | 0.619 | 0.575 | 1.20 | 0.96-1.51 | 0.12 |
| Sweden | 346 | 905 | 0.545 | 0.521 | 1.10 | 0.92-1.31 | 0.30 |
| Spain | 173 | 1,427 | 0.546 | 0.538 | 1.03 | 0.83-1.29 | 0.78 |
| All combined[b] | 4,190 | 34,885 | 0.578 | 0.535 | 1.12 | 1.06-1.18 | 4.05x10-5 |
| | | | | | | | |
| Prostate cancer | | | | | | | |
| Iceland | 2,294[a] | 28,787 | 0.569 | 0.545 | 1.10 | 1.03-1.18 | 5.69x10-3 |

(continued)

| Study population | Number | | Frequency | | OR | 95% CI | *P* value |
|---|---|---|---|---|---|---|---|
| | Cases | Controls | Cases | Controls | | | |
| The Netherlands | 994 | 1,832 | 0.576 | 0.570 | 1.02 | 0.92-1.14 | 0.67 |
| Chicago, US | 635 | 693 | 0.581 | 0.568 | 1.06 | 0.90-1.23 | 0.49 |
| Spain | 459 | 1,427 | 0.559 | 0.538 | 1.09 | 0.94-1.26 | 0.27 |
| CGEMS | 5,109 | 5,059 | 0.558 | 0.543 | 1.06 | 1.00-1.11 | 0.036 |
| All combined[b] | 9,491 | 37,798 | 0.569 | 0.553 | 1.07 | 1.03-1.11 | 4.45x10-4 |
| | | | | | | | |
| Cervical cancer | | | | | | | |
| Iceland | 369[a] | 28,787 | 0.611 | 0.545 | 1.31 | 1.12-1.53 | 7.48x10-4 |
| | | | | | | | |
| Thyroid cancer | | | | | | | |
| Iceland | 528[a] | 28,787 | 0.538 | 0.545 | 0.97 | 0.85-1.10 | 0.644 |
| | | | | | | | |
| Endometrial cancer | | | | | | | |
| Iceland | 470[a] | 28,787 | 0.592 | 0.545 | 1.21 | 1.06-1.38 | 5.50x10-3 |

All P values shown are two-sided. Shown are the corresponding numbers of cases and controls (N), allelic frequencies of variants in affected and control individuals, the allelic odds-ratio (OR) with P values based on the multiplicative model.
[a] The number reflects the effective sample size obtained by combining results from genotyped individuals and imputation of un-genotyped individuals.
[b] For the combined study populations, the reported control frequency was the average, unweighted control frequency of the individual populations, while the OR and the P value were estimated using the Mantel-Haenszel model.

**Table 5.** Markers in linkage disequilibrium (LD) with rs401681. The markers were selected from the Caucasian HapMap dataset. Shown are marker names, values for the LD measures D' and r2 to rs401681, the corresponding p-value, the location of the marker in NCBI Build 36, and reference to the sequence ID for flanking sequence of the marker.

| Marker | D' | r2 | *P* value | Pos in Bld 36 | Pos. Seq ID NO:1 |
|---|---|---|---|---|---|
| rs2736098 | 0.94371 | 0.394285 | 2.80E-12 | 1347086 | 51091 |
| rs2735845 | 0.812051 | 0.153473 | 0.000023 | 1353584 | 57589 |
| rs4635969 | 1 | 0.361702 | 2.79E-13 | 1361552 | 65557 |
| rs4975615 | 0.96143 | 0.772861 | 4.56E-24 | 1368343 | 72348 |
| rs4975616 | 0.964609 | 0.86912 | 1.61E-28 | 1368660 | 72665 |
| rs6554759 | 1 | 0.218769 | 1.31E-08 | 1370102 | 74107 |
| rs1801075 | 1 | 0.218769 | 1.53E-08 | 1370949 | 74954 |

(continued)

| Marker | D' | r2 | *P* value | Pos in Bld 36 | Pos. Seq ID NO:1 |
|---|---|---|---|---|---|
| rs451360 | 1 | 0.373833 | 1.48E-13 | 1372680 | 76685 |
| rs421629 | 1 | 1 | 2.78E-37 | 1373136 | 77141 |
| rs380286 | 1 | 1 | 2.78E-37 | 1373247 | 77252 |
| rs402710 | 1 | 0.667674 | 6.62E-23 | 1373722 | 77727 |
| rs10073340 | 1 | 0.201183 | 4.21E-08 | 1374873 | 78878 |
| rs401681 | 1 | 1 | - | 1375087 | 79092 |
| rs466502 | 1 | 0.966555 | 2.61E-35 | 1378767 | 82772 |
| rs465498 | 1 | 1 | 1.36E-37 | 1378803 | 82808 |
| rs452932 | 1 | 1 | 5.57E-37 | 1383253 | 87258 |
| rs452384 | 1 | 1 | 1.36E-37 | 1383840 | 87845 |
| rs467095 | 1 | 1 | 3.15E-37 | 1389221 | 93226 |
| rs31484 | 1 | 1 | 1.36E-37 | 1390906 | 94911 |
| rs31489 | 1 | 0.871795 | 7.70E-31 | 1395714 | 99719 |
| rs31490 | 1 | 0.966555 | 4.56E-35 | 1397458 | 101463 |
| rs27070 | 0.896536 | 0.777014 | 8.09E-25 | 1399303 | 103308 |
| rs37008 | 0.89493 | 0.748554 | 9.41E-24 | 1404538 | 108543 |
| rs37005 | 0.929849 | 0.804816 | 9.41E-26 | 1409450 | 113455 |
| rs27064 | 1 | 0.126214 | 3.66E-06 | 1412938 | 116943 |
| rs409932 | 0.550831 | 0.107998 | 0.00078 | 1420736 | 124741 |

**Table 8.** Known SNP markers within the TERT and 10 kb flanking the gene. Shown are the *position of the marker on Chr 5 in NCBI Build 36, marker names, and reference to the sequence ID* for flanking sequence of the marker.

| C05 Bld 36 Location | Marker | Pos SEQ ID NO:1 | C05 Bld 36 Location | Marker | Pos SEQ ID NO:1 |
|---|---|---|---|---|---|
| 1296296 | rs34614851 | 301 | 1307287 | rs2853689 | 11292 |
| 1296427 | rs13361701 | 432 | 1307451 | rs34742644 | 11456 |
| 1296475 | rs4075202 | 480 | 1307594 | rs35719940 | 11599 |
| 1296699 | rs35661976 | 704 | 1307753 | rs35080081 | 11758 |
| 1296847 | rs4994840 | 852 | 1307844 | rs35523995 | 11849 |
| 1296867 | rs35948576 | 872 | 1307873 | rs34321948 | 11878 |
| 1297286 | rs7448994 | 1291 | 1307943 | rs35013447 | 11948 |
| 1297425 | rs4073918 | 1430 | 1308052 | rs34630753 | 12057 |
| 1297575 | rs35952163 | 1580 | 1308361 | rs35970923 | 12366 |
| 1298073 | rs10623391 | 2078 | 1308520 | rs33954691 | 12525 |
| 1298074 | rs33970920 | 2079 | 1308622 | rs35083412 | 12627 |
| 1298102 | rs6871519 | 2107 | 1308844 | rs35387865 | 12849 |
| 1298363 | rs4975540 | 2368 | 1309228 | rs35976105 | 13233 |
| 1298588 | rs7716467 | 2593 | 1309237 | rs35548585 | 13242 |
| 1299972 | rs13176158 | 3977 | 1309256 | rs34539509 | 13261 |
| 1299995 | rs6883980 | 4000 | 1309283 | rs2853688 | 13288 |
| 1300873 | rs4975620 | 4878 | 1309393 | rs34042051 | 13398 |
| 1301354 | rs12513872 | 5359 | 1309545 | rs35412895 | 13550 |
| 1301690 | rs34985696 | 5695 | 1309569 | rs34468758 | 13574 |
| 1301775 | rs12656500 | 5780 | 1309585 | rs2853687 | 13590 |
| 1301873 | rs6554691 | 5878 | 1309604 | rs35354089 | 13609 |
| 1302047 | rs4583925 | 6052 | 1309652 | rs34240934 | 13657 |
| 1302353 | rs4507531 | 6358 | 1309809 | rs35295542 | 13814 |
| 1302356 | rs35988686 | 6361 | 1309906 | rs34927774 | 13911 |
| 1302594 | rs10078761 | 6599 | 1310011 | rs34821059 | 14016 |
| 1303056 | rs6870915 | 7061 | 1310024 | rs34044586 | 14029 |
| 1303292 | rs6875445 | 7297 | 1310175 | rs36115594 | 14180 |
| 1303463 | rs2853693 | 7468 | 1310183 | rs36107545 | 14188 |
| 1303545 | rs2853692 | 7550 | 1310215 | rs35082932 | 14220 |
| 1304712 | rs34288233 | 8717 | 1310288 | rs35041195 | 14293 |
| 1304901 | rs4975539 | 8906 | 1310335 | rs35738432 | 14340 |
| 1305108 | rs35870082 | 9113 | 1310563 | rs34153812 | 14568 |
| 1305111 | rs10710089 | 9116 | 1310587 | rs34439046 | 14592 |
| 1305127 | rs34378183 | 9132 | 1310620 | rs34461542 | 14625 |
| 1305163 | rs35355672 | 9168 | 1310621 | rs2736122 | 14626 |
| 1305364 | rs35430833 | 9369 | 1310700 | rs35526657 | 14705 |
| 1305398 | rs34097921 | 9403 | 1310813 | rs35167723 | 14818 |
| 1305463 | rs4975618 | 9468 | 1310904 | rs34452728 | 14909 |
| 1305475 | rs13179246 | 9480 | 1310905 | rs34599610 | 14910 |
| 1305534 | rs4975617 | 9539 | 1310920 | rs5865366 | 14925 |
| 1305709 | rs35096542 | 9714 | 1311231 | rs35689290 | 15236 |
| 1305765 | rs34344863 | 9770 | 1311559 | rs35867091 | 15564 |
| 1305950 | rs2853691 | 9955 | 1311667 | rs35092866 | 15672 |
| 1305972 | rs35535053 | 9977 | 1311974 | rs35300412 | 15979 |
| 1306592 | rs34527601 | 10597 | 1312060 | rs2736121 | 16065 |
| 1306744 | rs2853690 | 10749 | 1312063 | rs13182885 | 16068 |
| 1306780 | rs5031049 | 10785 | 1312073 | rs13182892 | 16078 |
| 1306918 | rs35033501 | 10923 | 1312079 | rs9764053 | 16084 |
| 1307021 | rs35196264 | 11026 | 1312088 | rs2736120 | 16093 |
| 1307098 | rs35749463 | 11103 | 1312108 | rs13165630 | 16113 |
| 1307201 | rs35699764 | 11206 | 1312112 | rs2736119 | 16117 |
| 1307247 | rs34506158 | 11252 | 1313033 | rs28576270 | 17038 |
| 1307251 | rs34996780 | 11256 | 1313053 | rs2853686 | 17058 |

| C05 Bld 36 Location | Marker | Pos SEQ ID NO:1 |
|---|---|---|
| 1313195 | rs2736118 | 17200 |
| 1313331 | rs34018970 | 17336 |
| 1313401 | rs35880956 | 17406 |
| 1313449 | rs34238050 | 17454 |
| 1313450 | rs34693615 | 17455 |
| 1313461 | rs35703455 | 17466 |
| 1313510 | rs35727757 | 17515 |
| 1313513 | rs35735738 | 17518 |
| 1313514 | rs35311994 | 17519 |
| 1313515 | rs35973242 | 17520 |
| 1313715 | rs34062885 | 17720 |
| 1313837 | rs35605907 | 17842 |
| 1313846 | rs34555789 | 17851 |
| 1313877 | rs35550096 | 17882 |
| 1313957 | rs34026153 | 17962 |
| 1313961 | rs36049021 | 17966 |
| 1313982 | rs35621472 | 17987 |
| 1314051 | rs34041736 | 18056 |
| 1314052 | rs11133715 | 18057 |
| 1314220 | rs36077395 | 18225 |
| 1314314 | rs2736117 | 18319 |
| 1314317 | rs34524651 | 18322 |
| 1314460 | rs34060726 | 18465 |
| 1314749 | rs35412024 | 18754 |
| 1314803 | rs34853903 | 18808 |
| 1314809 | rs35348962 | 18814 |
| 1314866 | rs33987455 | 18871 |
| 1315002 | rs35122668 | 19007 |
| 1315004 | rs36121240 | 19009 |
| 1315344 | rs35901859 | 19349 |
| 1315404 | rs34074935 | 19409 |
| 1315492 | rs34864919 | 19497 |
| 1315667 | rs34653167 | 19672 |
| 1316016 | rs34714150 | 20021 |
| 1316042 | rs34909002 | 20047 |
| 1316053 | rs36119674 | 20058 |
| 1316322 | rs35228187 | 20327 |
| 1316339 | rs36077524 | 20344 |
| 1316378 | rs2736116 | 20383 |
| 1316408 | rs34529095 | 20413 |
| 1316471 | rs35617524 | 20476 |
| 1316477 | rs35999328 | 20482 |
| 1316587 | rs34895517 | 20592 |
| 1316650 | rs34289611 | 20655 |
| 1316987 | rs35209454 | 20992 |
| 1317068 | rs2736115 | 21073 |
| 1317145 | rs34555101 | 21150 |
| 1317152 | rs2853685 | 21157 |
| 1317218 | rs34057268 | 21223 |
| 1317290 | rs34656059 | 21295 |
| 1317483 | rs34458182 | 21488 |
| 1317587 | rs34528119 | 21592 |
| 1318204 | rs2736114 | 22209 |
| 1318373 | rs2736113 | 22378 |
| 1318664 | rs7730303 | 22669 |
| 1318723 | rs2736112 | 22728 |
| 1318853 | rs34864337 | 22858 |
| 1318935 | rs2736111 | 22940 |
| 1319141 | rs34823760 | 23146 |

| C05 Bld 36 Location | Marker | Pos SEQ ID NO:1 |
|---|---|---|
| 1319223 | rs9282869 | 23228 |
| 1319226 | rs2853684 | 23231 |
| 1319310 | rs2075786 | 23315 |
| 1319361 | rs35596904 | 23366 |
| 1319425 | rs36070059 | 23430 |
| 1319919 | rs34033712 | 23924 |
| 1320059 | rs35971139 | 24064 |
| 1320202 | rs3891054 | 24207 |
| 1320213 | rs34194491 | 24218 |
| 1320356 | rs4246742 | 24361 |
| 1320497 | rs11956330 | 24502 |
| 1320659 | rs35359768 | 24664 |
| 1320736 | rs34476748 | 24741 |
| 1320747 | rs35706685 | 24752 |
| 1320751 | rs34246010 | 24756 |
| 1320805 | rs34673480 | 24810 |
| 1320848 | rs35973454 | 24853 |
| 1320881 | rs35812074 | 24886 |
| 1320944 | rs17402061 | 24949 |
| 1320967 | rs34285898 | 24972 |
| 1321239 | rs35949937 | 25244 |
| 1321294 | rs35667898 | 25299 |
| 1321446 | rs34181584 | 25451 |
| 1321464 | rs35243220 | 25469 |
| 1321580 | rs33988305 | 25585 |
| 1321596 | rs34881188 | 25601 |
| 1321836 | rs34923115 | 25841 |
| 1321847 | rs6899038 | 25852 |
| 1321944 | rs6863310 | 25949 |
| 1322006 | rs6863494 | 26011 |
| 1322066 | rs35883631 | 26071 |
| 1322132 | rs6863697 | 26137 |
| 1322148 | rs34948922 | 26153 |
| 1322175 | rs35333551 | 26180 |
| 1322244 | rs35218116 | 26249 |
| 1322278 | rs34020702 | 26283 |
| 1322316 | rs11951776 | 26321 |
| 1322365 | rs6882077 | 26370 |
| 1322923 | rs35209375 | 26928 |
| 1322945 | rs35958533 | 26950 |
| 1322974 | rs35278007 | 26979 |
| 1323358 | rs34701155 | 27363 |
| 1323389 | rs35657226 | 27394 |
| 1323434 | rs35029914 | 27439 |
| 1323539 | rs34860744 | 27544 |
| 1323546 | rs7447741 | 27551 |
| 1323547 | rs35440658 | 27552 |
| 1323872 | rs34146029 | 27877 |
| 1323877 | rs34002187 | 27882 |
| 1323983 | rs11742908 | 27988 |
| 1324069 | rs34554161 | 28074 |
| 1324101 | rs34503345 | 28106 |
| 1324524 | rs11133719 | 28529 |
| 1324585 | rs35664326 | 28590 |
| 1324661 | rs13172201 | 28666 |
| 1324714 | rs35442442 | 28719 |
| 1324793 | rs34980560 | 28798 |
| 1324849 | rs35884863 | 28854 |
| 1324861 | rs35928703 | 28866 |

| C05 Bld 36 Location | Marker | Pos SEQ ID NO:1 |
|---|---|---|
| 1324878 | rs34297995 | 28883 |
| 1324879 | rs35082205 | 28884 |
| 1325191 | rs35929262 | 29196 |
| 1325197 | rs34031216 | 29202 |
| 1325654 | rs10700998 | 29659 |
| 1325688 | rs10700999 | 29693 |
| 1325701 | rs6885542 | 29706 |
| 1325722 | rs10701000 | 29727 |
| 1325740 | rs6860512 | 29745 |
| 1325813 | rs3134645 | 29818 |
| 1325884 | rs6860815 | 29889 |
| 1325886 | rs2853682 | 29891 |
| 1325900 | rs10701001 | 29905 |
| 1325975 | rs35507727 | 29980 |
| 1325986 | rs4484476 | 29991 |
| 1326020 | rs34767663 | 30025 |
| 1326075 | rs34958852 | 30080 |
| 1326102 | rs34894456 | 30107 |
| 1326101 | rs5865363 | 30106 |
| 1326213 | rs36063319 | 30218 |
| 1326270 | rs34084612 | 30275 |
| 1326648 | rs5865362 | 30653 |
| 1326649 | rs5865361 | 30654 |
| 1326861 | rs2736123 | 30866 |
| 1327445 | rs35517815 | 31450 |
| 1327646 | rs35963133 | 31651 |
| 1327727 | rs35192176 | 31732 |
| 1327830 | rs34774976 | 31835 |
| 1327983 | rs35768726 | 31988 |
| 1328528 | rs4975605 | 32533 |
| 1328857 | rs13156167 | 32862 |
| 1328887 | rs13156282 | 32892 |
| 1328914 | rs13170634 | 32919 |
| 1328915 | rs13156298 | 32920 |
| 1328925 | rs13170637 | 32930 |
| 1328936 | rs13170644 | 32941 |
| 1328952 | rs13156311 | 32957 |
| 1328953 | rs13170656 | 32958 |
| 1330275 | rs35096965 | 34280 |
| 1330488 | rs35072952 | 34493 |
| 1330577 | rs33961405 | 34582 |
| 1330637 | rs11750711 | 34642 |
| 1330728 | rs35811757 | 34733 |
| 1330729 | rs35577391 | 34734 |
| 1330759 | rs7737938 | 34764 |
| 1330803 | rs7719661 | 34808 |
| 1330976 | rs7724028 | 34981 |
| 1331092 | rs35438621 | 35097 |
| 1331125 | rs34140705 | 35130 |
| 1331570 | rs34682571 | 35575 |
| 1331584 | rs2075785 | 35589 |
| 1331629 | rs34049290 | 35634 |
| 1332224 | rs35241335 | 36229 |
| 1332306 | rs33951489 | 36311 |
| 1332391 | rs34108128 | 36396 |
| 1332439 | rs33963617 | 36444 |
| 1332505 | rs33956095 | 36510 |
| 1332523 | rs34625402 | 36528 |
| 1332627 | rs28428579 | 36632 |

| C05 Bld 36 Location | Marker | Pos SEQ ID NO:1 |
|---|---|---|
| 1332639 | rs34795236 | 36644 |
| 1332701 | rs35247701 | 36706 |
| 1332790 | rs10069690 | 36795 |
| 1332813 | rs34227159 | 36818 |
| 1332815 | rs35278664 | 36820 |
| 1332837 | rs35656989 | 36842 |
| 1332904 | rs35045715 | 36909 |
| 1332964 | rs10054203 | 36969 |
| 1333022 | rs10078991 | 37027 |
| 1333028 | rs2242652 | 37033 |
| 1333128 | rs7734992 | 37133 |
| 1333152 | rs34859168 | 37157 |
| 1333189 | rs34197543 | 37194 |
| 1333252 | rs34471035 | 37257 |
| 1333258 | rs35695689 | 37263 |
| 1333263 | rs33948291 | 37268 |
| 1333387 | rs34170122 | 37392 |
| 1333411 | rs33959226 | 37416 |
| 1333477 | rs13167280 | 37482 |
| 1333573 | rs35868315 | 37578 |
| 1333589 | rs35659884 | 37594 |
| 1333830 | rs4975538 | 37835 |
| 1333938 | rs6897196 | 37943 |
| 1333948 | rs34002450 | 37953 |
| 1333950 | rs11278847 | 37955 |
| 1334079 | rs35079836 | 38084 |
| 1334418 | rs35071105 | 38423 |
| 1334429 | rs35818299 | 38434 |
| 1334529 | rs34989691 | 38534 |
| 1334693 | rs6866456 | 38698 |
| 1334743 | rs6881768 | 38748 |
| 1334890 | rs6554743 | 38895 |
| 1334975 | rs6554744 | 38980 |
| 1335020 | rs36002710 | 39025 |
| 1335159 | rs6867141 | 39164 |
| 1335165 | rs6867270 | 39170 |
| 1335194 | rs7722143 | 39199 |
| 1335220 | rs35300318 | 39225 |
| 1335319 | rs7726159 | 39324 |
| 1335407 | rs34301490 | 39412 |
| 1335414 | rs7725218 | 39419 |
| 1335452 | rs34399181 | 39457 |
| 1335654 | rs35809415 | 39659 |
| 1335746 | rs34846301 | 39751 |
| 1336104 | rs35888851 | 40109 |
| 1336312 | rs7713218 | 40317 |
| 1336334 | rs2853681 | 40339 |
| 1336339 | rs2853680 | 40344 |
| 1336371 | rs2853679 | 40376 |
| 1336375 | rs3134644 | 40380 |
| 1336399 | rs2853678 | 40404 |
| 1336486 | rs7717443 | 40491 |
| 1336841 | rs6420019 | 40846 |
| 1337046 | rs6420020 | 41051 |
| 1337135 | rs4449583 | 41140 |
| 1337151 | rs34785213 | 41156 |
| 1337389 | rs11951797 | 41394 |
| 1337421 | rs35903242 | 41426 |
| 1337484 | rs13189814 | 41489 |

| C05 Bld 36 Location | Marker | Pos SEQ ID NO:1 |
|---|---|---|
| 1337507 | rs11960793 | 41512 |
| 1337517 | rs11960795 | 41522 |
| 1337525 | rs11954060 | 41530 |
| 1337532 | rs13188694 | 41537 |
| 1337535 | rs11951801 | 41540 |
| 1337554 | rs13175402 | 41559 |
| 1337568 | rs6898599 | 41573 |
| 1337576 | rs13188816 | 41581 |
| 1337580 | rs13189988 | 41585 |
| 1337597 | rs13171544 | 41602 |
| 1337619 | rs13190026 | 41624 |
| 1337628 | rs13175540 | 41633 |
| 1337631 | rs13171555 | 41636 |
| 1337705 | rs28374414 | 41710 |
| 1337749 | rs2736101 | 41754 |
| 1337767 | rs3134643 | 41772 |
| 1337789 | rs34170979 | 41794 |
| 1337795 | rs35318915 | 41800 |
| 1337806 | rs36105933 | 41811 |
| 1337976 | rs35029535 | 41981 |
| 1338162 | rs10866498 | 42167 |
| 1338681 | rs11334193 | 42686 |
| 1338694 | rs11323794 | 42699 |
| 1338974 | rs7705526 | 42979 |
| 1339293 | rs35135137 | 43298 |
| 1339477 | rs34363858 | 43482 |
| 1339516 | rs2736100 | 43521 |
| 1339532 | rs35116243 | 43537 |
| 1339846 | rs35490698 | 43851 |
| 1340002 | rs34829399 | 44007 |
| 1340194 | rs2853677 | 44199 |
| 1340209 | rs35402043 | 44214 |
| 1340290 | rs35838177 | 44295 |
| 1340340 | rs2736099 | 44345 |
| 1340505 | rs7710703 | 44510 |
| 1340623 | rs11291391 | 44628 |
| 1340626 | rs34211134 | 44631 |
| 1341151 | rs34790490 | 45156 |
| 1341303 | rs35086922 | 45308 |
| 1341547 | rs2853676 | 45552 |
| 1341883 | rs34677523 | 45888 |
| 1342286 | rs2853675 | 46291 |
| 1342287 | rs2853674 | 46292 |
| 1342300 | rs2853673 | 46305 |
| 1342463 | rs11950844 | 46468 |
| 1342510 | rs35467152 | 46515 |
| 1342535 | rs35260354 | 46540 |
| 1342552 | rs35121132 | 46557 |
| 1342553 | rs35849820 | 46558 |
| 1342579 | rs34209796 | 46584 |
| 1342594 | rs35252439 | 46599 |
| 1342617 | rs34450169 | 46622 |
| 1342619 | rs35994758 | 46624 |
| 1342997 | rs34582601 | 47002 |
| 1343240 | rs11414507 | 47245 |
| 1345191 | rs34253063 | 49196 |
| 1345299 | rs35334674 | 49304 |
| 1345351 | rs36006348 | 49356 |
| 1345446 | rs34052286 | 49451 |
| 1345626 | rs34006362 | 49631 |
| 1345635 | rs34952664 | 49640 |
| 1345643 | rs33951612 | 49648 |
| 1345649 | rs35746398 | 49654 |
| 1345714 | rs7713080 | 49719 |
| 1345810 | rs35291888 | 49815 |
| 1345983 | rs2853672 | 49988 |
| 1346291 | rs2853671 | 50296 |
| 1346339 | rs35127005 | 50344 |
| 1346368 | rs35691354 | 50373 |
| 1346570 | rs35459373 | 50575 |
| 1346767 | rs34094720 | 50772 |
| 1347086 | rs2736098 | 51091 |
| 1347338 | rs35837567 | 51343 |
| 1347429 | rs11952056 | 51434 |
| 1348204 | rs2735943 | 52209 |
| 1348208 | rs2853670 | 52213 |
| 1348216 | rs35733142 | 52221 |
| 1348243 | rs35550267 | 52248 |
| 1348274 | rs34654879 | 52279 |
| 1348307 | rs35148557 | 52312 |
| 1348322 | rs34233268 | 52327 |
| 1348340 | rs35265333 | 52345 |
| 1348349 | rs2853669 | 52354 |
| 1348373 | rs35226131 | 52378 |
| 1348452 | rs35161420 | 52457 |
| 1348456 | rs34328523 | 52461 |
| 1348459 | rs34764648 | 52464 |
| 1348514 | rs35596874 | 52519 |
| 1348617 | rs10462697 | 52622 |
| 1348682 | rs33958877 | 52687 |
| 1348701 | rs2735942 | 52706 |
| 1348716 | rs34768248 | 52721 |
| 1348735 | rs13181701 | 52740 |
| 1348803 | rs34685900 | 52808 |
| 1349072 | rs7712562 | 53077 |
| 1349090 | rs33958769 | 53095 |
| 1349255 | rs3215401 | 53260 |
| 1349259 | rs5865365 | 53264 |
| 1349260 | rs33923311 | 53265 |
| 1349421 | rs36081204 | 53426 |
| 1349451 | rs2735941 | 53456 |
| 1349456 | rs2736110 | 53461 |
| 1349486 | rs2735940 | 53491 |
| 1349653 | rs33985695 | 53658 |
| 1349727 | rs33977403 | 53732 |
| 1349751 | rs35638571 | 53756 |
| 1349758 | rs33987166 | 53763 |
| 1349759 | rs2736109 | 53764 |
| 1350078 | rs10548207 | 54083 |
| 1350082 | rs36044608 | 54087 |
| 1350258 | rs6554754 | 54263 |
| 1350474 | rs10618795 | 54479 |
| 1350488 | rs2736108 | 54493 |
| 1350854 | rs2736107 | 54859 |
| 1350918 | rs7449190 | 54923 |
| 1351645 | rs3888705 | 55650 |
| 1351733 | rs13190087 | 55738 |
| 1351782 | rs2736106 | 55787 |

| C05 Bld 36 Location | Marker | Pos SEQ ID NO:1 |
|---|---|---|
| 1351806 | rs34736137 | 55811 |
| 1352213 | rs2735948 | 56218 |
| 1352379 | rs2735846 | 56384 |
| 1352388 | rs35821362 | 56393 |
| 1352392 | rs2735947 | 56397 |
| 1352756 | rs2736105 | 56761 |
| 1352859 | rs13174814 | 56864 |
| 1352862 | rs13174919 | 56867 |
| 1352980 | rs35266184 | 56985 |
| 1353025 | rs2853668 | 57030 |
| 1353070 | rs2853667 | 57075 |
| 1353310 | rs4975612 | 57315 |
| 1353401 | rs2736103 | 57406 |
| 1353429 | rs2735946 | 57434 |
| 1353439 | rs11749061 | 57444 |
| 1353497 | rs34868693 | 57502 |
| 1353580 | rs36037576 | 57585 |
| 1353584 | rs2735845 | 57589 |
| 1353643 | rs35535864 | 57648 |
| 1354874 | rs6880140 | 58879 |
| 1355115 | rs34218850 | 59120 |
| 1355144 | rs2736102 | 59149 |
| 1355914 | rs2853666 | 59919 |
| 1356580 | rs4975613 | 60585 |
| 1356901 | rs2735945 | 60906 |
| 1357238 | rs10052815 | 61243 |
| 1357432 | rs2735944 | 61437 |
| 1357445 | rs10070025 | 61450 |

**Table 9.** SNP markers in the coding and promoter sequences of the TERT gene. Shown are SNPs in exons of the TERT gene and one promoter SNP that has been associated with TERT expression (Matsubara, *et al., 2006, BBRC341,* 128-131)

| Region | Pos in Bld 36 | Db SNP rs# | Function | dbSNP | Protein | Amino acid |
|---|---|---|---|---|---|---|
| exon 16 | 1306918 | rs35033501 | synonymous | A | Pro [P] | 1108 |
| | | | contig reference | G | Pro [P] | 1108 |
| exon 15 | 1307594 | rs35719940 | missense | A | Thr [T] | 1062 |
| | | | contig reference | G | Ala [A] | 1062 |
| exon 14 | 1308520 | rs33954691 | synonymous | T | His [H] | 1013 |
| | | | contig reference | C | His [H] | 1013 |
| exon 12 | 1313715 | rs34062885 | missense | A | Arg [R] | 948 |
| | | | contig reference | C | Ser [S] | 948 |
| exon 11 | 1317587 | rs34528119 | synonymous | T | His [H] | 925 |
| | | | contig reference | C | His [H] | 925 |
| exon 5 | 1332439 | rs33963617 | synonymous | T | Ala [A] | 699 |
| | | | contig reference | C | Ala [A] | 699 |
| | 1332505 | rs33956095 | synonymous | T | Gly [G] | 677 |
| | | | contig reference | C | Gly [G] | 677 |

(continued)

| Region | Pos in Bld 36 | Db SNP rs# | Function | dbSNP | Protein | Amino acid |
|---|---|---|---|---|---|---|
| | 1332523 | rs34625402 | synonymous | A | Arg [R] | 671 |
| | | | contig reference | G | Arg [R] | 671 |
| exon 4 | 1333387 | rs34170122 | synonymous | G | Ala [A] | 612 |
| | | | contig reference | C | Ala [A] | 612 |
| | 1333411 | rs33959226 | synonymous | G | Ala [A] | 604 |
| | | | contig reference | A | Ala [A] | 604 |
| exon 3 | 1335654 | rs35809415 | synonymous, | T | Val [V] | 553 |
| | | | contig reference | C | Val [V] | 553 |
| exon 2 | 1346570 | rs35459373 | synonymous | G | Leu [L] | 477 |
| | | | contig reference | C | Leu [L] | 477 |
| | 1346767 | rs34094720 | missense | T | Tyr [Y] | 412 |
| | | | contig reference | C | His [H] | 412 |
| | 1347086 | rs2736098 | synonymous | A | Ala [A] | 305 |
| | | | contig reference | G | Ala [A] | 305 |
| | 1347338 | rs35837567 | synonymous | A | Ala [A] | 221 |
| | | | contig reference | G | Ala [A] | 221 |
| | 1347429 | rs11952056 | missense | C | Thr [T] | 191 |
| | | | contig reference | G | Ser [S] | 191 |
| exon 2 | 1347166 | rs61748181 | missense | C | Ala [A] | 279 |
| | | | | T | Thr [T] | |
| Promoter | 1349486 | rs2735940 | May affect | C | | |
| | | | | T | | |

## EXAMPLE 2

The C allele of marker rs401681 was found to be associated with a protection against cutaneous melanoma and colorectal cancer. Thus a significant association between rs401681(C) and protection against cutaneous melanoma (OR=0.88, P=$8.0\times10^{-4}$) in a sample set consisting of 2,443 melanoma cases and 30,839 controls from Iceland, Sweden and Spain. We note that a recently published study of telomere length in individuals with skin cancers showed that while short telomeres are associated with increased risk of BCC, long telomeres are associated with increased risk of melanoma (Han, J. et al. J Invest Dermatol 129, 415-21 (2009)). The rs401681(C) variant was also marginally associated with protection against colorectal cancer (OR=0.95, P=$8.4\times10^{-3}$) although this was not significant after taking into account the number of cancer sites tested.

## EXAMPLE 3

We examined the joint effect of rs401681(C) and rs2736098 (A), for 5 cancers, using only samples typed for both SNPs (Table 10). After adjusting for rs2736098 (A), the association of rs401681(C) remained significant in all except prostate cancer. After adjusting for rs401681(C), rs2736098 (A) remained significant for 3 cancers, lung, bladder and prostate. Overall, these results indicate that neither rs401681(C) nor rs2736098 (A) can, by themselves, fully account for the association observed between sequence variants in this region and the 5 cancer types. This suggests that a unique variant capturing the effect of both rs401681(C) and rs2736098(A) remains to be discovered or, alternatively, that the region contains more than one variant that predisposes to cancers at the same or different sites, analogous to the region on 8q24 where independent variants have been found that associate with different cancer types. We analyzed the association between 27 SNPs surrounding rs401681 and rs2736098 and the 17 cancer types studied using the Icelandic sample sets and found that 15 sites showed an association with one or more of these SNPs at the P<0.05 level (Table 11).

**Table 10**. Joint analysis of rs401681(C) and rs2736098 (A) of BCC and cancers of the lung, bladder, prostate and cervix.

| Cancer type | # populations | rs401681(C) adjusted for rs2736098(A) | | | rs2736098(A) adjusted for rs401681(C) | | |
|---|---|---|---|---|---|---|---|
| | | OR | 95% CI | *P* value | OR | 95% CI | *P* value |
| Basal cell carcinoma | 2 | 1.20 | 1.10-1.31 | $7.8\times10^{-5}$ | 1.09 | 0.99-1.21 | 0.091 |
| Lung cancer | 3 | 1.11 | 1.01-1.21 | 0.024 | 1.14 | 1.03-1.25 | 0.010 |
| Bladder cancer | 9 | 1.07 | 1.00-1.16 | 0.036 | 1.12 | 1.04-1.20 | 0.0034 |
| prostate cancer | 4 | 1.01 | 0.95-1.08 | 0.68 | 1.13 | 1.05-1.21 | 0.0015 |
| Cervical cancer | 1 | 1.27 | 1.03-1.55 | 0.022 | 0.97 | 0.77-1.22 | 0.80 |

**Table 11**. SNPs in the region depicted in Figure 1 with a P value <0.05 for one or more of 17 cancer sites, using chip genotyped and *in silico* genotyped cases and controls in Iceland

| SNP | DISEASE | D' | R2 |
|---|---|---|---|
| rs10060827 | SCC | 0.261966 | 0.029914 |
| rs13159461 | bladder | 0.059183 | 0.001709 |
| rs13159461 | cervix | 0.059183 | 0.001709 |
| rs13159461 | pancreas | 0.059183 | 0.001709 |
| rs2736100 | BCC | 0.139999 | 0.018319 |
| rs2736100 | lung | 0.139999 | 0.018319 |
| rs2736100 | thyroid | 0.139999 | 0.018319 |
| rs2736122 | colorectal | 0.244406 | 0.015226 |
| rs2736122 | prostate | 0.244406 | 0.015226 |
| rs2736122 | thyroid | 0.244406 | 0.015226 |
| rs2853668 | BCC | 0.298694 | 0.02924 |
| rs2853668 | thyroid | 0.298694 | 0.02924 |
| rs2853676 | bladder | 0.148461 | 0.010933 |
| rs2853676 | lung | 0.148461 | 0.010933 |
| rs2963265 | endometrium | 0.359744 | 0.08954 |
| rs31489 | BCC | 1 | 0.871795 |
| rs31489 | bladder | 1 | 0.871795 |
| rs31489 | cervix | 1 | 0.871795 |
| rs31489 | endometrium | 1 | 0.871795 |
| rs31489 | lung | 1 | 0.871795 |
| rs31489 | prostate | 1 | 0.871795 |
| rs401681 | BCC | NA | NA |
| rs401681 | bladder | NA | NA |
| rs401681 | cervix | NA | NA |
| rs401681 | endometrium | NA | NA |
| rs401681 | lung | NA | NA |
| rs401681 | prostate | NA | NA |
| rs402710 | BCC | 1 | 0.667674 |

(continued)

| SNP | DISEASE | D' | R2 |
|---|---|---|---|
| rs402710 | endometrium | 1 | 0.667674 |
| rs402710 | lung | 1 | 0.667674 |
| rs402710 | prostate | 1 | 0.667674 |
| rs4073918 | BCC | 0.001157 | 0.000001 |
| rs4073918 | bladder | 0.001157 | 0.000001 |
| rs4073918 | thyroid | 0.001157 | 0.000001 |
| rs4246736 | bladder | 0.054681 | 0.001421 |
| rs4246736 | kidney | 0.054681 | 0.001421 |
| rs4246736 | pancreas | 0.054681 | 0.001421 |
| rs4635969 | bladder | 1 | 0.361702 |
| rs4635969 | cervix | 1 | 0.361702 |
| rs4975536 | breast | 0.247364 | 0.032948 |
| rs4975542 | colorectal | 0.324438 | 0.005749 |
| rs4975596 | breast | 0.178476 | 0.0158 |
| rs4975605 | melanoma | 0.019886 | 0.00038 |
| rs4975605 | multi_myeloma | 0.019886 | 0.00038 |
| rs4975605 | thyroid | 0.019886 | 0.00038 |
| rs4975616 | BCC | 0.964609 | 0.86912 |
| rs4975616 | bladder | 0.964609 | 0.86912 |
| rs4975616 | cervix | 0.964609 | 0.86912 |
| rs4975616 | endometrium | 0.964609 | 0.86912 |
| rs4975616 | lung | 0.964609 | 0.86912 |
| rs4975625 | lung | 0.215441 | 0.027142 |
| rs4975625 | SCC | 0.215441 | 0.027142 |
| rs6554667 | endometrium | 0.230769 | 0.002143 |
| rs6554667 | prostate | 0.230769 | 0.002143 |
| rs7445640 | lung | 0.287008 | 0.044994 |
| rs7727745 | bladder | 0.025954 | 0.000063 |
| rs7727745 | lymphoma | 0.025954 | 0.000063 |
| rs7727745 | multi_myeloma | 0.025954 | 0.000063 |

D' and R2 with reference to rs401681
NA = not applicable

## EXAMPLE 4

We postulated that the cancer-associated sequence variants in the *TERT* gene might be associated with shorter telomeres. In order to test this hypothesis, we examined the association between rs401681 and rs2736098 and telomere length in DNA from whole blood, using a quantitative PCR assay. To limit variability, we took into account several factors that have been reported to affect telomere length, including age, gender and smoking status (Valdes, AM, et al., Lancet 366:662-664 (2005); Frenck, R.W Jr., et al. Proc Natl Acad Sci USA 95:5607-10 (1998)) and selected from our database 276 females born between 1925 and 1935 who reported to have never smoked and who had not been diagnosed with cancer. To maximize the contrast, only women homozygous for allele C or allele T at rs401681 were included in the test. In these subjects, rs401681(C) and rs2736098(A) were associated with shorter telomeres with nominal significance (P=0.017 and 0.027, respectively) (Figure 3, Table 12). However, when we tested telomere length in a group of 260 younger women (selected by the same criteria regarding smoking and cancer, but born between 1940 and 1950), there was no association between telomere length and the risk alleles. Indeed, the effect estimates, while insignificant (P=0.08 and 0.28 for rs401681 and rs2736098, respectively) were in the opposite direction. These results suggest that the variants may lead to an increase in the gradual shortening of telomeres over time, the effect only becoming apparent after a certain age.

**Table 12.** Multiple linear regression for log (telomerase/RNAseP)

| **Women born 1925-1935** | | | |
|---|---|---|---|
| **Variable** | **Estimate** | **Standard error** | ***P*-value** |
| (Intercept) | 2.67 | 0.632 | 3.31 E-05 |
| age | -0.001 | 0.008 | 0.891 |
| plate 2 | -0.061 | 0.064 | 0.343 |
| plate 3 | 0.071 | 0.099 | 0.471 |
| rs401681 allele C | -0.053 | 0.022 | 0.017 |
| **Variable** | **Estimate** | **Standard error** | ***P*-value** |
| (Intercept) | 2.607 | 0.634 | 5.31 E-05 |
| age | -0.001 | 0.008 | 0.921 |
| plate 2 | -0.069 | 0.064 | 0.285 |
| plate 3 | 0.068 | 0.099 | 0.49 |
| rs2736098 allele A | -0.064 | 0.029 | 0.027 |
| **Women born 1940-1950** | | | |
| **Variable** | **Estimate** | **Standard error** | ***P*-value** |
| (Intercept) | 2.29 | 0.367 | 1.78E-09 |
| age | -0.005 | 0.006 | 0.441 |
| plate 5 | -0.012 | 0.046 | 0.79 |
| plate 6 | -0.05 | 0.065 | 0.444 |
| rs401681 allele C | 0.028 | 0.016 | 0.081 |
| **Variable** | **Estimate** | **Standard error** | ***P*-value** |
| (Intercept) | 2.292 | 0.371 | 2.56E-09 |
| age | -0.004 | 0.006 | 0.451 |
| plate 5 | -0.005 | 0.046 | 0.921 |
| plate 6 | -0.044 | 0.065 | 0.504 |
| rs2736098 allele A | 0.025 | 0.024 | 0.283 |

## EXAMPLE 5

We assessed the association of rs401681(C) and rs2736098(A) with the major histological types of lung cancer (Table 13). For all histological types except carcinoids, the frequency of the risk variants was higher than in controls with the highest frequencies found in squamous cell carcinomas.

**Table 13.** Frequencies of rs401681(C) and rs2736098(A) in different histological subtypes of lung cancer

| **Histology** | **Iceland 28,890 controls, freq. 0.545** | | **Spain 1,427 controls, freq. 0.538** | | **Netherlands 1,832 controls, freq. 0.570** | |
|---|---|---|---|---|---|---|
| **rs401681 (C)** | **N** | **freq.** | **N** | **freq.** | **N** | **freq.** |
| Adenocarcinoma | 305 | 0.582 | 83 | 0.596 | 184 | 0.595 |
| Squamous cell carcinoma | 178 | 0.624 | 132 | 0.617 | 208 | 0.637 |
| Small cell carcinoma | 104 | 0.577 | 70 | 0.528 | 74 | 0.601 |
| Carcinoma NOS | 79 | 0.551 | 40 | 0.589 | 10 | 0.700 |
| Large cell carcinoma | 31 | 0.661 | 25 | 0.440 | 16 | 0.531 |
| Other (incl. Carcinoid) | 56 | 0.508 | 2 | 1.000 | 32 | 0.578 |

(continued)

| | 3,667 controls, freq. 0.272 | | 1,384 controls, freq. 0.229 | | 1,740 controls, freq. 0.286 | |
|---|---|---|---|---|---|---|
| **rs2736098 (A)** | **N** | **freq.** | **N** | **freq.** | **N** | **freq.** |
| Adenocarcinoma | 305 | 0.305 | 84 | 0.238 | 183 | 0.325 |
| Squamous cell carcinoma | 153 | 0.281 | 134 | 0.299 | 209 | 0.316 |
| Small cell carcinoma | 95 | 0.316 | 69 | 0.268 | 73 | 0.288 |
| Carcinoma NOS | 68 | 0.397 | 45 | 0.267 | 10 | 0.300 |
| Large cell carcinoma | 30 | 0.300 | 24 | 0.167 | 16 | 0.468 |
| Other (incl. Carcinoid) | 46 | 0.239 | 2 | 0.750 | 32 | 0.437 |

**EXAMPLE 6**

***Study populations and Methods***

**Icelandic study populations**

All projects at deCODE Genetics have been approved by the National Bioethics Committee and the Data Protection Authority of Iceland. Cancer cases were identified based on records from the nation-wide Icelandic Cancer Registry (ICR; www.krabbameinsskra.is) which includes information on the year and age at diagnosis, age at death, SNOMED code and ICD-10 classification. The ICR has been in operation since 1954, covers the entire population of Iceland and determines incidence of cancer by site. The registry receives information from all pathology and cytology laboratories in Iceland, in addition to hematology laboratories, hospital wards, private medical practitioners and other individual health care workers. Approximately 94.5% of diagnoses in the ICR have histological confirmation (Tulinius, H., et al. Cancer Epidemiol Biomarkers Prev 6:863-73 (1997)). All participants are recruited by trained nurses through special recruitment clinics where they donate a blood sample and answer a lifestyle questionnaire. Clinical information on cancer patients were extracted from medical records at treatment centers. Written informed consent was obtained from all participants. Personal identifiers associated with medical information and blood samples were encrypted with a third-party encryption system in which the Data Protection Authority of Iceland maintains the code.

*Controls*

The 28,890 controls (41.7 % males, 58.3% females; mean age 61 years; SD = 21) used in this study consisted of individuals from other ongoing genome-wide association studies at deCODE. The controls had not been diagnosed with any of the cancers under study according to nation-wide lists from the Icelandic Cancer Registry (ICR). No individual disease group accounts for more than 10% of the total control group. If we include all 36,139 chip-genotyped individuals in our control group (also those who have been diagnosed with cancer), the frequency of rs401681 (C) is 0.547 which is very similar to the frequency of 0.545 in the control group (N= 28,890) containing individuals that have not been diagnosed with a cancer.

*Skin cancer cases (BCC, SCC and melanoma)*

A detailed description of the skin cancer populations can be found in previous reports (Gudbjartsson, DF et al. Nat Genet 40:886-91 (2008); Stacey, SN et al. Nat Genet 40:1313-18 (2008)). The ICR has maintained records of BCC diagnoses since 1981. The records contain all new occurrences of histologically verified BCC, sourced from all the pathology laboratories in the country that deal with such lesions. Diagnoses of BCC made up to the end of 2007 were included and were identified by ICD10 code C44 with a SNOMED morphology code indicating BCC. The ICR has recorded histologically confirmed diagnoses of squamous cell carcinoma (SCC) of the skin since 1955. SCC diagnoses made up to the end of 2007 were included and were identified by ICD10 code C44 with a SNOMED morphology code indicating SCC. Invasive cutaneous melanoma (CM) was identified through ICD10 code C43. Metastatic melanoma (where the primary lesion had not been identified) was identified by a SNOMED morphology code indicating melanoma with a /6 suffix, regardless of the ICD10 code. Ocular melanoma (OM) and melanomas arising at mucosal sites were not included.

*Breast cancer cases*

A detailed description of the breast cancer population is given by Stacey et al. (2007) (Stacey SN et al. Nat Genet 39:865-9 (2007)). In brief, all prevalent cases living in Iceland who had a diagnosis entered into the ICR up to the end of December 2006 were eligible to participate in the study. The ICR contained the records of 4,785 individuals diagnosed during this period. Consent, samples and successful genotypes were obtained from approximately 1,945 patients. The median age at diagnosis for genotyped cases was 56 years as compared to 61 years for all breast cancer cases in the ICR.

*Cervical cancer cases*

A total of 803 women were diagnosed with invasive cervical cancer between January 1, 1955 and December 31, 2007. Samples from 276 women were available for direct genotyping and an equivalent of 93 women could be genotyped *in silico.* The median age at diagnosis for directly genotyped cases was 42 years (range 19-91 years) as compared to 46.5 years for all cervical cancer cases in the ICR.

*Lung cancer cases*

The Icelandic lung cancer study population has been described previously (Thorgeirsson, TE et al. Nature 452:638-42 (2008)). Briefly, a total of 3,665 lung cancer patients were diagnosed from January 1, 1955, to December 31, 2007. Recruitment of both prevalent and incident cases was initiated in the year 1998. Samples from 797 cases were available for genotyping and an equivalent of 652 cases were genotyped *in silico.* The lung cancer patients participating in the genetic study answer a lifestyle questionnaire that includes questions on smoking status (never, former, current), and the quantity and duration of smoking. The median age at diagnosis for directly genotyped cases was 67 years (range 16-91 years) as compared to 68 years for all lung cancer cases in the ICR.

*Prostate cancer cases*

A detailed description of the prostate cancer study population has been published previously (Amundadottir, LT et al Nat Genet 38:652-8 (2006)). Briefly, a total of 4,457 Icelandic prostate cancer patients were diagnosed from January 1, 1955, to December 31, 2007. The Icelandic prostate cancer sample included 1,754 directly genotyped patients and an equivalent of 522 cases genotyped *in silico.* The mean age at diagnosis for directly genotyped patients was 71 years (median 71 years) and the range was from 40 to 96 years, while the mean age at diagnosis was 73 years for all prostate cancer cases in the ICR.

*Urinary bladder cancer (UBC) cases*

A description of Icelandic UBC cases has been published previously (Kiemeney, LA et al. Nat Genet 40:1307-12 (2008)). The ICR contains records of 1,642 Icelandic UBC patients diagnosed from January 1, 1955 to December 31, 2006. Recruitment started in the year 2001 and both prevalent and incident cases were included. The mean participation rate for newly diagnosed cases was 65%. Samples from 578 cases (76% males; diagnosed from December 1974 to June 2006) were available for direct genotyping and an equivalent of 202 cases were genotyped *in silico.* The median age at diagnosis for directly genotyped cases was 67 years (range 22-94 years) as compared to 68.5 years for all UBC cases in the ICR.

*Colorectal, cancer*

A total of 3,615 individuals were diagnosed with colorectal cancer between January 1, 1955 and December 31, 2007. Samples from 1,044 cases were available for direct genotyping and an equivalent of 529 cases were genotyped *in silico.* The median age at diagnosis for directly genotyped cases was 68 years as compared to 72 years for all colorectal cancer cases in the ICR.

*Endometrial cancer*

A total of 889 women were diagnosed with endometrial cancer between January 1, 1955 and December 31, 2007. Samples from 387 women were available for genotyping and an equivalent of 83 women were genotyped *in silico.* The median age at diagnosis for directly genotyped cases was 60 years as compared to 63 years for all endometrial cancer patients in the ICR.

*Kidney cancer*

A total of 1,472 individuals were diagnosed with renal cell cancer between January 1, 1955 and December 31, 2007. Samples from 422 cases were available for genotyping and an equivalent of 203 cases were genotyped *in silico.* The median age at diagnosis for all directly genotyped cases was 65 years, or the same as for all renal cell cancer cases in the ICR.

*Lymphoma*

A total of 1,137 individuals were diagnosed with lymphoma between January 1, 1955 and December 31, 2007. Samples from 178 cases were available for genotyping and an equivalent of 70 cases were genotyped *in silico.* The median age at diagnosis for directly genotyped cases was 49 years as compared to 56 years for all lymphoma cases in the ICR

*Multiple Myeloma*

A total of 483 individuals were diagnosed with multiple myeloma between January 1, 1955 and December 31, 2007. Samples from 64 cases were available for genotyping and an equivalent of 62 cases were genotyped *in silico.* The median age at diagnosis for directly genotyped cases was 68 years as compared to 69 years for all multiple myeloma cases in the ICR

*Ovarian cancer*

A total of 1,072 women were diagnosed with ovarian cancer between January 1, 1955 and December 31, 2007. Samples from 363 women were available for genotyping and an equivalent of 134 women were genotyped *in silico.* The median age at diagnosis for all directly genotyped cases was 51 years as compared to 60 years for all ovarian cancer cases in the *ICR.Pancreatic cancer*

A total of 1,134 individuals were diagnosed with pancreatic cancer between January 1, 1955 and December 31, 2007. Samples from 75 cases were available for genotyping and an equivalent of 226 cases were genotyped *in silico.* The median age at diagnosis for directly genotyped cases was 70 years as compared to 71 years for all pancreatic cancer cases in the ICR

*Stomach cancer*

A total of 3,210 individuals were diagnosed with stomach cancer between January 1, 1955 and December 31, 2007. Samples from 277 cases were available for genotyping and an equivalent of 485 cases were genotyped *in silico.* The median age at diagnosis for directly genotyped cases was 68 years as compared to 71 years for all stomach cancer cases in the ICR.

*Thyroid cancer*

A detailed description of the thyroid cancer population can be found in Gudmundson et al. (Gudmundsson, J *et al. submitted* (2008)). A total of 1,110 individuals were diagnosed with thyroid cancer between January 1, 1955 and December 31, 2007. Samples from 413 cases were available for direct genotyping and an equivalent of 115 cases were genotyped *in silico.* The median age at diagnosis for directly genotyped cases was 44 years as compared to 56 years for all thyroid cancer cases in the ICR.

**Dutch study populations**

*Controls*

The 1,832 cancer-free control individuals (46% males) were recruited within a project entitled "Nijmegen Biomedical Study" (NBS). The details of this study were reported previously (Wetzels, JF et al. Kidney Int 72:632-7 (2007)). Briefly, this is a population-based survey conducted by the Department of Epidemiology and Biostatistics and the Department of Clinical Chemistry of the Radboud University Nijmegen Medical Centre (RUNMC), in which 9,371 individuals participated from a total of 22,500 age- and sex stratified, randomly selected inhabitants of Nijmegen. Control individuals from the NBS were invited to participate in a study on gene-environment interactions in multi-factorial diseases, such as cancer. The 1,832 controls is a subsample of all the participants to the NBS, frequency-age-matched to a series of breast cancer and a series of prostate cancer patients. All the 1,832 participants are of self-reported European descent and were fully informed about the goals and the procedures of the study. The study protocols of the NBS were approved by the Institutional Review Board of the RUNMC and all study subjects signed a written informed consent form.

*Prostate cancer cases*

The details of the recruitment of prostate cancer cases was reported previously (Gudmundsson, J et al. Nat Genet 39:631-7 (2007)). In short, the case series (994 genotyped cases) was comprised of two recruitment-sets; Group-A was comprised of hospital-based cases recruited from January 1999 to June 2006 at the Urology Outpatient Clinic of the Radboud University Nijmegen Medical Centre (RUNMC); Group-B consisted of cases recruited from June 2006 to December 2006 through a population-based cancer registry held by the Comprehensive Cancer Centre IKO that serves a region of 1.3 million inhabitants in the eastern part of the Netherlands (www.ikcnet.nl). This recruitment took place within the EU 6$^{th}$ Framework POLYGENE project, a project on the identification of susceptibility genes for prostate and breast cancer (www.polygene.eu)). Both A and B groups were of self-reported European descent. The average age at diagnosis for patients in Group-A was 63 years (median 63 years) and the range was from 43 to 83 years. The average age at diagnosis for patients in Group-B was 65 years (median 66 years) and the range was from 43 to 75 years. The study protocol was approved by the Institutional Review Board of Radboud University and all study subjects gave written informed consent.

*Bladder cancer cases*

The Dutch bladder cancer population has been described in a previous publication (Kiemeney, LA et al. Nat Genet 40:1307-12 (2008)). Briefly, patients were recruited for the Nijmegen Bladder Cancer Study (NBCS) (see http://dceg.cancer.gov/icbc/membership.html). As with the recruitment of the prostate cancer patients, the NBCS identified patients through the population-based regional cancer registry held by the Comprehensive Cancer Centre East, Nijmegen. Patients diagnosed between 1995 and 2006 under the age of 75 years were selected and their vital status and current addresses updated through the hospital information systems of the 7 community hospitals and one university hospital (RUNMC) that are covered by the cancer registry. All patients still alive on August 1, 2007 were invited to the study by the Comprehensive Cancer Center on behalf of the patients' treating physicians. In case of consent, patients were sent a lifestyle questionnaire to fill out and blood samples were collected by Thrombosis Service centers which hold offices in all the communities in the region. 1,651 patients were invited to participate. Of all the invitees, 1,082 gave informed consent (66%): 992 filled out the questionnaire (60%) and 1016 (62%) provided a blood sample. The number of participating patients was increased with a non-overlapping series of 376 bladder cancer patients who were recruited previously for a study on gene-environment interactions in three hospitals (RUNMC, Canisius Wilhelmina Hospital, Nijmegen, and Streekziekenhuis Midden-Twente, Hengelo, the Netherlands). Ultimately, completed questionnaires and blood samples were available for 1,276 and 1,392 patients, respectively. All the patients that were selected for the analyses (N=1,277) were of self-reported European descent. The median age at diagnosis was 62 (range 25-93) years and 82% of the participants were males. Data on tumor stage and grade were obtained through the cancer registry. The study protocols of the NBCS were approved by the Institutional Review Board of the RUNMC and all study subjects gave written informed consent.

*Lung cancer cases*

The collection of patients with lung cancer took place as an extension of the prostate, breast, and bladder cancer studies within the framework of the EU 6$^{th}$ framework POLYGENE project. Patients with lung cancer were identified through the population-based cancer registry of the Comprehensive Cancer Center IKO, Nijmegen, the Netherlands. Patients who were diagnosed in one of three hospitals (Radboud University Nijmegen Medical Center and Canisius Wilhelmina Hospital in Nijmegen and Rijnstate Hospital in Arnhem) and who were still alive at April 15$^{th}$, 2008 were recruited for a study on gene-environment interactions in lung cancer. 458 patients gave informed consent and donated a blood sample. This case series was increased with 94 patients to a total of 552 by linking three other studies to the population-based cancer registry in order to identify new occurrences of lung cancer among the participants of these other studies. All three other studies (i.e., POLYGENE, the Nijmegen Biomedical Study, and the RUNMC Urology Outpatient Clinic Epidemiology Study) were initiated to study genetic risk factors for disease and participants to these studies gave general informed consent for DNA-related research and linkage with disease registries. Information on histology, stage of disease, and age at diagnoses was obtained through the cancer registry.

*Kidney cancer cases*

The Dutch patients with kidney cancer were recruited through the outpatient urology clinic of the Radboud University Nijmegen Medical Center. From January 1999 onwards, blood samples and lifestyle data have been collected from patients visiting the outpatient clinic for studies into gene-environment interactions for urological diseases. The 8,000 patients who participated in this study gave informed consent for the study and for linking their data with disease registries. The study was linked with the population-based cancer registry in order to identify patients who were diagnosed with renal cell cancer. 362 patients were identified.

**Spanish study populations**

*Controls*

The 1,427 Spanish control samples are from individuals that attended the University Hospital in Zaragoza, Spain, for diseases other than cancer between November 2001 and May 2007. The controls were of both genders and median age was 52 years. Controls were questioned to rule out prior cancers before the blood sample was collected. All patients and controls were of self-reported European descent. Study protocols were approved by the Institutional Review Board of Zaragoza University Hospital. All subjects gave written informed consent

*Bladder cancer cases*

The patients were recruited from the Urology and Oncology Departments of Zaragoza Hospital between September 2007 and February 2008. A total of 173 patients with histologically-proven urothelial cell carcinoma of the bladder were enrolled (response 77%). The median time interval from bladder cancer diagnosis to collection of blood samples was 9 months (range 1 to 29 months). Clinical information including age at onset, grade and stage was obtained from medical records. The median age at diagnosis for the patients was 65 years (range 27 to 94) and 87% were males. Study protocols were approved by the Institutional Review Board of Zaragoza University Hospital. All subjects gave written informed consent.

*Lung cancer cases*

The patients were recruited from the Oncology Department of Zaragoza Hospital in Zaragoza, from June 2006 to June 2008. During the 24 month interval of recruitment, 367 patients were enrolled (88% participation rate). Clinical information including age at onset and histology were collected from medical records. Study protocols were approved by the Institutional Review Board of Zaragoza University Hospital. All subjects gave written informed consent.

*Prostate cancer cases*

The study population consisted of 560 prostate cancer cases of which 459 (82%) were successfully genotyped. The cases were recruited from the Oncology Department of Zaragoza Hospital in Zaragoza, Spain, from June 2005 to September 2006. All patients were of self-reported European descent. Clinical information including age at onset, grade and stage was obtained from medical records. The average age at diagnosis for the patients was 69 years (median 70 years) and the range was from 44 to 83 years. Study protocols were approved by the Institutional Review Board of Zaragoza University Hospital. All subjects were gave written informed consent.

**The Eastern Europe study population**

The details of this study population have been described previously (Thurumaran, RK et al. Carcinogenesis 27:1676-81 (2006)). Cases and controls were recruited as part of a study designed to evaluate the risk of various cancers due to environmental arsenic exposure in Hungary, Romania and Slovakia between 2002 and 2004. The recruitment was carried out in the counties of Bacs, Bekes, Csongrad and Jasz-Nagykun-Szolnok in Hungary; Bihor and Arad in Romania; and Banska Bytrica and Nitra in Slovakia. The BCC cases (525), bladder cancer cases (N=214) and controls (N=525) were of Hungarian, Romanian and Slovak nationalities. BCC and bladder cancer cases were invited on the basis of histopathological examinations by pathologists. Hospital-based controls were included in the study, subject to fulfillment of a set of criteria. All general hospitals in the study areas were involved in the process of control recruitment. The controls were frequency matched with cases for age, gender, country of residence and ethnicity. Controls included general surgery, orthopedic and trauma patients aged 30-79 years. Patients with malignant tumors, diabetes and cardiovascular diseases were excluded as controls. The median age for the bladder cancer patients was 65 years (range 36-90) and 83% of the patients were males. The median age at diagnosis for BCC cases was 67 years (range 30-85) and the median age for the controls was 61 years (range 28-83). 51% of the controls were males. The response rates among cases and controls were ~70%. Clinicians took venous blood from cases and controls after consent forms had been signed. Cases and controls recruited to the study were interviewed by trained personnel and completed a general lifestyle questionnaire. Ethnic background for cases and controls was recorded along with other characteristics of the study population. Local ethical boards approved the study.

**Leeds Bladder Cancer Study, United Kingdom**

Details of the Leeds Bladder Cancer Study have been reported previously (Sak, SC et al. Br J Cancer 92:2262-65 (2005)). In brief, patients from the urology department of St James's University Hospital, Leeds were recruited from August 2002 to March 2006. All those patients attending for cystoscopy or transurethral resection of a bladder tumor (TURBT) who had previously been found, or were subsequently shown, to have urothelial cell carcinoma of the bladder were included. Exclusion criteria were significant mental impairment or a blood transfusion in the past month. All non-Caucasians were excluded from the study leaving 764 patients. The median age at diagnosis of the patients was 73 years (range 30-101). 71% of the patients were male and 61% of all the patients had a low risk tumor (pTaG1/2). Genotyping was successful in 707 patients. The controls were recruited from the otolaryngology outpatients and ophthalmology inpatient and outpatient departments at St James's Hospital, Leeds, from August 2002 to March 2006. All controls of appropriate age for frequency matching with the cases were approached and recruited if they gave their informed consent. As for the cases, exclusion criteria for the controls were significant mental impairment or a blood transfusion in the past month. Also, controls were excluded if they had symptoms suggestive of bladder cancer, such as haematuria. 2.8% of the controls were non-Caucasian leaving 530 Caucasian controls for the study. 71% of the controls were male. Data were collected by a health questionnaire on smoking habits and smoking history (non- ex- or current smoker, smoking dose in pack-years), occupational exposure history (to plastics, rubber, laboratories, printing, dyes and paints, diesel fumes), family history of bladder cancer, ethnicity and place of birth, and places of birth of parents. The response rate of cases was approximately 99%, that among the controls approximately 80%. Ethical approval for the study was obtained from Leeds (East) Local Research Ethics Committee, project number 02/192.

**Torino Bladder Cancer Case Control Study, Italy**

The source of cases for the Torino bladder cancer study are two urology departments of the main hospital in Torino, the San Giovanni Battista Hospital (Matullo, G. et al. Cancer Epidemiol Biomarkers Prev 14:2569-78 (2005)). Cases are all Caucasian men, aged 40 to 75 years (median 63 years) and living in the Torino metropolitan area. They were newly diagnosed between 1994 and 2006 with a histologically confirmed, invasive or in situ, bladder cancer. The sources of controls are urology, medical and surgical departments of the same hospital in Torino. All controls are Caucasian men resident in the Torino metropolitan area. They were diagnosed and treated between 1994 and 2006 for benign diseases (such as prostatic hyperplasia, cystitis, hernias, heart failure, asthma, and benign ear diseases). Controls with cancer, liver or renal diseases and smoking related conditions were excluded. The median age of the controls was 57 years (range 40 to 74). Data were collected by a professional interviewer who used a structured questionnaire to interview both cases and controls face-to-face. Data collected included demographics (age, sex, ethnicity, region and education) and smoking. For cases, additional data were collected on tumor histology, tumor site, size, stage, grade, and treatment of the primary tumor. The response rates were 90% for cases and 75% for controls. Genotyping was successful for 329 cases and 379 controls. Ethical approval for the study was obtained from Comitato Etico Interaziendale, A.O.U. San Giovanni Batista - A.). C.T.O./ Maria Adelaide.

**The Brescia bladder cancer study, Italy**

The Brescia bladder cancer study is a hospital-based case-control study. The study was reported in detail previously (Shen M. et al. Cancer Epidemiol Biomarkers Prev 12:1234-40 (2003)). In short, the catchment area of the cases and controls was the Province of Brescia, a highly industrialized area in Northern Italy (mainly metal and mechanical industry, construction, transport, textiles) but also with relevant agricultural areas. Cases and controls were enrolled in 1997 to 2000 from the two main city hospitals. The total number of eligible subjects was 216 cases and 220 controls. The response rate (enrolled/eligible) was 93% (N=201) for cases and 97% (N=214) for controls. Genotyping was successful in 122 cases and 156 controls. Only males were included. All cases and controls had Italian nationality and were of Caucasian ethnicity. All cases had to be residents of the Province of Brescia, aged between 20 and 80, and newly diagnosed with histologically confirmed bladder cancer. The median age of the patients was 63 years (range 22-80). Controls were patients admitted for various urological non-neoplastic diseases and were frequency matched to cases on age, hospital and period of admission. The study was formally approved by the ethical committee of the hospital where the majority of subjects were recruited. A written informed consent was obtained from all participants. Data were collected from clinical charts (tumor histology, site, grade, stage, treatments, etc.) and by means of face-to-face interviews during hospital admission, using a standardized semi-structured questionnaire. The questionnaire included data on demographics (age, ethnicity, region, education, residence, etc.), and smoking. ISCO and ISIC codes and expert assessments were used for occupational coding. Blood samples were collected from cases and controls for genotyping and DNA adducts analyses.

**The Belgian Case Control Study of Bladder Cancer**

The Belgian study has been reported in detail (Kellen, E. et al. Int J Cancer 118:2572-78 (2006)). In brief, cases were selected from the Limburg Cancer Registry (LIKAR) and were approached through urologists and general practitioners. All cases were diagnosed with histologically confirmed urothelial cell carcinoma of the bladder between 1999 and 2004, and were Caucasian inhabitants of the Belgian province of Limburg. The median age of the patients was 68 years. 86% of all the patients were males. For the recruitment of controls, a request was made to the "Kruispuntbank" of the social security for simple random sampling, stratified by municipality and socio-economic status, among all citizens above 50 years of age of the province. The median age of the controls was 64 years; 59% of the controls were males. Three trained interviewers visited cases and controls at home. Information was collected through a structured interview and a standardized food frequency questionnaire. In addition, biological samples were collected. Data collected included medical history, lifetime smoking history, family history of bladder cancer and a lifetime occupational history. Informed consent was obtained from all participants and the study was approved by the ethical review board of the Medical School of the Catholic University of Leuven, Belgium.

**The Swedish Bladder Cancer Study**

The Swedish patients come from a population-based study of urinary bladder cancer patients diagnosed in the Stockholm region in 1995-1996 (Larsson, P. et al. Scand J Urol Nephrol 37:195-201 (2003)). Blood samples from 346 patients were available out of a collection of 538 patients with primary urothelial carcinoma of the bladder. The average age at onset for these patients is 69 years (range 32-97 years) and 67% of the patients are males. Clinical data, including age at onset, grade and stage of tumor, were prospectively obtained from hospitals and urology units in the region. The control samples came from blood donors in the Stockholm region and were from cancer free individuals of both genders. The regional ethical committee approved of the study and all participants gave informed consent.

**Prostate Cancer Study, Chicago**

The Chicago study population consisted of 680 prostate cancer cases of which 635 (93%) were successfully genotyped. The cases were recruited from the Pathology Core of Northwestern University's Prostate Cancer Specialized Program of Research Excellence (SPORE) from May 2002 to May 2007. The average age at diagnosis for the patients was 60 years (median 59 years) and the range was from 39 to 87 years. The 693 controls were recruited as healthy control subjects for genetic studies at the University of Chicago and Northwestern University Medical School, Chicago, US. All individuals from Chicago included in this report were of self-reported European descent. Study protocols were approved by the Institutional Review Boards of Northwestern University and the University of Chicago. All subjects gave written informed consent.

**IARC's dataset on lung cancer**

The International Agency for Research on Cancer (IARC), Lyon, France, the CEPH and the CNG, have conducted a genome-wide association study of lung cancer consisting of 1,926 lung cancer cases and 2,522 controls from five eastern European countries (Hung, RJ et al. Nature 452:633-37 (2008)). The results and data from the genome-wide phase of the study (310,023 SNPs) have been made available on the IARC website for other groups to use for meta-analyses and other studies. (http://www.cng.fr/prog_cancergenomics/lung_cancer.html).

**ICR's dataset on colorectal cancer**

The Institute of Cancer Research (ICR) has generated genotype data for 547,487 SNPs in 922 individuals with colorectal neoplasia and 927 controls ascertained through the Colorectal Tumour Gene Identification (CoRGI) consortium (Tomlionson IP et al. Nat Genet 40:623-30 (2008)). In order to facilitate the identification of additional loci predisposing to colorectal cancer, the genotype count data and allelic test results from the genome-wide phase of this study have been made available to other groups for meta-analyses and further studies. (http://www.icr.ac.uk/research/research_sections/cancer_genetics/cancer_genetics_teams/mole cular_and_population_genetics/software_and_databases/index.shtml)

### GCEMS dataset on prostate and breast cancer

The Cancer Genetics Markers of Susceptibility (CGEMS) initiative of the National Cancer Institute is conducting GWA studies with follow-up replication studies to identify common, inherited gene variations for cancers of the prostate and breast. Publicly available data from the GWA scans were retrieved from the projects website (https://caintegrator.nci.nih.gov/cgems).

### Genotyping

Whole-genome association studies have been performed on the following cancers in the Icelandic population; prostate cancer, breast cancer, lung cancer, BCC, melanoma, urinary bladder cancer and colorectal cancer (Stacey, SN et al Nat Genet 40:1313-18 (2008)); Stacey SN et al Nat Genet 39:865-9 (2007)); Thorgeirsson, TE et al Nature 452:638-42 (2008)); Kiemeney, LA et al. Nat Genet 40:1307-12 (2008)); Gudmundsson, J. et al. Nat Genet 39:631-37 (2007)). All cases and controls were assayed using genotyping systems and specialized software from Illumina (Human Hap300 and HumanCNV370-duo Bead Arrays, Illumina). Furthermore, all Dutch bladder cancer cases and controls have been genotyped with the HumanCNV370-duo Bead Arrays. These chips provide about 75% genomic coverage in the Utah CEPH (CEU) HapMap samples for common SNPs at r2>0.8 (Barrett, JC & Cardon, LR Nat Genet 38:659-62 (2006)). SNP data were discarded if the minor allele frequency in the combined case and control was <0.001 or had less than 95% yield or showed a very significant distortion from Hardy-Weinberg equilibrium in the controls ($P<1\times10^{-10}$). Any chips with a call rate below 98% of the SNPs were excluded from the genome-wide association analysis.

All single SNP genotyping was carried out applying the Centaurus (Nanogen) platform (Kutyavin, IV et al. Nucleic Acids Res 34:e128 (2006)). The quality of each Centaurus SNP assay was evaluated by genotyping each assay in the CEU HapMap samples and comparing the results with the HapMap publicly released data. Assays with > 1.5% mismatch rate were not used and a linkage disequilibrium (LD) test was used for markers known to be in LD. Approximately 10% of the Icelandic case samples that were genotyped on the Illumina platform were also genotyped using the Centaurus assays and the observed mismatch rate was lower than 0.5%. All genotyping was carried out at deCODE Genetics.

### Assessment of telomere length

We selected whole blood as the tissue for analyzing telomere length for its accessibility but studies have shown that the length of telomeres is very similar within different tissues of the same individual but vary significantly between individuals (Marten UM et al. Nat Genet 18:76-80 (2998)). Telomeres were measured utilizing quantitative Taqman® PCR as described by Cawthon (Cawthon, RM Nucleic Acids Res 30:e47 (2002)). RNAseP endogenous control assay (Cat.no. 4316844) (Applied Biosystems Inc.) was used to correct for DNA input. This quantitative PCR method has been shown to give consistent results as Southern blot and FISH based telomere measurements (Schwob, AE et al. Mol Biol Cell 19:1548-60 (2008)). All reactions were run on ABI7900TH real time PCR system (Applied Biosystems Inc.). All assays were done in duplicate and repeated in an independent experiment. The use of RNAseP is a standard procedure in gene dosage measurements with real time qRT-PCR (Schwob, AE et al. Mol Biol Cell 19:1548-60 (2008);Writzl, K. et al. Hum Reprod 21:753-4 (2006)). The main limitation of the method is that it measures relative telomere length rather than actual telomere length. For us, the relative telomere length is sufficient for determining if there is a difference in telomere length between individuals depending on their genotype.

### Regression Analysis of Telomere Length Data

A total of 528 females were analyzed in two batches, each batch done with 3 plates, batch 1 included 268 women with a mean age at blood sampling of 72.8 (SD 5.0) years, batch 2 included 260 women with a mean age at blood sampling of 57.8 (SD 4.6) years. The relationship between the SNPs showing association and telomerase length was analyzed by multiple regression. The logarithm of the ratio between telomerase and RNAseP was taken as dependent variable, and the covariates age at blood sampling and plate were included in the models. SNPs showing association were analyzed using multiple linear regression. The experiments were carried out at two different points in time and were analyzed separately.

### Association analysis

A likelihood procedure described in a previous publication (Gretarsdottir, S. et al. Nat Genet 35:131-8 (2003)) and implemented in the NEMO software was used for the association analyses. An attempt was made to genotype all individuals and all SNPs reported, and for each of the SNPs, the yield was higher than 95% in every study group. We tested the association of an allele to cancer using a standard likelihood ratio statistic that, if the subjects were unrelated, would have asymptotically a $\chi^2$ distribution with one degree of freedom under the null hypothesis. Allelic frequencies rather than carrier frequencies are presented for the markers in the main text. Allele-specific ORs and associated P values were calculated assuming a multiplicative model for the two chromosomes of an individual (Falk CT & Rubinstein P Ann Hum Genet 51(Pt3):227-33 (1987). Results from multiple case-control groups were combined using a Mantel-Haenszel model (Mantel N & Haenszel W J Natl cancer Inst 22:719-48 (1959)) in which the groups were allowed to have different population frequencies for alleles, haplotypes and genotypes but were assumed to have common relative risks. All P values are reported as two-sided.

For the analysis of the Icelandic samples, the same set of cancer free controls used in the BCC discovery analysis was used for all other cancer types, introducing a potential bias. However, due to the lack of association with common cancers like breast and colorectal cancer and also because of the modest effect sizes for the cancers associating with rs401681(C), the frequency of the variant is not substantially different in the Icelandic cancer free controls (0.545) compared to the whole group of Icelanders (N=36,139) genotyped with the BeadChips (0.547) which includes all cancer cases. Therefore, the potential bias introduced into the estimation of the association of the sixteen cancers with rs401681(C) is small. Furthermore, this effect is confined to the Icelandic part of our study.

**Test of un-genotyped Hapmap markers**

To test for SNP that are in the CEU section of the Hapmap database, but that are absent on the Illumina chip, we use a method based on haplotypes of two markers on the chip. We used a method we have previously employed (Styrkarsdottir, U et al. N Eng J Med 358:2355-65 (2008)), that is an extension of the two-marker haplotype tagging method (Pe'er, I et al. Nat Genet 38:663-7 (2006)) and is similar in spirit to two other proposed methods (Nicolae, DL Genet Epidemiol 30:718-27 (2006); Zaitlen N. et al. Am J Hum Genet 80:683-91 (2007)). We computed associations with a linear combination of the different haplotypes chosen to act as surrogates to HapMap markers in the regions. In the 5p13.33 region displayed in Figure 2 (corresponding to a 200 kb interval), we tested with this method 95 markers in addition to the ones on the chip. These calculations were based on 1,025 BCC cases and 28,890 controls genotyped on chip. Of those markers, rs2736098 had the most significantly association with BCC.

**Genomic control and inflation factors**

To adjust for possible population stratification and the relatedness amongst individuals, we divided the $\chi^2$ statistics from the initial scan of basal cell carcinoma in Iceland, using the method of genomic control, i.e. the 304 thousand test statistics were divided by their means, which was 1.22. In the cases where the method of genomic control is not directly applicable (i.e. if the genome wide association results are not available for the same groups), we used the genealogy to estimate the inflation factor. Since some of the Icelandic patients and controls are related to each other, both within and between groups, the $\chi^2$ statistics have a mean >1. We estimated the inflation factor by simulating genotypes through the Icelandic genealogy, as described previously, and corrected the $\chi^2$ statistics for Icelandic OR's accordingly. The estimated inflation factor for different analyses is presented in Table 15.

***In silico* genotyping of un-genotyped individuals**

We extend the classical SNP case-control association study design by including un-genotyped cases with genotyped relatives. For every un-genotyped case, we calculate the probability of the genotypes of its relatives given its four possible phased genotypes (see Figure below for an example). In practice we have chosen to include only the genotypes of the case's parents, children, siblings, half-siblings (and the half-sibling's parents), grand-parents, grand-children (and the grand-children's parents) and spouses. We assume that the individuals in the small sub-pedigrees created around each case are not related through any path not included in the pedigree. We also assume all alleles that are not transmitted to the case have the same frequency - the population allele frequency. The probability of the genotypes of the case's relatives can then be computed by:

$$\Pr(\text{genotypes of relatives};\theta) = \sum_{h\in\{AA,AG,GA,GG\}} \Pr(h;\theta)\Pr(\text{genotypes of relatives} \mid h),$$

where $\theta$ denotes the A allele's frequency in the cases. Assuming the genotypes of each set of relatives are independent, this allows us to write down a likelihood function for $\theta$:

$$L(\theta)=\prod_i \Pr(\text{genotypes of relatives of case } i;\theta)\,. \qquad (*)$$

This assumption of independence is usually false. Accounting for the dependence between individuals is a difficult and potentially prohibitively expensive computational task. The likelihood function in (*) may be thought of as a pseudolikelihood approximation of the full likelihood function for θ which properly accounts for all dependencies. In general, the genotyped cases and controls in a case-control association study are not independent and applying the case-control method to related cases and controls is an analogous approximation. The method of genomic control (Devlin B. et al. Nat Genet 36:1129-30 (2004)) has proven to be successful at adjusting case-control test statistics for relatedness. We therefore apply the method of genomic control to account for the dependence between the terms in our pseudolikelihood and produce a valid test statistic.

Fisher's information was used to estimate the effective sample size of the part of the pseudolikelihood due to un-genotyped cases. Breaking the total fisher information, $I$, into the part due to genotyped cases, $I_g$, and the part due to ungenotyped cases, $I_u$, $I = I_g + I_u$, and denoting the number of genotyped cases with $N$, the effective sample size due to the ungenotyped cases is estimated by $\frac{I_u}{I_g}N$.

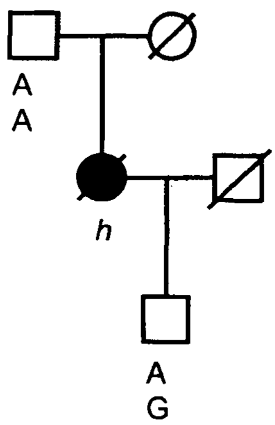


| Transmitted (*h*) | | |
|---|---|---|
| Paternally | Maternally | Prob(genotypes \| *h*)[a] |
| A | A | f |
| A | G | ½ |
| G | A | 0 |
| G | G | 0 |

**An example of how the genotypes of relatives are used to obtain information about the genotypes of an un-genotyped case.** Un-genotyped individuals are indicated by a strike-through. The case's father is homozygous for the A allele and the case's son is heterozygous AG. Therefore, the case must have received an A allele from her father and either transmitted an A or a G allele to her son, the probability of which depends upon the population frequency of A (denoted by f). [a]Probabilities are given up to a normalization constant.

**Table 14.** Frequency of rs401681 in cancer cases and controls genotyped directly by chip or single track assays and cancer cases genotyped *in silico*in Iceland.

| Cancer site | Genotyped | | Genotyped *in silico* | | Total | |
|---|---|---|---|---|---|---|
| | N cases | Freq. | effective N cases[a] (available N cases)[b] | Freq. | N cases | Freq. |

(continued)

| Cancer site | Genotyped | | Genotyped *in silico* | | Total | |
|---|---|---|---|---|---|---|
| | N cases | Freq. | effective N cases[a] (available N cases)[b] | Freq. | N cases | Freq. |
| BCC | 1,769 | 0.602 | 271 (959) | 0.619 | 2040 | 0.604 |
| Lung | 797 | 0.584 | 652 (2,092) | 0.565 | 1,449 | 0.575 |
| Bladder | 578 | 0.583 | 202(718) | 0.582 | 780 | 0.583 |
| Prostate | 1,754 | 0.564 | 522 (1,778) | 0.589 | 2,276 | 0.569 |
| Cervix | 276 | 0.611 | 93(388) | 0.611 | 369 | 0.611 |
| Breast | 1,945 | 0.543 | 556(1863) | 0.533 | 2,501 | 0.541 |
| Colorectal | 1,044 | 0.538 | 529 (1,716) | 0.533 | 1,573 | 0.536 |
| Cutaneous melanoma | 577 | 0.523 | 62 (204) | 0.500 | 639 | 0.520 |
| Endometrium | 387 | 0.580 | 83 (332) | 0.636 | 470 | 0.592 |
| Kidney | 422 | 0.585 | 203 (770) | 0.547 | 625 | 0.572 |
| Lymphoma | 178 | 0.497 | 70(206) | 0.544 | 248 | 0.510 |
| Multiple myeloma | 64 | 0.617 | 62(193) | 0.564 | 126 | 0.591 |
| Ovary | 363 | 0.541 | 134(447) | 0.541 | 497 | 0.541 |
| Pancreas | 75 | 0.513 | 226(712) | 0.553 | 301 | 0.543 |
| SCC (skin) | 547 | 0.578 | ND | ND | 547 | 0.578 |
| Stomach | 277 | 0.528 | 485(1,975) | 0.540 | 762 | 0.536 |
| Thyroid | 413 | 0.551 | 115(384) | 0.496 | 528 | 0.538 |

[a]Effective sample size from cases genotyped *in silico*
[b] Available for *in silico* genotyping (having a 1$^{st}$ or 2$^{nd}$ degree relative)
ND = not done

**Table 15.** Inflation factors used for correction of chi-square statistics in different analyses for relatedness and Genomic Control

| Cancer | rs401681 | | | | | | rs401681 and rs2736098 | | |
|---|---|---|---|---|---|---|---|---|---|
| | genotyped directly[a] | | | genotyped directly and *in silico*[b] | | | genotyped directly[a] | | |
| | N cases | N controls | Corr. factor | N cases | N controls | Corr. factor | N cases | N controls | Corr. Factor |
| Basal cell carcinoma | 1,769 | 28,890 | 1.11 | 2,040 | 28,890 | 1.16 | 1,600 | 3,667 | 1.17 |
| Lung cancer | 797 | 28,890 | 1.06 | 1,449 | 28,890 | 1.18 | 687 | 3,667 | 1.08 |
| Bladder cancer | 578 | 28,890 | 1.04 | 780 | 28,890 | 1.07 | 460 | 3,667 | 1.05 |
| Prostate cancer | 1,754 | 28,890 | 1.09 | 2,276 | 28,890 | 1.19 | 1,640 | 3,667 | 1.17 |
| Cervix cancer | 276 | 28,890 | 1.00 | 369 | 28.890 | 1.02 | 249 | 3,667 | 1.03 |

[a] Inflation factor calculated through a simulation of genealogy
[b] Inflation factor calculated by the method of genomic control, using the 300,000 markers from the chip

**EXAMPLE 7.**

The association of rs401681 with cutaneous melanoma was analyzed further. The initial discovery that allele C of this marker confers protection against melanoma was confirmed when expanding the analysis. As shown in Table 16, the association was assessed in additional cohorts from Sweden, Spain, Holland, Austria and Italy. All cohorts independently indicated a protective effect of the C allele, with overall OR value of 0.86 and the overall p-value of

$5.0 \times 10^{-8}$ (Table 16). These results confirm that the C allele of rs401681 confers protection against cutaneous melanoma, and as a consequence the alternate allele T of rs401681 is a risk allele of cutaneous melanoma, with an OR value of 1.16.

**Table 16.** Association of rs401681 with cutaneous melanoma (CM) in several populations.

| | | Number | | Frequency | | | | |
|---|---|---|---|---|---|---|---|---|
| Sample Group | Risk Allele | Cases | Controls | Cases | Controls | OR | 95% CI | *P* |
| Iceland CM | C | 591 | 34,998[a] | 0.52 | 0.55 | 0.90 | (0.80, 1.01) | $7.9 \times 10^{-2}$ |
| Sweden CM | C | 1,056 | 2,631 | 0.49 | 0.54 | 0.85 | (0.77, 0.94) | $10^{-3}$ $1.2 \times 10^{-3}$ |
| Spain CM | C | 748 | 1,758 | 0.51 | 0.54 | 0.90 | (0.80, 1.02) | $10^{-2}$ $9.4 \times 10^{-2}$ |
| Holland CM | C | 736 | 1,832 | 0.53 | 0.57 | 0.83 | (0.73,0.94) | $3.9 \times 10^{-3}$ |
| Austria CM | C | 152 | 376 | 0.53 | 0.53 | 0.98 | (0.75, 1.27) | 0.88 |
| Italy CM | C | 560 | 368 | 0.49 | 0.56 | 0.74 | (0.62, 0.89) | $1.2 \times 10^{-3}$ |
| **All CM Combined** | C | **3,843** | **41,963** | **NA** | **NA** | 0.86 | **(0.81, 0.91)** | $\mathbf{5.0 \times 10^{-8}}$ |

[a] Skin cancer-free controls.

## Claims

1. A method of determining a susceptibility to a cancer selected from the group consisting of basal cell carcinoma, lung cancer, bladder cancer, prostate cancer, cervical cancer, endometrial cancer and cutaneous melanoma in a human individual from a Caucasian population, the method comprising:

   obtaining nucleic acid sequence data about a human individual identifying at least one allele of at least one polymorphic marker selected from the group consisting of rs401681, and markers in linkage disequilibrium therewith selected from the group consisting of rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 and rs37005, wherein different alleles of the at least one polymorphic marker are associated with different susceptibilities to cancer in humans,
   wherein obtaining nucleic acid sequence data comprises analyzing sequence of the at least one polymorphic marker in a nucleic acid in a sample obtained from the human individual, and
   determining a susceptibility to the cancer from the nucleic acid sequence data.

2. The method of claim 1, wherein determination of a susceptibility comprises comparing the nucleic acid sequence data to a database containing correlation data between the at least one polymorphic marker and susceptibility to cancer.

3. The method of claim 1, wherein analyzing sequence of the at least one polymorphic marker comprises determining the presence or absence of at least one allele of the at least one polymorphic marker.

4. The method of any one of the preceding claims, further comprising reporting the susceptibility to at least one entity selected from the group consisting of the individual, a guardian of the individual, a genetic service provider, a physician, a medical organization, and a medical insurer.

5. A method of identification of a marker for use in assessing susceptibility to a cancer selected from the group consisting of basal cell carcinoma, lung cancer, bladder cancer, prostate cancer, cervical cancer, endometrial cancer and cutaneous melanoma in humans from a Caucasian population, the method comprising:

   a. identifying at least one polymorphic marker in linkage disequilibrium with at least one of rs401681, wherein linkage disequilibrium is **characterized by** values of $r^2$ greater than 0.2 in Caucasians;
   b. determining the genotype status of a sample of individuals diagnosed with, or having a susceptibility to, the cancer; and
   c. determining the genotype status of a sample of control individuals;
   wherein a significant difference in frequency of at least one allele in at least one polymorphism in individuals diagnosed with, or having a susceptibility to, cancer, as compared with the frequency of the at least one allele in the control sample is indicative of the at least one polymorphism being useful for assessing susceptibility to the cancer.

6. The method according to Claim 5, wherein an increase in frequency of the at least one allele in the at least one polymorphism in individuals diagnosed with, or having a susceptibility to, the cancer, as compared with the frequency of the at least one allele in the control sample is indicative of the at least one polymorphism being useful for assessing increased susceptibility to the cancer, and wherein a decrease in frequency of the at least one allele in the at least one polymorphism in individuals diagnosed with, or having a susceptibility to, the cancer, as compared with the frequency of the at least one allele in the control sample is indicative of the at least one polymorphism being useful for assessing decreased susceptibility to, or protection against, the cancer.

7. A method of genotyping a nucleic acid sample obtained from a human individual from a Caucasian population comprising genotyping nucleic acid in said sample for determining whether at least one allele of at least one polymorphic marker is present in a nucleic acid sample from the individual sample, wherein the at least one marker is selected from rs401681, and markers in linkage disequilibrium therewith, selected from the group consisting of rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 and rs37005, and wherein the presence of the at least one allele in the sample is indicative of a susceptibility to a cancer selected from the group consisting of basal cell carcinoma, lung cancer, bladder cancer, prostate cancer, cervical cancer, endometrial cancer and cutaneous melanoma in the individual.

**8.** An apparatus for determining a genetic indicator for a cancer selected from the group consisting of basal cell carcinoma, lung cancer, bladder cancer, prostate cancer, cervical cancer, endometrial cancer and cutaneous melanoma in a human individual from a Caucasian population, comprising:

a processor
a computer readable memory having computer executable instructions adapted to be executed on the processor to analyze marker and/or haplotype information for at least one human individual with respect to at least one polymorphic marker selected from rs401681, and markers in linkage disequilibrium therewith selected from the group consisting of rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 and rs37005, and
generate an output based on the marker or haplotype information, wherein the output comprises a risk measure of the at least one marker or haplotype as a genetic indicator of the cancer for the human individual.

**9.** The apparatus according to Claim 8, wherein the computer readable memory further comprises data indicative of the risk of developing the cancer associated with at least one allele of at least one polymorphic marker or at least one haplotype, and wherein a risk measure for the human individual is based on a comparison of the at least one marker and/or haplotype status for the human individual to the risk of the cancer associated with the at least one allele of the at least one polymorphic marker or the at least one haplotype.

**10.** A method for determining a susceptibility to a cancer selected from the group consisting of basal cell carcinoma, lung cancer, bladder cancer, prostate cancer, cervical cancer, endometrial cancer and cutaneous melanoma in a human individual from a Caucasian population, comprising determining whether at least one allele of at least one polymorphic marker is present in a nucleic acid sample obtained from the individual wherein the at least one polymorphic marker is selected from the group consisting of rs401681, and markers in linkage disequilibrium therewith selected from the group consisting of rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 and rs37005, and wherein the presence of the at least one allele is indicative of a susceptibility to the cancer for the individual.

**11.** The method or apparatus of any one of the claims 1 to 4 or 7 to 10, wherein the cancer is selected from the group consisting of basal cell carcinoma, lung cancer, bladder cancer, prostate cancer, cervical cancer and endometrial cancer, and wherein determination of the presence of allele C in rs401681 and/or allele A of rs2736098 is indicative of an increased susceptibility to the cancer for the individual.

**12.** The method or apparatus of any one of the claims 1 to 4 or 7 to 10, wherein the cancer is cutaneous melanoma, and wherein determination of the presence of allele C in rs401681 is indicative of a decreased susceptibility to the cancer for the individual.

## Patentansprüche

**1.** Verfahren zur Bestimmung einer Anfälligkeit für eine aus der aus Basaliom, Lungenkrebs, Blasenkrebs, Prostatakrebs, Gebärmutterhalskrebs, Endometriumkarzinom und kutanem Melanom bestehenden Gruppe ausgewählte Krebserkrankung bei einem menschlichen Individuum aus einer kaukasischen Population, wobei man bei dem Verfahren:

wenigstens ein Allel wenigstens eines aus der aus rs401681 und damit im Kopplungsungleichgewicht stehenden, aus der aus rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 und rs37005 bestehenden Gruppe ausgewählten Markern bestehenden Gruppe ausgewählten polymorphen Markers identifizierende Nukleinsäuresequenzdaten über ein menschliches Individuum gewinnt, wobei unterschiedliche Allele des wenigstens einen polymorphen Markers mit unterschiedlichen Anfälligkeiten für Krebs bei Menschen assoziiert sind,
wobei das Gewinnen von Nukleinsäuresequenzdaten die Sequenzanalyse des wenigstens einen polymorphen Markers in einer Nukleinsäure in einer von dem menschlichen Individuum erhaltenen Probe umfasst, und anhand der Nukleinsäuresequenzdaten eine Anfälligkeit für die Krebserkrankung bestimmt.

2. Verfahren nach Anspruch 1, wobei die Bestimmung einer Anfälligkeit das Vergleichen der Nukleinsäuresequenzdaten mit einer Korrelationsdaten zwischen dem wenigstens einen polymorphen Marker und Krebsanfälligkeit enthaltenden Datenbank umfasst.

3. Verfahren nach Anspruch 1, wobei die Sequenz-analyse des wenigstens einen polymorphen Markers das Bestimmen des Vorliegens oder Fehlens wenigstens eines Allels des wenigstens einen polymorphen Markers umfasst.

4. Verfahren nach einem der vorhergehenden Ansprüche, bei dem man ferner die Anfälligkeit wenigstens einer aus der aus dem Individuum, einem Vormund des Individuums, einem gentechnischen Dienstleister, einem Arzt, einer medizinischen Organisation und einem Krankenversicherer bestehenden Gruppe ausgewählten Instanz meldet.

5. Verfahren zur Identifizierung eines Markers zur Verwendung bei der Beurteilung von Anfälligkeit für eine aus der aus Basaliom, Lungenkrebs, Blasenkrebs, Prostatakrebs, Gebärmutterhalskrebs, Endometriumkarzinom und kutanem Melanom bestehenden Gruppe ausgewählte Krebserkrankung bei Menschen aus einer kaukasischen Population, wobei man bei dem Verfahren:

a. wenigstens einen polymorphen Marker im Kopplungsungleichgewicht mit wenigstens einem von rs401681 identifiziert, wobei das Kopplungsungleichgewicht durch $r^2$-Werte größer als 0,2 bei Kaukasiern gekennzeichnet ist;
b. den Genotypstatus einer Probe von mit der Krebserkrankung diagnostizierten oder eine Anfälligkeit dafür aufweisenden Individuen bestimmt; und
c. den Genotypstatus einer Probe von Kontrollindividuen bestimmt;

wobei ein signifikanter Unterschied der Häufigkeit wenigstens eines Allels in wenigstens einem Polymorphismus bei mit Krebs diagnostizierten oder eine Anfälligkeit dafür aufweisenden Individuen verglichen mit der Häufigkeit des wenigstens einen Allels in der Kontrollprobe darauf hindeutet, dass der wenigstens eine Polymorphismus für die Beurteilung von Anfälligkeit für die Krebserkrankung geeignet ist.

6. Verfahren gemäß Anspruch 5, wobei eine Erhöhung der Häufigkeit des wenigstens einen Allels in dem wenigstens einen Polymorphismus bei mit der Krebserkrankung diagnostizierten oder eine Anfälligkeit dafür aufweisenden Individuen verglichen mit der Häufigkeit des wenigstens einen Allels in der Kontrollprobe darauf hindeutet, dass der wenigstens eine Polymorphismus für die Beurteilung von erhöhter Anfälligkeit für die Krebserkrankung geeignet ist und wobei eine Abnahme der Häufigkeit des wenigstens einen Allels in dem wenigstens einen Polymorphismus bei mit der Krebserkrankung diagnostizierten oder eine Anfälligkeit dafür aufweisenden Individuen verglichen mit der Häufigkeit des wenigstens einen Allels in der Kontrollprobe darauf hindeutet, dass der wenigstens eine Polymorphismus für die Beurteilung von verminderter Anfälligkeit für die Krebserkrankung oder Schutz gegen die Krebserkrankung geeignet ist.

7. Verfahren zur Genotypisierung einer von einem menschlichen Individuum aus einer kaukasischen Population erhaltenen Nukleinsäureprobe, wobei Nukleinsäure in der Probe genotypisiert wird, um zu bestimmen, ob wenigstens ein Allel wenigstens eines polymorphen Markers in einer Nukleinsäureprobe von der Individuumprobe vorliegt, wobei der wenigstens eine Marker aus rs401681 und damit im Kopplungsungleichgewicht stehenden, aus der aus rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 und rs37005 bestehenden Gruppe ausgewählten Markern ausgewählt ist und wobei das Vorliegen des wenigstens einen Allels in der Probe auf eine Anfälligkeit für eine aus der aus Basaliom, Lungenkrebs, Blasenkrebs, Prostatakrebs, Gebärmutterhalskrebs, Endometriumkarzinom und kutanem Melanom bestehenden Gruppe ausgewählte Krebserkrankung bei dem Individuum hindeutet.

8. Vorrichtung zur Bestimmung eines genetischen Indikators für eine aus der aus Basaliom, Lungenkrebs, Blasenkrebs, Prostatakrebs, Gebärmutterhalskrebs, Endometriumkarzinom und kutanem Melanom bestehenden Gruppe ausgewählte Krebserkrankung bei einem menschlichen Individuum aus einer kaukasischen Population, umfassend:

einen Prozessor,
einen computerlesbaren Speicher mit per Computer ausführbaren Anweisungen, die zur Ausführung auf dem Prozessor adaptiert sind, um Marker- und/oder Haplotyp-Informationen für wenigstens ein menschliches Individuum in Bezug auf wenigstens einen aus rs401681 und damit im Kopplungsungleichgewicht stehenden, aus der aus rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 und rs37005 bestehenden Gruppe ausgewählten Markern ausgewählten polymorphen Marker zu analysieren und einen auf den Marker- bzw. Haplotyp-Informationen basierenden Output zu erzeugen, wobei der Output ein Risikomaß des wenigstens einen Markers bzw. Haplotyps als genetischen Indikator der Krebserkrankung für das menschliche Individuum umfasst.

**9.** Vorrichtung gemäß Anspruch 8, wobei der computerlesbare Speicher ferner Daten umfasst, die auf das Risiko hindeuten, die mit wenigstens einem Allel wenigstens eines polymorphen Markers oder wenigstens eines Haplotyps assoziierte Krebserkrankung zu entwickeln, und wobei ein Risikomaß für das menschliche Individuum auf einem Vergleich des Status des wenigstens einen Markers und/oder Haplotyps für das menschliche Individuum gegenüber dem Risiko der mit dem wenigstens einen Allel des wenigstens einen polymorphen Markers oder des wenigstens einen Haplotyps assoziierten Krebserkrankung beruht.

**10.** Verfahren zur Bestimmung einer Anfälligkeit für eine aus der aus Basaliom, Lungenkrebs, Blasenkrebs, Prostatakrebs, Gebärmutterhalskrebs, Endometriumkarzinom und kutanem Melanom bestehenden Gruppe ausgewählte Krebserkrankung bei einem menschlichen Individuum aus einer kaukasischen Population, wobei man bestimmt, ob wenigstens ein Allel wenigstens eines polymorphen Markers in einer von dem Individuum erhaltenen Nukleinsäureprobe vorliegt, wobei der wenigstens eine polymorphe Marker aus der aus rs401681 und damit im Kopplungsungleichgewicht stehenden, aus der aus rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 und rs37005 bestehenden Gruppe ausgewählten Markern bestehenden Gruppe ausgewählt ist und wobei das Vorliegen des wenigstens einen Allels auf eine Anfälligkeit für die Krebserkrankung für das Individuum hindeutet.

**11.** Verfahren oder Vorrichtung nach einem der Ansprüche 1 bis 4 oder 7 bis 10, wobei die Krebserkrankung aus der aus Basaliom, Lungenkrebs, Blasenkrebs, Prostatakrebs, Gebärmutterhalskrebs und Endometriumkarzinom bestehenden Gruppe ausgewählt ist und wobei die Bestimmung des Vorliegens von Allel C in rs401681 und/oder Allel A von rs2736098 auf eine erhöhte Anfälligkeit für die Krebserkrankung für das Individuum hindeutet.

**12.** Verfahren oder Vorrichtung nach einem der Ansprüche 1 bis 4 oder 7 bis 10, wobei es sich bei der Krebserkrankung um kutanes Melanom handelt und wobei die Bestimmung des Vorliegens von Allel C in rs401681 auf eine verminderte Anfälligkeit für die Krebserkrankung für das Individuum hindeutet.

## Revendications

**1.** Procédé de détermination d'une susceptibilité à un cancer choisi dans le groupe constitué d'un carcinome basocellulaire, un cancer du poumon, un cancer de la vessie, un cancer de la prostate, un cancer du col de l'utérus, un cancer de l'endomètre et un mélanome cutané chez un individu humain d'une population caucasienne, le procédé comprenant :

l'obtention de données de séquence d'acide nucléique concernant un individu humain identifiant au moins un allèle d'au moins un marqueur polymorphe choisi dans le groupe constitué de rs401681, et des marqueurs en déséquilibre de liaison avec celui-ci choisis dans le groupe constitué de rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070,
rs37008 et rs37005, différents allèles de l'au moins un marqueur polymorphe étant associés à différentes susceptibilités au cancer chez des humains,
dans lequel l'obtention de données de séquence d'acide nucléique comprend l'analyse de séquence de l'au moins un marqueur polymorphe dans un acide nucléique dans un échantillon obtenu à partir de l'individu humain, et
la détermination d'une susceptibilité au cancer à partir des données de séquence d'acide nucléique.

**2.** Procédé de la revendication 1, dans lequel la détermination d'une susceptibilité comprend la comparaison des données de séquence d'acide nucléique à une base de données contenant des données de corrélation entre l'au moins un marqueur polymorphe et la susceptibilité au cancer.

**3.** Procédé de la revendication 1, dans lequel l'analyse de séquence de l'au moins un marqueur polymorphe comprend la détermination de la présence ou l'absence d'au moins un allèle de l'au moins un marqueur polymorphe.

**4.** Procédé de l'une quelconque des revendications précédentes, comprenant en outre le signalement de la susceptibilité à au moins une entité choisie dans le groupe constitué de l'individu, un tuteur de l'individu, un fournisseur de service génétique, un médecin, une organisation médicale, et un assureur médical.

**5.** Procédé d'identification d'un marqueur pour utilisation dans l'évaluation de la susceptibilité à un cancer choisi dans le groupe constitué d'un carcinome basocellulaire, un cancer du poumon, un cancer de la vessie, un cancer de la prostate, un cancer du col de l'utérus, un cancer de l'endomètre et un mélanome cutané chez des humains d'une population caucasienne, le procédé comprenant :

a. l'identification d'au moins un marqueur polymorphe en déséquilibre de liaison avec au moins l'un de rs401681, le déséquilibre de liaison étant **caractérisé par** des valeurs de $r^2$ supérieures à 0,2 chez des caucasiens ;
b. la détermination du statut génotypique d'un échantillon d'individus diagnostiqués avec, ou ayant une susceptibilité à, un cancer ; et
c. la détermination du statut génotypique d'un échantillon d'individus témoins ;
dans lequel une différence significative de fréquence d'au moins un allèle dans au moins un polymorphisme chez des individus diagnostiqués avec, ou ayant une susceptibilité à, un cancer, par rapport à la fréquence de l'au moins un allèle dans l'échantillon témoin est indicative du fait que l'au moins un polymorphisme est utile pour évaluer la susceptibilité au cancer.

**6.** Procédé selon la revendication 5, dans lequel une augmentation de fréquence de l'au moins un allèle dans l'au moins un polymorphisme chez des individus diagnostiqués avec, ou ayant une susceptibilité à, un cancer, par rapport à la fréquence de l'au moins un allèle dans l'échantillon témoin est indicative du fait que l'au moins un polymorphisme est utile pour évaluer une susceptibilité augmentée au cancer, et dans lequel une diminution de fréquence de l'au moins un allèle dans l'au moins un polymorphisme chez des individus diagnostiqués avec, ou ayant une susceptibilité à, un cancer, par rapport à la fréquence de l'au moins un allèle dans l'échantillon témoin est indicative du fait que l'au moins un polymorphisme est utile pour évaluer une susceptibilité réduite à, ou une protection contre, le cancer.

**7.** Procédé de génotypage d'un échantillon d'acide nucléique obtenu à partir d'un individu humain d'une population caucasienne comprenant le génotypage d'acide nucléique dans ledit échantillon pour déterminer si au moins un allèle d'au moins un marqueur polymorphe est présent dans un échantillon d'acide nucléique de l'échantillon de l'individu, dans lequel l'au moins un marqueur est choisi parmi rs401681, et des marqueurs en déséquilibre de liaison avec celui-ci, choisis dans le groupe constitué de rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 et rs37005, et dans lequel la présence de l'au moins un allèle dans l'échantillon est indicative d'une susceptibilité à un cancer choisi dans le groupe constitué d'un carcinome basocellulaire, un cancer du poumon, un cancer de la vessie, un cancer de la prostate, un cancer du col de l'utérus, un cancer de l'endomètre et un mélanome cutané chez l'individu.

**8.** Appareil pour déterminer un indicateur génétique pour un cancer choisi dans le groupe constitué d'un carcinome basocellulaire, un cancer du poumon, un cancer de la vessie, un cancer de la prostate, un cancer du col de l'utérus, un cancer de l'endomètre et un mélanome cutané chez un individu humain d'une population caucasienne, comprenant :

un processeur
une mémoire lisible par ordinateur ayant des instructions exécutables par ordinateur adaptées pour être exécutées sur le processeur pour analyser des informations de marqueur et/ou d'haplotype pour au moins un individu humain en ce qui concerne au moins un marqueur polymorphe choisi parmi rs401681, et des marqueurs en déséquilibre de liaison avec celui-ci choisis dans le groupe constitué de rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 et rs37005, et
la génération d'une sortie sur la base des informations de marqueur ou d'haplotype, la sortie comprenant une mesure de risque de l'au moins un marqueur ou haplotype en tant qu'indicateur génétique du cancer pour l'individu humain.

**9.** Appareil selon la revendication 8, dans lequel la mémoire lisible par ordinateur comprend en outre des données indicatives du risque de développement d'un cancer associé à au moins un allèle d'au moins un marqueur polymorphe ou au moins un haplotype, et dans lequel une mesure de risque pour l'individu humain est basée sur une comparaison de l'au moins un statut de marqueur et/ou d'haplotype pour l'individu humain au risque de cancer associé à l'au moins un allèle de l'au moins un marqueur polymorphe ou l'au moins un haplotype.

**10.** Procédé pour déterminer une susceptibilité à un cancer choisi dans le groupe constitué d'un carcinome basocellulaire, un cancer du poumon, un cancer de la vessie, un cancer de la prostate, un cancer du col de l'utérus, un cancer de l'endomètre et un mélanome cutané chez un individu humain d'une population caucasienne, comprenant la détermination du fait qu'au moins un allèle d'au moins un marqueur polymorphe est présent dans un échantillon d'acide nucléique obtenu à partir de l'individu dans lequel l'au moins un marqueur polymorphe est choisi dans le groupe constitué de rs401681, et de marqueurs en déséquilibre de liaison avec celui-ci choisis dans le groupe constitué de rs2736098, rs4635969, rs4975615, rs4975616, rs6554759, rs1801075, rs451360, rs402710, rs421629, rs380286, rs466502, rs465498, rs452932, rs452384, rs467095, rs31484, rs31489, rs31490, rs27070, rs37008 et rs37005, et la présence de l'au moins un allèle est indicative d'une susceptibilité au cancer pour l'individu.

**11.** Procédé ou appareil de l'une quelconque des revendications 1 à 4 ou 7 à 10, dans lequel le cancer est choisi dans le groupe constitué d'un carcinome basocellulaire, un cancer du poumon, un cancer de la vessie, un cancer de la prostate, un cancer du col de l'utérus et un cancer de l'endomètre, et dans lequel la détermination de la présence de l'allèle C dans rs401681 et/ou l'allèle A de rs2736098 est indicative d'une susceptibilité augmentée au cancer pour l'individu.

**12.** Procédé ou appareil de l'une quelconque des revendications 1 à 4 ou 7 à 10, dans lequel le cancer est un mélanome cutané, et dans lequel la détermination de la présence de l'allèle C dans rs401681 est indicative d'une susceptibilité diminuée au cancer pour l'individu.

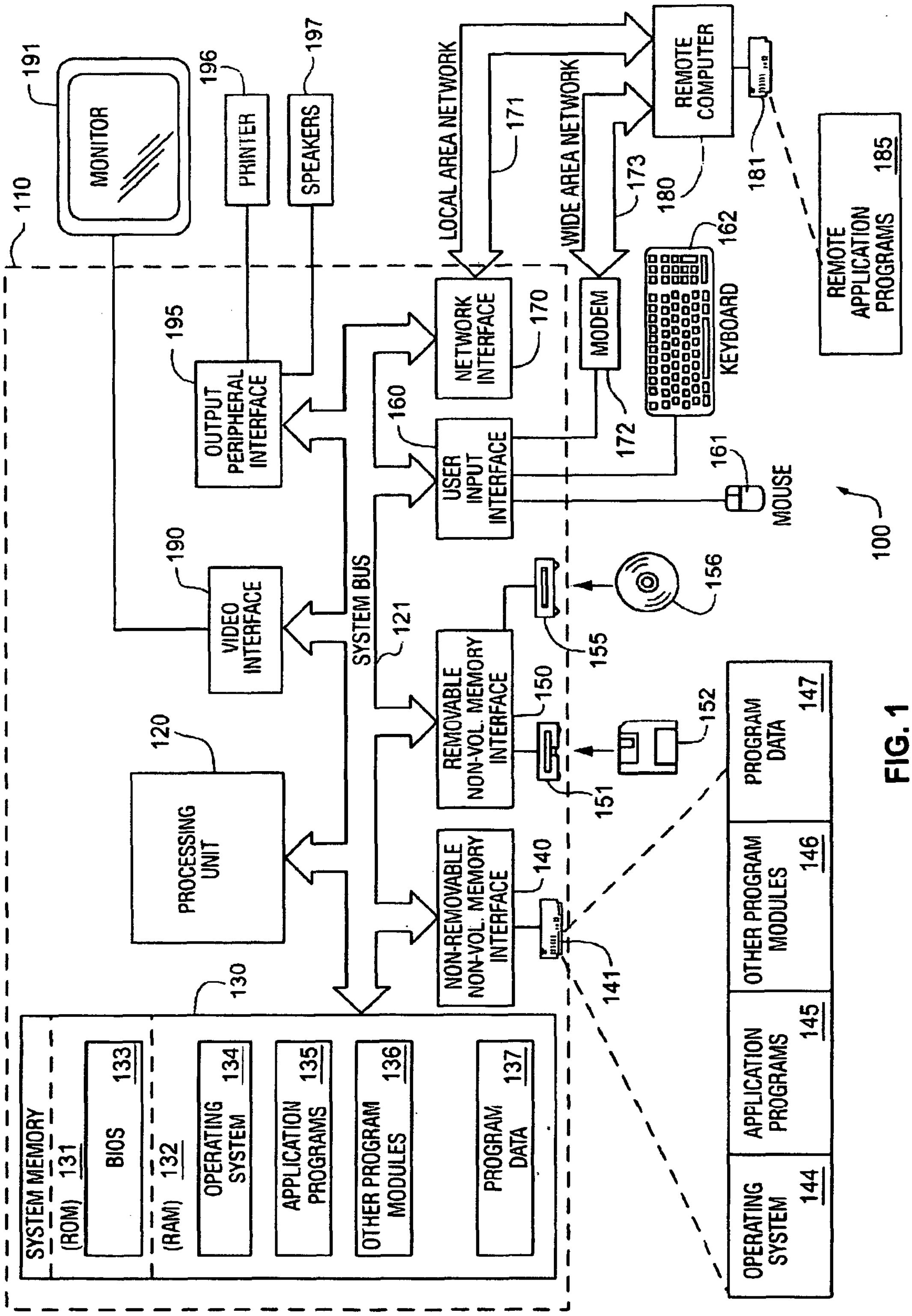

SYSTEM MEMORY
(ROM) 131
BIOS 133
(RAM) 132
OPERATING SYSTEM 134
APPLICATION PROGRAMS 135
OTHER PROGRAM MODULES 136
PROGRAM DATA 137
130
PROCESSING UNIT
120
VIDEO INTERFACE
190
OUTPUT PERIPHERAL INTERFACE
195
MONITOR
191
PRINTER
196
SPEAKERS
197
110
SYSTEM BUS
121
NON-REMOVABLE NON-VOL. MEMORY INTERFACE
140
141
REMOVABLE NON-VOL. MEMORY INTERFACE
150
151
152
155
156
USER INPUT INTERFACE
160
NETWORK INTERFACE
170
MODEM
172
LOCAL AREA NETWORK
171
WIDE AREA NETWORK
173
REMOTE COMPUTER
180
181
REMOTE APPLICATION PROGRAMS 185
KEYBOARD
162
MOUSE
161
100
OPERATING SYSTEM 144
APPLICATION PROGRAMS 145
OTHER PROGRAM MODULES 146
PROGRAM DATA 147


FIG. 1

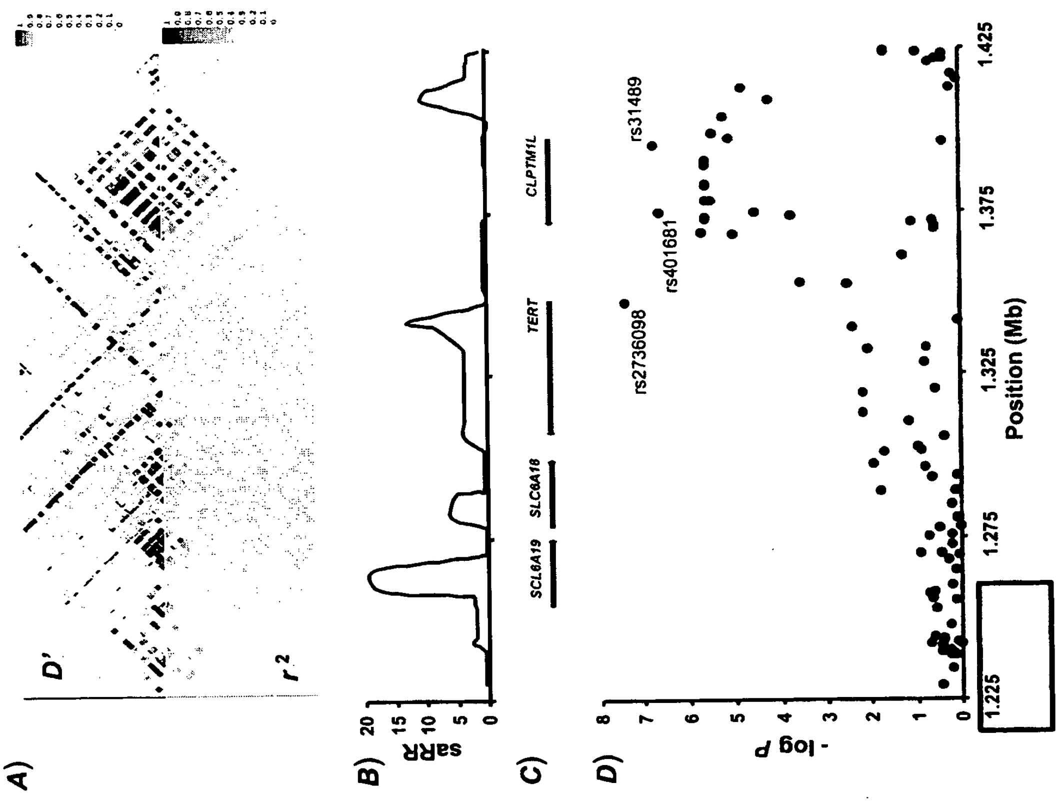
A)
D'
r2
B)
saRR
20
15
10
5
0
C)
SCL6A19
SLC6A18
TERT
CLPTM1L
D)
- log P
rs2736098
rs401681
rs31489
1.225
1.275
1.325
1.375
1.425
Position (Mb)

FIG. 2

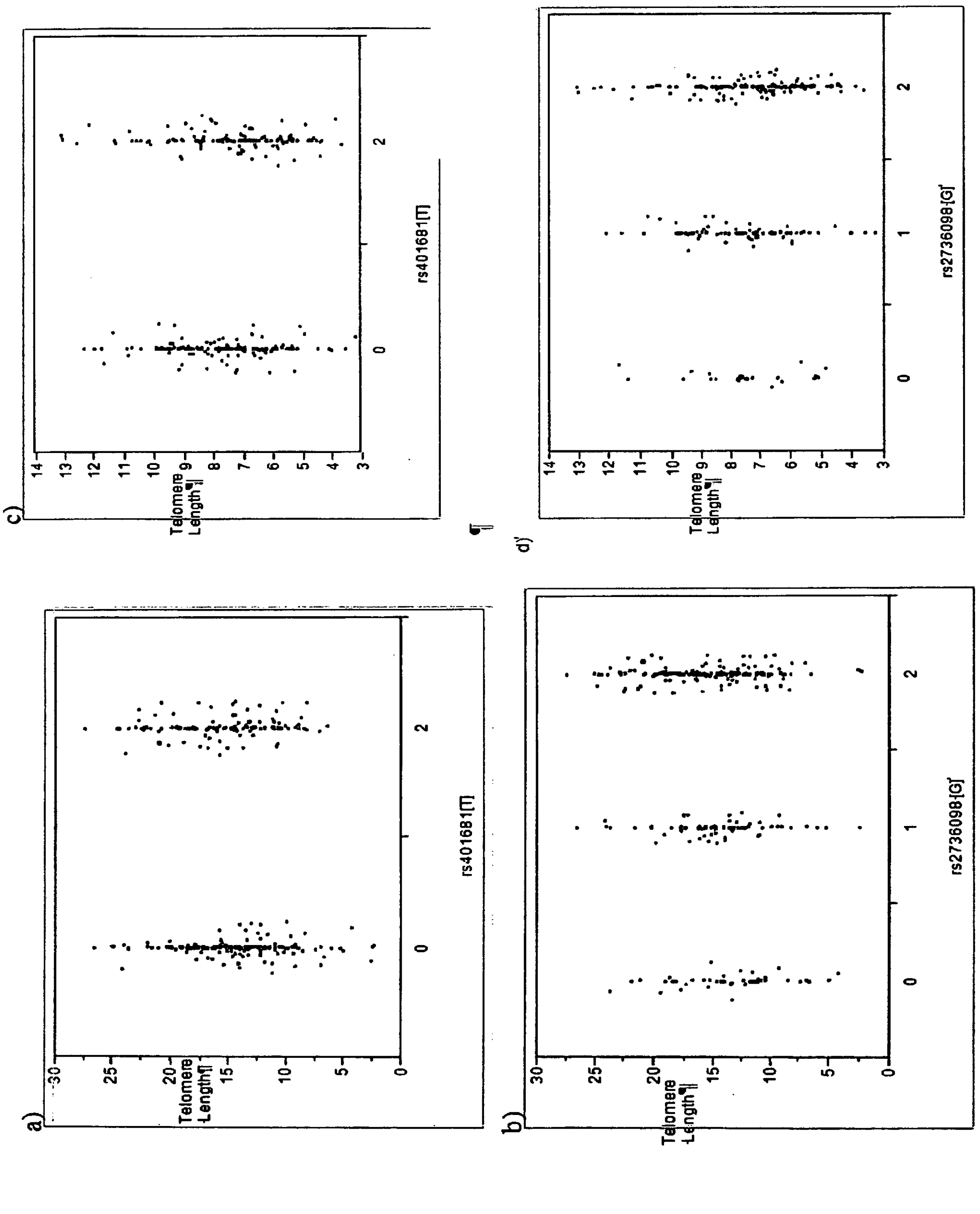
a)
Telomere Length
rs401681[T]
b)
Telomere Length
rs2736098[G]
c)
Telomere Length
rs401681[T]
d)
Telomere Length
rs2736098[G]

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6858394 B
- US 6429027 B
- US 5445934 A
- US 5700637 A
- US 5744305 A
- US 5945334 A
- US 6054270 A
- US 6300063 B
- US 6733977 B
- US 7364858 B
- EP 619321 A
- EP 373203 A
- US 5288644 A, Beavis
- US 4376110 A, David
- US 5223409 A
- WO 9218619 A
- WO 9117271 A
- WO 9220791 A
- WO 9215679 A
- WO 9301288 A
- WO 9201047 A
- WO 9209690 A
- WO 9002809 A


### Non-patent literature cited in the description


- **JEMAL, A. et al.** *CA Cancer J. Clin.,* 2002, vol. 52, 23-47
- **JONSSON, S. et al.** *JAMA,* 2004, vol. 292, 2977-83
- **HEMMINKI, K. et al.** *Genet Epidemiol,* 2001, vol. 20, 107-116
- **HANAHAN, D. ; WEINBER, R.A.** *Cell,* 2000, vol. 100, 57-70
- **CANNON-ALBRIGHT, L.A. et al.** *Cancer Res,* 1994, vol. 54, 2378-85
- **AMUNDADOTTIR, L.T. et al.** *PLoS Med,* 2004, vol. 1, 65
- **MALKIN, D. et al.** *Science,* 1990, vol. 250, 1233-38
- **ROEWERT-HUBER et al.** *Br J Dermatol,* 2007, vol. 157 (2), 47-51
- **LEAR et al.** *Clin Exp Dermatol,* 2005, vol. 30, 49-55
- **XU ; KOO.** *Int J Dermatol,* 2006, vol. 45, 1275-83
- **BASTIAENS et al.** *Am J Hum Genet,* 2001, vol. 68, 884-94
- **HAN et al.** *Int J Epidemiol,* 2006, vol. 35, 1514-21
- **HARTEVELT et al.** *Transplantation,* 1990, vol. 49, 506-9
- **LINDELOF et al.** *Br Dermatol,* 2000, vol. 143, 513-9
- **PARKIN et al.** *Int J Cancer,* 2001, vol. 94, 153-6
- **LENS ; DAWES.** *Br Dermatol,* 2004, vol. 150, 179-85
- **MARKOVIC et al.** *Mayo Clin Proc,* 2007, vol. 82, 364-80
- **GOODMAN, G.E.** *Thorax,* 2002, vol. 57, 994-999
- **JONSSON.** *JAMA,* 2004, vol. 292 (24), 2977-83
- **AMUNDADOTTIR.** *PLoS Med.,* 2004, vol. 1 (3), 65
- **PETO et al.** *BMJ,* 2000, vol. 321, 323-32
- **BRENNAN et al.** *Am J Epidemiol,* 2006, vol. 164, 1233-1241
- **DOLL et al.** *BMJ,* 1994, vol. 309, 901-911
- **ZHU et al.** *Cancer Res,* 2001, vol. 61, 7825-7829
- **KJAERHEIM et al.** *Cancer Causes Control,* 1998, vol. 9, 99-108
- **SPITZ et al.** *J Clin Oncol,* 2005, vol. 23, 267-275
- **HUNG et al.** *J Natl Cancer Inst,* 2005, vol. 97, 567-576
- **HUNG et al.** *Cancer Res,* 2006, vol. 66, 8280-8286
- **LANDI et al.** *Carcinogenesis,* 2006
- **BRENNAN et al.** *Lancet,* 2005, vol. 366, 1558-60
- **HUNG et al.** *Carcinogenesis,* 2007, vol. 28, 1334-40
- **CAMPA et al.** *Cancer Causes Control,* 2007, vol. 18, 449-455
- **GEMIGNANI et al.** *Carcinogenesis,* 2007, vol. 28 (6), 1287-93
- **HALL et al.** *Carcinogenesis,* 2007, vol. 28, 665-671

- **CAMPA et al.** *Cancer Epidemiol Biomarkers Prev,* 2005, vol. 14, 2457-2458
- **CAMPA et al.** *Cancer Epidemiol Biomarkers Prev,* 2005, vol. 14, 538-539
- **HASHIBE et al.** *Cancer Epidemiol Biomarkers Prev,* 2006, vol. 15, 696-703
- **JONSSON et al.** *JAMA,* 2004, vol. 292, 2977-2983
- **LI ; HEMMINKI.** *Lung Cancer,* 2005, vol. 47, 301-307
- **GOLDGAR et al.** *J Natl Cancer Inst,* 1994, vol. 86, 1600-1608
- **PHAROAH et al.** *NatGenet,* 2002, vol. 31, 33-36
- **ABEN, K.K. et al.** *Int J Cancer,* 2002, vol. 98, 274-8
- **AMUNDADOTTIR, L.T. et al.** *PLoS Med,* 28 December 2004, vol. 1, 65
- **MURTA-NASCIMENTO, C. et al.** *Cancer Epidemiol Biomarkers Prev,* 2007, vol. 16, 1595-600
- **ABEN, K.K. et al.** *Eur J Cancer,* 2006, vol. 42, 1428-33
- **SANDERSON, S. et al.** *Am J Epidemiol,* 2007, vol. 166, 741-51
- **LOKESHWAR, V.B. et al.** *Urology,* 2005, vol. 66, 35-63
- **FRIEDRICH, M.G et al.** *BJU Int,* 2003, vol. 92, 389-92
- **RAMAKUMAR, S. et al.** *J Urol,* 1999, vol. 161, 388-94
- **SOZEN, S. et al.** *Eur Urol,* 1999, vol. 36, 225-9
- **HEICAPPELL, R. et al.** *Urol Int,* 2000, vol. 65, 181-4
- **PESCHEL, R.E. ; J.W. COLBERG.** *Lancet,* 2003, vol. 4, 233-41
- **NELSON, W.G. et al.** *N. Engl. J. Med.,* 2003, vol. 349 (4), 366-81
- **SIMARD, J. et al.** *Endocrinology,* 2002, vol. 143 (6), 2029-40
- **LICHTENSTEIN P.** *N. Engl. J. Med.,* 2000, vol. 343 (2), 78-85
- **AMUNDADOTTIR.** *PLoS Medicine,* 2004, vol. 1 (3), 65
- **RIES, L.A.G. et al.** *NIH Pub. No. 99,* 1999, 4649
- **PUNGLIA.** *N Engl J Med.,* 2003, vol. 349 (4), 335-42
- **COOKSTON, M.S.** *Cancer Control,* 2001, vol. 8 (2), 133-40
- **THOMPSON, I.M.** *N Engl J Med.,* 2004, vol. 350, 2239-46
- **MISTRY K.J.** *Am. Board Fam. Pract.,* 2003, vol. 16 (2), 95-101
- **NWOSU, V. et al.** *Hum. Mol. Genet.,* 2001, vol. 10 (20), 2313-18
- **OSTRANDER E.A. ; J.L. STANFORD.** *Am. J. Hum. Genet.,* 2000, vol. 67, 1367-75
- **CUNNINGHA.** *Prostate,* 2005, vol. 57
- **CARPTEN, J. et al.** *Nat. Genet.,* 2002, vol. 30, 181-84
- **CASEY, G. et al.** *Nat. Genet.,* 2002, vol. 32 (4), 581-83
- **ROKMAN, A. et al.** *Am J. Hum. Genet.,* 2002, vol. 70, 1299-1304
- **RENNERT, H. et al.** *Am J. Hum. Genet.,* 2002, vol. 71, 981-84
- **STANFORD, J.L.** *Cancer Epidemiol. Biomarkers Prev.,* 2003, vol. 12 (9), 876-81
- **WANG, L. et al.** *Am. J. Hum. Genet.,* 2002, vol. 71, 116-23
- **WIKLUND, F. et al.** *Clin. Cancer Res.,* 2004, vol. 10 (21), 7150-56
- **MAIER, C.** *Br. J. Cancer,* 2005, vol. 92 (6), 1159-64
- **XU, J. et al.** *Nat. Genet.,* 2002, vol. 32, 321-25
- **LINDMARK, F. et al.** *Prostate,* 2004, vol. 59 (2), 132-40
- **SEPPALA, E.H. et al.** *Clin. Cancer Res.,* 2003, vol. 9 (14), 5252-56
- **WANG, L. et al.** *Nat Genet.,* 2003, vol. 35 (2), 128-29
- **MILLER, D.C. et al.** *Cancer Res.,* 2003, vol. 63 (13), 3486-89
- **TAVTIGIAN, S.V. et al.** *Nat. Genet.,* 2001, vol. 27 (2), 172-80
- **REBBECK, T.R. et al.** *Am. J. Hum. Genet.,* 2000, vol. 67 (4), 1014-19
- **WANG, L. et al.** *Cancer Res.,* 2001, vol. 61 (17), 6494-99
- **VESPRINI, D. et al.** *Am. J. Hum. Genet.,* 2001, vol. 68 (4), 912-17
- **SHEA, P.R. et al.** *Hum. Genet.,* 2002, vol. 111 (4-5), 398-400
- **SUAREZ, B.K. et al.** *Cancer Res.,* 2001, vol. 61 (13), 4982-84
- **SEVERI, G. et al.** *J. Natl. Cancer Inst.,* 2003, vol. 95 (11), 818-24
- **FUJIWARA, H. et al.** *J. Hum. Genet.,* 2002, vol. 47 (12), 641-48
- **CAMP, N.J. et al.** *Am. J. Hum. Genet.,* 2002, vol. 71 (6), 1475-78
- **CHANG, B. et al.** *Int. J. Cancer,* 2001, vol. 95, 354-59
- **MAKRIDAKIS, N.M. et al.** *Lancet,* 1999, vol. 354, 975-78
- **NAM, R.K. et al.** *Urology,* 2001, vol. 57, 199-204
- **GUDMUNDSSON, J. et al.** *Nat Genet,* 2008, vol. 40, 281-283
- **THOMAS, G. et al.** *Nat Genet,* 2008, vol. 40, 310-315
- **GUDMUNDSSON, J. et al.** *Nat Genet,* 2007, vol. 39, 977-983
- **YEAGER, M. et al.** *Nat Genet,* 2007, vol. 39, 645-649

- **GUDMUNDSSON, J. et al.** *Nat Genet,* 2007, vol. 39, 631-637
- **AMUNDADOTTIR, L.T. et al.** *Nat Genet,* 2007, vol. 38, 652-658
- **HAIMAN, C.A. et al.** *Nat Genet,* 2007, vol. 39, 638-644
- **PARKIN DM.** *CA Cancer J Clin,* 2005, vol. 55, 74-108
- **JEMAL A.** *CA Cancer J Clin.,* 2006, vol. 56, 106-30
- **LICHTENSTEIN P.** *N Engl J Med,* 2000, vol. 343, 78-85
- **PETO J ; MACK TM.** *Nat Genet,* 2000, vol. 26, 411-4
- **RISCH N.** *Cancer Epidemiol Biomarkers Prev,* 2001, vol. 10, 733-41
- **DE LA CHAPELLE, A.** *Fam Cancer,* 2005, vol. 4, 233-7
- **PARKIN, D. M. et al.** *CA Cancer J Clin,* 2005, vol. 55, 74-108
- SEER Cancer Statistics Review, 1975-2005. National Cancer Institute, 2007
- **MUNOZ, N.** *J Clin Virol,* 2000, vol. 19, 1-5
- **WALBOOMERS, J. M. et al.** *J Pathol,* 1999, vol. 189, 12-9
- **HAUSEN, H.** *J Natl Cancer Inst,* 2000, vol. 92, 690-8
- **ANTTILA, T. et al.** *JAMA,* 2001, vol. 285, 47-51
- **MURTHY, N. S. ; MATHEW, A.** *Eur J Cancer Prev,* 2000, vol. 9, 5-14
- **CZENE, K. et al.** *Int J Cancer,* 2002, vol. 99, 260-6
- **HEMMINKI, K. ; CHEN, B.** *Cancer Epidemiol Biomarkers Prev,* 2006, vol. 15, 1413-4
- **COUTO, E. ; HEMMINKI, K.** *Int J Cancer,* 2006, vol. 119, 2699-701
- **SCHIFFMAN, M. et al.** *Lancet,* 2007, vol. 370, 890-907
- **CUTTS, F. T. et al.** *Bull World Health Organ,* 2007, vol. 85, 719-26
- **STACEY, S.N. et al.** *Nat Genet.,* 2007, vol. 39, 865-69
- **YEAGER, M. et al.** *Nat Genet,* vol. 39, 645-649
- **HAIMAN, C.A. et al.** *Nat Genet,* vol. 39, 638-644
- **EASON, D.F. et al.** *Nature,* 2007, vol. 447, 1087-1093
- **TOMLINSON, I. et al.** *Nat Genet,* 2007, vol. 39, 984-988
- **GUDBJARTSSON, D.F. et al.** *Nat Genet,* 2008, vol. 40, 886-891
- **STACEY, S.N. et al.** *Nat Genet,* 2008, vol. 40, 703-706
- **THORGEIRSSON, T.E. et al.** *Nature,* 2008, vol. 452, 638-642
- **EELES, R.A. et al.** *Nat Genet,* 2008, vol. 40, 316-321
- **HUNG, R.J. et al.** *Nature,* 2008, vol. 452, 633-637
- **AMOS, C.I. et al.** *Nat Genet,* 2008, vol. 40, 616-622
- **FRAZER, K.A. et al.** *Nature,* 2007, vol. 449, 851-61
- **YAMAMOTO, K. et al.** *Biochem Biophys Res Comm,* 2001, vol. 280, 1148-54
- **AYOUAZ, A. et al.** *Biochimie,* 2008, vol. 90, 60-72
- **COLLINS, K.** *Curr Opin Cell Biol,* 2000, vol. 12, 378-83
- **ALLSOPP, R. C. et al.** *Proc Natl Acad Sci U S A,* 1992, vol. 89, 10114-8
- **CAMPISI.** *J. Eur J Cancer,* 1997, vol. 33, 703-9
- **GREIDER, C. W. ; BLACKBURN, E. H.** *Cell,* 1985, vol. 43, 405-13
- **DENG, Y. ; CHANG, S.** *Lab Invest,* 2007, vol. 87, 1071-6
- **HARLEY, C. B.** *Nat Rev Cancer,* 2008, vol. 8, 167-79
- **HASTIE, N. D. et al.** *Nature,* 1990, vol. 346, 866-8
- **SLAGBOOM, P. E. et al.** *Am J Hum Genet,* 1994, vol. 55, 876-82
- **WU, X. et al.** *J Natl Cancer Inst,* 2003, vol. 95, 1211-8
- **SHEN, J. et al.** *Cancer Res,* 2007, vol. 67, 5538-44
- **JANG, J. S. et al.** *Cancer Sci,* 2008, vol. 99, 1385-9
- **MCGRATH, M. et al.** *Cancer Epidemiol Biomarkers Prev,* 2007, vol. 16, 815-9
- **EPEL, E. S. et al.** *Proc Natl Acad Sci U S A,* 2004, vol. 101, 17312-5
- **ESTIVILL, X ; ARMENGOL; L.** *PloS Genetics,* 2007, vol. 3, 1787-99, http://projects.tcag.ca/variation
- **REDON, R. et al.** *Nature,* 2006, vol. 23, 444-454
- **CARTER.** *Nature Genetics,* 2007, vol. 39, S16-S21
- **CHEN, X. et al.** *Genome Res.,* 1999, vol. 9 (5), 492-98
- **KUTYAVIN et al.** *Nucleic Acid Res.,* 2006, vol. 34, 128
- **GUDMUNDSSON, J. et al.** *Nat Genet,* 2007, vol. 39, 631-7
- **GUDMUNDSSON, J. et al.** *Nat Genet,* 2007, vol. 39, 977-83
- **YEAGER, M. et al.** *Nat Genet,* 2007, vol. 39, 645-49
- **AMUNDADOTTIR, L. et al.** *Nat Genet,* 2006, vol. 38, 652-8

- **HAIMAN, C.A. et al.** *Nat Genet,* 2007, vol. 39, 638-44
- **MYERS, S. et al.** *Biochem Soc Trans,* 2006, vol. 34, 526-530
- **JEFFREYS, A.J.** *Nature Genet,* 2001, vol. 29, 217-222
- **MAY, C.A. et al.** *Nature Genet,* 2002, vol. 31, 272-275
- **DEVLIN, B. ; RISCH, N.** *Genomics,* 1995, vol. 29, 311-22
- **LEWONTIN, R.** *Genetics,* 1964, vol. 49, 49-67
- **HILL, W.G. ; ROBERTSON, A.** *Theor. Appl. Genet.,* 1968, vol. 22, 226-231
- **RISCH, N. ; MERKIANGAS, K.** *Science,* 1996, vol. 273, 1516-1517
- **MANIATIS, N. et al.** *Proc Natl Acad Sci USA,* 2002, vol. 99, 2228-2233
- **REICH, DE et al.** *Nature,* 2001, vol. 411, 199-204
- **WALL., J.D. ; PRITCHARD, J.K.** *Nature Reviews Genetics,* 2003, vol. 4, 587-597
- **DALY, M. et al.** *Nature Genet.,* 2001, vol. 29, 229-232
- **GABRIEL, S.B. et al.** *Science,* 2002, vol. 296, 2225-2229
- **PATIL, N. et al.** *Science,* 2001, vol. 294, 1719-1723
- **DAWSON, E. et al.** *Nature,* 2002, vol. 418, 544-548
- **PHILLIPS, M.S. et al.** *Nature Genet.,* 2003, vol. 33, 382-387
- **ZHANG, K. et al.** *Proc. Natl. Acad. Sci. USA,* 2002, vol. 99, 7335-7339
- **WANG, N. et al.** *Am. J. Hum. Genet.,* 2002, vol. 71, 1227-1234
- **STUMPF, M.P. ; GOLDSTEIN, D.B.** *Curr. Biol.,* 2003, vol. 13, 1-8
- **MYERS, S. et al.** *Science,* 2005, vol. 310, 321-32324
- **MYERS, S. et al.** *Biochem Soc Trans,* 2006, vol. 34, 526530
- **DEMPSTER A. et al.** *J. R. Stat. Soc. B,* 1977, vol. 39, 1-38
- **GRETARSDOTTIR S. et al.** *Nat. Genet.,* 2003, vol. 35, 131-38
- *Biometrics,* 2004, vol. 60 (2), 368-75
- **RISCH, N. ; TENG, J.** *Genome Res.,* 1998, vol. 8, 1273-1288
- **DEVLIN, B. ; ROEDER, K.** *Biometrics,* 1999, vol. 55, 997
- **TERWILLIGER, J.D. ; OTT, J.** *Hum. Hered.,* 1992, vol. 42, 337-46
- **FALK, C.T. ; RUBINSTEIN, P.** *Ann. Hum. Genet.,* 1987, vol. 51, 227-33
- **MANTEL ; HAENSZEL.** *J Natl Cancer Inst,* 1959, vol. 22, 719-48
- **STYRKARSDOTTIR, U. et al.** *N Engl J Med,* 29 April 2008
- **THORGEIRSSON, T. et al.** *Nature,* 2008, vol. 452, 638-42
- **GUDMUNDSSON, J. et al.** *Nat Genet.,* 2008, vol. 40, 281-3
- **STACEY, S.N et al.** *Nat Genet.,* 2007, vol. 39, 865-69
- **HELGADOTTIR, A. et al.** *Science,* 2007, vol. 316, 1491-93
- **STEINTHORSDOTTIR, V. et al.** *Nat Genet.,* 2007, vol. 39, 770-75
- **GUDMUNDSSON, J. et al.** *Nat Genet.,* 2007, vol. 39, 631-37
- **FRAYLING, TM.** *Nature Reviews Genet,* 2007, vol. 8, 657-662
- **AMUNDADOTTIR, L.T. et al.** *Nat Genet.,* 2006, vol. 38, 652-58
- **GRANT, S.F. et al.** *Nat Genet.,* 2006, vol. 38, 320-23
- **SMITH et al.** *Am J Hum Genet,* 2004, vol. 74, 1001-13
- **HEMMINKI, K. et al.** *Arch Dermatol,* 2003, vol. 139, 885
- **VITASA, B.C. et al.** *Cancer,* 1990, vol. 65, 2811
- **WONG et al.** *BMJ,* 2003, vol. 327, 794
- **GUDBJARTSSON.** *Nat. Gen.,* 2008, vol. 40, 886
- **BASTIAENS et al.** *Am J Hum Genet,* 2001, vol. 68, 884
- **HAN et al.** *Int J Epidemiol,* 2006, vol. 35, 1514
- **BOWDEN.** *Nat Rev Cancer,* 2004, vol. 4, 23
- **AMUNDADOTTIR et al.** *PLoS Med,* 28 December 2004, vol. 1, 65
- **BATAILLE.** *Eur J Cancer,* 2003, vol. 39, 1341-7
- **ZUO et al.** *Nat Genet,* 1996, vol. 12, 97-9
- **BISHOP et al.** *J Natl Cancer Inst,* 2002, vol. 94, 894-903
- **BOWDEN.** *Nat Rev Cancer,* 2004, vol. 4, 23-35
- **GUDBJARTSSON.** *Nature Genetics,* 2008, vol. 40, 886-91
- **LICHTENSTEIN et al.** *N Engl J Med,* 2000, vol. 343, 78
- **RISCH.** *Cancer Epidemiol Biomarkers Prev,* 2001, vol. 7, 733
- **AMUNDADOTTIR et al.** *Nat Gen,* 2006, vol. 38, 652
- **GUDMUNDSSON et al.** *Nat Gen,* 2007, vol. 39, 631
- **GUDMUNDSSON et al.** *Nat Gen,* 2007, vol. 39, 977
- **GUDMUNDSSON et al.** *Nat Gen,* 2008, vol. 40, 281
- **EELES et al.** *Nat Gen,* 2008, vol. 40, 316
- **YEAGER et al.** *Nature Gen,* 2007, vol. 39, 645
- **DRAISMA G et al.** *J Natl Cancer Inst,* 2003, vol. 95, 868

- **JONSSON.** *JAMA,* 2004, vol. 292, 2977
- **AMUNDADOTTIR.** *PLoS Med.,* 2004, vol. 1, 65
- **THORGEIRSSON et al.** *Nature,* 2008, vol. 452, 638
- **ABEN, K.K. et al.** *Int J Cancer,* 2002, vol. 98, 274
- **MURTA-NASCIMENTO, C. et al.** *Cancer Epidemiol Biomarkers Prev,* 2007, vol. 16, 1595
- **ABEN, K.K. et al.** *Eur J Cancer,* 2006, vol. 42, 1428
- **CZENE, K. et al.** *Int J Cancer,* 2002, vol. 99, 260
- **HEMMINKI, K. ; CHEN, B.** *Cancer Epidemiol Biomarkers Prev,* 2006, vol. 15, 1413
- **COUTO, E. ; HEMMINKI, K.** *Int J Cancer,* 2006, vol. 119, 2699
- **HILDESHEIM ; WANG.** *Virus Res,* 2002, vol. 89, 229
- **SCHIFFMAN, M. et al.** *Lancet,* 2007, vol. 370, 890
- **DAVIES, L. ; WELCH, H. G.** *Jama,* 2006, vol. 295, 2164
- **ROJESKI, M. T. ; GHARIB, H.** *N Engl J Med,* 1985, vol. 313, 428
- **ROSS, D. S.** *J Clin Endocrinol Metab,* 2006, vol. 91, 4253
- **BONDESON, L. ; LJUNGBERG, O.** *Cancer,* 1981, vol. 47, 319
- **HARACH, H. R. et al.** *Cancer,* 1985, vol. 56, 531
- **SOLARES, C. A. et al.** *Am J Otolaryngol,* 2005, vol. 26, 87
- **SOBRINHO-SIMOES, M. et al.** *Cancer,* 1979, vol. 43, 1702
- Current Protocols in Molecular Biology. John Wiley & Sons
- **NIELSEN, P et al.** *Bioconjug. Chem.,* 1994, vol. 5, 3-7
- **BIER, F.F. et al.** *Adv Biochem Eng Biotechnol,* 2008, vol. 109, 433-53
- **HOHEISEL, J.D.** *Nat Rev Genet,* 2006, vol. 7, 200-10
- **FAN, J.B. et al.** *Methods Enzymol,* 2006, vol. 410, 57-73
- **RAQOUSSIS, J. ; ELVIDGE, G.** *Expert Rev Mol Diagn,* 2006, vol. 6, 145-52
- **MOCKLER, T.C. et al.** *Genomics,* 2005, vol. 85, 1-15
- **CHURCH ; GILBERT.** *Proc. Natl. Acad. Sci. USA,* 1988, vol. 81, 1991-1995
- **SANGER, F. et al.** *Proc. Natl. Acad. Sci. USA,* 1977, vol. 74, 5463-5467
- **SHEFFIELD, V. et al.** *Proc. Natl. Acad. Sci. USA,* 1989, vol. 86, 232-236
- **ORITA, M. et al.** *Proc. Natl. Acad. Sci. USA,* 1989, vol. 86, 2766-2770
- **FLAVELL, R. et al.** *Cell,* 1978, vol. 15, 25-41
- **GEEVER, R. et al.** *Proc. Natl. Acad. Sci. USA,* 1981, vol. 78, 5081-5085
- **COTTON, R. et al.** *Proc. Natl. Acad. Sci. USA,* 1985, vol. 85, 4397-4401
- **MYERS, R. et al.** *Science,* 1985, vol. 230, 1242-1246
- AntisenseDrug Technology: Principles, Strategies, and Applications. Marcel Dekker Inc, 2001
- **THOMPSON.** *Drug Discovery Today,* 2002, vol. 7, 912-917
- **LAVERY et al.** *Curr. Opin. Drug Discov. Devel.,* 2003, vol. 6, 561-569
- **STEPHENS et al.** *Curr. Opin. Mol. Ther.,* 2003, vol. 5, 118-122
- **KURRECK.** *Eur. J. Biochem.,* 2003, vol. 270, 1628-44
- **DIAS et al.** *Mol. Cancer Ter.,* 2002, vol. 1, 347-55
- **CHEN.** *Methods Mol. Med.,* 2003, vol. 75, 621-636
- **WANG et al.** *Curr. Cancer Drug Targets,* 2001, vol. 1, 177-96
- **BENNETT.** *Antisense Nucleic Acid Drug.Dev.,* 2002, vol. 12, 215-24
- **FIRE et al.** *Nature,* 1998, vol. 391, 806-11
- **KIM ; ROSSI.** *Nature Rev. Genet.,* 2007, vol. 8, 173-204
- *FEBS Lett.,* 2005, vol. 579, 5974-81
- **KIM et al.** *Nature Biotechnol.,* 2005, vol. 23, 222-226
- **SIOLAS et al.** *Nature Biotechnol.,* 2005, vol. 23, 227-231
- **MARQUES et al.** *Nature Biotechnol.,* 2006, vol. 23, 559-565
- **BRUMMELKAMP et al.** *Science,* 2002, vol. 296, 550-553
- **KIM ; ROSSI.** *Nat. Rev. Genet.,* 2007, vol. 8, 173-184
- **CHEN ; RAJEWSKY.** *Nat. Rev. Genet.,* 2007, vol. 8, 93-103
- **REYNOLDS et al.** *Nat. Biotechnol.,* 2004, vol. 22, 326-330
- **CHI et al.** *Proc. Natl. Acad. Sci. USA,* 2003, vol. 100, 6343-6346
- **VICKERS et al.** *J. Biol. Chem.,* 2003, vol. 278, 7108-7118
- **AGAMI.** *Curr. Opin. Chem. Biol.,* 2002, vol. 6, 829-834
- **SHI.** *Trends Genet.,* 2003, vol. 19, 9-12
- **SHUEY et al.** *Drug Discov. Today,* 2002, vol. 7, 1040-46
- **MCMANUS et al.** *Nat. Rev. Genet.,* 2002, vol. 3, 737-747
- **XIA et al.** *Nat. Biotechnol.,* 2002, vol. 20, 1006-10
- **PLASTERK et al.** *curr. Opin. Genet. Dev.,* 2000, vol. 10, 562-7

- **BOSHER et al.** *Nat. Cell Biol.,* 2000, vol. 2, 31-6
- **HUNTER.** *Curr. Biol.,* 1999, vol. 9, R440-442
- Current Protocols in Molecular Biology. John Wiley & Sons, 1998
- **KRAUS, M. ; AARONSON, S.** *Methods Enzymol.,* 1991, vol. 200, 546-556
- **KARLIN, S. ; ALTSCHUL, S.** *Proc. Natl. Acad. Sci. USA,* 1993, vol. 90, 5873-5877
- **ALTSCHUL, S. et al.** *Nucleic Acids Res.,* 1997, vol. 25, 3389-3402
- **KENT, W.J.** *Genome Res.,* 2002, vol. 12, 656-64
- **MYERS ; MILLER.** *CABIOS,* 1989
- **TORELLIS, A ; ROBOTTI, C.** *Comput. Appl. Biosci.,* 1994, vol. 10, 3-5
- **PEARSON, W. ; LIPMAN, D.** *Proc. Natl. Acad. Sci. USA,* 1988, vol. 85, 2444-48
- **NIELSEN, P. et al.** *Science,* 1991, vol. 254, 1497-1500
- **KOHLER ; MILSTEIN.** *Nature,* 1973, vol. 256, 495-497
- **KOZBOR et al.** *Immunol. Today,* 1983, vol. 4, 72
- **COLE et al.** Monoclonal Antibodies and Cancer Therapy. Alan R. Liss, 1985, 77-96
- Current Protocols in Immunology. John Wiley & Sons, Inc, 1994
- **GALFRE et al.** *Nature,* 1977, vol. 266, 55052
- **R.H. KENNETH.** Monoclonal Antibodies: A New Dimension In Biological Analyses. Plenum Publishing Corp, 1980
- **LERNER.** *Yale J. Biol. Med.,* 1981, vol. 54, 387-402
- **FUCHS et al.** *Bio/Technology,* 1991, vol. 9, 1370-1372
- **HAY et al.** *Hum. Antibod. Hybridomas,* 1992, vol. 3, 81-85
- **HUSE et al.** *Science,* 1989, vol. 246, 1275-1281
- **GRIFFITHS et al.** *EMBO J.,* 1993, vol. 12, 725-734
- **YEAGER, M et al.** *Nat Genet,* 2007, vol. 39, 645-649
- **GUDMUNDSSON J et al.** *Nature Genetics,* 2007, vol. 39, 631-637
- **THORGEIRSSON TE et al.** *Nature,* vol. 452, 638-642
- **KIEMENEY LA et al.** *Nature Genetics*
- **STACEY SN et al.** *Nature Genetics*
- **BARRETT, J.C. ; CARDON, L.R.** *Nat Genet,* 2006, vol. 38, 659-662
- **KUTYAVIN, I.V. et al.** *Nucleic Acids Res,* 2006, vol. 34, 128
- **GUDBJARTSSON, D.F. et al.** *Nat Genet,* 2008, vol. 40, 609-15
- **RAFNAR, T. et al.** *Nat Rev Cancer,* 2004, vol. 4, 488-92
- **SLAGBOOM, PE et al.** *Am J Hum Genet,* 1994, vol. 55, 876-82
- **VALDES, IP et al.** *Nat Genet,* 2008, vol. 40, 623-30
- **HAN, J. et al.** *J Invest Dermatol,* 2009, vol. 129, 415-21
- **VALDES, AM et al.** *Lancet,* 2005, vol. 366, 662-664
- **FRENCK, R.W JR. et al.** *Proc Natl Acad Sci USA,* 1998, vol. 95, 5607-10
- **TULINIUS, H. et al.** *Cancer Epidemiol Biomarkers Prev,* 1997, vol. 6, 863-73
- **GUDBJARTSSON, DF et al.** *Nat Genet,* 2008, vol. 40, 886-91
- **STACEY, SN et al.** *Nat Genet,* 2008, vol. 40, 1313-18
- **STACEY SN et al.** *Nat Genet,* 2007, vol. 39, 865-9
- **THORGEIRSSON, TE et al.** *Nature,* 2008, vol. 452, 638-42
- **AMUNDADOTTIR, LT et al.** *Nat Genet,* 2006, vol. 38, 652-8
- **KIEMENEY, LA et al.** *Nat Genet,* 2008, vol. 40, 1307-12
- **WETZELS, JF et al.** *Kidney Int,* 2007, vol. 72, 632-7
- **GUDMUNDSSON, J et al.** *Nat Genet,* 2007, vol. 39, 631-7
- **THURUMARAN, RK et al.** *Carcinogenesis,* 2006, vol. 27, 1676-81
- **SAK, SC et al.** *Br J Cancer,* 2005, vol. 92, 2262-65
- **MATULLO, G. et al.** *Cancer Epidemiol Biomarkers Prev,* 2005, vol. 14, 2569-78
- **SHEN M. et al.** *Cancer Epidemiol Biomarkers Prev,* 2003, vol. 12, 1234-40
- **KELLEN, E et al.** *Int J Cancer,* 2006, vol. 118, 2572-78
- **LARSSON, P. et al.** *Scand J Urol Nephrol,* 2003, vol. 37, 195-201
- **HUNG, RJ et al.** *Nature,* 2008, vol. 452, 633-37
- **TOMLIONSON IP et al.** *Nat Genet,* 2008, vol. 40, 623-30
- **GUDMUNDSSON, J. et al.** *Nat Genet,* 2007, vol. 39, 631-37
- **BARRETT, JC ; CARDON, LR.** *Nat Genet,* 2006, vol. 38, 659-62
- **KUTYAVIN, IV et al.** *Nucleic Acids Res,* 2006, vol. 34, 128
- **MARTEN UM et al.** *Nat,* vol. 18, 76-80
- **CAWTHON, RM.** *Nucleic Acids Res,* 2002, vol. 30, 47
- **SCHWOB, AE et al.** *Mol Biol Cell,* 2008, vol. 19, 1548-60
- **WRITZL, K. et al.** *Hum Reprod,* 2006, vol. 21, 753-4

- **GRETARSDOTTIR, S. et al.** *Nat Genet,* 2003, vol. 35, 131-8
- **FALK CT ; RUBINSTEIN P.** *Ann Hum Genet,* 1987, vol. 51, 227-33
- **MANTEL N ; HAENSZEL W.** *J Natl cancer Inst,* 1959, vol. 22, 719-48
- **STYRKARSDOTTIR, U et al.** *N Eng J Med,* 2008, vol. 358, 2355-65
- **PE'ER, I et al.** *Nat Genet,* 2006, vol. 38, 663-7
- **NICOLAE, DL.** *Genet Epidemiol,* 2006, vol. 30, 718-27
- **ZAITLEN N. et al.** *Am J Hum Genet,* 2007, vol. 80, 683-91
- **DEVLIN B. et al.** *Nat Genet,* 2004, vol. 36, 1129-30